(19) Europäisches Patentamt
European Patent Office
Office européen des brevets

(11) EP 4 786 451 A1

(12) EUROPEAN PATENT APPLICATION
published in accordance with Art. 153(4) EPC

(43) Date of publication:
**05.08.2026 Bulletin 2026/32**

(21) Application number: **24870970.1**

(22) Date of filing: **27.09.2024**

(51) International Patent Classification (IPC):
***C07D 213/74*** $^{(2006.01)}$ ***A61K 31/44*** $^{(2006.01)}$

(52) Cooperative Patent Classification (CPC):
**A61K 31/44; C07D 213/74**

(86) International application number:
**PCT/CN2024/121785**

(87) International publication number:
**WO 2025/067433 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311286916**

(71) Applicant: **YUN HO BIO CO., LTD**
**Shanghai 201206 (CN)**

(72) Inventors:
- **WU, Yun**
  **Shanghai 201206 (CN)**
- **ZHU, Yun**
  **Shanghai 201206 (CN)**
- **MAO, Song**
  **Shanghai 201206 (CN)**

(74) Representative: **Simmons & Simmons LLP (Munich)**
**Lehel Carré**
**Gewürzmühlstraße 11**
**80538 Munich (DE)**

# (54) COMPOUND TARGETING INTEGRIN AND USE THEREOF IN RNAI AGENT

(57) Provided in the present invention is a ligand compound targeting integrin. The compound can be conjugated to small nucleic acid drug molecules by means of covalent conjugation, implementing in vivo delivery of small nucleic acid drug molecules, and is especially used for delivery to non-hepatic cells. Further provided in the present invention is an RNAi agent containing the integrin ligand compound.



Processed by Luminess, 75001 PARIS (FR)

## Description

## Technical Filed

The present invention relates to small molecule compounds targeting integrins and their use as targeting ligands in RNAi agents. The present invention also relates to intermediates for preparing the small molecule compounds, and RNAi agents comprising the small molecule compounds, pharmaceutical compositions, disease treatment methods, and uses.

## Background

Integrin proteins are ubiquitous heterodimeric transmembrane glycoprotein receptors, primarily serving as signaling proteins in mammals. Each integrin consists of an α-subunit and a β-subunit, with 18 and 8 variants respectively, generating 24 known heterodimers. The α- and β-subunits associate non-covalently to form a complex, creating a surface ligand-binding site. Integrins function as adhesion receptors, possessing an extraordinary capacity to transmit signals across the membrane in both directions. They either bind extracellular ligands or interact with the cytoskeleton through intracellular integrin domains. Thus, integrins enable human cells to respond to changes in the extracellular environment (via outside-in signaling) and can influence the extracellular environment itself (via inside-out signaling). When a ligand binds the receptor, information from outside the cell is transmitted inside, leading to changes in cell polarity, cytoskeletal structure, gene expression, cell survival, and proliferation. Conversely, intracellular activators such as talin-1 binding to the cytoplasmic tail of the β subunit cause a conformational change, placing the integrin in a high-affinity state more prone to binding extracellular ligands, thereby promoting cell migration and extracellular matrix (ECM) assembly and remodeling. Currently, drugs targeting integrins have been extensively developed in areas including cardiovascular diseases, inflammatory bowel disease/multiple sclerosis, and dry eye disease.

siRNA drugs have demonstrated significant therapeutic advantages, with several siRNA drugs already approved for marketing. However, due to delivery vehicles, their application is currently limited to liver targeting. Expanding the application of siRNA drugs to non-liver diseases relies on the development of suitable delivery vehicles. Based on the properties of integrins as transmembrane proteins, research on using integrin-targeting RGD peptides to deliver small nucleic acid drugs has been reported early on, but no related drugs have yet been approved. Designing small molecule targeting moieties that specifically target integrin receptors and conjugating small nucleic acid drug molecules via covalent coupling to achieve non-liver delivery for treating non-liver diseases holds significant clinical importance.

## Summary

The present invention designs and synthesizes a series of small molecule ligand targeting moieties targeting integrins, which can be conjugated to small nucleic acid drug molecules via covalent coupling to achieve *in vivo* delivery of the small nucleic acid drug molecules, thereby expanding the therapeutic areas for small nucleic acid drugs.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ia:

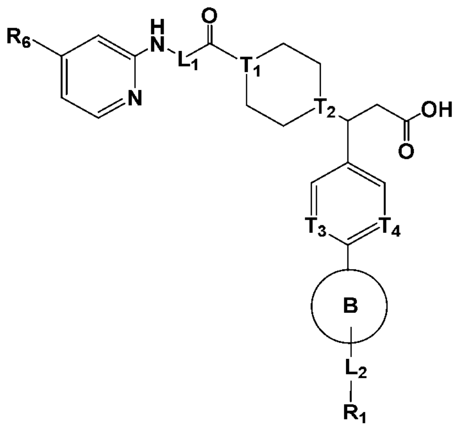

(Ia)

wherein,

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
ring B is

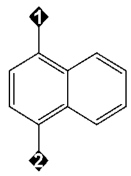 , 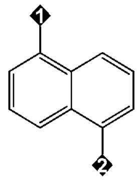 , 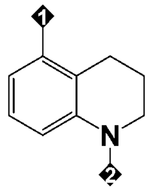 , 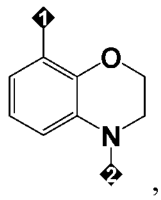 , 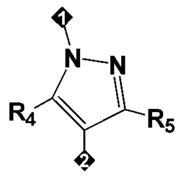 , 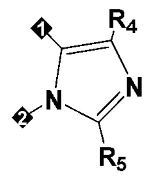 ,

or absent, wherein attachment point 2 is connected to $L_2$;
$R_4$, $R_5$ and $R_6$ are each independently H, methyl, ethyl, n-propyl, isopropyl, cyclopropyl, or cyclobutyl; preferably $R_4$, $R_5$ and $R_6$ are each independently methyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;
$R_1$ is $-N_3$, $-NH_2$, -COOH, $-NHC(O)CH_2SC(O)CH_3$, or

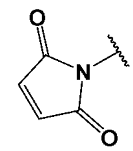 ;

preferably, the compound is selected from Compounds 4, 21, 22, 23, 24, 25, 26, 27, 28, 29, 30, 31, 32, 33, and 34.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib:

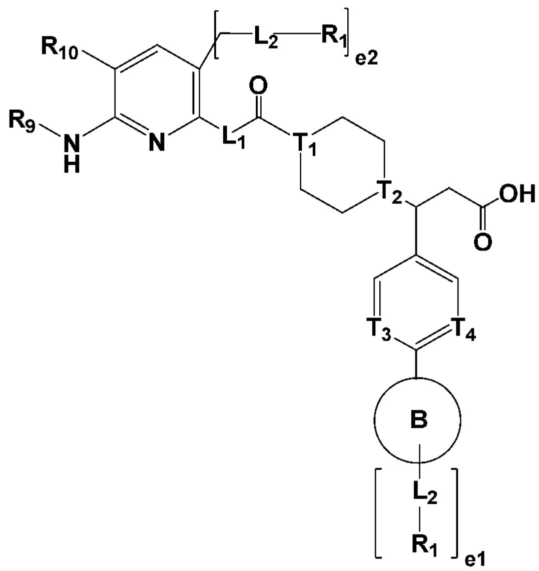

(Ib)

wherein,

$T_1$ and $T_2$ are each independently C or N, and at least one is N; preferably $T_1$ and $T_2$ are both N;

$T_3$ and $T_4$ are each independently C or N; preferably one of $T_3$ and $T_4$ is N; more preferably $T_3$ and $T_4$ are both C; one of e1 and e2 is 1, and the other is 0;

ring B is

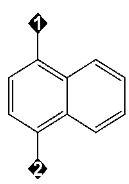 , 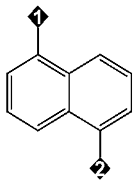 , 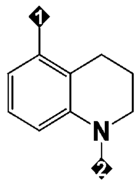 , 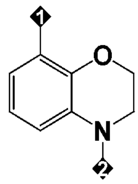 , 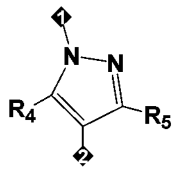 , 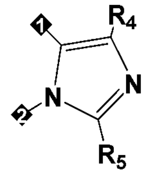 ,

or absent, wherein attachment point 2 is connected to $L_2$, and attachment point 2 is absent when e1 is 0;

$R_4$ and $R_5$ are each independently H, methyl, ethyl, n-propyl, isopropyl, cyclopropyl, or cyclobutyl; preferably $R_4$, $R_5$ and $R_6$ are each independently methyl;

$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;

when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;

when e2 is 1, $L_2$ is $-NHC(O)(CH_2)_q(OCH_2CH_2)_p-$, wherein the right side is connected to $R_1$, p and q are each independently an integer from 1 to 10; preferably q is 2 or 3; preferably p is 3, 4, 5, 6, or 7;

$R_1$ is $-N_3$, $-NH_2$, -COOH, $-NHC(O)CH_2SC(O)CH_3$, or

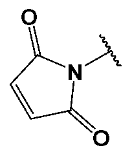 ;

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group;

preferably, the compound is Compound 35.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa or IIb:

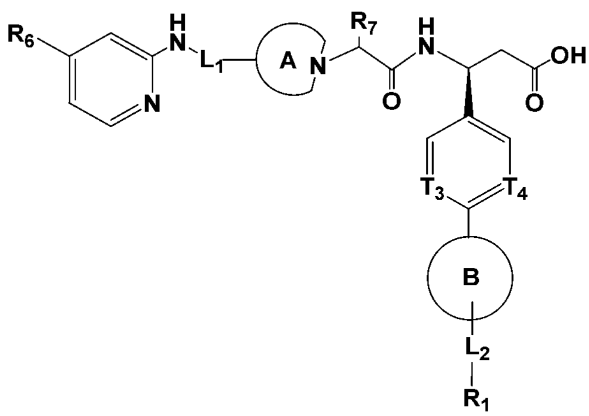

(IIa)

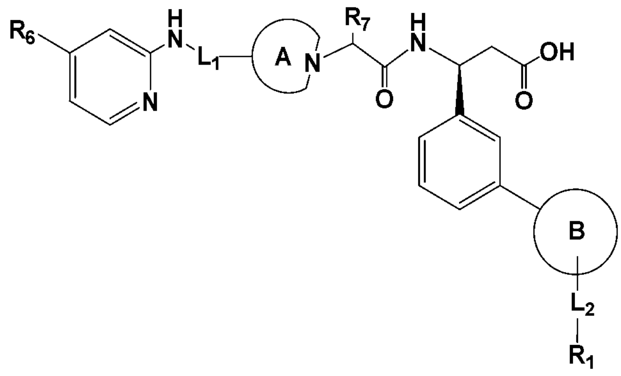

(IIb)

wherein,

$T_3$ and $T_4$ are each independently C or N; preferably one of $T_3$ and $T_4$ is N; more preferably $T_3$ and $T_4$ are both C; ring A is

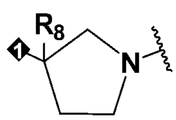
,
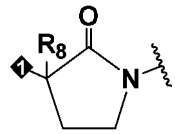
,
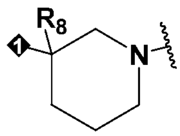
, or
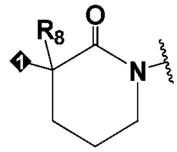
,

wherein attachment point 1 is connected to $L_1$; $R_8$ is H, methyl, ethyl, n-propyl, isopropyl, cyclopropyl, cyclobutyl, or halogen, preferably H or halogen, more preferably H or F;
ring B is

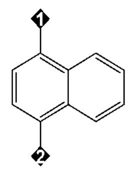
,
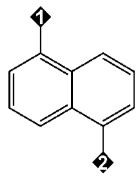
,
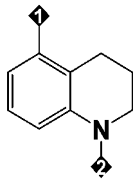
,
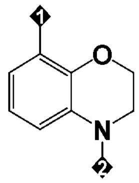
,
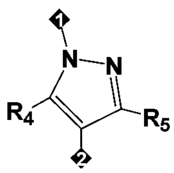
,
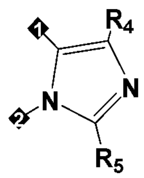
,

or absent, wherein attachment point 2 is connected to $L_2$;
$R_4$, $R_5$, $R_6$, and $R_7$ are each independently H, methyl, ethyl, n-propyl, isopropyl, cyclopropyl, or cyclobutyl; preferably $R_4$, $R_5$ and $R_6$ are each independently methyl; preferably $R_7$ is H;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;
$R_1$ is $-N_3$, $-NH_2$, -COOH, $-NHC(O)CH_2SC(O)CH_3$, or

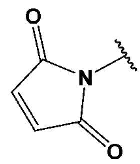
;

preferably, the compound is any one of Compounds 5, 36, 37, 38, 39, 40, 41, 42, 43, or 44.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by

Formula IIIa or IIIb:

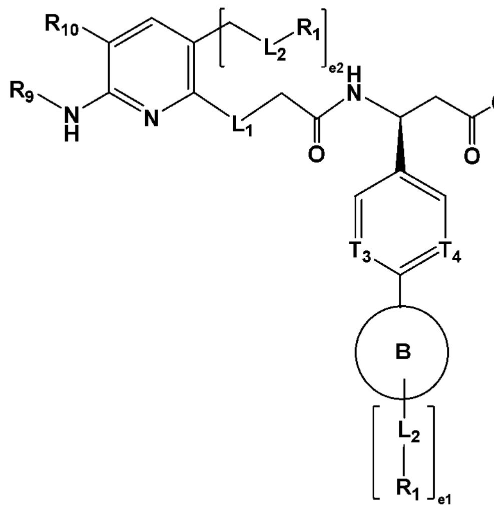

(IIIa)

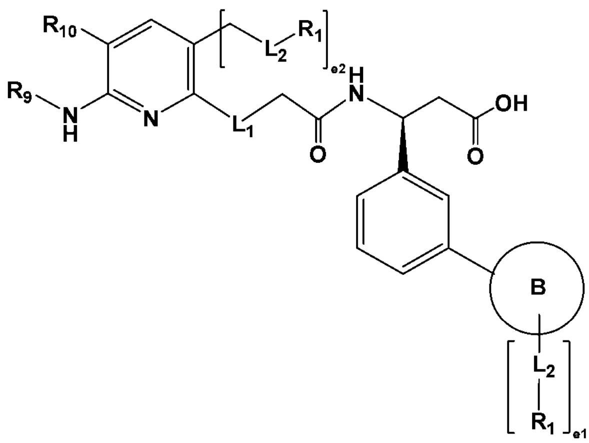

(IIIb)

wherein,

$T_3$ and $T_4$ are each independently C or N; preferably one of $T_3$ and $T_4$ is N; more preferably $T_3$ and $T_4$ are both C;
one of e1 and e2 is 1, and the other is 0;
ring B is

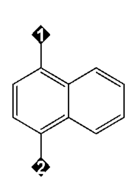 , 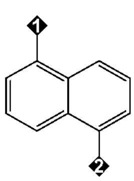 , 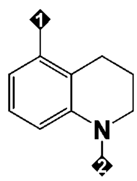 , 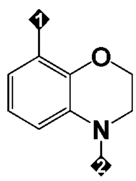 , 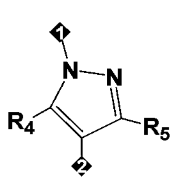 , 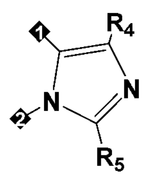 ,

or absent, wherein attachment point 2 is connected to $L_2$, and attachment point 2 is absent when e1 is 0;
$R_4$ and $R_5$ are each independently H, methyl, ethyl, n-propyl, isopropyl, cyclopropyl, or cyclobutyl, preferably methyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-NHC(O)(CH_2)_q(OCH_2CH_2)_p-$, wherein the right side is connected to $R_1$, p and q are each independently an integer from 1 to 10; preferably q is 2 or 3; preferably p is 3, 4, 5, 6, or 7;

$R_1$ is $-N_3$, $-NH_2$, -COOH, $-NHC(O)CH_2SC(O)CH_3$, or

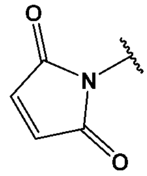

;

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group;
preferably, the compound is Compound 6, 7, 8, 9, 45, 46, or 47.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa:

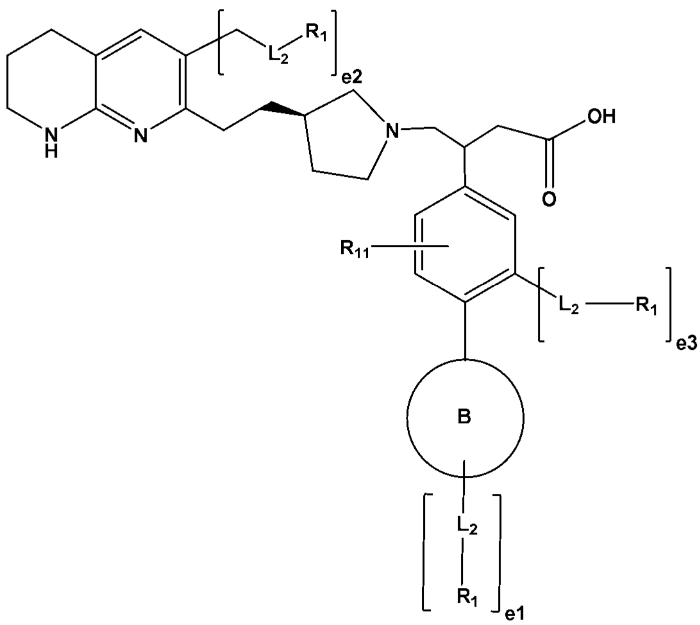

(IVa)

wherein,

one of e1, e2, and e3 is 1, and the other two are 0;
ring B is

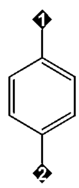 , 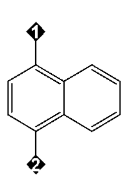 , 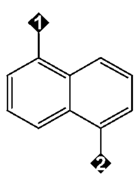 , 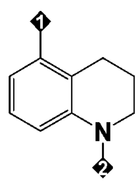 , 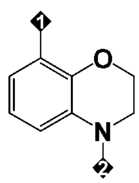 , 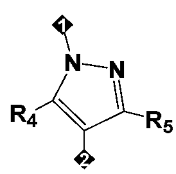 , 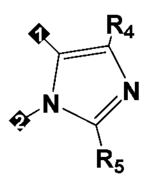 ,

or absent, wherein attachment point 2 is connected to $L_2$, and attachment point 2 is absent when e1 is 0;
$R_4$ and $R_5$ are each independently H, methyl, ethyl, n-propyl, isopropyl, cyclopropyl, or cyclobutyl; preferably $R_4$ and $R_5$ are both methyl;
when e1 or e3 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein

the right side is connected to $R_1$, and p and q are each independently an integer from 1 to 10; preferably q is 2 or 3; preferably p is 3, 4, 5, 6, or 7;

when e2 is 1, $L_2$ is $-NHC(O)(CH_2)_q(OCH_2CH_2)_p-$, wherein the right side is connected to $R_1$, p and q are each independently an integer from 1 to 10; preferably q is 2 or 3; preferably p is 3, 4, 5, 6, or 7;

$R_1$ is $-N_3$, $-NH_2$, -COOH, $-NHC(O)CH_2SC(O)CH_3$, or

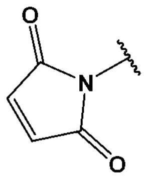 ;

$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy;

preferably, the compound is Compound 13, 14, 15, 18, 20, 48, 49, 53, 54, 55, 56, 57, 58, 59, 60, 61, 62, 63, 71, 72, 73, 83, 84, 85, 86, or 87.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVb:

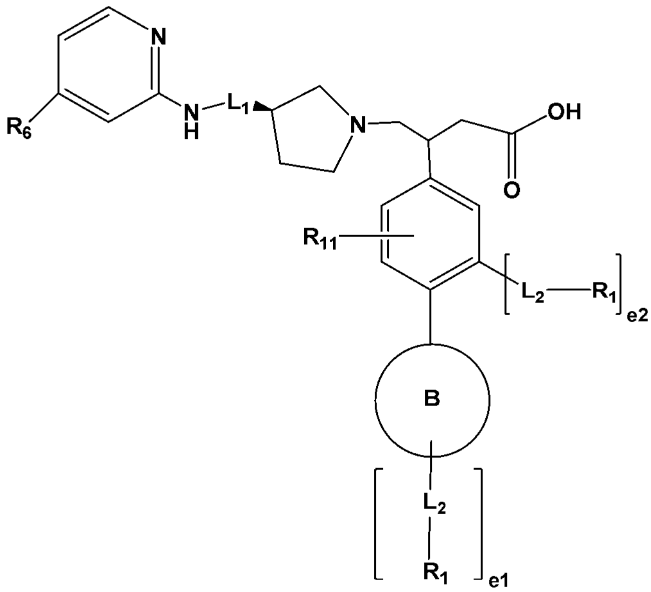

(IVb)

one of e1 and e2 is 1, and the other is 0;

ring B is

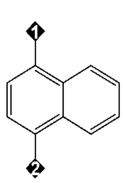 , 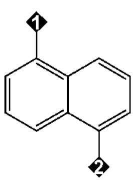 , 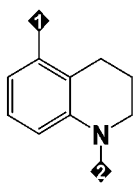 , 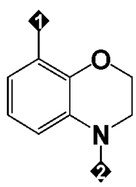 , 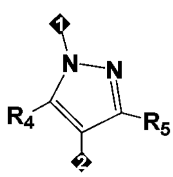 , 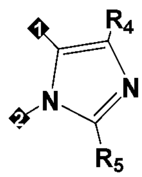 ,

or absent, wherein attachment point 2 is connected to $L_2$;

$R_4$, $R_5$ and $R_6$ are each independently H, methyl, ethyl, n-propyl, isopropyl, cyclopropyl, or cyclobutyl; preferably $R_4$, $R_5$ and $R_6$ are all methyl;

$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, -C(O)

$NH(CH_2CH_2O)_p$-, $-C(O)NH(CH_2)_q(OCH_2CH_2)_p-$, $-C(O)(CH_2CH_2O)_p-$, or - $C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;

$R_1$ is $-N_3$, $-NH_2$, -COOH, $-NHC(O)CH_2SC(O)CH_3$, or

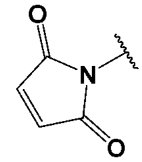 ;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

$R_{11}$ is H, halogen, halomethyl, methyl, ethyl, n-propyl, isopropyl, cyclopropyl, cyclobutyl, phenyl, morpholinyl, methoxyethoxy, or ethoxymethoxy; preferably $R_{11}$ is H;

preferably, the compound is Compound 50, 51, 52, 74, 75, 76, 77, 78, 79, or 80.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va:

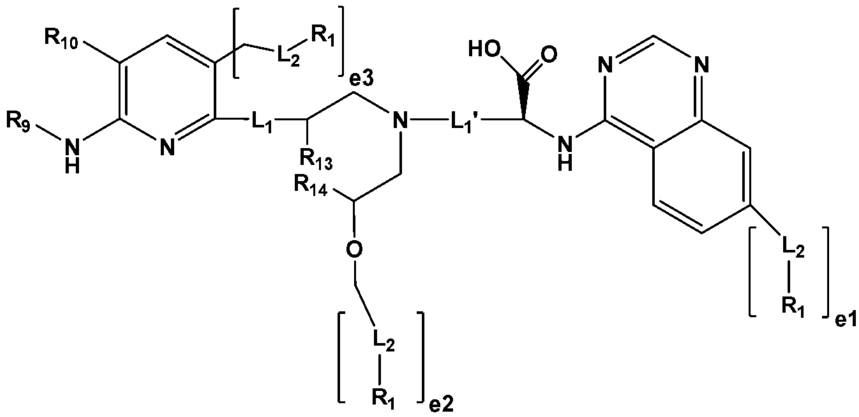

(Va)

one of e1, e2, and e3 is 1, and the other two are 0;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;

$R_{13}$ and $R_{14}$ are each H, or $R_{13}$, $R_{14}$, together with the C and N atoms to which they are attached, form a piperidinyl group;

$L_1$ and $L_1$' are each independently $-(CH_2)_m-$, $-(CH_2)_mC(O)NH(CH_2)_n-$, or - $(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

when e1 is 1, $L_2$ is $-(OCH_2CH_2)_p-$, $-(CH_2)_q(OCH_2CH_2)_p-$, $-NHC(O)(CH_2CH_2O)_p-$, - $NHC(O)(CH_2)_q(OCH_2CH_2)_p-$, $-C(O)NH(CH_2CH_2O)_p-$, $-C(O)NH(CH_2)_q(OCH_2CH_2)_p-$, - $C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;

when e2 is 1, $L_2$ is $-(CH_2)q(OCH_2CH_2)_p-$, wherein the right side is connected to $R_1$, p and q are each independently an integer from 1 to 10; preferably q is 1 or 2; preferably p is 3, 4, 5, 6, or 7;

when e3 is 1, $L_2$ is $-NHC(O)(CH_2)_q(OCH_2CH_2)_p-$, wherein the right side is connected to $R_1$, p and q are each independently an integer from 1 to 10; preferably q is 2 or 3; preferably p is 3, 4, 5, 6, or 7;

$R_1$ is $-N_3$, $-NH_2$, -COOH, $-NHC(O)CH_2SC(O)CH_3$, or

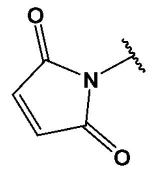

;

preferably, the compound is Compound 16, 17, 64, 65, 66, or 67.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb:

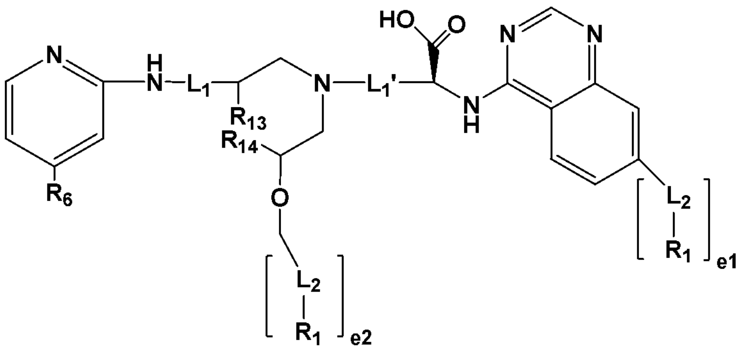

(Vb)

wherein

one of e1 and e2 is 1, and the other is 0;
$R_6$ is H, methyl, ethyl, n-propyl, isopropyl, cyclopropyl, or cyclobutyl, preferably methyl;
$R_{13}$ and $R_{14}$ are each H, or $R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-(CH_2)q(OCH_2CH_2)_p-$, wherein the right side is connected to $R_1$, p and q are each independently an integer from 1 to 10; preferably q is 1 or 2; preferably p is 3, 4, 5, 6, or 7;
$R_1$ is $-N_3$, $-NH_2$, $-COOH$, $-NHC(O)CH_2SC(O)CH_3$, or

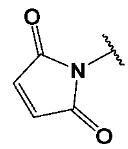

;

preferably, the compound is Compound 69 or 70.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc:

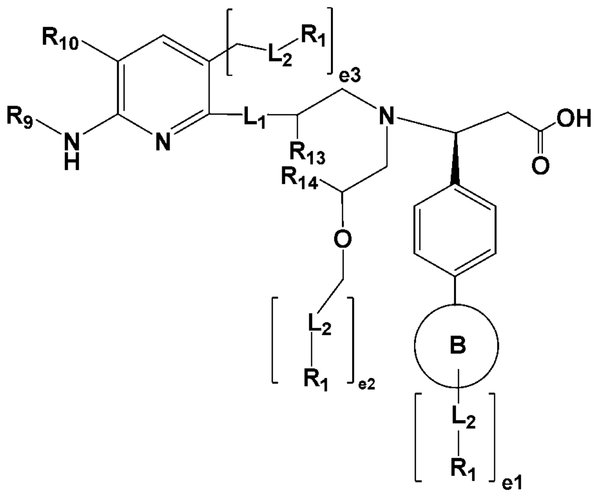

(Vc)

one of e1, e2, and e3 is 1, and the other two are 0;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;

$R_{13}$ and $R_{14}$ are each H, or $R_{13}$, $R_{14}$, together with the C and N atoms to which they are attached, form a piperidinyl group;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;

when e2 is 1, $L_2$ is $-(CH_2)q(OCH_2CH_2)_p-$, wherein the right side is connected to $R_1$, p and q are each independently an integer from 1 to 10; preferably q is 1 or 2; preferably p is 3, 4, 5, 6, or 7;

when e3 is 1, $L_2$ is $-NHC(O)(CH_2)_q(OCH_2CH_2)_p-$, wherein the right side is connected to $R_1$, p and q are each independently an integer from 1 to 10; preferably q is 2 or 3; preferably p is 3, 4, 5, 6, or 7;

$R_1$ is $-N_3$, $-NH_2$, $-COOH$, $-NHC(O)CH_2SC(O)CH_3$, or

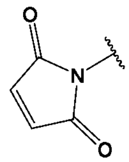

;

ring B is

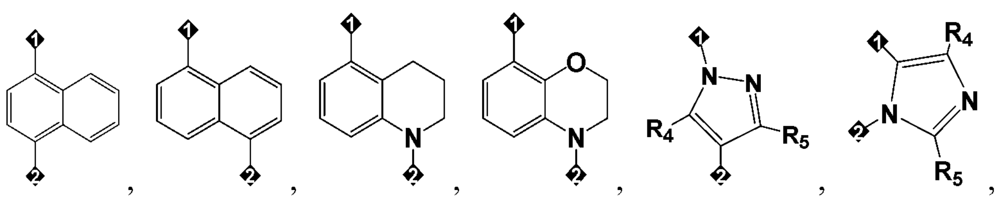

or absent, wherein attachment point 2 is connected to $L_2$, and attachment point 2 is absent when e1 is 0;

$R_4$ and $R_5$ are each independently H, methyl, ethyl, n-propyl, isopropyl, cyclopropyl, or cyclobutyl; preferably $R_4$ and $R_5$ are both methyl;

preferably, the compound is Compound 68.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by

Formula Ia':

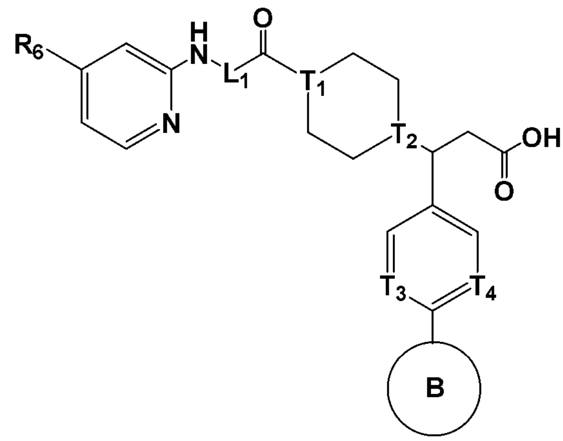

(Ia')

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
ring B is

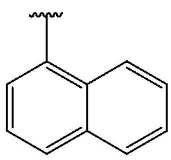 , 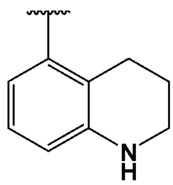 , 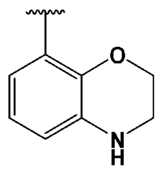 , 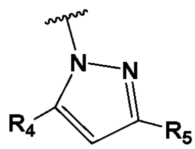 , 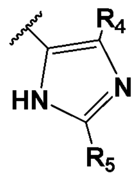 ,

or absent;
$R_4$, $R_5$ and $R_6$ are each independently H, methyl, ethyl, n-propyl, isopropyl, cyclopropyl, or cyclobutyl; preferably $R_4$, $R_5$ and $R_6$ are each independently methyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
preferably, the compound is selected from Compounds 4', 21', 22', 23', 24', 31', 32', 33', and 34'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib':

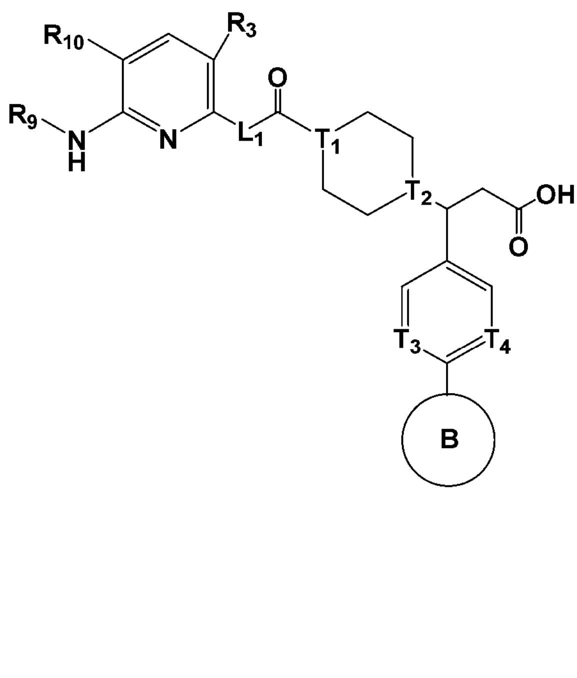

(Ib')

wherein,

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
ring B is

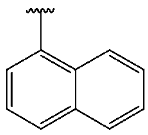, 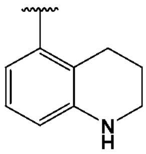, 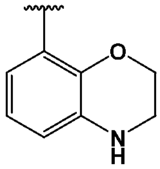, 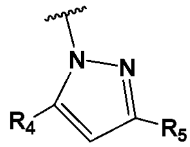, 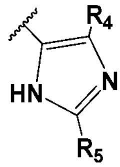,

or absent;
$R_4$ and $R_5$ are each independently H, methyl, ethyl, n-propyl, isopropyl, cyclopropyl, or cyclobutyl; preferably $R_4$, $R_5$ and $R_6$ are each independently methyl;
$R_3$ is H, methyl, ethyl, n-propyl, isopropyl, cyclopropyl, cyclobutyl, amino, or $C_{1-3}$ alkylamino; preferably $R_3$ is H or methylamino;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group;
preferably, the compound is Compound 35'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa' or IIb':

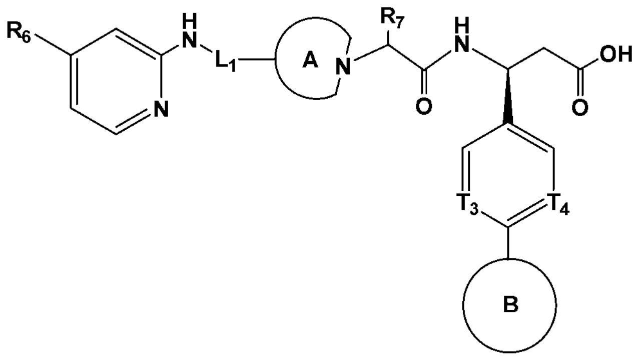
(IIa')

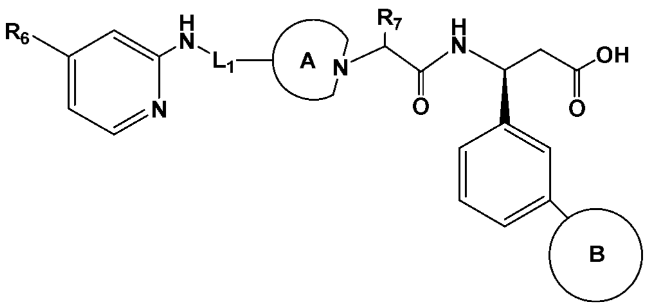
(IIb')

wherein,

$T_3$ and $T_4$ are each independently C or N; preferably one of $T_3$ and $T_4$ is N; more preferably $T_3$ and $T_4$ are both C;
ring A is

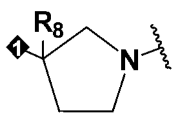 , 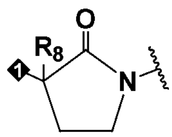 , 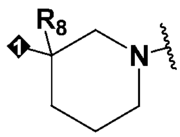 , or 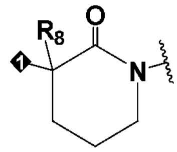 ,

wherein attachment point 1 is connected to $L_1$; $R_8$ is H, methyl, ethyl, n-propyl, isopropyl, cyclopropyl, cyclobutyl, or halogen, preferably H or halogen, more preferably H or F;

ring B is

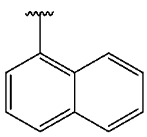 , 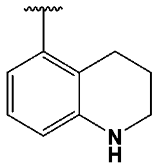 , 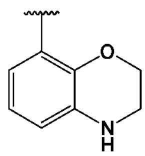 , 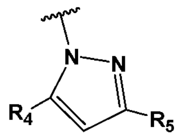 , 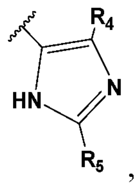 ,

or absent;

$R_4$, $R_5$, $R_6$, and $R_7$ are each independently H, methyl, ethyl, n-propyl, isopropyl, cyclopropyl, or cyclobutyl; preferably $R_4$, $R_5$ and $R_6$ are each independently methyl; preferably $R_7$ is H;

$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;

preferably, the compound is any one of Compounds 5', 36', 37', 38', 39', 40', 41', 42', 43', or 44'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa' or IIIb':

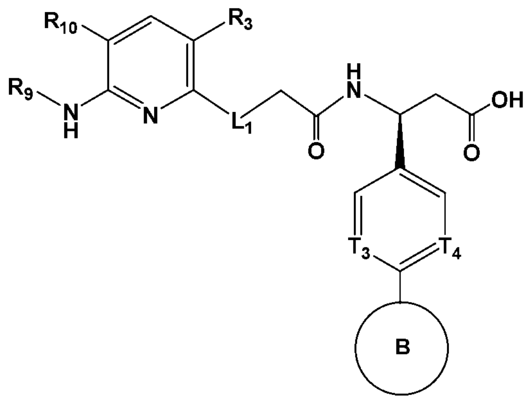

(IIIa')

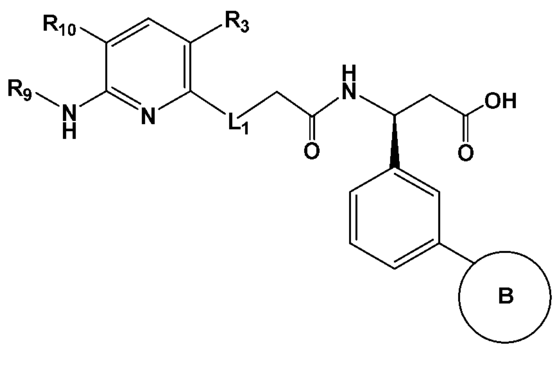

(IIIb')

wherein,

$T_3$ and $T_4$ are each independently C or N; preferably one of $T_3$ and $T_4$ is N; more preferably $T_3$ and $T_4$ are both C; ring B is

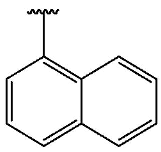, 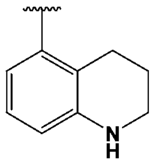, 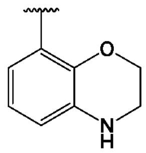, 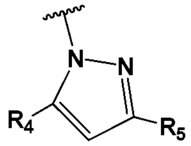, or 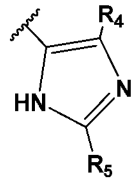 ;

$R_4$ and $R_5$ are each independently H, methyl, ethyl, n-propyl, isopropyl, cyclopropyl, or cyclobutyl, preferably methyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
$R_3$ is H, methyl, ethyl, n-propyl, isopropyl, cyclopropyl, cyclobutyl, amino, or $C_{1-3}$ alkylamino; preferably $R_3$ is H or methylamino;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group;
preferably, the compound is Compound 6', 7', 8', 9', or 47'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa':

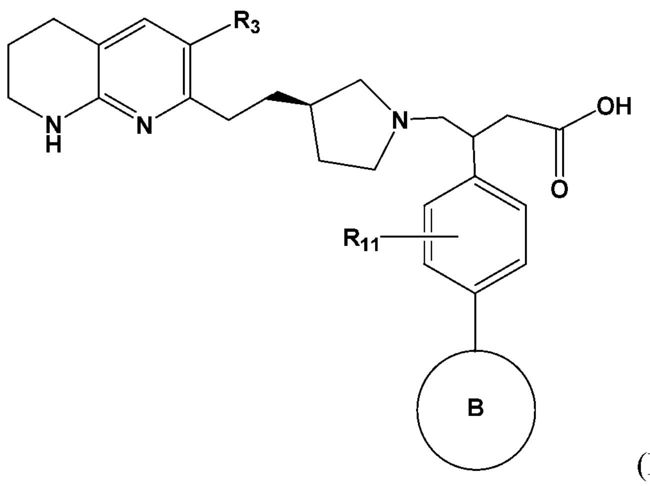

(IVa')

wherein,

ring B is

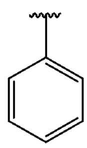, 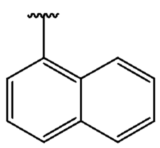, 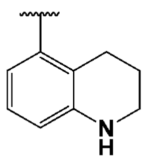, or 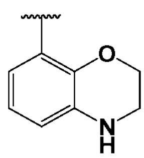 ;

$R_4$ and $R_5$ are each independently H, methyl, ethyl, n-propyl, isopropyl, cyclopropyl, or cyclobutyl; preferably $R_4$ and $R_5$ are both methyl;
$R_3$ is H, methyl, ethyl, n-propyl, isopropyl, cyclopropyl, cyclobutyl, amino, or $C_{1-3}$ alkylamino; preferably $R_3$ is H or methylamino;
$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy;

preferably, the compound is Compound 48', 49', 59', 60', 73', or 85'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVb':

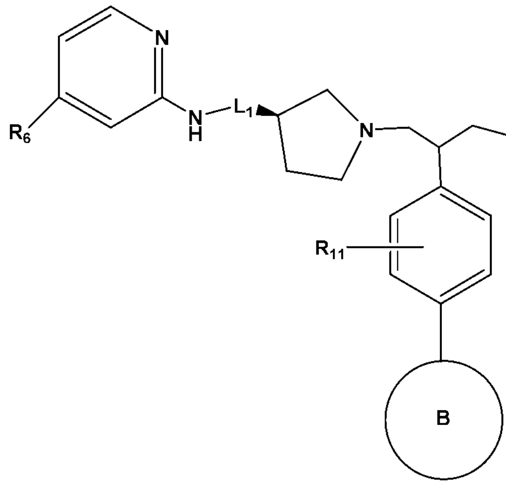

(IVb')

wherein

ring B is

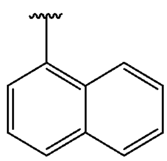, 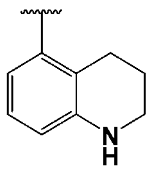, 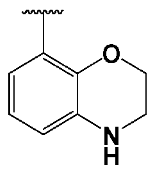, 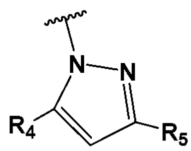, 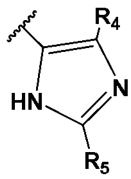,

or absent;

$R_4$, $R_5$ and $R_6$ are each independently H, methyl, ethyl, n-propyl, isopropyl, cyclopropyl, or cyclobutyl; preferably $R_4$, $R_5$ and $R_6$ are all methyl;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

$R_{11}$ is H, halogen, halomethyl, methyl, ethyl, n-propyl, isopropyl, cyclopropyl, cyclobutyl, phenyl, morpholinyl, methoxyethoxy, or ethoxymethoxy; preferably $R_{11}$ is H;

preferably, the compound is Compound 50', 51', 52', 76', or 79'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va':

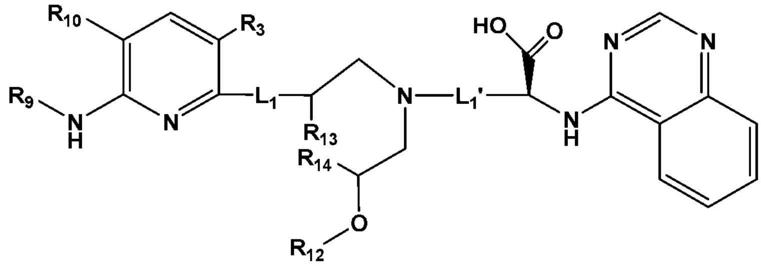

(Va')

wherein

$R_3$ is H, methyl, ethyl, n-propyl, isopropyl, cyclopropyl, cyclobutyl, amino, or $C_{1-3}$ alkylamino; preferably $R_3$ is H or methylamino;
$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;
$R_{12}$ is methyl, ethyl, n-propyl, isopropyl, cyclopropyl, or cyclobutyl; preferably $R_{12}$ is methyl;
$R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
preferably, the compound is Compound 64'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb':

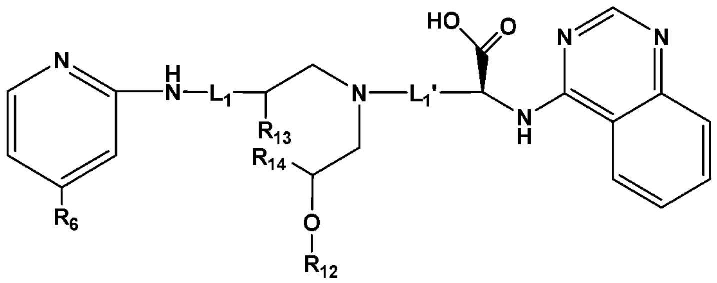

(Vb')

wherein,

$R_6$ is H, methyl, ethyl, n-propyl, isopropyl, cyclopropyl, or cyclobutyl, preferably methyl;
$R_{12}$ is methyl, ethyl, n-propyl, isopropyl, cyclopropyl, or cyclobutyl; preferably $R_{12}$ is methyl;
$R_{13}$ and $R_{14}$ are each H, or $R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
preferably, the compound is Compound 69' or 70'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc':

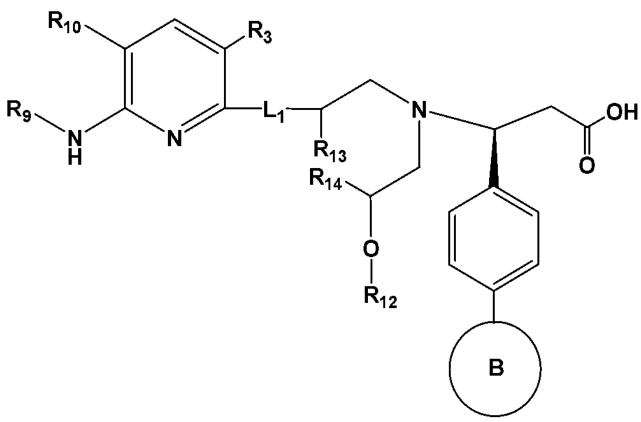

(Vc')

$R_3$ is H, methyl, ethyl, n-propyl, isopropyl, cyclopropyl, cyclobutyl, amino, or $C_{1-3}$ alkylamino; preferably $R_3$ is H or

methylamino;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;

$R_{12}$ is methyl, ethyl, n-propyl, isopropyl, cyclopropyl, or cyclobutyl; preferably $R_{12}$ is methyl;

$R_{13}$ and $R_{14}$ are each H, or $R_{13}$, $R_{14}$, together with the C and N atoms to which they are attached, form a piperidinyl group;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

ring B is

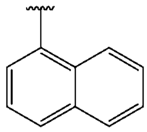 , 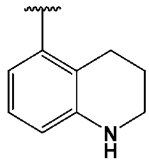 , 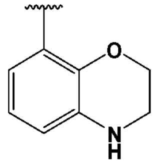 , 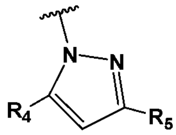 , 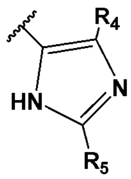 ,

or absent;

$R_4$ and $R_5$ are each independently H, methyl, ethyl, n-propyl, isopropyl, cyclopropyl, or cyclobutyl; preferably $R_4$ and $R_5$ are both methyl;

preferably, the compound is Compound 68'.

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ia":

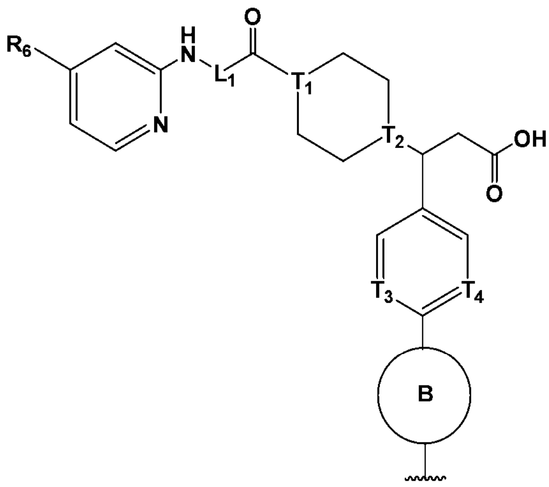

(Ia")

$T_1$ and $T_2$ are each independently C or N, and at least one is N; preferably $T_1$ and $T_2$ are both N;

$T_3$ and $T_4$ are each independently C or N; preferably one of $T_3$ and $T_4$ is N; more preferably $T_3$ and $T_4$ are both C;

ring B is

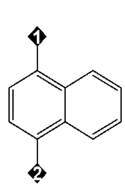 , 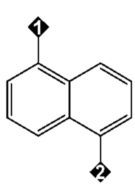 , 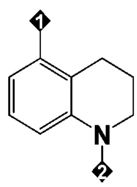 , 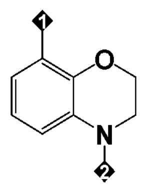 , 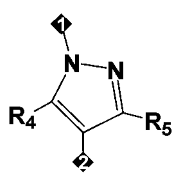 , 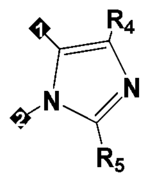 ,

or absent, wherein attachment point 2 is the wave line in Formula Ia", and the wave line - indicates a bond;

$R_4$, $R_5$ and $R_6$ are each independently H, methyl, ethyl, n-propyl, isopropyl, cyclopropyl, or cyclobutyl; preferably $R_4$,

$R_5$ and $R_6$ are each independently methyl;

$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;

preferably, the compound is selected from: 4", 21", 22", 23", 24", 31", 32", 33", and 34".

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib":

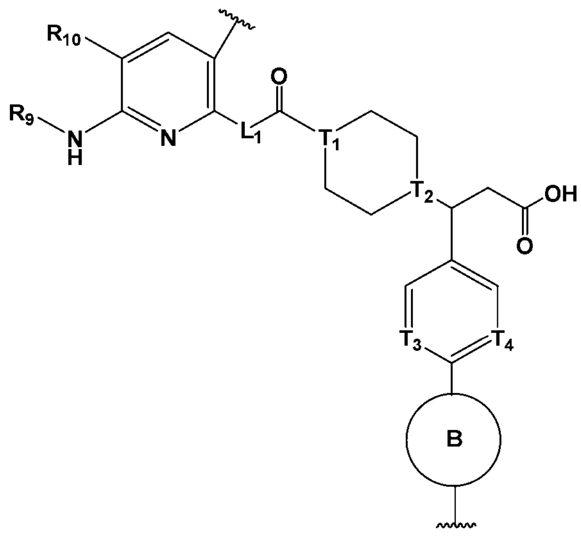

(Ib")

wherein,

$T_1$ and $T_2$ are each independently C or N, and at least one is N; preferably $T_1$ and $T_2$ are both N;

$T_3$ and $T_4$ are each independently C or N; preferably one of $T_3$ and $T_4$ is N; more preferably $T_3$ and $T_4$ are both C;

ring B is

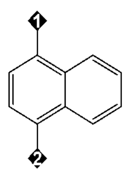 , 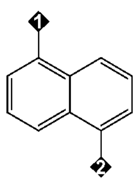 , 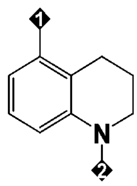 , 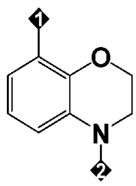 , 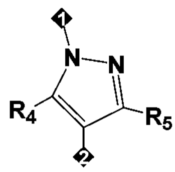 , 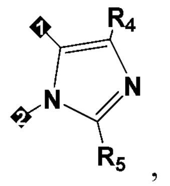 ,

or absent, wherein attachment point 2 is the wave line connected to ring B in Formula Ib";

$R_4$ and $R_5$ are each independently H, methyl, ethyl, n-propyl, isopropyl, cyclopropyl, or cyclobutyl; preferably $R_4$ and $R_5$ are each independently methyl;

$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group; and

the wave line in Formula Ib" indicates a bond, and one of the two wave lines is absent;

preferably, wherein the compound has a structure represented by Formula Ib"-1:

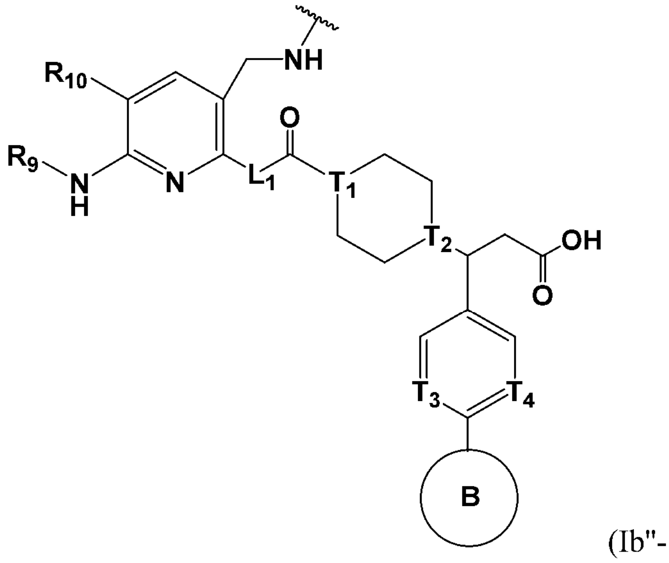

(Ib"-1)

wherein $T_1$, $T_2$, $T_3$, $T_4$, $L_1$, $R_9$, $R_{10}$, and the wave line have the same definitions as the corresponding substituents in Formula Ib",
ring B is

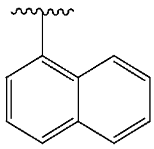 , 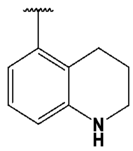 , 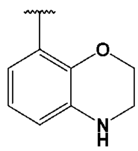 , 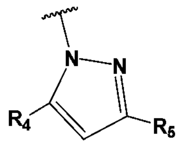 , 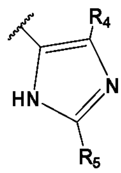 ,

or absent, wherein $R_4$ and $R_5$ are each independently H, methyl, ethyl, n-propyl, isopropyl, cyclopropyl, or cyclobutyl;
preferably $R_4$ and $R_5$ are each independently methyl;
preferably, the compound is Compound 35".

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa" or IIb":

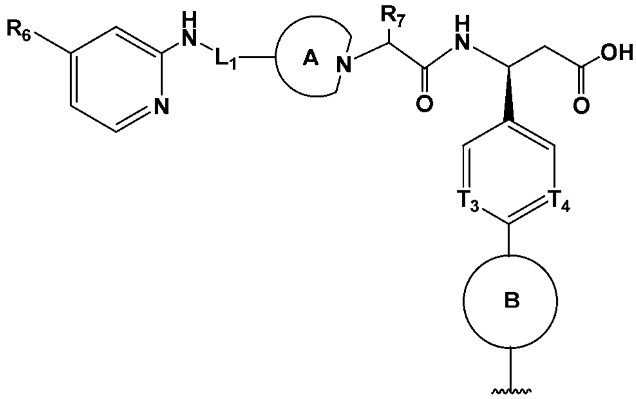

(IIa")

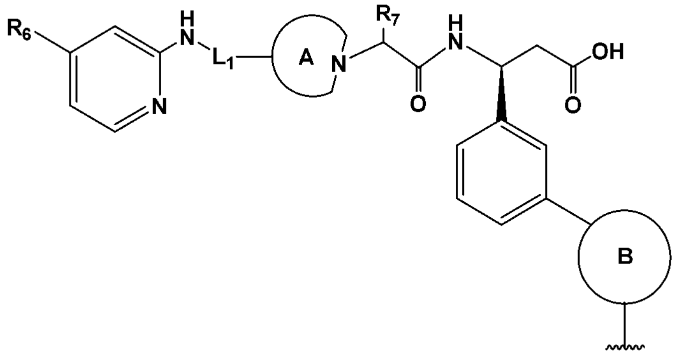

(IIb")

wherein,

$T_3$ and $T_4$ are each independently C or N; preferably one of $T_3$ and $T_4$ is N; more preferably $T_3$ and $T_4$ are both C; ring A is

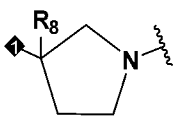
,
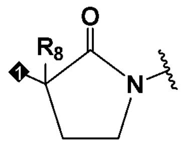
,
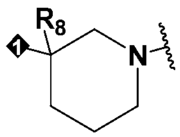
, or
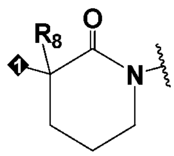
,

wherein attachment point 1 is connected to $L_1$; $R_8$ is H, methyl, ethyl, n-propyl, isopropyl, cyclopropyl, cyclobutyl, or halogen, preferably H or halogen, more preferably H or F;
ring B is

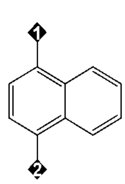
,
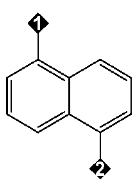
,
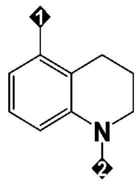
,
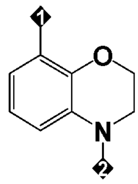
,
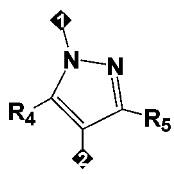
,
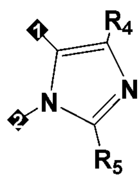
,

or absent, wherein attachment point 2 is the wave line in Formula IIa" or IIb", and the wave line indicates a bond;
$R_4$, $R_5$, $R_6$, and $R_7$ are each independently H, methyl, ethyl, n-propyl, isopropyl, cyclopropyl, or cyclobutyl; preferably $R_4$, $R_5$ and $R_6$ are each independently methyl; preferably $R_7$ is H;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
preferably, the compound is selected from: 5", 36", 37", 38", 39", 40", 41", 42", 43", and 44".

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa" or IIIb":

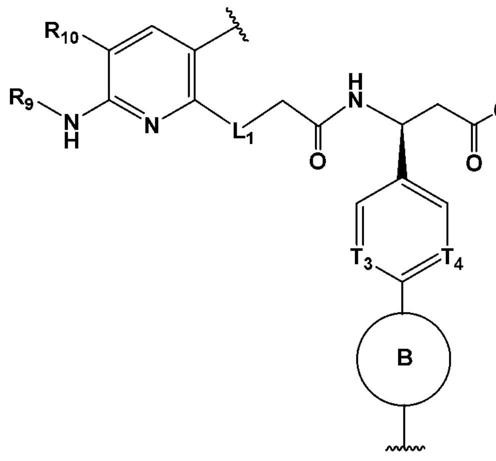

(IIIa")

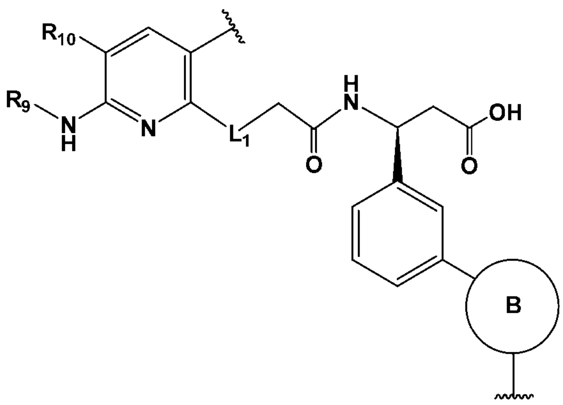

(IIIb")

wherein,

$T_3$ and $T_4$ are each independently C or N; preferably one of $T_3$ and $T_4$ is N; more preferably $T_3$ and $T_4$ are both C; ring B is

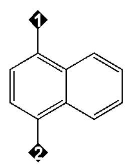 , 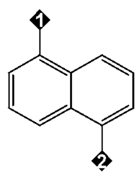 , 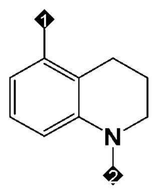 , 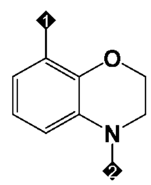 , 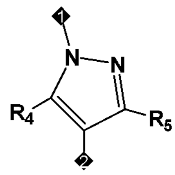 , 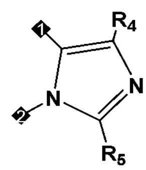 ,

or absent, wherein attachment point 2 is the wave line connected to ring B in Formula IIIa" or IIIb";
$R_4$ and $R_5$ are each independently H, methyl, ethyl, n-propyl, isopropyl, cyclopropyl, or cyclobutyl, preferably methyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group; and
the wave line in Formula IIIa" and Formula IIIb" indicates a bond, and one of the two wave lines is absent;
or, wherein the compound has a structure represented by Formula IIIa"-1 or IIIb"-1:

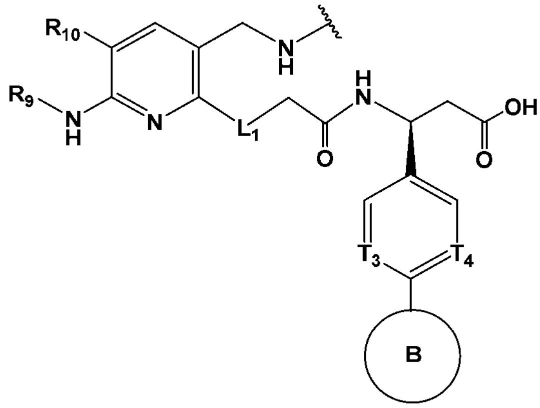

(IIIa"-1)

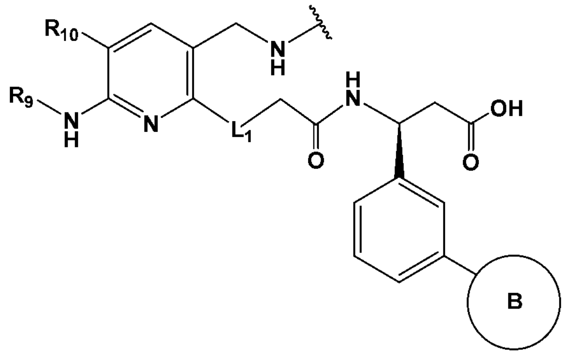

(IIIb"-1)

wherein $T_3$, $T_4$, $L_1$, $R_9$, $R_{10}$, and the wave line have the same definitions as the corresponding substituents in Formula IIIa" or IIIb",
ring B is

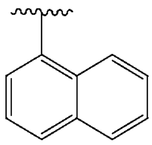, 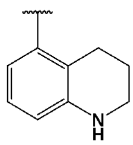, 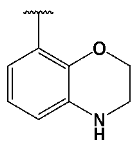, 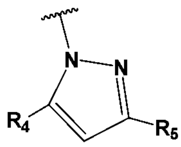, 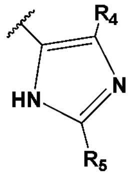,

or absent, wherein $R_4$ and $R_5$ are each independently H, methyl, ethyl, n-propyl, isopropyl, cyclopropyl, or cyclobutyl;
preferably $R_4$ and $R_5$ are each independently methyl;
preferably, the compound is selected from: 6", 7", 8", 9", 45", 46", and 47".

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa":

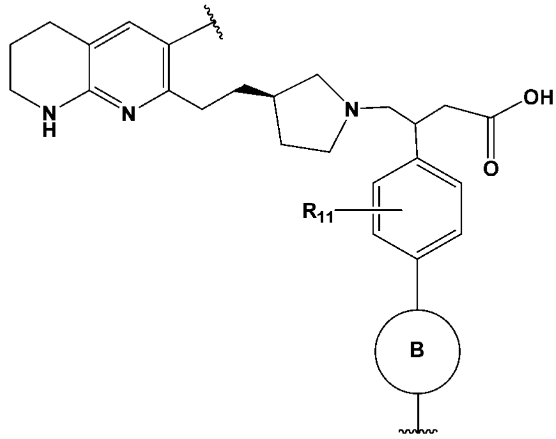

(IVa")

wherein,

ring B is

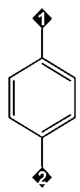 , 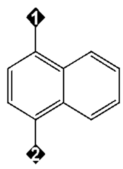 , 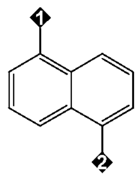 , 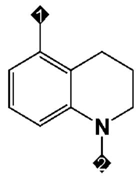 , 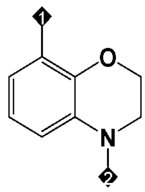 , 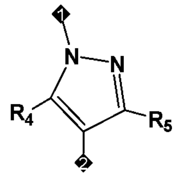 , 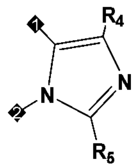 ,

or absent, wherein attachment point 2 is the wave line connected to ring B in Formula IVa";

$R_4$ and $R_5$ are each independently H, methyl, ethyl, n-propyl, isopropyl, cyclopropyl, or cyclobutyl; preferably $R_4$ and $R_5$ are both methyl;

$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy; and

the wave line in Formula IVa" indicates a bond, and one of the two wave lines is absent;

or, wherein the compound has a structure represented by Formula IVa"-1:

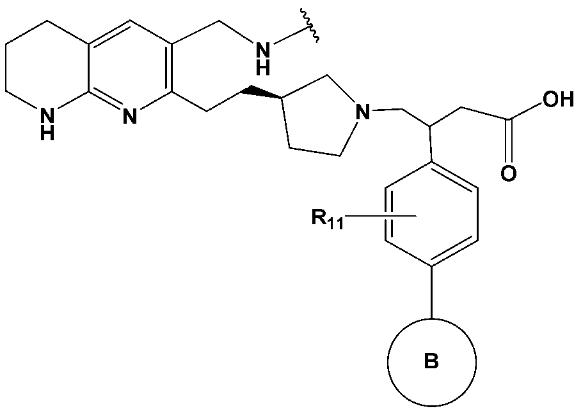

(IVa"-1)

wherein $R_{11}$ and the wave line have the same definitions as $R_{11}$ and the wave line in Formula IVa",

ring B is

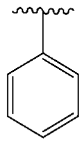, 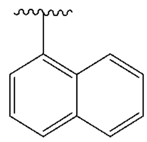, 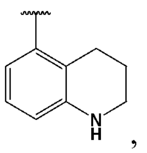, 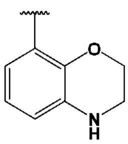, 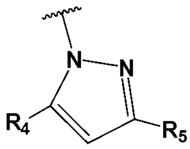, 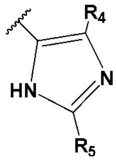,

or absent, wherein $R_4$ and $R_5$ are each independently H, methyl, ethyl, n-propyl, isopropyl, cyclopropyl, or cyclobutyl; preferably $R_4$ and $R_5$ are each independently methyl;
preferably, the compound is selected from: 13", 14", 15", 18", 20", 48", 49", 53", 54", 55", 56", 57", 58", 59", 60", 61", 62", 63", 71", 72", 73", 83", 84", 85", 86", and 87".

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVb":

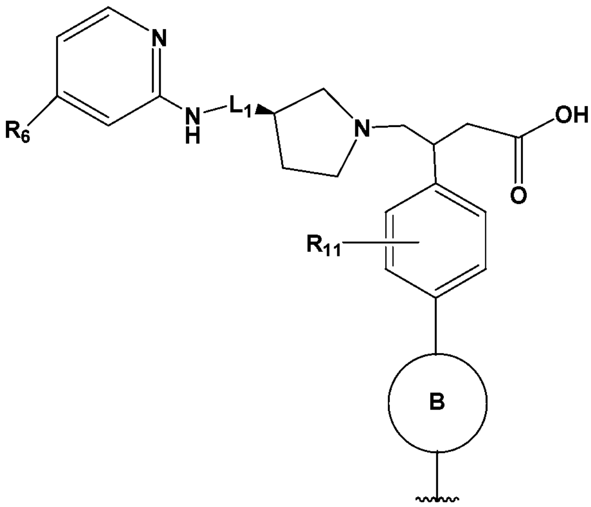

(IVb")

ring B is

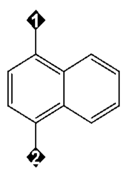, 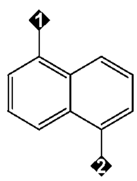, 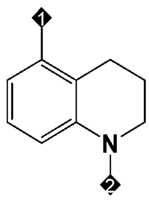, 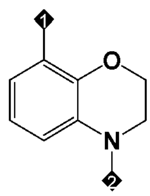, 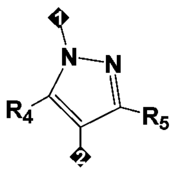, 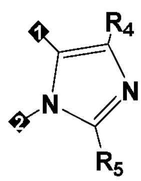,

or absent, wherein attachment point 2 is the wave line in Formula IVb", and the wave line indicates a bond;
$R_4$, $R_5$ and $R_6$ are each independently H, methyl, ethyl, n-propyl, isopropyl, cyclopropyl, or cyclobutyl; preferably $R_4$, $R_5$ and $R_6$ are all methyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
$R_{11}$ is H, halogen, halomethyl, methyl, ethyl, n-propyl, isopropyl, cyclopropyl, cyclobutyl, phenyl, morpholinyl, methoxyethoxy, or ethoxymethoxy; preferably $R_{11}$ is H;
preferably, the compound is selected from: 50", 51", 52", 74", 75", 76", 77", 78", 79", and 80".

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va":

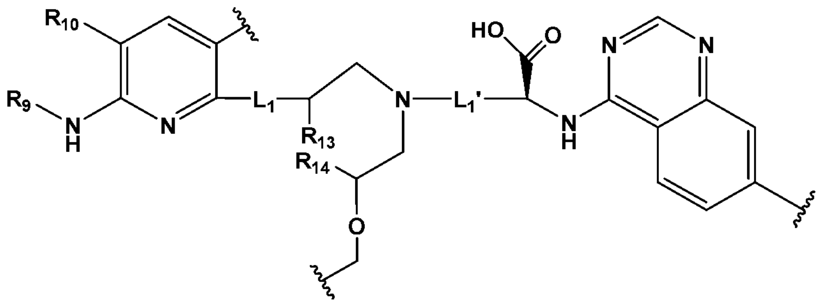

(Va")

wherein

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;

$R_{13}$ and $R_{14}$ are each H, or $R_{13}$, $R_{14}$, together with the C and N atoms to which they are attached, form a piperidinyl group;

$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3; and

the wave line ~~~~ in Formula Va" indicates a bond, and two of the three wave lines are absent;

or, wherein the compound has a structure represented by Formula Va"-1, Va"-2, or Va"-3:

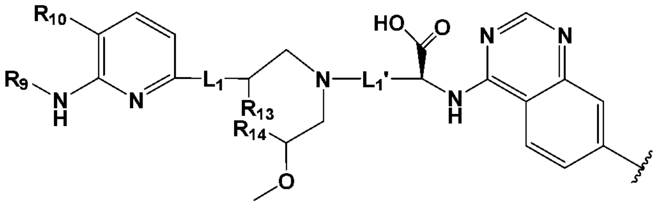

(Va"-1)

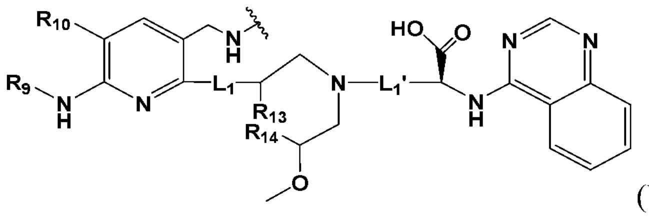

(Va"-2)

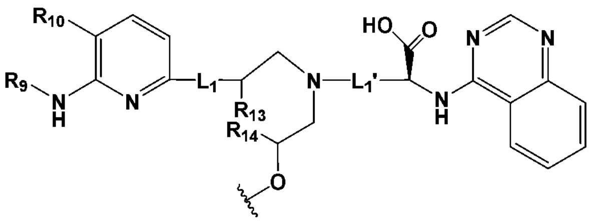

(Va"-3)

wherein, $R_9$, $R_{10}$, $R_{13}$, $R_{14}$, $L_1$, $L_1$', and the wave line have the same definitions as the corresponding substituents in Formula Va";

preferably, the compound is selected from: 16", 17", 64", 65", 66", and 67".

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb":

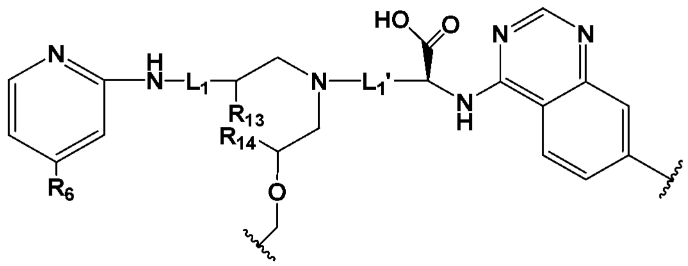

(Vb")

wherein,

$R_6$ is H, methyl, ethyl, n-propyl, isopropyl, cyclopropyl, or cyclobutyl, preferably methyl;
$R_{13}$ and $R_{14}$ are each H, or $R_{13}$, $R_{14}$, together with the C and N atoms to which they are attached, form a piperidinyl group;
$L_1$ and $L_1$' are each independently -$(CH_2)_m$-, -$(CH_2)_m$C(O)NH$(CH_2)_n$-, or - $(CH_2)_m$NHC(O)$(CH_2)_n$-; m is an integer from 1 to 10; preferably m is 1, 2, 3, 4, 5, or 6; n is an integer from 0 to 10; preferably n is 0, 1, 2, or 3; and
the wave line ∿∿∿ in Formula Vb" indicates a bond, and one of the two wave lines is absent;
or, wherein the compound has a structure represented by Formula Vb"-1 or Vb"-2:

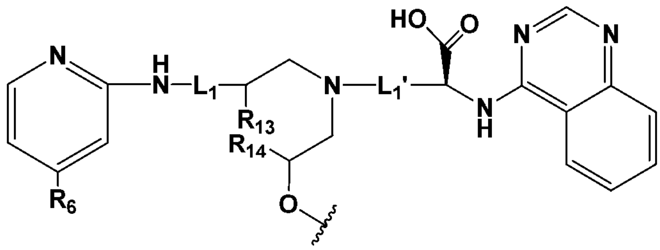

(Vb"-1)

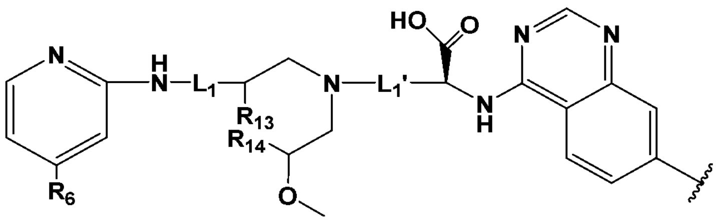

(Vb"-2)

wherein $R_6$, $R_{13}$, $R_{14}$, $L_1$, $L_1$', and the wave line have the same definitions as the corresponding substituents in Formula Vb";
preferably, the compound is selected from: 69" and 70".

In one aspect, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc":

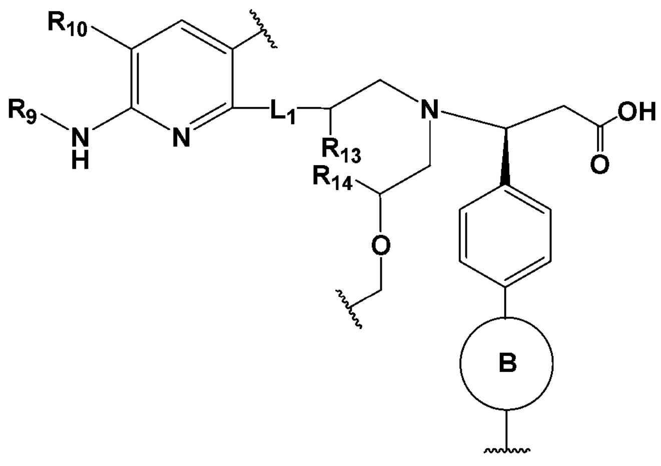

(Vc")

wherein

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;

$R_{13}$ and $R_{14}$ are each H, or $R_{13}$, $R_{14}$, together with the C and N atoms to which they are attached, form a piperidinyl group;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

ring B is

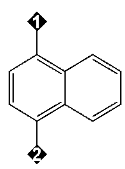 , 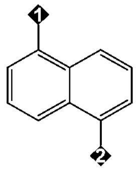 , 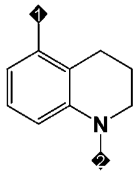 , 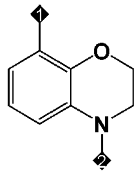 , 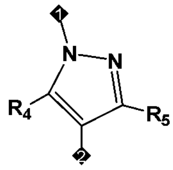 , 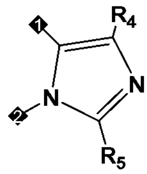 ,

or absent, wherein attachment point 2 is the wave line connected to ring B in Formula Vc";

$R_4$ and $R_5$ are each independently H, methyl, ethyl, n-propyl, isopropyl, cyclopropyl, or cyclobutyl; preferably $R_4$ and $R_5$ are both methyl; and

the wave line in Formula Vc" indicates a bond, and two of the three wave lines are absent;

or, wherein the compound has a structure represented by Formula Vc"-1:

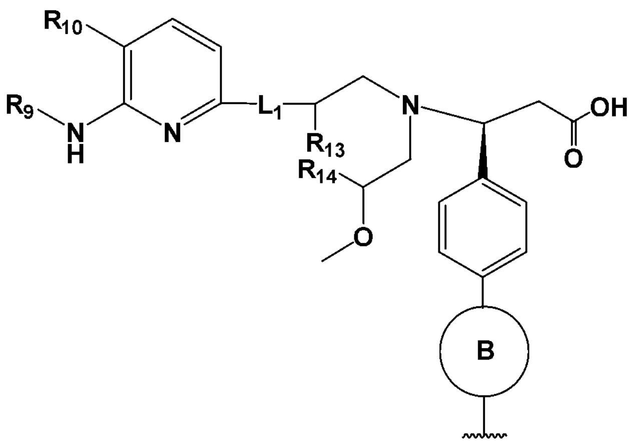

(Vc"-1)

wherein $R_9$, $R_{10}$, $R_{13}$, $R_{14}$, $L_1$, ring B, and the wave line have the same definitions as the corresponding substituents in Formula Vc";

or, wherein the compound has a structure represented by Formula Vc"-2 or Vc"-3:

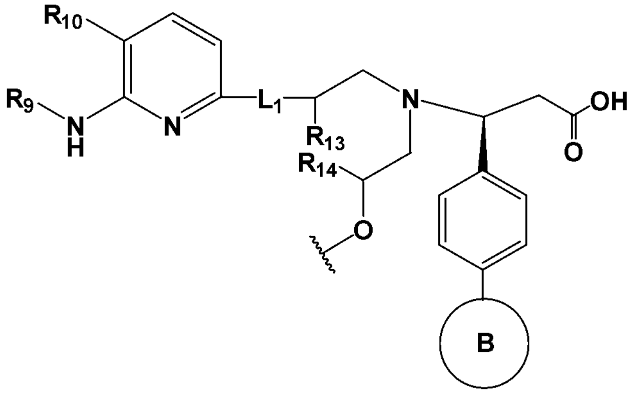

(Vc"-2)

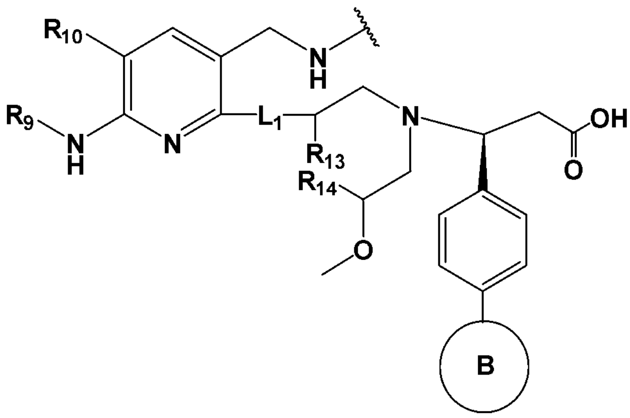

(Vc"-3)

wherein $R_9$, $R_{10}$, $R_{13}$, $R_{14}$, $L_1$, and the wave line have the same definitions as the corresponding substituents in Formula Vc", and
ring B is

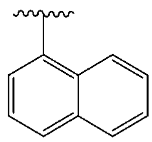 , 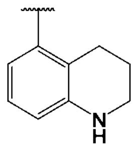 , 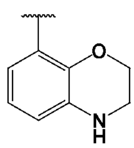 , 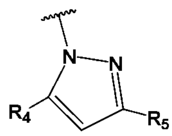 , 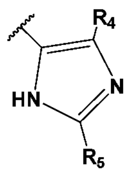 ,

or absent, wherein $R_4$ and $R_5$ are each independently H, methyl, ethyl, n-propyl, isopropyl, cyclopropyl, or cyclobutyl;
preferably $R_4$ and $R_5$ are each independently methyl;
preferably, the compound is Compound 68".

In one aspect, the present invention provides an RNAi agent, or a pharmaceutically acceptable salt, prodrug, or solvate thereof, wherein the RNAi agent has a structure represented by Formula VI, VIa, VIb, VIc, VId, VIe, VIf, VIg, VIh, VIi, VIj, or VIk:

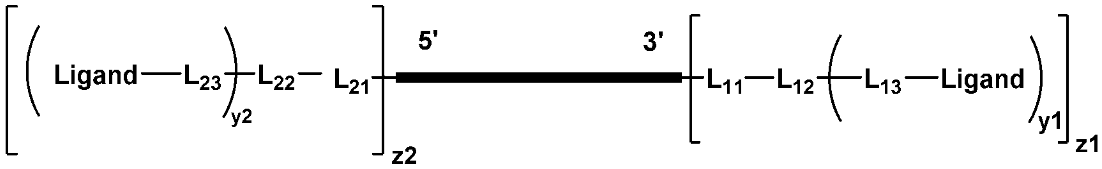

**(VI)**

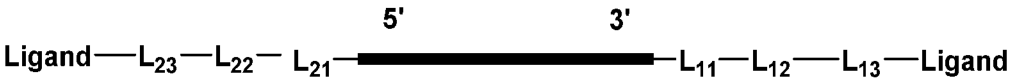

(VIa)

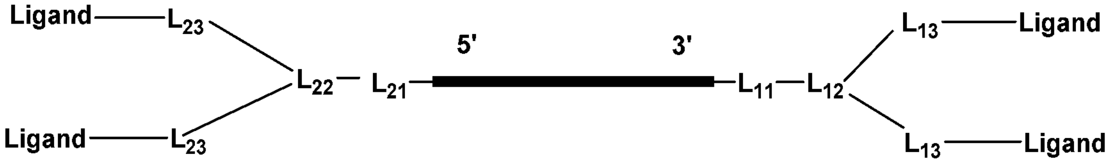

(VIb)

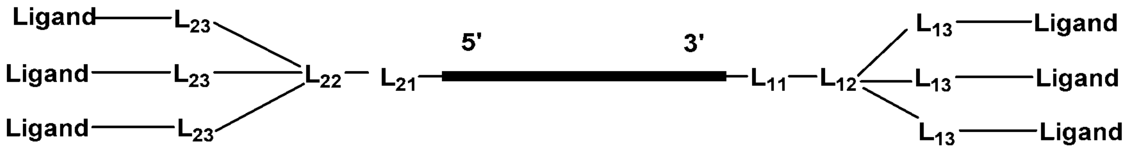

(VIc)

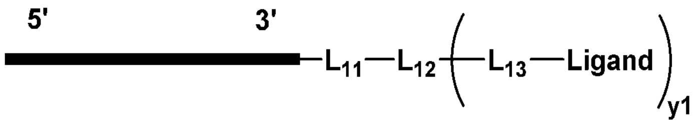

(VId)

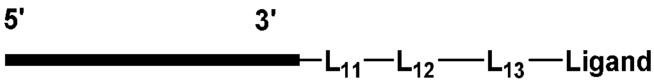

(VIe)

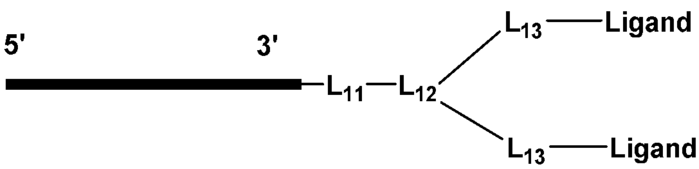

(VIf)

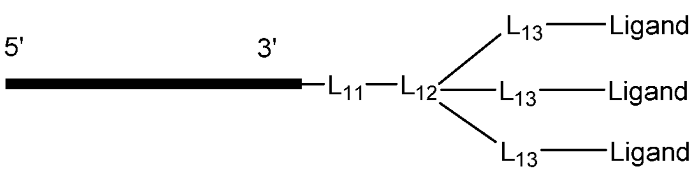

(VIg)

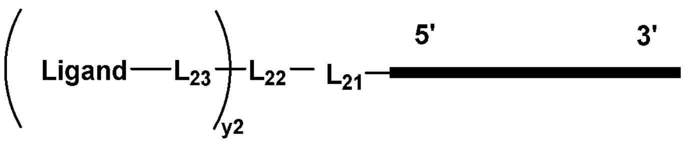

(VIh)

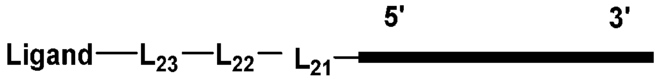


(VIi)

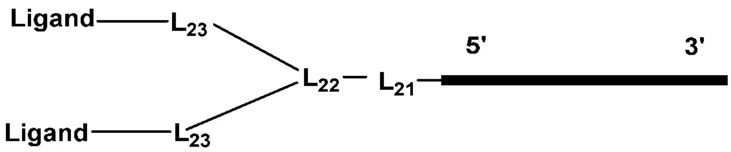


(VIj)

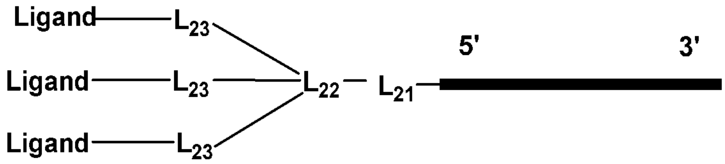


(VIk)

wherein,

is an oligonucleotide, preferably siRNA or ASO, the siRNA or ASO being connected to $L_{21}$ and/or $L_{11}$ via a phosphate group or phosphorothioate group;
z1 and z2 are each independently 0 or 1, and z1 and z2 are not both 0;
y1 and y2 are each independently 1, 2, or 3;
each Ligand is independently selected from Formula Ia" of claim 19, Formula Ib" or Ib"-1 of claim 20, Formula IIa" or IIb" of claim 21, Formula IIIa", IIIb", IIIa"-1, or IIIb"-1 of claim 22, Formula IVa" or IVa"-1 of claim 23, Formula IVb" of claim 24, Formula Va", Va"-1, Va"-2, or Va"-3 of claim 25, Formula Vb", Vb"-1, or Vb"-2 of claim 26, and Formula Vc", Vc"-1, Vc"-2, or Vc"-3 of claim 27;
$L_{11}$ and $L_{21}$ are each independently selected from:

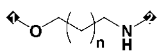
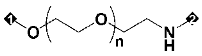
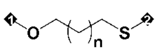
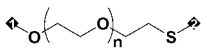
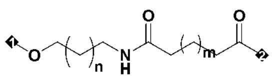
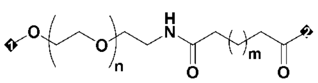
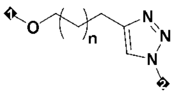
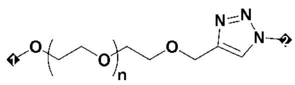
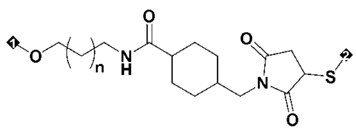
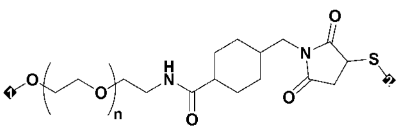
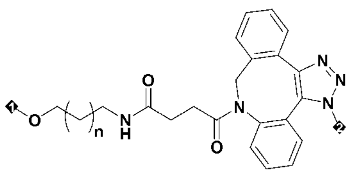
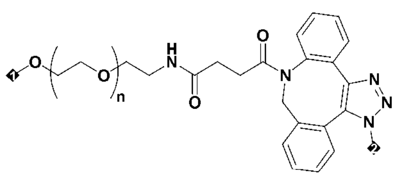
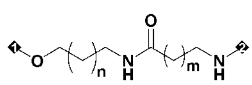

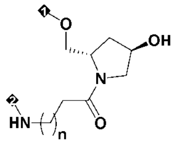
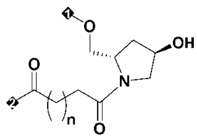
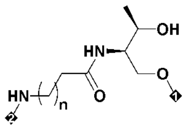
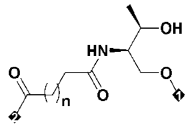
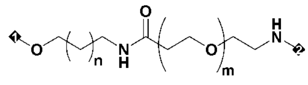
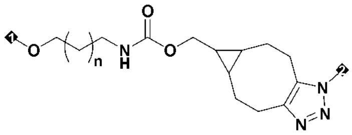
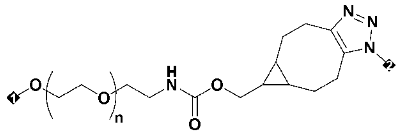

wherein n, m are integers from 0 to 10, preferably integers from 2 to 6, and attachment point 1 is connected to the oligonucleotide, attachment point 2 is connected to $L_{12}$ or $L_{22}$;

$L_{12}$ and $L_{22}$ are each independently a bond or a branching group, the branching group being independently selected from:

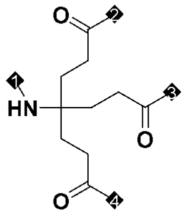
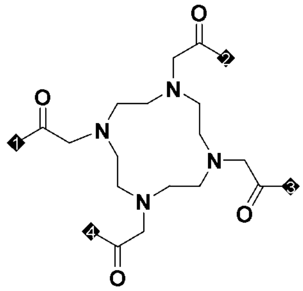
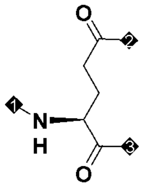
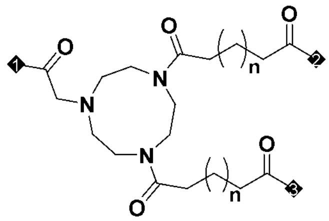

wherein n is an integer from 0 to 10, preferably an integer from 2 to 6, and attachment point 1 is connected to $L_{11}$ or $L_{21}$, attachment points 2, 3, and 4 are connected to $L_{13}$ or $L_{23}$;

$L_{13}$ and $L_{23}$ are each independently selected from:

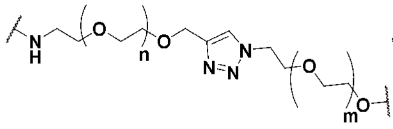
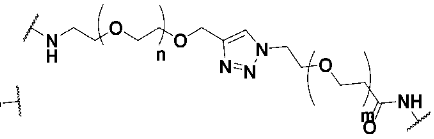
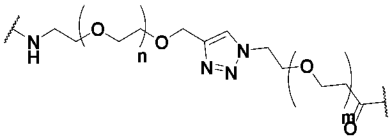
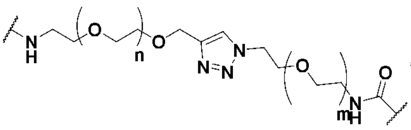
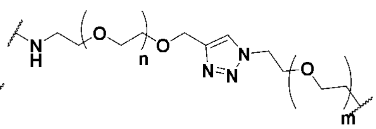
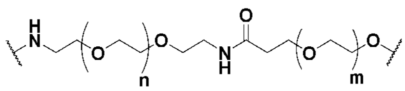
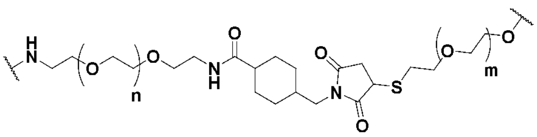
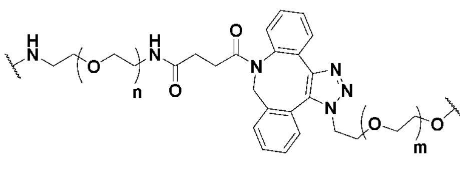
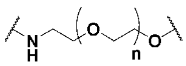
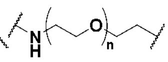
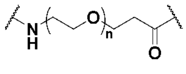
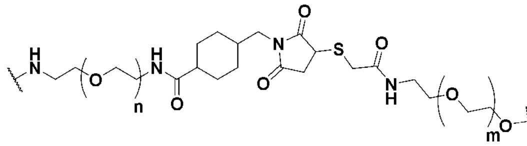
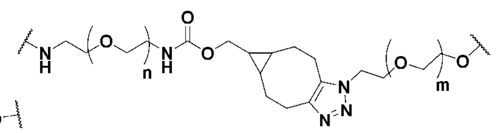
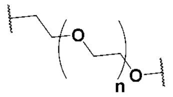

wherein n, m are integers from 0 to 10, preferably integers from 2 to 6, and the wave line on the left side of the formula is connected to $L_{12}$ or $L_{22}$, the wave line on the right side of the formula is connected to the Ligand;

preferably, $L_{21}$, $L_{22}$ and $L_{23}$ together form a structure selected from the following, wherein the left side of the formula is

connected to the oligonucleotide via a phosphate or phosphorothioate group:

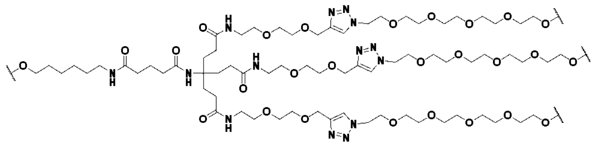

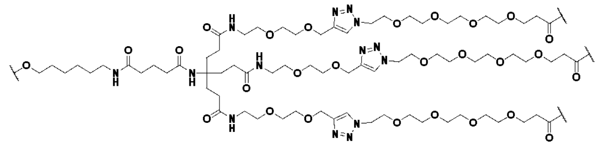

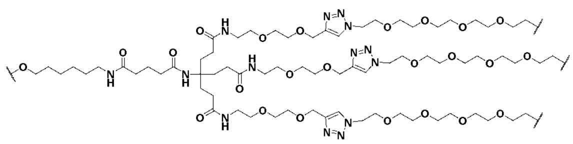

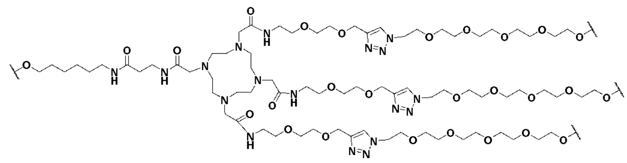

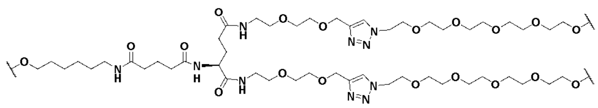

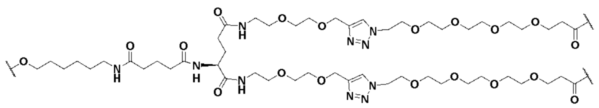

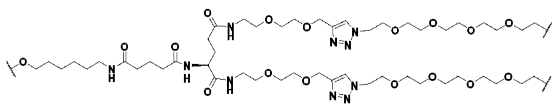

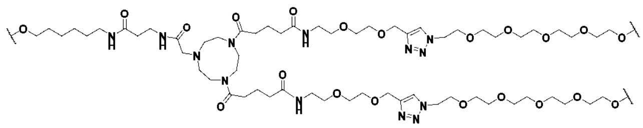

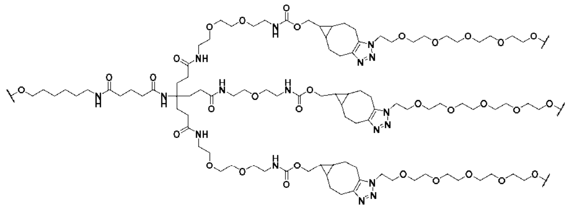

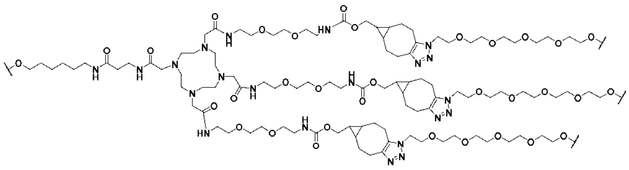

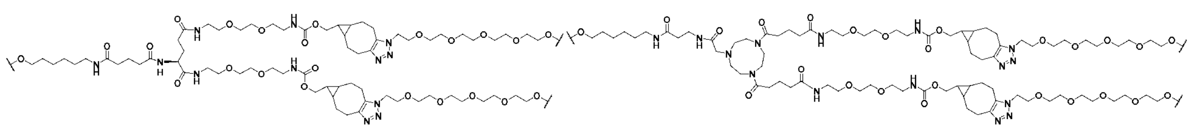
;

preferably, $L_{11}$, $L_{12}$ and $L_{13}$ together form a structure selected from the following, wherein the left side of the formula is connected to the oligonucleotide via a phosphate or phosphorothioate group:

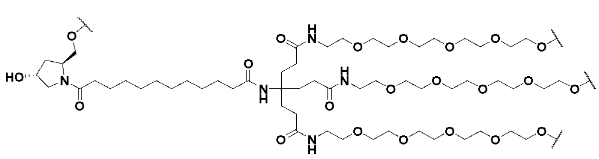

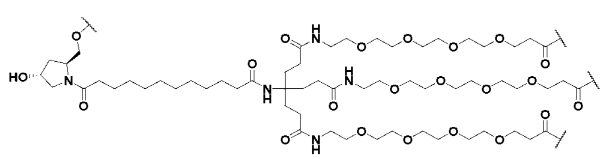

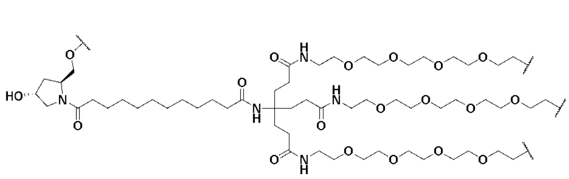

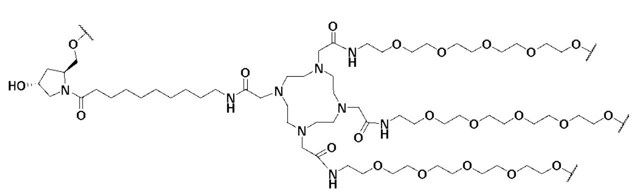

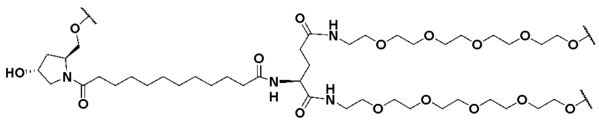 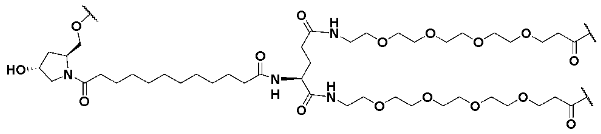

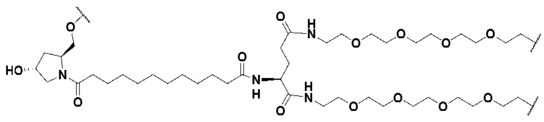 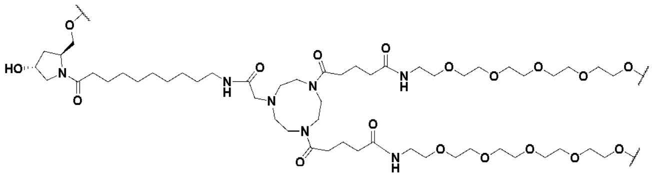

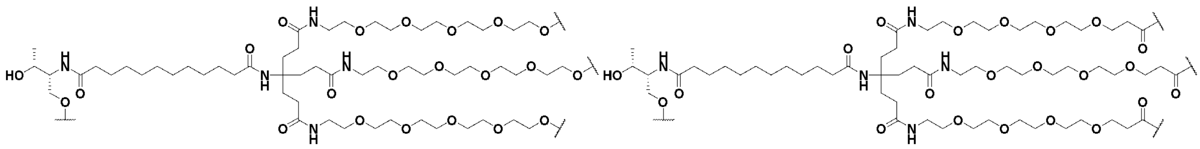

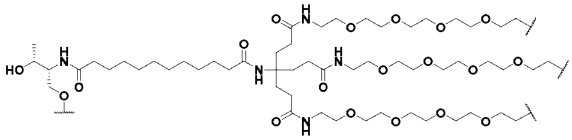 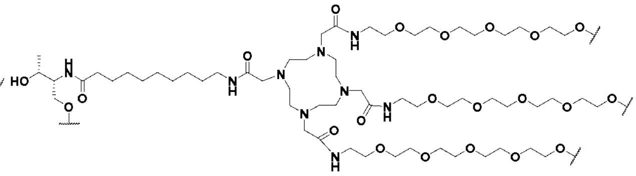

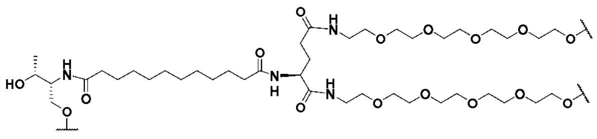 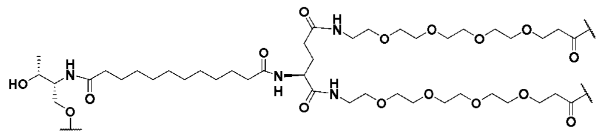

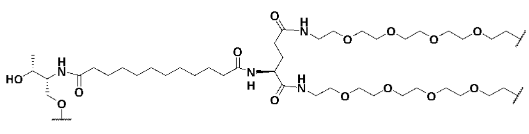 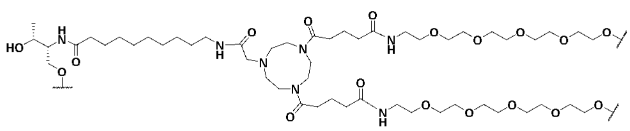 ;

preferably, the structure of the RNAi agent is selected from Formulas 201-21, 202-21, 203-21, 204-21, 205-21, 206-21, 207-21, 208-21, 209-21, 210-21, 211-21, 212-21, 213-5, 214-5, 215-5, 216-5, 217-5, 218-5, 219-5, 220-5, 221-5, 222-5, 223-5, 224-5, 225-6, 226-6, 227-6, 228-6, 229-6, 230-6, 231-6, 232-6, 233-6, 234-6, 235-6, 236-6, 237-7, 238-7, 239-7, 240-7, 241-7, 242-7, 243-7, 244-7, 245-7, 246-7, 247-7, 248-7, 249-8, 250-8, 251-8, 252-8, 253-8, 254-8, 255-8, 256-8, 257-8, 258-8, 259-8, 260-8, 261-9, 262-9, 263-9, 264-9, 265-9, 266-9, 267-9, 268-9, 269-9, 270-9, 271-9, 272-9, 273-13, 274-13, 275-13, 276-13, 277-13, 278-13, 279-13, 280-13, 281-13, 282-13, 283-13, 284-13, 285-14, 286-14, 287-14, 288-14, 289-14, 290-14, 291-14, 292-14, 293-14, 294-14, 295-14, 296-14, 297-15, 298-15, 299-15, 300-15, 301-15, 302-15, 303-15, 304-15, 305-15, 306-15, 307-15, 308-15, 309-18, 310-18, 311-18, 312-18, 313-18, 314-18, 315-18, 316-18, 317-18, 318-18, 319-18, 320-18, 321-20, 322-20, 323-20, 324-20, 325-20, 326-20, 327-20, 328-20, 329-20, 330-20, 331-20, 332-20, 333-16, 334-16, 335-16, 336-16, 337-16, 338-16, 339-16, 340-16, 341-16, 342-16, 343-16, 344-16, 345-17, 346-17, 347-17, 348-17, 349-17, 350-17, 351-17, 352-17, 353-17, 354-17, 355-17, and 356-17.

**Brief Description of the Figures**

Fig. 1 shows that some compounds of the present invention can effectively inhibit RAGE-mRNA *in vivo* in rats.

Fig. 2 shows that some compounds of the present invention can effectively inhibit serum RAGE protein expression in rats.

**Detailed Description of the Invention**

Definition

In the present invention, the compounds of the present invention include tautomers, mesomers, racemates, enantiomers, and/or diastereomers of the compounds.

In the present invention, the term "diastereomer" generally refers to stereoisomers that have two or more chiral centers and whose molecules are not mirror images of each other. Diastereomers can have different physical properties, such as melting point, boiling point, spectral properties, and reactivity.

In the present invention, the terms "tautomer" and "tautomeric form" are used interchangeably and generally refer to structural isomers of differing energy that can interconvert via a low-energy barrier. For example, proton tautomers (also known as prototropic tautomers) include interconversions via proton migration, such as keto-enol and imine-enamine isomerization. Valence tautomers include interconversions via reorganization of some bonding electrons.

In the present invention, the term "mesomer" generally refers to a molecule containing asymmetric atoms but having symmetry elements that result in a total optical rotation of zero within the molecule. The term "racemate" or "racemic mixture" refers to a composition consisting of equimolar amounts of two enantiomeric substances.

In the present invention, certain atoms of the compounds of the present invention may occur in more than one isotopic form. For example, hydrogen may exist as protium ($^{1}H$), deuterium ($^{2}H$), and tritium ($^{3}H$), and carbon naturally occurs in three different isotopes ($^{12}C$, $^{13}C$, and $^{14}C$). Isotopes that can be incorporated into the compounds of the present invention include, but are not limited to, $^{15}N$, $^{18}O$, $^{17}O$, $^{18}F$, $^{32}P$, $^{33}P$, $^{129}I$, $^{131}I$, $^{123}I$, $^{124}I$, $^{125}I$, or similar isotopes. Therefore, relative to the natural abundance of these isotopes, the compounds of the present invention can be enriched in one or more of these isotopes. As known to those skilled in the art, such isotopically enriched compounds can be used for various purposes. For example, substitution with heavy isotopes such as deuterium ($^{2}H$) may provide certain therapeutic advantages, possibly due to higher metabolic stability. For example, the natural abundance of deuterium ($^{2}H$) is about 0.015%. Therefore, for about every 6500 hydrogen atoms in nature, there is one deuterium atom. Thus, the deuterium-containing compounds of the present invention have a deuterium abundance greater than 0.015% at one or more positions (as appropriate). Unless otherwise indicated, the structures described in the present invention also encompass compounds that differ only in the presence or absence of one or more isotopically enriched atoms. For example, compounds where hydrogen atoms are replaced by deuterium or tritium, or carbon atoms are replaced by carbon-13 or carbon-14, while the rest of the structure is consistent with the present invention, are all within the scope of the present invention.

The term "halogen" refers to a halogen group. For example, "halogen" may refer to a fluorine (F), chlorine (Cl), bromine (Br), or iodine (I) group.

In the present invention, the term "pharmaceutical composition" generally refers to a mixture containing one or more compounds of the present invention or their physiologically/pharmaceutically acceptable salts or prodrugs, along with other chemical components such as physiologically/ pharmaceutically acceptable carriers and excipients. The pharmaceutical composition can facilitate administration to an organism, aid in the absorption of the active ingredient, and thus exert biological activity. Conventional preparation of pharmaceutical compositions can be found in commonly used techniques in the field.

In the present invention, the term "pharmaceutically acceptable salt" generally refers to a salt of a compound or ligand-drug conjugate of the present invention, or a salt of a compound described herein, which can be safe and/or effective when administered to a mammal and can possess the intended biological activity. The compounds of the present invention can form salts with acids. Nonlimiting examples of pharmaceutically acceptable salts include: hydrochloride, hydrobromide, hydroiodide, sulfate, bisulfate, citrate, acetate, succinate, ascorbate, oxalate, nitrate, sorbate, phosphate, hydrogen phosphate, dihydrogen phosphate, salicylate, hydrogen citrate, tartrate, maleate, fumarate, formate, benzoate, methanesulfonate, ethanesulfonate, benzenesulfonate, or p-toluenesulfonate.

In the present invention, the term "pharmaceutically acceptable carrier" generally refers to a carrier for delivering a therapeutic agent. The term refers to any pharmaceutical carrier that itself does not induce the production of antibodies harmful to the individual receiving the composition and can be administered without causing excessive toxicity. For example, a pharmaceutically acceptable carrier can be distinguished from a nucleic acid vector used in genetic engineering to contain a target gene. Suitable carriers can be large, slowly metabolized macromolecules such as proteins, polysaccharides, polylactic acid, polyglycolic acid, polyamino acids, amino acid copolymers, lipid aggregates, and inactivated virus particles. These carriers are well known to those skilled in the art. Pharmaceutically acceptable carriers in therapeutic compositions can include liquids such as water, saline, glycerol, and ethanol. Auxiliary substances such as wetting agents or emulsifiers, pH buffering agents, etc., can also be present in these carriers.

In the present invention, the term "effective amount" generally refers to an amount of a therapeutic agent that treats, alleviates, or prevents a target disease or condition, or exhibits a detectable therapeutic or preventive effect. The precise effective amount for a subject depends on the subject’s size and health status, the nature and extent of the condition, and the chosen therapeutic agent and/or combination of therapeutic agents. Therefore, it is not useful to pre-specify an exact effective amount. However, an effective amount for a given condition can be determined by routine experimentation, which a clinician can judge.

Unless otherwise specified, in the present invention, all compounds mentioned are intended to include all possible optical isomers, such as single chiral compounds, or mixtures of various chiral compounds (i.e., racemates). For all compounds of the present invention, each chiral carbon atom may optionally be R configuration or S configuration, or a mixture of R and S configurations.

As used herein, the term "RNAi agent" refers to a reagent comprising an RNA molecule that, when introduced into a cell, is capable of down-regulating the expression of a target gene through an RNA interference mechanism. The terms "RNAi agent of the present invention", "RNAi agent described herein", or similar expressions encompass naked or modified RNAi agents, irrespective of sequence and target gene. RNA interference refers to a process whereby a nucleic acid molecule induces cleavage and degradation of a target RNA molecule (e.g., an mRNA molecule) in a sequence-specific manner, such as through the RNA-induced silencing complex (RISC) pathway. RNAi agents herein include siRNA, shRNA, and DNA/RNA hybrid molecules, also sometimes collectively referred to as double-stranded RNA (dsRNA), which comprise two antiparallel continuous nucleotide strands sufficiently complementary to hybridize and form a double-stranded region. "Hybridization" refers to the pairing of complementary polynucleotides, typically through hydrogen bonding between complementary bases in the two polynucleotides (e.g., Watson-Crick hydrogen bonding, Wobble hydrogen bonding, Hoogsteen hydrogen bonding, or reverse Hoogsteen hydrogen bonding). The "double-stranded region" refers to an area within two complementary or substantially complementary polynucleotides that form base pairs through hybridization, thereby creating a duplex between the two polynucleotide strands.

The term "antisense strand" refers to the strand in a dsRNA that contains a region substantially complementary to a target sequence. The term "sense strand" or "passenger strand" refers to the strand in a dsRNA that contains a region substantially complementary to the antisense strand region as defined herein. The term "region substantially complementary" denotes a region that is either perfectly complementary or imperfectly complementary. When the complementary region is imperfectly complementary to the target sequence, mismatches may occur in internal or terminal regions of the molecule. Typically, the most tolerated mismatches are located in terminal regions, for example, within 5, 4, 3, or 2 nucleotides at the 5' and/or 3' termini of the dsRNA.

"siRNA" refers to a nucleic acid that forms a double-stranded RNA capable of reducing or inhibiting the expression of a target gene when present in the same cell as the target gene. An siRNA is typically approximately 15 to approximately 30 base pairs in length, most typically approximately 19 to 25 base pairs in length, for example, 19, 20, 21, 22, 23, 24, or 25 nucleotide pairs in length.

"shRNA" refers to short hairpin RNA, which comprises two short inverted repeats and an intervening stem-loop structure connecting them. The stem-loop may comprise at least one unpaired nucleotide, for example, at least 2, at least 3, at least 4, at least 5, at least 6, at least 7, at least 8, at least 9, at least 10, at least 20, at least 23, or more unpaired nucleotides. The stem-loop may be 10 or fewer nucleotides in length. The stem-loop may comprise 8 or fewer unpaired nucleotides. The stem-loop may comprise 4 to 10 unpaired nucleotides. The stem-loop may be 4 to 8 nucleotides in length.

The two substantially complementary strands of a dsRNA need not be, but may be, covalently linked. The maximum number of base pairs is the number of nucleotides in the shortest strand of the dsRNA minus any overhangs present in the duplex. In addition to the duplex structure, a dsRNA may comprise one or more nucleotide overhangs. An overhanging nucleotide refers to one or more unpaired nucleotides extending beyond the double-stranded region at the terminus of a strand. Nucleotide overhangs typically occur when the 3' end of one strand extends beyond the 5' end of the other strand, or when the 5' end of one strand extends beyond the 3' end of the other strand. For example, at least one strand comprises a 3' overhang of at least 1 nucleotide, e.g., 1 to 4 nucleotide overhangs. In another example, at least one strand comprises a 5' overhang of at least 1 nucleotide, e.g., 1 to 4 nucleotide overhangs. In other embodiments, both the 3' terminus and the 5' terminus of one strand of the dsRNA comprise an overhang of at least 1 nucleotide.

As used herein, the terms "blunt end" or "blunt terminus" with respect to dsRNA refer to the absence of unpaired nucleotides or nucleotide analogs at a given terminus of the dsRNA, i.e., no nucleotide overhang. One end or both ends of the dsRNA may be blunt. Where both ends of the dsRNA are blunt, the dsRNA is referred to as blunt-ended. It is to be expressly understood that a "blunt-ended" dsRNA is one having blunt termini at both ends, meaning no nucleotide overhang exists at either end of the molecule. In most cases, such molecules are double-stranded throughout their entire length. As used herein, the term "nucleotide overhang" refers to at least one unpaired nucleotide projecting from the duplex structure of the dsRNA. For example, a nucleotide overhang exists when the 3' terminus of one strand of the dsRNA extends beyond the 5' terminus of the other strand, or vice versa. The overhang may comprise or consist of nucleotides/nucleosides analogs, including deoxyribonucleotides/deoxyribonucleosides. Overhangs may be present on the sense strand, antisense strand, or any combination thereof. Furthermore, the overhanging nucleotides may reside at the 5' terminus, 3' terminus, or both termini of the antisense strand or sense strand of the dsRNA.

The dsRNA molecule may comprise chemical modifications to ribonucleotides, including modifications to the ribose, base, or backbone components of ribonucleic acid, as described herein or known in the art. Any such modifications, when used in double-stranded ribonucleic acid molecules (e.g., siRNA, shRNA, etc.), are encompassed by the term

"dsRNA" for purposes of this disclosure. A "modified" nucleotide refers to a nucleotide that independently has a modified sugar moiety, a modified internucleoside linkage, and/or a modified nucleobase. Accordingly, the term "modified nucleotide" includes substitutions, additions, or removals of functional groups or atoms to the internucleoside linkage, sugar moiety, or nucleobase. Modifications suitable for the present invention include all types of modifications disclosed herein or known in the art. For example, the modified nucleotide is selected from the group consisting of: 2'-deoxythymidine (dT) nucleotide, 2'-O-methyl-modified nucleotide, 2'-fluoro-modified nucleotide, 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide, 2'-amino-modified nucleotide, 2'-O-allyl-modified nucleotide, 2'-C-alkyl-modified nucleotide, 2'-methoxyethyl-modified nucleotide, abasic nucleotide, inverted abasic nucleotide, inverted nucleotide, morpholino nucleotide, phosphoramidate, tetrahydropyran-modified nucleotide, 1,5-anhydrohexitol-modified nucleotide, cyclohexenyl-modified nucleotide, nucleotide comprising phosphorothioate group, nucleotide comprising methylphosphonate group, nucleotide comprising 5'-phosphate, and nucleotide comprising 5'-phosphate mimic; preferably selected from 2'-O-methyl-modified nucleotide, 2'-fluoro-modified nucleotide, and nucleotide comprising phosphorothioate internucleoside linkage.

The term "treatment" encompasses prevention, therapy, and cure. The patient receiving such treatment is typically any animal in need, including primates (particularly humans) and other mammals such as horses, cattle, swine, sheep, poultry, and companion animals.

**Integrin Ligands for RNAi Agents**

Compounds described herein that have affinity for certain integrins, including $\alpha v\beta 6$, are useful as ligands (herein referred to as "integrin ligands" or "$\alpha v\beta 6$ ligands") to selectively target the compounds or other molecules to which they are linked to cells or tissues expressing integrin $\alpha v\beta 6$. The integrin ligands disclosed herein can be conjugated to oligonucleotide molecules (e.g., oligonucleotides in an RNAi agent, such as siRNA or ASO) to facilitate delivery of the oligonucleotide molecules to cells or tissues expressing integrin $\alpha v\beta 6$.

**In** some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ia:

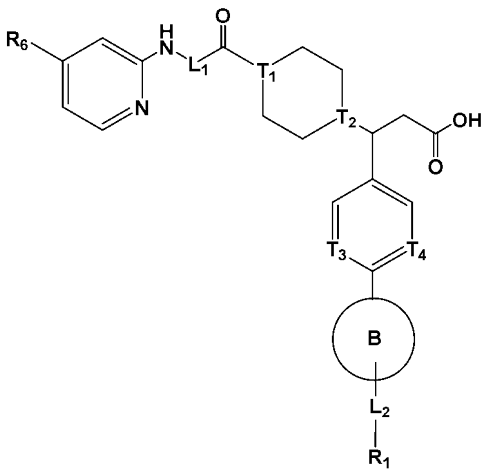

(Ia)

wherein,

$T_1$ and $T_2$ are each independently C or N, and at least one is N;
$T_3$ and $T_4$ are each independently C or N;
ring B is

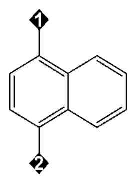, 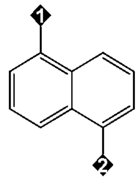, 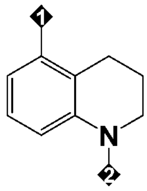, 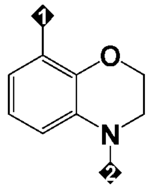, 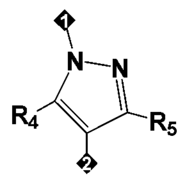, 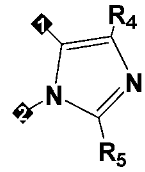,

or absent, wherein attachment point 2 is connected to $L_2$;

$R_4$, $R_5$ and $R_6$ are each independently H, methyl, ethyl, n-propyl, isopropyl, cyclopropyl, or cyclobutyl;

$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10;

$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;

$R_1$ is $-N_3$, $-NH_2$, -COOH, $-NHC(O)CH_2SC(O)CH_3$, or

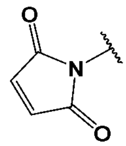.

In preferred embodiments, $T_1$ and $T_2$ are both N. In preferred embodiments, one of $T_3$ and $T_4$ is N. In preferred embodiments, $T_3$ and $T_4$ are both C. In preferred embodiments, $R_4$, $R_5$ and $R_6$ are each independently methyl. In preferred embodiments, ring B is

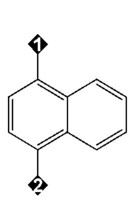, 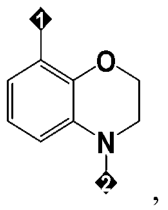,

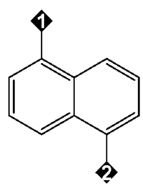 or 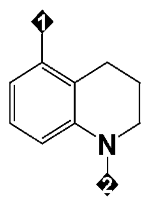.

In preferred embodiments, m and n are each independently 1, 2, 3, 4, 5, or 6. In preferred embodiments, $L_1$ is $-(CH_2)_n-$, n is 1, 2, 3, 4, 5, or 6. In preferred embodiments, p is 3, 4, 5, 6, or 7. In preferred embodiments, $L_2$ is $-(OCH_2CH_2)_p-$, p is 3, 4, 5, 6, or 7. In preferred embodiments, $R_1$ is $-N_3$. In preferred embodiments, the compound is selected from Compounds 4, 21, 22, 23, 24, 25, 26, 27, 28, 29, 30, 31, 32, 33, and 34.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib:

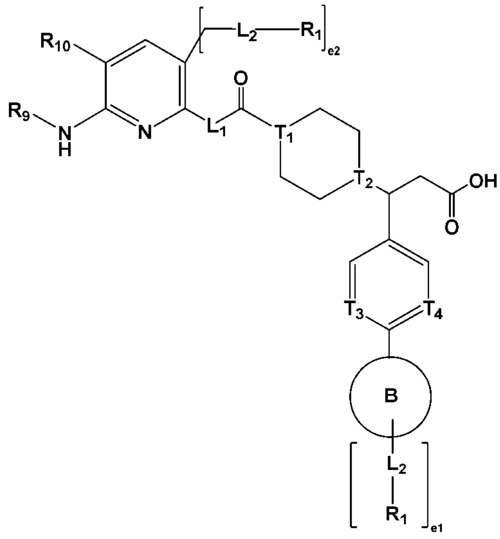

(Ib)

wherein,

$T_1$ and $T_2$ are each independently C or N, and at least one is N;
$T_3$ and $T_4$ are each independently C or N;
one of e1 and e2 is 1, and the other is 0;
ring B is

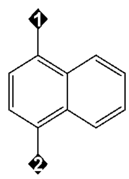 , 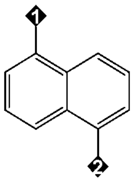 , 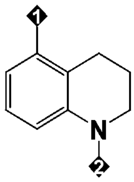 , 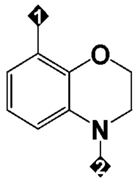 , 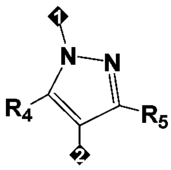 , 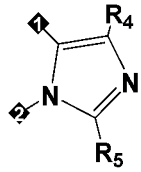 ,

or absent, wherein attachment point 2 is connected to $L_2$, and attachment point 2 is absent when e1 is 0;
$R_4$ and $R_5$ are each independently H, methyl, ethyl, n-propyl, isopropyl, cyclopropyl, or cyclobutyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10;
when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-NHC(O)(CH_2)_q(OCH_2CH_2)_p-$, wherein the right side is connected to $R_1$, p and q are each independently an integer from 1 to 10;
$R_1$ is $-N_3$, $-NH_2$, $-COOH$, $-NHC(O)CH_2SC(O)CH_3$, or

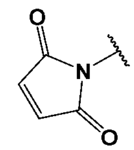 ;

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group.

In preferred embodiments, $T_1$ and $T_2$ are both N. In preferred embodiments, one of $T_3$ and $T_4$ is N. In preferred embodiments, $T_3$ and $T_4$ are both C. In preferred embodiments, ring B is

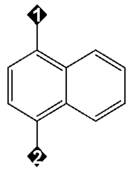, 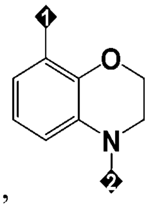, 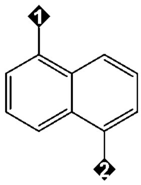 or 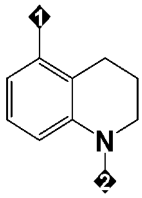.

In preferred embodiments, $R_4$, $R_5$ and $R_6$ are each independently methyl. In preferred embodiments, m and n are each independently 1, 2, 3, 4, 5, or 6. In preferred embodiments, when e1 is 1, $L_1$ is $-(CH_2)_n-$, n is 1, 2, 3, 4, 5, or 6. In preferred embodiments, p is 3, 4, 5, 6, or 7. In preferred embodiments, $L_2$ is $-(OCH_2CH_2)_p-$, p is 3, 4, 5, 6, or 7. In preferred embodiments, q is 2 or 3. In preferred embodiments, $R_1$ is $-N_3$. In preferred embodiments, $R_9$ is methyl and $R_{10}$ is H. In preferred embodiments, $R_9$, $R_{10}$, together with the C and N to which they are attached, form a piperidinyl group. In preferred embodiments, the compound is Compound 35.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa or IIb:

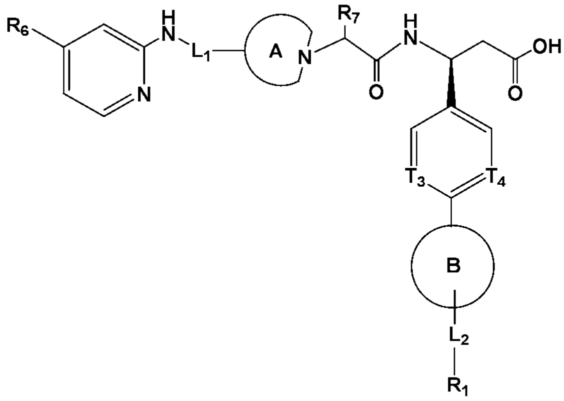
(IIa)

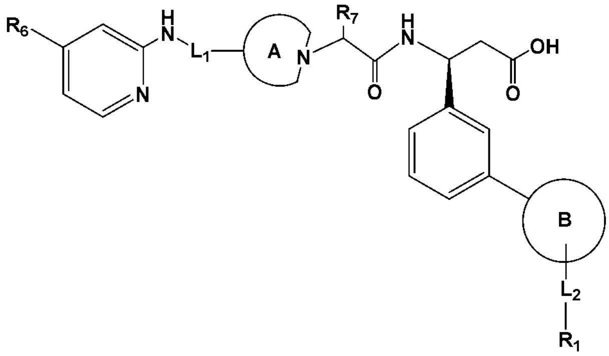
(IIb)

wherein,

$T_3$ and $T_4$ are each independently C or N;
ring A is

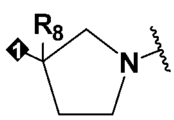, 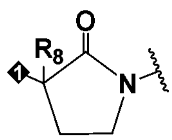, 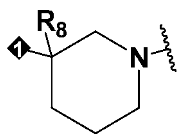, or 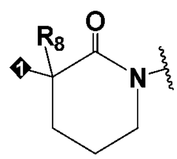,

wherein attachment point 1 is connected to $L_1$; $R_8$ is H, methyl, ethyl, n-propyl, isopropyl, cyclopropyl, cyclobutyl, or

halogen;
ring B is

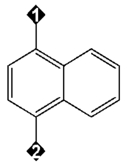, 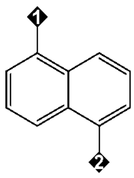, 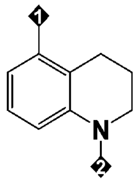, 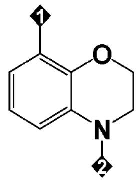, 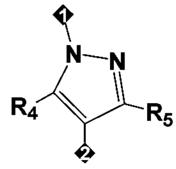, 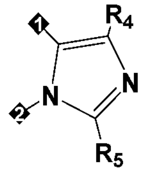,

or absent, wherein attachment point 2 is connected to $L_2$;
$R_4$, $R_5$, $R_6$, and $R_7$ are each independently H, methyl, ethyl, n-propyl, isopropyl, cyclopropyl, or cyclobutyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10;
$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;
$R_1$ is $-N_3$, $-NH_2$, $-COOH$, $-NHC(O)CH_2SC(O)CH_3$, or

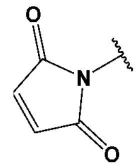.

In preferred embodiments, one of $T_3$ and $T_4$ is N. In preferred embodiments, $T_3$ and $T_4$ are both C. In preferred embodiments, $R_8$ is H or halogen, more preferably H or F. In preferred embodiments, ring A is

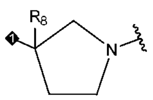

and $R_8$ is H.

In preferred embodiments, ring B is

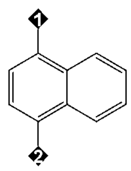, 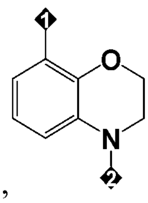, 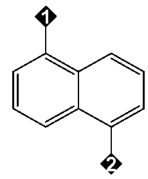 or 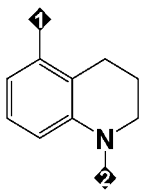.

In preferred embodiments, $R_4$, $R_5$ and $R_6$ are each independently methyl. In preferred embodiments, $R_7$ is H. In preferred embodiments, m and n are each independently 1, 2, 3, 4, 5, or 6. In preferred embodiments, $L_1$ is $-(CH_2)_n-$, n is 1, 2, 3, 4, 5, or 6. In preferred embodiments, p is 3, 4, 5, 6, or 7. In preferred embodiments, $R_1$ is $-N_3$. In preferred embodiments, the compound is any one of Compounds 5, 36, 37, 38, 39, 40, 41, 42, 43, or 44.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa or IIIb:

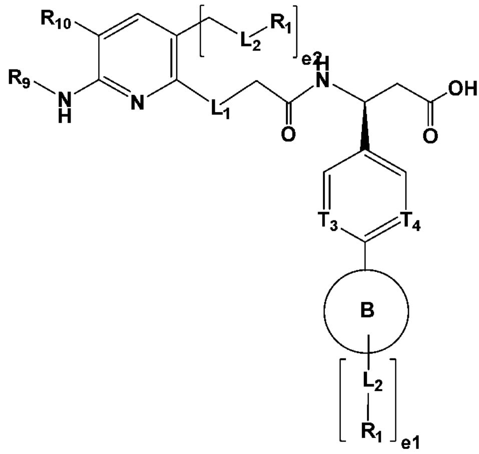

(IIIa)

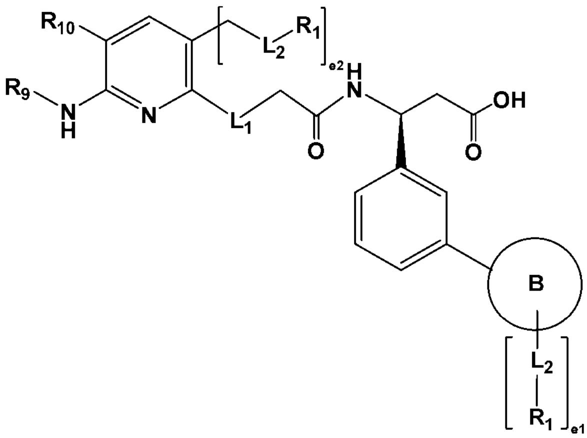

(IIIb)

wherein,

$T_3$ and $T_4$ are each independently C or N;
one of e1 and e2 is 1, and the other is 0;
ring B is

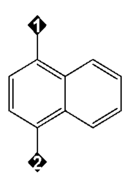, 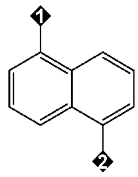, 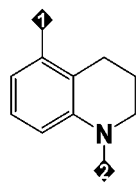, 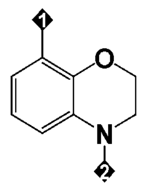, 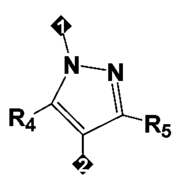, 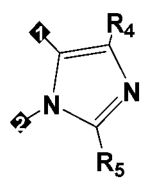,

or absent, wherein attachment point 2 is connected to $L_2$, and attachment point 2 is absent when e1 is 0;
$R_4$ and $R_5$ are each independently H, methyl, ethyl, n-propyl, isopropyl, cyclopropyl, or cyclobutyl;
$L_1$ is $-(CH_2)_m-$, $-(CH_2)_mC(O)NH(CH_2)_n-$, or $-(CH_2)_mNHC(O)(CH_2)_n-$, m is an integer from 1 to 10, n is an integer from 0 to 10;
when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-NHC(O)(CH_2)_q(OCH_2CH_2)_p-$, wherein the right side is connected to $R_1$, p and q are each independently an integer from 1 to 10;
$R_1$ is $-N_3$, $-NH_2$, $-COOH$, $-NHC(O)CH_2SC(O)CH_3$, or

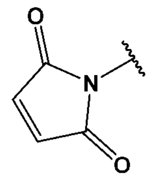

;

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group.

In preferred embodiments, one of $T_3$ and $T_4$ is N. In preferred embodiments, $T_3$ and $T_4$ are both C. In preferred embodiments, ring B is

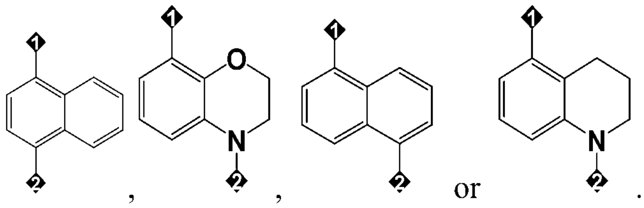

, , or .

In preferred embodiments, $R_4$ and $R_5$ are each independently methyl. In preferred embodiments, m is 1, 2, 3, 4, 5, or 6. In preferred embodiments, n is 0, 1, 2, or 3. In preferred embodiments, $L_1$ is $-(CH_2)_m-$, m is 1, 2, 3, 4, 5, or 6. In preferred embodiments, p is 3, 4, 5, 6, or 7. In preferred embodiments, q is 2 or 3. In preferred embodiments, when e1 is 1, $L_2$ is $-(OCH_2CH_2)_p-$, p is 3, 4, 5, 6, or 7. In preferred embodiments, $R_1$ is $-N_3$. In preferred embodiments, $R_9$ is methyl and $R_{10}$ is H. In preferred embodiments, $R_9$, $R_{10}$, together with the C and N to which they are attached, form a piperidinyl group. In preferred embodiments, the compound is Compound 6, 7, 8, 9, 45, 46, or 47.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa:

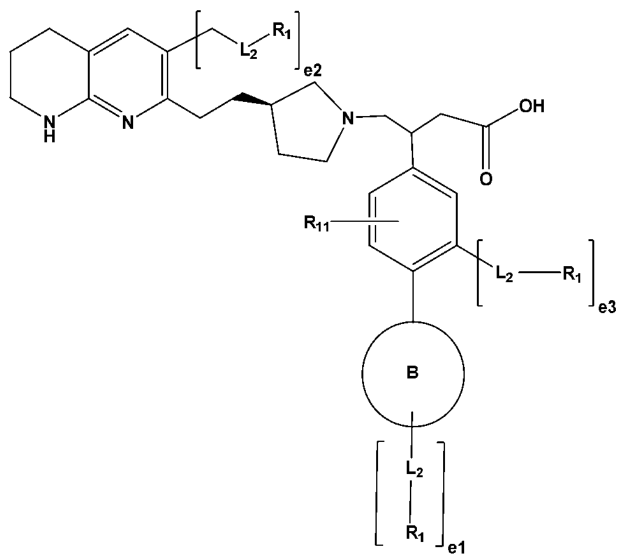

(IVa)

wherein,

one of e1, e2, and e3 is 1, and the other two are 0;
ring B is

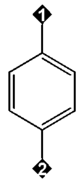 , 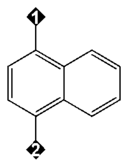 , 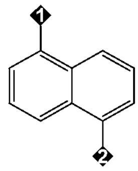 , 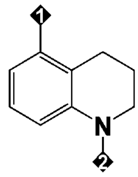 , 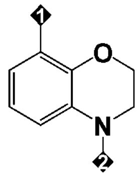 , 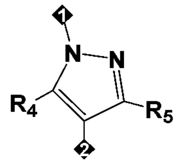 , 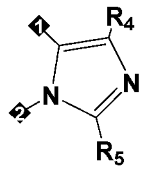 ,

or absent, wherein attachment point 2 is connected to $L_2$, and attachment point 2 is absent when e1 is 0; $R_4$ and $R_5$ are each independently H, methyl, ethyl, n-propyl, isopropyl, cyclopropyl, or cyclobutyl; when e1 or e3 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;

when e2 is 1, $L_2$ is $-NHC(O)(CH_2)_q(OCH_2CH_2)_p-$, wherein the right side is connected to $R_1$, p and q are each independently an integer from 1 to 10;

$R_1$ is $-N_3$, $-NH_2$, $-COOH$, $-NHC(O)CH_2SC(O)CH_3$, or

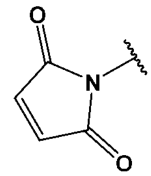 ;

$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy.

In preferred embodiments, ring B is

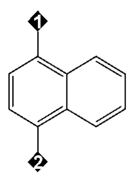 , 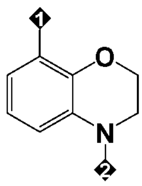 , 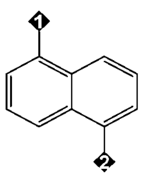 , 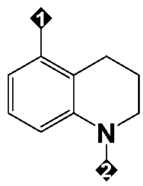 , 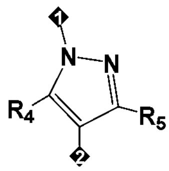 or 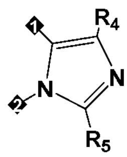 .

In preferred embodiments, $R_4$ and $R_5$ are both methyl. In preferred embodiments, q is 1, 2, or 3. In preferred embodiments, p is 3, 4, 5, 6, or 7. In preferred embodiments, when e1 or e3 is 1, $L_2$ is $-(OCH_2CH_2)_p-$, p is 3, 4, 5, 6, or 7. In preferred embodiments, $R_1$ is $-N_3$. In preferred embodiments, $R_{11}$ is H. In preferred embodiments, the compound is Compound 13, 14, 15, 18, 20, 48, 49, 53, 54, 55, 56, 57, 58, 59, 60, 61, 62, 63, 71, 72, 73, 83, 84, 85, 86, or 87.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVb:

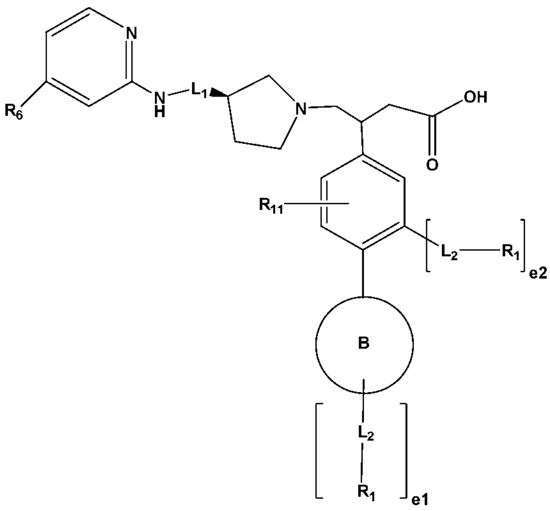

(IVb)

one of e1 and e2 is 1, and the other is 0;
ring B is

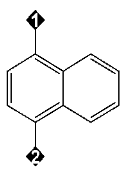 , 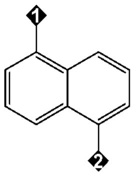 , 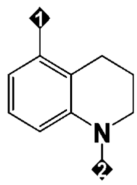 , 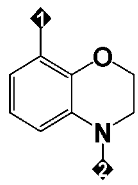 , 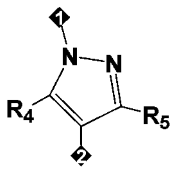 , 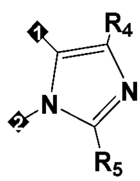 ,

or absent, wherein attachment point 2 is connected to $L_2$;
$R_4$, $R_5$ and $R_6$ are each independently H, methyl, ethyl, n-propyl, isopropyl, cyclopropyl, or cyclobutyl;
$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;
$R_1$ is $-N_3$, $-NH_2$, -COOH, $-NHC(O)CH_2SC(O)CH_3$, or

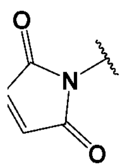 ;

$L_1$ is $-(CH_2)_m-$, $-(CH_2)_mC(O)NH(CH_2)_n-$, or $-(CH_2)_mNHC(O)(CH_2)_n-$; m is an integer from 1 to 10;
$R_{11}$ is H, halogen, halomethyl, methyl, ethyl, n-propyl, isopropyl, cyclopropyl, cyclobutyl, phenyl, morpholinyl, methoxyethoxy, or ethoxymethoxy;

In preferred embodiments, ring B is

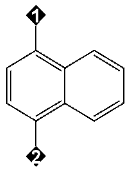 , 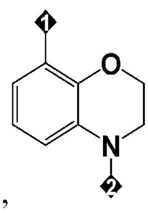 , 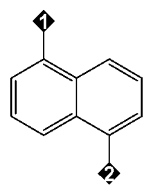 or 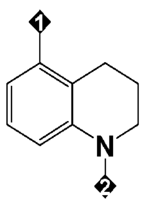 .

In preferred embodiments, $R_4$, $R_5$ and $R_6$ are all methyl. In preferred embodiments, q is 2 or 3. In preferred embodiments, p is 3, 4, 5, 6, or 7. In preferred embodiments, $L_2$ is $-(OCH_2CH_2)_p-$, p is 3, 4, 5, 6, or 7. In preferred embodiments, $R_1$ is $-N_3$. In

preferred embodiments, m is 1, 2, 3, 4, 5, or 6. In preferred embodiments, n is 0, 1, 2, or 3. In preferred embodiments, $L_1$ is $-(CH_2)_m-$, m is 1, 2, 3, 4, 5, or 6. In preferred embodiments, preferably $R_{11}$ is H. In preferred embodiments, the compound is Compound 50, 51, 52, 74, 75, 76, 77, 78, 79, or 80.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va:

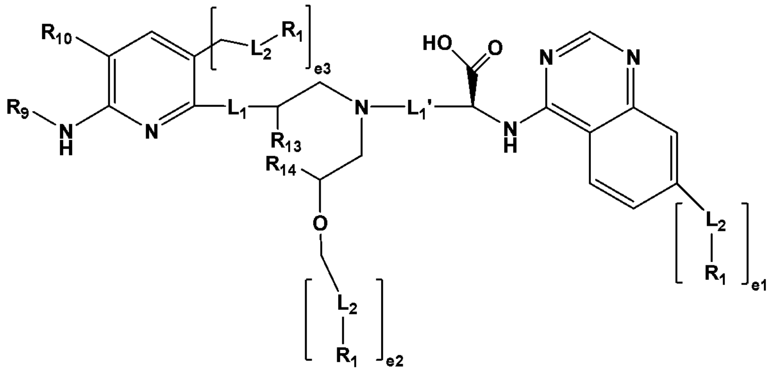

(Va)

one of e1, e2, and e3 is 1, and the other two are 0;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group;

$R_{13}$ and $R_{14}$ are each H, or $R_{13}$, $R_{14}$, together with the C and N atoms to which they are attached, form a piperidinyl group;

$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10;

when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;

when e2 is 1, $L_2$ is $-(CH_2)q(OCH_2CH_2)_p-$, wherein the right side is connected to $R_1$, p and q are each independently an integer from 1 to 10;

when e3 is 1, $L_2$ is $-NHC(O)(CH_2)q(OCH_2CH_2)_p-$, wherein the right side is connected to $R_1$, p and q are each independently an integer from 1 to 10;

$R_1$ is $-N_3$, $-NH_2$, $-COOH$, $-NHC(O)CH_2SC(O)CH_3$, or

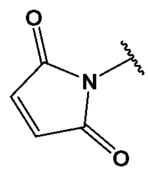

.

In preferred embodiments, $R_9$ is methyl and $R_{10}$ is H. In preferred embodiments, m is 1, 2, 3, 4, 5, or 6. In preferred embodiments, n is 0, 1, 2, or 3. In preferred embodiments, $L_1$ and $L_1$' are $-(CH_2)_m-$, m is 1, 2, 3, 4, 5, or 6. Preferably, the compound is Compound 16, 17, 64, 65, 66, or 67. In preferred embodiments, q is 1, 2, or 3. In preferred embodiments, p is 3, 4, 5, 6, or 7. In preferred embodiments, when e1 is 1, $L_2$ is $-(OCH_2CH_2)_p-$, p is 3, 4, 5, 6, or 7. In preferred embodiments, $R_1$ is $-N_3$. In preferred embodiments, the compound is Compound 16, 17, 64, 65, 66, or 67.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb:

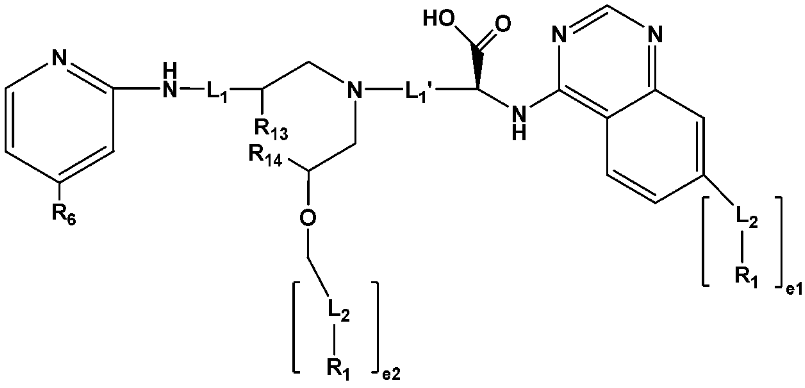

(Vb)

wherein

one of e1 and e2 is 1, and the other is 0;
$R_6$ is H, methyl, ethyl, n-propyl, isopropyl, cyclopropyl, or cyclobutyl;
$R_{13}$ and $R_{14}$ are each H, or $R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10;
when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-(CH_2)q(OCH_2CH_2)_p-$, wherein the right side is connected to $R_1$, p and q are each independently an integer from 1 to 10;
$R_1$ is $-N_3$, $-NH_2$, -COOH, $-NHC(O)CH_2SC(O)CH_3$, or

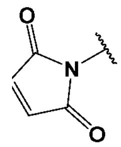 .

In preferred embodiments, $R_6$ is methyl. In preferred embodiments, m is 1, 2, 3, 4, 5, or 6. In preferred embodiments, n is 0, 1, 2, or 3. In preferred embodiments, $L_1$ and $L_1$' are $-(CH_2)_m-$, m is 1, 2, 3, 4, 5, or 6. In preferred embodiments, p is 3, 4, 5, 6, or 7. In preferred embodiments, q is 1, 2, or 3. In preferred embodiments, when e1 is 1, $L_2$ is $-(OCH_2CH_2)_p-$, p is 3, 4, 5, 6, or 7. In preferred embodiments, $R_1$ is $-N_3$. In preferred embodiments, the compound is Compound 69 or 70.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc:

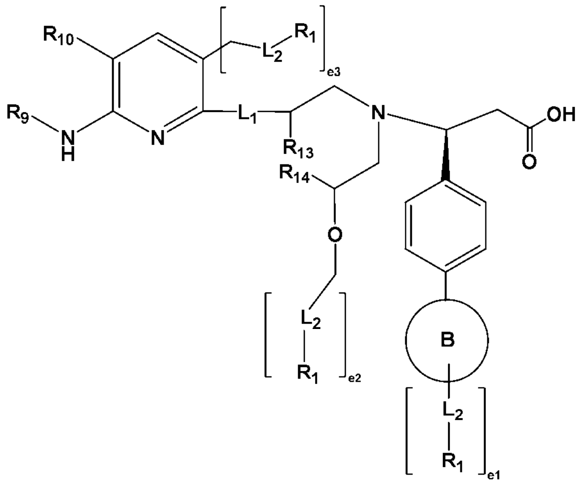

(Vc)

one of e1, e2, and e3 is 1, and the other two are 0;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group;

$R_{13}$ and $R_{14}$ are each H, or $R_{13}$, $R_{14}$, together with the C and N atoms to which they are attached, form a piperidinyl group;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10;

when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;

when e2 is 1, $L_2$ is $-(CH_2)q(OCH_2CH_2)_p-$, wherein the right side is connected to $R_1$, p and q are each independently an integer from 1 to 10;

when e3 is 1, $L_2$ is $-NHC(O)(CH_2)q(OCH_2CH_2)_p-$, wherein the right side is connected to $R_1$, p and q are each independently an integer from 1 to 10;

$R_1$ is $-N_3$, $-NH_2$, -COOH, $-NHC(O)CH_2SC(O)CH_3$, or

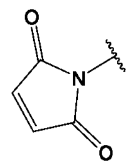;

ring B is

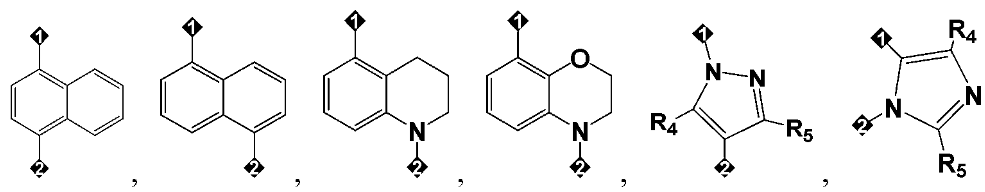

or absent, wherein attachment point 2 is connected to $L_2$, and attachment point 2 is absent when e1 is 0;

$R_4$ and $R_5$ are each independently H, methyl, ethyl, n-propyl, isopropyl, cyclopropyl, or cyclobutyl.

In preferred embodiments, $R_9$ is methyl and $R_{10}$ is H. In preferred embodiments, m is 1, 2, 3, 4, 5, or 6. In preferred embodiments, n is 0, 1, 2, or 3. In preferred embodiments, $L_1$ is $-(CH_2)_m-$, m is 1, 2, 3, 4, 5, or 6. In preferred embodiments, p is 3, 4, 5, 6, or 7. In preferred embodiments, q is 1, 2, or 3. In preferred embodiments, when e1 is 1, $L_2$ is $-(OCH_2CH_2)_p-$, p is 3, 4, 5, 6, or 7. In preferred embodiments, $R_1$ is $-N_3$.

In preferred embodiments, ring B is

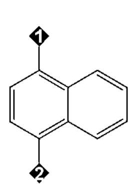, 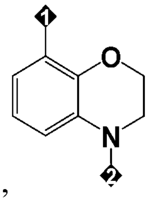, 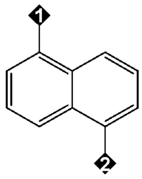 or 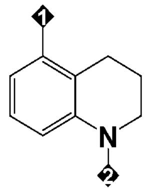.

In preferred embodiments, $R_4$ and $R_5$ are both methyl. In preferred embodiments, the compound is Compound 68.

The structures of the specific compounds mentioned above are as follows.

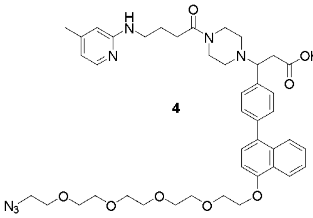
4

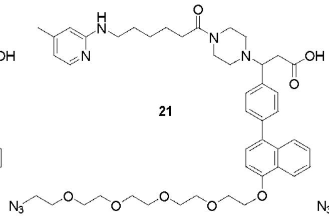
21

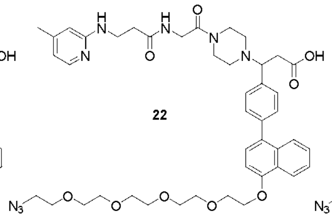
22

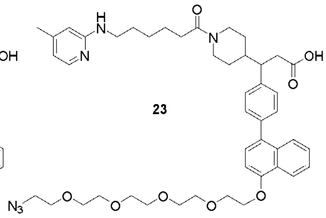
23

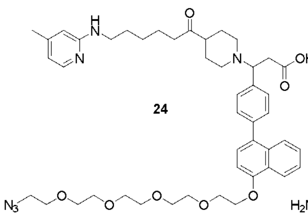
24

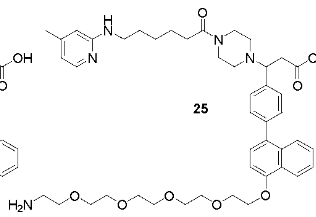
25

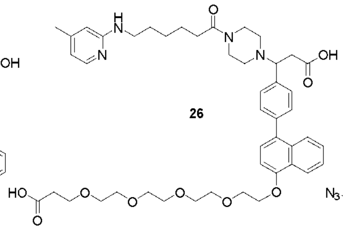
26

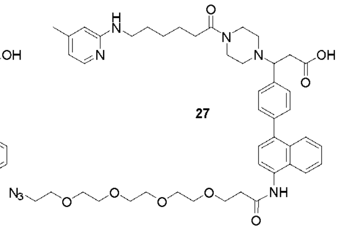
27

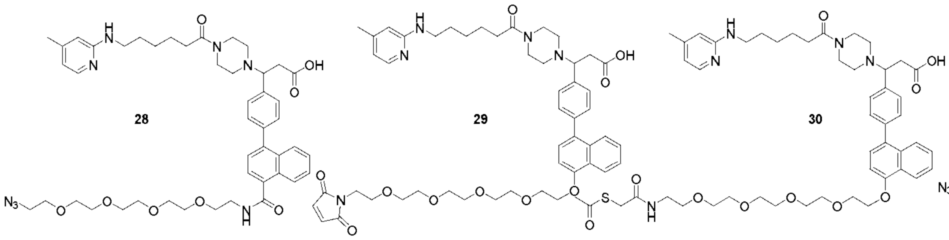
28

29

30

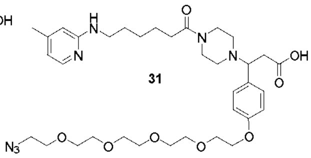
31

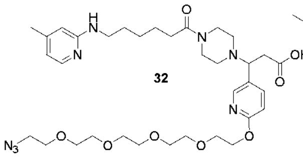
32

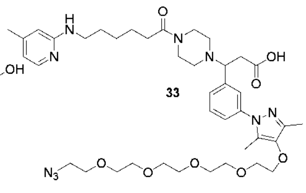
33

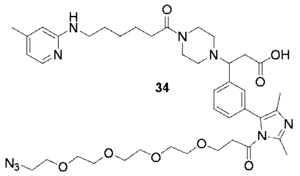
34

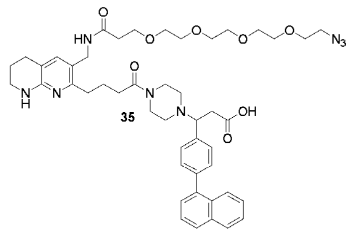
35

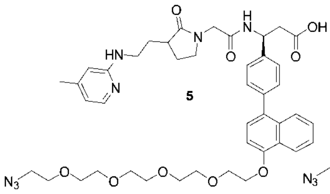
5

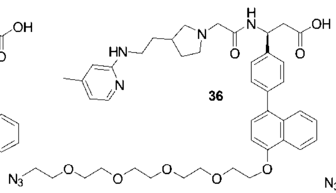
36

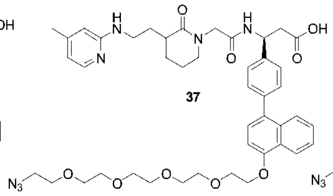
37

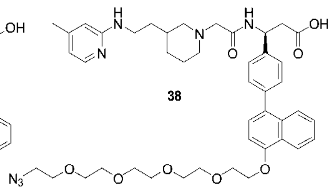
38

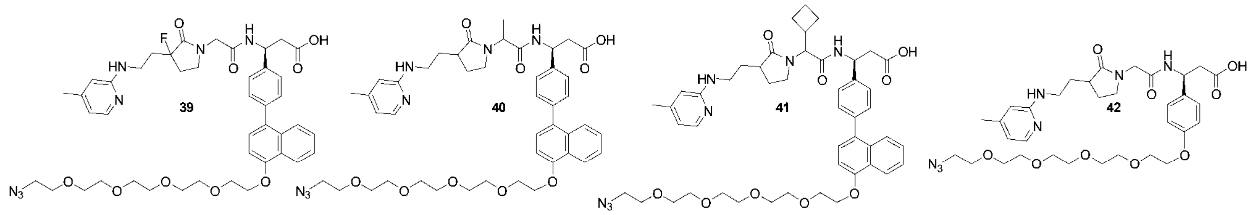

39 40 41 42

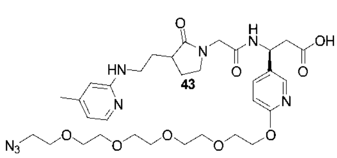

43

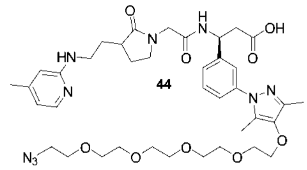

44

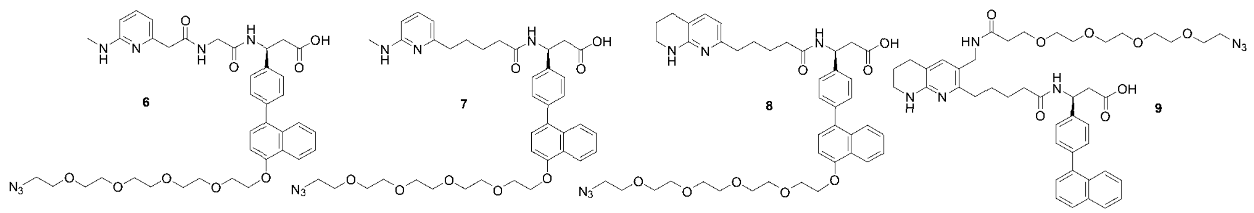

6 7 8 9

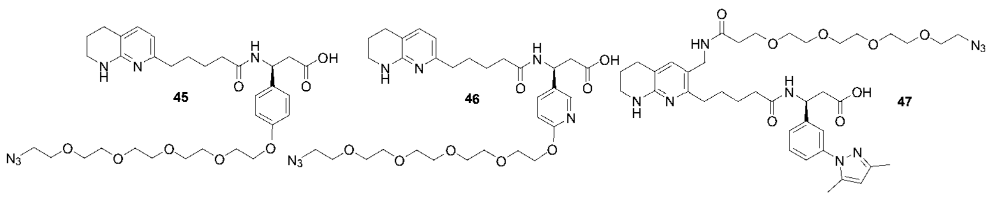

45 46 47

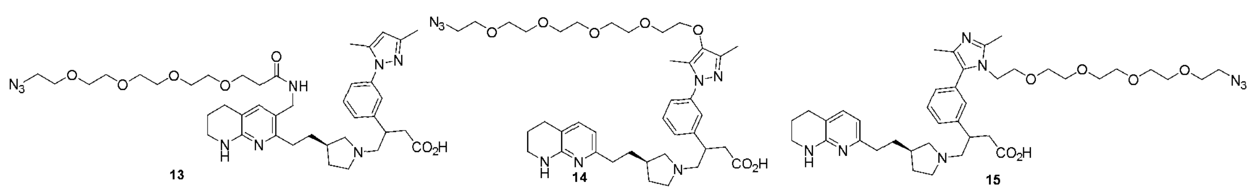

13 14 15

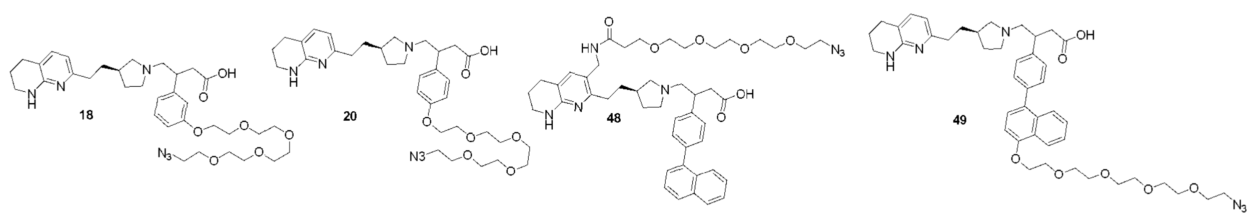

18 20 48 49

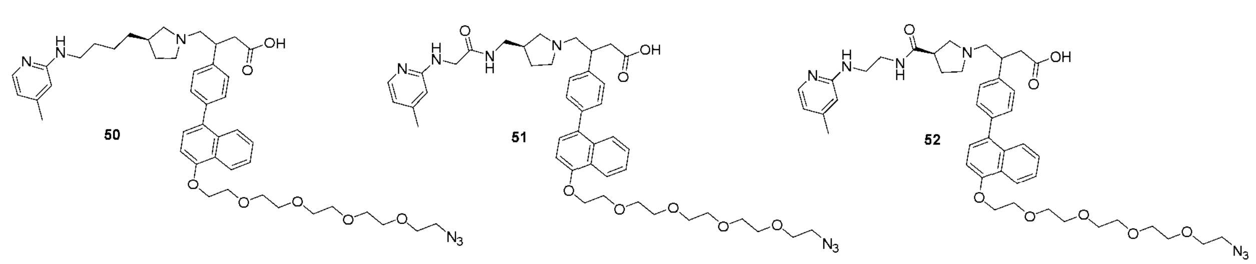

50 51 52

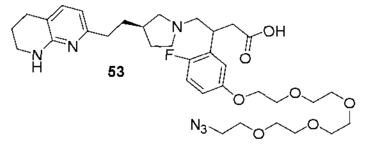

53

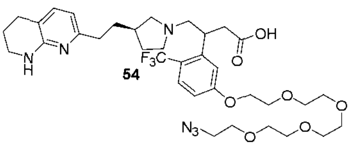

54

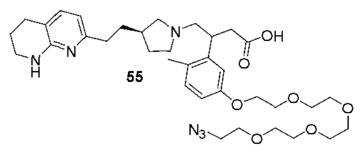

55

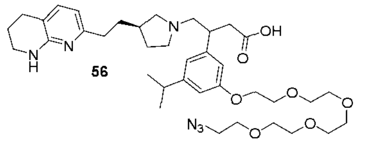

56

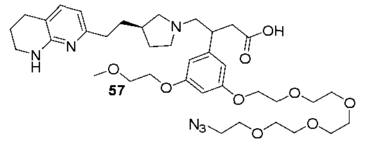

57

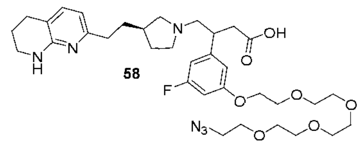

58

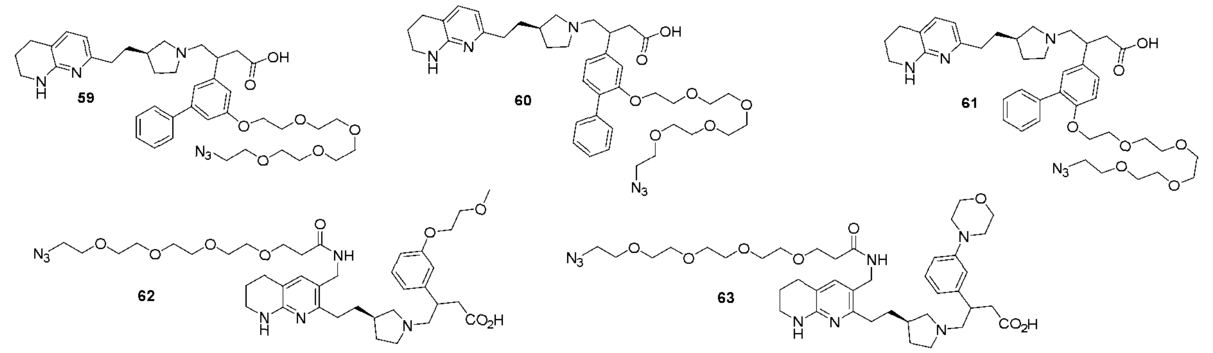

59 60 61

62 63

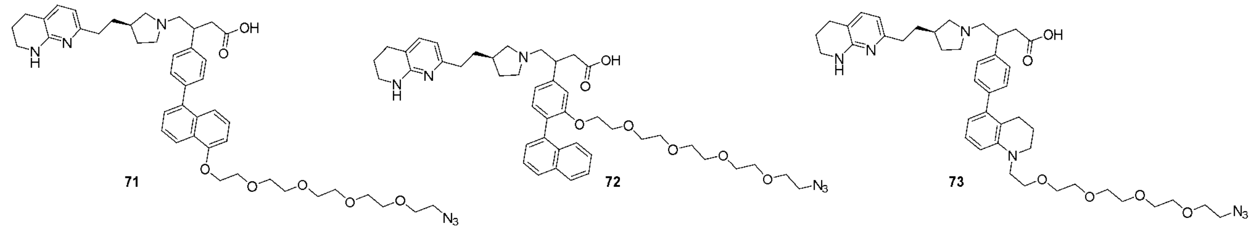

71 72 73

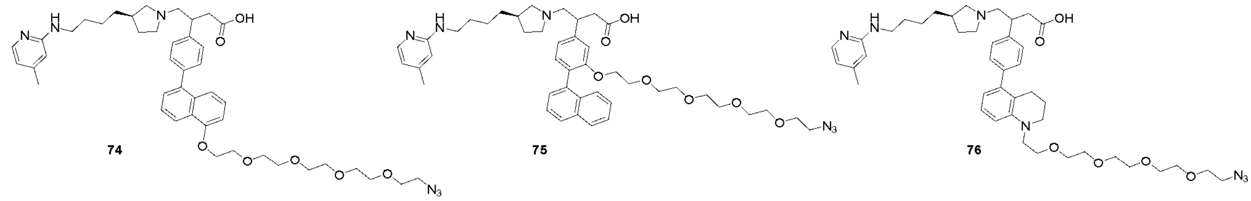

74 75 76

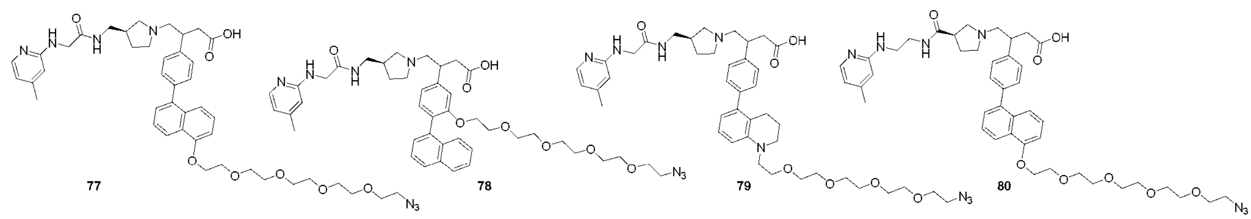

77 78 79 80

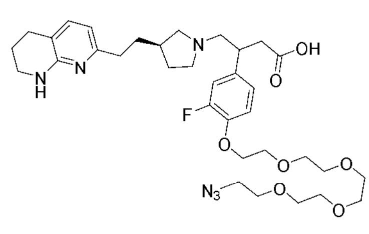

83

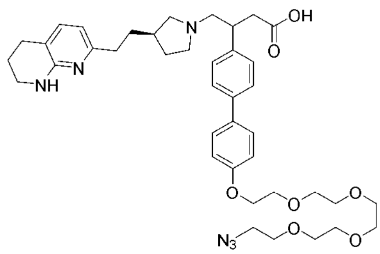

84

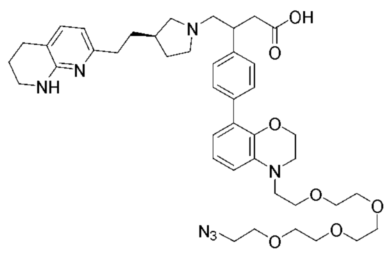

85

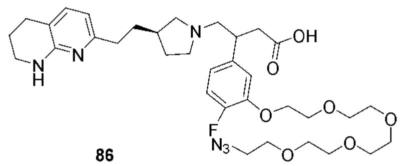

86

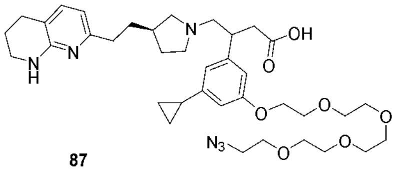

87

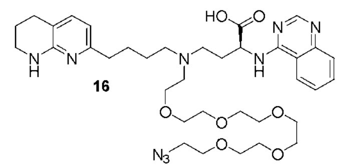

16

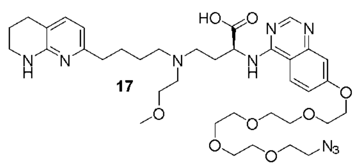

17

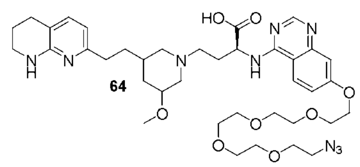

64

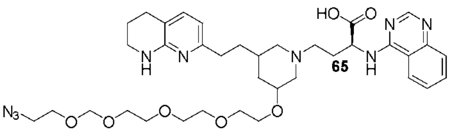

65

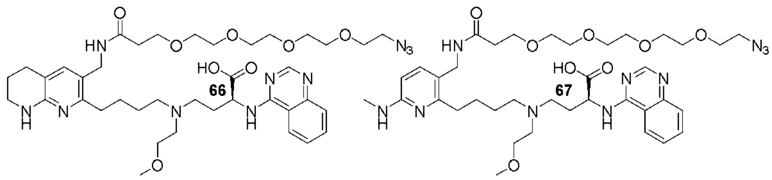

66

67

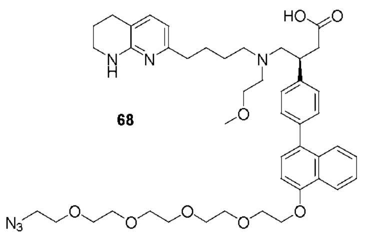

68

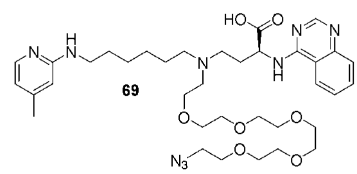

69

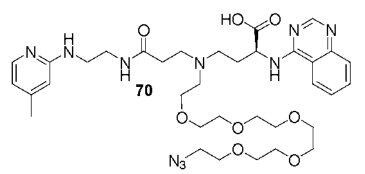

70

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ia':

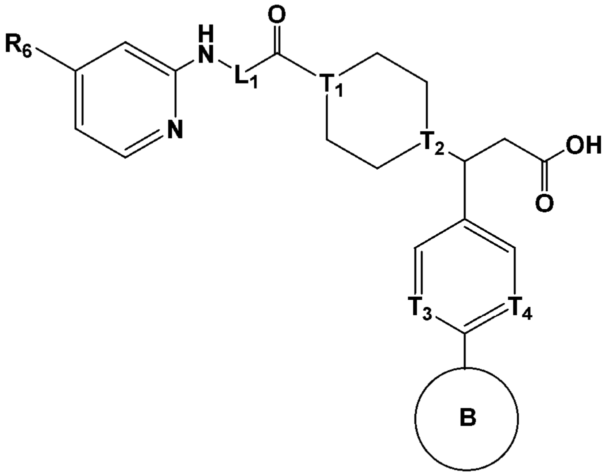

(Ia')

$T_1$ and $T_2$ are each independently C or N, and at least one is N;

$T_3$ and $T_4$ are each independently C or N;

ring B is

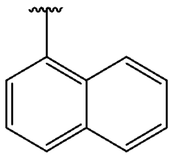,
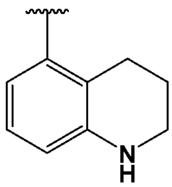,
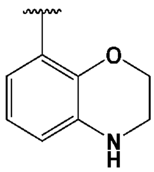,
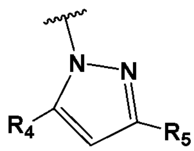,
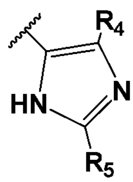;

or absent;

$R_4$, $R_5$ and $R_6$ are each independently H, methyl, ethyl, n-propyl, isopropyl, cyclopropyl, or cyclobutyl;

$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10.

In preferred embodiments, $T_1$ and $T_2$ are both N. In preferred embodiments, one of $T_3$ and $T_4$ is N. In preferred embodiments, $T_3$ and $T_4$ are both C.

In preferred embodiments, ring B is

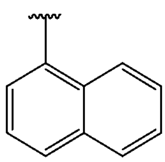,
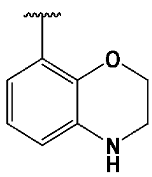, or
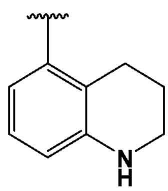.

In preferred embodiments, $R_4$, $R_5$ and $R_6$ are each independently methyl. In preferred embodiments, m and n are each independently 1, 2, 3, 4, 5, or 6. In preferred embodiments, $L_1$ is $-(CH_2)_n-$, n is 1, 2, 3, 4, 5, or 6. In preferred embodiments, the compound is selected from Compounds 4', 21', 22', 23', 24', 31', 32', 33', and 34'.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib':

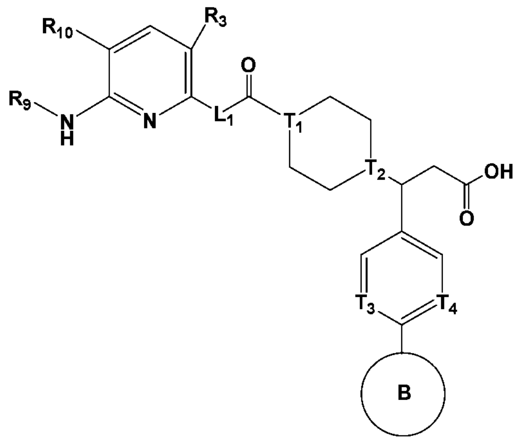

(Ib')

wherein,

$T_1$ and $T_2$ are each independently C or N, and at least one is N;
$T_3$ and $T_4$ are each independently C or N;
ring B is

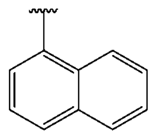, 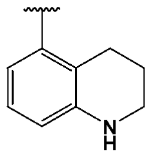, 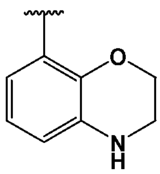, 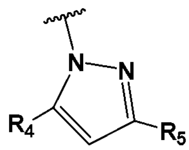, 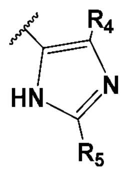,

or absent;
$R_4$ and $R_5$ are each independently H, methyl, ethyl, n-propyl, isopropyl, cyclopropyl, or cyclobutyl;
$R_3$ is H, methyl, ethyl, n-propyl, isopropyl, cyclopropyl, cyclobutyl, amino, or $C_{1-3}$ alkylamino; preferably $R_3$ is H or methylamino;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group.

In preferred embodiments, $T_1$ and $T_2$ are both N. In preferred embodiments, one of $T_3$ and $T_4$ is N. In preferred embodiments, $T_3$ and $T_4$ are both C.
In preferred embodiments, ring B is

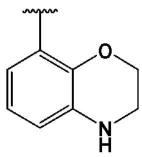, 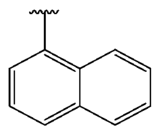, or 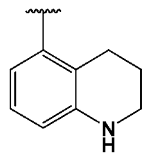.

In preferred embodiments, $R_4$, $R_5$ and $R_6$ are each independently methyl. In preferred embodiments, m and n are each independently 1, 2, 3, 4, 5, or 6. In preferred embodiments, $L_1$ is $-(CH_2)_n-$, n is 1, 2, 3, 4, 5, or 6. In preferred embodiments, $R_9$ is methyl and $R_{10}$ is H. In preferred embodiments, $R_9$, $R_{10}$, together with the C and N to which they are attached, form a piperidinyl group. In preferred embodiments, the compound is Compound 35'.
In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa' or IIb':

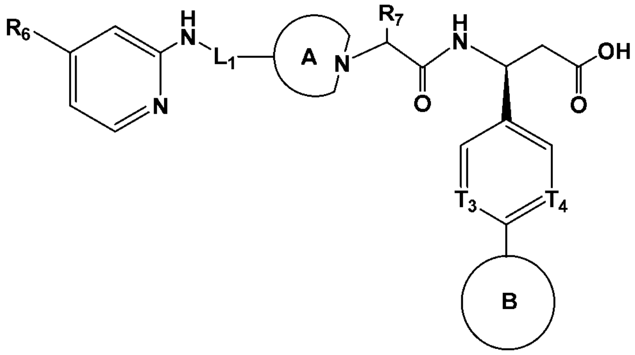

(IIa')

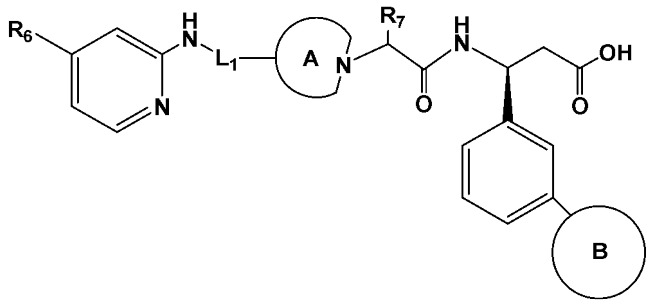

(IIb')

wherein,

$T_3$ and $T_4$ are each independently C or N;
ring A is

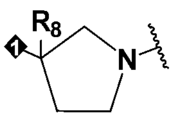, 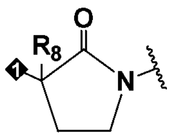, 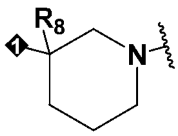, or 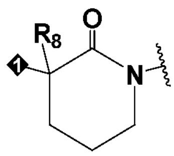,

wherein attachment point 1 is connected to $L_1$; $R_8$ is H, methyl, ethyl, n-propyl, isopropyl, cyclopropyl, cyclobutyl, or halogen;
ring B is

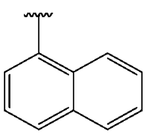, 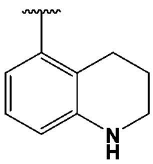, 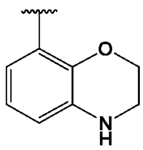, 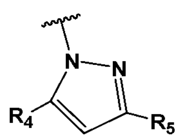, 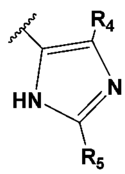,

or absent;
$R_4$, $R_5$, $R_6$, and $R_7$ are each independently H, methyl, ethyl, n-propyl, isopropyl, cyclopropyl, or cyclobutyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10.

In preferred embodiments, one of $T_3$ and $T_4$ is N. In preferred embodiments, $T_3$ and $T_4$ are both C. In preferred embodiments, $R_8$ is H or halogen, more preferably H or F. In preferred embodiments, ring A is

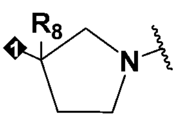

and $R_8$ is H. In preferred embodiments, ring B is

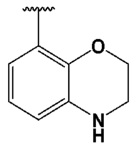

,

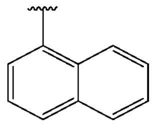

, or

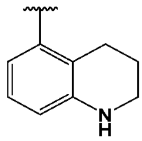

.

In preferred embodiments, $R_4$, $R_5$ and $R_6$ are each independently methyl. In preferred embodiments, $R_7$ is H. In preferred embodiments, m and n are each independently 1, 2, 3, 4, 5, or 6. In preferred embodiments, $L_1$ is $-(CH_2)_n-$, n is 1, 2, 3, 4, 5, or 6. In preferred embodiments, the compound is any one of Compounds 5', 36', 37', 38', 39', 40', 41', 42', 43', or 44'.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa' or IIIb':

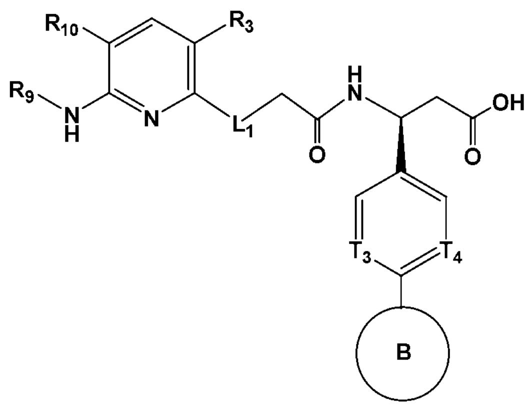

(IIIa')

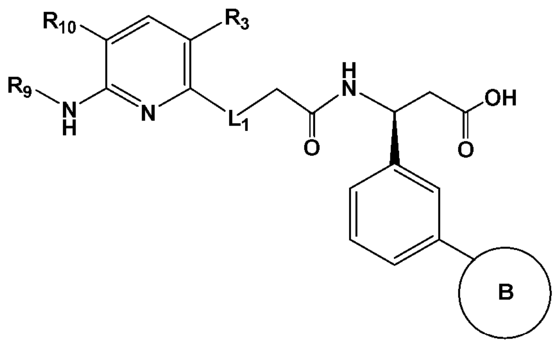

(IIIb')

wherein,

$T_3$ and $T_4$ are each independently C or N;
ring B is

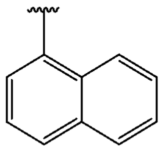, 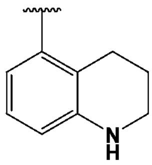, 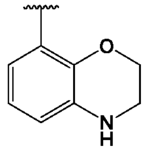, 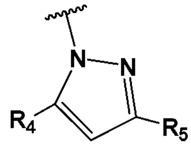, or 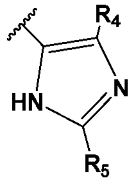;

$R_4$ and $R_5$ are each independently H, methyl, ethyl, n-propyl, isopropyl, cyclopropyl, or cyclobutyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10;
$R_3$ is H, methyl, ethyl, n-propyl, isopropyl, cyclopropyl, cyclobutyl, amino, or $C_{1-3}$ alkylamino;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group.

In preferred embodiments, one of $T_3$ and $T_4$ is N. In preferred embodiments, $T_3$ and $T_4$ are both C. In preferred embodiments, ring B is

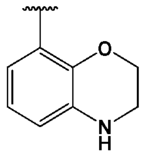, 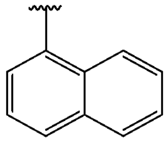, or 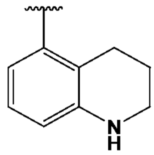.

In preferred embodiments, $R_4$ and $R_5$ are methyl. In preferred embodiments, m is 1, 2, 3, 4, 5, or 6. In preferred embodiments, n is 0, 1, 2, or 3. In preferred embodiments, $L_1$ is $-(CH_2)_m-$, m is 1, 2, 3, 4, 5, or 6. In preferred embodiments, $R_3$ is H or methylamino. In preferred embodiments, $R_9$ is methyl and $R_{10}$ is H. In preferred embodiments, $R_9$, $R_{10}$, together with the C and N to which they are attached, form a piperidinyl group. In preferred embodiments, the compound is Compound 6', 7', 8', 9', or 47'.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa':

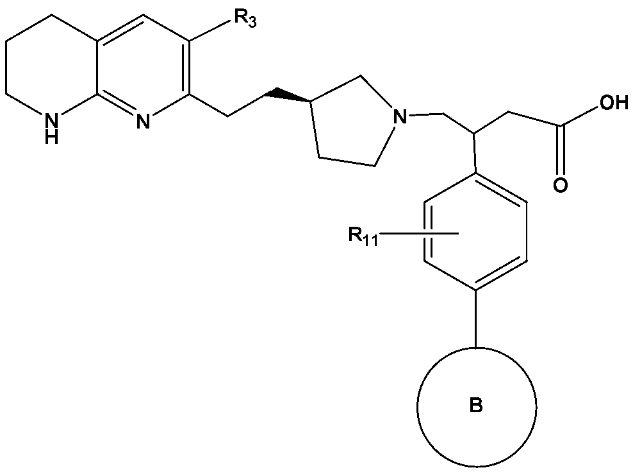

(IVa')

wherein,

ring B is

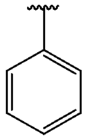, 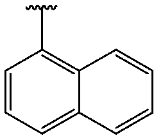, 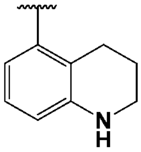, or 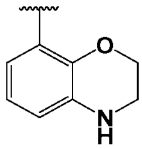;

$R_4$ and $R_5$ are each independently H, methyl, ethyl, n-propyl, isopropyl, cyclopropyl, or cyclobutyl;
$R_3$ is H, methyl, ethyl, n-propyl, isopropyl, cyclopropyl, cyclobutyl, amino, or $C_{1-3}$ alkylamino;
$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy.

In preferred embodiments, ring B is

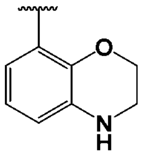, 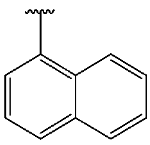, or 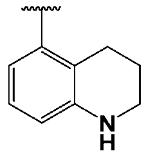.

In preferred embodiments, $R_4$ and $R_5$ are methyl. In preferred embodiments, $R_3$ is H or methylamino. In preferred embodiments, the compound is Compound 48', 49', 59', 60', 73', or 85'.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVb':

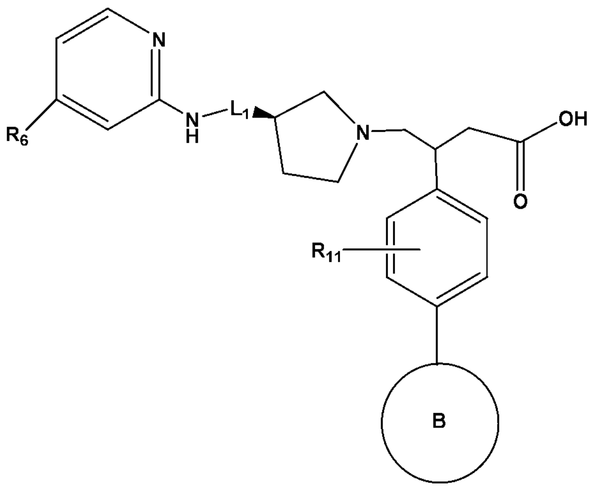

(IVb')

wherein

ring B is

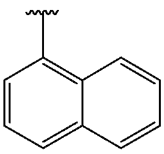, 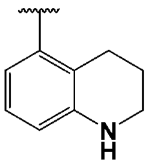, 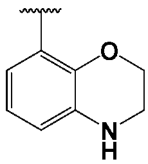, 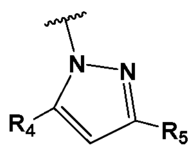, 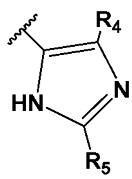,

or absent;
$R_4$, $R_5$ and $R_6$ are each independently H, methyl, ethyl, n-propyl, isopropyl, cyclopropyl, or cyclobutyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; n is an integer from 0

to 10;

$R_{11}$ is H, halogen, halomethyl, methyl, ethyl, n-propyl, isopropyl, cyclopropyl, cyclobutyl, phenyl, morpholinyl, methoxyethoxy, or ethoxymethoxy.

In preferred embodiments, ring B is

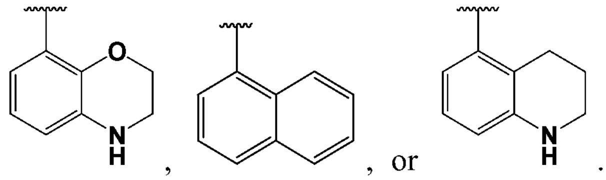

, , or .

In preferred embodiments, $R_4$, $R_5$ and $R_6$ are all methyl. In preferred embodiments, m is 1, 2, 3, 4, 5, or 6. In preferred embodiments, preferably n is 0, 1, 2, or 3. In preferred embodiments, $L_1$ is $-(CH_2)_m-$, m is 1, 2, 3, 4, 5, or 6. In preferred embodiments, $R_{11}$ is H. In preferred embodiments, the compound is Compound 50', 51', 52', 76', or 79'.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va':

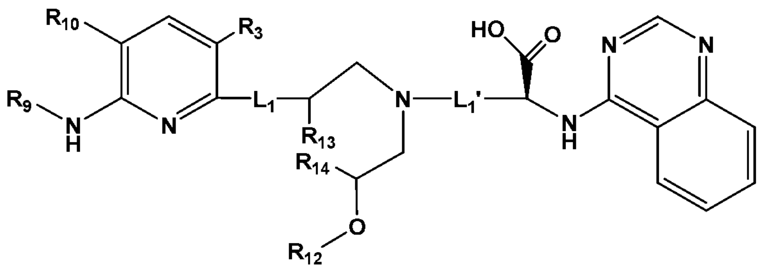

(Va')

wherein

$R_3$ is H, methyl, ethyl, n-propyl, isopropyl, cyclopropyl, cyclobutyl, amino, or $C_{1-3}$ alkylamino;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group;

$R_{12}$ is methyl, ethyl, n-propyl, isopropyl, cyclopropyl, or cyclobutyl;

$R_{13}$, $R_{14}$, together with the C and N atoms to which they are attached, form a piperidinyl group;

$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10.

In preferred embodiments, $R_3$ is H or methylamino. In preferred embodiments, $R_9$ is methyl and $R_{10}$ is H. In preferred embodiments, $R_{12}$ is methyl. In preferred embodiments, m is 1, 2, 3, 4, 5, or 6. In preferred embodiments, preferably n is 0, 1, 2, or 3. In preferred embodiments, $L_1$ is $-(CH_2)_m-$, m is 1, 2, 3, 4, 5, or 6. In preferred embodiments, the compound is Compound 64'.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb':

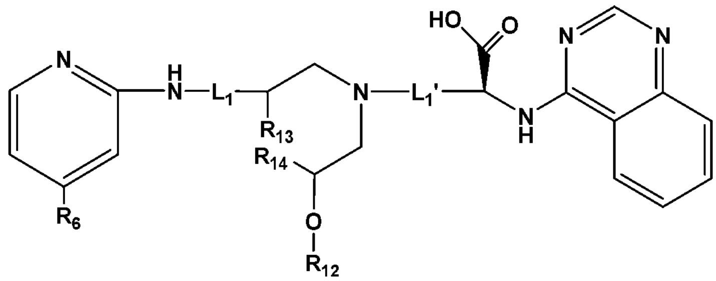

(Vb')

wherein,

$R_6$ is H, methyl, ethyl, n-propyl, isopropyl, cyclopropyl, or cyclobutyl;
$R_{12}$ is methyl, ethyl, n-propyl, isopropyl, cyclopropyl, or cyclobutyl;
$R_{13}$ and $R_{14}$ are each H, or $R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10.

In preferred embodiments, $R_6$ is methyl. In preferred embodiments, $R_{12}$ is methyl. In preferred embodiments, m is 1, 2, 3, 4, 5, or 6. In preferred embodiments, preferably n is 0, 1, 2, or 3. In preferred embodiments, $L_1$ is $-(CH_2)_m-$, m is 1, 2, 3, 4, 5, or 6. In preferred embodiments, the compound is Compound 69' or 70'.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc':

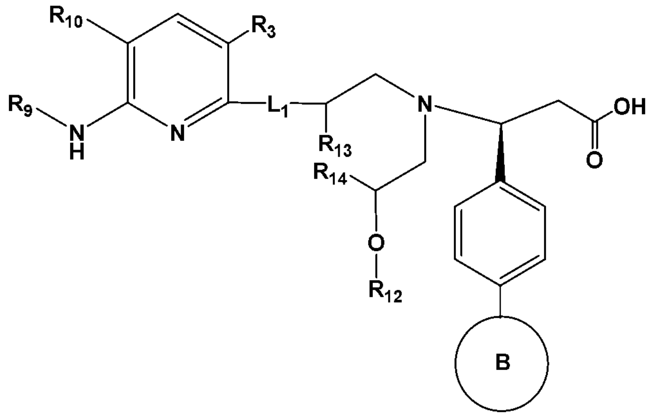

(Vc')

$R_3$ is H, methyl, ethyl, n-propyl, isopropyl, cyclopropyl, cyclobutyl, amino, or $C_{1-3}$ alkylamino;
$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group;
$R_{12}$ is methyl, ethyl, n-propyl, isopropyl, cyclopropyl, or cyclobutyl;
$R_{13}$ and $R_{14}$ are each H, or $R_{13}$, $R_{14}$, together with the C and N atoms to which they are attached, form a piperidinyl group;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10;
ring B is

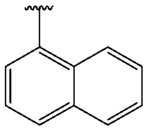, 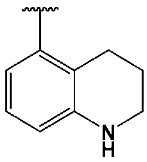, 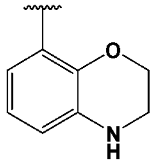, 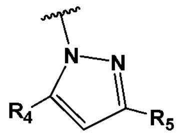, 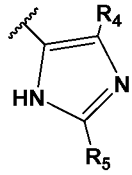,

or absent;

$R_4$ and $R_5$ are each independently H, methyl, ethyl, n-propyl, isopropyl, cyclopropyl, or cyclobutyl.

In preferred embodiments, $R_3$ is H or methylamino. In preferred embodiments, $R_9$ is methyl and $R_{10}$ is H. In preferred embodiments, $R_{12}$ is methyl. In preferred embodiments, m is 1, 2, 3, 4, 5, or 6. In preferred embodiments, preferably n is 0, 1, 2, or 3. In preferred embodiments, $L_1$ is $-(CH_2)_m-$, m is 1, 2, 3, 4, 5, or 6. In preferred embodiments, ring B is

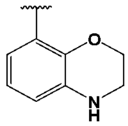, 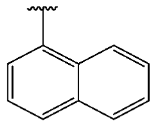, or 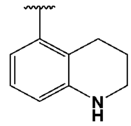.

In preferred embodiments, $R_4$ and $R_5$ are both methyl. In preferred embodiments, the compound is Compound 68'.

In specific embodiments, the compounds provided by the present invention are selected from:

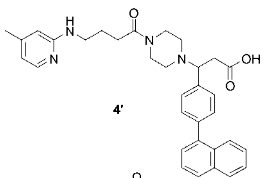
4'

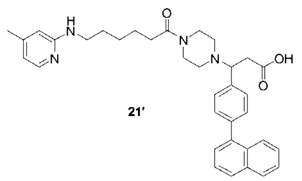
21'

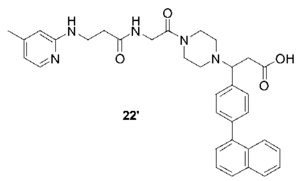
22'

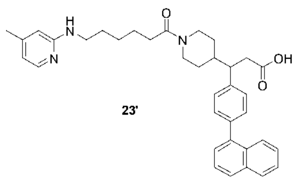
23'

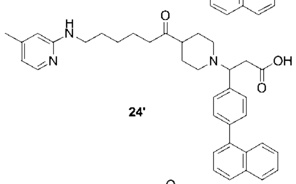
24'

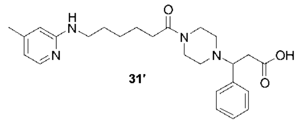
31'

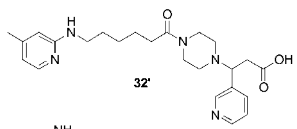
32'

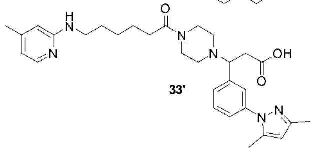
33'

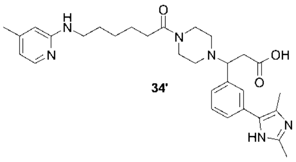
34'

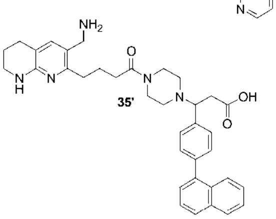
35'

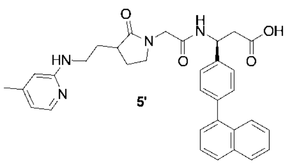
5'

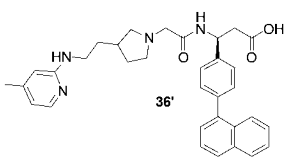
36'

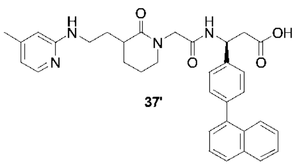
37'

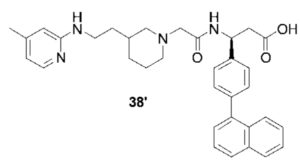
38'

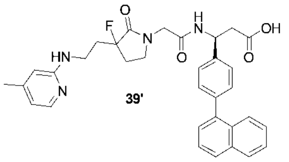
39'

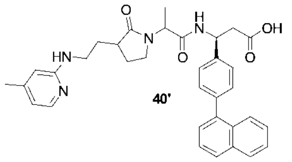
40'

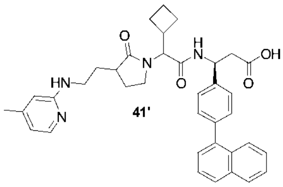
41'

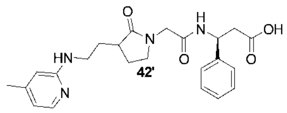
42'

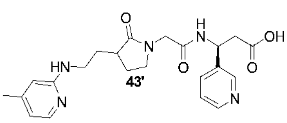
43'

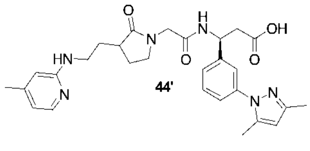
44'

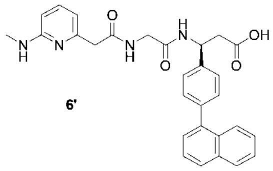
6'

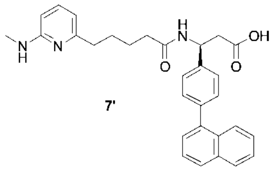
7'

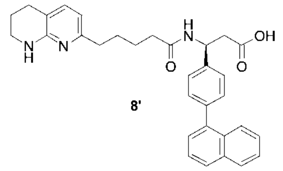
8'

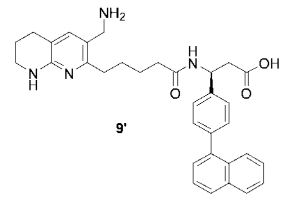
9'

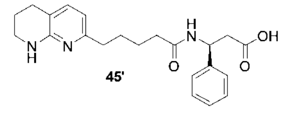
45'

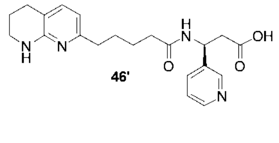
46'

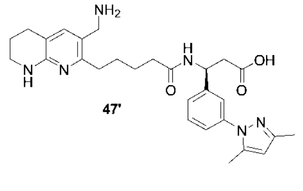
47'

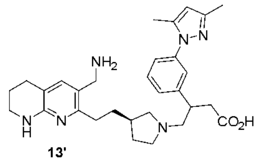
13'

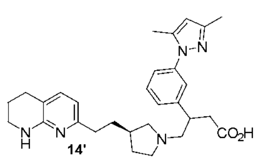
14'

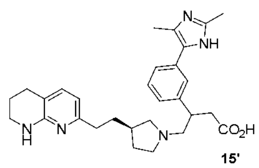
15'

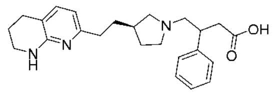
18'

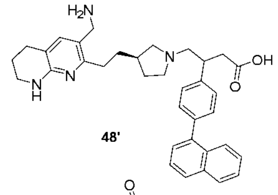
48'

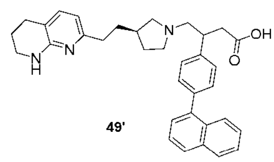
49'

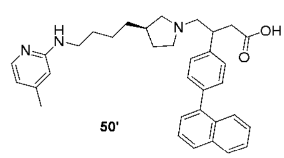
50'

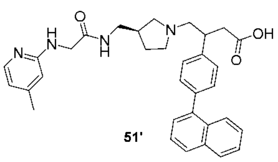
51'

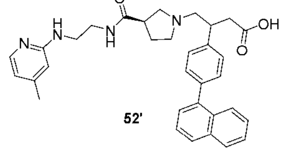
52'

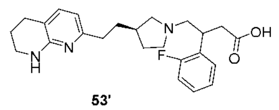
53'

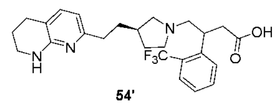
54'

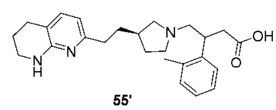
55'

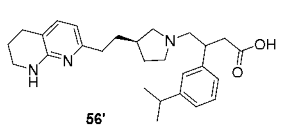
56'

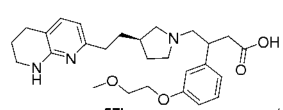
57'

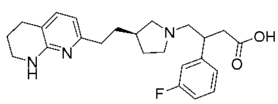
58'

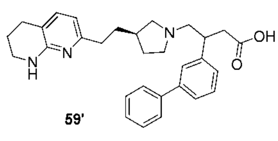
59'

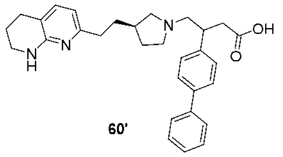
60'

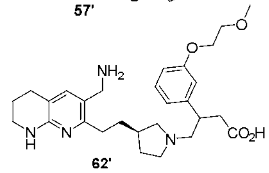
62'

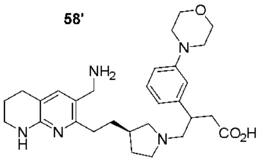
63'

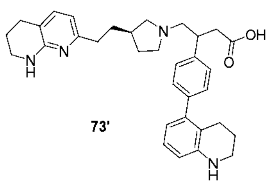
73'

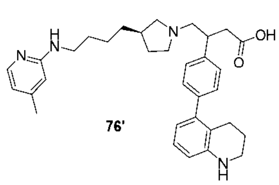
76'

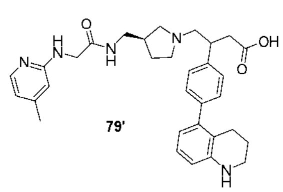
79'

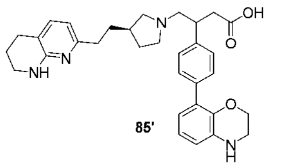
85'

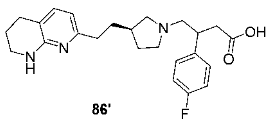
86'

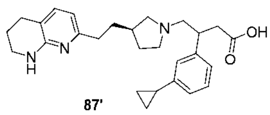
87'

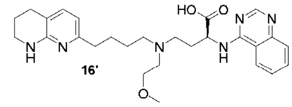
16'

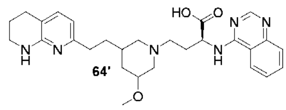
64'

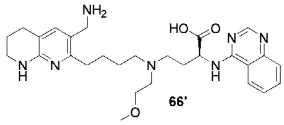
66'

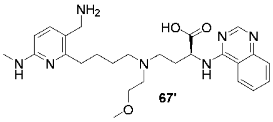
67'

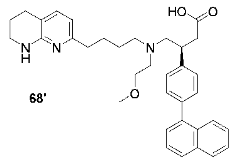
68'

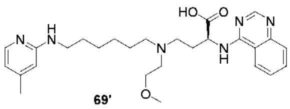
69'

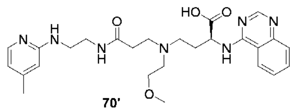
70'

.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ia":

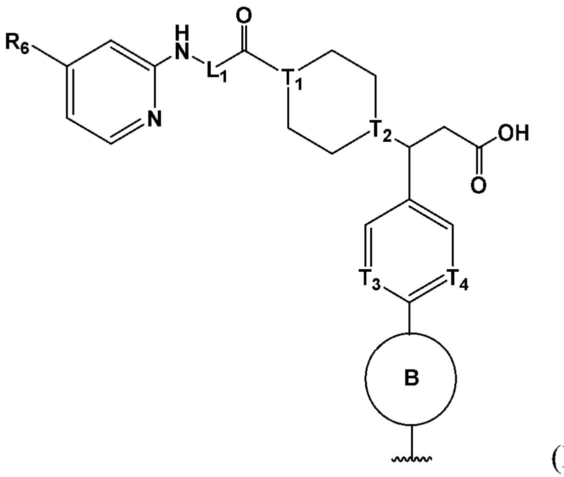
(Ia")

$T_1$ and $T_2$ are each independently C or N, and at least one is N;
$T_3$ and $T_4$ are each independently C or N;
ring B is

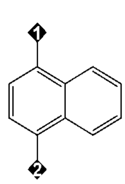
,
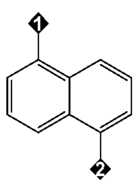
,
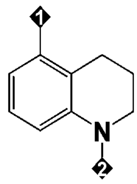
,
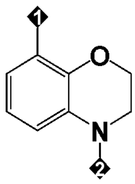
,
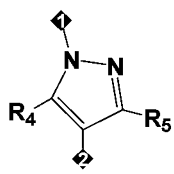
,
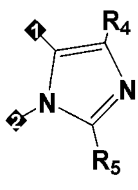
,

or absent, wherein attachment point 2 is the wave line in Formula Ia", and the wave line ~~~~ indicates a bond;
$R_4$, $R_5$ and $R_6$ are each independently H, methyl, ethyl, n-propyl, isopropyl, cyclopropyl, or cyclobutyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10.

In preferred embodiments, $T_1$ and $T_2$ are both N. In preferred embodiments, one of $T_3$ and $T_4$ is N. In preferred embodiments, $T_3$ and $T_4$ are both C.

In preferred embodiments, ring B is

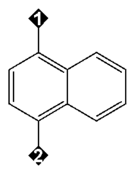 , 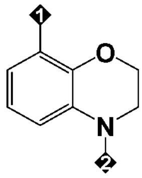 , 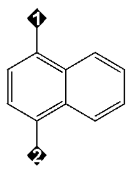 , or 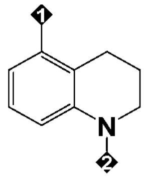 .

In preferred embodiments, $R_4$, $R_5$ and $R_6$ are each independently methyl. In preferred embodiments, m and n are each independently 1, 2, 3, 4, 5, or 6. In preferred embodiments, $L_1$ is $-(CH_2)_n-$, n is 1, 2, 3, 4, 5, or 6.

In preferred embodiments, the compound has a structure as shown below:

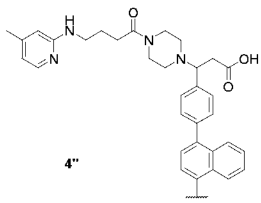
4''

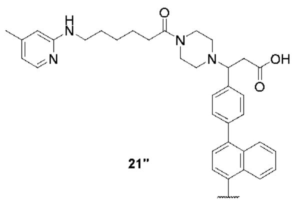
21''

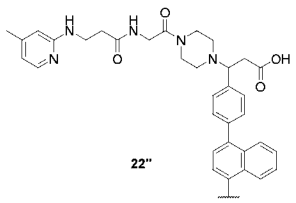
22''

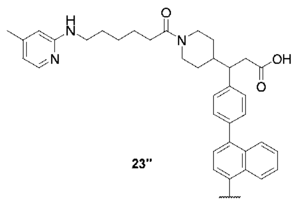
23''

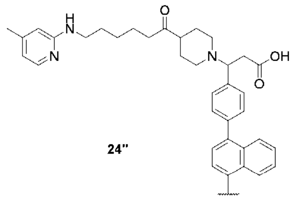
24''

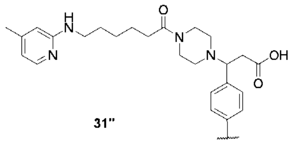
31''

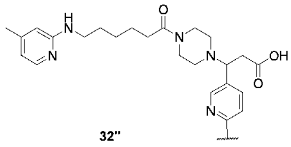
32''

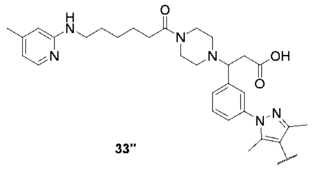
33''

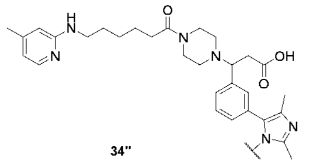
34''

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib":

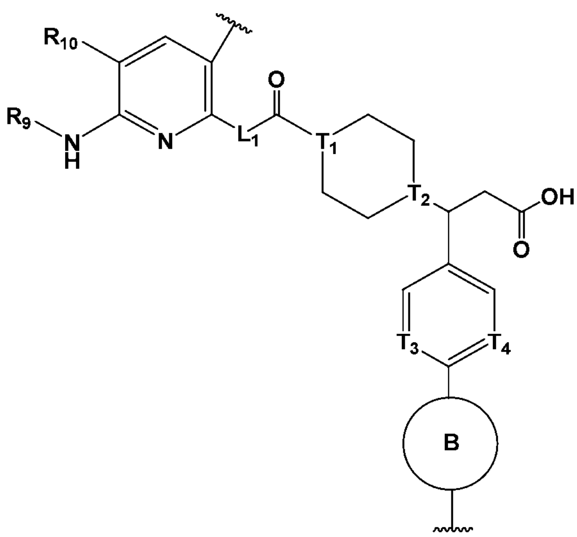 (Ib")

wherein,

$T_1$ and $T_2$ are each independently C or N, and at least one is N;
$T_3$ and $T_4$ are each independently C or N;
ring B is

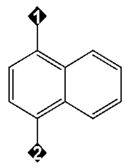 , 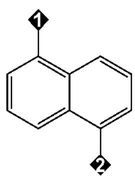 , 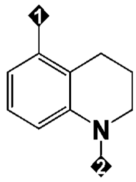 , 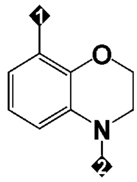 , 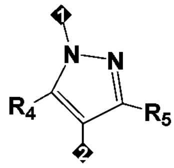 , 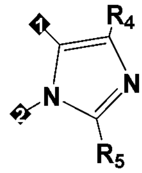 ,

or absent, wherein attachment point 2 is the wave line connected to ring B in Formula Ib";
$R_4$ and $R_5$ are each independently H, methyl, ethyl, n-propyl, isopropyl, cyclopropyl, or cyclobutyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; and
the wave line in Formula Ib" indicates a bond, and one of the two wave lines is absent.

In preferred embodiments, $T_1$ and $T_2$ are both N. In preferred embodiments, one of $T_3$ and $T_4$ is N. In preferred embodiments, $T_3$ and $T_4$ are both C.

In preferred embodiments, ring B is

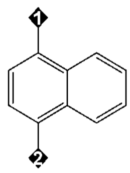 , 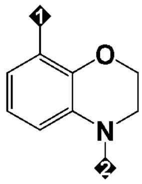 , 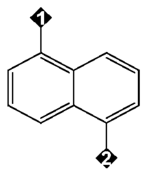 , or 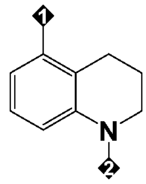 .

In preferred embodiments, $R_4$ and $R_5$ are each independently methyl. In preferred embodiments, m and n are each independently 1, 2, 3, 4, 5, or 6. In preferred embodiments, $L_1$ is $-(CH_2)_n-$, n is 1, 2, 3, 4, 5, or 6. In preferred embodiments, $R_9$ is methyl and $R_{10}$ is H. In preferred embodiments, $R_9$, $R_{10}$, together with the C and N to which they are attached, form a piperidinyl group.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib"-1:

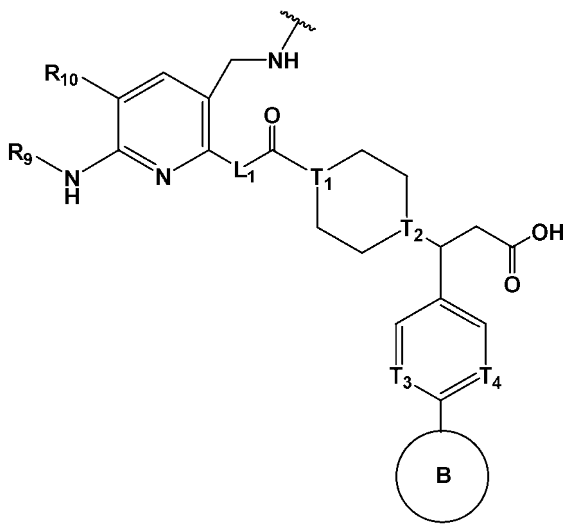

(Ib"-1)

wherein $T_1$, $T_2$, $T_3$, $T_4$, $L_1$, $R_9$, $R_{10}$, and the wave line have the same definitions and preferred embodiments as the corresponding substituents in Formula Ib",
ring B is

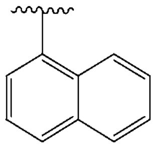, 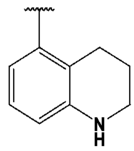, 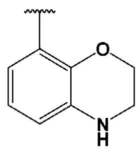, 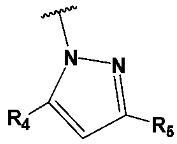, 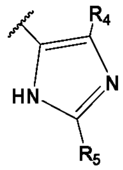,

or absent, wherein $R_4$ and $R_5$ are each independently H, methyl, ethyl, n-propyl, isopropyl, cyclopropyl, or cyclobutyl; preferably $R_4$ and $R_5$ are each independently methyl.

In preferred embodiments, the compound has a structure as shown below:

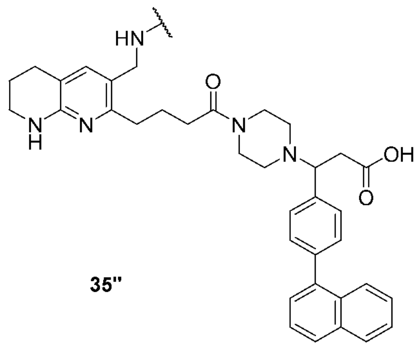

35"

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa" or IIb":

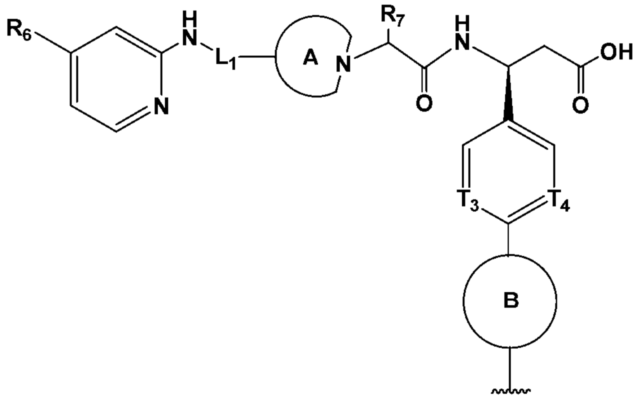

(IIa")

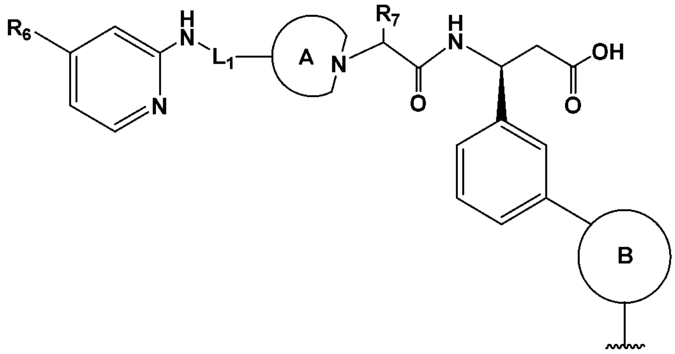

(IIb")

wherein,

$T_3$ and $T_4$ are each independently C or N;
ring A is

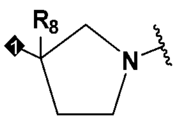, 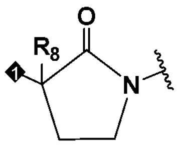, 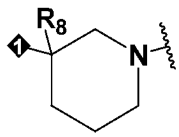, or 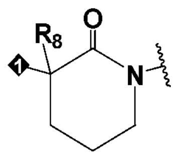,

wherein attachment point 1 is connected to $L_1$; $R_8$ is H, methyl, ethyl, n-propyl, isopropyl, cyclopropyl, cyclobutyl, or halogen;
ring B is

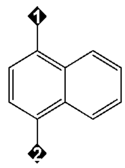, 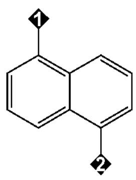, 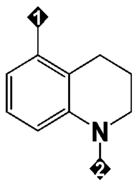, 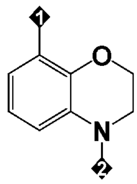, 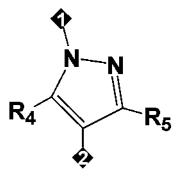, 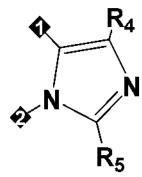,

or absent, wherein attachment point 2 is the wave line in Formula IIa" or IIb", and the wave line indicates a bond;
$R_4$, $R_5$, $R_6$, and $R_7$ are each independently H, methyl, ethyl, n-propyl, isopropyl, cyclopropyl, or cyclobutyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10.

In preferred embodiments, one of $T_3$ and $T_4$ is N. In preferred embodiments, $T_3$ and $T_4$ are both C. In preferred embodiments, $R_8$ is H or halogen, more preferably H or F. In preferred embodiments, ring A is

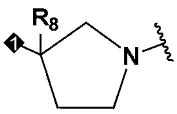

and $R_8$ is H. In preferred embodiments, ring B is

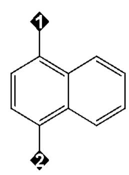,

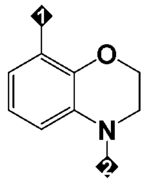, 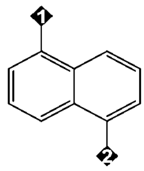, or 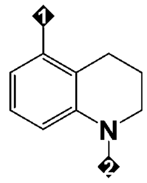.

In preferred embodiments, $R_4$, $R_5$ and $R_6$ are each independently methyl. In preferred embodiments, $R_7$ is H. In preferred embodiments, m and n are each independently 1, 2, 3, 4, 5, or 6. In preferred embodiments, $L_1$ is $-(CH_2)_n-$, n is 1, 2, 3, 4, 5, or 6.

In preferred embodiments, the compound has a structure as shown below:

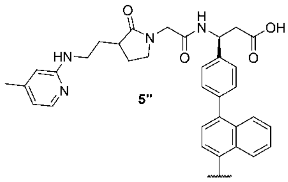
5''

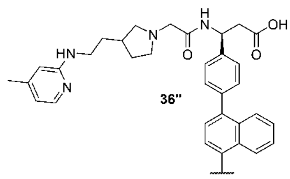
36''

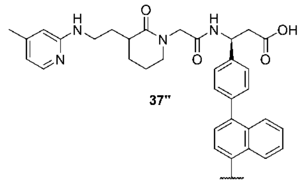
37''

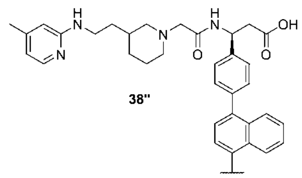
38''

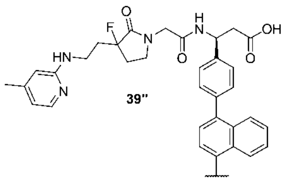
39''

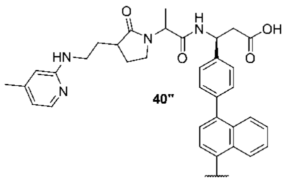
40''

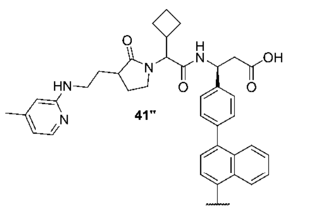
41''

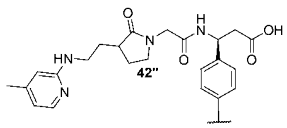
42''

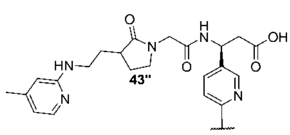
43''

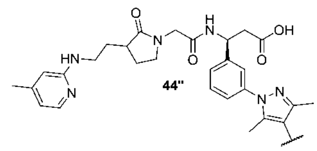
44''

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa" or IIIb":

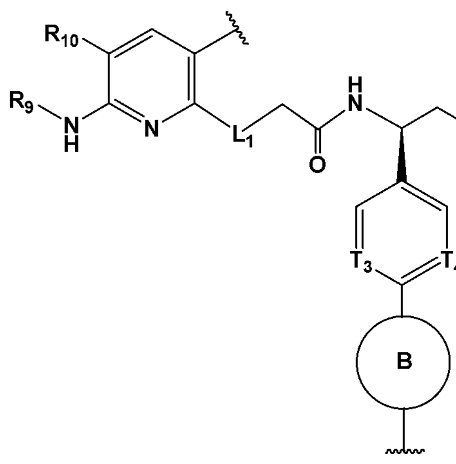
(IIIa")

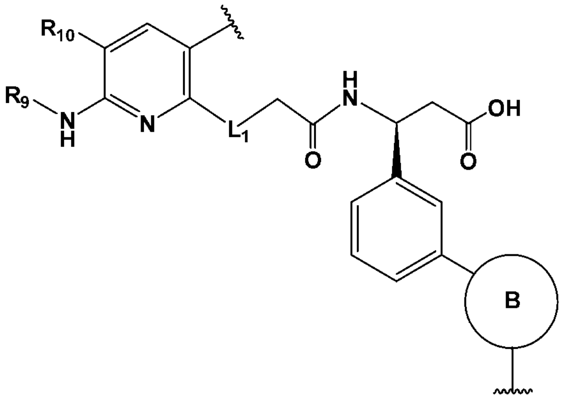

(IIIb")

wherein,

$T_3$ and $T_4$ are each independently C or N;
ring B is

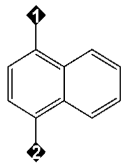 , 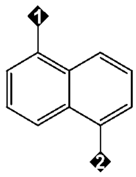 , 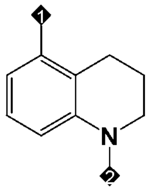 , 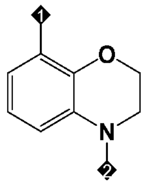 , 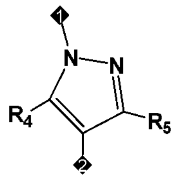 , 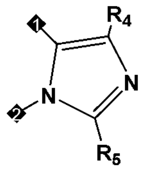 ,

or absent, wherein attachment point 2 is the wave line connected to ring B in Formula IIIa" or IIIb";
$R_4$ and $R_5$ are each independently H, methyl, ethyl, n-propyl, isopropyl, cyclopropyl, or cyclobutyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; and
the wave line in Formula IIIa" and Formula IIIb" indicates a bond, and one of the two wave lines is absent.

In preferred embodiments, one of $T_3$ and $T_4$ is N. In preferred embodiments, $T_3$ and $T_4$ are both C. In preferred embodiments, ring B is

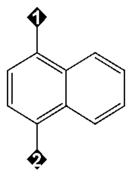 , 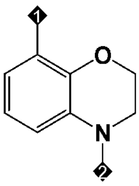 , 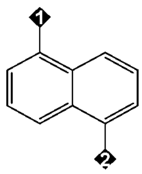 , or 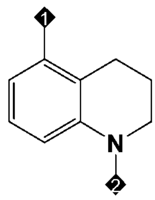 .

In preferred embodiments, $R_4$ and $R_5$ are each independently methyl. In preferred embodiments, m is 1, 2, 3, 4, 5, or 6. In preferred embodiments, n is 0, 1, 2, or 3. In preferred embodiments, $L_1$ is $-(CH_2)_m-$, m is 1, 2, 3, 4, 5, or 6. In preferred embodiments, $R_9$ is methyl and $R_{10}$ is H. In preferred embodiments, $R_9$, $R_{10}$, together with the C and N to which they are attached, form a piperidinyl group.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa"-1 or IIIb"-1:

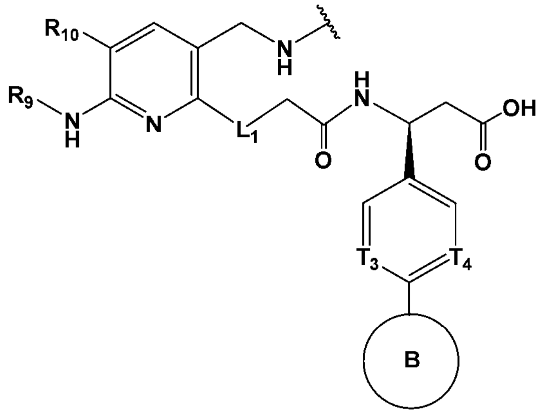

(IIIa"-1)

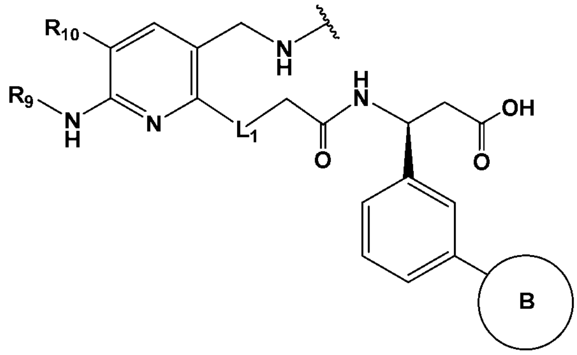

(IIIb"-1)

wherein $T_3$, $T_4$, $L_1$, $R_9$, $R_{10}$, and the wave line have the same definitions and preferred embodiments as the corresponding substituents in Formula IIIa" or IIIb",

ring B is

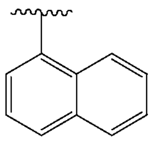 , 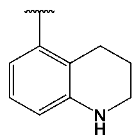 , 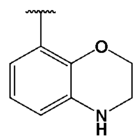 , 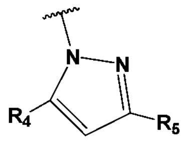 , 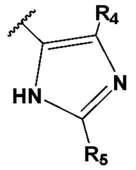 ,

or absent, wherein $R_4$ and $R_5$ are each independently H, methyl, ethyl, n-propyl, isopropyl, cyclopropyl, or cyclobutyl; preferably $R_4$ and $R_5$ are each independently methyl.

In preferred embodiments, the compound has a structure as shown below:

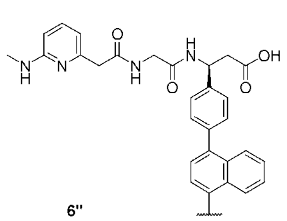

6''

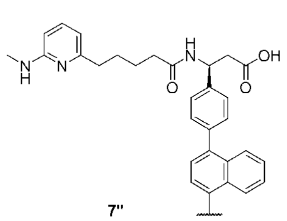

7''

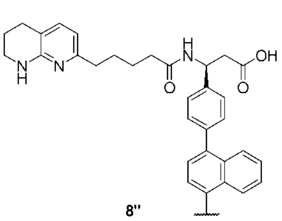

8''

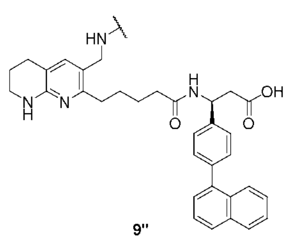

9''

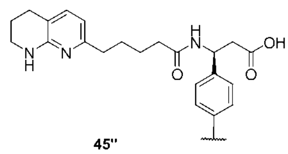
45''

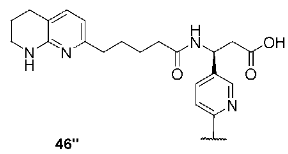
46''

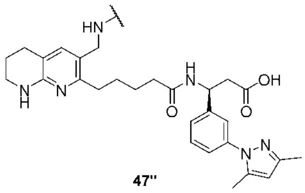
47''

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa":

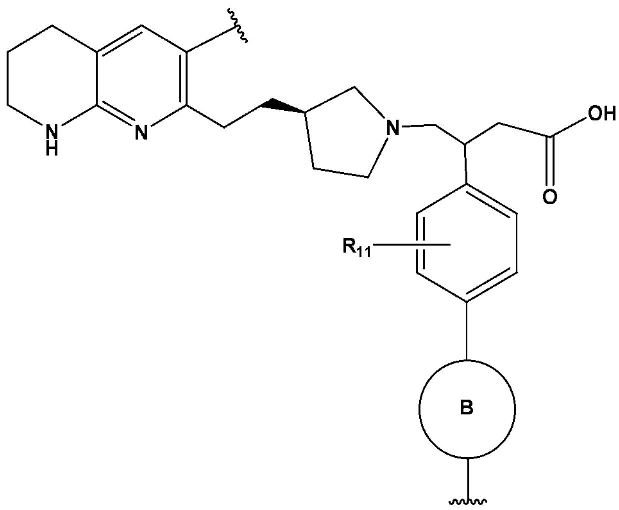
(IVa")

wherein,

ring B is

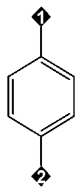
,
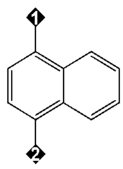
,
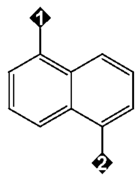
,
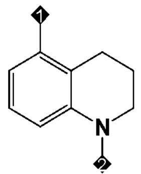
,
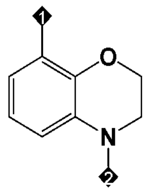
,
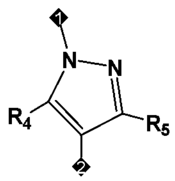
,
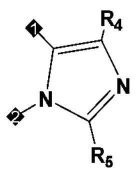
,

or absent, wherein attachment point 2 is the wave line connected to ring B in Formula IVa";

$R_4$ and $R_5$ are each independently H, methyl, ethyl, n-propyl, isopropyl, cyclopropyl, or cyclobutyl;

$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy; and

the wave line in Formula IVa" indicates a bond, and one of the two wave lines is absent.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa"-1:

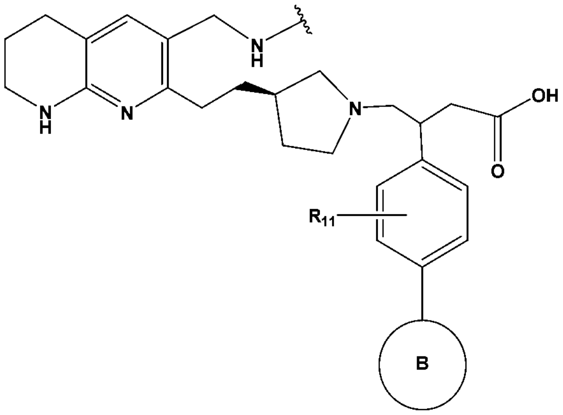

(IVa"-1)

wherein $R_{11}$ and the wave line have the same definitions and preferred embodiments as $R_{11}$ and the wave line in Formula IVa",
ring B is

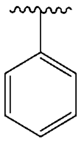 , 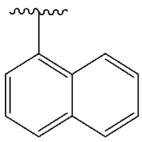 , 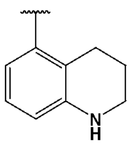 , 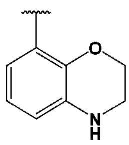 , 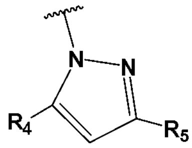 , 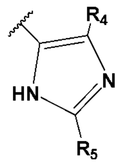 ,

or absent, wherein $R_4$ and $R_5$ are each independently H, methyl, ethyl, n-propyl, isopropyl, cyclopropyl, or cyclobutyl; preferably $R_4$ and $R_5$ are each independently methyl.

In preferred embodiments, the compound has a structure as shown below:

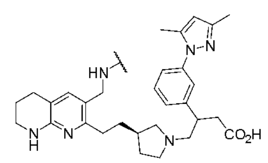

13''

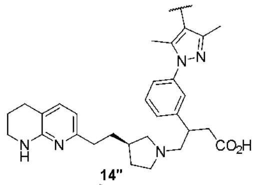

14''

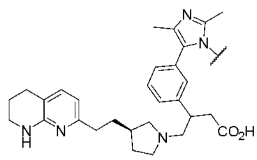

15''

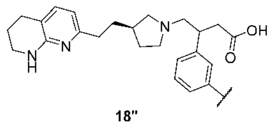

18''

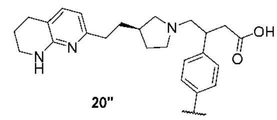

20''

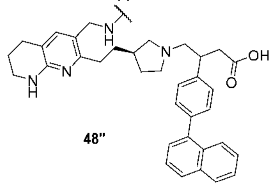

48''

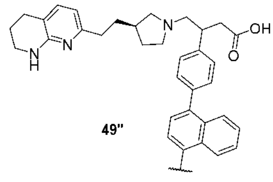

49''

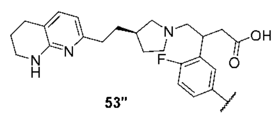

53''

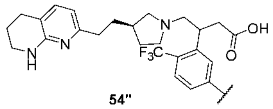

54''

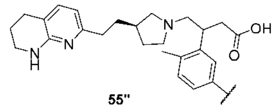

55''

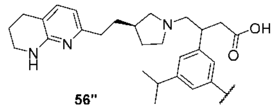

56''

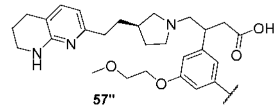

57''

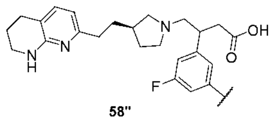

58''

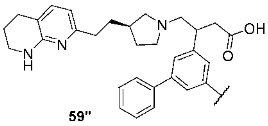

59''

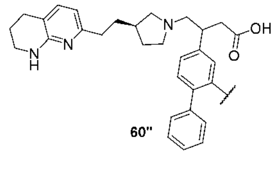

60''

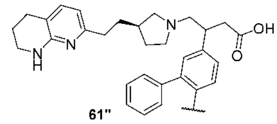

61''

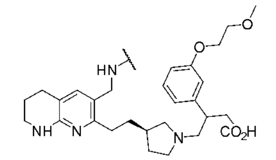
62"

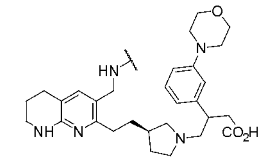
63"

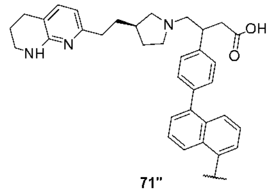
71"

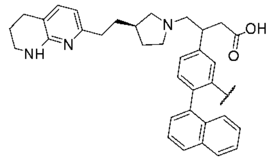
72"

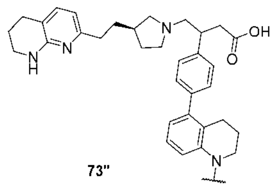
73"

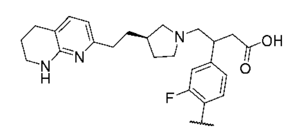
83"

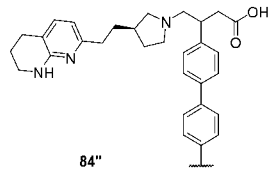
84"

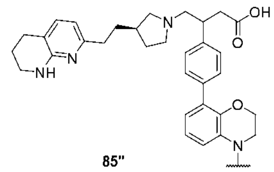
85"

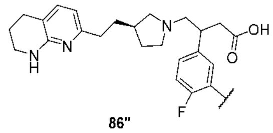
86"

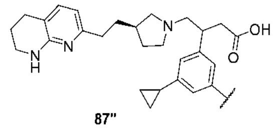
87"

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVb":

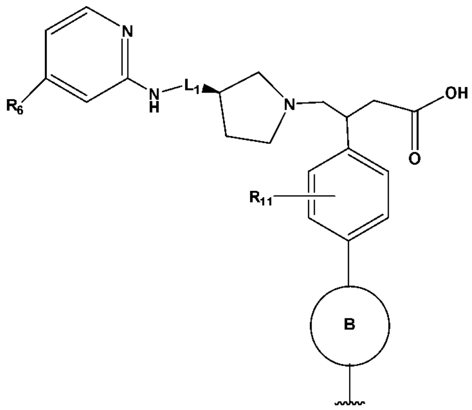
(IVb")

ring B is

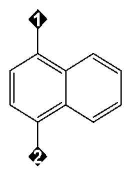 , 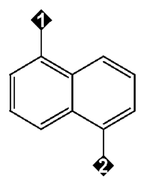 , 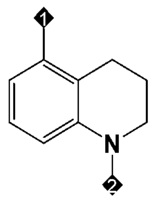 , 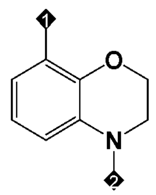 , 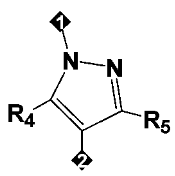 , 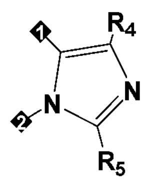 ,

or absent, wherein attachment point 2 is the wave line in Formula IVb", and the wave line indicates a bond;

$R_4$, $R_5$ and $R_6$ are each independently H, methyl, ethyl, n-propyl, isopropyl, cyclopropyl, or cyclobutyl;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10;

$R_{11}$ is H, halogen, halomethyl, methyl, ethyl, n-propyl, isopropyl, cyclopropyl, cyclobutyl, phenyl, morpholinyl, methoxyethoxy, or ethoxymethoxy.

In preferred embodiments, ring B is

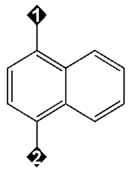, 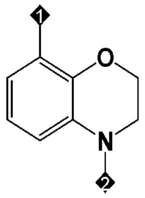, 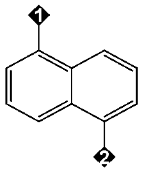, or 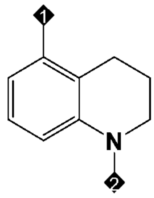.

In preferred embodiments, $R_4$, $R_5$ and $R_6$ are all methyl. In preferred embodiments, m is 1, 2, 3, 4, 5, or 6. In preferred embodiments, n is 0, 1, 2, or 3. In preferred embodiments, $L_1$ is $-(CH_2)_m-$, m is 1, 2, 3, 4, 5, or 6. In preferred embodiments, $R_{11}$ is H.

In preferred embodiments, the compound has a structure as shown below:

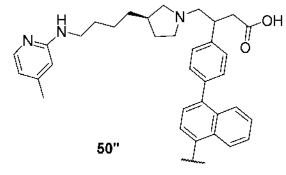
50''

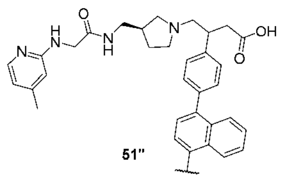
51''

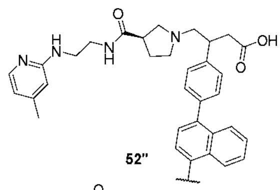
52''

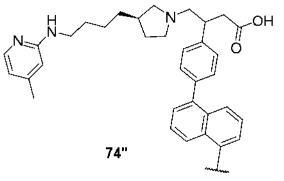
74''

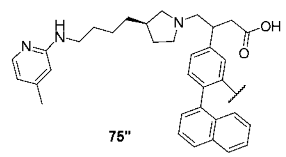
75''

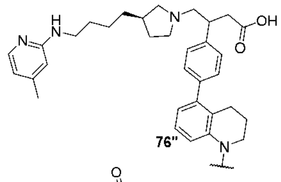
76''

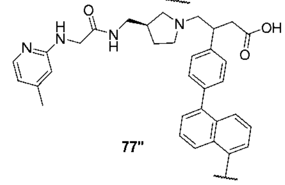
77''

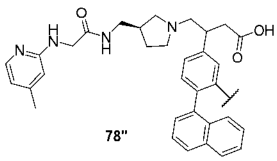
78''

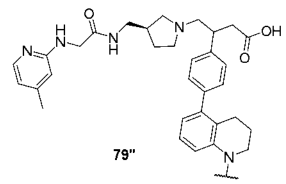
79''

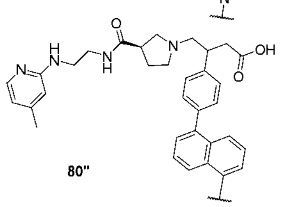
80''

.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va'':

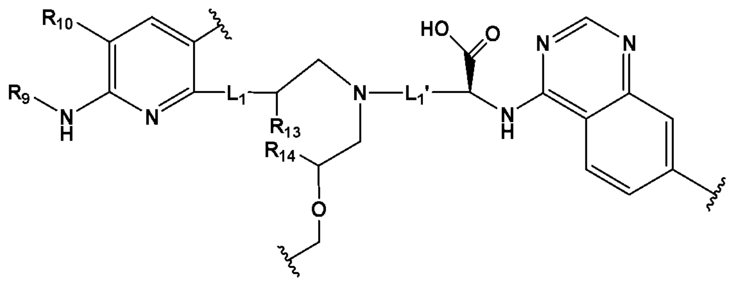

(Va'')

wherein

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group;

$R_{13}$ and $R_{14}$ are each H, or $R_{13}$, $R_{14}$, together with the C and N atoms to which they are attached, form a piperidinyl group;

$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10; and

the wave line in Formula Va'' indicates a bond, and two of the three wave lines are absent.

In preferred embodiments, $R_9$ is methyl and $R_{10}$ is H. In preferred embodiments, m is 1, 2, 3, 4, 5, or 6. In preferred

embodiments, n is 0, 1, 2, or 3. In preferred embodiments, $L_1$ and $L_1$' are each independently $-(CH_2)_m-$, m is 1, 2, 3, 4, 5, or 6.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va"-1, Va"-2, or Va"-3:

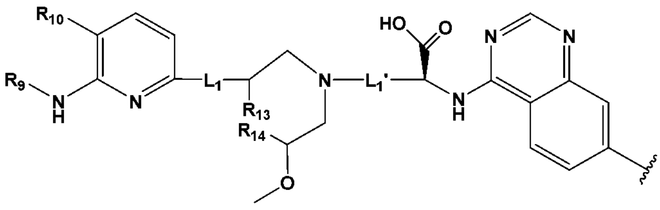

(Va"-1)

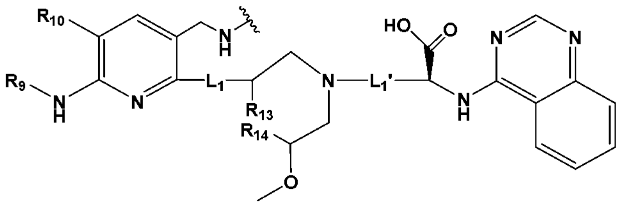

(Va"-2)

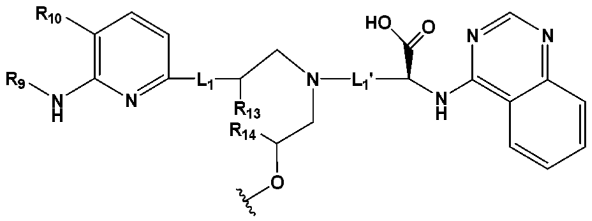

(Va"-3)

wherein, $R_9$, $R_{10}$, $R_{13}$, $R_{14}$, $L_1$, $L_1$', and the wave line have the same definitions and preferred embodiments as the corresponding substituents in Formula Va".

In preferred embodiments, the compound has a structure as shown below:

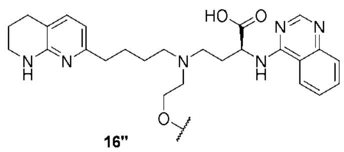

16"

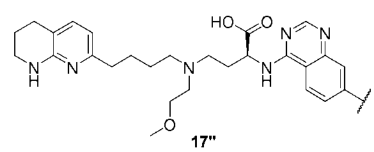

17"

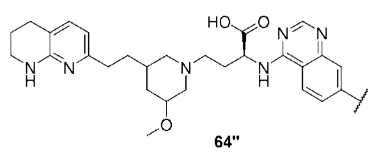

64"

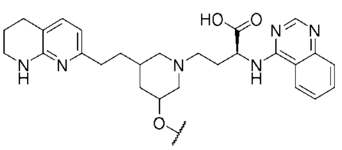

65"

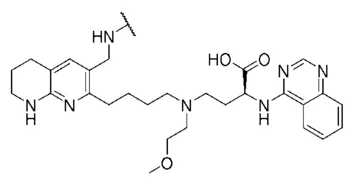

66"

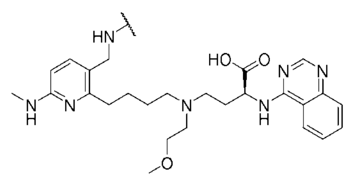

67"

.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb":

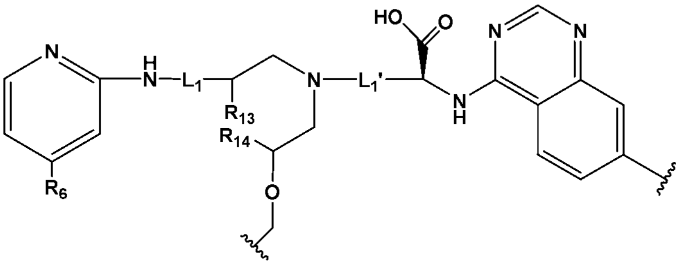

(Vb")

wherein,

- $R_6$ is H, methyl, ethyl, n-propyl, isopropyl, cyclopropyl, or cyclobutyl;
- $R_{13}$ and $R_{14}$ are each H, or $R_{13}$, $R_{14}$, together with the C and N atoms to which they are attached, form a piperidinyl group;
- $L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10; and
- the wave line in Formula Vb" indicates a bond, and one of the two wave lines is absent.

In preferred embodiments, $R_6$ is methyl. In preferred embodiments, m is 1, 2, 3, 4, 5, or 6. In preferred embodiments, n is 0, 1, 2, or 3. In preferred embodiments, $L_1$ and $L_1$' are each independently $-(CH_2)_m-$, m is 1, 2, 3, 4, 5, or 6.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb"-1 or Vb"-2:

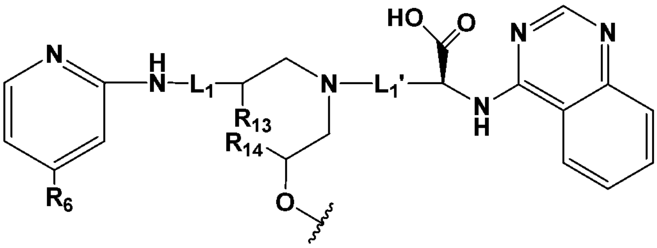

(Vb"-1)

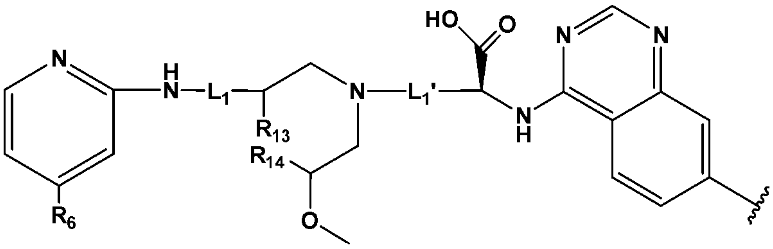

(Vb"-2)

wherein $R_6$, $R_{13}$, $R_{14}$, $L_1$, $L_1$', and the wave line have the same definitions and preferred embodiments as the corresponding substituents in Formula Vb".

In preferred embodiments, the compound has a structure as shown below:

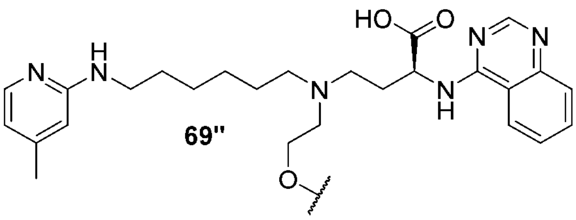

69"

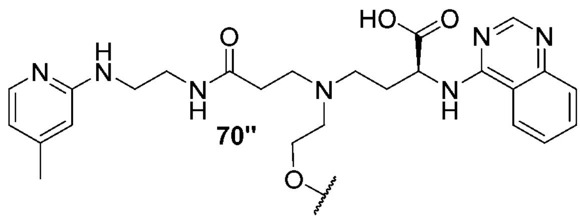

70"

.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc":

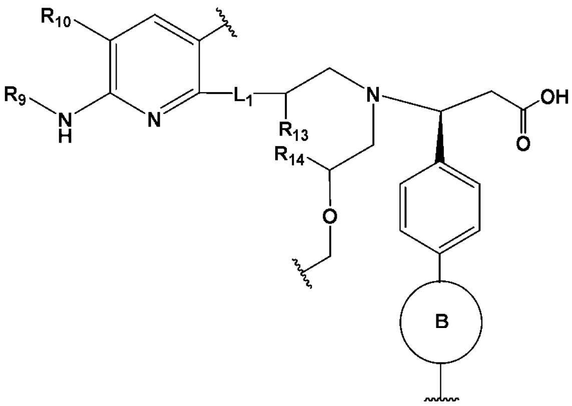

(Vc")

wherein

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group;
$R_{13}$ and $R_{14}$ are each H, or $R_{13}$, $R_{14}$, together with the C and N atoms to which they are attached, form a piperidinyl group;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; n is an integer from 0 to 10;
ring B is

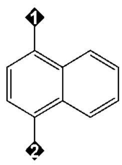 , 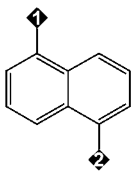 , 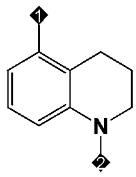 , 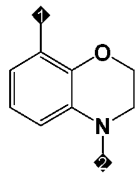 , 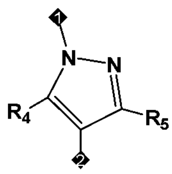 , 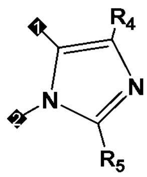 ,

or absent, wherein attachment point 2 is the wave line connected to ring B in Formula Vc";
$R_4$ and $R_5$ are each independently H, methyl, ethyl, n-propyl, isopropyl, cyclopropyl, or cyclobutyl; and
the wave line ~~~~ in Formula Vc" indicates a bond, and two of the three wave lines are absent.

In preferred embodiments, $R_9$ is methyl and $R_{10}$ is H, or $R_9$, $R_{10}$, together with the C and N atoms to which they are attached, form a piperidinyl group. In preferred embodiments, m is 1, 2, 3, 4, 5, or 6. In preferred embodiments, n is 0, 1, 2, or 3. In preferred embodiments, $L_1$ is $-(CH_2)_m-$, m is 1, 2, 3, 4, 5, or 6. In preferred embodiments, ring B is

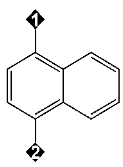 , 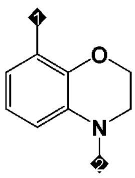 , 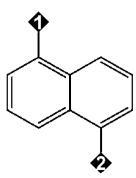 ,

or

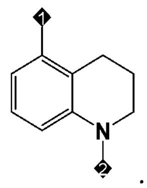

In preferred embodiments, $R_4$ and $R_5$ are both methyl.

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc"-1:

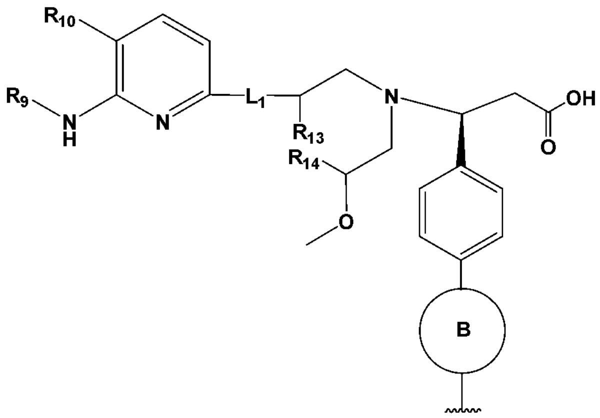

(Vc"-1)

wherein $R_9$, $R_{10}$, $R_{13}$, $R_{14}$, $L_1$, ring B, and the wave line have the same definitions and preferred embodiments as the corresponding substituents in Formula Vc".

In some embodiments, the present invention provides a compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc"-2 or Vc"-3:

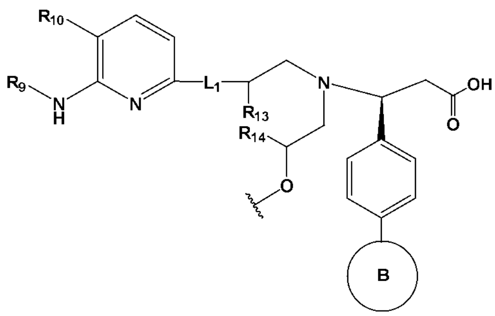

(Vc"-2)

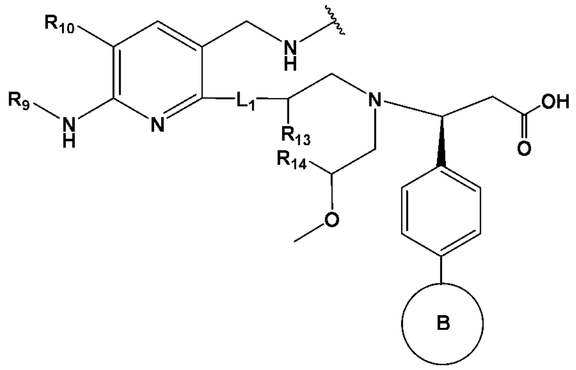

(Vc"-3)

wherein $R_9$, $R_{10}$, $R_{13}$, $R_{14}$, $L_1$, and the wave line have the same definitions and preferred embodiments as the corresponding substituents in Formula Vc", and
ring B is

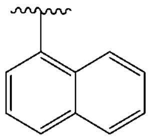 , 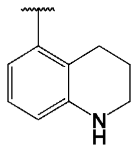 , 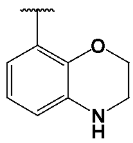 , 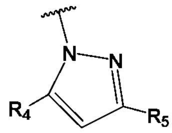 , 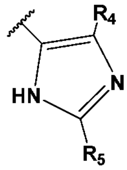 ,

or absent, wherein $R_4$ and $R_5$ are each independently H, methyl, ethyl, n-propyl, isopropyl, cyclopropyl, or cyclobutyl; preferably $R_4$ and $R_5$ are each independently methyl.

In preferred embodiments, the compound has a structure as shown below:

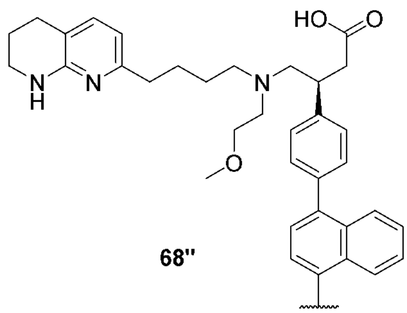

68''

RNAi Agents

Another aspect of the present invention provides an RNAi agent, or a pharmaceutically acceptable salt, prodrug, or solvate thereof, wherein the RNAi agent has a structure represented by Formula VI, VIa, VIb, VIc, VId, VIe, VIf, VIg, VIh, VIi, VIj, or VIk:

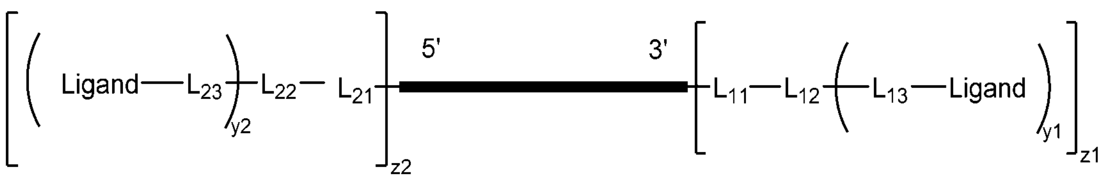

(VI)

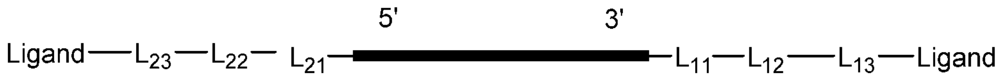

(VIa)

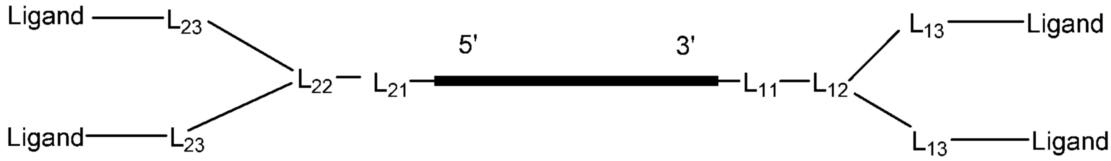

(VIb)

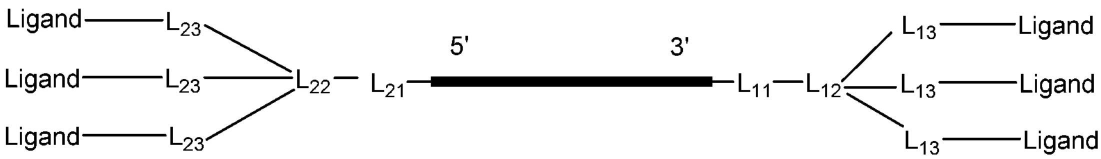

(VIc)

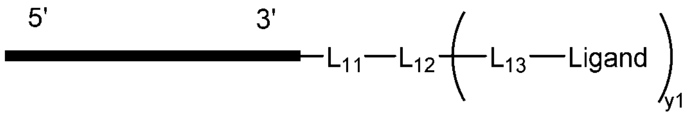

(VId)

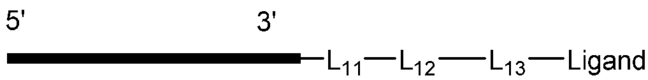

(VIe)

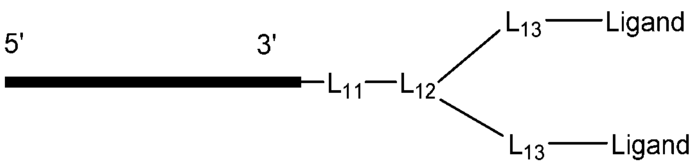

(VIf)

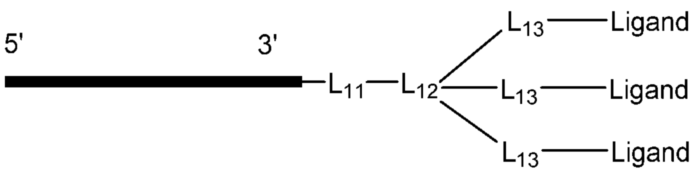

(VIg)

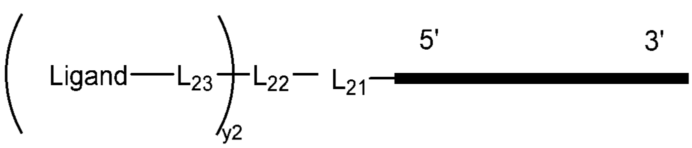

(VIh)

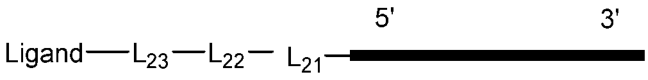


(VIi)

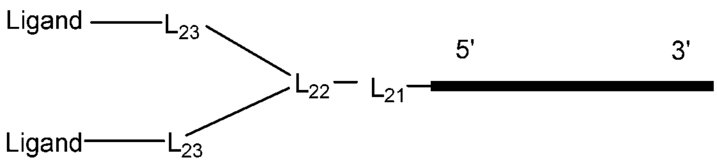


(VIj)

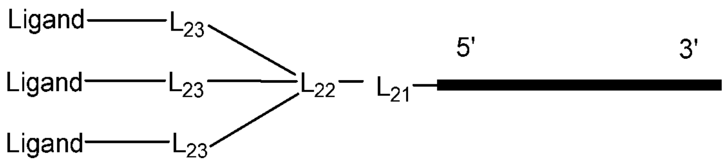


(VIk)

wherein,

is an oligonucleotide, preferably siRNA or ASO, the siRNA or ASO being connected to $L_{21}$ and/or $L_{11}$ via a phosphate group or phosphorothioate group;
z1 and z2 are each independently 0 or 1, and z1 and z2 are not both 0;
y1 and y2 are each independently 1, 2, or 3;
each Ligand is independently selected from compounds of Formula Ia", Formula Ib", Formula Ib"-1, Formula IIa", Formula IIb", Formula IIIa", Formula IIIb", Formula IIIa"-1, Formula IIIb"-1, Formula IVa", Formula IVa"-1, Formula IVb", Formula Va", Formula Va"-1, Formula Va"-2, Formula Va"-3, Formula Vb", Formula Vb"-1, Formula Vb"-2, Formula Vc", Formula Vc"-1, Formula Vc"-2, and Formula Vc"-3;
$L_{11}$ and $L_{21}$ are each independently selected from:

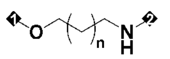
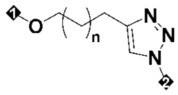
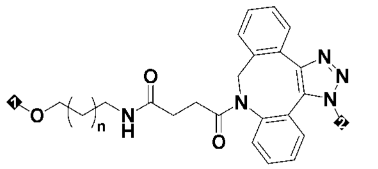
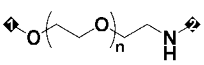
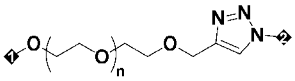
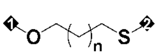
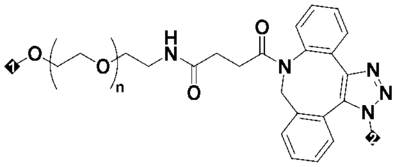
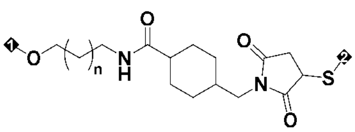
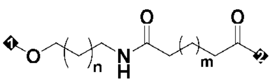
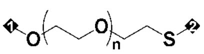
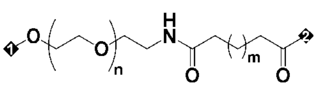
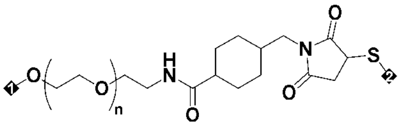
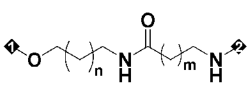
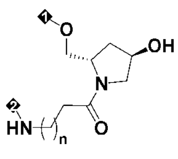
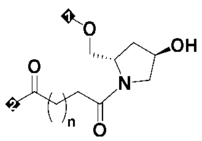
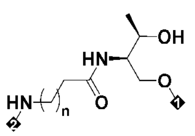
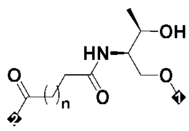
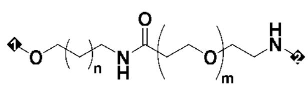

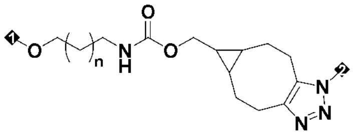

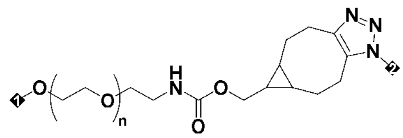

wherein n, m are integers from 0 to 10, preferably integers from 2 to 6, and attachment point 1 is connected to the oligonucleotide, attachment point 2 is connected to $L_{12}$ or $L_{22}$;

$L_{12}$ and $L_{22}$ are each independently a bond or a branching group, the branching group being independently selected from:

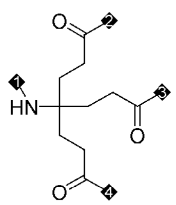

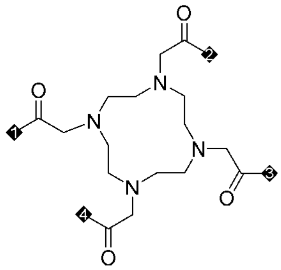

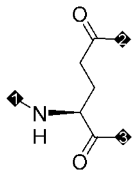

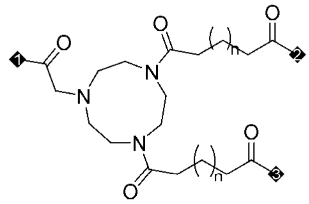

wherein n is an integer from 0 to 10, preferably an integer from 2 to 6, and attachment point 1 is connected to $L_{11}$ or $L_{21}$, attachment points 2, 3, and 4 are connected to $L_{13}$ or $L_{23}$;

$L_{13}$ and $L_{23}$ are each independently selected from:

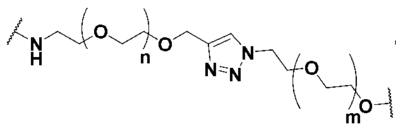

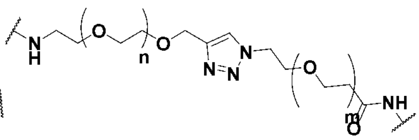

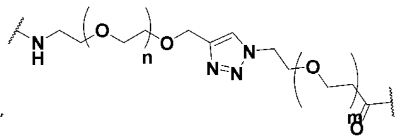

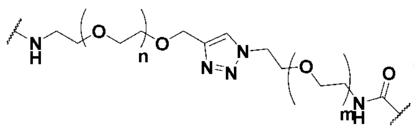

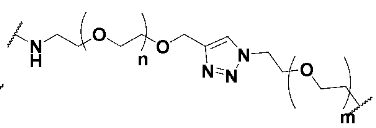

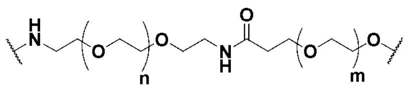

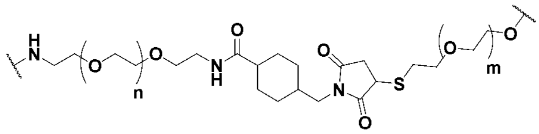

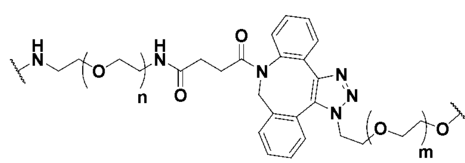

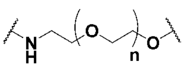

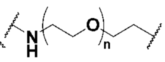

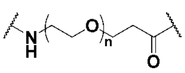

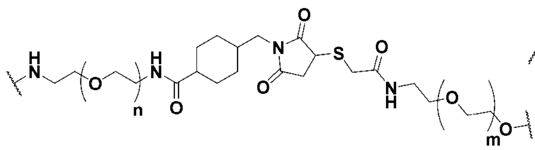

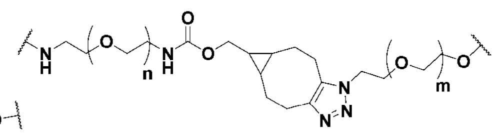

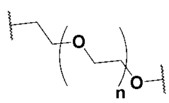

wherein n, m are integers from 0 to 10, preferably integers from 2 to 6, and the wave line on the left side of the formula is connected to $L_{12}$ or $L_{22}$, the wave line on the right side of the formula is connected to the Ligand.

In preferred embodiments, $L_{21}$, $L_{22}$ and $L_{23}$ together form a structure selected from the following, wherein the left side of the formula is connected to the oligonucleotide via a phosphate or phosphorothioate group:

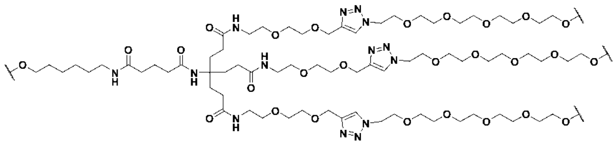
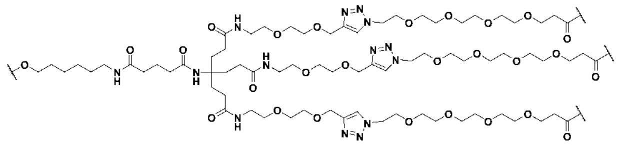
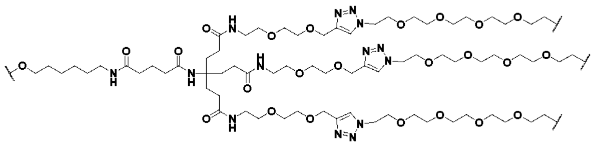
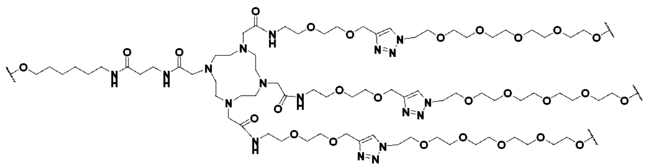
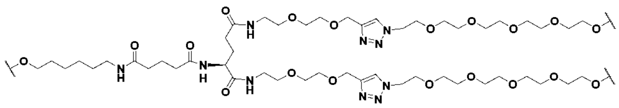
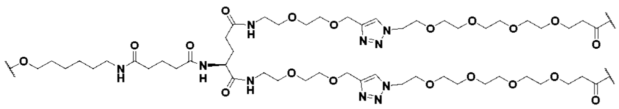
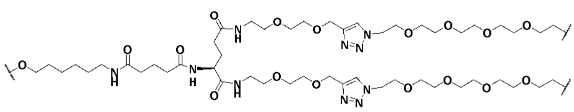
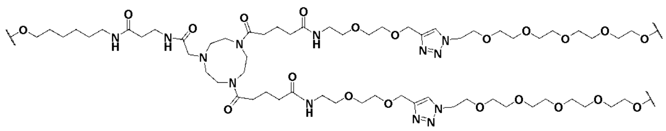
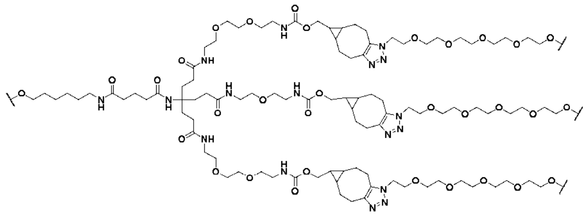
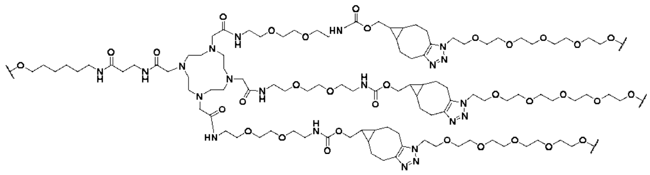
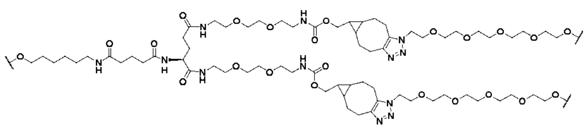
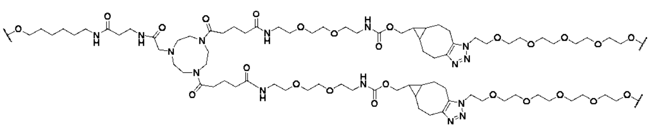
;

In preferred embodiments, $L_{11}$, $L_{12}$ and $L_{13}$ together form a structure selected from the following, wherein the left side of the formula is connected to the oligonucleotide via a phosphate or phosphorothioate group:

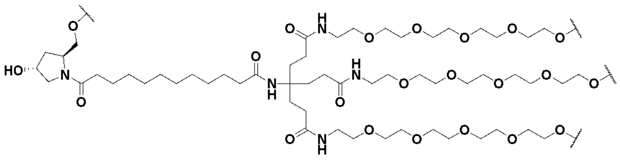
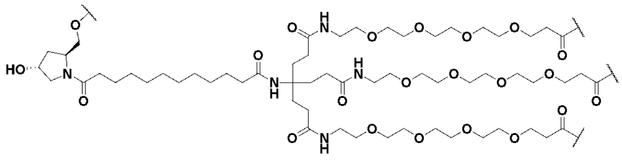
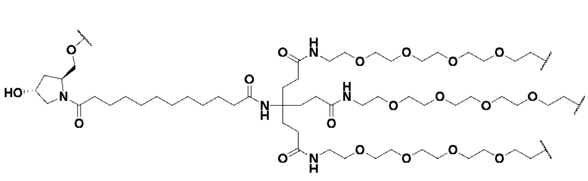
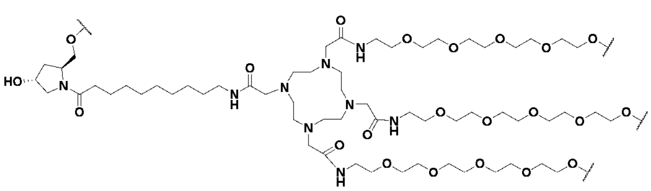
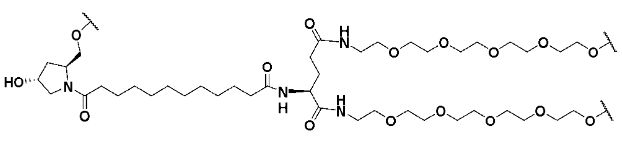
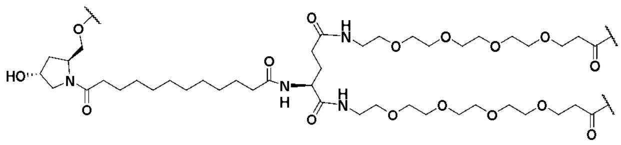

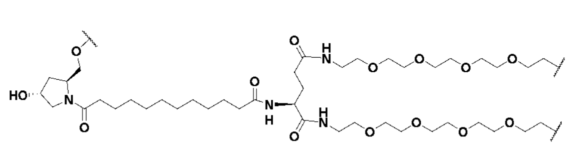 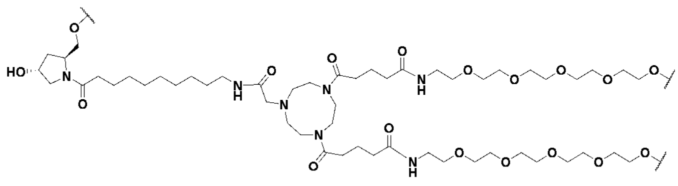

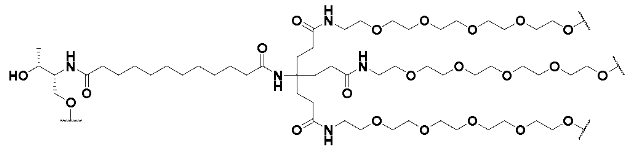 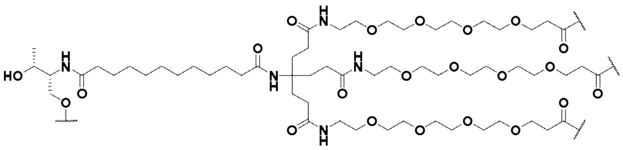

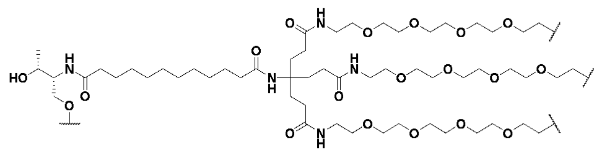 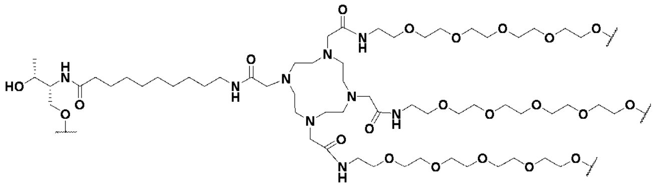

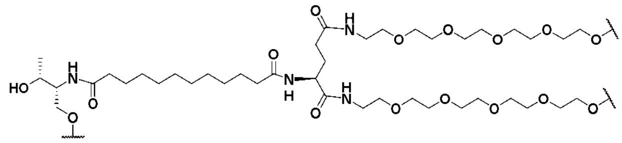 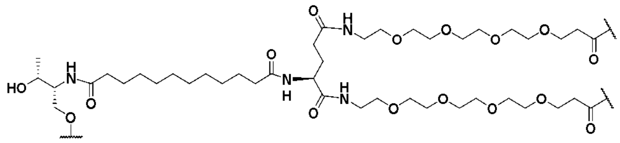

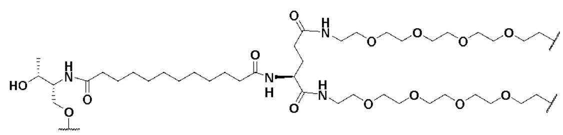 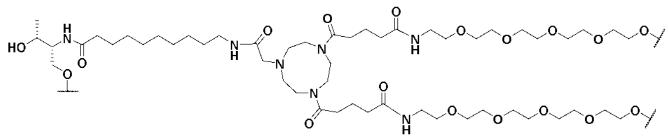 .

In some embodiments, the oligonucleotide molecule can be directly covalently coupled to the integrin ligand compound of the present invention. An oligonucleotide molecule suitable for such covalent coupling may have the following structure, where "oligonucleotides" represents an oligonucleotide, e.g., siRNA or ASO, and n is an integer from 0 to 10, preferably an integer from 2 to 6.

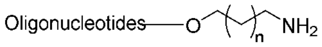 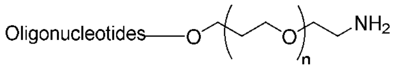

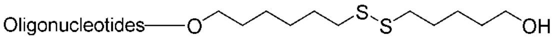

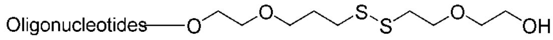

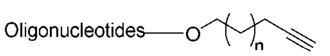 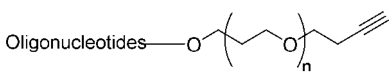

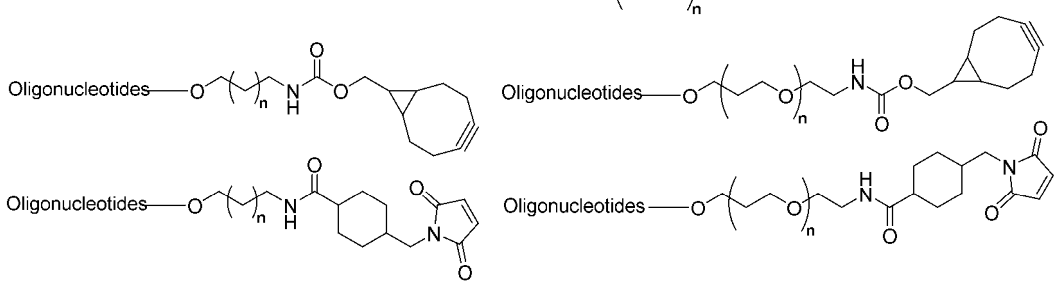

In some embodiments, the oligonucleotide molecule can be covalently coupled to multiple (e.g., 1 to 4) integrin ligand compounds of the present invention. An oligonucleotide molecule suitable for such covalent coupling may have the following structure, where "oligonucleotides" represents an oligonucleotide, e.g., siRNA or ASO; n is an integer from 0 to 10, preferably an integer from 2 to 6.

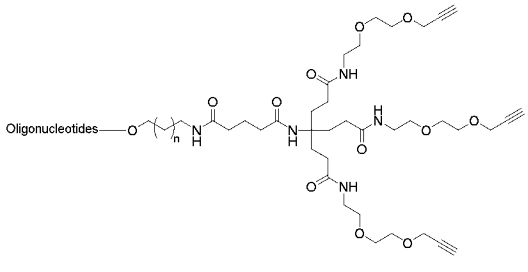

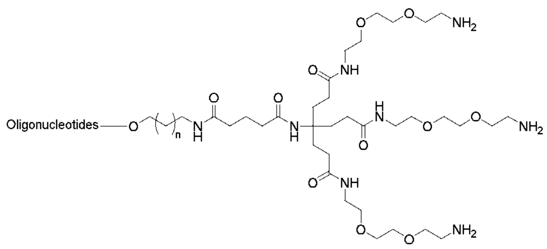

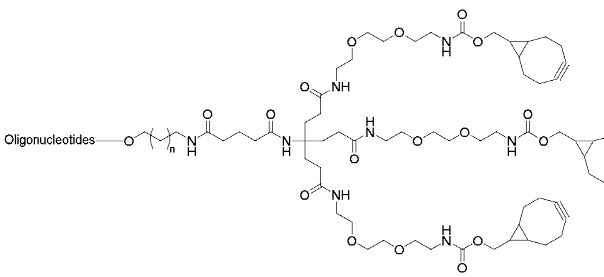

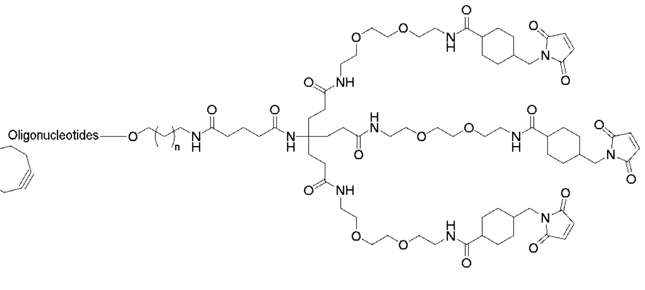

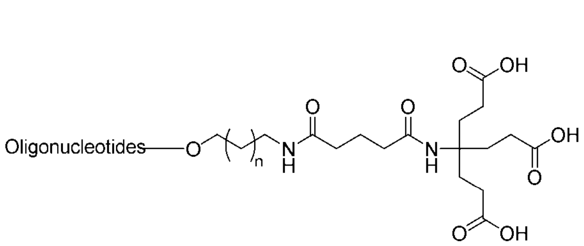

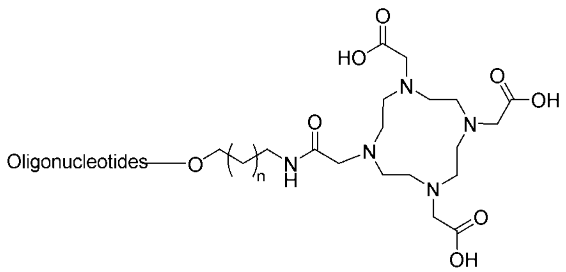

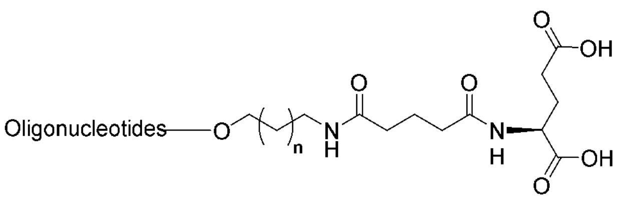

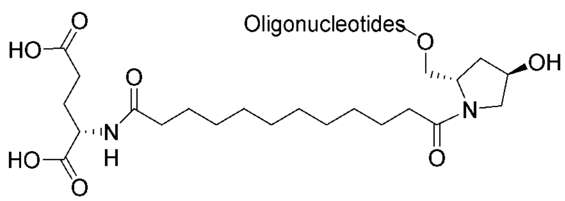

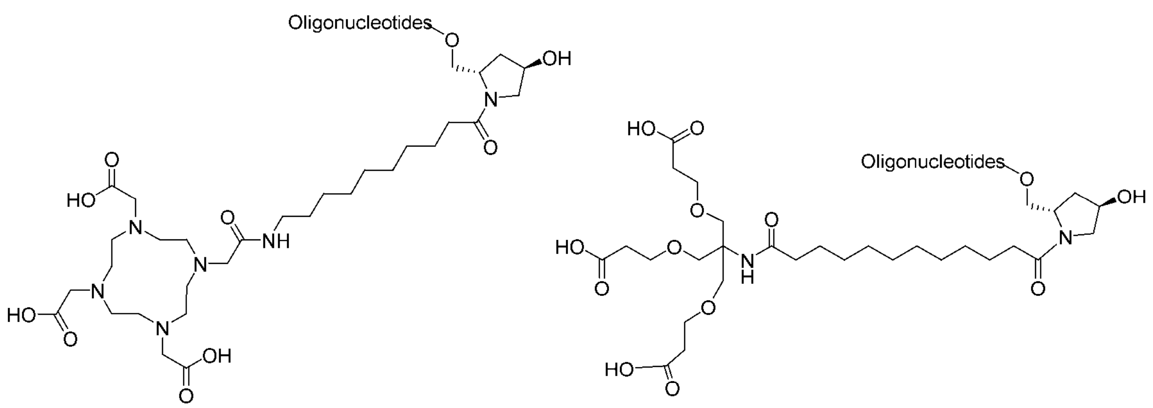

In preferred embodiments, the present invention provides an RNAi agent having a structure as shown in any one in the following table, wherein

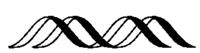

represents siRNA or ASO, X = O or S.

| | |
|---|---|
| 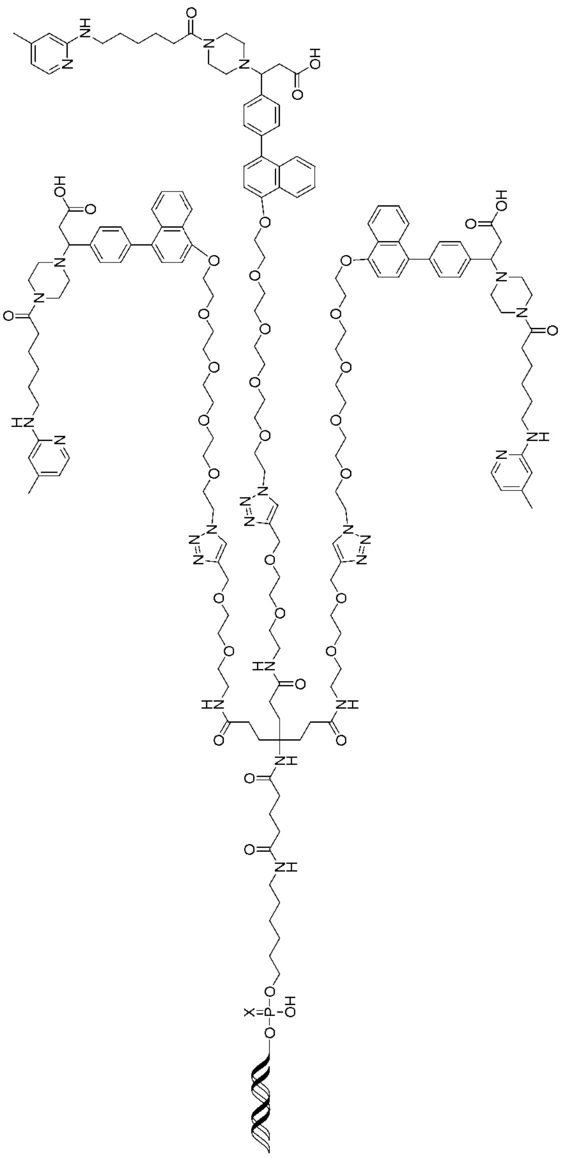 | 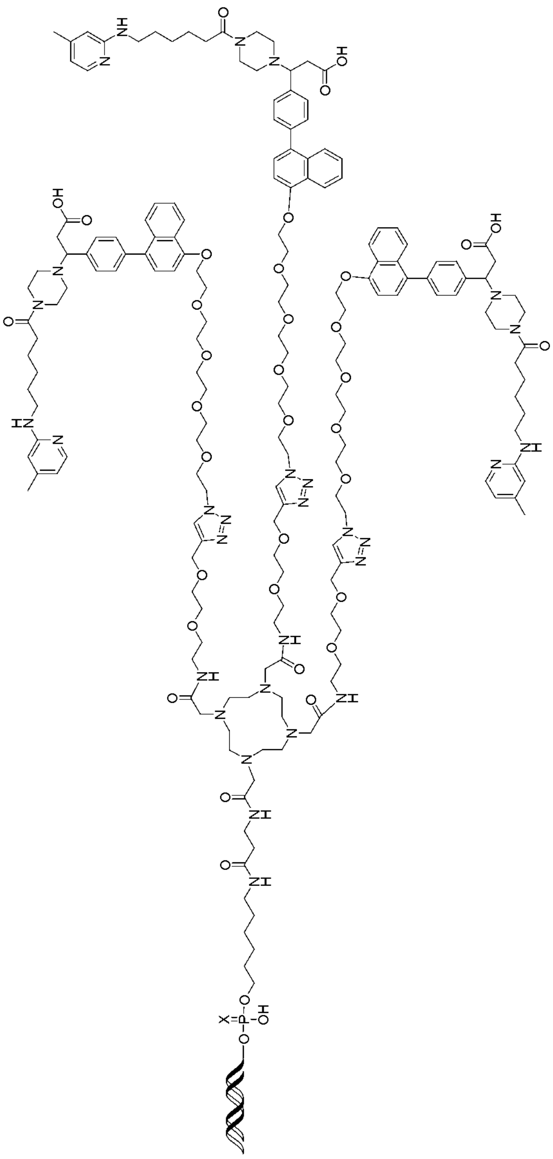 |
| 201-21 | 202-21 |

(continued)

| | |
|---|---|
| 203-21 | 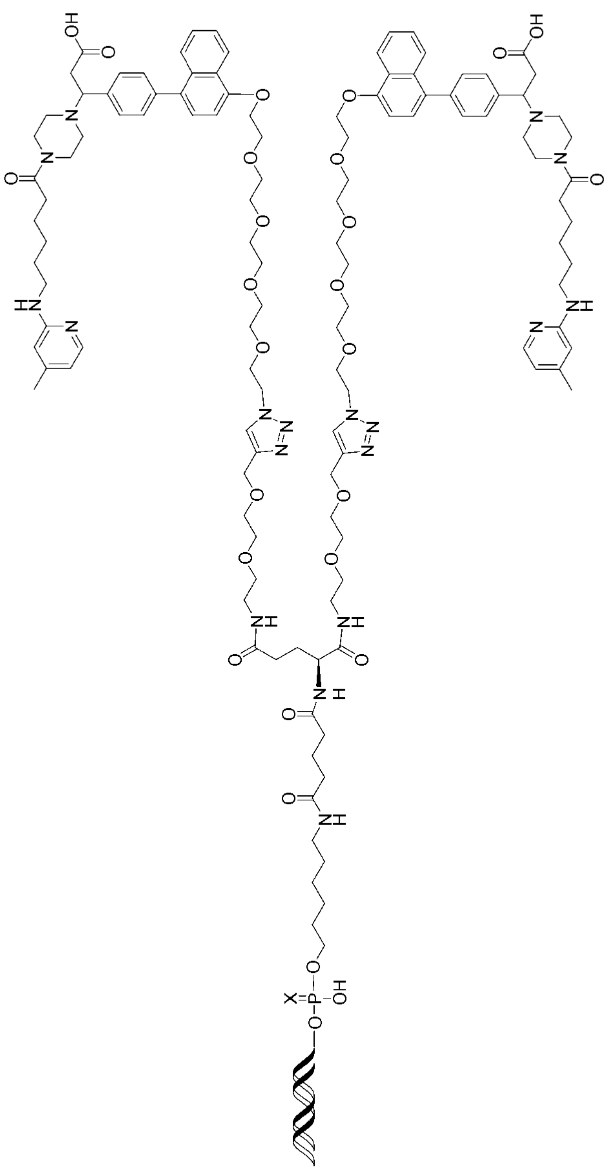 |
| 204-21 | 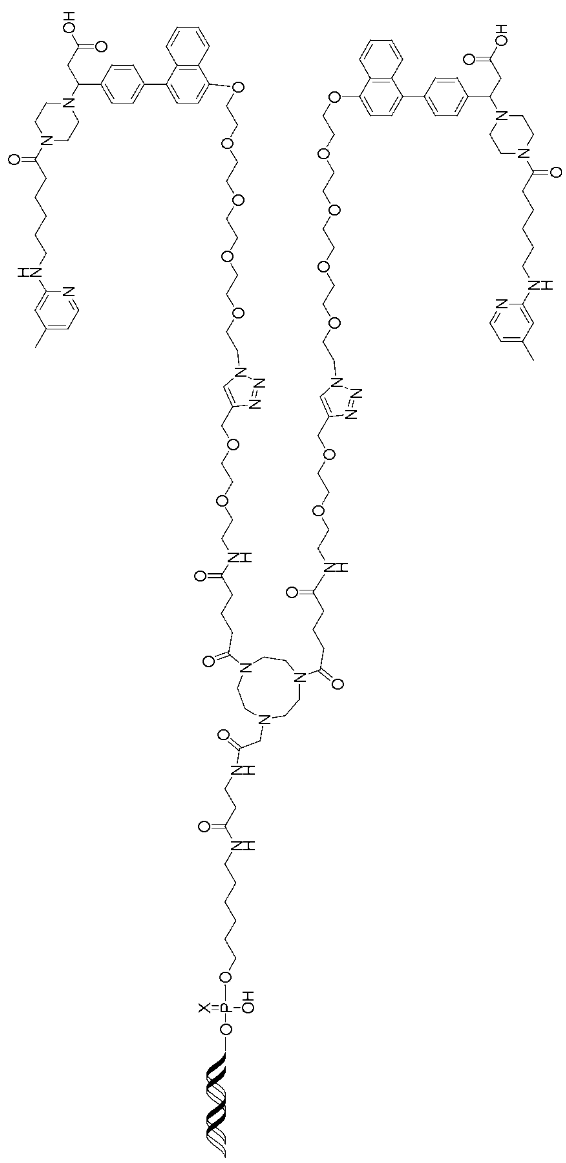 |

(continued)

| | |
|---|---|
| 205-21 | 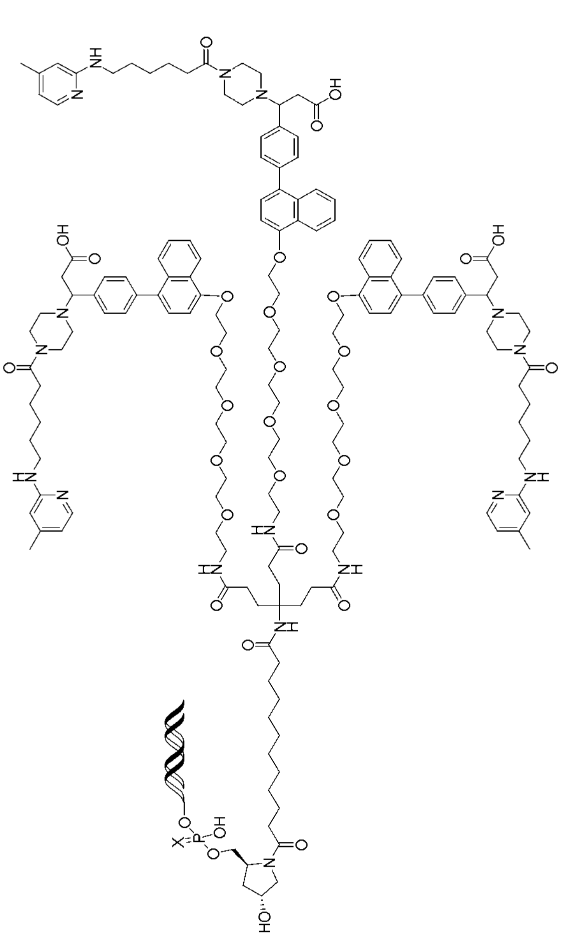 |
| 206-21 | 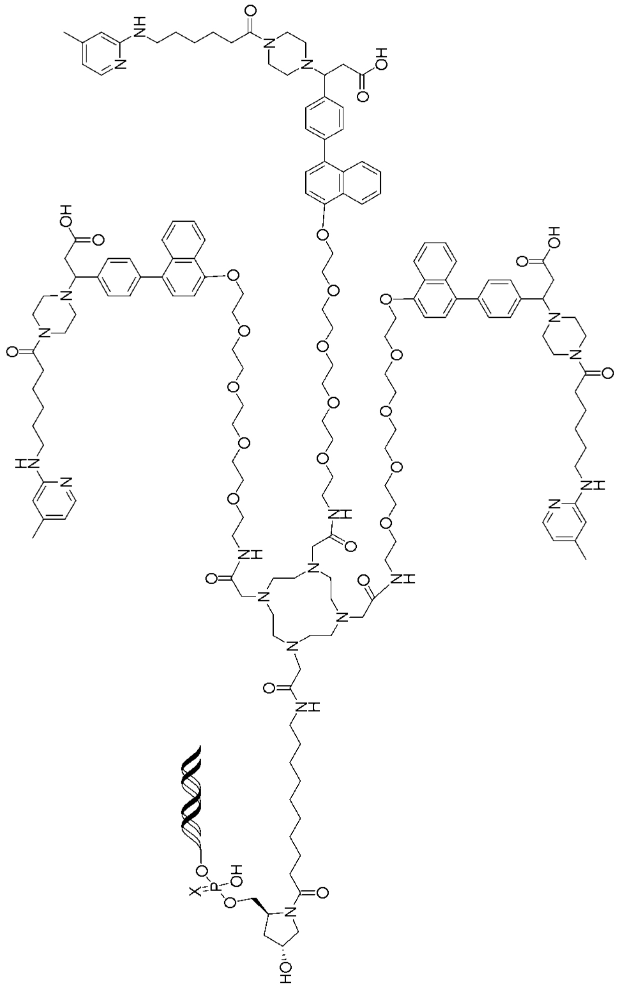 |

(continued)

| | |
|---|---|
| 207-21 | 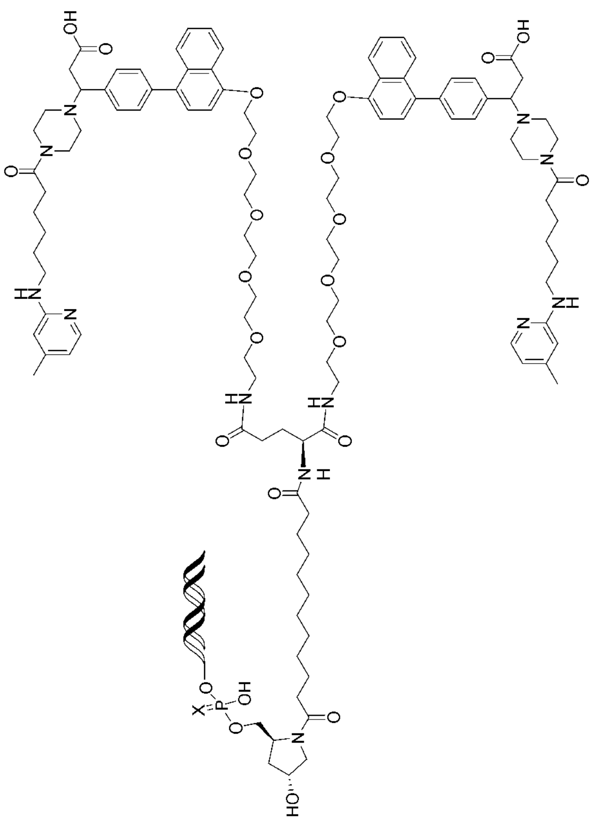 |
| 208-21 | 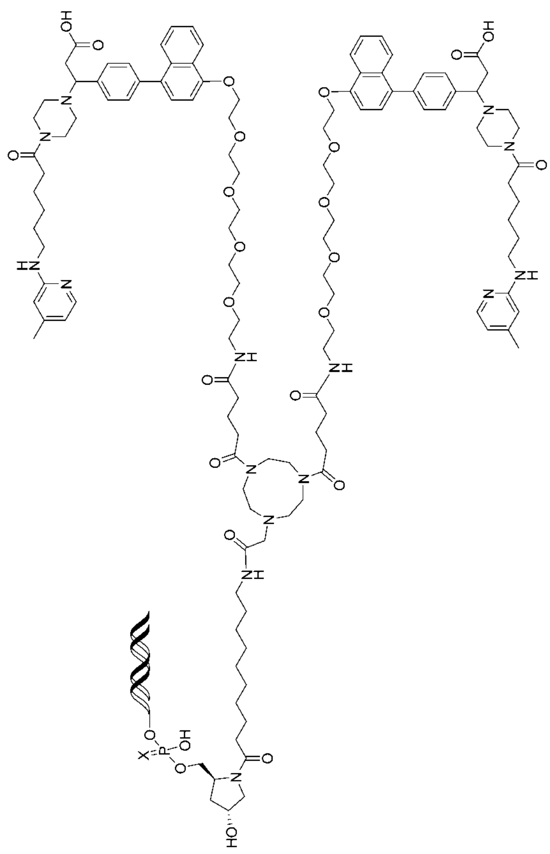 |

(continued)

| | |
|---|---|
| 209-21 | 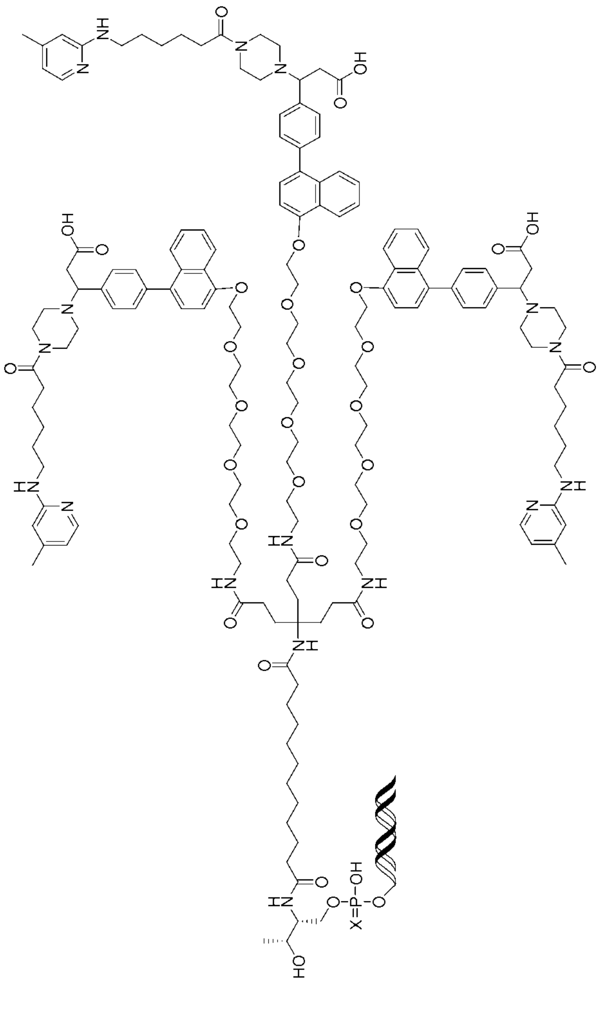 |
| 210-21 | 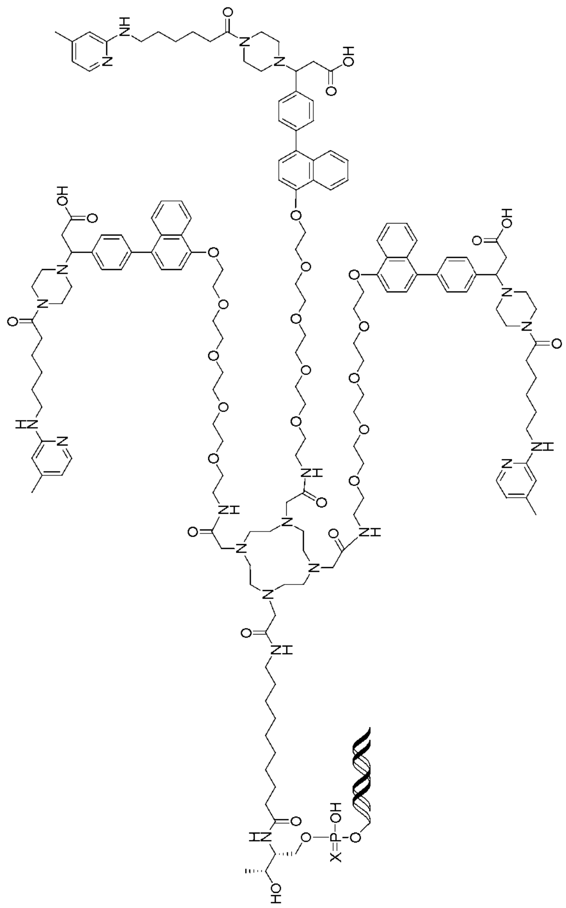 |

(continued)

| | |
|---|---|
| 211-21 | 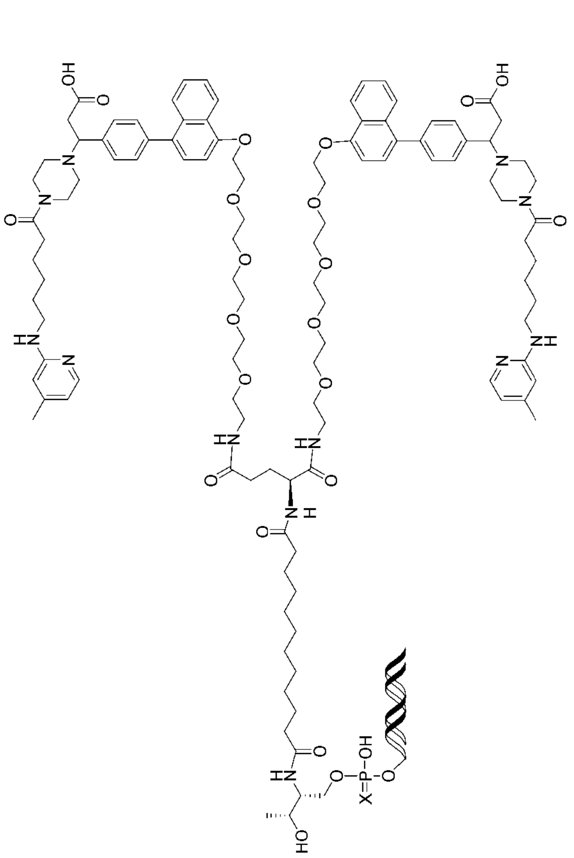 |
| 212-21 | 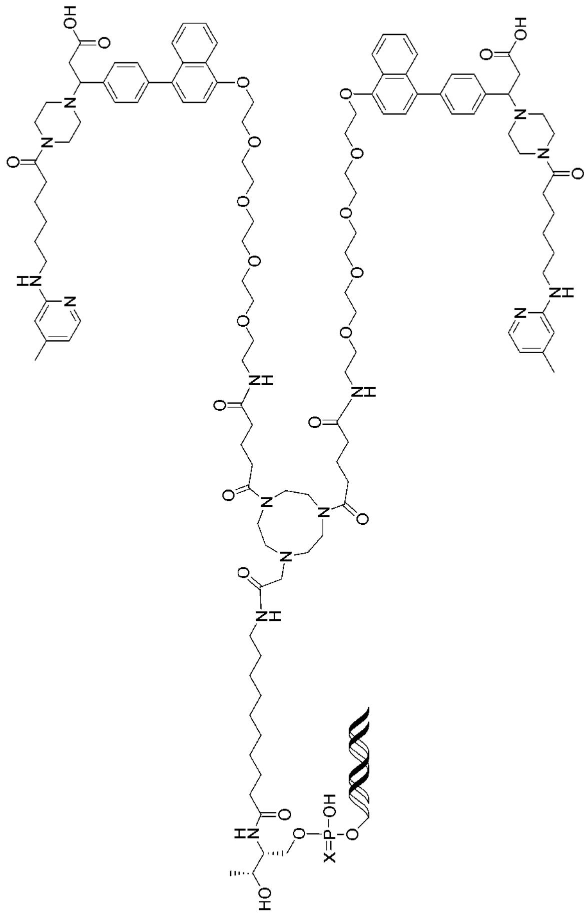 |

(continued)

| | |
|---|---|
| 213-5 | 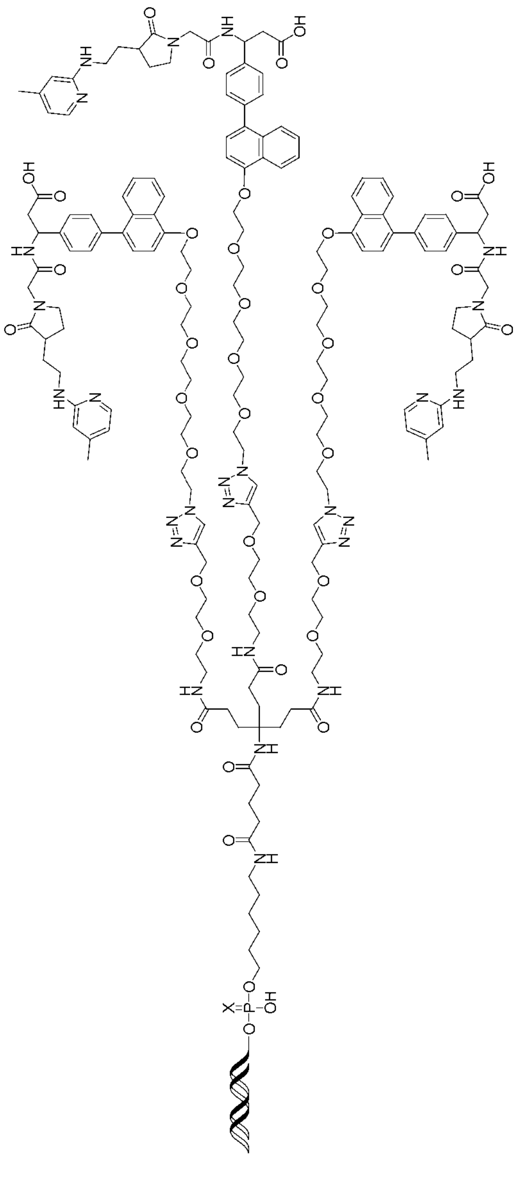 |
| 214-5 | 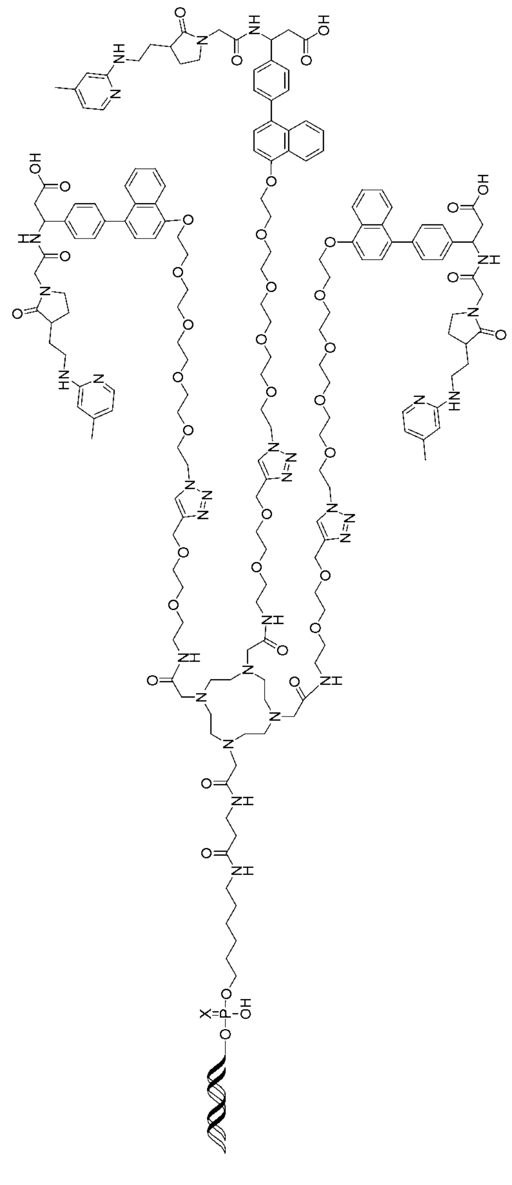 |

(continued)

| | |
|---|---|
| 215-5 | 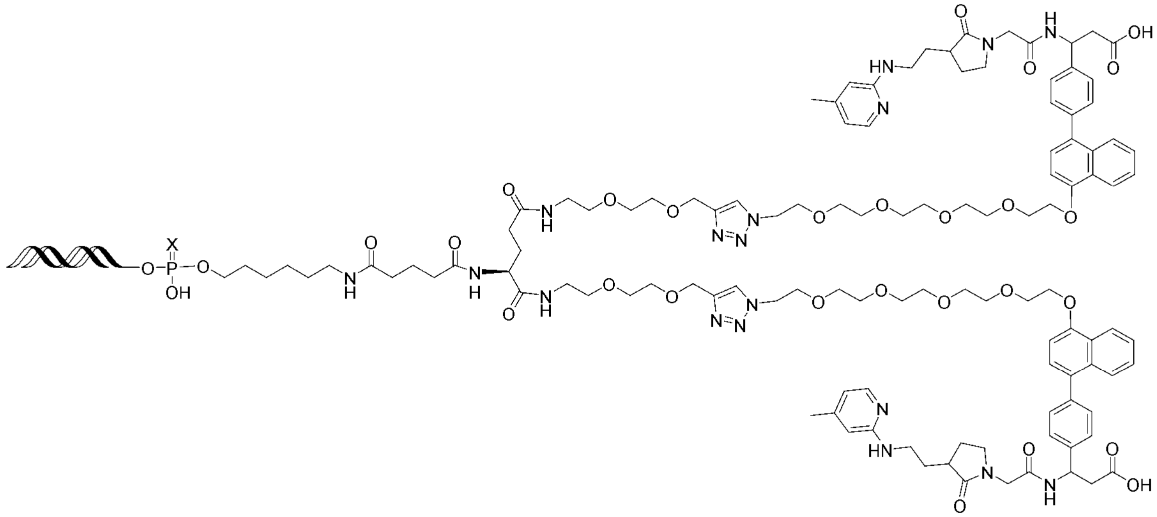 |
| 216-5 | 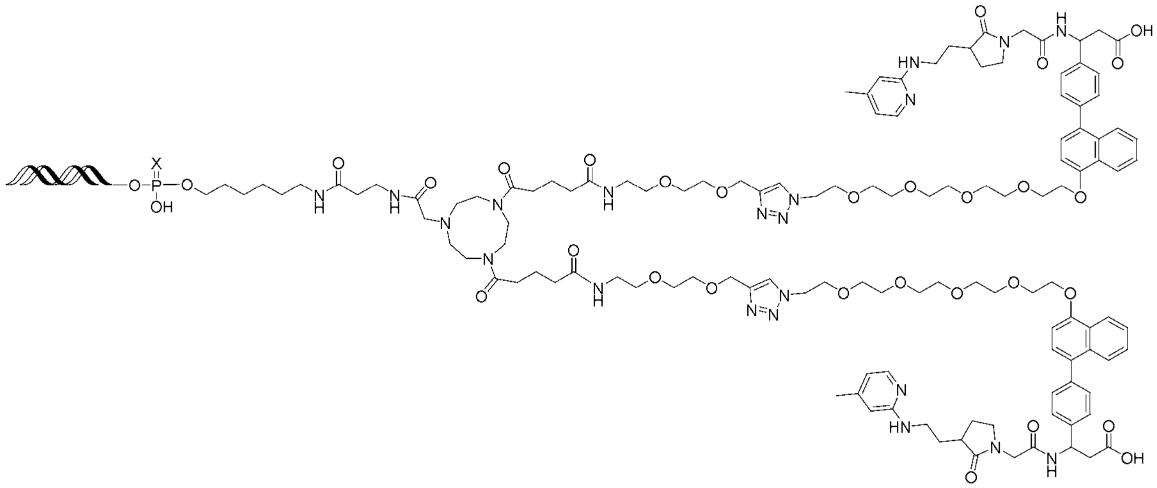 |

(continued)

| | |
|---|---|
| 217-5 | 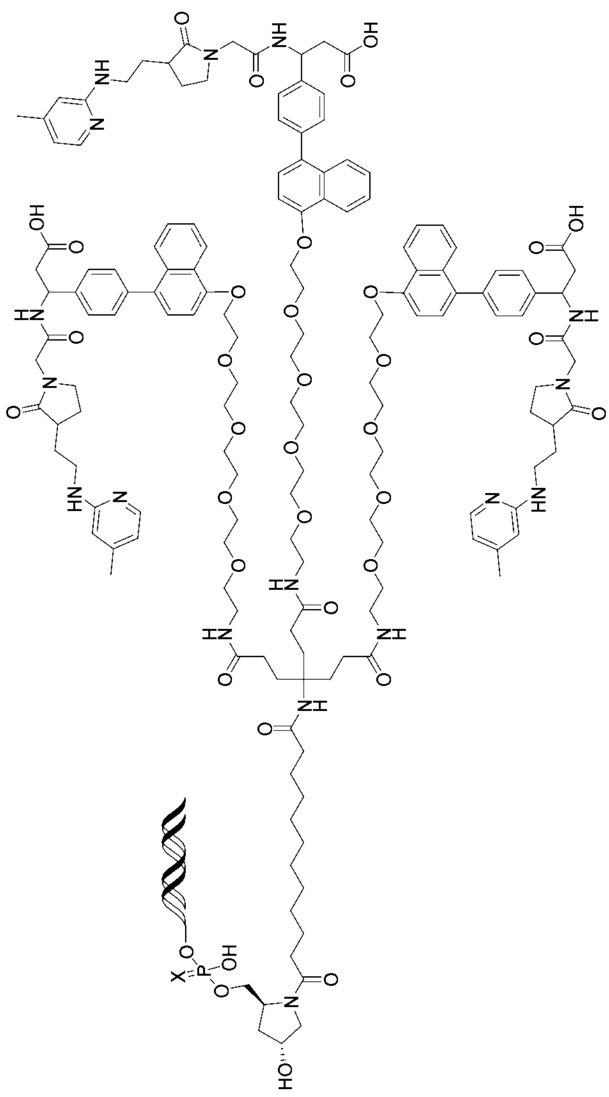 |

(continued)

| | |
|---|---|
| 218-5 | 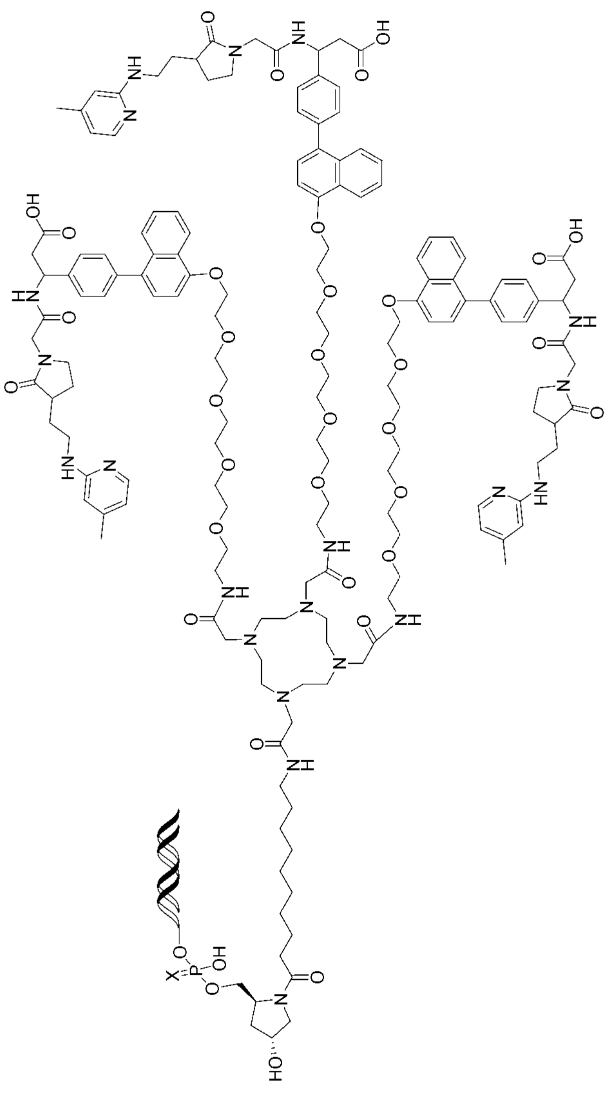 |
| 219-5 | 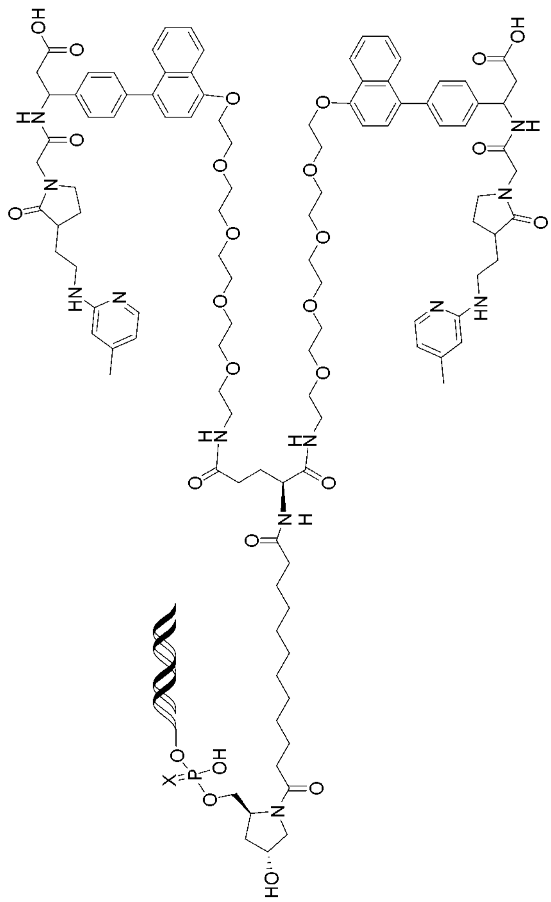 |

(continued)

| | |
|---|---|
| 220-5 | 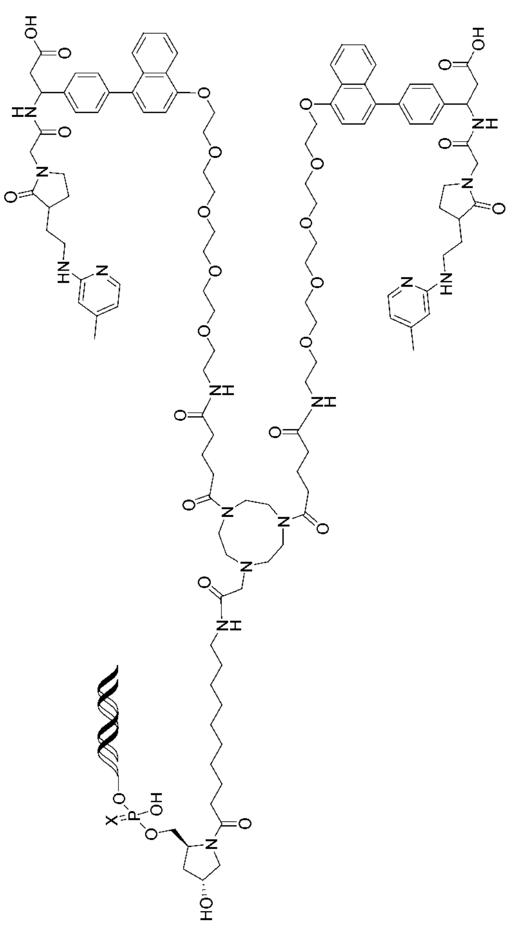 |
| 221-5 | 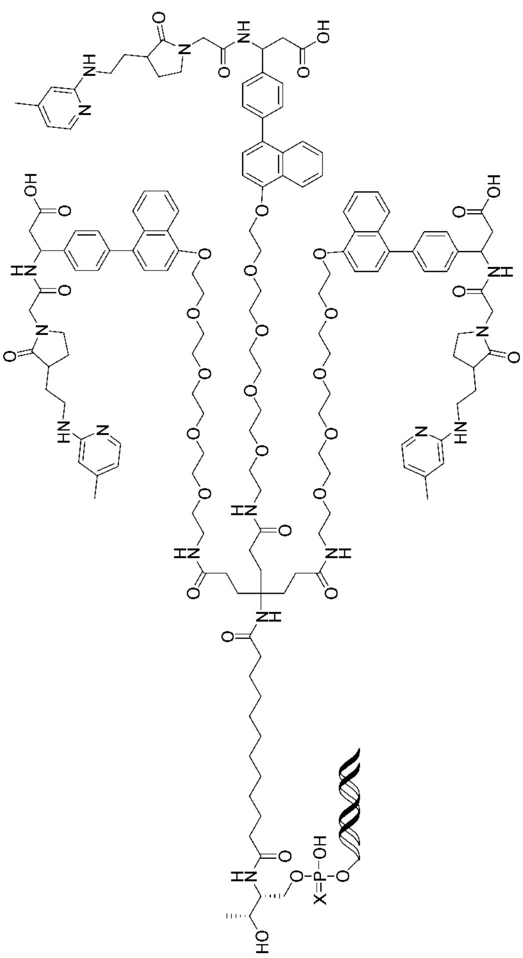 |

(continued)

| | |
|---|---|
| 222-5 | 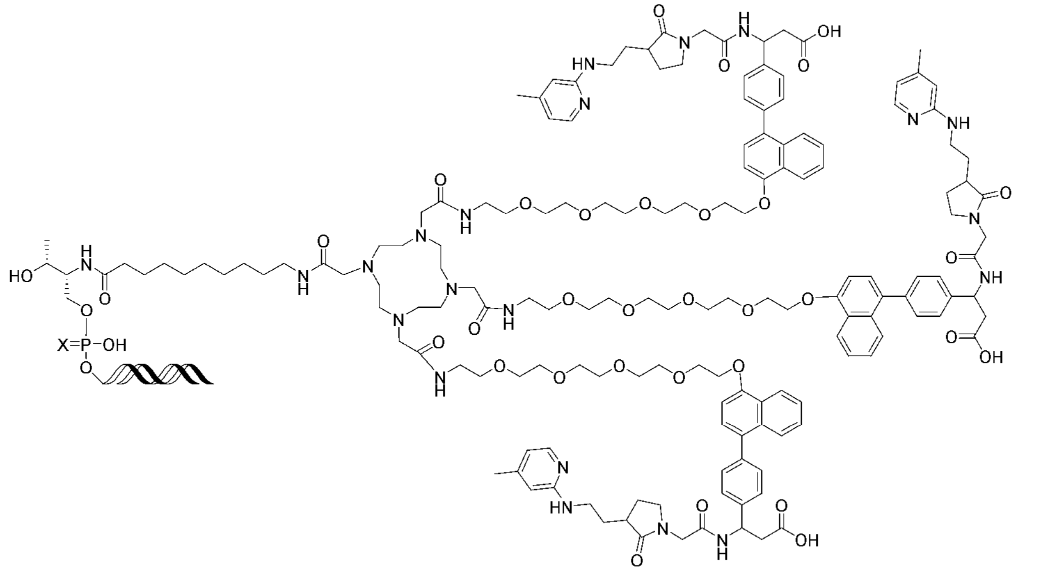 |
| 223-5 | 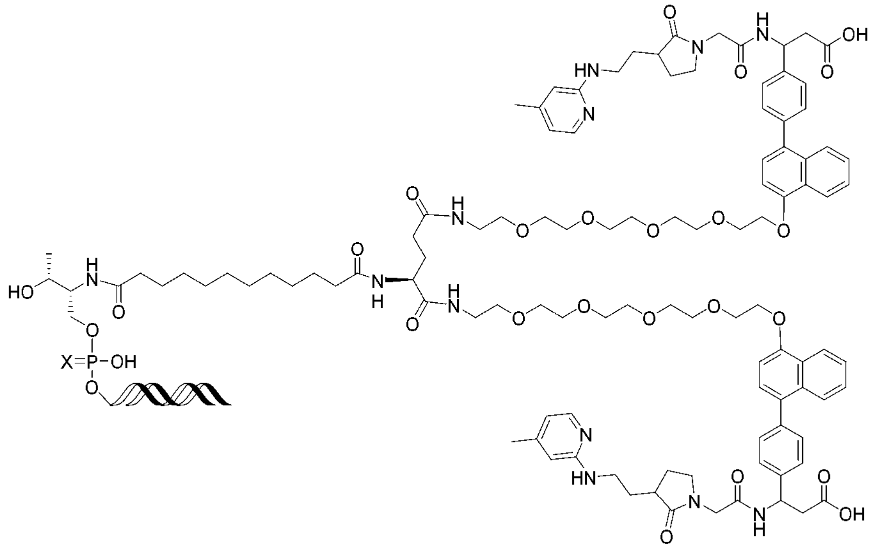 |

(continued)

| | |
|---|---|
| 224-5 | 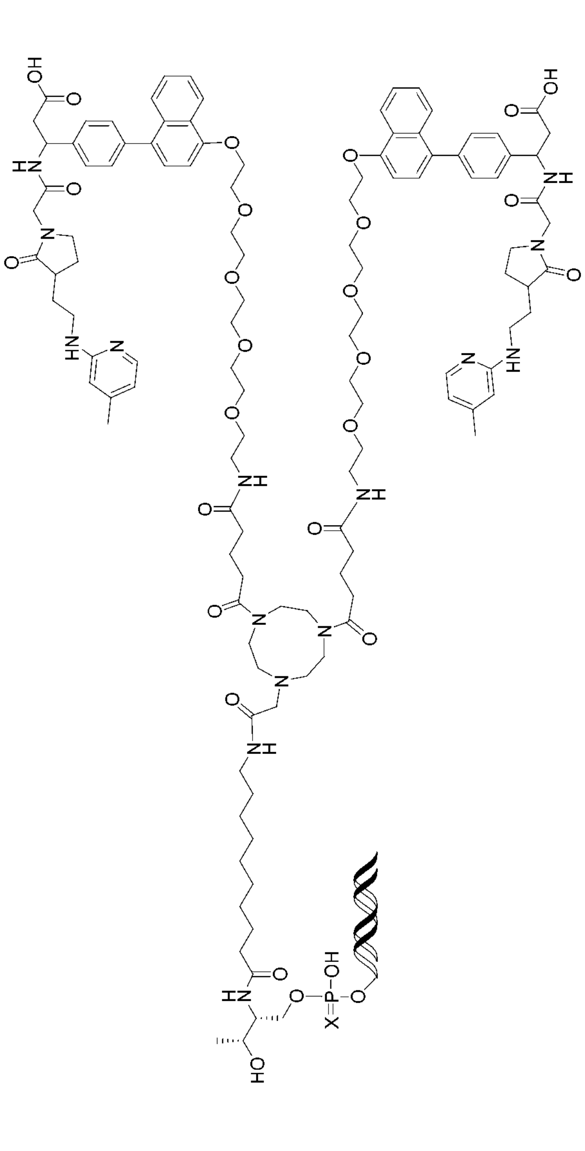 |
| 225-6 | 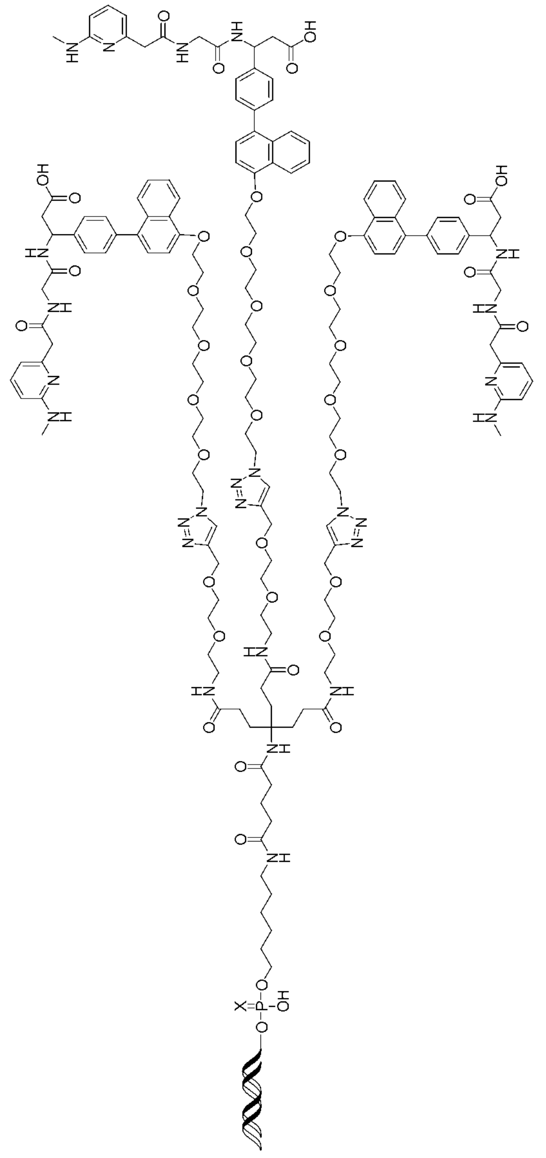 |

(continued)

| | |
|---|---|
| 226-6 | 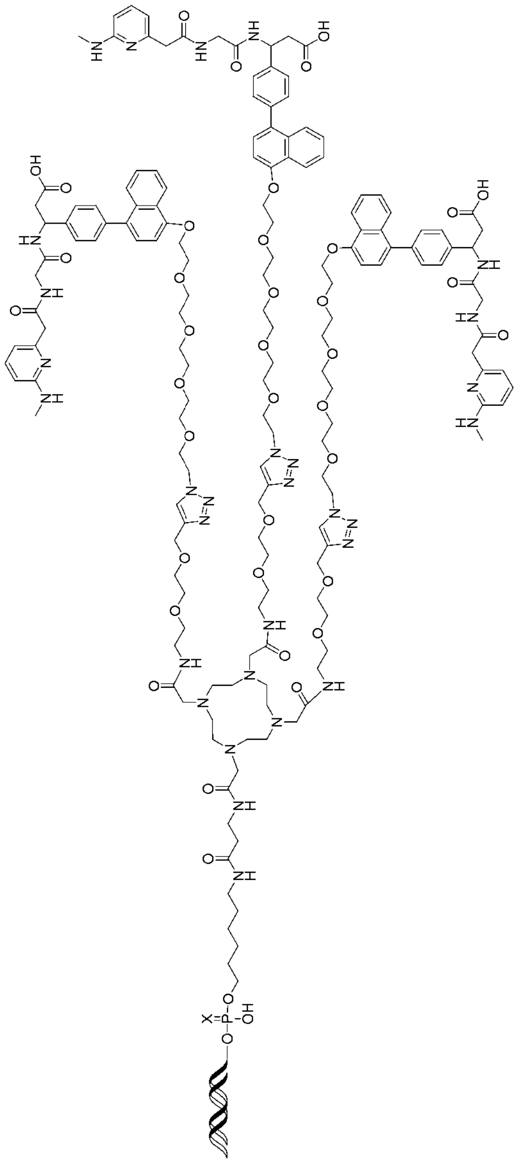 |
| 227-6 | 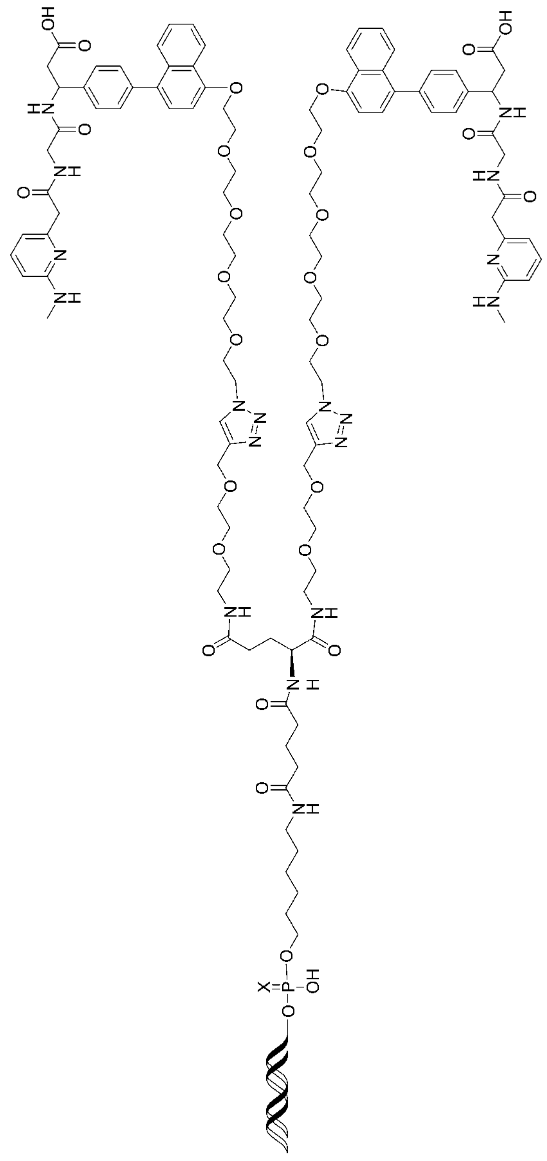 |

(continued)

| | |
|---|---|
| 228-6 | 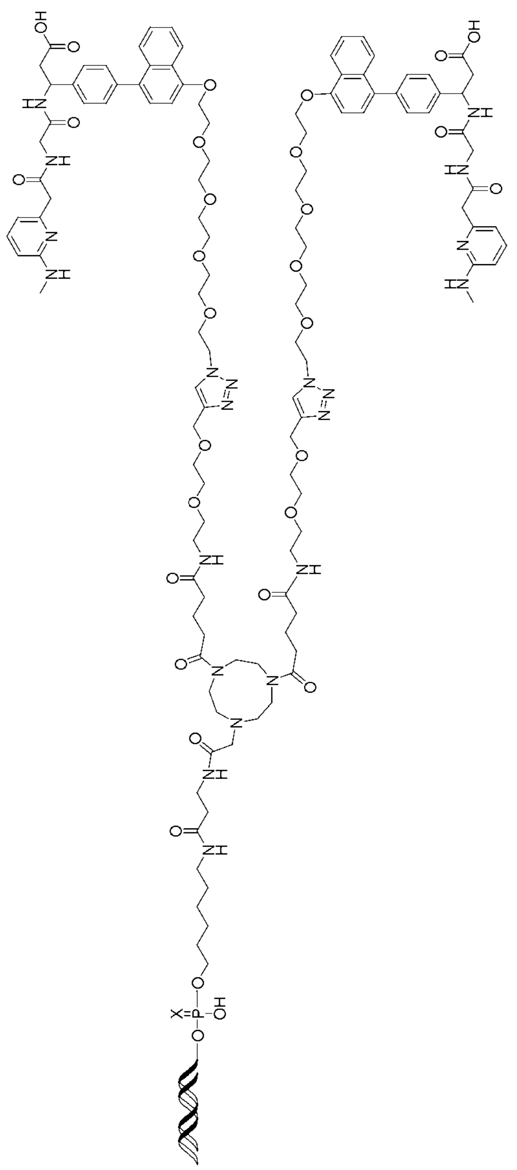 |
| 229-6 | 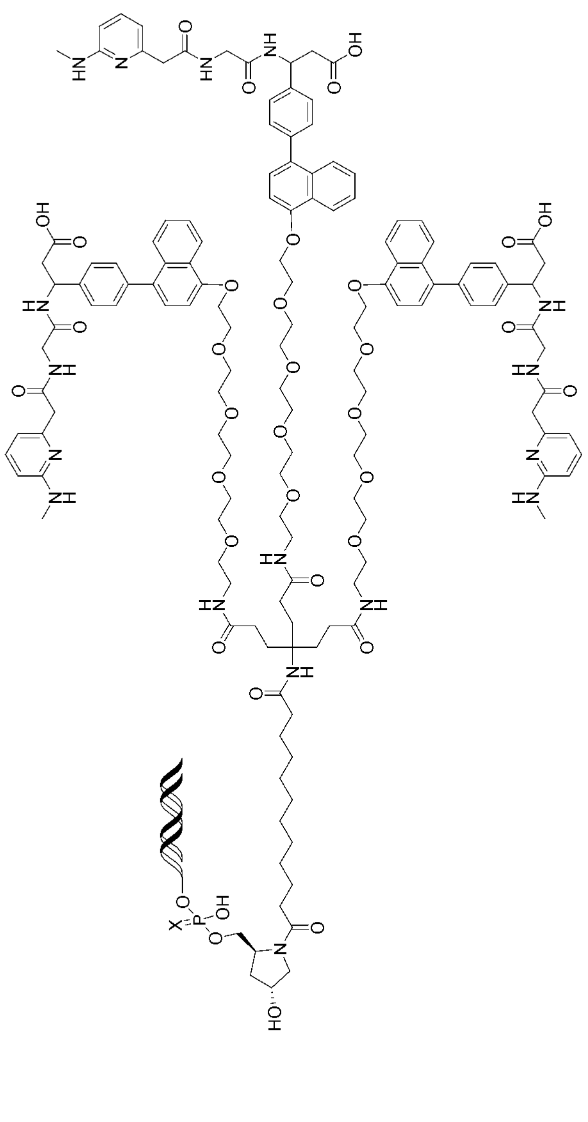 |

(continued)

| | |
|---|---|
| 230-6 | 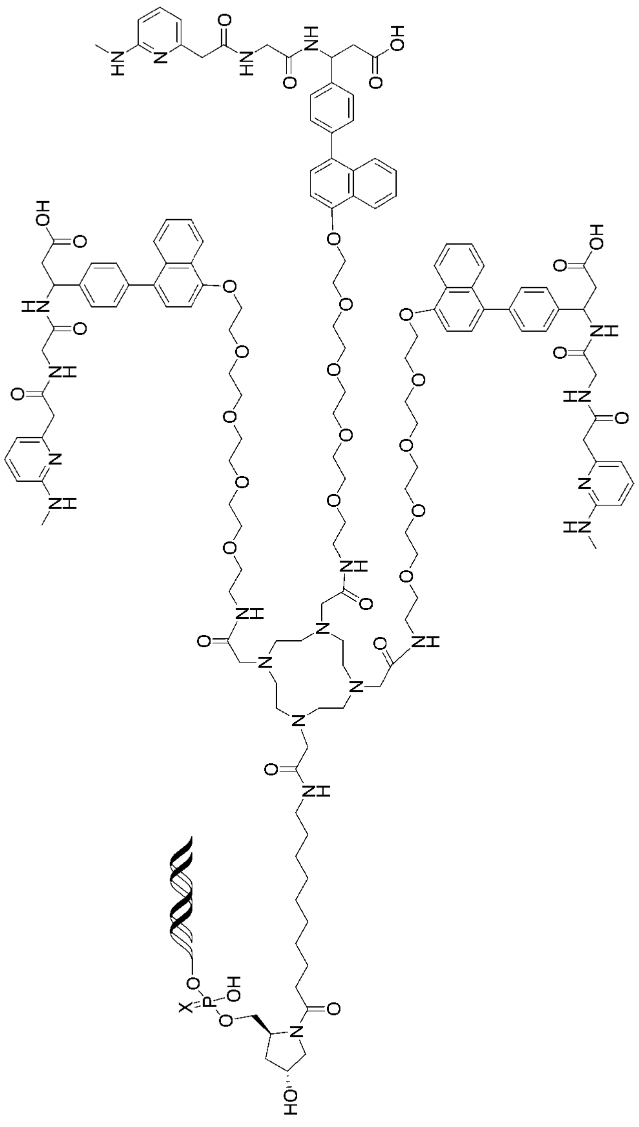 |

(continued)

| | |
|---|---|
| 231-6 | 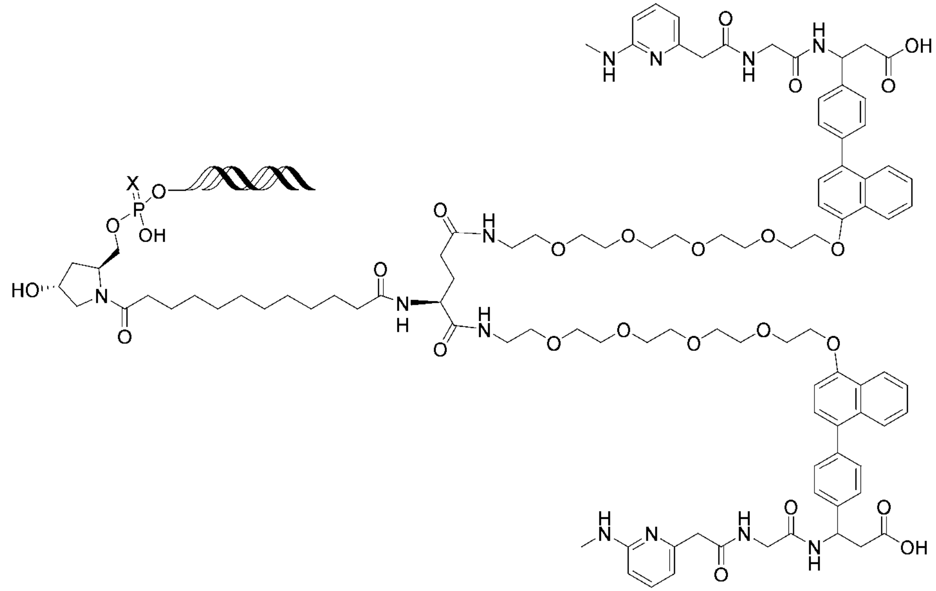 |
| 232-6 | 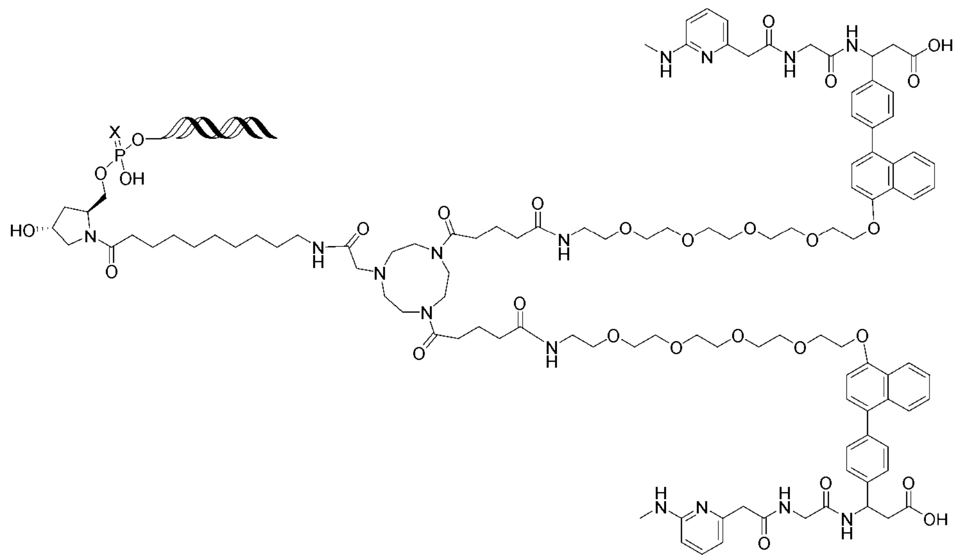 |

(continued)

| | |
|---|---|
| 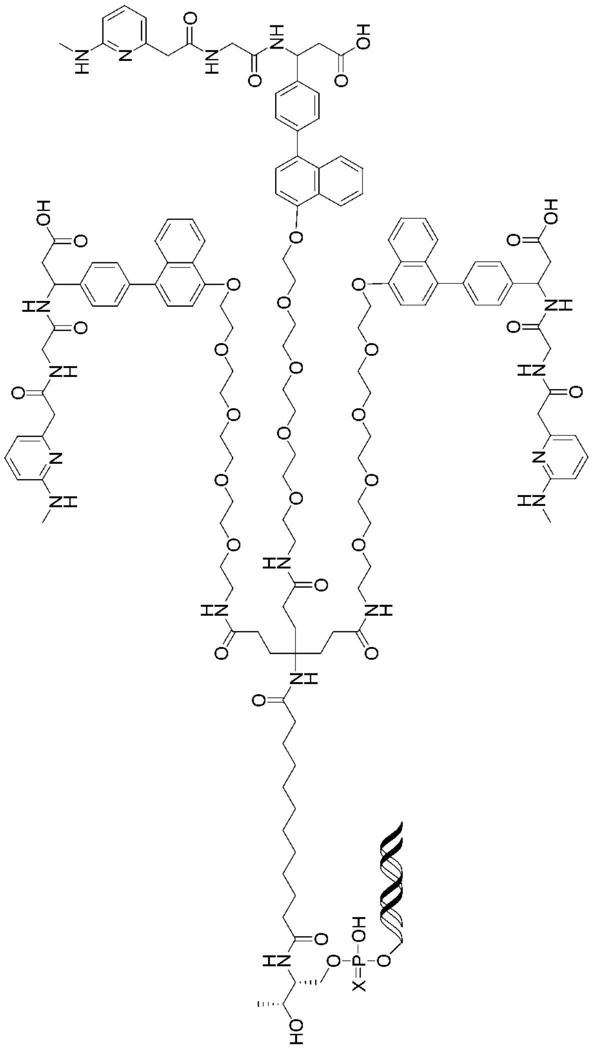 | 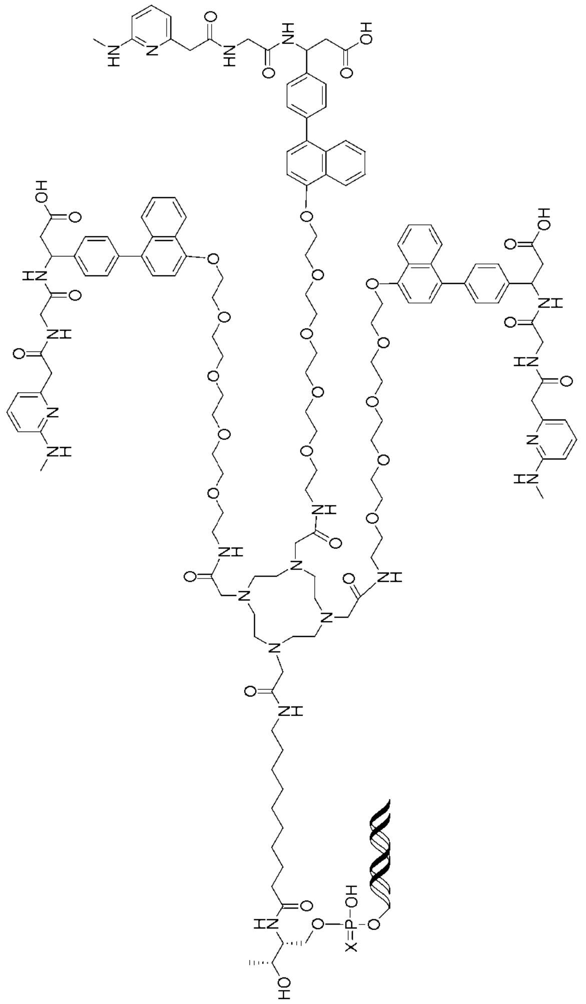 |
| 233-6 | 234-6 |

(continued)

| | |
|---|---|
| 235-6 | 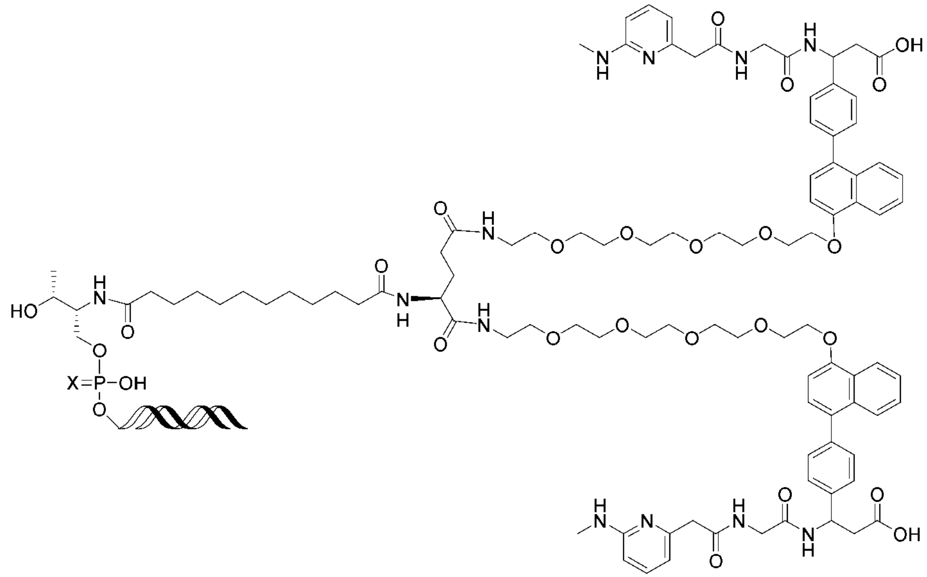 |

(continued)

| | |
|---|---|
| 236-6 | 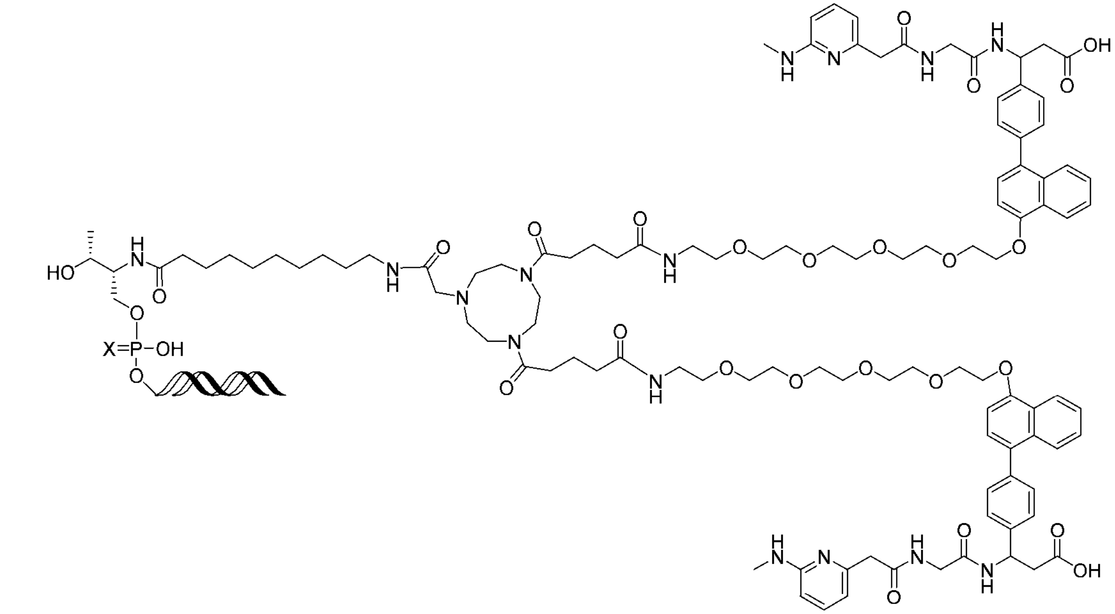 |
| 237-7 | 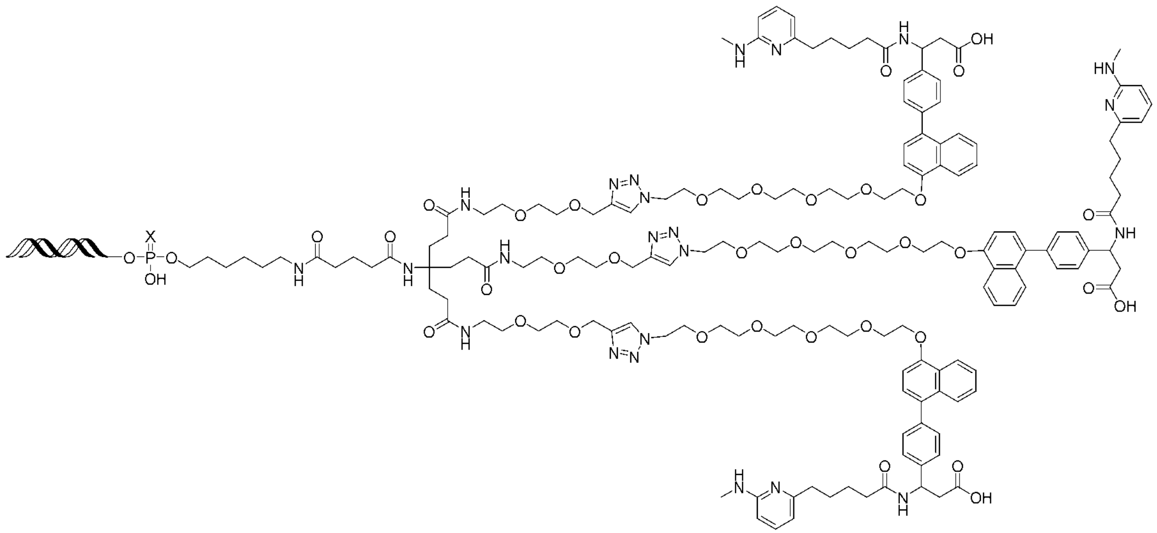 |

(continued)

| | |
|---|---|
| 238-7 | 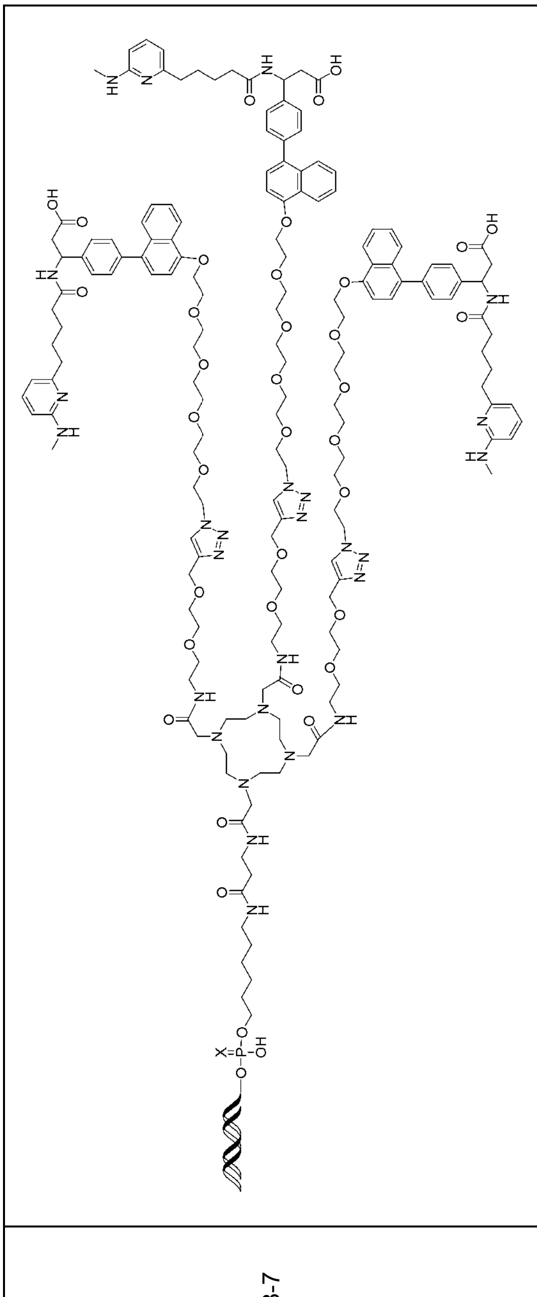 |
| 239-7 | 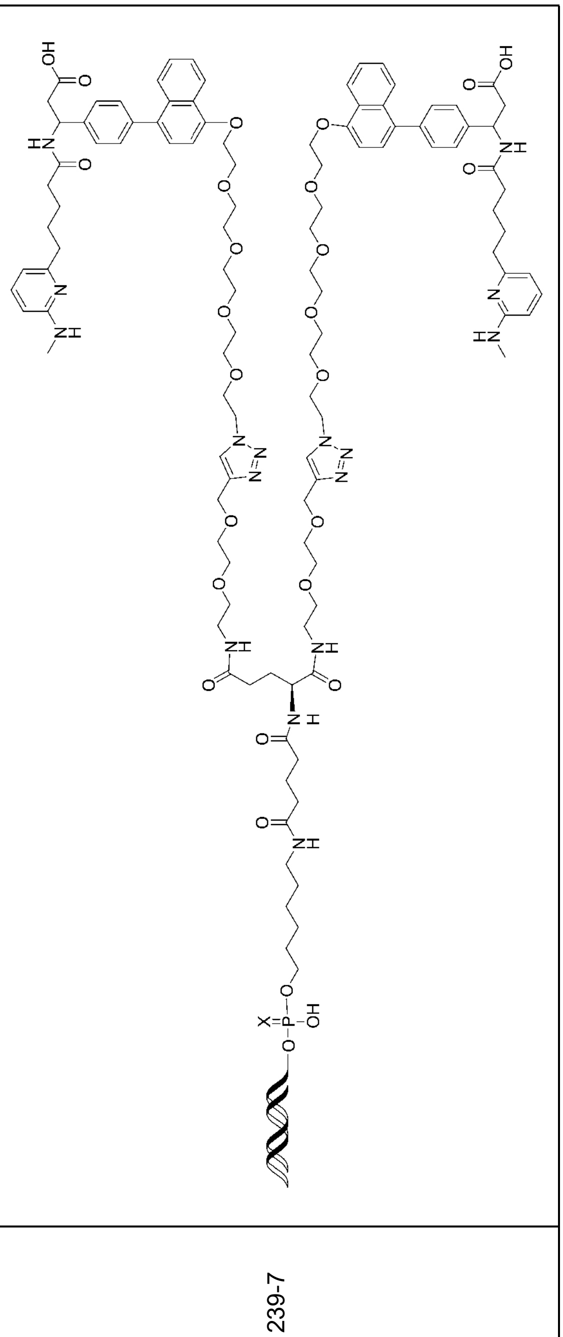 |

(continued)

| | |
|---|---|
| 240-7 | 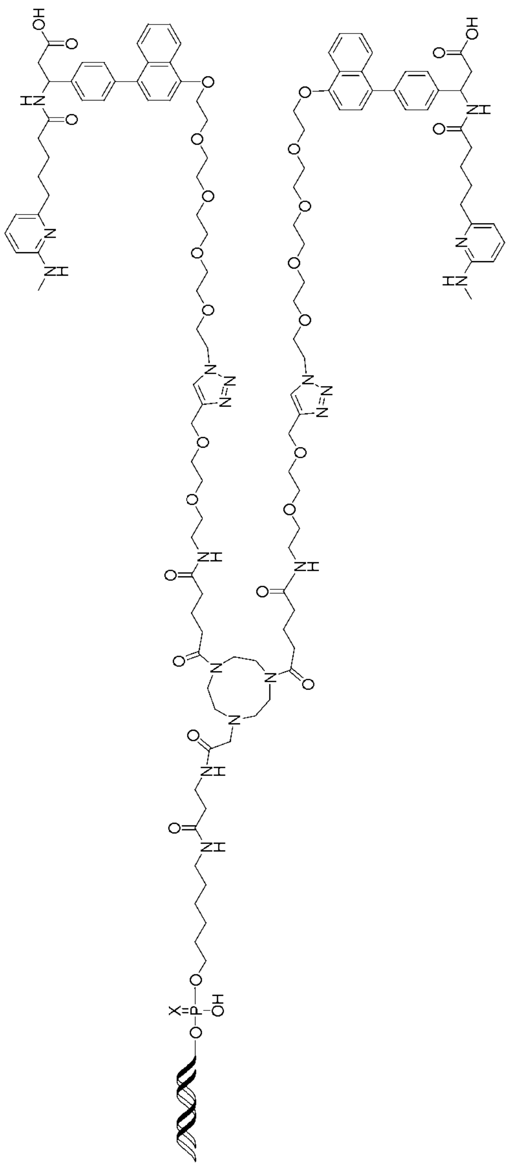 |
| 241-7 | 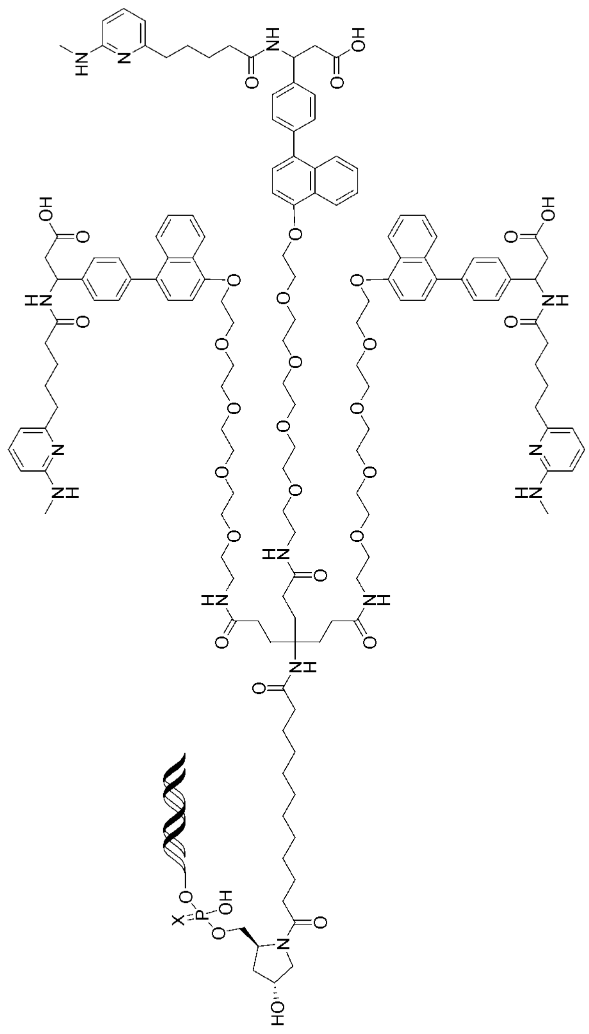 |

(continued)

| | |
|---|---|
| 242-7 | 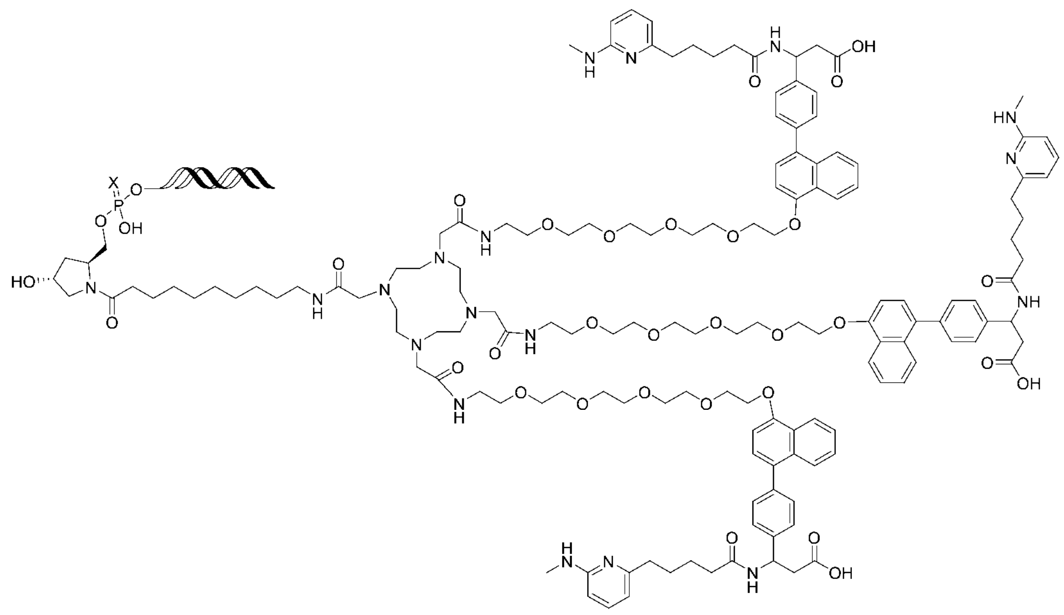 |

(continued)

| | |
|---|---|
| 243-7 | 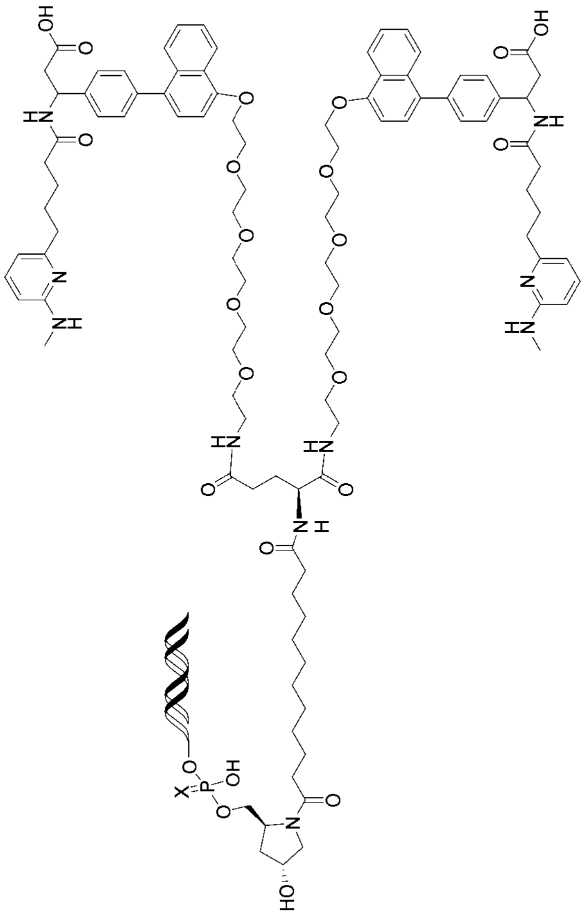 |
| 244-7 | 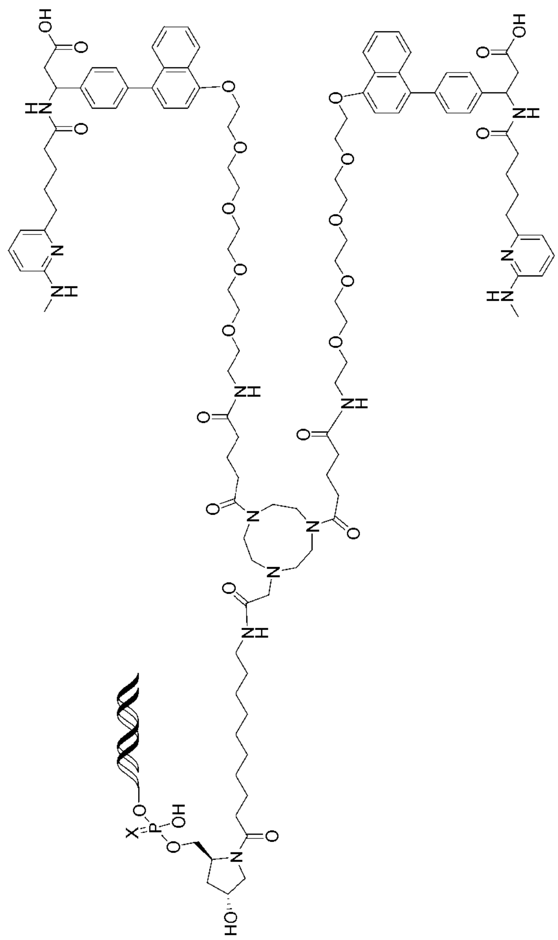 |

(continued)

| | |
|---|---|
| 245-7 | 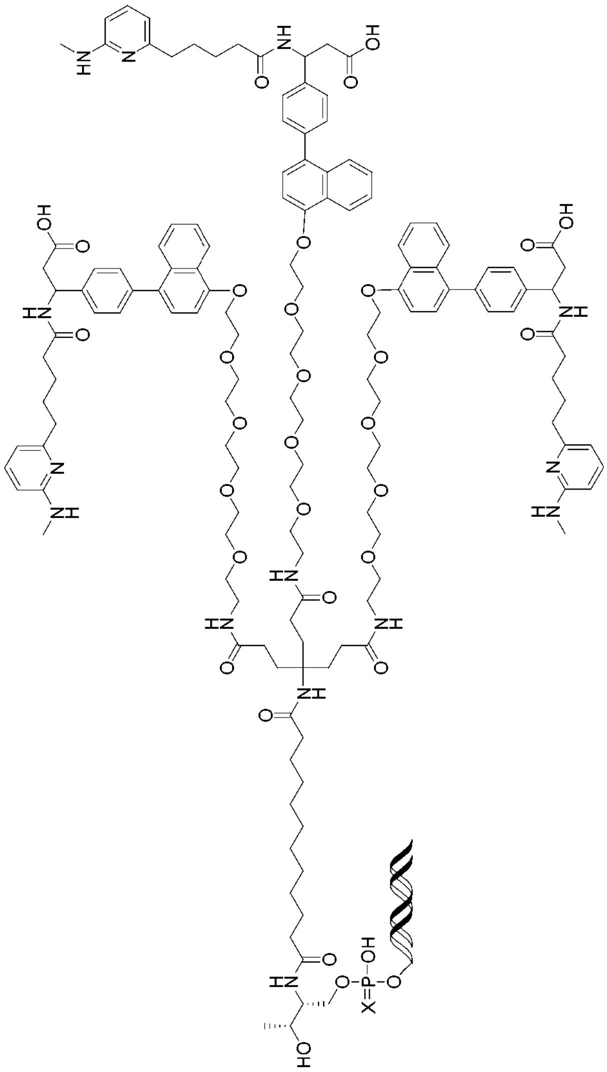 |

(continued)

| | |
|---|---|
| 246-7 | 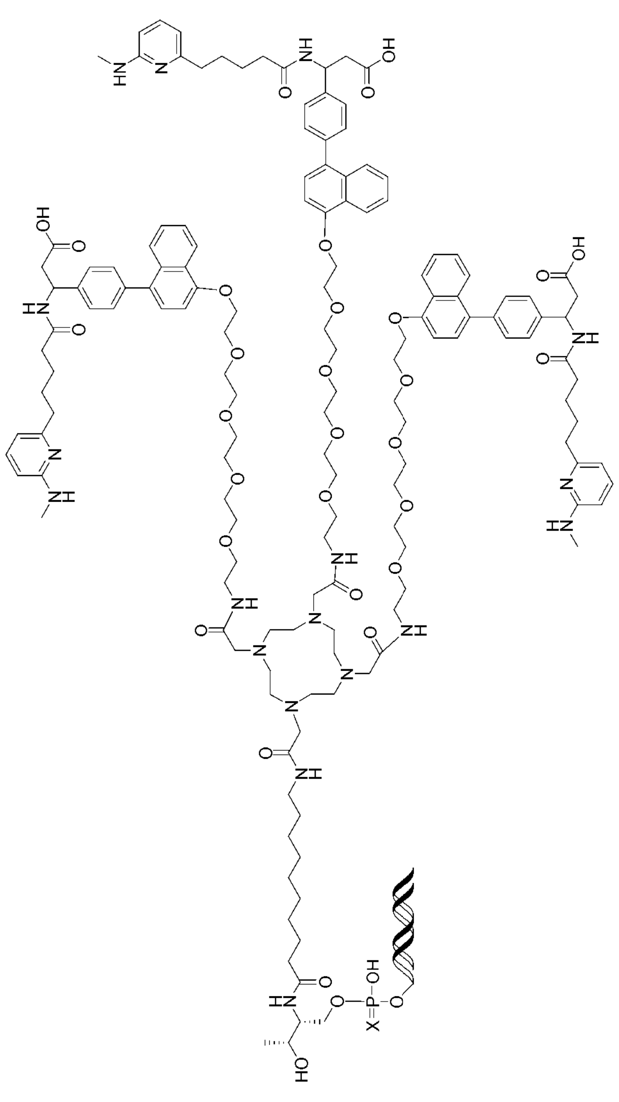 |

(continued)

| | |
|---|---|
| 247-7 | 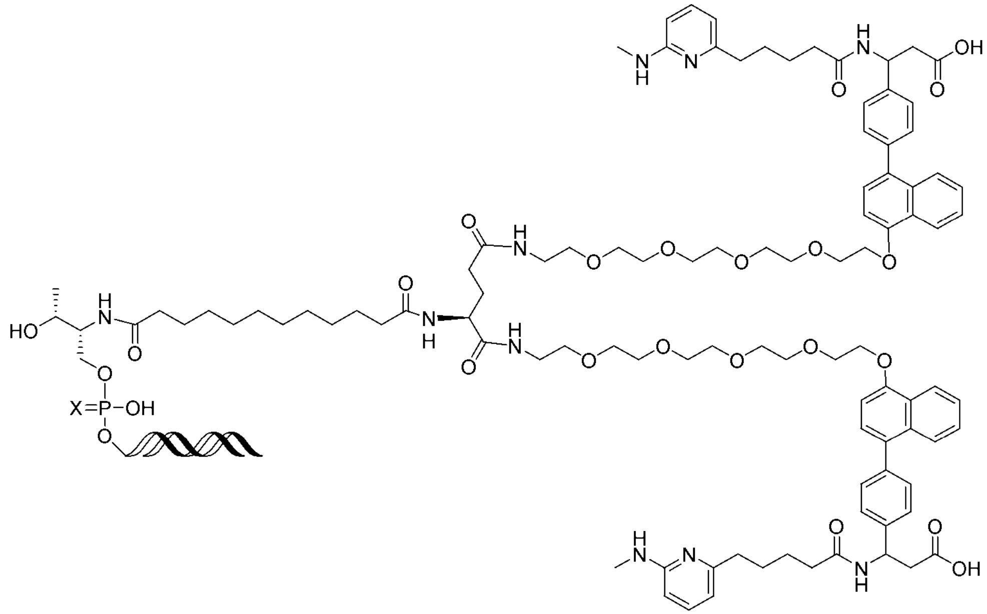 |

(continued)

| | |
|---|---|
| 248-7 | 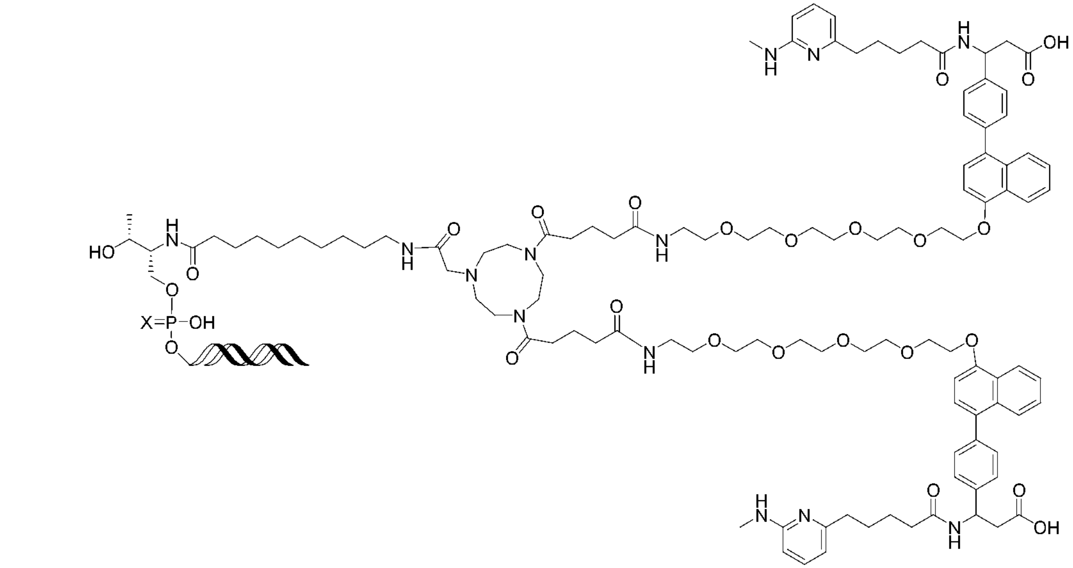 |
| 249-8 | 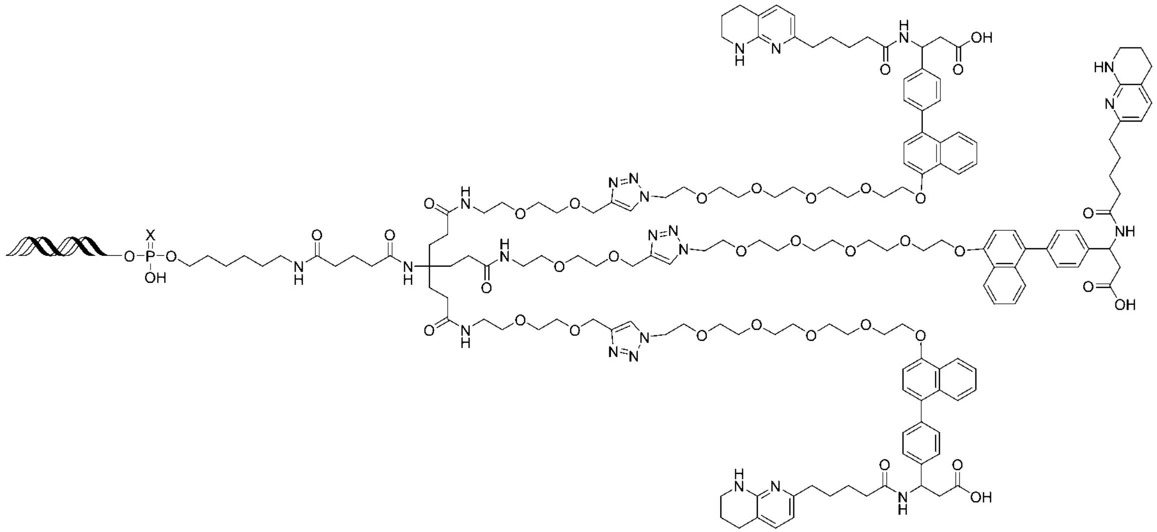 |

(continued)

| | |
|---|---|
| 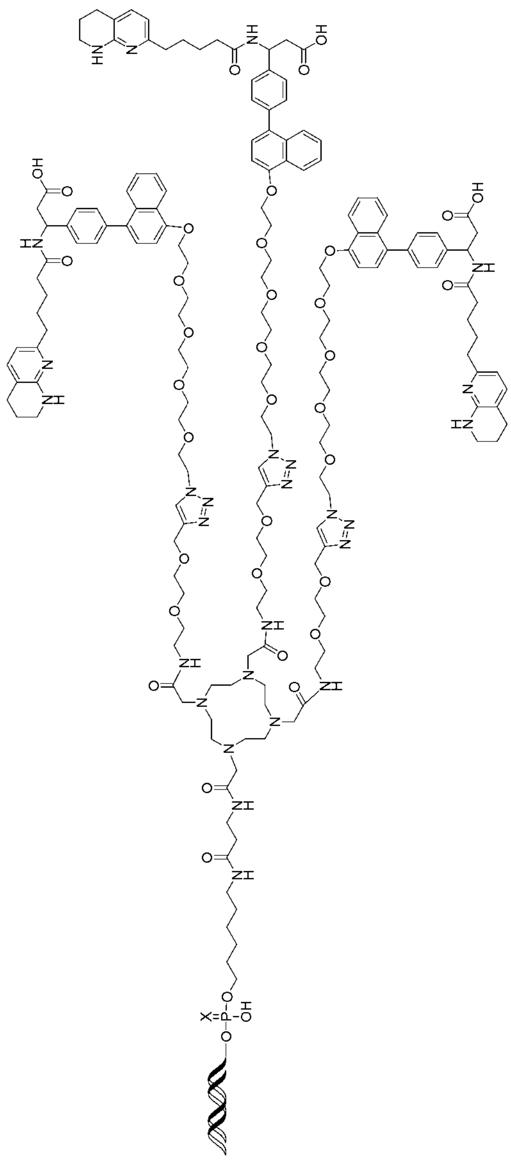 | 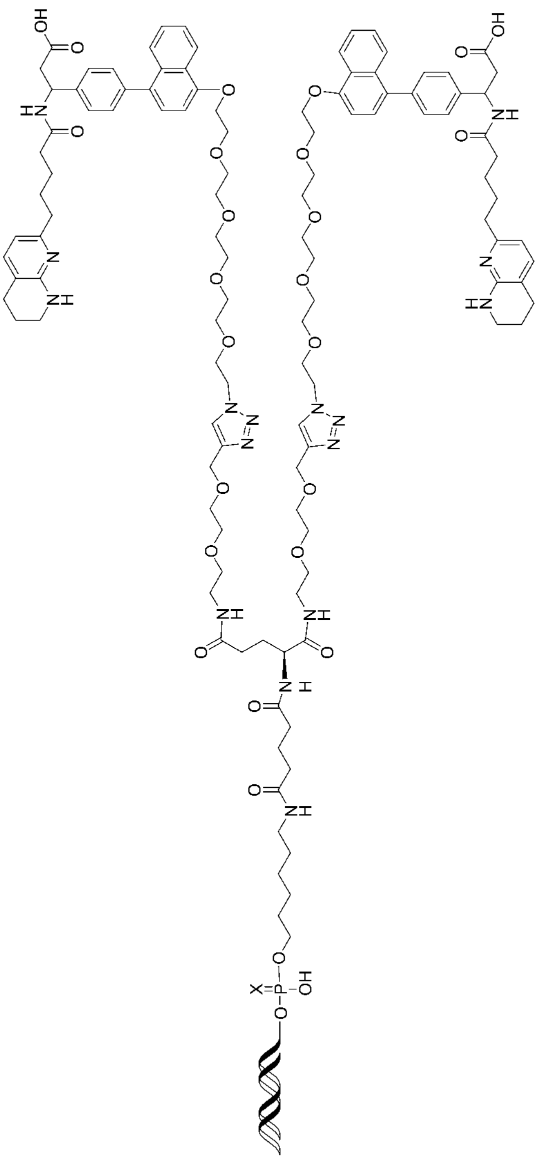 |
| 250-8 | 251-8 |

(continued)

| | |
|---|---|
| 252-8 | 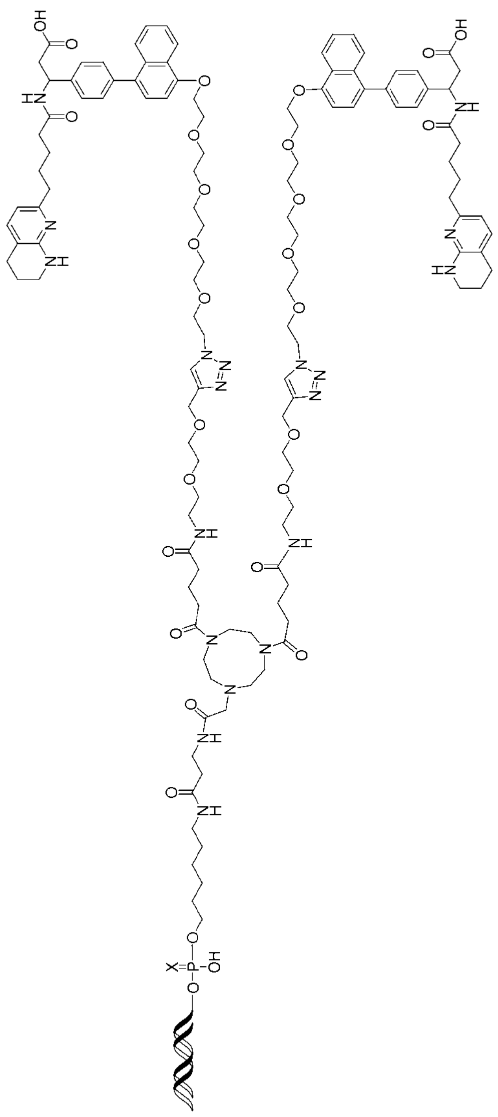 |
| 253-8 | 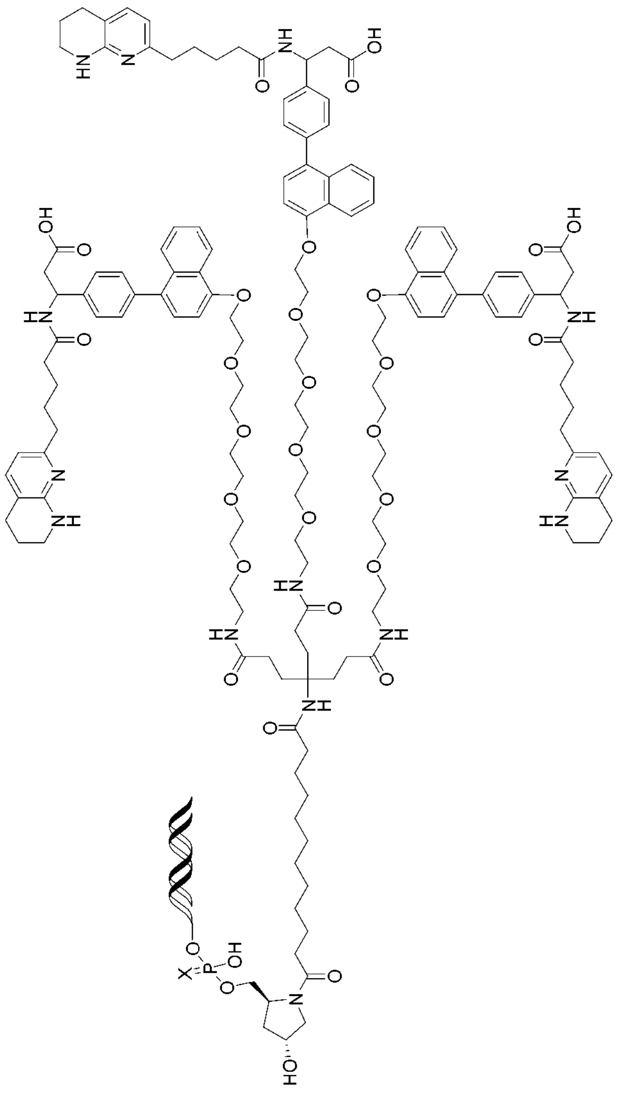 |

(continued)

| | |
|---|---|
| 254-8 | 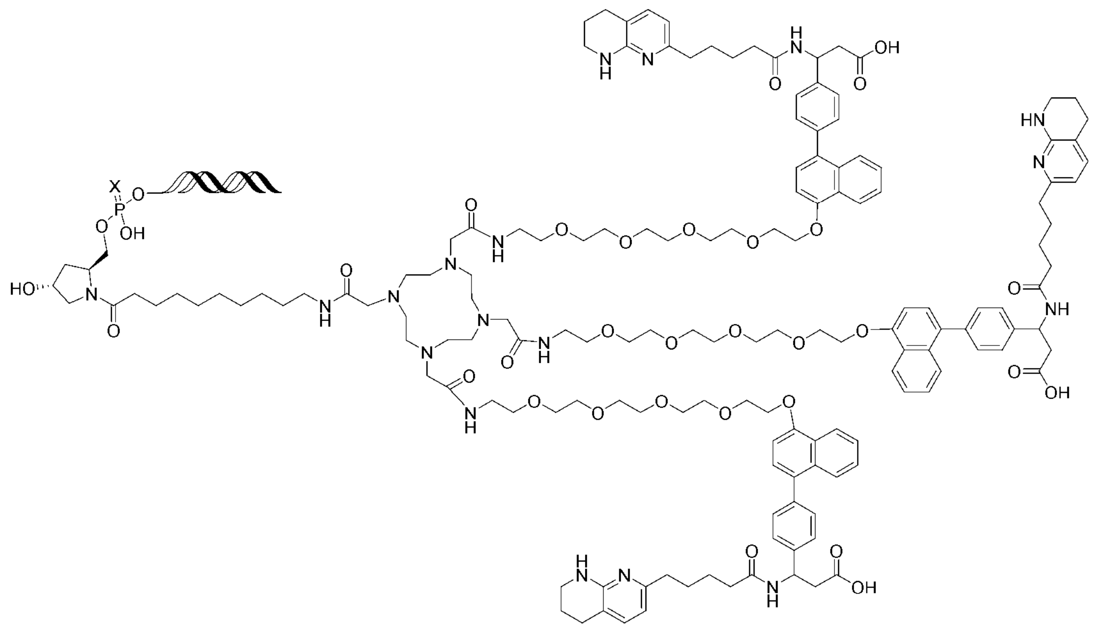 |

(continued)

| | |
|---|---|
| 255-8 | 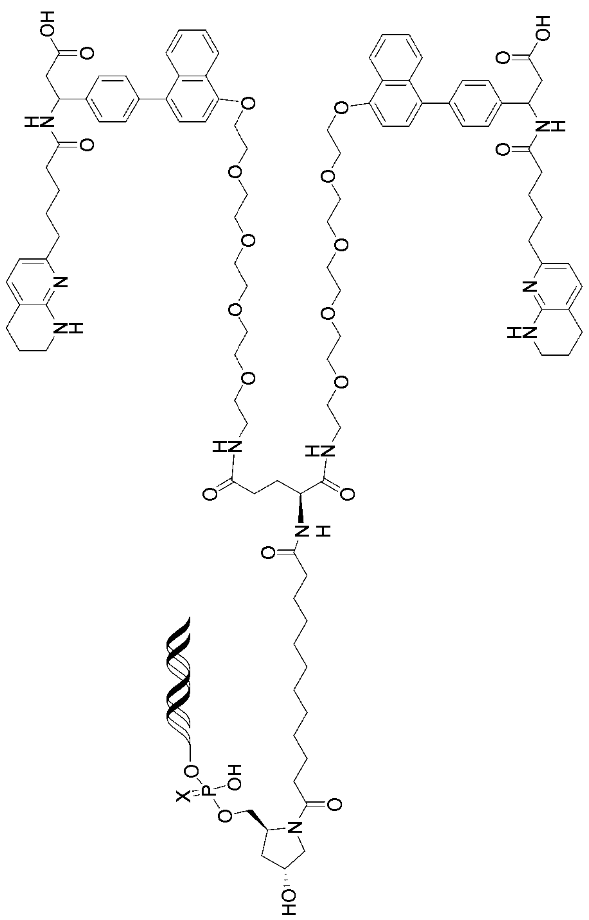 |
| 256-8 | 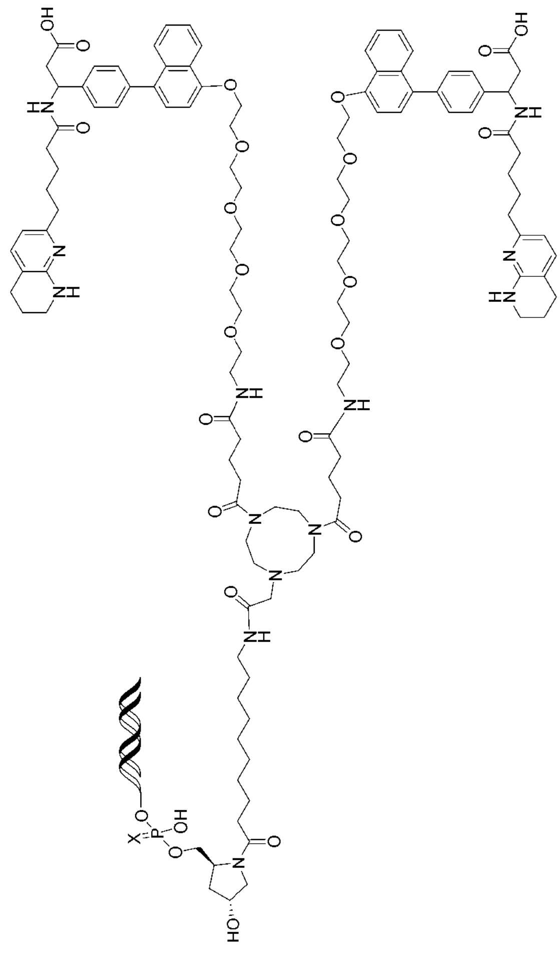 |

(continued)

| | |
|---|---|
| 257-8 | 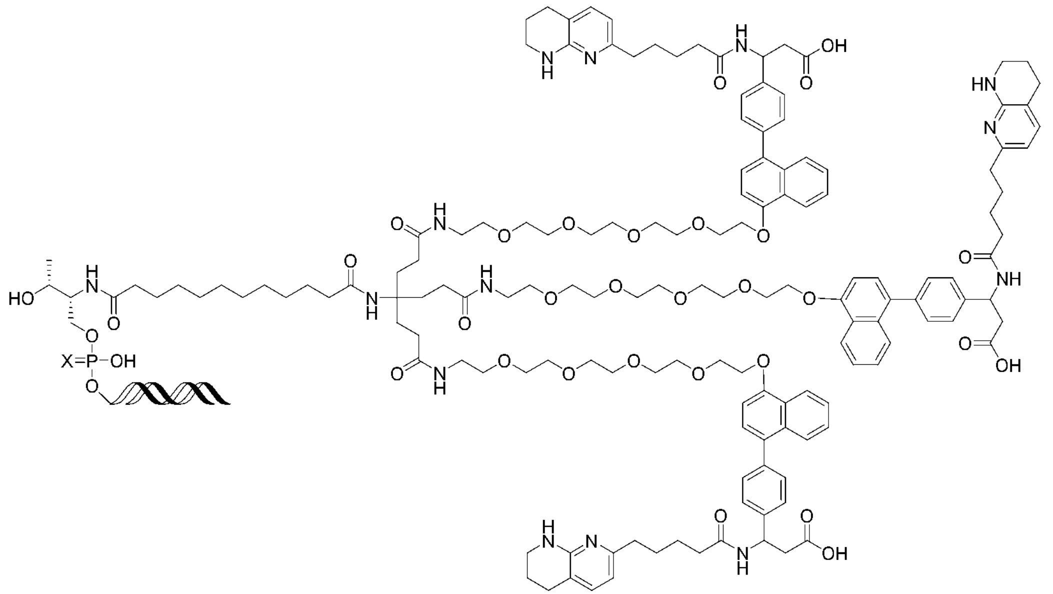 |

(continued)

| | |
|---|---|
| 258-8 | 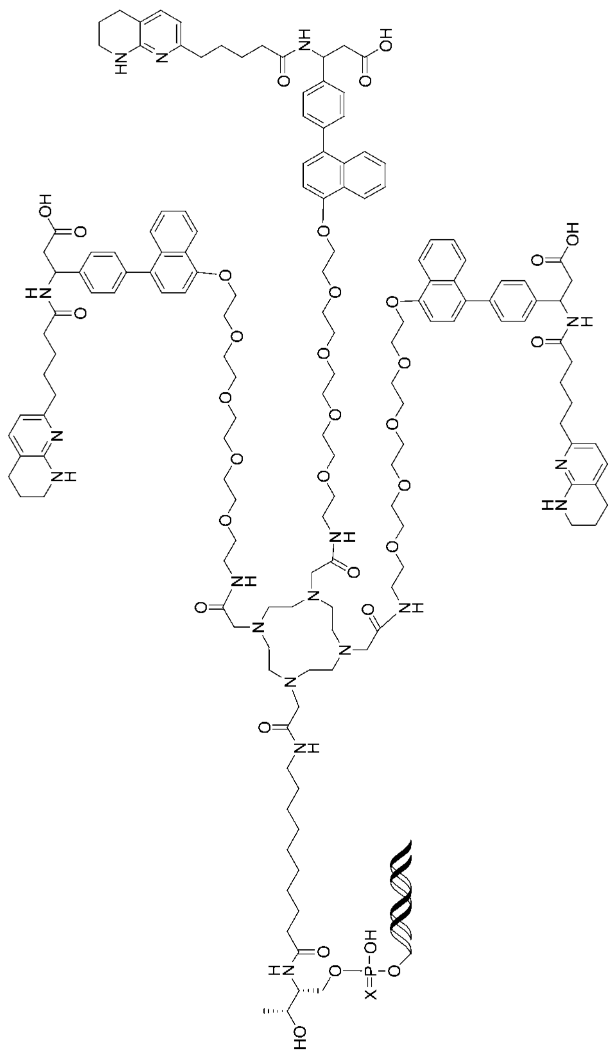 |
| 259-8 | 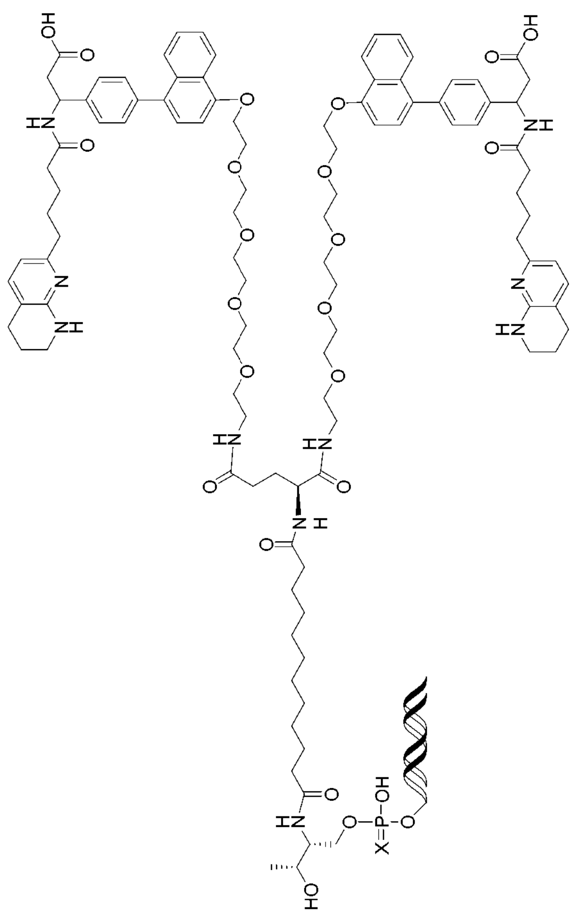 |

(continued)

| | |
|---|---|
| 260-8 | 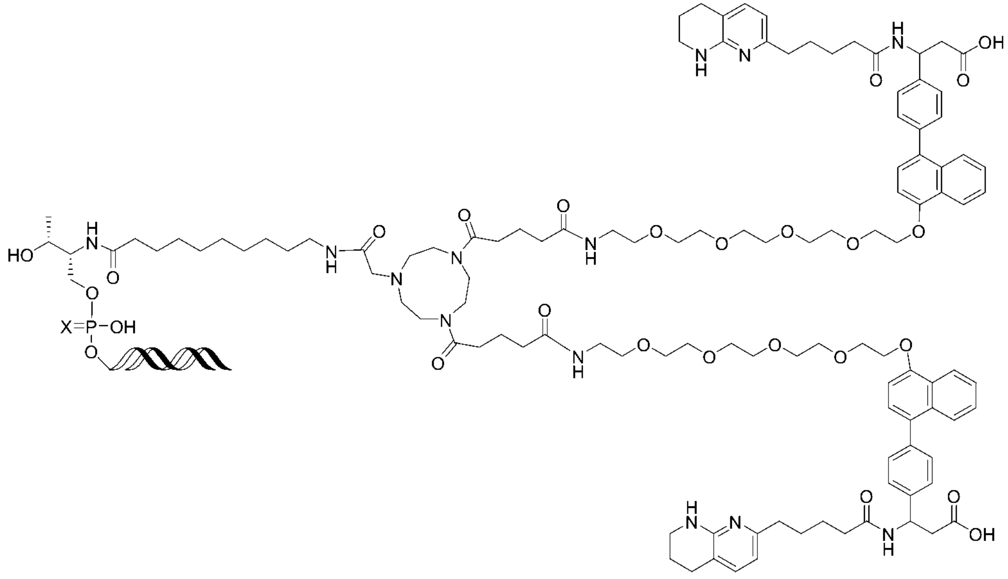 |
| 261-9 | 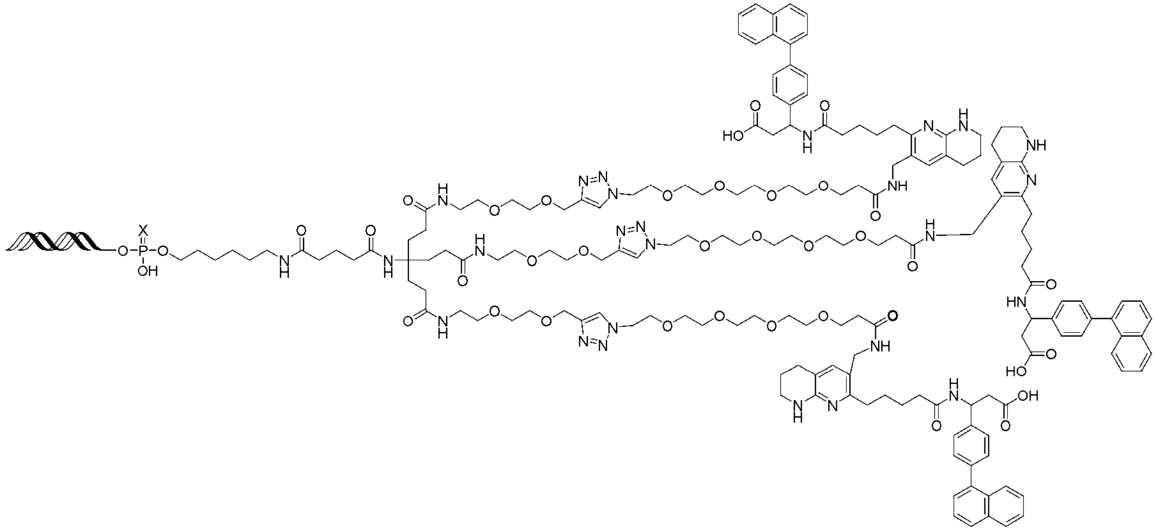 |

(continued)

| | |
|---|---|
| 262-9 | 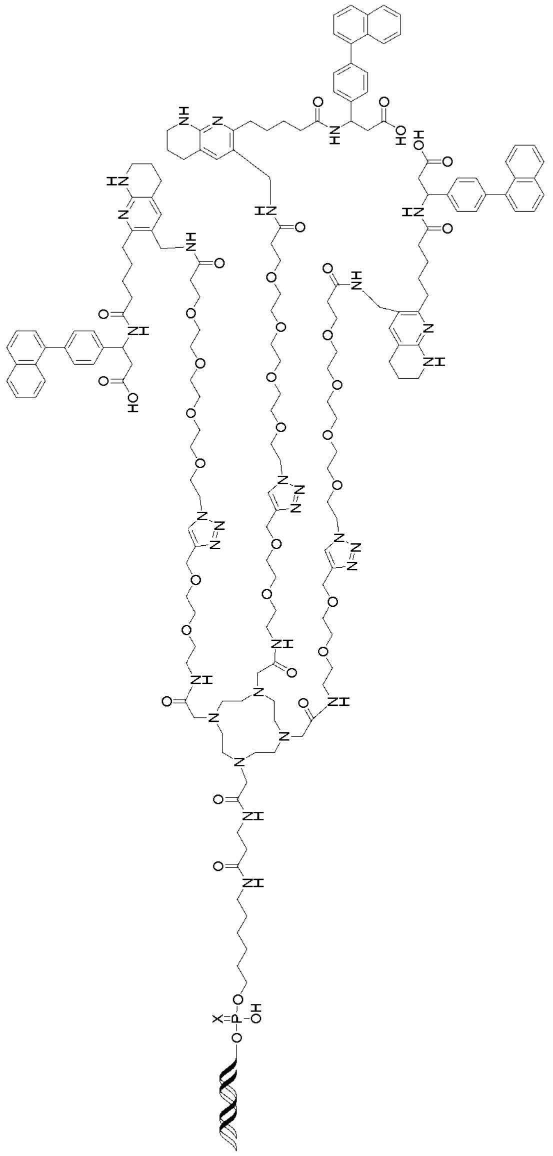 |
| 263-9 | 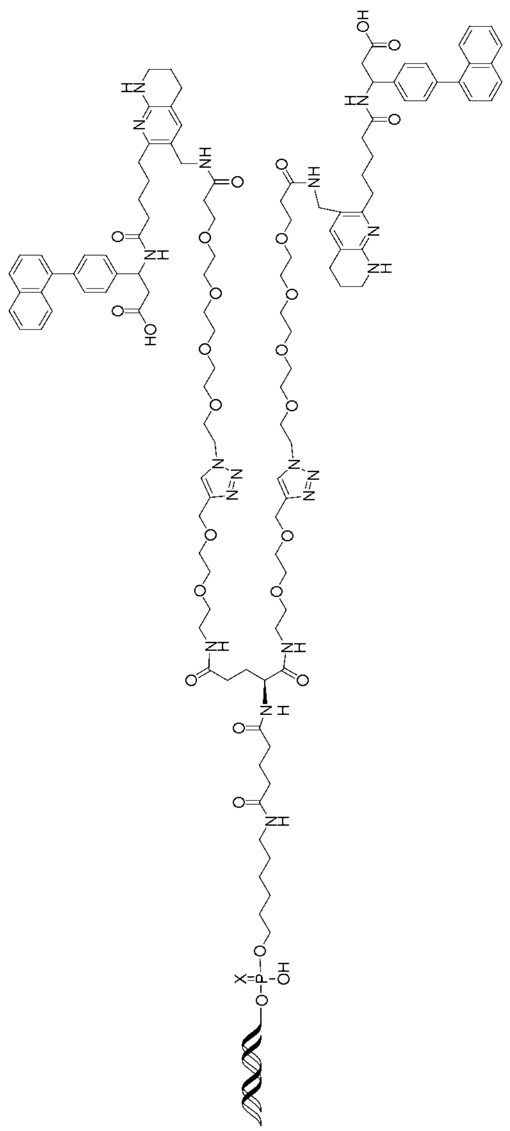 |

(continued)

| | |
|---|---|
| 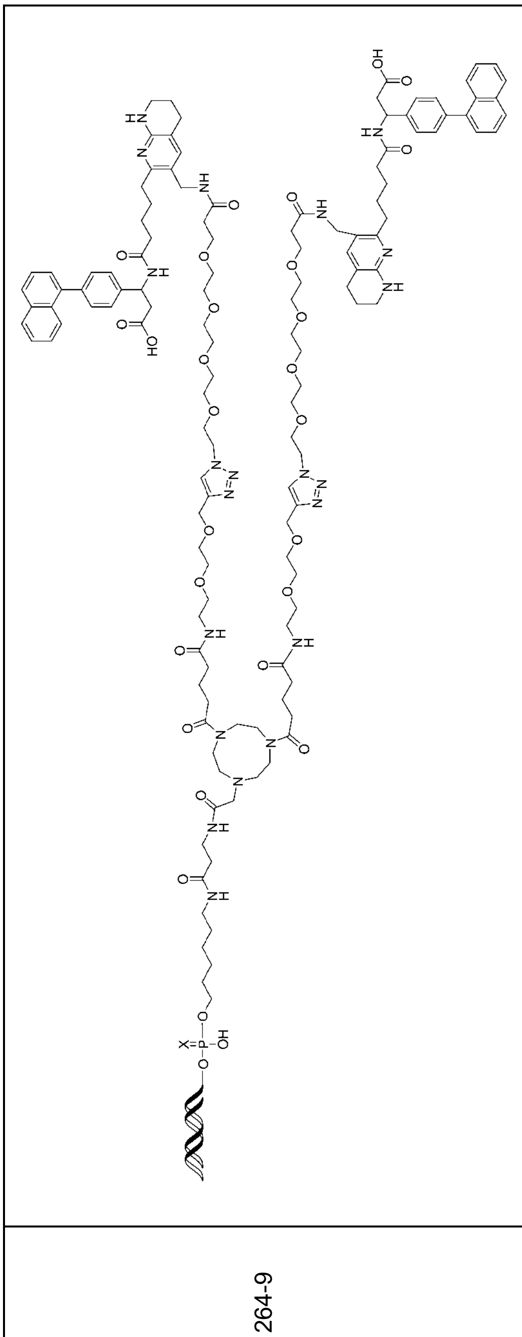 | 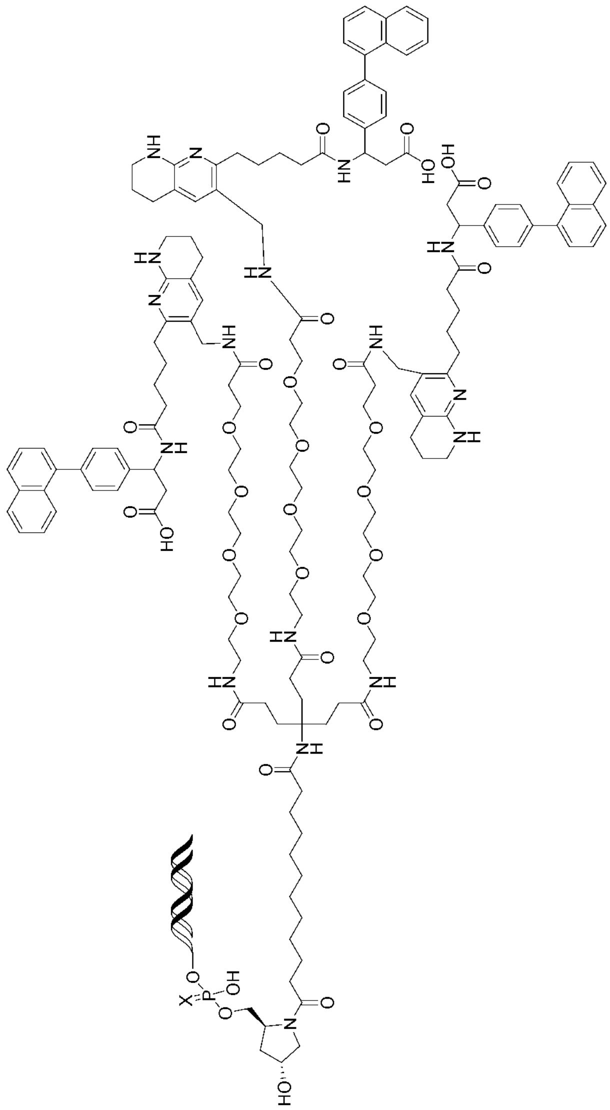 |
| 264-9 | 265-9 |

(continued)

| | |
|---|---|
| 266-9 | 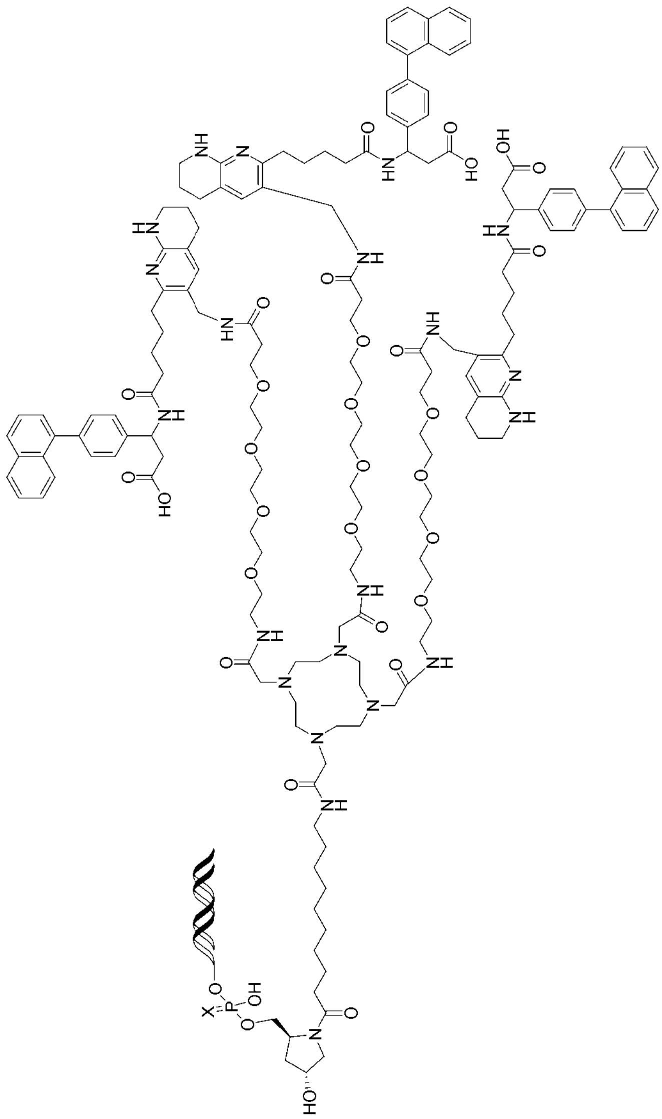 |

(continued)

| | |
|---|---|
| 267-9 | 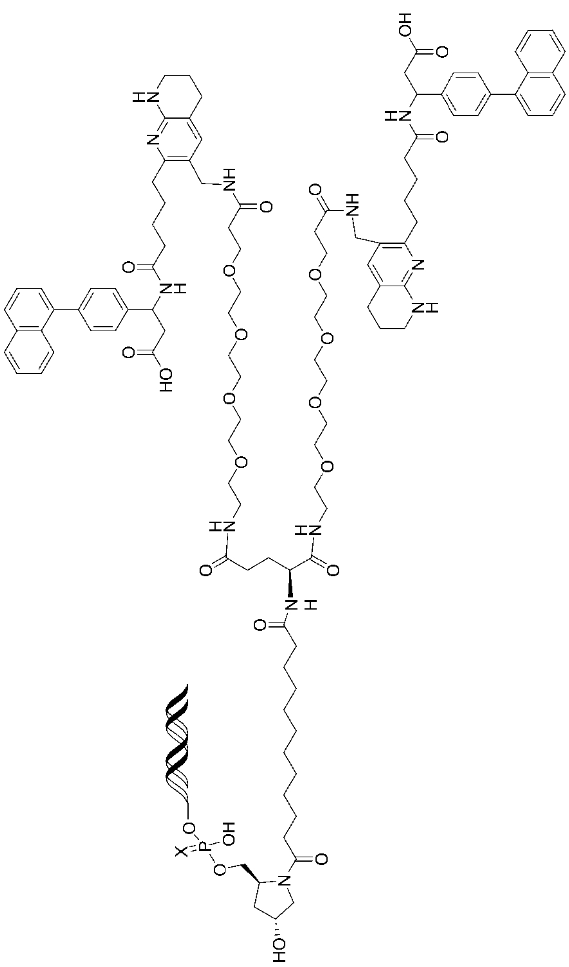 |
| 268-9 | 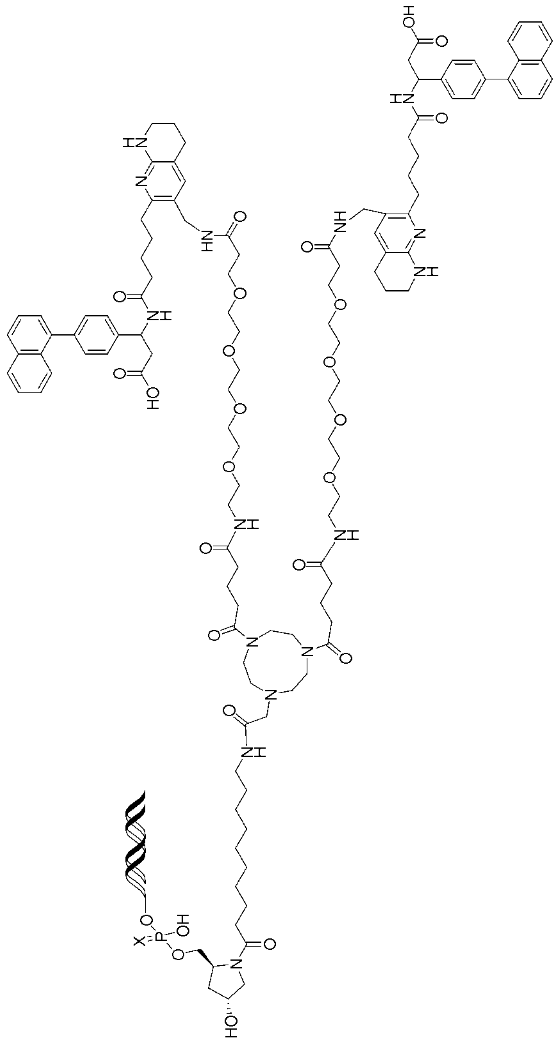 |

(continued)

| | |
|---|---|
| 269-9 | 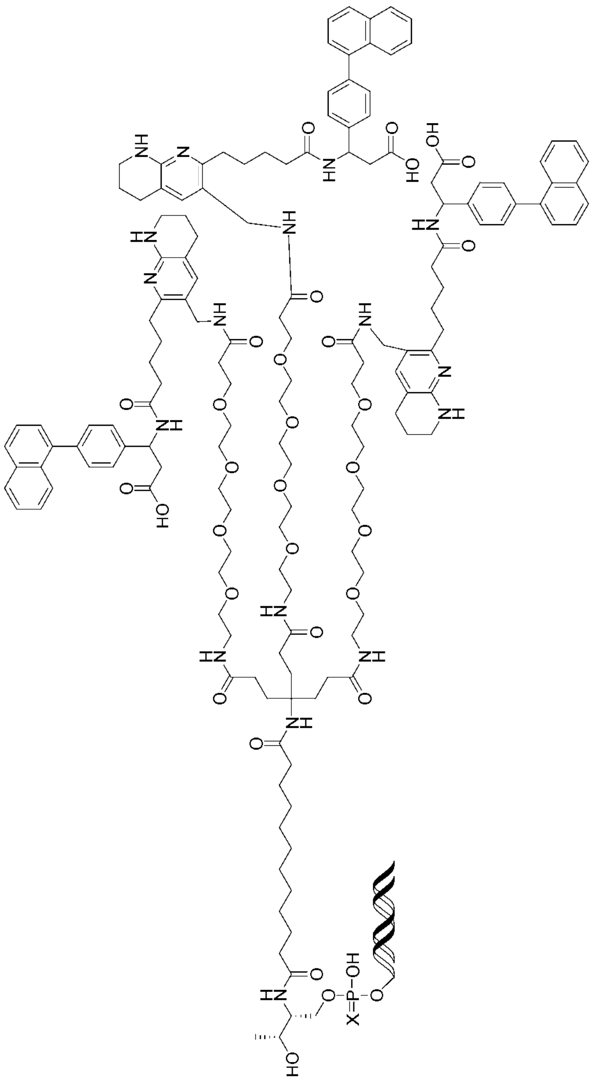 |

(continued)

| | |
|---|---|
| 270-9 | 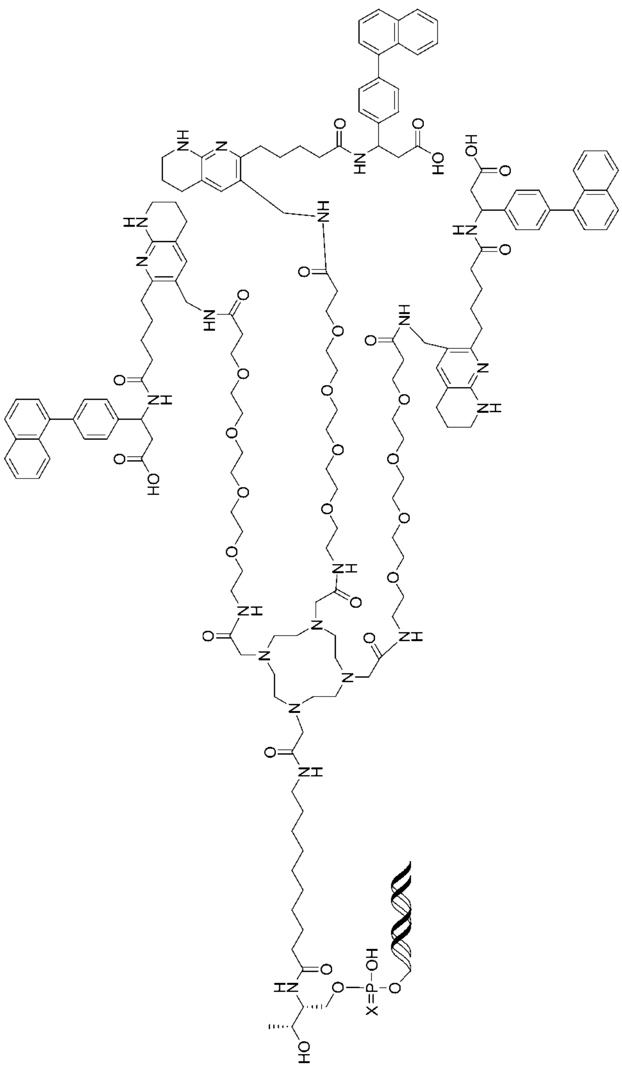 |

(continued)

| | |
|---|---|
| 271-9 | 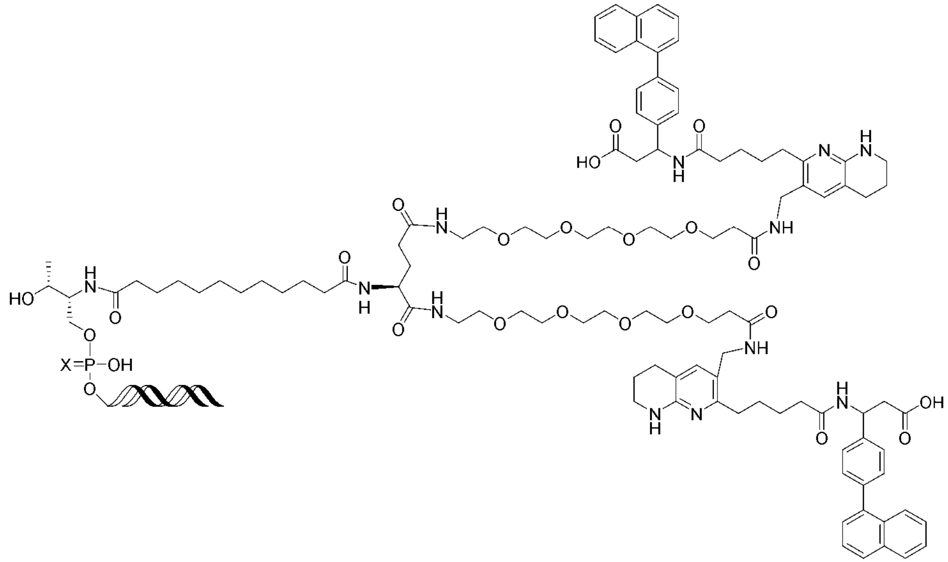 |
| 272-9 | 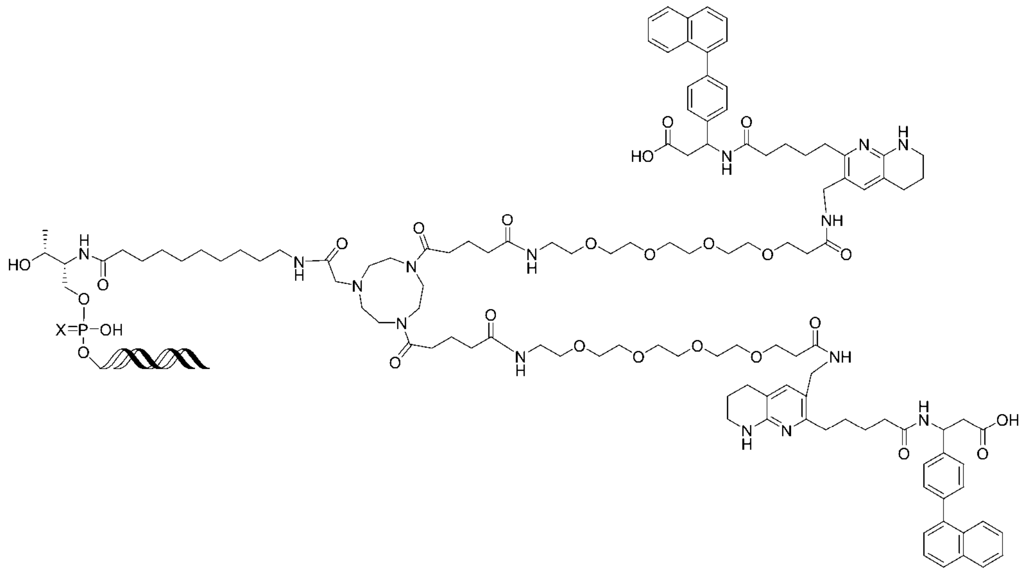 |

(continued)

| | |
|---|---|
| 273-13 | 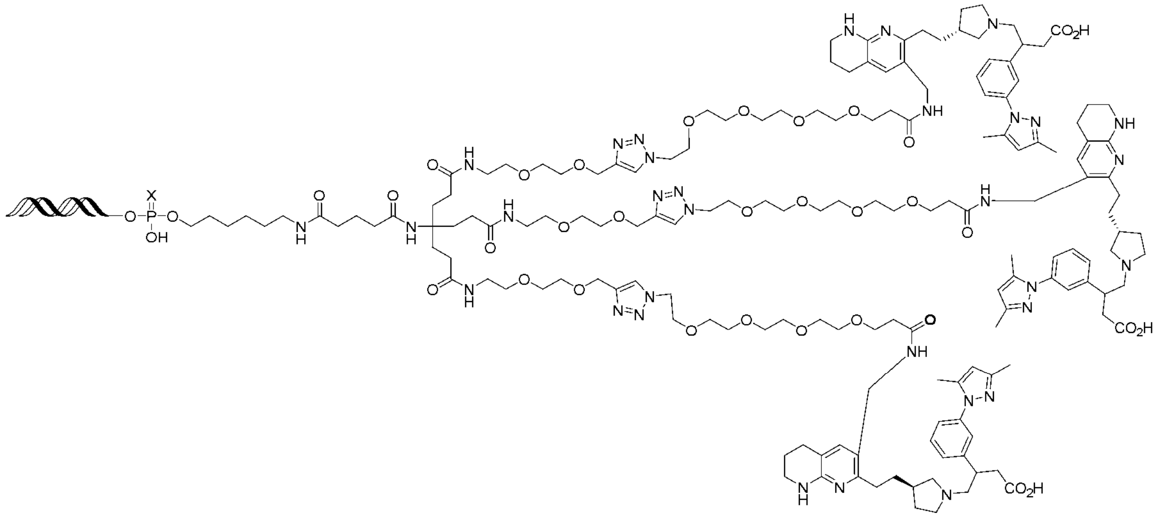 |
| 274-13 | 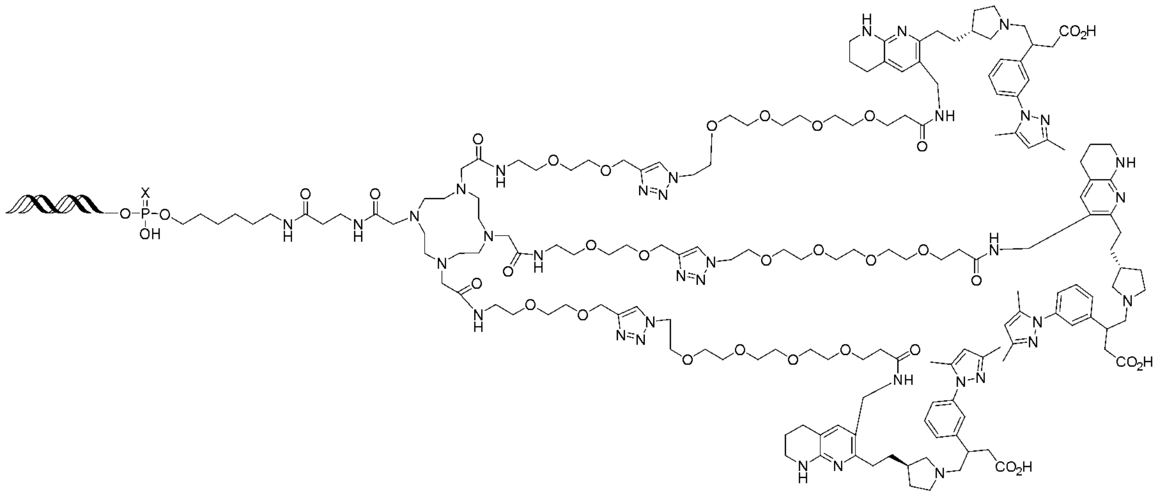 |

(continued)

| | |
|---|---|
| 275-13 | 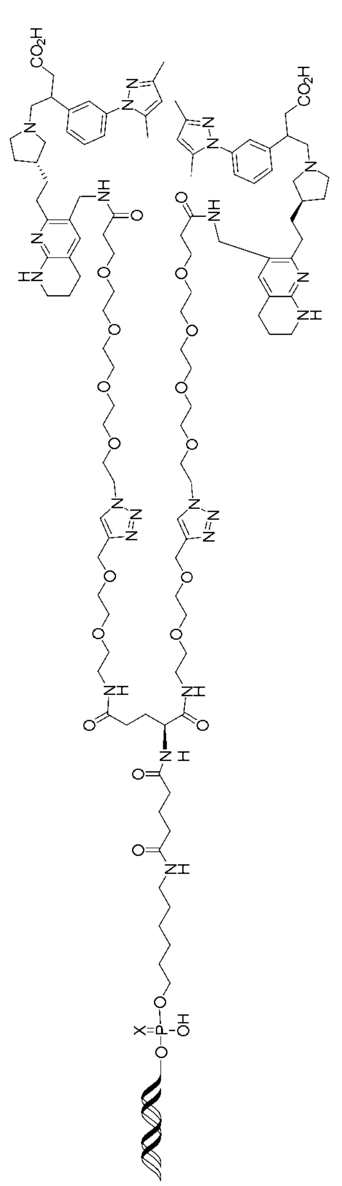 |
| 276-13 | 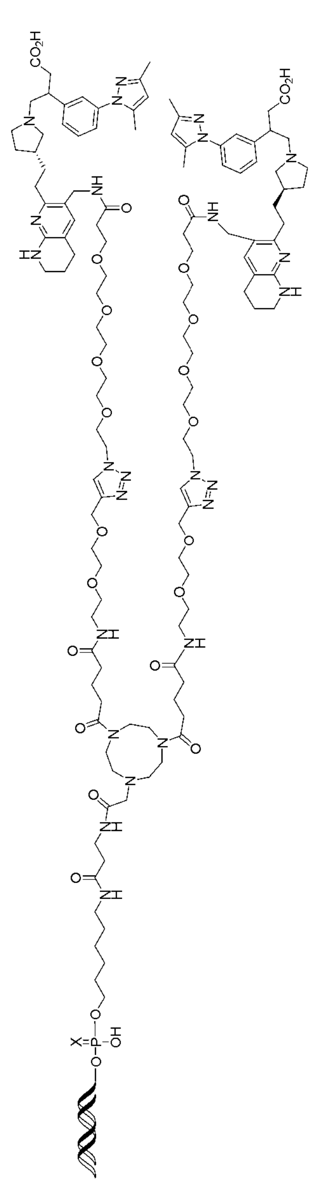 |

(continued)

| | |
|---|---|
| 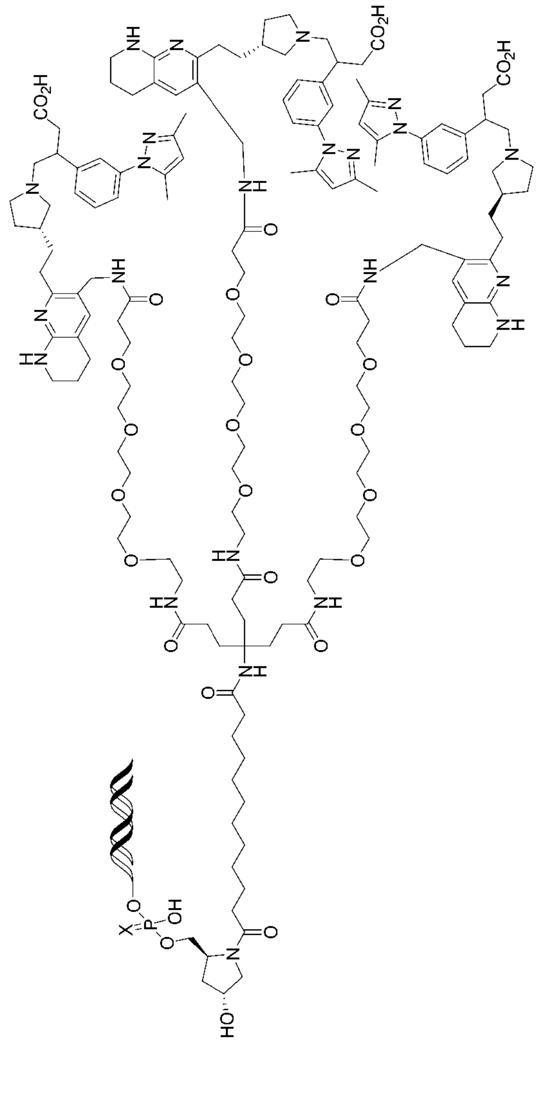 | 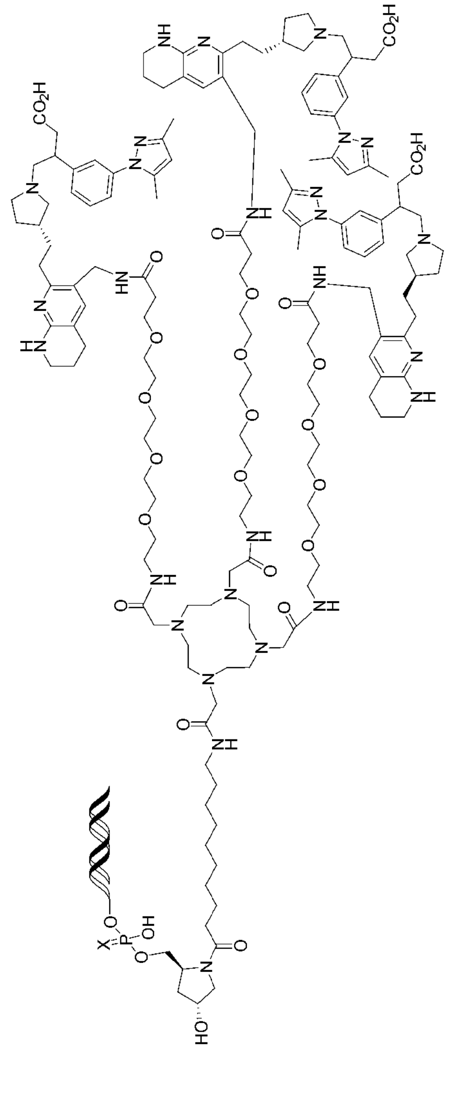 |
| 277-13 | 278-13 |

(continued)

| | |
|---|---|
| 279-13 | 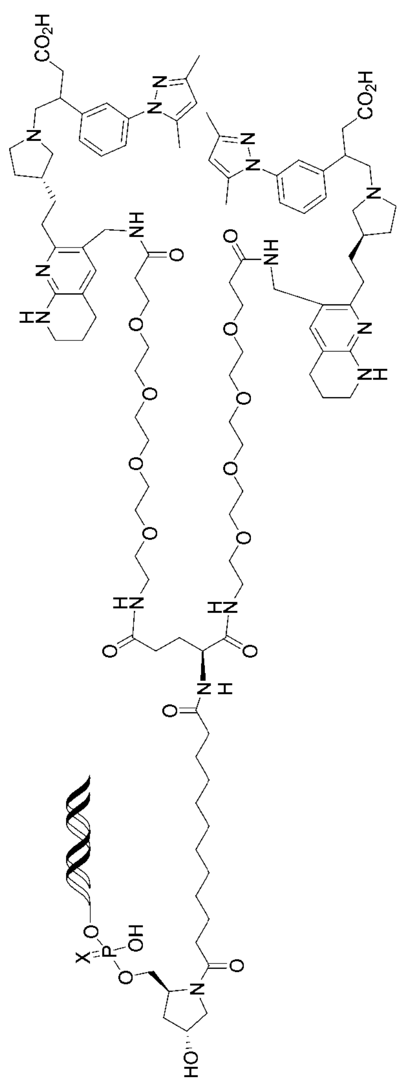 |
| 280-13 | 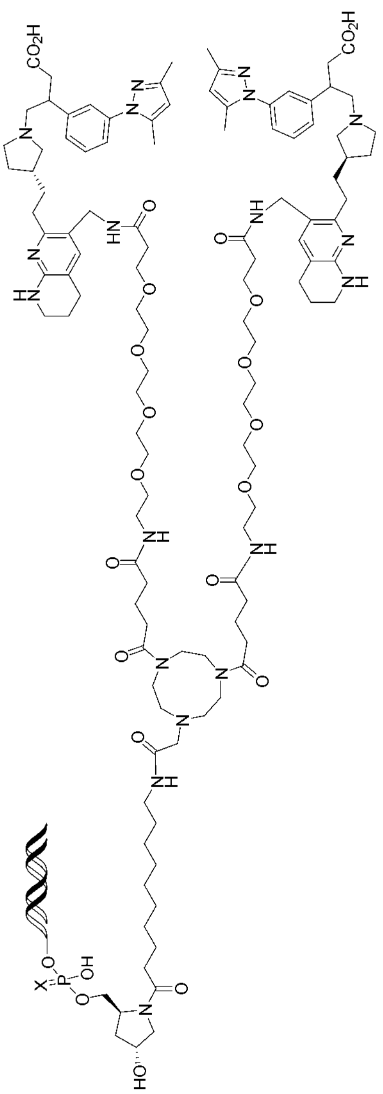 |

(continued)

| | |
|---|---|
| 281-13 | 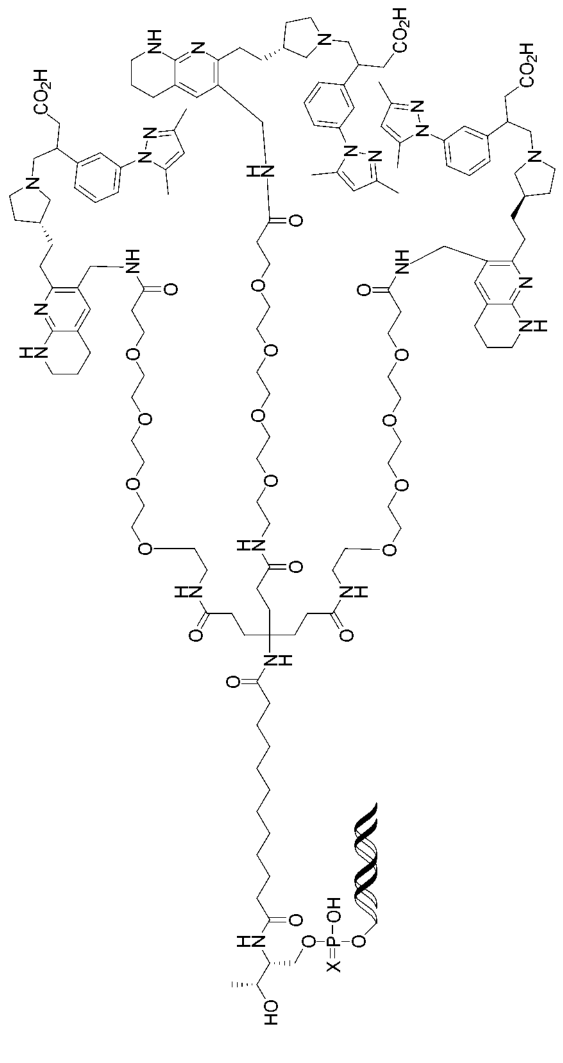 |
| 282-13 | 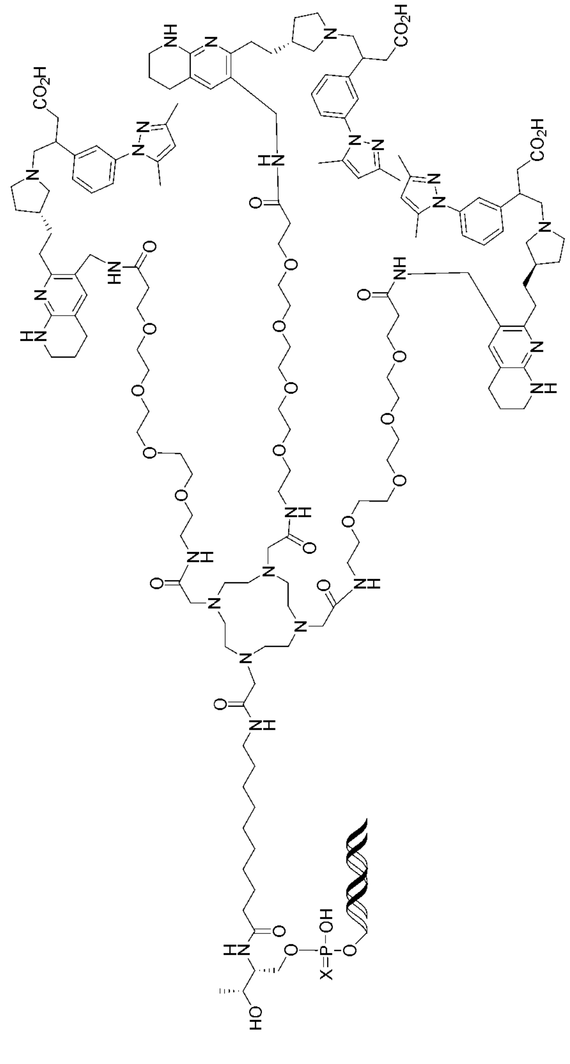 |

(continued)

| | |
|---|---|
| 283-13 | 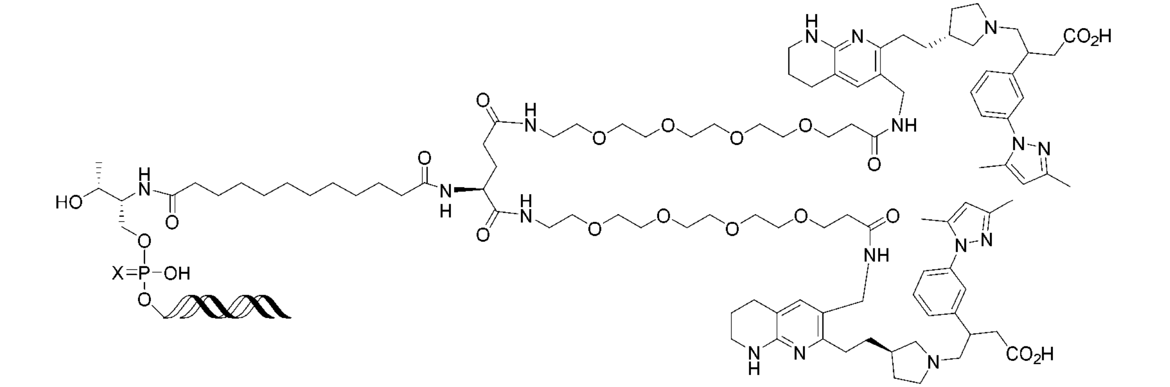 |
| 284-13 | 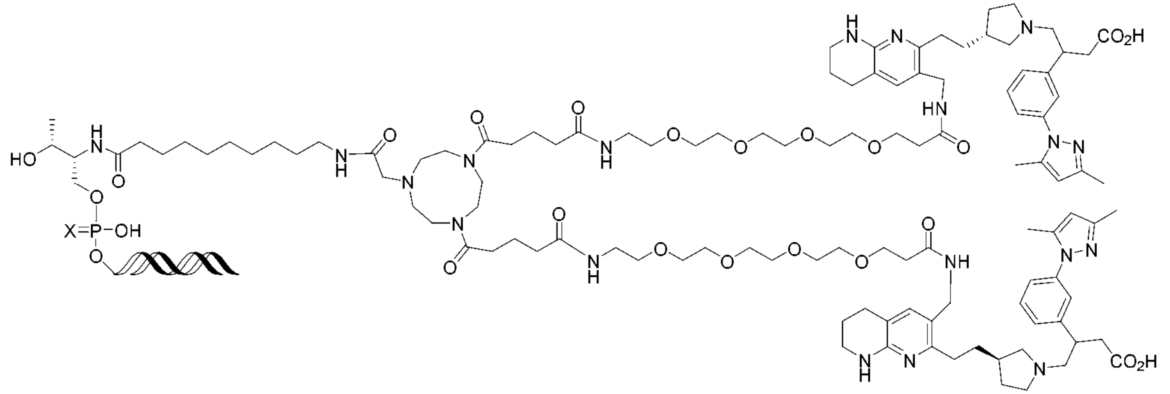 |

(continued)

| | |
|---|---|
| 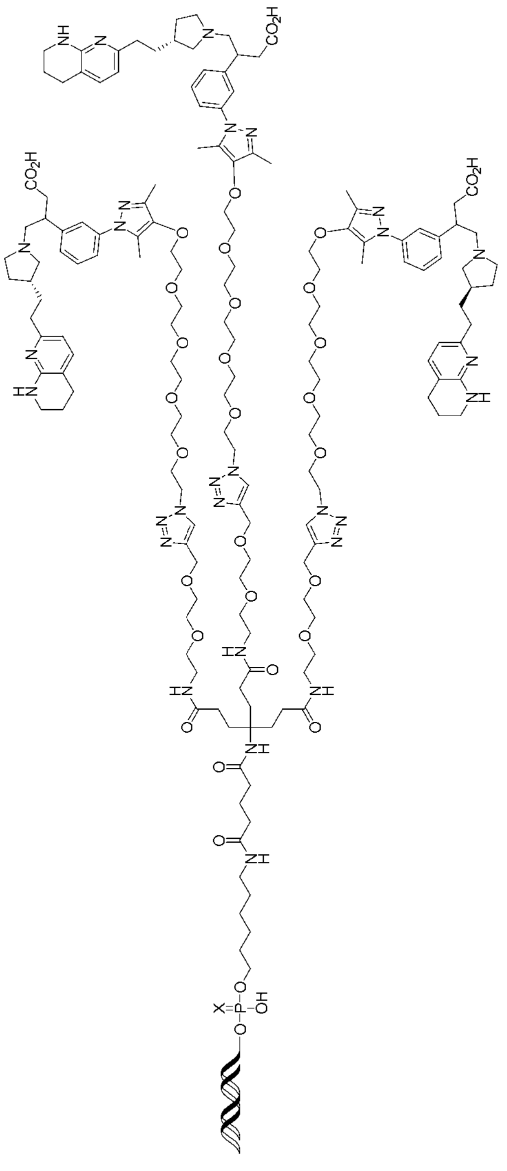 | 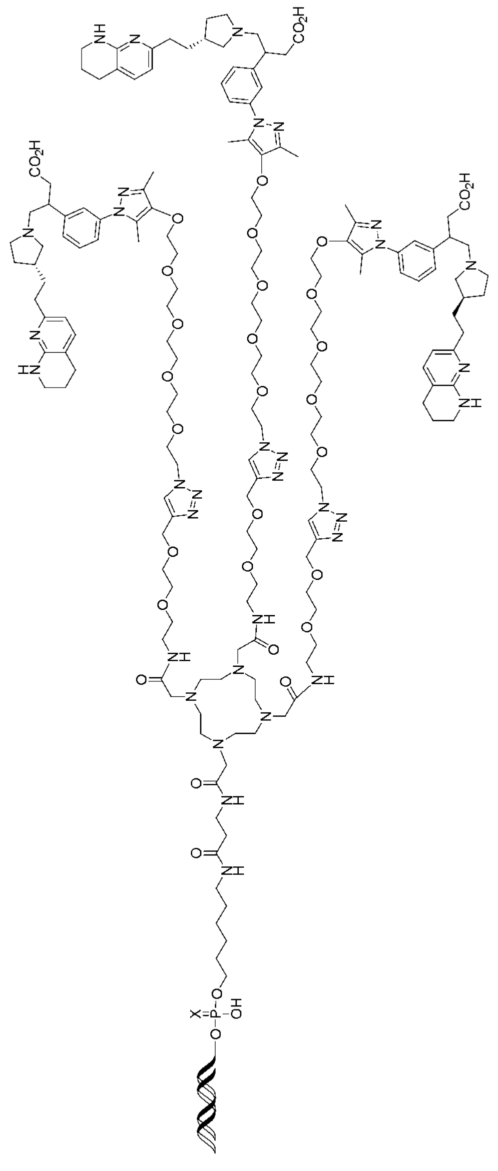 |
| 285-14 | 286-14 |

(continued)

| | |
|---|---|
| 287-14 | 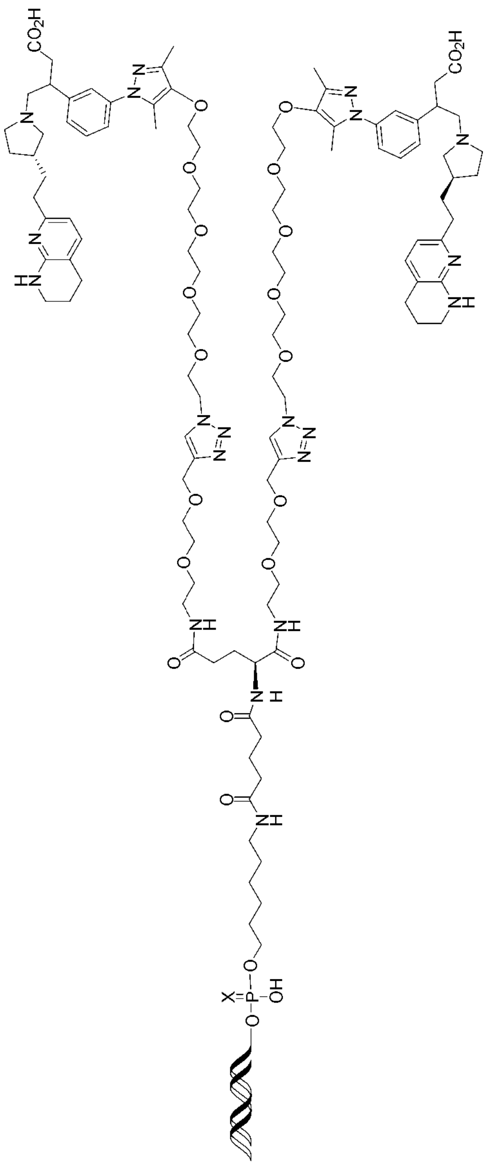 |
| 288-14 | 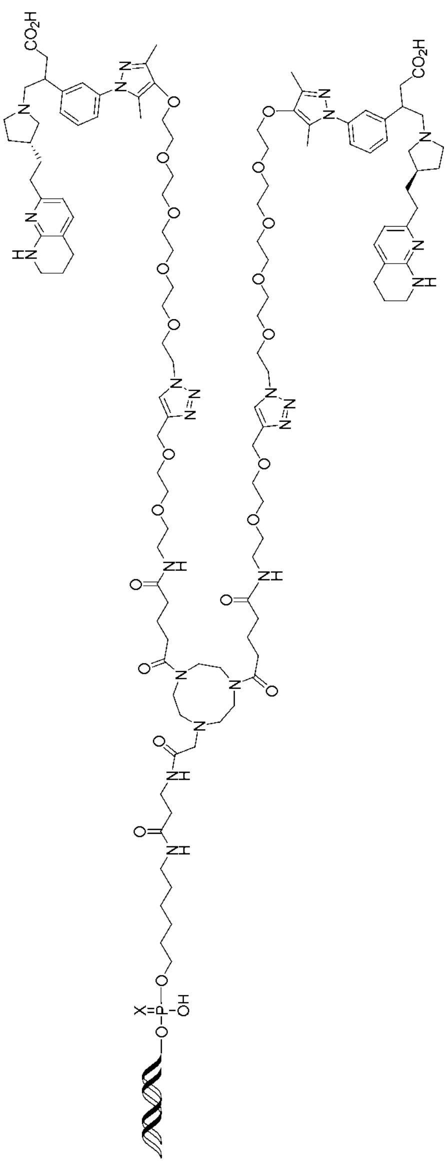 |

(continued)

| | |
|---|---|
| 289-14 | 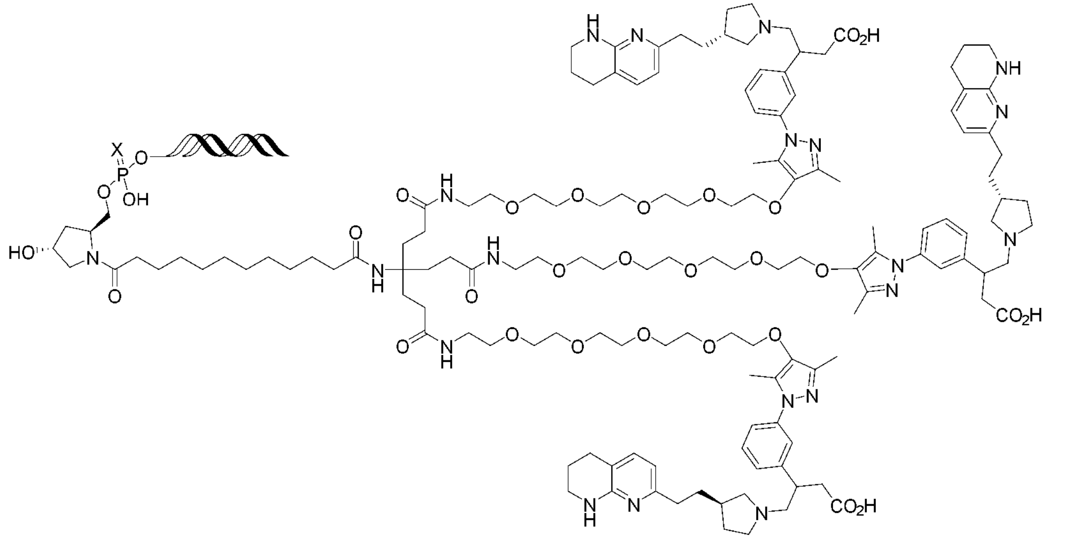 |
| 290-14 | 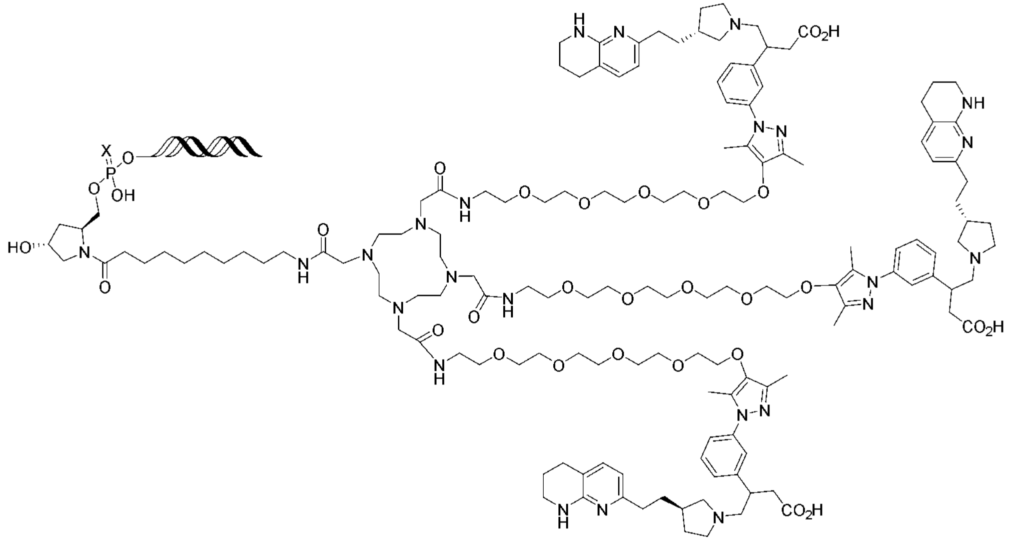 |

(continued)

| | |
|---|---|
| 291-14 | 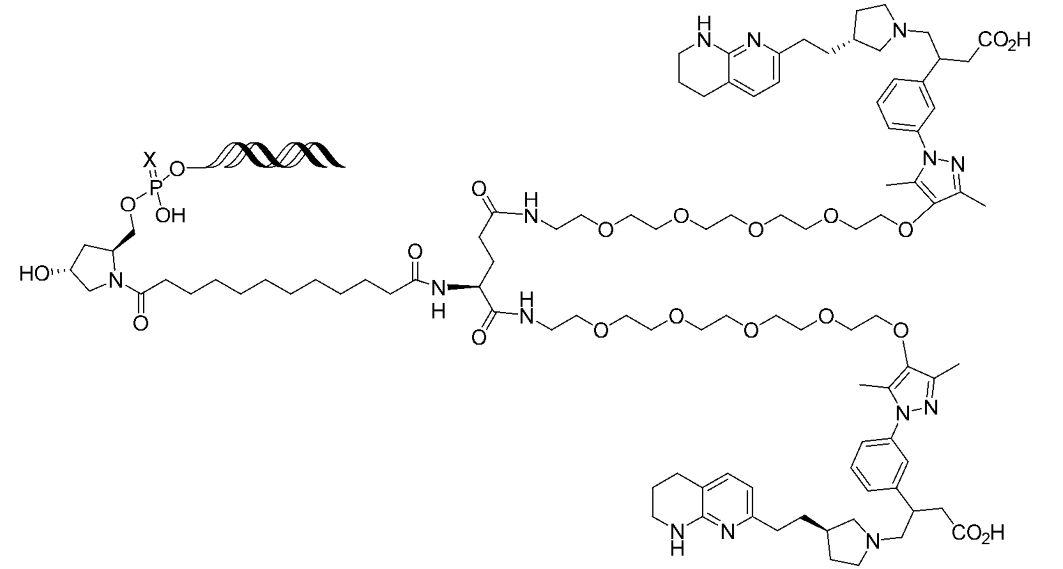 |
| 292-14 | 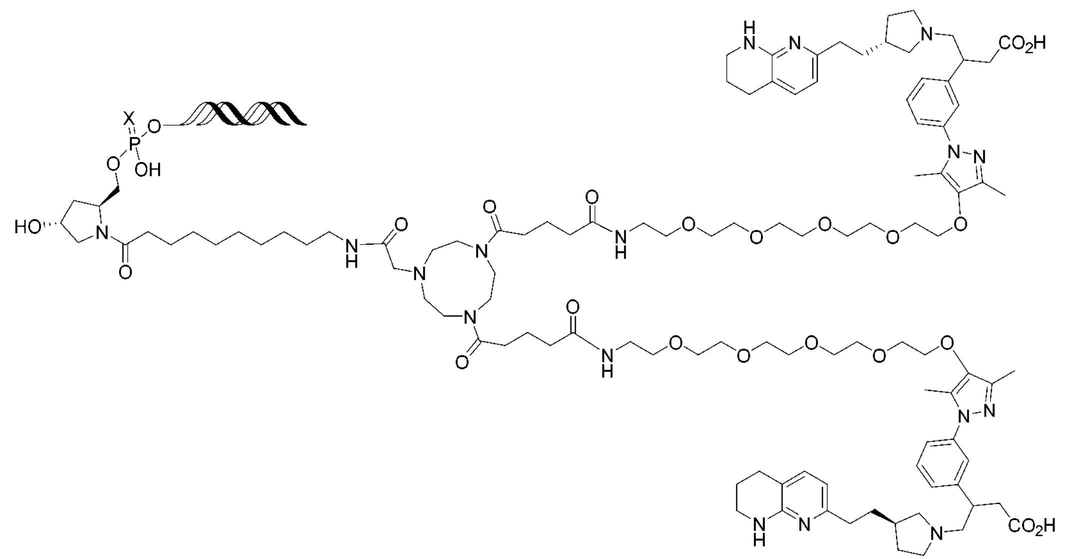 |

(continued)

| | |
|---|---|
| 293-14 | 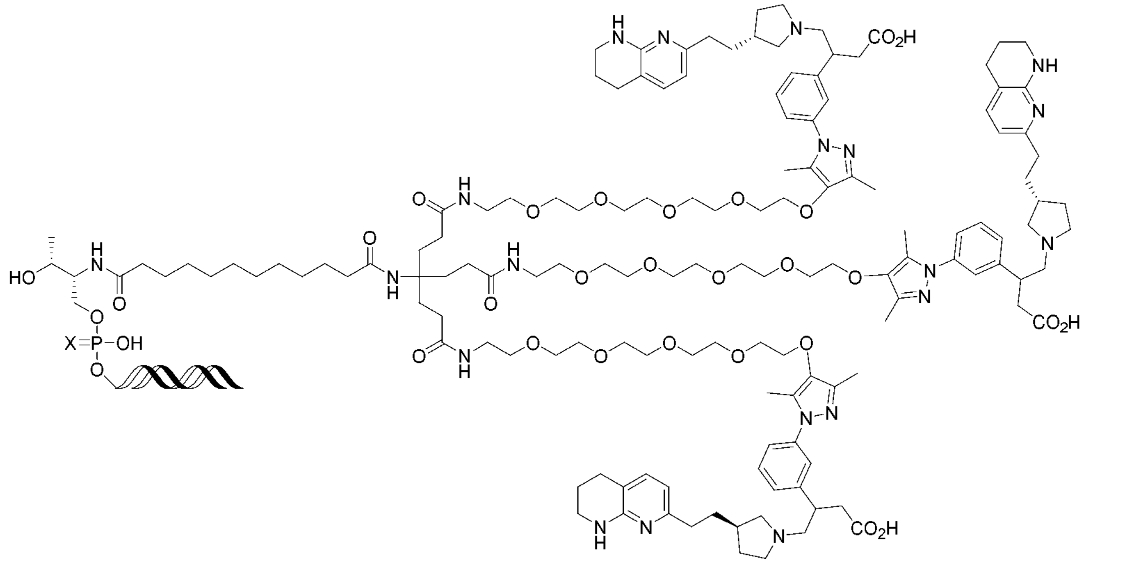 |
| 294-14 | 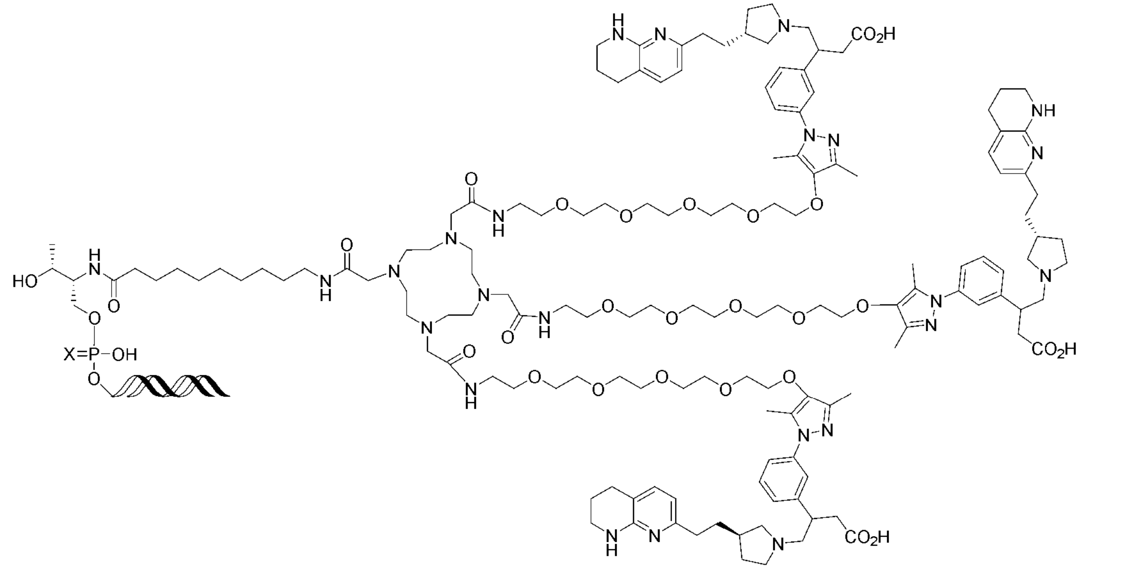 |

(continued)

| | |
|---|---|
| 295-14 | 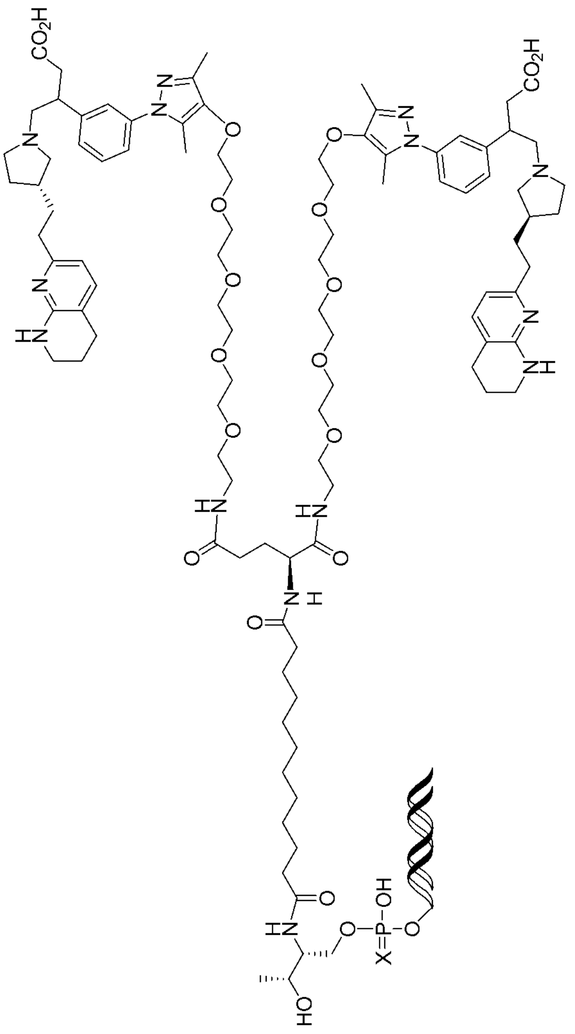 |
| 296-14 | 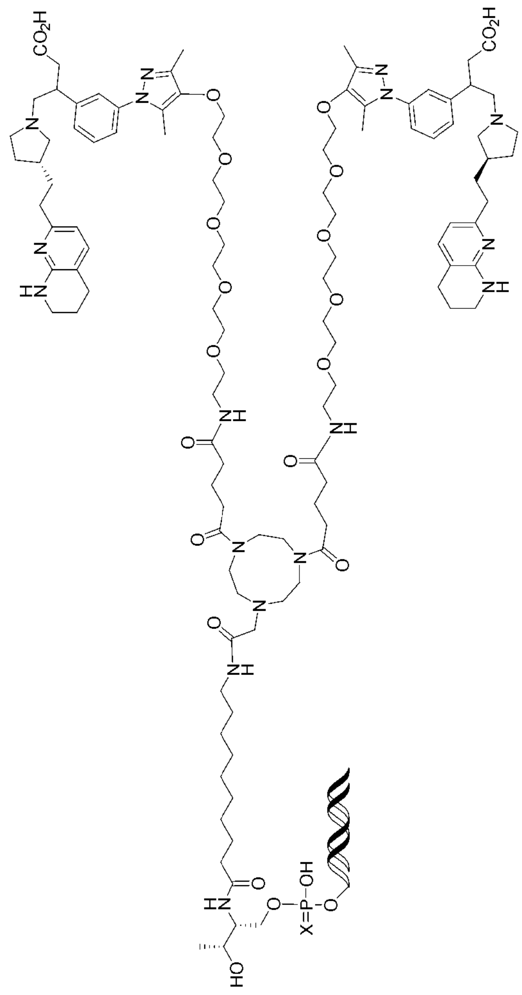 |

(continued)

| | |
|---|---|
| 297-15 | 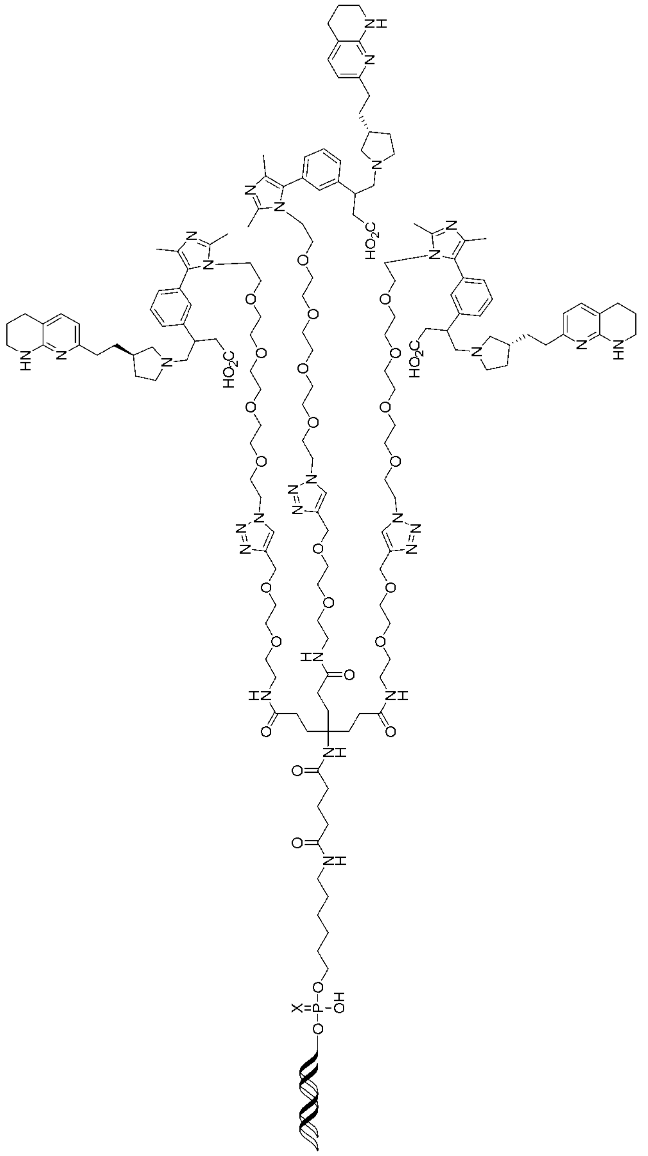 |

(continued)

| | |
|---|---|
| 298-15 | 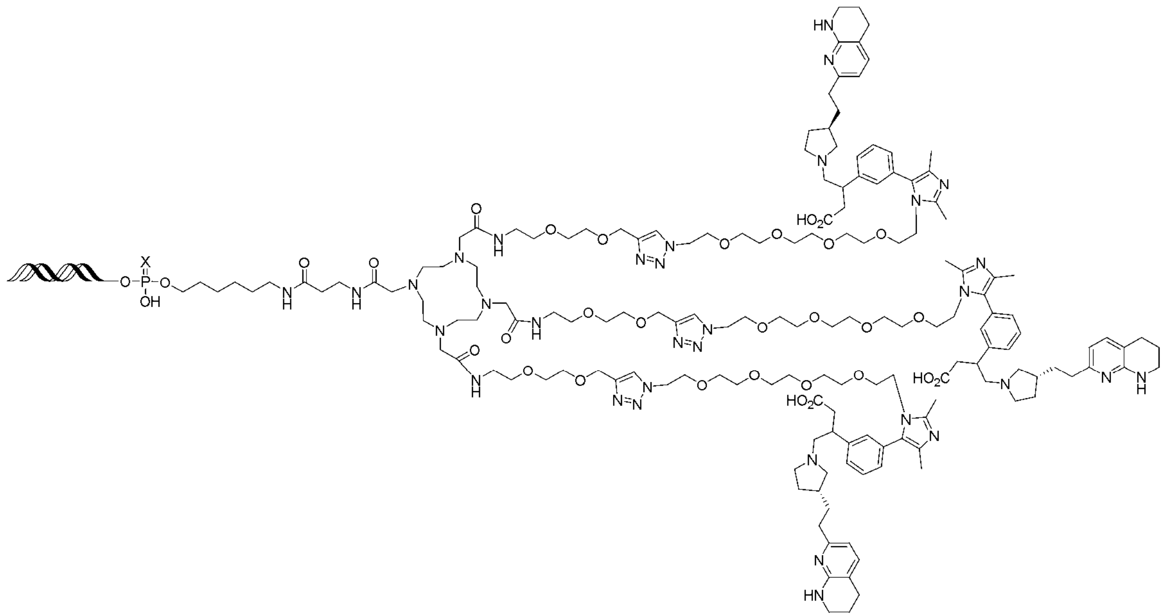 |

(continued)

| | |
|---|---|
| 299-15 | 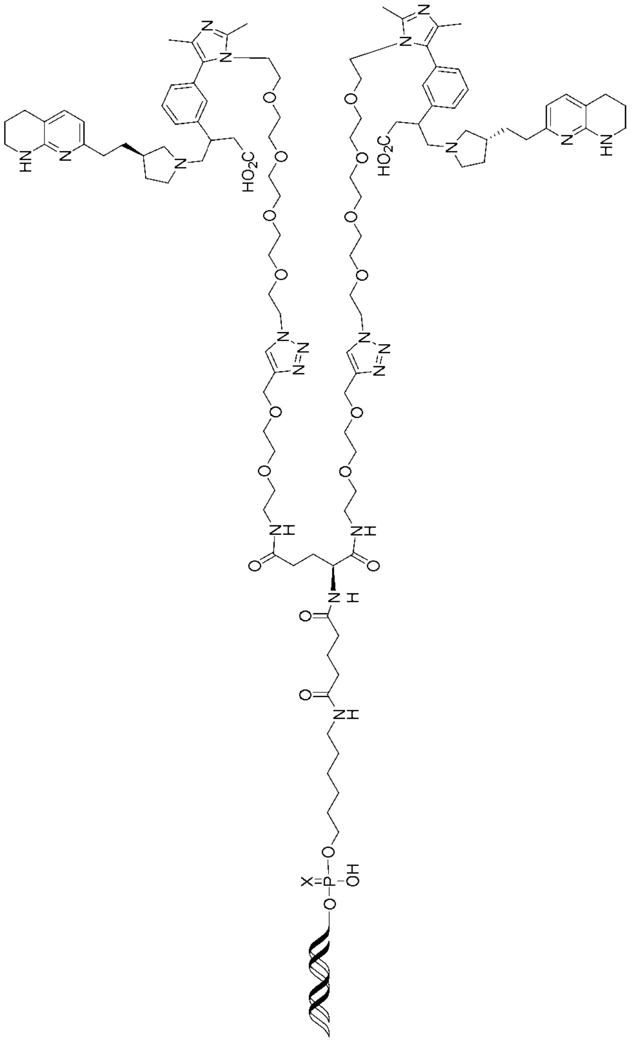 |

(continued)

| | |
|---|---|
| 300-15 | 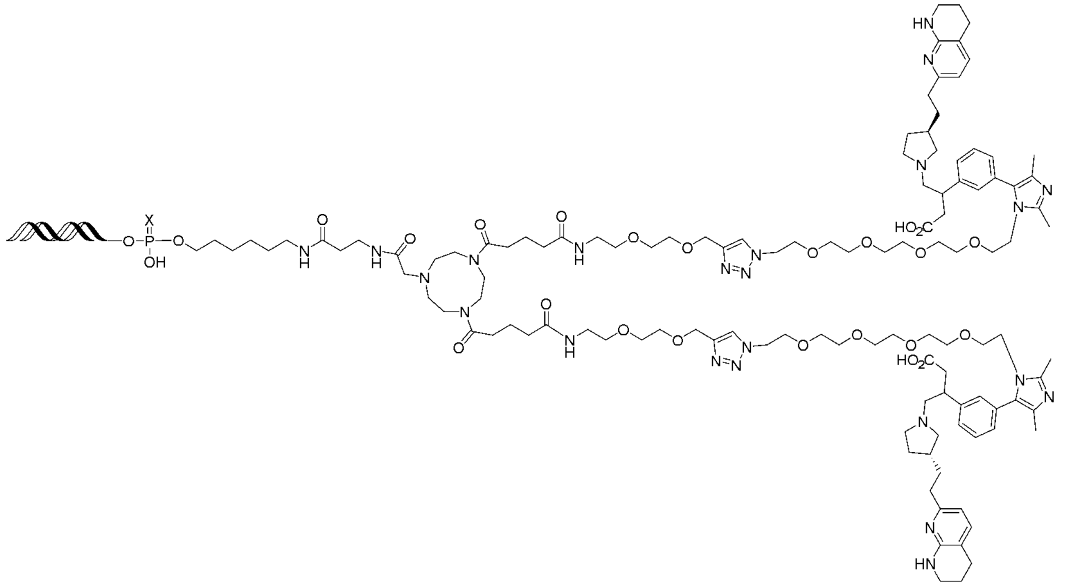 |

(continued)

| | |
|---|---|
| 301-15 | 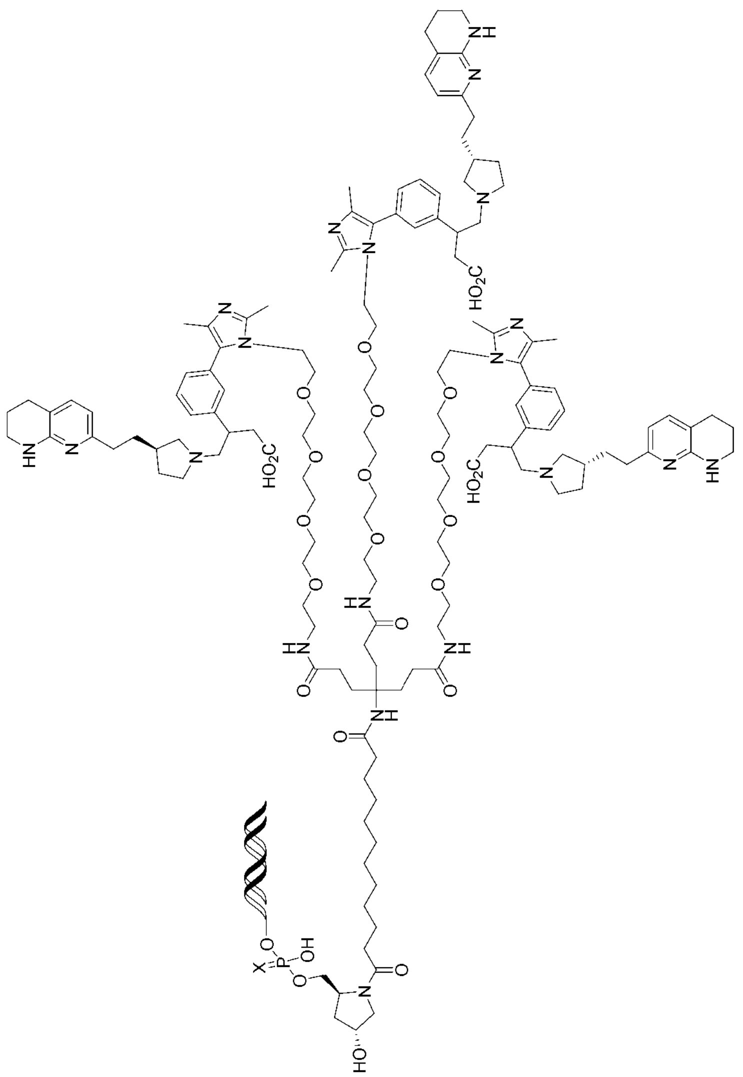 |

(continued)

| | |
|---|---|
| 302-15 | 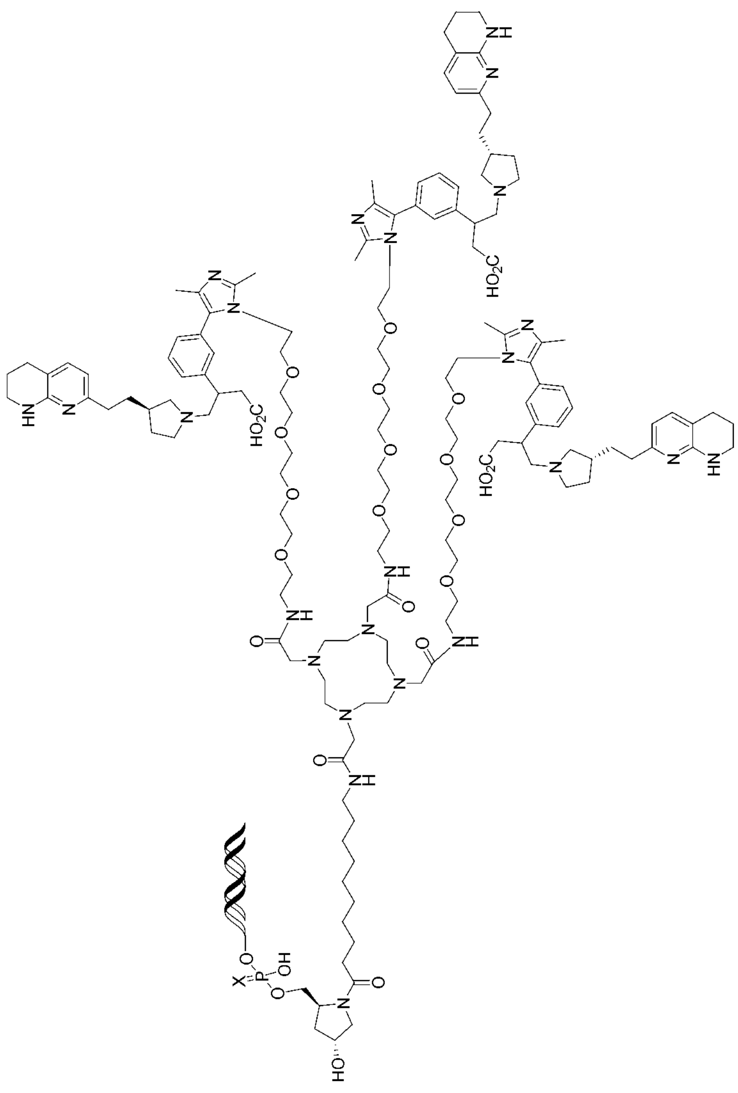 |

(continued)

| | |
|---|---|
| 303-15 | 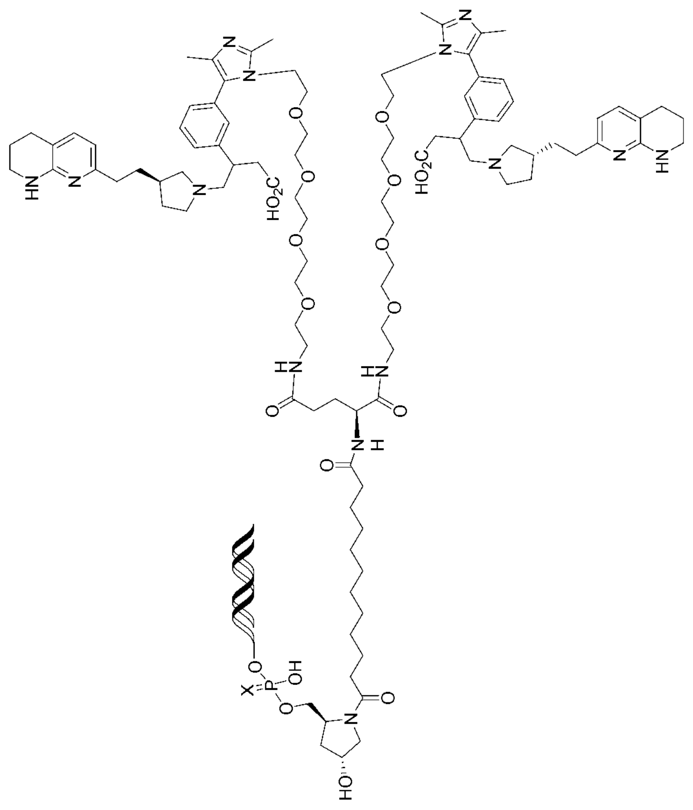 |

(continued)

| | |
|---|---|
| 304-15 | 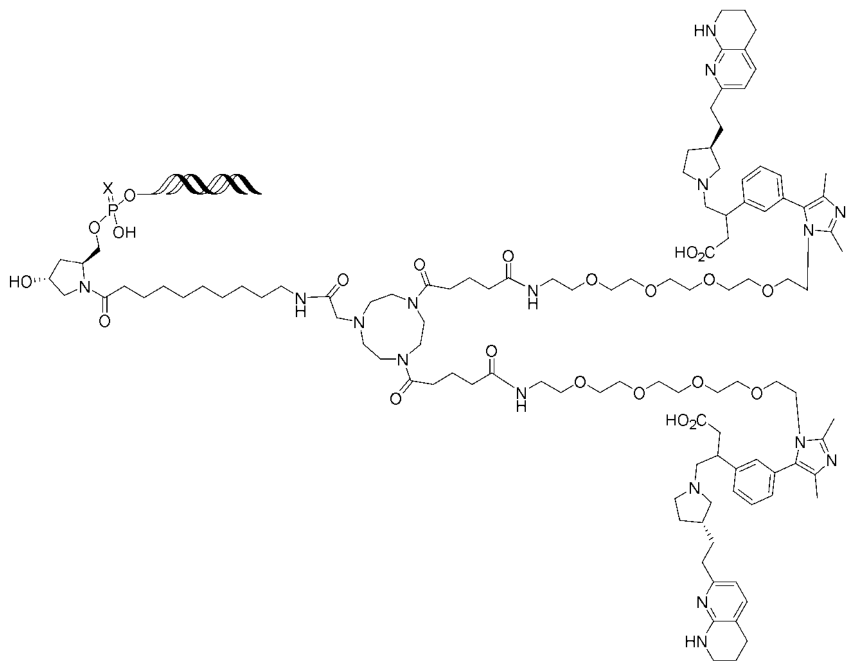 |

(continued)

| | |
|---|---|
| 305-15 | 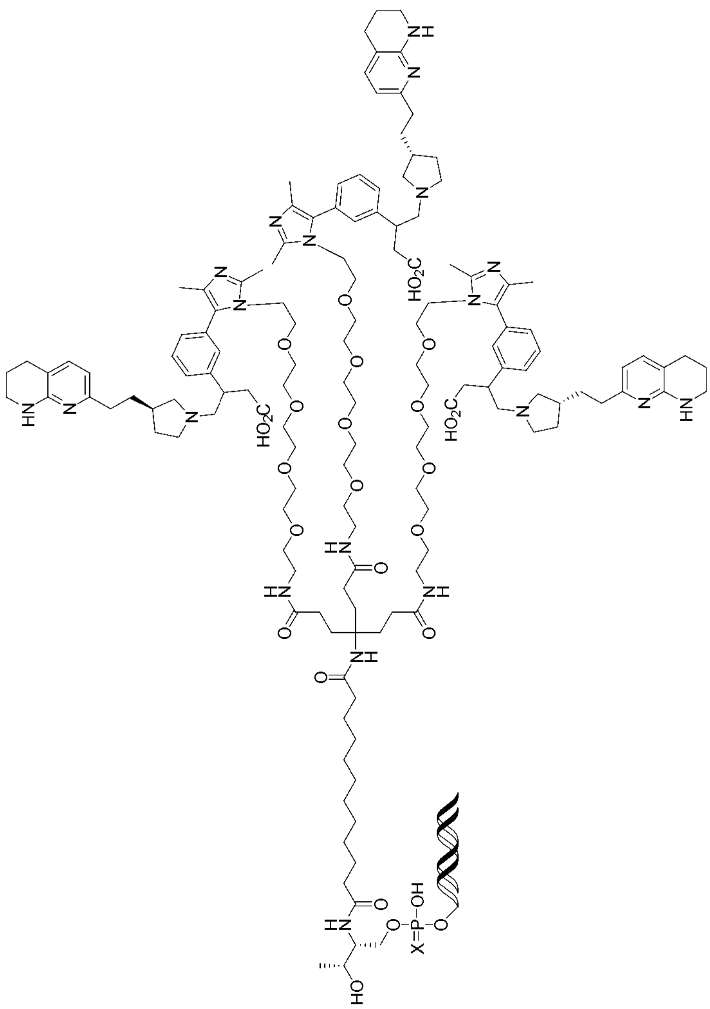 |

(continued)

| | |
|---|---|
| 306-15 | 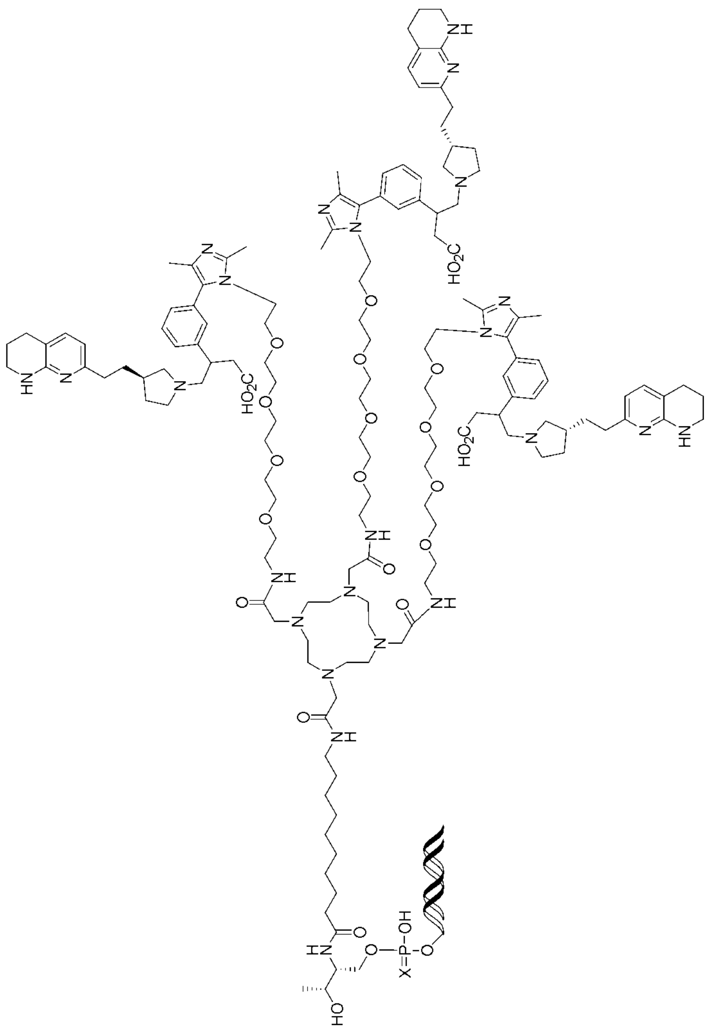 |

(continued)

| | |
|---|---|
| 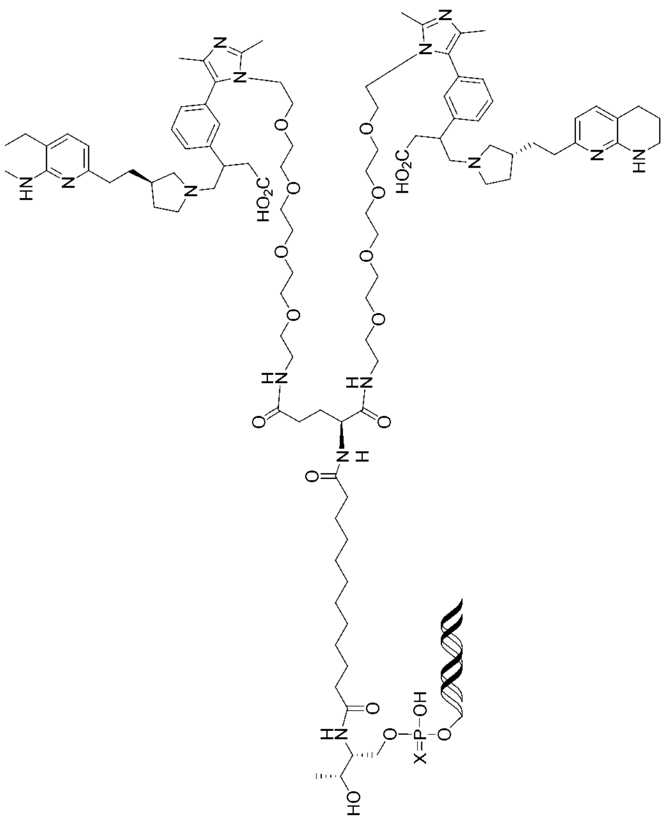 | 307-15 |

(continued)

| | |
|---|---|
| 308-15 | 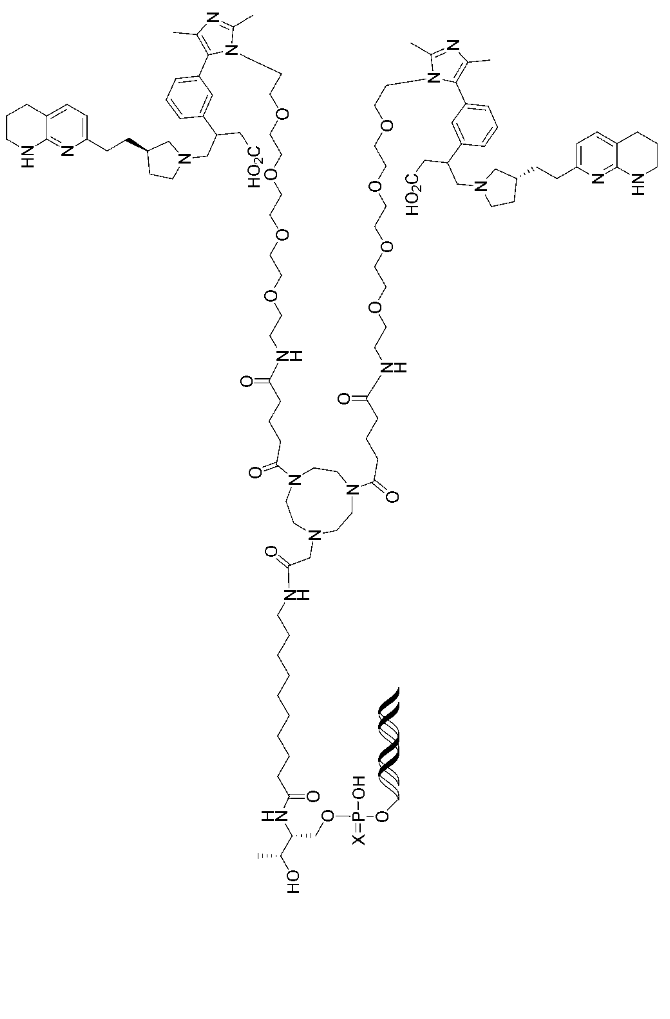 |
| 309-18 | 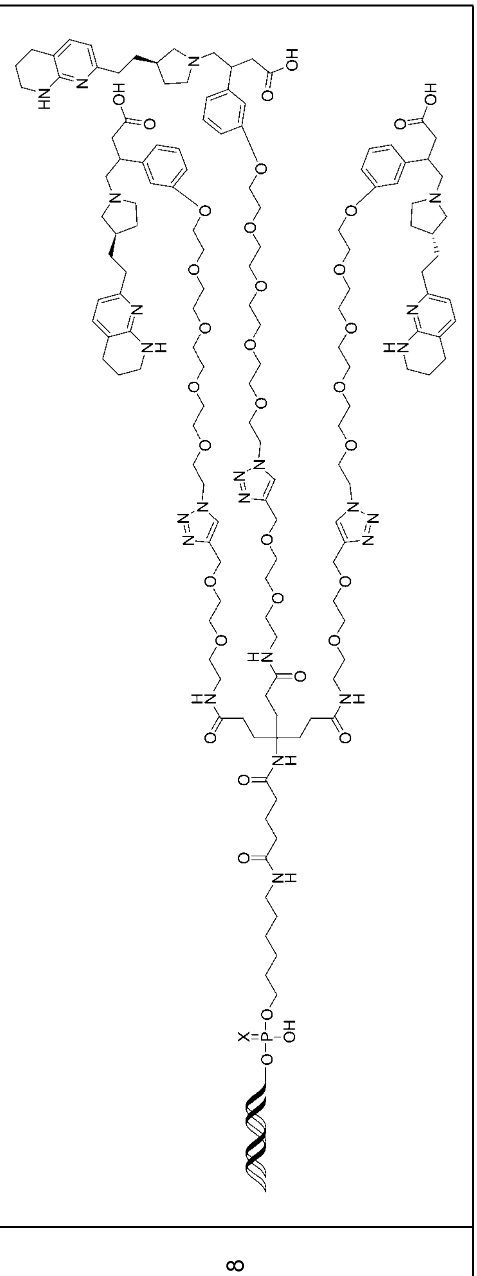 |

(continued)

| | |
|---|---|
| 310-18 | 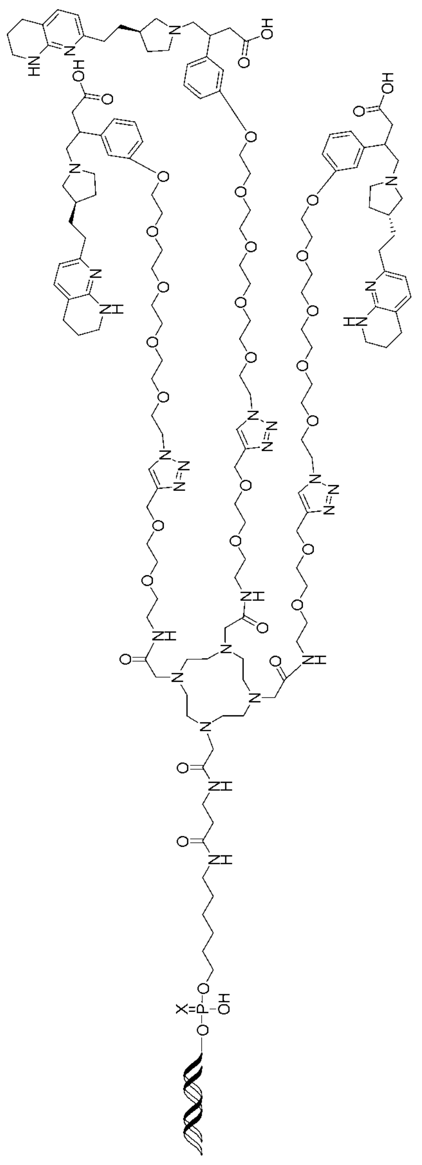 |
| 311-18 | 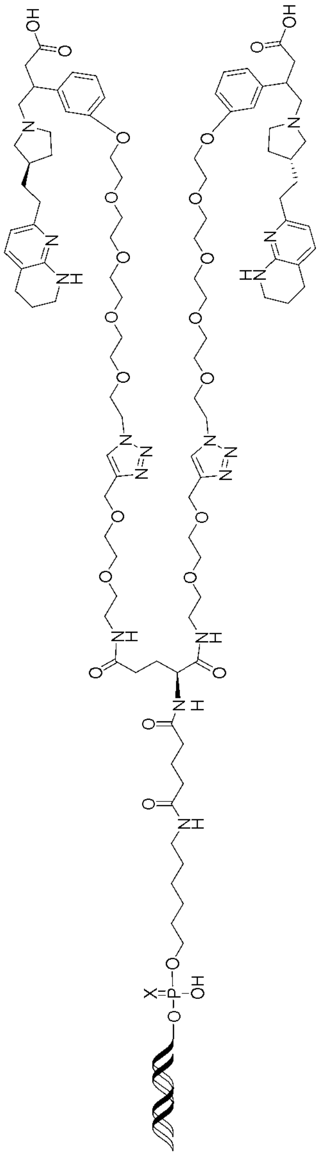 |
| 312-18 | 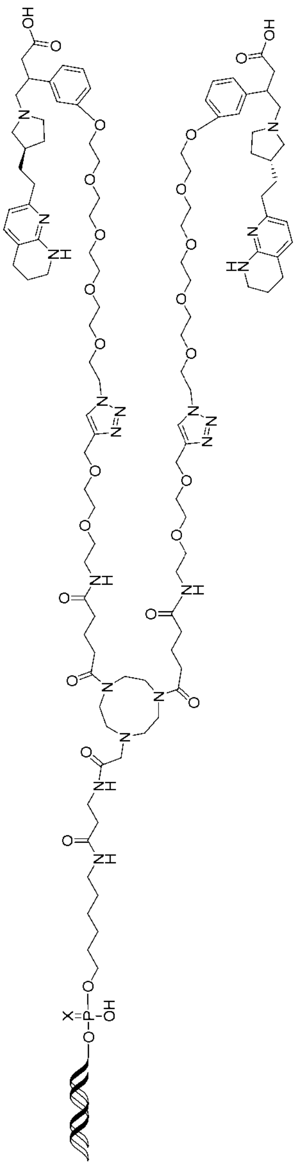 |

(continued)

| | |
|---|---|
| 313-18 | 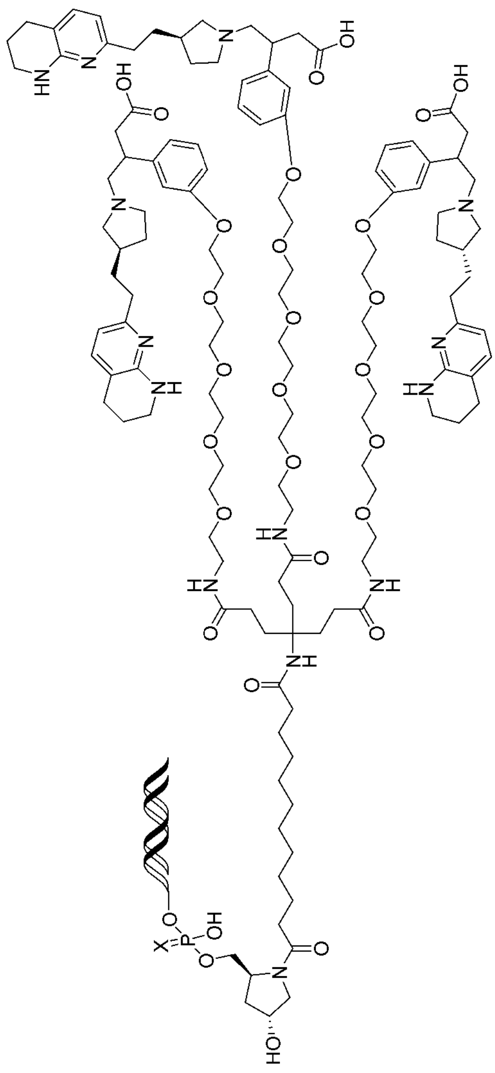 |
| 314-18 | 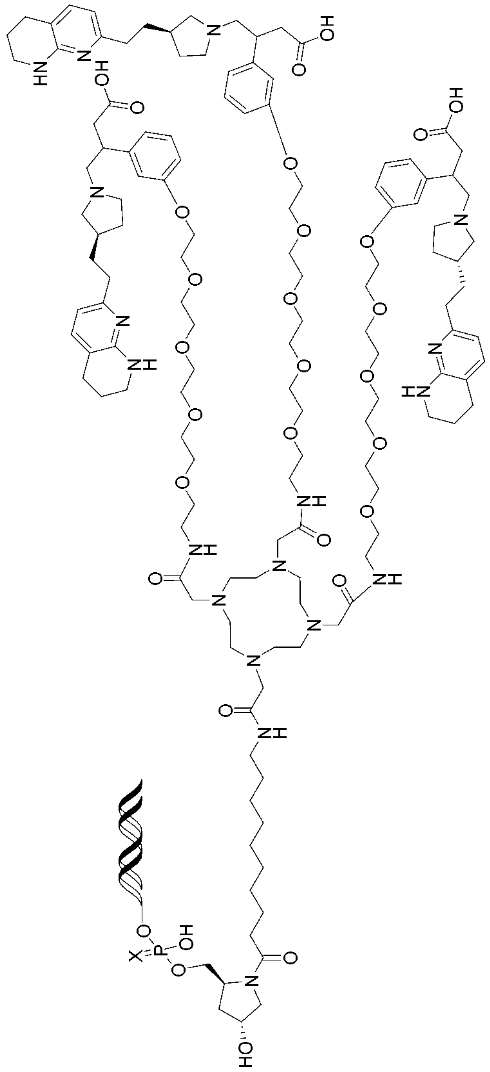 |

(continued)

| | |
|---|---|
| 315-18 | 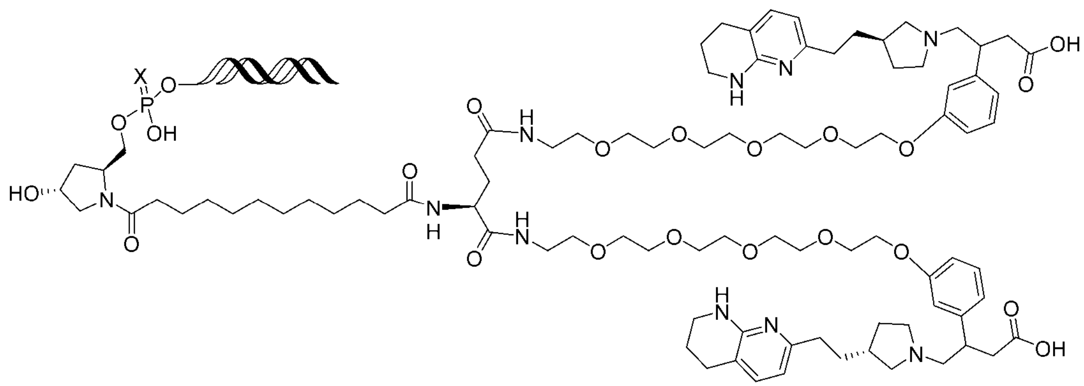 |
| 316-18 | 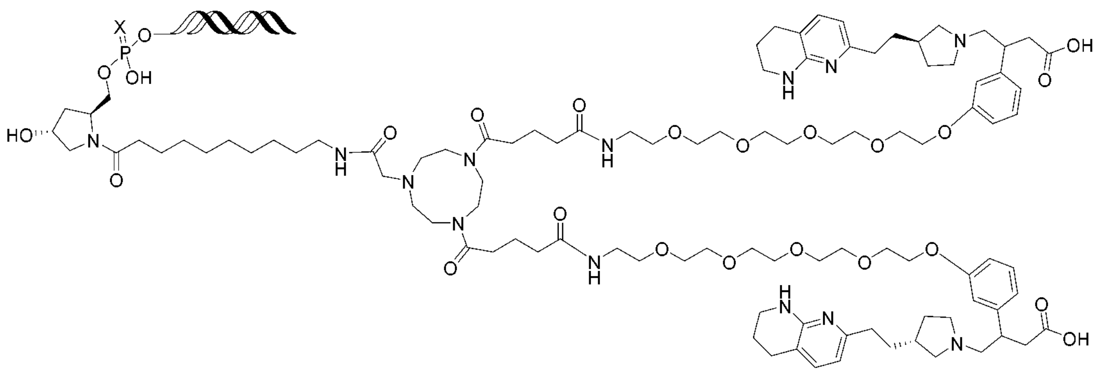 |

(continued)

| | |
|---|---|
| 317-18 | 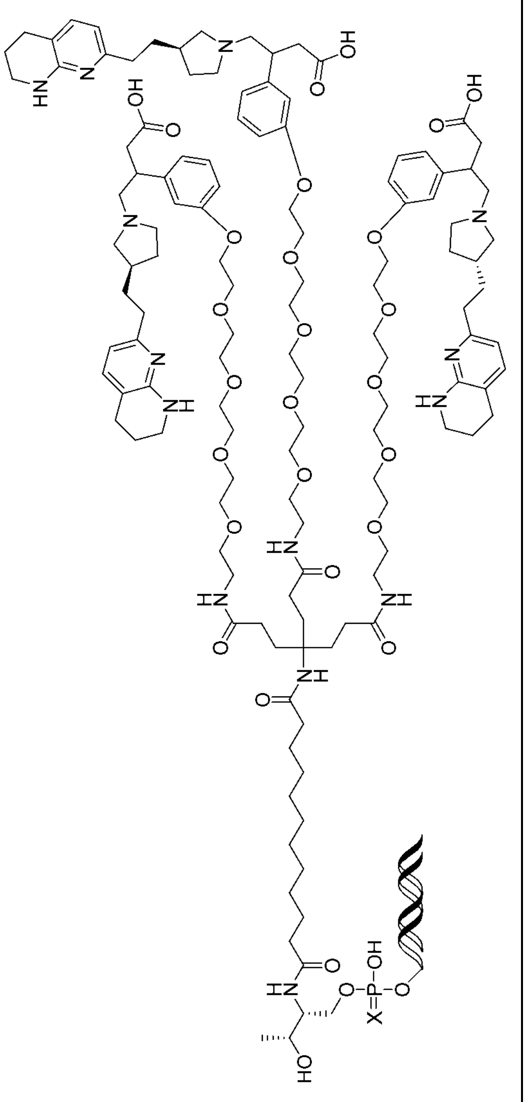 |
| 318-18 | 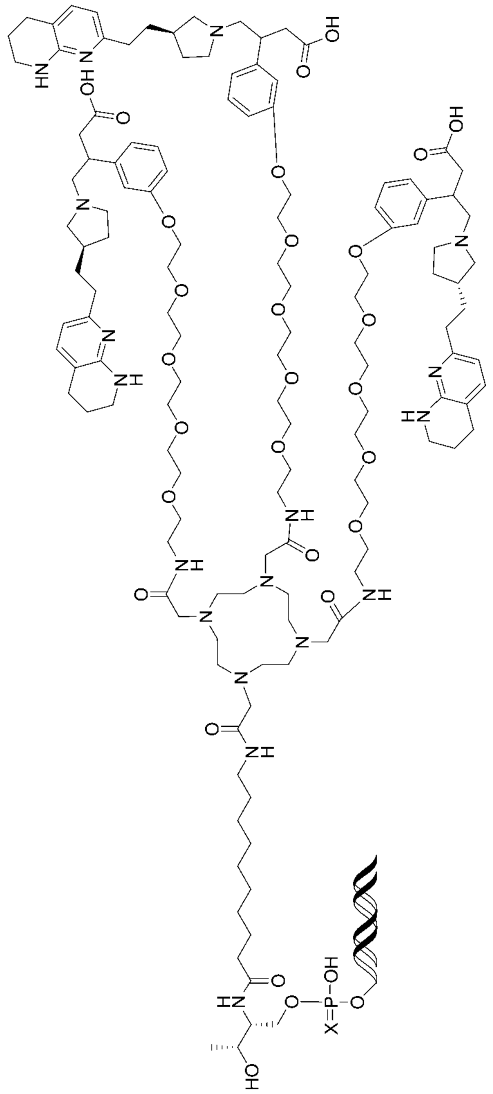 |

(continued)

| | |
|---|---|
| 319-18 | 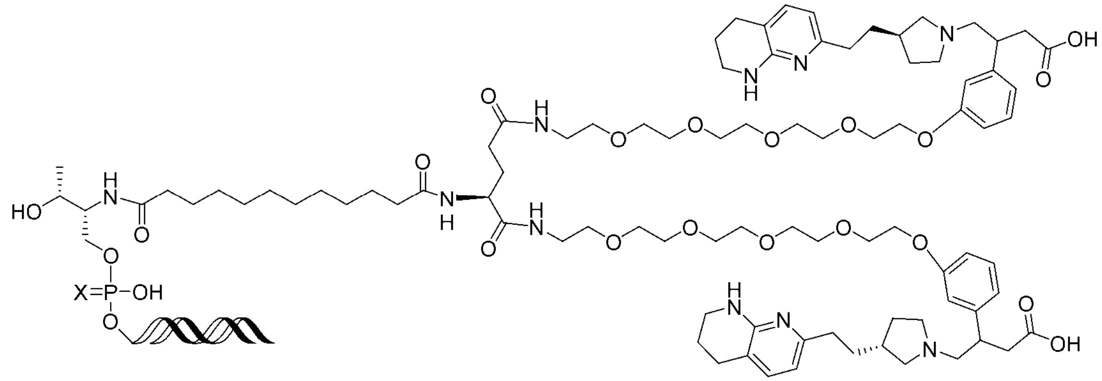 |
| 320-18 | 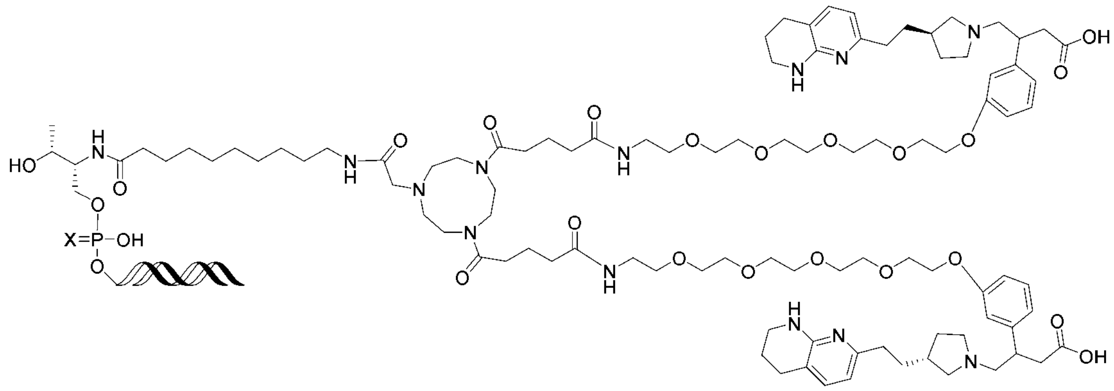 |

(continued)

| | |
|---|---|
| 321-20 | 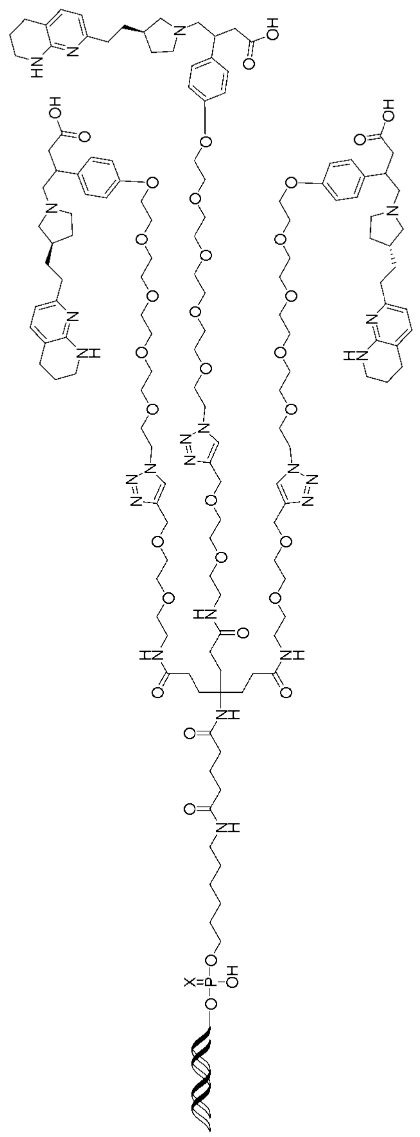 |
| 322-20 | 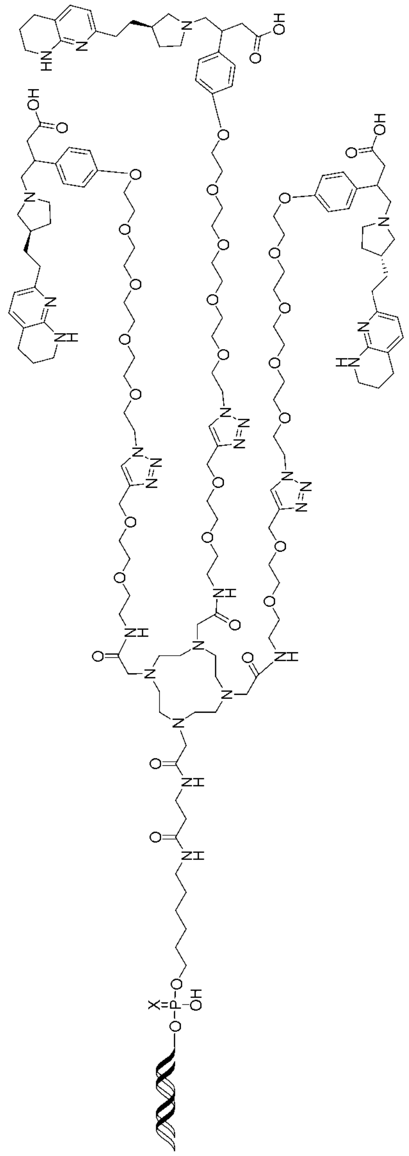 |

(continued)

| | |
|---|---|
| 323-20 | 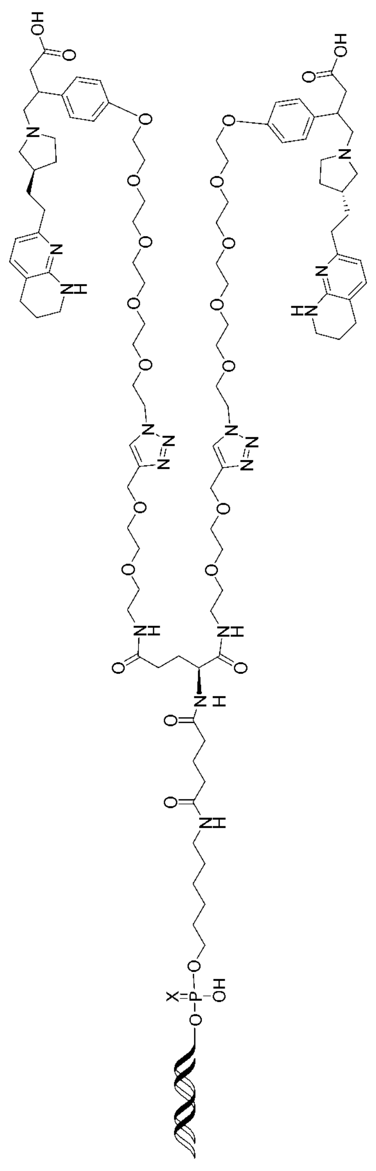 |
| 324-20 | 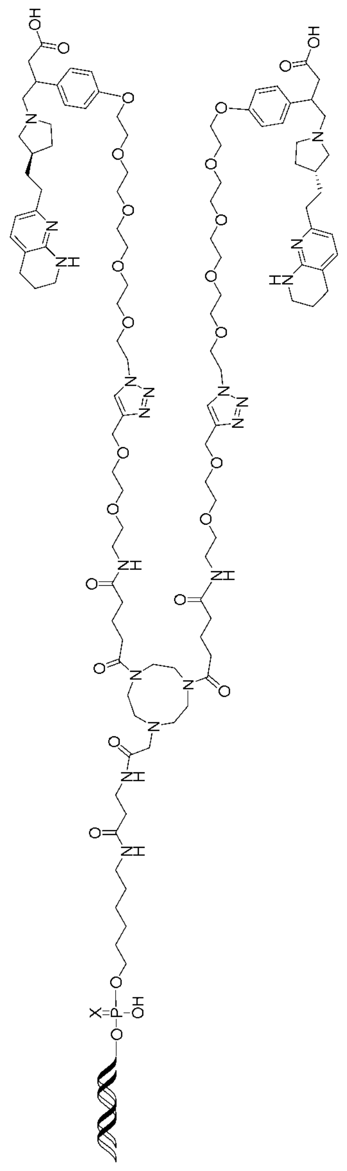 |

(continued)

| | |
|---|---|
| 325-20 | 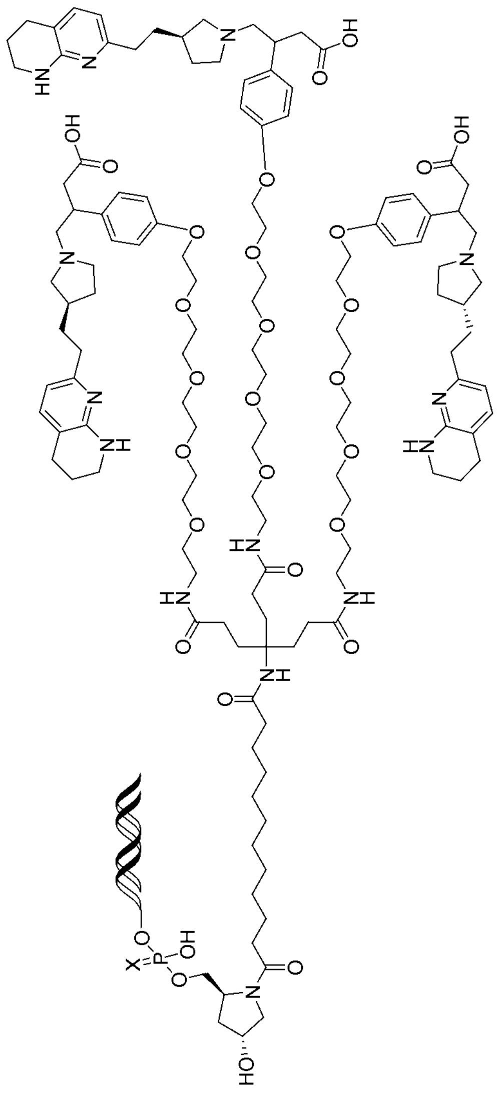 |
| 326-20 | 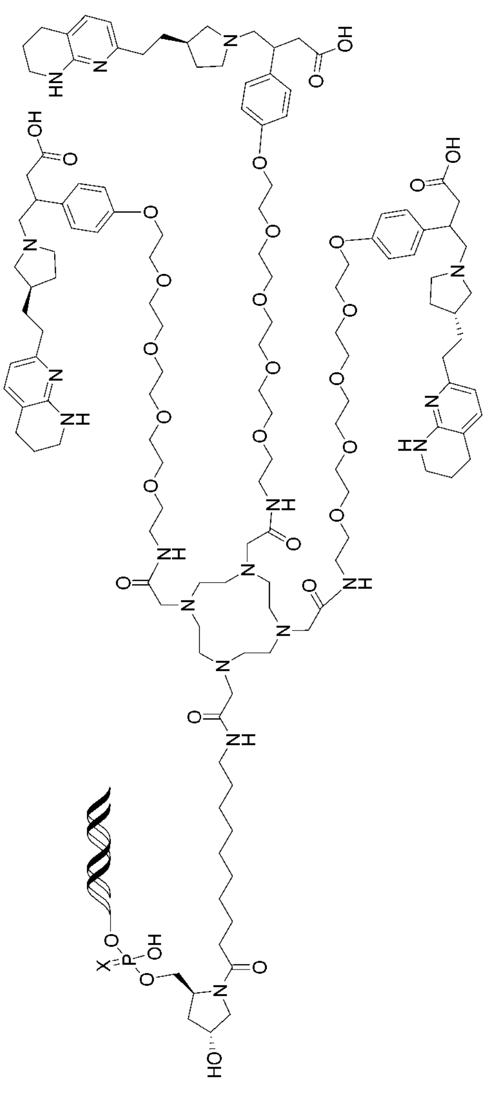 |

(continued)

| | |
|---|---|
| 327-20 | 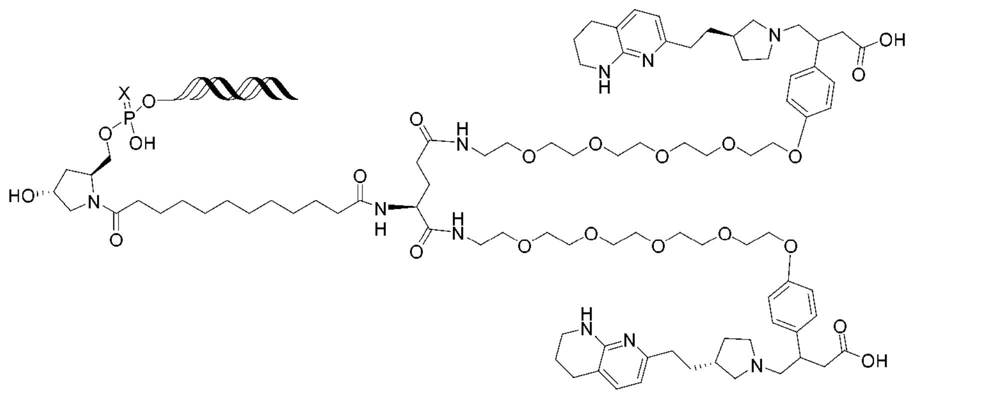 |
| 328-20 | 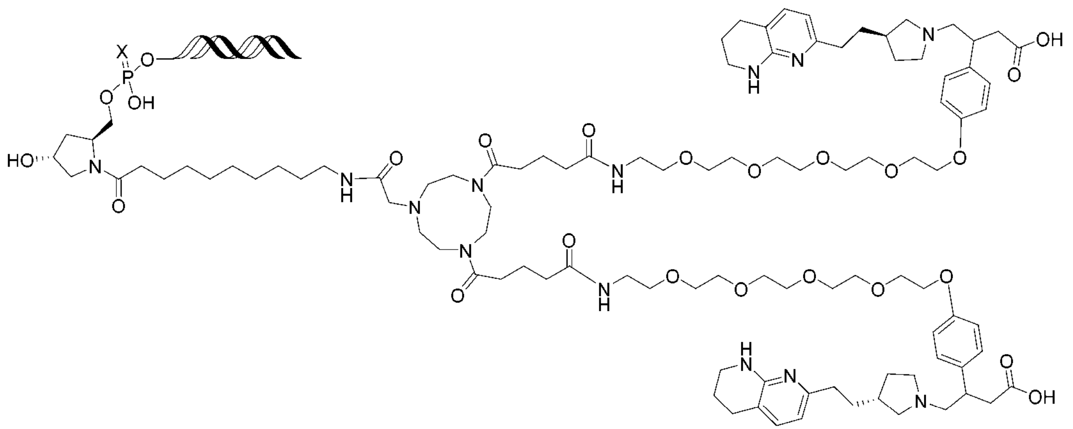 |

(continued)

| | |
|---|---|
| 329-20 | 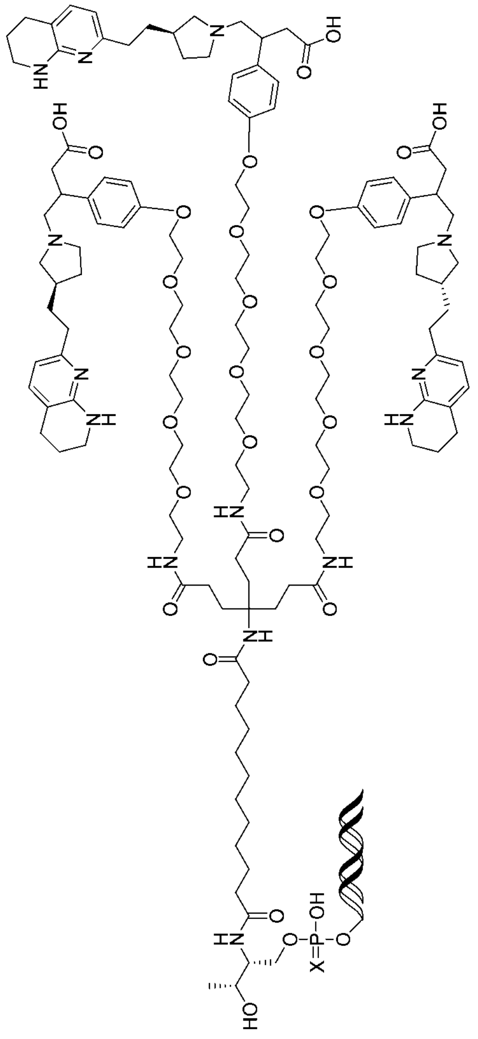 |
| 330-20 | 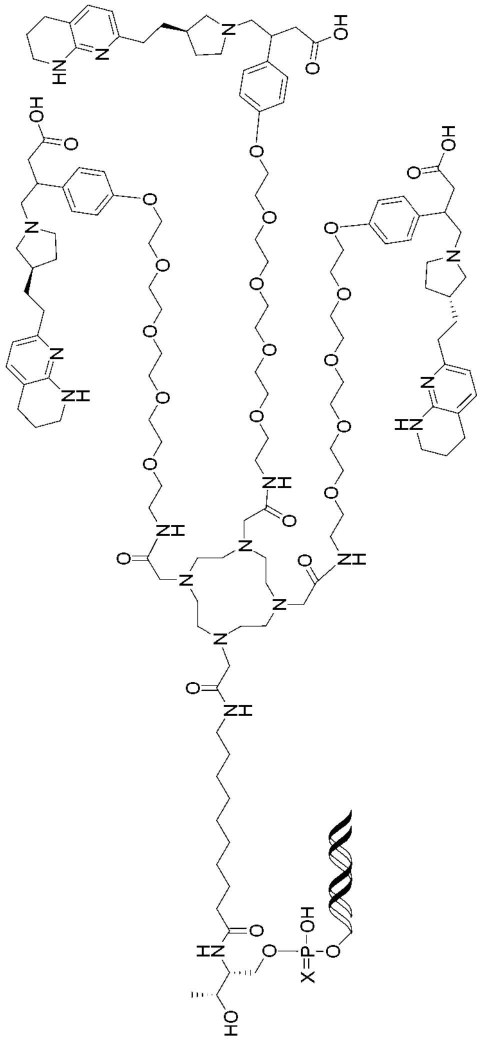 |

(continued)

| | |
|---|---|
| 331-20 | 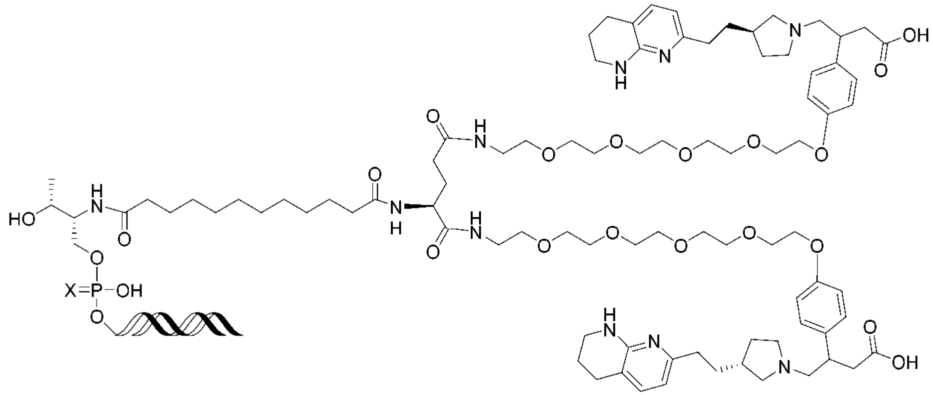 |
| 332-20 | 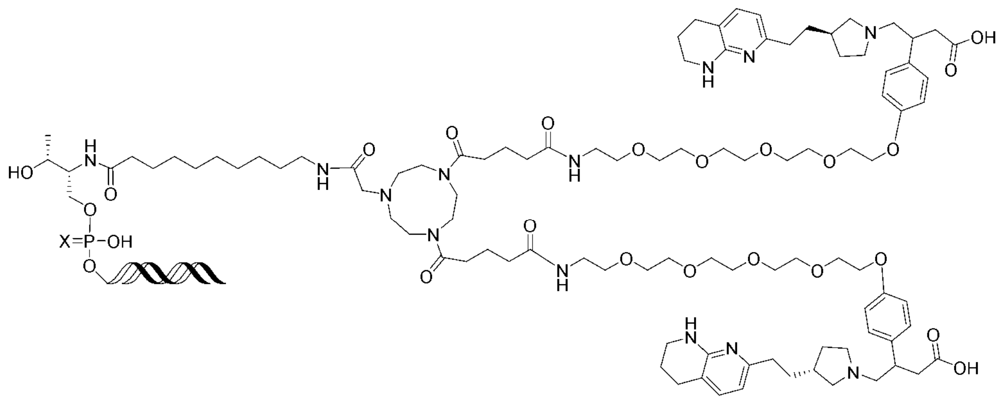 |
| 333-16 | 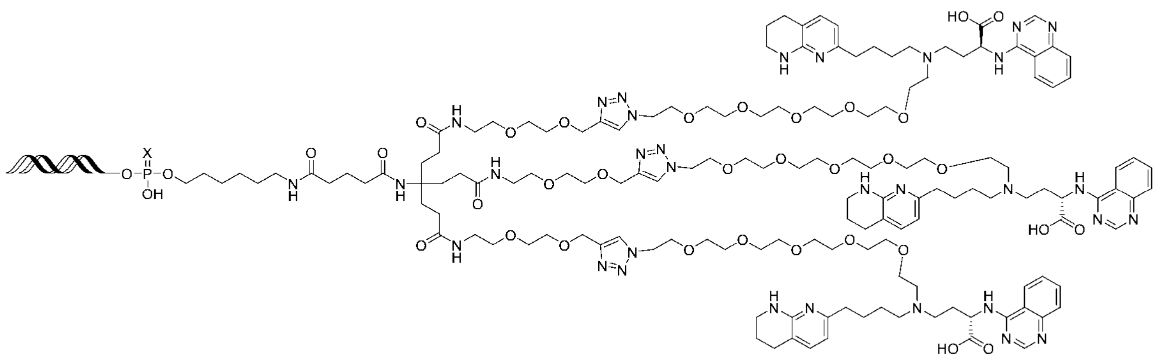 |

(continued)

| | |
|---|---|
| 334-16 | 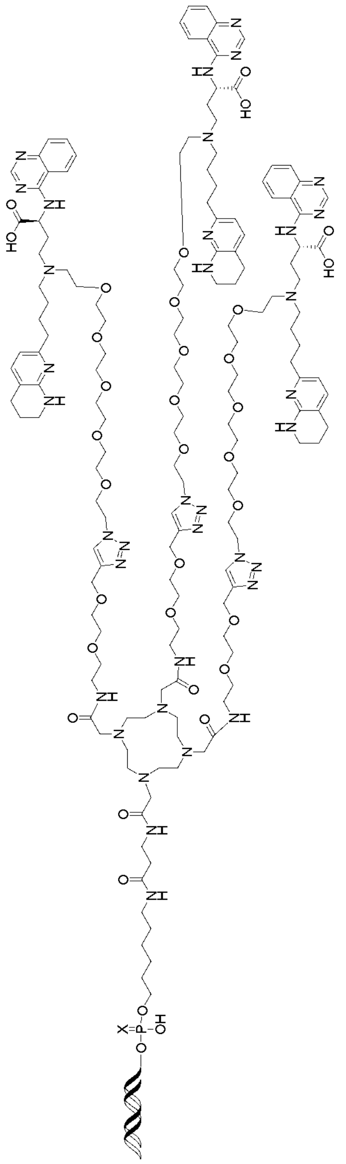 |
| 335-16 | 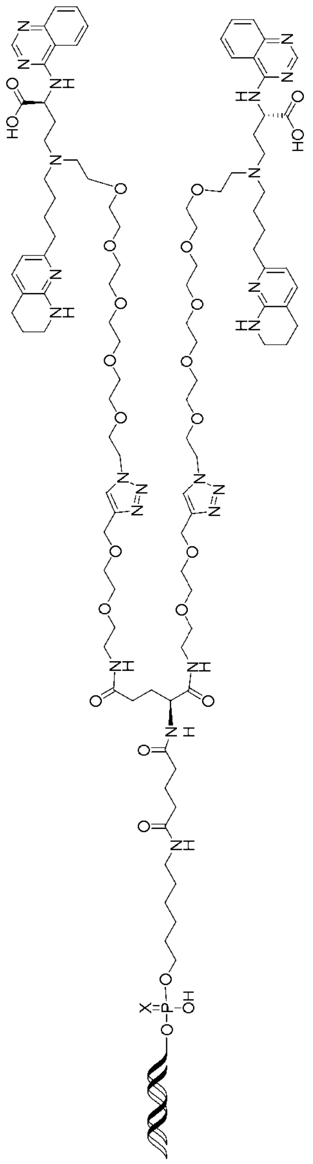 |
| 336-16 | 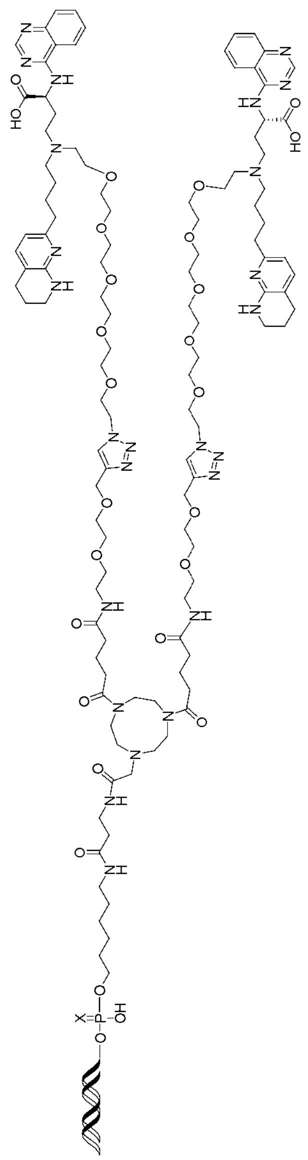 |

(continued)

| | |
|---|---|
| 337-16 | 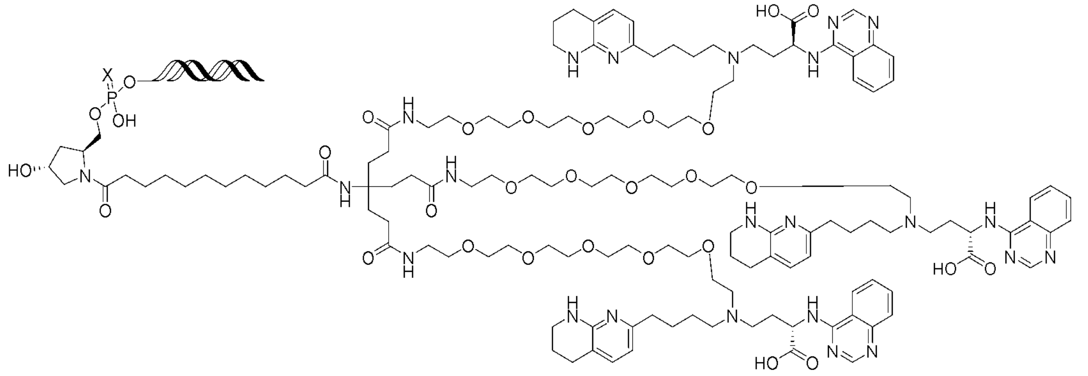 |
| 338-16 | 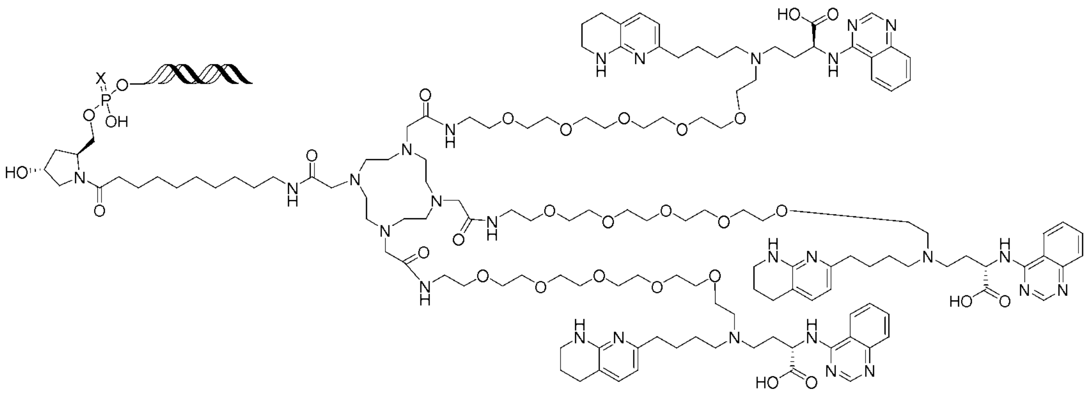 |
| 339-16 | 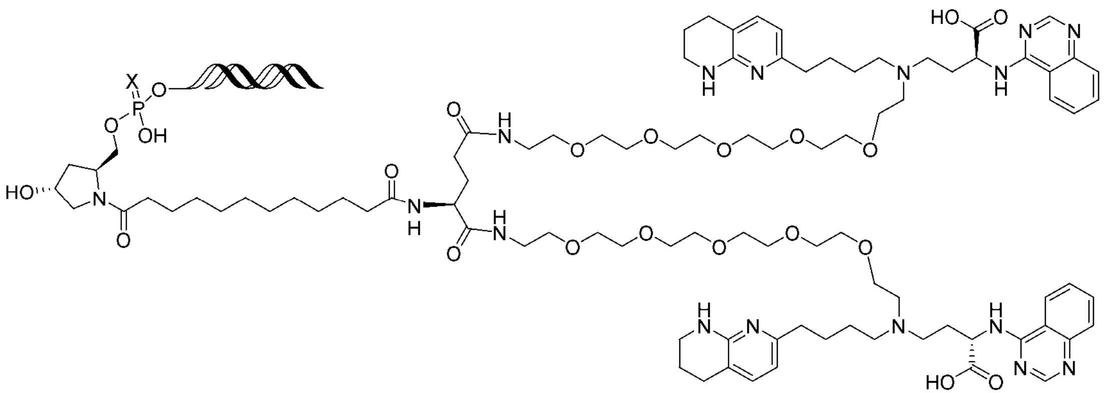 |

(continued)

| | |
|---|---|
| 340-16 | 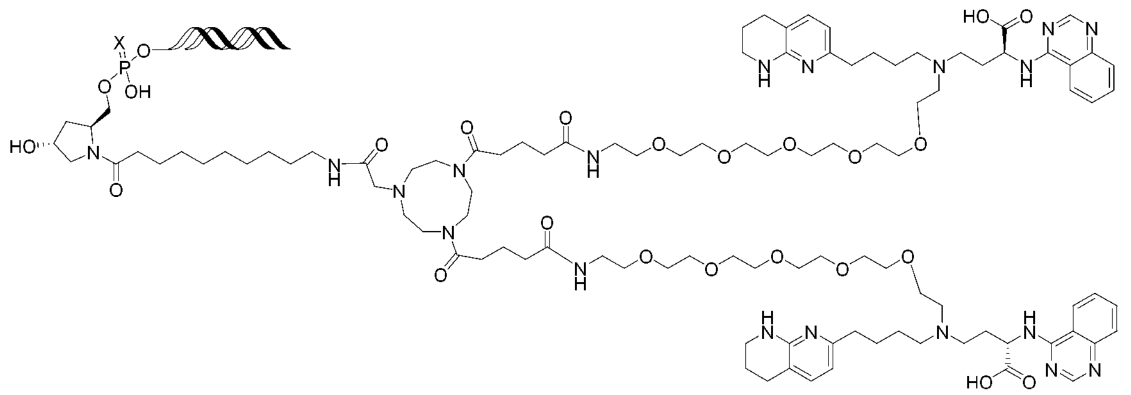 |
| 341-16 | 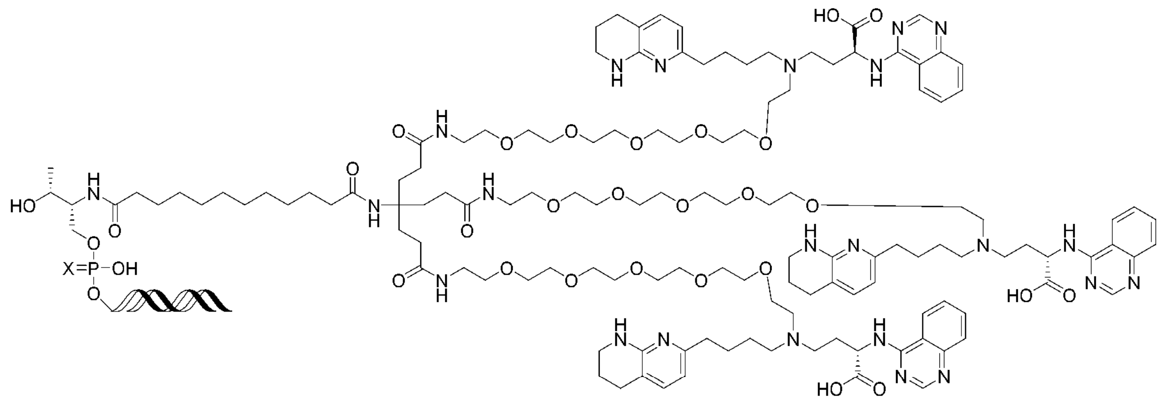 |

(continued)

| | |
|---|---|
| 342-16 | 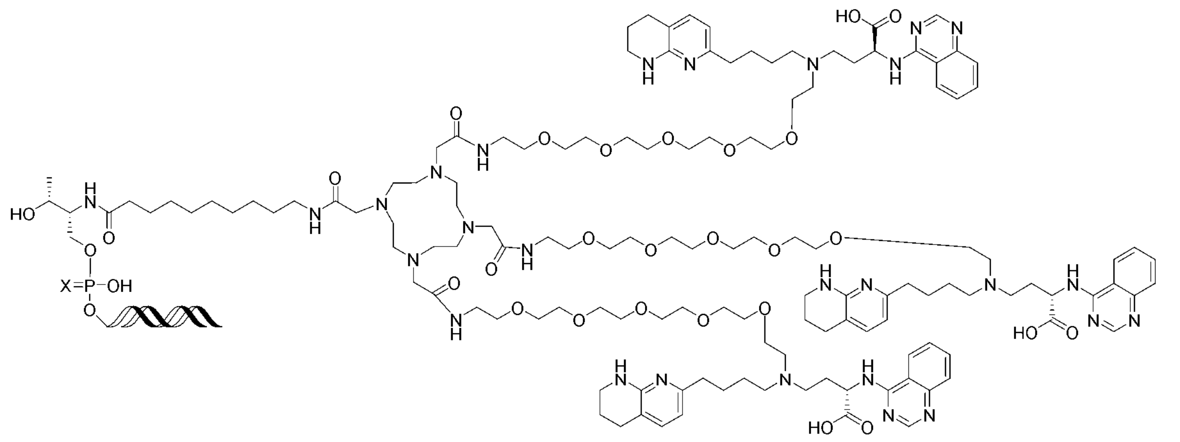 |
| 343-16 | 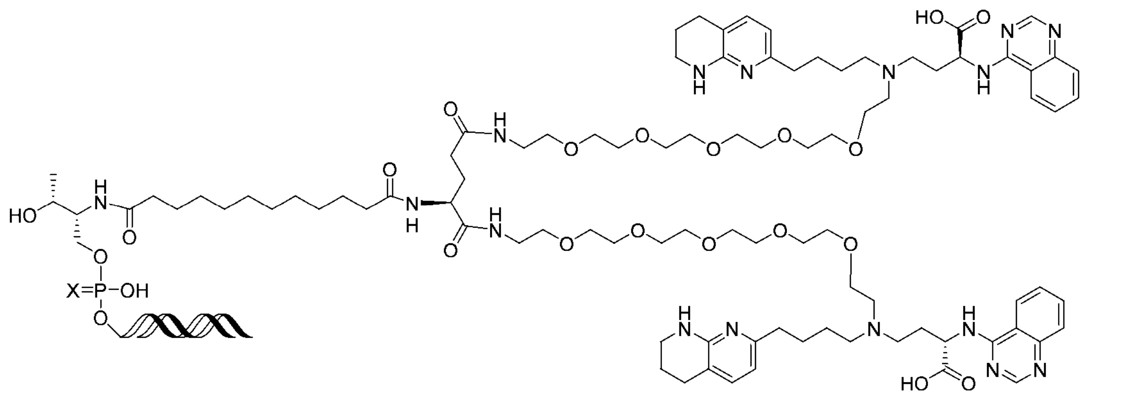 |

(continued)

| | |
|---|---|
| 344-16 | 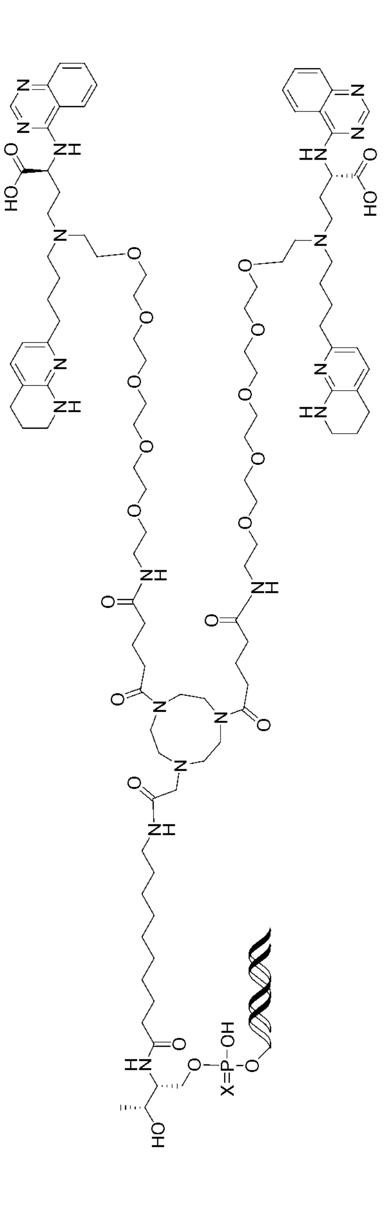 |
| 345-17 | 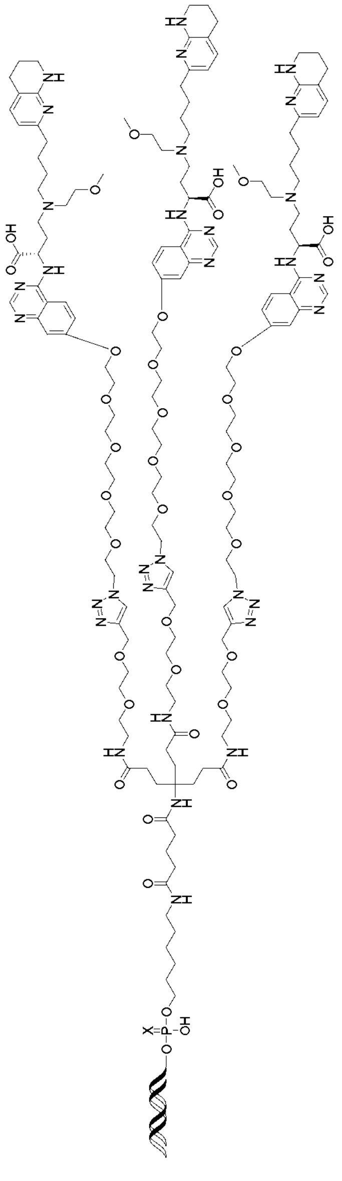 |
| 346-17 | 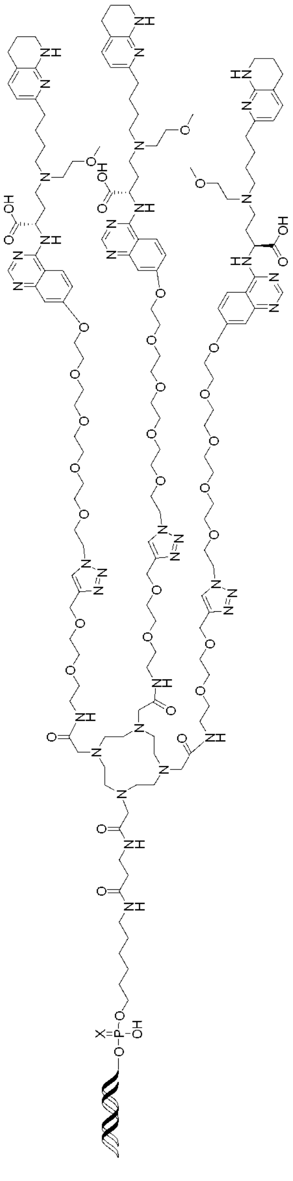 |

(continued)

| | |
|---|---|
| 347-17 | 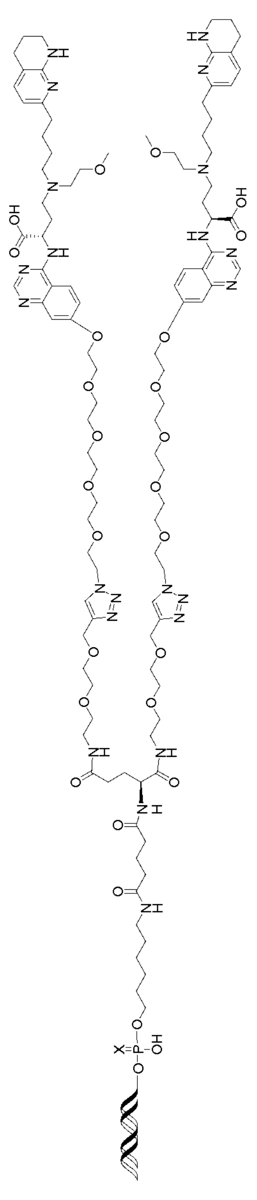 |
| 348-17 | 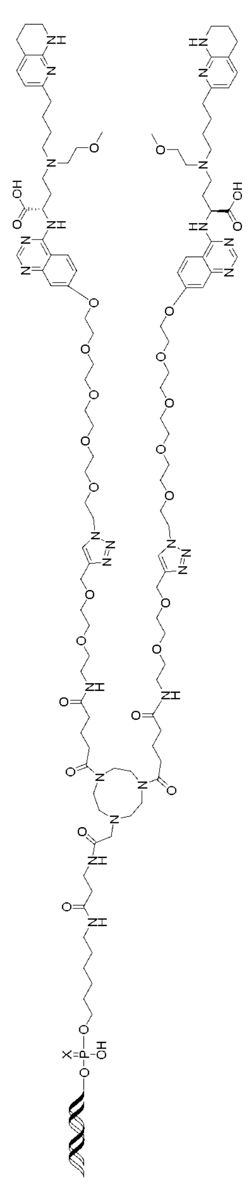 |
| 349-17 | 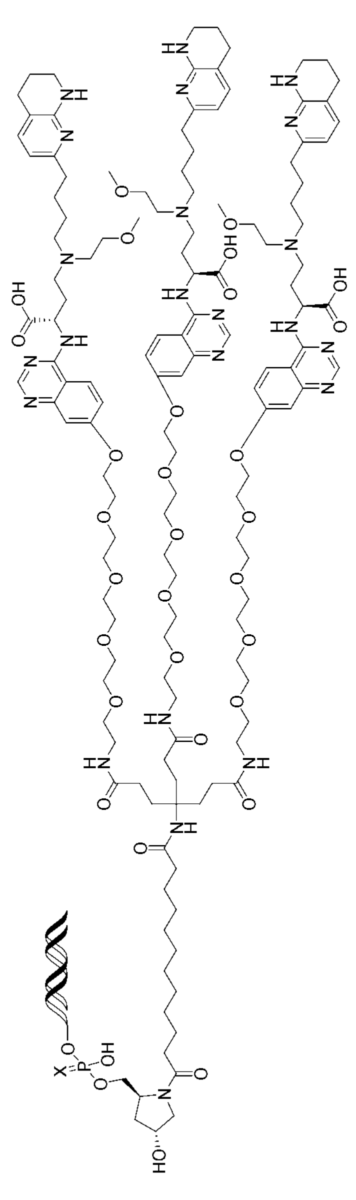 |

(continued)

| | |
|---|---|
| 350-17 | 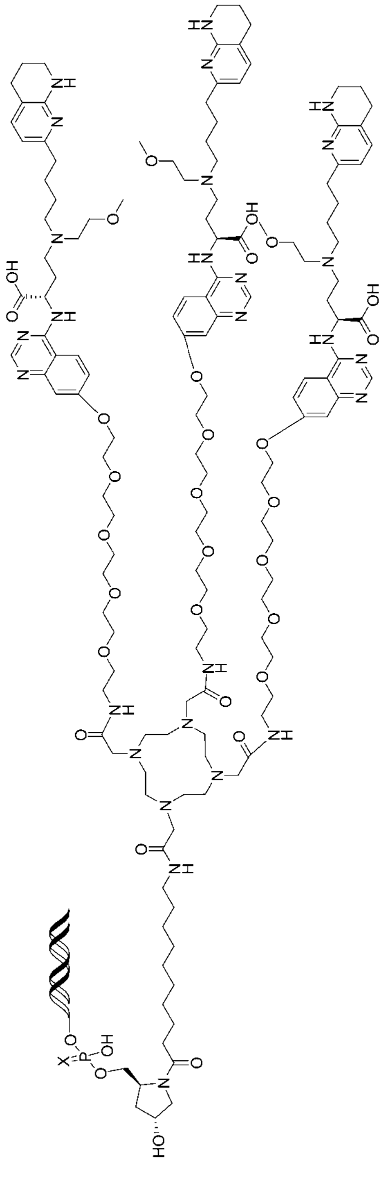 |
| 351-17 | 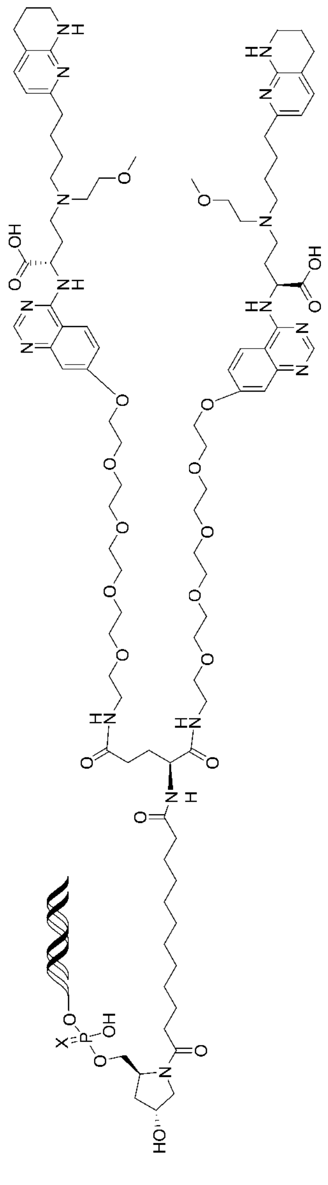 |
| 352-17 | 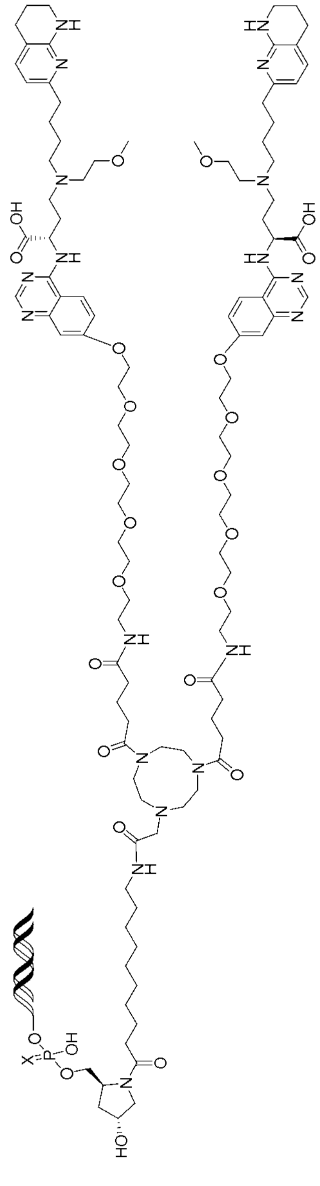 |

(continued)

| | |
|---|---|
| 353-17 | 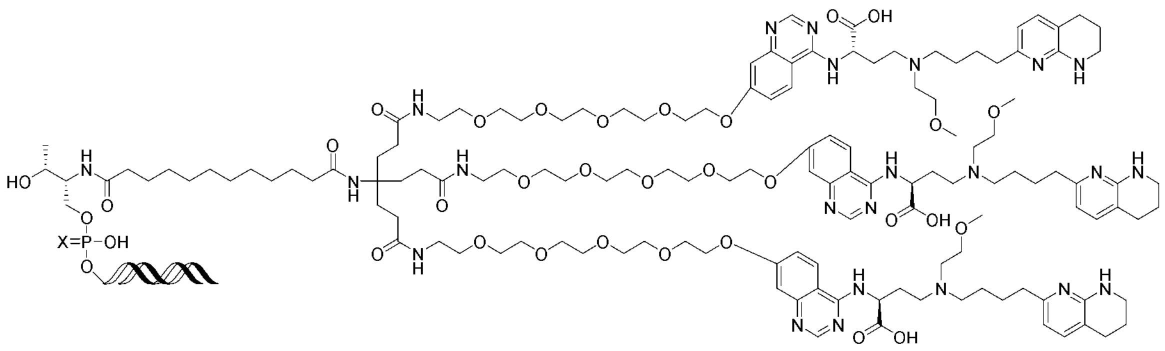 |
| 354-17 | 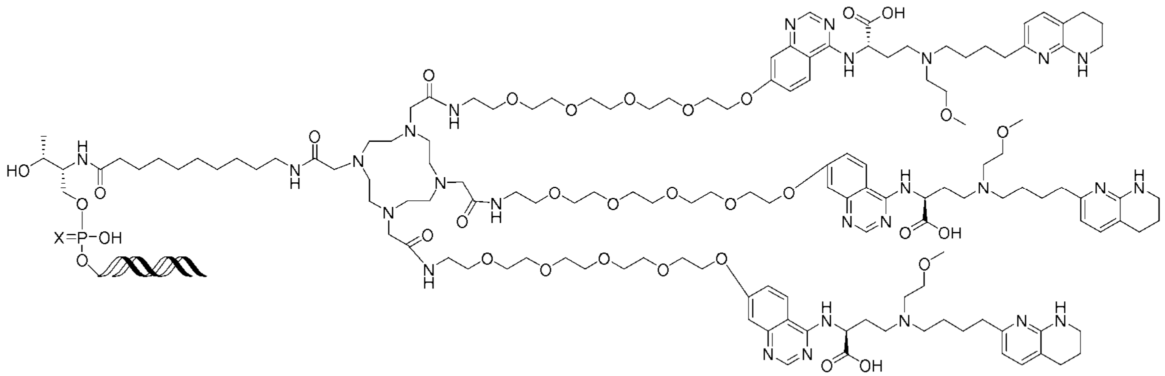 |
| 355-17 | 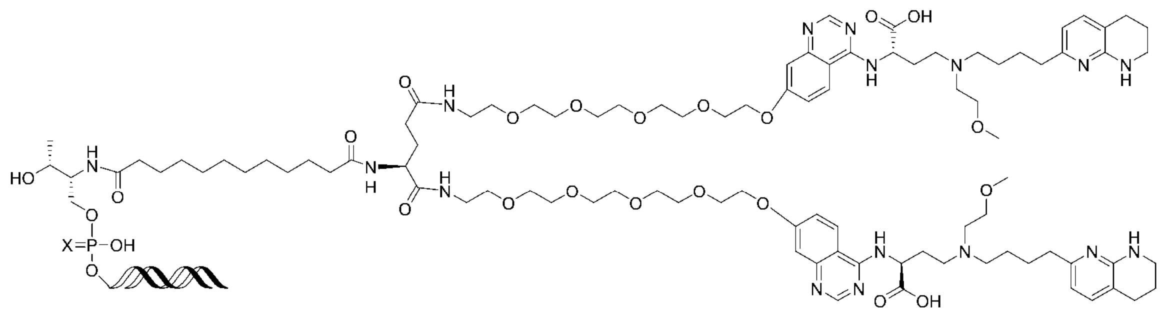 |

(continued)

| | |
|---|---|
| 356-17 | 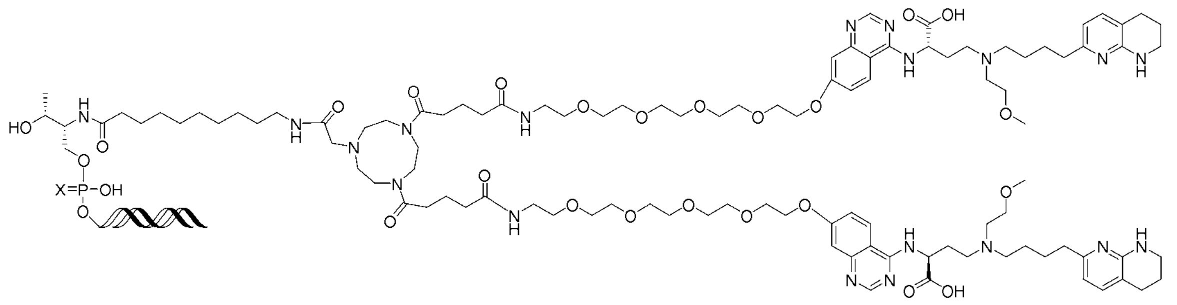 |

**Pharmaceutical Compositions**

The present invention also encompasses pharmaceutical compositions and formulations comprising the RNAi agents described herein (e.g., any one of Formulas 201 to 356) and a pharmaceutically acceptable carrier, excipient, or diluent. Such compositions and formulations can be used to reduce the expression of a target gene in a patient in need thereof. Considering clinical application, the pharmaceutical compositions and formulations will be prepared in a form suitable for the intended application. Typically, this will involve preparing compositions substantially free of pyrogens and other impurities that may be harmful to humans or animals.

The ingredients and methods for formulating pharmaceutical compositions depend on many criteria, including, but not limited to, the route of administration, the type and extent of the disease or condition to be treated, or the dosage to be administered. In some embodiments, the pharmaceutical composition is formulated based on the intended delivery route. For example, in certain embodiments, the pharmaceutical composition is formulated for parenteral delivery. Parenteral administration forms include intravenous, intra-arterial, subcutaneous, intrathecal, intraperitoneal, or intramuscular injection or infusion. In one embodiment, the pharmaceutical composition is formulated for intravenous delivery. In such embodiments, the pharmaceutical composition may comprise a lipid-based delivery vehicle. In another embodiment, the pharmaceutical composition is formulated for subcutaneous delivery.

In some embodiments, the pharmaceutical composition comprises an effective amount of the RNAi agent described herein. An "effective amount" refers to an amount sufficient to produce a beneficial or desired clinical outcome. In some embodiments, the effective amount is an amount sufficient to reduce the expression of a target gene in a specific tissue or cell type of the patient. The effective amount of the RNAi agent of the present invention can be from about 0.01 mg/kg body weight to about 100 mg/kg body weight, and can be administered daily, weekly, monthly, or at longer intervals. The precise determination of the specific effective dosage amount and administration frequency may be based on several factors, including the patient's body size, age, and general condition, the type of disease to be treated, the specific RNAi agent used, and the route of administration.

Administration of the pharmaceutical compositions of the invention can be by any common route provided the target tissue is accessible via that route. These routes include, but are not limited to, parenteral (e.g., subcutaneous, intramuscular, intraperitoneal, or intravenous), oral, nasal, buccal, intradermal, transdermal, and sublingual routes, or by direct injection into liver tissue or delivery via the hepatic portal vein. In some embodiments, the pharmaceutical composition is administered parenterally. For example, in certain embodiments, the pharmaceutical composition is administered intravenously. In other embodiments, the pharmaceutical composition is administered subcutaneously.

Colloidal dispersion systems can be used as delivery vehicles for the RNAi agents of the present invention, such as macromolecular complexes, nanocapsules, microspheres, beads, and lipid-based systems, including oil-in-water emulsions, micelles, mixed micelles, and liposomes. Commercially available fat emulsions suitable for delivering the nucleic acids of the present invention include Intralipid (Baxter International Inc.), Liposyn (Abbott Pharmaceuticals), Lipsyn II (Hospira), Liposyn III (Hospira), Nutrilipid (B. Braun Medical Inc.), and other similar fat emulsions. A preferred colloidal system for use as an *in vivo* delivery vehicle is the liposome (i.e., an artificial membrane vesicle). The RNAi agents of the present invention can be encapsulated within liposomes or can form complexes therewith, particularly with cationic liposomes. Alternatively, the RNAi agents of the present invention can be complexed with lipids, particularly with cationic lipids. Suitable cationic lipids are, for example, dioleoyltrimethylammonium propane (DOTAP) and dioleoylphosphatidylethanolamine (DOTMA).

Liposomal formulations are particularly suitable for topical administration, with liposomes conferring several advantages over alternative formulations. Such advantages include reduced incidence of adverse effects associated with high systemic absorption of administered therapeutics, enhanced accumulation of administered therapeutics at desired target sites, and capability for dermal delivery of RNAi agents. In some embodiments, liposomes are employed to deliver RNAi agents to epidermal cells while concurrently enhancing permeation of said RNAi agents into dermal tissues, e.g., within the skin.

The RNAi agents of the present invention may be fully encapsulated within lipid-based formulations, such as LNPs or other nucleic acid-lipid particles. As used herein, the term "LNP" refers to stable nucleic acid-lipid particles. LNPs typically comprise a cationic lipid, a non-cationic lipid, and a lipid that prevents particle aggregation (e.g., a PEG-lipid conjugate). LNPs are particularly useful for systemic applications as they exhibit prolonged circulation times following intravenous (i.v.) injection and accumulate at distal sites (e.g., sites physically separated from the administration site). LNPs include "pSPLP," which comprise encapsulated condensing agent-nucleic acid complexes as described in WO00/03683. The LNP particles of the present invention generally possess a mean diameter ranging from about 50 nm to about 150 nm, more typically from about 60 nm to about 130 nm, more typically from about 70 nm to about 110 nm, and most typically from about 70 nm to about 90 nm, and are substantially non-toxic. Additionally, when nucleic acids are present within the nucleic acid-lipid particles of the invention, they demonstrate resistance to nuclease degradation in aqueous solutions. Nucleic acid-lipid particles and methods for their preparation are disclosed in, for example, U.S. Pat. Nos. 5,976,567; 5,981,501; 6,534,484; 6,586,410; 6,815,432; U.S. Publication No. 2010/0324120; and WO 96/40964. In

one embodiment, the lipid-to-drug ratio (mass/mass basis) (e.g., lipid-to-RNAi agent ratio) will range from about 1:1 to about 50:1, about 1:1 to about 25:1, about 3:1 to about 15:1, about 4:1 to about 10:1, about 5:1 to about 9:1, or about 6:1 to about 9:1.

Cationic lipids may include, for example, N,N-dioleyl-N,N-dimethylammonium chloride (DODAC), N,N-distearyl-N,N-dimethylammonium bromide (DDAB), N-(1-(2,3-dioleyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2,3-dioleyloxy)propylamine (DODMA), 1,2-dioleyloxy-N,N-dimethylaminopropane (DLinDMA), 1,2-dilinoleyloxy-N,N-dimethylaminopropane (DLenDMA), 1,2-dilinoleylcarbamoyloxy-3-dimethylaminopropane (DLin-C-DAP), 1,2-dilinoleyoxy-3-(dimethylamino) acetoxypropane (DLin-DAC), 1,2-dilinoleyoxy-3-morpholinopropane (DLin-MA), 1,2-dilinolenyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleoyloxy-3-(N-methylpiperazinyl)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 1,2-dilinolenyloxy-N,N-dimethylaminopropane (DLinDMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-(bis(2-hydroxydodecyl)amino)ethyl)(2-hydroxydodecyl)amino)ethyl)piperazin-1-yl)ethylazanediyl)didodecan-2-ol (Tech G1), or mixtures thereof. The cationic lipid may constitute about 20 mol% to about 50 mol%, or about 40 mol% of the total lipid present in the particle.

In some embodiments, the compound 2,2-dilinoleyl-4-dimethylaminomethyl-[1,3]-dioxolane may be utilized in the preparation of lipid-siRNA nanoparticles. In certain embodiments, the lipid-siRNA particles comprise 40% 2,2-dilinoleyl-4-dimethylaminomethyl-[1,3]-dioxolane : 10% DSPC : 40% cholesterol : 10% PEG-C-DOMG (mol%), with a particle size of 63.0 ± 20 nm and an siRNA-to-lipid ratio of 0.027.

Ionizable/non-cationic lipids may be anionic lipids or neutral lipids, including but not limited to distearoylphosphatidylcholine (DSPC), dioleoylphosphatidylcholine (DOPC), dipalmitoylphosphatidylcholine (DPPC), dioleoylphosphatidylglycerol (DOPG), dipalmitoyl (DP), dioleoylphosphatidylethanolamine (DOPE), palmitoyloleoylphosphatidylcholine (POPC), palmitoyloleoylphosphatidylethanolamine (POPE), N-(4-(p-maleimidophenyl)butyryl)-dioleoylphosphatidylethanolamine (DOPE-mal), dipalmitoylphosphatidylethanolamine (DPPE), dimyristoylphosphatidylethanolamine (DMPE), distearoylphosphatidylethanolamine (DSPE), 16-O-monomethyl PE, 16-O-dimethyl PE, 18-1-trans PE, 1-stearoyl-2-oleoyl-phosphatidylethanolamine (SOPE), cholesterol, or mixtures thereof. The non-cationic lipid may constitute about 5 mol% to about 90 mol%, about 10 mol%, or about 58 mol% (when cholesterol is included) of the total lipid present in the particle.

Lipids conjugated to prevent particle aggregation may include, for example, polyethylene glycol (PEG)-lipids, comprising but not limited to PEG-diacylglycerol (PEG-DAG), PEG-dialkoxypropyl (PEG-DAA), PEG-phospholipid, PEG-ceramide (PEG-Cer), or mixtures thereof. PEG-DAA conjugates may include, for instance, PEG-didodecyloxypropyl (C12), PEG-dimyristyloxypropyl (C14), PEG-dipalmityloxypropyl (C14), or PEG-distearoyloxypropyl (C18). The lipid conjugated to prevent particle aggregation may constitute 0 mol% to about 20 mol%, or about 2 mol% of the total lipid present in the particle. In some embodiments, the nucleic acid-lipid particles further comprise cholesterol, for example constituting about 10 mol% to about 60 mol%, or about 48 mol% of the total lipid present in said particle.

In one embodiment, the lipidoid ND98·4HCl (molecular weight 1487) (see U.S. Patent Application No. 12/056,230, incorporated herein by reference in its entirety), cholesterol (Sigma-Aldrich), and PEG-ceramide C16 (Avanti Polar Lipids) may be utilized to prepare lipid-dsRNA nanoparticles (i.e., LNP01 particles). Each stock solution in ethanol may be prepared as follows: ND98 at 133 mg/mL; cholesterol at 25 mg/mL; PEG-ceramide C16 at 100 mg/mL. The stock solutions of ND98, cholesterol, and PEG-ceramide C16 may then be mixed at a mol ratio of, for example, 42:48: 10. The combined lipid solution may be mixed with aqueous siRNA (e.g., in sodium acetate buffer at pH 5) such that the final ethanol concentration is about 35-45% and the final sodium acetate concentration is about 100-300 mM. Lipid-siRNA nanoparticles typically form spontaneously upon mixing.

Other exemplary lipid-siRNA formulations are available from *e.g.* WO2009/127060(SNALP), PCT/US2010/022614(XTC), US 2010/0324120(MC3), PCT/US09/63933(ALNY-100) and WO2010/129709(C12-200).

Pharmaceutical compositions suitable for injectable use comprise, for example, sterile aqueous solutions or dispersions and sterile powders for the extemporaneous preparation of sterile injectable solutions or dispersions. Generally, such formulations are sterile and possess sufficient fluidity for ease of syringeability. The formulations shall remain stable under manufacturing and storage conditions and shall be preserved against the contaminating action of microorganisms such as bacteria and fungi. Suitable solvents or dispersion media may include, for instance, water, ethanol, polyhydric alcohols (e.g., glycerol, propylene glycol, liquid polyethylene glycols, and the like), suitable mixtures thereof, and vegetable oils. Appropriate fluidity may be maintained, for example, using a coating material such as lecithin, by maintaining required particle size in dispersions, or by employing surfactants. Prevention of microbial action may be

achieved through various antibacterial and antifungal agents, including but not limited to parabens, chlorobutanol, phenol, sorbic acid, thimerosal, and the like. In many cases, it is preferable to include isotonic agents, such as sugars or sodium chloride. Prolonged absorption of injectable compositions may be accomplished by incorporating agents delaying absorption in the composition, for example, aluminum monostearate and gelatin.

Sterile injectable solutions may be prepared by incorporating the requisite quantity of the active compound with any additional ingredients (e.g., those enumerated supra) into an appropriate solvent, followed by filtration sterilization. Generally, dispersions are prepared by incorporating various sterilized active ingredients into a sterile vehicle medium containing essential dispersion agents and other requisite components, for instance, as described above. In the case of sterile powders for preparing sterile injectable solutions, preferred preparation methods include vacuum desiccation and lyophilization techniques, which yield powders of the active ingredient(s) together with any additional desired constituents from their prior sterile-filtered solutions.

The compositions of the present invention may generally be formulated in neutral form or as pharmaceutically acceptable salts. Such salts include, for example, acid addition salts (formed with free amino groups) derived from inorganic acids (e.g., hydrochloric or phosphoric acids) or organic acids (e.g., acetic, oxalic, tartaric, mandelic acids, etc.). Salts formed with free carboxyl groups may likewise be derived from inorganic bases (e.g., sodium, potassium, ammonium, calcium, or ferric hydroxides) or organic bases (e.g., isopropylamine, trimethylamine, histidine, procaine, etc.). In some embodiments, the RNAi agent of the invention is formulated as a sodium salt.

For parenteral administration in aqueous solution form, for example, the solution is typically appropriately buffered, and the liquid diluent is first rendered isotonic with sufficient saline or glucose, for instance. Such aqueous solutions may be employed for intravenous, intramuscular, subcutaneous, and intraperitoneal administration, among others. Preferably, sterile aqueous media are utilized. By way of example, a unit dose may be dissolved in 1 mL of isotonic NaCl solution and either added to 1000 mL of subcutaneous infusion fluid or injected at the proposed infusion site. For administration to humans, the formulations shall comply with sterility, pyrogenicity, general safety, and purity standards as required by local Food and Drug Administration regulations. In certain embodiments, the pharmaceutical composition of the invention comprises the sterile saline solution described herein and the RNAi agent, or consists essentially of these components. In other embodiments, the pharmaceutical composition of the invention comprises the RNAi agent described herein and sterile water (e.g., Water for Injection, WFI), or consists essentially thereof. In yet other embodiments, the pharmaceutical composition of the invention comprises the RNAi agent described herein and Phosphate-Buffered Saline (PBS), or consists essentially thereof.

In certain embodiments, the pharmaceutical compositions of the present invention are packaged with or stored within an administration device. Devices for injectable formulations comprise, but are not limited to, injection ports, pre-filled syringes, auto-injectors, infusion pumps, implantable injectors, and injection pens. Devices for nebulized or powdered formulations comprise, but are not limited to, inhalers, insufflators, nebulizers, and the like. Accordingly, the present invention encompasses an administration device containing the pharmaceutical composition of the invention, for treating or preventing one or more diseases or disorders as described herein.

## Therapeutic Methods and Uses

The present invention provides a method for reducing or inhibiting the expression of a target gene in a cell (e.g., a hepatocyte) by contacting the cell with any of the RNAi agents described herein (e.g., any one of Formulas 201 to 356). The cell can be *in vitro* or *in vivo.* Target gene expression can be assessed by measuring the amount or level of target gene mRNA or the protein expressed by the target gene (target protein).

In certain embodiments, the expression of a target gene in a cell is reduced by the RNAi agent of the invention by at least 40%, at least 45%, or at least 50%. In some embodiments, the expression of a target gene in a cell is reduced by the RNAi agent of the invention by at least 60%, at least 65%, at least 70%, at least 75%, at least 80%, or at least 85%. In other embodiments, the expression of a target gene in a cell is reduced by the RNAi agent of the invention by about 90% or more, e.g., 91%, 92%, 93%, 94%, 95%, 96%, 97%, 98%, 99%, or more. The percentage reduction in target gene expression can be measured by any of the methods described herein, as well as other methods known in the art. In some embodiments, the cell is not a liver cell.

In some embodiments, the present invention enables targeted delivery of oligonucleotide molecules to cells other than liver cells. In some embodiments, the RNAi agents provided by the invention can be targeted for delivery to skeletal muscle cells, type I alveolar epithelial cells, type II alveolar epithelial cells, goblet cells, secretory epithelial cells, ciliated epithelial cells, corneal epithelial cells, conjunctival epithelial cells, dermal epithelial cells, cholangiocytes, intestinal epithelial cells, ductal epithelial cells, glandular epithelial cells, or epithelial tumor cells.

In some embodiments, disclosed herein are methods for delivering an oligonucleotide molecule to skeletal muscle cells, type I alveolar epithelial cells, type II alveolar epithelial cells, goblet cells, secretory epithelial cells, ciliated epithelial cells, corneal epithelial cells, conjunctival epithelial cells, dermal epithelial cells, cholangiocytes, intestinal epithelial cells, ductal epithelial cells, glandular epithelial cells, or epithelial tumor cells *in vivo,* wherein the method

comprises administering to a subject one or more RNAi agents of the invention. In some embodiments, disclosed herein are methods for inhibiting the expression of a target gene in skeletal muscle cells, type I alveolar epithelial cells, type II alveolar epithelial cells, goblet cells, secretory epithelial cells, ciliated epithelial cells, corneal epithelial cells, conjunctival epithelial cells, dermal epithelial cells, cholangiocytes, intestinal epithelial cells, ductal epithelial cells, glandular epithelial cells, or epithelial tumor cells *in vivo,* wherein the method comprises administering to a subject one or more RNAi agents of the invention.

## Examples

### Example 1: Synthesis of Small Molecule Ligand Compounds

Compound **1** was synthesized according to patent WO2019089765A1

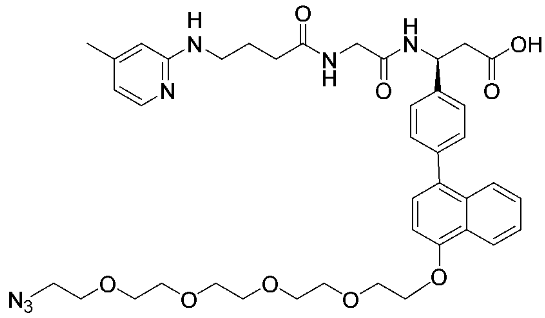

**1**

LCMS: (ESI) *m/z* = 786.4 $[M+H]^+$; 379.9 $[M+2H]/2^+$
HPLC: RT = 11.459 min
Mobile Phase: A: Water (0.05% TFA) B: ACN (0.05% TFA)
Gradient: 20% B to 80% B over 20 minutes, then to 95% B over 1 minute, hold for 5 minutes, then to 5% B over 0.1 minutes.
Flow Rate: 1 mL/min
Column: XBridge Peptide BEH C18, 4.6 x 150 mm, 3.5 μm, 300 Å
Column Temperature: 40 °C

Compound **2** was synthesized according to literature (Anderson NA, Campbell IB, Fallon BJ, Lynn SM, Macdonald SJ, Pritchard JM, Procopiou PA, Sollis SL, Thorp LR. Synthesis and determination of absolute configuration of a non-peptidic αvβ6 integrin antagonist for the treatment of idiopathic pulmonary fibrosis. Org Biomol Chem. 2016 Jul 7;14(25):5992-6009. doi: 10.1039/c6ob00496b. Epub 2016 May 26. PMID: 27226381).

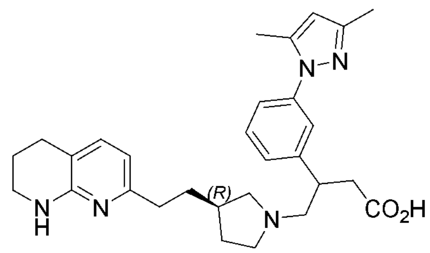

**2**

50 mg of Compound **2** was purified by SFC (Column: OJ-H 4.6*100mm, 5μm), Mobile Phase: [Carbon dioxide - Methanol (0.2% Ammonia, 7M in MeOH)], B%: 20%, isocratic elution mode) to afford white solids **isomer1: 2-P1** (Minor) and **isomer2: 2-P2** (Major).

**Isomer1**: 2.4 mg

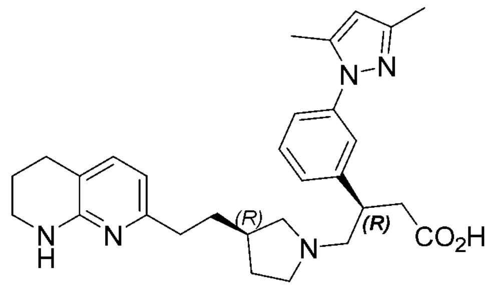

**2-P1**

MS *m/z* (ESI): 488.2 [M+H]+;

Analytical Chiral SFC: RT = 1.591 min, >99.5%, Column: OJ-H 4.6*100mm, 5μm, Carbon dioxide - Methanol (0.2% Ammonia, 7M in MeOH), B%: 20%, Flow rate 3 mL/min. $^1$H NMR (400 MHz, DMSO-*d6*) δ 7.38 (dd, *J* = 7.6, 8.0 Hz, 1H), 7.33 (s, 1H), 7.29 (d, *J* = 8.0 Hz, 1H), 7.24 (d, *J* = 8.0 Hz, 1H), 7.00 (d, *J* = 7.2 Hz, 1H), 6.25 (s, 1H, NH), 6.23 (d, *J* = 7.6 Hz, 1H), 6.05 (s, 1H), 3.31-3.25 (m, 1H), 3.24-3.18 (m, 2H), 2.89-2.74 (m, 4H), 2.68-2.55 (m, 4H), 2.48-2.31 (m, 4H), 2.27 (s, 3H), 2.17 (s, 3H), 2.06-1.96 (m, 1H), 1.94-1.82 (m, 1H), 1.77-1.69 (m, 2H), 1.64-1.53 (m, 2H), 1.38-1.28 (m, 1H).

**Isomer2:** 15 mg

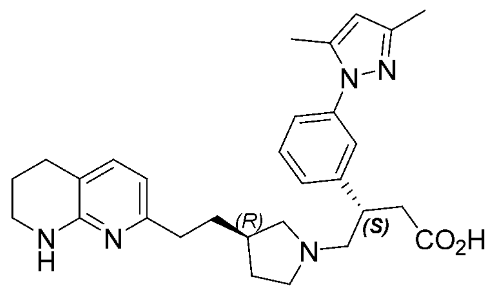

**2-P2**

MS *m/z* (ESI): 488.5 [M+H]+;

Analytical Chiral SFC: RT = 2.485 min, >99.5%, Column: OJ-H 4.6*100mm, 5μm, Carbon dioxide - Methanol (0.2% Ammonia, 7M in MeOH), B%: 20%, Flow rate 3 mL/min. $^1$H NMR (400 MHz, DMSO-*d6*+$D_2O$) δ 7.41 (dd, *J* = 7.6, 8.0 Hz, 1H), 7.33 (s, 1H), 7.30 (d, *J* = 8.0 Hz, 1H), 7.25 (d, *J* = 8.0 Hz, 1H), 7.03 (d, *J* = 7.6 Hz, 1H), 6.26 (d, *J* = 7.6 Hz, 1H), 6.07 (s, 1H), 3.33-3.25 (m, 1H), 3.24-3.19 (m, 2H), 2.99-2.91 (m, 1H), 2.90-2.84 (m, 1H), 2.84-2.72 (m, 2H), 2.69-2.56 (m, 4H), 2.48-2.32 (m, 4H), 2.27 (s, 3H), 2.18 (s, 3H), 2.09-1.99 (m, 1H), 1.97-1.86 (m, 1H), 1.78-1.70 (m, 2H), 1.65-1.54 (m, 2H), 1.42-1.31 (m, 1H).

Synthesis of Compound **4**

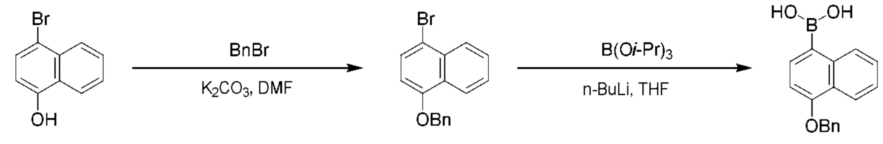


4-1 4-2 4-3

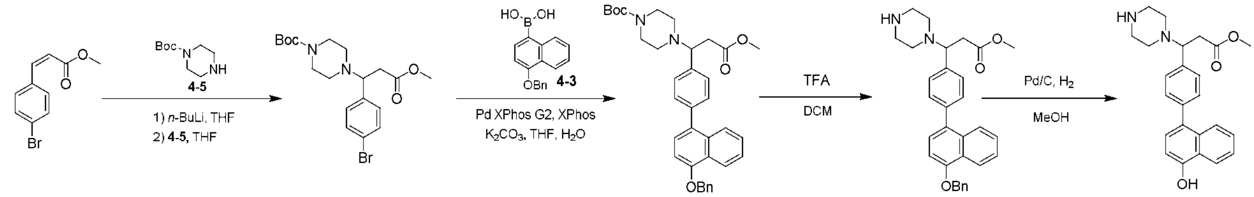


4-4 4-6 4-7 4-8 4-9

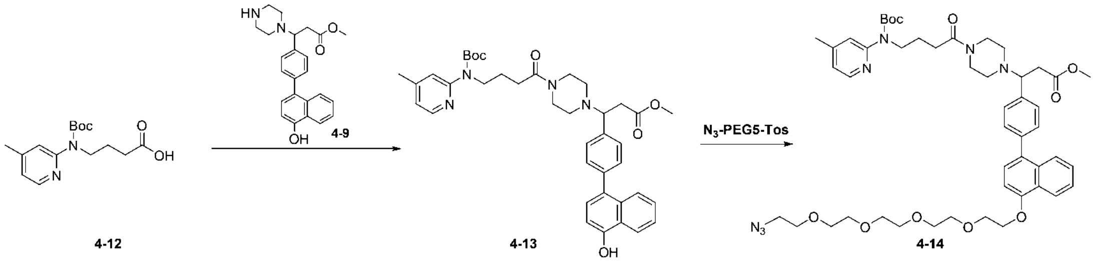


4-12 4-13 4-14

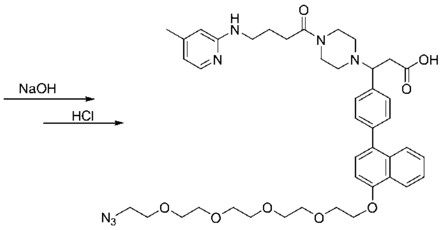


4

Synthesis of Intermediate **4-2**

At 25 °C, Compound **4-1** (5.00 g, 22.41 mmol) was dissolved in N,N-Dimethylformamide (50 mL), potassium carbonate (4.65 g, 33.62 mmol) and benzyl bromide (3.83 g, 22.41 mmol, 2.66 mL) were added, and the mixture was stirred at 25 °C for 4 hours. After completion of the reaction, the mixture was quenched by adding saturated ammonium chloride solution (10 mL), diluted with water (200 mL), and extracted with ethyl acetate (200 mL). The organic phases were combined, washed with saturated brine, dried over anhydrous sodium sulfate, filtered, and evaporated to dryness to obtain white solid Compound **4-2** (5.00 g, 71.2% yield). $^1$H NMR (400 MHz, DMSO-$d_6$) δ 8.28 (d, *J* = 8.00 Hz, 1H), 8.08 (d, *J* = 8.38 Hz, 1H), 7.77 (d, *J* = 8.38 Hz, 1H), 7.71 (m, *J* = 8.32, 7.00, 1.19 Hz, 1H), 7.65 - 7.59 (m, 1H), 7.55 (d, *J* = 7.13 Hz, 2H), 7.47 - 7.40 (m, 2H), 7.40 - 7.32 (m, 1H), 7.03 (d, *J* = 8.38 Hz, 1H), 5.31 (s, 2H).

Synthesis of Intermediate **4-3**

At 25 °C, Compound **4-2** (8.33 g, 26.59 mmol) was dissolved in tetrahydrofuran (140 mL), then purged with nitrogen 3 times, n-butyllithium (2.5 M, 12.49 mL) was added at -78 °C and stirred at -78 °C for half an hour, finally triisopropyl borate (5.00 g, 26.59 mmol, 6.11 mL) was added dropwise, and the reaction solution was slowly warmed to room temperature and stirred for 1.5 hours. After completion of the reaction, the mixture was quenched by adding saturated ammonium chloride solution (150 mL), diluted with water (300 mL), and extracted with ethyl acetate (300 mL). The organic phases were combined, washed with saturated brine, dried over anhydrous sodium sulfate, filtered, and evaporated to dryness to obtain yellow oily Compound **4-3** (6.00 g, 81.2% yield). MS *m/z* (ESI): 277.0 [M-H]$^-$.

Synthesis of Intermediate **4-6**

At 25 °C, N-BOC piperazine (**4-5**, 17.18 g, 92.25 mmol) was dissolved in tetrahydrofuran (150 mL), then purged with nitrogen 3 times, n-butyllithium (2.5 M, 35.75 mL) was added at -78 °C and stirred at -78 °C for half an hour, finally Compound **4-4** (13.9 g, 57.66 mmol) was added dropwise and the reaction solution was stirred at -78 °C for 1.5 hours. After

completion of the reaction, the mixture was quenched by adding saturated ammonium chloride solution (100 mL), diluted with water (200 mL), and extracted with ethyl acetate (200 mL). The organic phases were combined, washed with saturated brine, dried over anhydrous sodium sulfate, filtered, and evaporated to dryness. The residue was purified by flash silica gel chromatography (eluent 0~40% tetrahydrofuran/petroleum ether) to obtain white solid Compound **4-6** (6.00 g, 24.4% yield). MS *m/z* (ESI): 427.2 $[M+H]^+$; $^1H$ NMR (400 MHz, DMSO-$d_6$) δ 7.52 (d, *J* = 8.25 Hz, 2H), 7.24 (d, *J* = 8.38 Hz, 2H), 3.98 (t, *J* = 7.75 Hz, 1H), 3.52 (s, 3H), 3.23 (d, *J* = 3.38 Hz, 4H), 3.06 - 2.71 (m, 2H), 2.33 - 2.14 (m, 4H), 1.34 (s, 9H).

Synthesis of Intermediate **4-7**

At 25 °C, Compound **4-3** (3.58 g, 12.87 mmol), **4-6** (5.50 g, 12.87 mmol) and potassium carbonate (5.34 g, 38.61 mmol) were dissolved in tetrahydrofuran (80 mL) and water (10 mL), then purged with nitrogen 3 times, chloro(2-dicyclohexylphosphino-2',4',6'-triisopropyl-1,1'-biphenyl)[2-(2'-amino-1,1'-biphenyl)]palladium(II) (1.01 g, 1.29 mmol) and 2-dicyclohexylphosphino-2',4',6'-triisopropylbiphenyl (1.23 g, 2.57 mmol) were added and stirred at 60 °C for 8 hours. After completion of the reaction, the mixture was diluted with water (100 mL) and extracted with ethyl acetate (100 mL). The organic phases were combined, washed with saturated brine, dried over anhydrous sodium sulfate, filtered, and evaporated to dryness. The residue was purified by flash silica gel chromatography (eluent 0~70% tetrahydrofuran/petroleum ether) to obtain yellow oily Compound **4-7** (6.60 g, 88.3% yield). MS *m/z* (ESI): 581.4 $[M+H]^+$; $^1H$ NMR (400 MHz, DMSO-$d_6$) δ 8.37 - 8.29 (m, 1H), 7.83 - 7.75 (m, 1H), 7.59 (d, *J* = 7.13 Hz, 2H), 7.54 (m, *J* = 7.82, 5.75, 1.56 Hz, 2H), 7.48 - 7.43 (m, 2H), 7.42 - 7.32 (m, 6H), 7.15 (d, *J* = 8.13 Hz, 1H), 5.37 (s, 2H), 4.11 (t, *J* = 7.69 Hz, 1H), 3.58 (s, 3H), 3.28 (d, *J* = 3.13 Hz, 4H), 3.10 (dd, *J* = 14.88, 7.75 Hz, 1H), 2.83 (dd, *J* = 15.01, 7.63 Hz, 1H), 2.46 - 2.38 (m, 2H), 2.32 - 2.25 (m, 2H), 1.36 (s, 9H).

Synthesis of Intermediate **4-8**

At 25 °C, Compound **4-7** (5.00 g, 8.61 mmol) was dissolved in dichloromethane (50 mL), then trifluoroacetic acid (12 mL) was added and stirred at 25 °C for 1 hour. After completion of the reaction, the mixture was evaporated to dryness to obtain black solid Compound **4-8** (4.00 g, 96.7% yield). MS m/z (ESI): 481.3 $[M+H]^+$.

Synthesis of Intermediate **4-9**

At 25 °C, Compound **4-8** (4.00 g, 8.32 mmol) was dissolved in methanol (50 mL), then purged with nitrogen 3 times, wet palladium on carbon (4.43 g, 10% purity) was added followed by purging with hydrogen 3 times, and stirred under hydrogen atmosphere (20 psi) at 25 °C for 12 hours. After completion of the reaction, the mixture was filtered and evaporated to dryness to obtain crude product. The crude product was purified by reversed-phase high performance liquid chromatography (0.1% trifluoroacetic acid) to obtain white solid Compound **4-9** (1.00 g, 30.8% yield). MS *m/z* (ESI): 391.2 $[M+H]^+$; $^1H$ NMR (400 MHz, DMSO-$d_6$) δ 10.41 - 10.22 (m, 1H), 8.46 (s, 2H), 8.26 - 8.18 (m, 1H), 7.82 - 7.71 (m, 1H), 7.53 - 7.38 (m, 6H), 7.23 (d, *J* = 7.75 Hz, 1H), 6.95 (d, *J* = 7.75 Hz, 1H), 4.23 (t, *J* = 7.63 Hz, 1H), 3.59 (s, 3H), 3.18 - 2.88 (m, 7H), 2.76 - 2.56 (m, 4H).

Synthesis of Intermediate **4-13**

**4-12** (80 mg, 0.27 mmol) was dissolved in DMF (3 mL), then **4-9** (100 mg, 0.26 mmol), EDCI (67 mg, 0.35 mmol), HOBT (47 mg, 0.35 mmol) and DIPEA (208 mg, 1.56 mmol) were added sequentially. The reaction solution was stirred at room temperature for 3 hours. The reaction mixture was directly purified by preparative chromatography column (Xbridge C18, 19 x 250 mm, 10 μm, 130 Å; Mobile phase: A: Water (containing 10 mmol/L $NH_4HCO_3$), B: ACN; Gradient: 35% B to 95% B over 20 minutes) to obtain **4-13** (39 mg, yield: 25%) as a white solid. LCMS: (ESI) *m/z* = 667.5 $[M+H]^+$.

Synthesis of Intermediate **4-14**

**4-13** (33 mg, 0.048 mmol) was dissolved in DMF (5 mL), and **$N_3$-PEG5-Tos** (azido-triethylene glycol-carboxylic acid) (40 mg, 0.096 mmol) was added. The reaction solution was stirred at 75 °C for 16 hours. The reaction mixture was directly purified by preparative chromatography column (Xbridge C18, 19 x 250 mm, 10 μm, 130 Å; Mobile phase: A: Water (containing 10 mmol/L $NH_4HCO_3$) B: ACN; Gradient: 35% B to 95% B over 20 minutes) to obtain **4-14** (38 mg, yield: 83%) as a white solid. LCMS: (ESI) *m/z* = 913.3 $[M+H]^+$.

Synthesis of Compound **4**

**4-14** (35 mg, 0.038 mmol) was dissolved in THF (1 mL) and $H_2O$ (1 mL), and $LiOH.H_2O$ (6 mg, 0.15 mmol) was

added. The reaction solution was stirred at room temperature for 16 hours. LCMS monitored complete reaction. The reaction mixture was acidified with 3 N dilute hydrochloric acid and concentrated. The concentrated compound was dissolved in DCM (3 mL), and ice-cold TFA (3 mL) was added. The reaction solution was stirred at room temperature for 16 hours. LCMS monitored complete reaction. The reaction solution was concentrated and dried in vacuo to obtain crude product **4,** which was used directly in the next step. LCMS: (ESI) *m/z* = 798.3 [M+H]+.

**Synthesis of Compound 5**

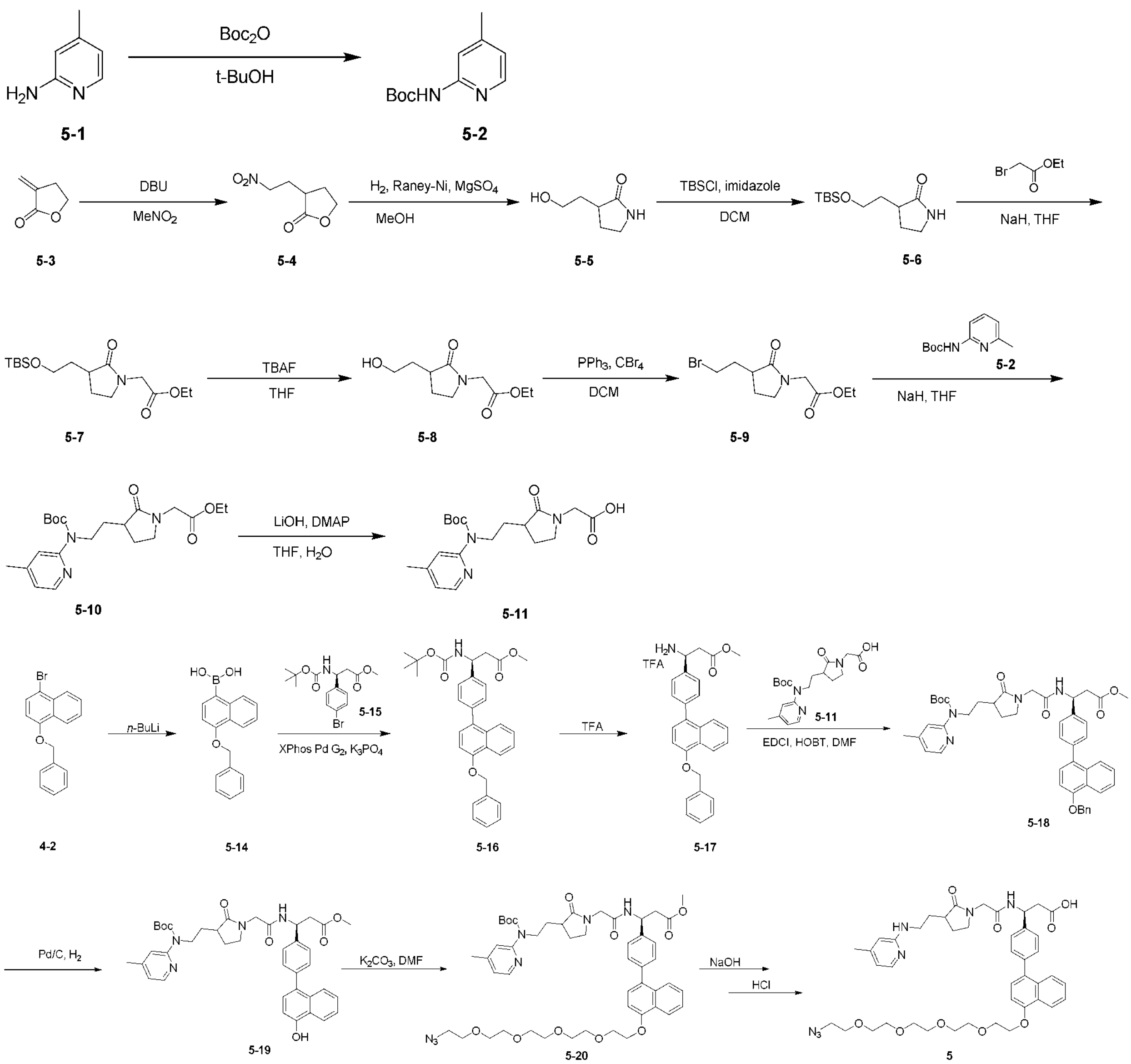


Synthesis of Intermediate **5-2**

At room temperature, Compound **5-1** (20.0 g, 185 mmol) was dissolved in tert-butanol (200 mL), di-tert-butyl dicarbonate (44.4 g, 203 mmol, 46.7 mL) was added, and the reaction solution was stirred at 25 °C for 3 hours. The reaction solution was concentrated. The residue was slurried with 200 mL of methyl tert-butyl ether. The filtrate was concentrated and dried. The residue was recrystallized from isopropanol to obtain white crystalline Compound **5-2** (23.0 g, 59.7% yield). $^1H$ NMR (400 MHz, $CDCl_3$) δ 9.42 (s, 1H), 8.28 - 8.07 (m, 1H), 7.85 (s, 1H), 6.77 (d, *J* = 5.0 Hz, 1H), 2.35 (s, 3H), 1.55 (s, 9H).

Synthesis of Intermediate **5-4**

At room temperature, Compound **5-3** (24.0 g, 245 mmol) was added to nitromethane (360 mL), followed by 1,8-diazabicyclo[5.4.0]undec-7-ene (4.84 g, 31.8 mmol). The reaction solution was stirred at 25 °C for 16 hours. After the reaction, the reaction solution was concentrated. The obtained residue was dissolved in 800 mL of dichloromethane. It was washed sequentially with 3.0 M hydrochloric acid (500 mL), water (500 mL), saturated aqueous sodium bicarbonate solution (500 mL), and saturated brine (500 mL). The organic phase was dried over anhydrous sodium sulfate, filtered, and concentrated to obtain amber oily Compound **5-4** (30.0 g, 77.1% yield). $^1$H NMR (400 MHz, $CDCl_3$) δ 4.74 - 4.51 (m, 2H), 4.42 - 4.33 (m, 1H), 4.25 - 4.14 (m, 1H), 2.72 - 2.59 (m, 1H), 2.52 - 2.37 (m, 2H), 2.27 - 2.16 (m, 1H), 2.05 - 1.93 (m, 1H).

Synthesis of Intermediate **5-5**

At room temperature, Compound **5-4** (28.0 g, 176 mmol), magnesium sulfate (23.3 g, 194 mmol) and methanol (60 mL) were added to a stainless steel hydrogenation bottle. Raney nickel (21.1 g) was slowly added under nitrogen flow. After addition, the mixture was purged with hydrogen three times and reacted under hydrogen atmosphere (50 psi) at 50 °C for 30 hours. After the reaction, the mixture was filtered, and the filtrate was concentrated to obtain brown oily Compound **5-5** (30.5 g, crude). $^1$H NMR (400 MHz, $CDCl_3$) δ 7.08 (s, 1H), 4.53 (s, 1H), 3.71 - 3.57 (m, 2H), 3.37 - 3.26 (m, 2H), 2.57 - 2.46 (m, 1H), 2.35 - 2.24 (m, 1H), 1.89 - 1.76 (m, 2H), 1.65 (m, *J* = 5.3, 9.3, 14.4 Hz, 1H).

Synthesis of Intermediate **5-6**

At room temperature, Compound **5-5** (37.0 g, 286 mmol), imidazole (48.8 g, 716 mmol) and tert-butyldimethylchlorosilane (64.8 g, 430 mmol) were added to dichloromethane (370 mL), and the reaction solution was stirred at 25 °C for 8 hours. The reaction solution was poured into 500 mL of water and extracted with dichloromethane (500 mL × 2). The combined organic phases were dried over anhydrous sodium sulfate, filtered, and concentrated to obtain a residue, which was purified by flash silica gel chromatography (eluent 0~50% ethyl acetate/petroleum ether) to obtain colorless liquid Compound **5-6** (26.4 g, 37.9% yield). $^1$H NMR (400 MHz, $CDCl_3$) δ 6.35 (s, 1H), 3.81 - 3.66 (m, 2H), 3.39 - 3.26 (m, 2H), 2.47 (m, *J* = 4.3, 9.2 Hz, 1H), 2.38 - 2.27 (m, 1H), 2.12 (m, *J* = 4.4, 6.6, 13.6 Hz, 1H), 1.84 (m, *J* = 8.9, 12.5 Hz, 1H), 1.58 - 1.45 (m, 1H), 0.89 (s, 9H), 0.05 (s, 6H).

Synthesis of Intermediate **5-7**

Sodium hydride (4.77 g, 119 mmol, 60% purity) was added to tetrahydrofuran (130 mL) under nitrogen flow. The reactor was purged with nitrogen 3 times and then cooled to 0 °C. A solution of Compound **5-5** (26.4 g, 108 mmol) previously dissolved in tetrahydrofuran (130 mL) was added to the reaction solution, and the reaction solution was stirred at 0 °C for 30 minutes. Subsequently, ethyl 2-bromoacetate (19.0 g, 114 mmol) was added at 0 °C, and the reaction solution was stirred at 25 °C for 16 hours. After the reaction, the reaction was quenched with saturated aqueous ammonium chloride solution (500 mL) at 25 °C, and extracted with ethyl acetate (400 mL × 2). The combined organic phases were washed with saturated brine (400 mL), dried over anhydrous sodium sulfate, filtered, and concentrated to obtain pale yellow liquid Compound **5-7** (34.0 g, 99.4% yield). $^1$H NMR (400 MHz, $CDCl_3$) δ 4.18 (q, *J* = 7.1 Hz, 2H), 4.12 - 3.96 (m, 2H), 3.80 - 3.69 (m, 2H), 3.47 - 3.34 (m, 2H), 2.58 (m, *J* = 4.6, 8.9 Hz, 1H), 2.33 - 2.22 (m, 1H), 2.19 - 2.09 (m, 1H), 1.80 (m, *J* = 8.7, 12.5 Hz, 1H), 1.54 (dt, *J* = 5.9, 14.5 Hz, 1H), 1.27 (t, *J* = 7.1 Hz, 3H), 0.89 (s, 9H), 0.05 (s, 6H).

Synthesis of Intermediate **5-8**

Compound **5-7** (45.0 g, 143 mmol) was dissolved in tetrahydrofuran (675 mL), and a solution of tetrabutylammonium fluoride (1 M, 172 mL) in tetrahydrofuran was added at 0 °C. The reaction solution was stirred at 25 °C for 4 hours. The reaction solution was concentrated. The residue was purified by flash silica gel chromatography (eluent 0~40% tetrahydrofuran/petroleum ether) to obtain pale orange liquid Compound **5-8** (14.8 g, 51.6% yield). $^1$H NMR (400 MHz, $CDCl_3$) δ 4.19 (q, *J* = 7.1 Hz, 2H), 4.14 - 3.95 (m, 2H), 3.87 - 3.70 (m, 3H), 3.55 - 3.46 (m, 1H), 3.46 - 3.39 (m, 1H), 2.66 (m, *J* = 5.7, 8.9 Hz, 1H), 2.35 - 2.24 (m, 1H), 1.92 (m, *J* = 4.5, 9.0, 13.9 Hz, 1H), 1.86 - 1.72 (m, 2H), 1.27 (t, *J* = 7.1 Hz, 3H).

Synthesis of Intermediate **5-9**

Compound **5-8** (16.2 g, 80.5 mmol) was dissolved in dichloromethane (100 mL). While stirring, triphenylphosphine (31.7 g, 121 mmol) and a solution of carbon tetrabromide (32.0 g, 96.6 mmol) previously dissolved in dichloromethane (100 mL) were added sequentially, and stirred at 25 °C for 4 hours. The reaction solution was concentrated. The residue was purified by flash silica gel chromatography (eluent 0~50% ethyl acetate/petroleum ether) to obtain colorless

liquid Compound **5-9** (18.2 g, 85.6% yield). $^1$H NMR (400 MHz, $CDCl_3$) δ 4.18 (q, *J* = 7.1 Hz, 2H), 4.04 (s, 2H), 3.63 (td, *J* = 6.6, 10.0 Hz, 1H), 3.55 - 3.48 (m, 1H), 3.47 - 3.36 (m, 2H), 2.68 (m, *J* = 6.1, 8.7 Hz, 1H), 2.48 - 2.37 (m, 1H), 2.36 - 2.26 (m, 1H), 1.98 - 1.86 (m, 1H), 1.74 (m, *J* = 8.8, 12.5 Hz, 1H), 1.27 (t, *J* = 7.1 Hz, 3H).

Synthesis of Intermediate **5-10**

Under nitrogen flow, sodium hydride (2.75 g, 68.8 mmol, 60% purity) was added to N,N-dimethylformamide (15 mL), then the reaction solution was cooled to 0 °C. A solution of Compound **5-2** (13.3 g, 63.7 mmol) previously dissolved in N,N-dimethylformamide (15 mL) was added to the reaction solution, and stirred at 0 °C for 30 minutes under nitrogen flow. Subsequently, Compound **5-9** (18.0 g, 68.2 mmol) was added, and stirred at 25 °C for 3 hours. After the reaction, the reaction was quenched with saturated aqueous ammonium chloride solution (100 mL) at 25 °C, then 100 mL of water was added, and extracted with ethyl acetate (250 mL × 2). The organic phases were combined, washed with saturated brine (500 mL), dried over anhydrous sodium sulfate, filtered, and concentrated. The residue was purified by flash silica gel chromatography (eluent 0~60% ethyl acetate/petroleum ether) to obtain colorless oil **5-10** (16.1 g, 64.6% yield). $^1$H NMR (400 MHz, $CDCl_3$) δ 8.20 (d, *J* = 4.9 Hz, 1H), 7.37 (s, 1H), 6.83 (d, *J* = 4.8 Hz, 1H), 4.16 (q, *J* = 7.1 Hz, 2H), 4.09 - 3.93 (m, 4H), 3.45 - 3.32 (m, 2H), 2.51 - 2.40 (m, 1H), 2.32 (s, 3H), 2.29 - 2.15 (m, 2H), 1.80 (m, *J* = 8.4, 12.5 Hz, 1H), 1.62 (m, *J* = 4.6, 8.9, 13.6 Hz, 1H), 1.49 (s, 9H), 1.28 - 1.22 (m, 3H).

Synthesis of Intermediate **5-11**

At room temperature, Compound **5-10** (5.00 g, 12.8 mmol) was dissolved in tetrahydrofuran (40 mL), and water (10 mL), lithium hydroxide monohydrate (2.14 g, 51.09 mmol) and 4-dimethylaminopyridine (156 mg, 1.28 mmol) were added. The reaction solution was stirred at 25 °C for 2 hours. Water (100 mL) and dichloromethane (100 mL) were added to the reaction solution. The aqueous phase was taken, and saturated aqueous citric acid solution was added dropwise to adjust the pH to about 5, followed by extraction with dichloromethane (100 mL × 2). The organic phases were combined, dried over anhydrous sodium sulfate, filtered, and concentrated. The residue was purified by flash silica gel chromatography (eluent 0~30% tetrahydrofuran/petroleum ether) to obtain a colorless oily liquid, which was finally slurried with methyl tert-butyl ether to obtain white solid Compound **5-11** (2.00 g, 43.1% yield). MS *m/z* (ESI): 378.1 $[M+H]^+$; $^1$H NMR (400 MHz, $CDCl_3$) δ 8.74 - 8.36 (m, 1H), 8.26 (d, *J* = 5.1 Hz, 1H), 7.31 (s, 1H), 6.90 (d, *J* = 5.0 Hz, 1H), 4.11 - 3.87 (m, 4H), 3.48 - 3.34 (m, 2H), 2.50 (m, *J* = 4.1, 9.0 Hz, 1H), 2.34 (s, 3H), 2.29 - 2.19 (m, 1H), 2.19 - 2.08 (m, 1H), 1.82 - 1.73 (m, 1H), 1.69 - 1.57 (m, 1H), 1.48 (s, 9H).

Synthesis of Intermediate **5-14**

**4-2** (1.57 g, 5.0 mmol, 1.0 equiv.) was dissolved in THF (25 mL), cooled to -78 °C in a dry ice-acetone bath, n-butyllithium (2.4 mL, 6.0 mmol) was added dropwise. After addition, stirring was continued at -78 °C for 10 minutes, then triisopropyl borate (1.22 g, 6.5 mmol) was added dropwise. After addition, the reaction solution was slowly warmed to room temperature and stirred for another 2 hours. The reaction solution was quenched with 2 N hydrochloric acid (10 mL), extracted with ethyl acetate (100 mL), washed with saturated brine (20 mL), dried over anhydrous sodium sulfate, filtered, and concentrated. The residue was dissolved in ethyl acetate (5 mL), and petroleum ether (50 mL) was added dropwise with stirring. A white precipitate formed, which was filtered and dried to obtain **5-14** (1.0 g, yield: 71.9%) as a white solid. LCMS: (ESI) *m/z* = 279.3 $[M+H]^+$, 301.1 $[M+Na]^+$.

Synthesis of Intermediate **5-16**

**5-14** (0.582 g, 2.1 mmol, 1.5 equiv.), **5-15** (500 mg, 1.4 mmol), XPhos Pd G2 (220 mg, 0.28 mmol), potassium phosphate (594 mg, 2.8 mol) and tetrahydrofuran (10 mL) were added to a 100 mL flask. The system was purged with nitrogen twice and stirred at room temperature for 16 hours. Water (10 mL) was added to the reaction solution, extracted with ethyl acetate (50 mL x 2), washed with saturated brine (20 mL), filtered, dried over anhydrous sodium sulfate, and concentrated. The residue was purified by silica gel column to obtain **5-16** (640 mg, yield: 88.5%) as a pale yellow solid. LCMS: (ESI) *m/z* = 534.3 $[M+Na]^+$, 1045.4 $[2M+Na]^+$; $^1$H NMR (DMSO-*d6*, 400 MHz) δ 8.31 (d, *J* = 8.0 Hz, 1H), 7.78 (d, *J* = 8.4 Hz, 1H), 7.61-7.36 (m, 12 H), 7.33 (d, *J* = 8.4 Hz, 1H), 7.14 (d, *J* = 8.0 Hz, 1H), 5.36 (s, 2H), 5.09-4.99 (m, 1H), 3.61 (s, 3H), 2.87-1.74 (m, 2H), 1.39 (s, 9H).

Synthesis of Intermediate **5-17**

Intermediate **5-16** (0.64 g) was dissolved in dichloromethane (3 mL), trifluoroacetic acid (3 mL) was carefully added, and the mixture was stirred at room temperature for 2 hours. The reaction solution was concentrated, and the

residue was purified by reverse phase column to obtain **5-17** (600 mg, yield: 91.3%) as a white solid. LCMS: (ESI) *m*/*z* = 434.3 $[M+Na]^+$.

Synthesis of Intermediate **5-18**

**5-17** (118 mg, 0.27 mmol) was dissolved in DMF (3 mL), and **5-11** (100 mg, 0.27 mmol), EDCI (62 mg, 0.33 mmol), HOBT (44 mg, 0.33 mmol) and DIPEA (208 mg, 1.62 mmol) were added sequentially. The reaction solution was stirred at room temperature for 3 hours. The reaction mixture was directly purified by preparative chromatography column (Xbridge C18, 19 × 250 mm, 10 μm, 130 Å; Mobile phase: A: Water (containing 10 mmol/L $NH_4HCO_3$); B: ACN; Gradient: 35% B to 65% B over 20 minutes) to obtain **5-18** (103 mg, yield: 49%) as a white solid. LCMS: (ESI) *m*/*z* = 771.5 $[M+H]^+$.

Synthesis of Intermediate **5-19**

**5-18** (100 mg, 0.153 mmol) was dissolved in MeOH (5 mL), and Pd/C (26 mg, 10% purity) was added. The reaction solution was stirred under hydrogen flow at room temperature for 2 hours. The reaction mixture was filtered and concentrated to obtain crude product **5-19** as a white solid, which was used directly in the next step. LCMS: (ESI) *m*/*z* 681.2 $[M+H]^+$.

Synthesis of Intermediate **5-20**

**5-19** (100 mg, crude) was dissolved in DMF (5 mL), and **$N_3$-PEG5-Tos** (112 mg, 0.268 mmol) was added. The reaction solution was stirred at 75 °C for 16 hours. The reaction mixture was directly purified by preparative chromatography column (Xbridge C18, 19 × 250 mm, 10 μm, 130 Å; Mobile phase: A: Water (containing 10 mmol/L $NH_4HCO_3$); B: ACN; Gradient: 35% B to 95% B over 20 minutes) to obtain **5-20** (89 mg, yield: 73%) as a white solid. LCMS: (ESI) *m*/*z* = 926.5 $[M+H]^+$.

Synthesis of Compound **5**

**5-20** (87 mg, 0.094 mmol, 1.0 equiv.) was dissolved in THF (3 mL) and $H_2O$ (3 mL), and $LiOH \cdot H_2O$ (15 mg, 0.38 mmol) was added. The reaction solution was stirred at room temperature for 16 hours. LCMS monitored complete reaction. The reaction mixture was acidified with 3M dilute hydrochloric acid, concentrated, dried, dissolved in DCM (2 mL), and ice-cold TFA (2 mL) was added. The reaction solution was stirred at room temperature for 16 hours. LCMS monitored complete reaction, then concentrated and dried in vacuo to obtain Compound **5 (HPLC purity 95.27%).** LCMS: (ESI) *m*/*z* = 812.5 $[M+H]^+$. The compound was purified by SFC (Column: OD-3 4.6*100 mm, 3μm, Mobile phase: [Carbon dioxide - Methanol (0.2%$NH_3$ in MeOH)], B%: 50%, isocratic elution mode) to obtain yellow oily compound **isomer1: 5-P1** and yellow oily compound **isomer2: 5-P2.**

300 mg of Compound **5** was purified by SFC (Column: OD 25*250mm, 10μm, Mobile phase: [Carbon dioxide - Methanol (0.5% $NH_3$ in MeOH)], B%: 50%, isocratic elution mode) to obtain yellow solid compound **isomer1: 5-P1** and yellow solid compound **isomer2: 5-P2.**

**Isomer1:** 100 mg

MS *m*/*z* (ESI): 812.2 $[M+H]^+$;
Analytical Chiral SFC: RT = 2.004 min, >99.5%, OD-3 4.6*100 mm, 3μm, Carbon dioxide - Methanol (0.2% NH3 in MeOH)], B%: 40%, Flow rate 3 mL/min. $^1H$ NMR (500 MHz, $CDCl_3$) δ 9.46 (s, 1H), 9.16 (d, *J* = 7.1 Hz, 1H), 8.36 (d, *J* = 8.3 Hz, 1H), 7.87 (d, *J* = 8.4 Hz, 1H), 7.61 (d, *J* = 6.2 Hz, 1H), 7.55 - 7.32 (m, 7H), 7.28 (d, *J* = 7.3 Hz, 1H), 6.86 (d, *J* = 7.9 Hz, 1H), 6.49 - 6.36 (m, 2H), 5.39 (dt, *J* = 8.5, 4.4 Hz, 1H), 4.37 (dd, *J* = 15.3, 10.7 Hz, 3H), 4.12 - 3.98 (m, 2H), 3.84 (dd, *J* = 5.7, 3.8 Hz, 2H), 3.79 (d, *J* = 16.4 Hz, 1H), 3.76 - 3.60 (m, 12H), 3.58 - 3.41 (m, 4H), 3.41 - 3.34 (m, 2H), 2.91 (d, *J* = 4.5 Hz, 2H), 2.84 (dt, *J* = 16.0, 8.2 Hz, 1H), 2.33 (s, 3H), 2.30 - 2.19 (m, 1H), 2.19 - 1.98 (m, 3H).

**Isomer2:** 80 mg

MS *m*/*z* (ESI): 812.2 $[M+H]^+$;
Analytical Chiral SFC: RT = 2.564 min, 99.1%, OD-3 4.6*100 mm, 3μm, Carbon dioxide - Methanol (0.2% NH3 in

MeOH)], B%: 40%, Flow rate 3 mL/min. $^1H$ NMR (500 MHz, $CDCl_3$) δ 9.65 (s, 1H), 8.93 (d, *J* = 7.4 Hz, 1H), 8.35 (d, *J* = 7.8 Hz, 1H), 7.87 (d, *J* = 8.4 Hz, 1H), 7.64 (d, *J* = 6.1 Hz, 1H), 7.54 - 7.33 (m, 6H), 7.28 (d, *J* = 3.3 Hz, 1H), 6.86 (d, *J* = 8.0 Hz, 1H), 6.50 - 6.35 (m, 2H), 5.31 (dd, *J* = 12.4, 5.4 Hz, 1H), 4.41 - 4.27 (m, 2H), 4.20 (d, *J* = 16.4 Hz, 1H), 4.09 - 3.98 (m, 2H), 3.90 (d, *J* = 16.4 Hz, 1H), 3.83 (dd, *J* = 5.7, 3.8 Hz, 2H), 3.75 - 3.63 (m, 12H), 3.60 (dd, *J* = 16.6, 6.9 Hz, 2H), 3.51 - 3.40 (m, 2H), 3.40 - 3.32 (m, 2H), 2.86 (ddd, *J* = 39.3, 15.4, 5.3 Hz, 2H), 2.73 (dt, *J* = 16.3, 8.1 Hz, 1H), 2.44 - 2.20 (m, 4H), 2.17 - 1.92 (m, 3H).

**Synthesis of Compound 6**

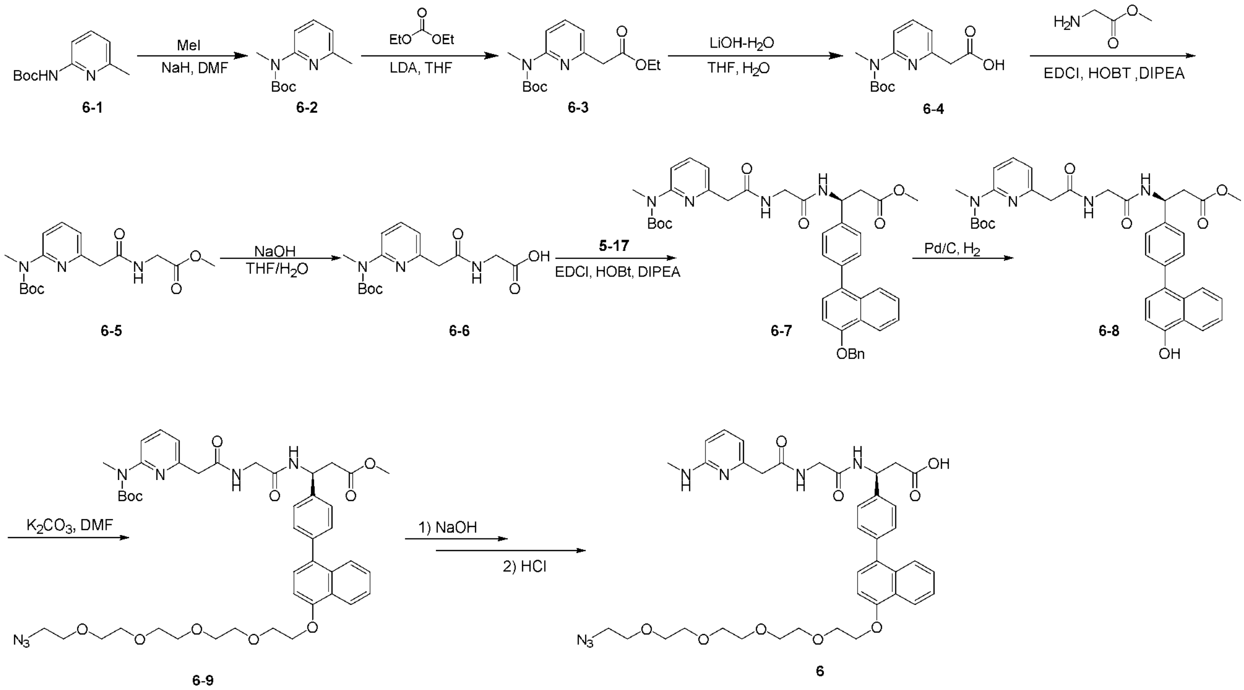


Synthesis of Intermediate **6-2**

Compound **6-1** (5.00 g, 24.0 mmol) was dissolved in N,N-dimethylformamide (100 mL), sodium hydride (1.20 g, 30.0 mmol, 60% purity) was added at 0 °C, stirred at 0 °C for half an hour, then iodomethane (5.11 g, 36.0 mmol) was added dropwise, and stirred at 25 °C for 2 hours. After completion of the reaction, the mixture was quenched by adding water (200 mL), and extracted with ethyl acetate (100 mL × 2). The organic phases were combined, washed with saturated brine, dried over anhydrous sodium sulfate, filtered, and evaporated to dryness to obtain white solid Compound **6-2** (5.00 g, 93.7% yield). $^1H$ NMR (400 MHz, $CDCl_3$) *δ* 7.51-7.38 (m, 1H), 7.31 (d, *J* = 8.3 Hz, 1H), 6.79 (d, *J* = 7.4 Hz, 1H), 3.31 (s, 3H), 2.42 (s, 3H), 1.43 (s, 9H).

Synthesis of Intermediate **6-3**

Compound **6-3** (4.80 g, 21.6 mmol) was dissolved in tetrahydrofuran (50 mL), then purged with nitrogen 3 times. At -78 °C, lithium diisopropylamide (2.5 M, 21.6 mL) was added dropwise over 10 minutes and stirred at -78 °C for 10 minutes. Finally, a solution of diethyl carbonate (3.06 g, 25.9 mmol) in tetrahydrofuran (50 mL) was added dropwise, and the reaction solution was stirred at -78 °C for 1 hour. After completion of the reaction, the mixture was quenched by adding saturated ammonium chloride solution (100 mL), diluted with water (200 mL), and extracted with ethyl acetate (250 mL × 2). The combined organic phases were washed with saturated brine, dried over anhydrous sodium sulfate, filtered, and evaporated to dryness. The residue was purified by flash silica gel chromatography (eluent 0~40% ethyl acetate/petroleum ether) to obtain white solid Compound **6-4** (5.00 g, 78.7% yield). $^1H$ NMR (400 MHz, $CDCl_3$) δ 7.68-7.51 (m, 2H), 7.08-6.95 (m, 1H), 4.21 (q, *J* = 7.1 Hz, 2H), 3.83 (s, 2H), 3.41 (s, 3H), 1.54 (s, 9H), 1.29 (t, *J* = 7.1 Hz, 3H).

Synthesis of Intermediate **6-4**

Compound **6-3** (2.00 g, 6.79 mmol) was dissolved in tetrahydrofuran (20 mL), and methanol (4 mL), water (4 mL) and lithium hydroxide monohydrate (285.1 mg, 6.79 mmol) were added sequentially. The reaction solution was stirred at 25 °C for 12 hours. After completion of the reaction, the mixture was concentrated under reduced pressure to remove tetrahydrofuran and methanol. The aqueous phase was adjusted to pH = 5 with 12 M hydrochloric acid, and extracted with ethyl acetate (25 mL × 2). The combined organic phases were washed with saturated brine, dried over anhydrous sodium sulfate, filtered, and evaporated to dryness. The residue was purified by flash silica gel chromatography (eluent 0~60% tetrahydrofuran/petroleum ether) to obtain white solid Compound **6-4** (1.00 g, 55.4% yield). MS *m/z* (ESI): 267.0 $[M+H]^+$; $^1H$ NMR (400 MHz, $CDCl_3$) δ 14.36-13.68 (m, 1H), 7.77-7.57 (m, 2H), 7.01-6.85 (m, 1H), 3.88 (s, 2H), 3.43 (s, 3H), 1.55 (s, 9H).

Synthesis of Intermediate **6-5**

**6-4** (100 mg, 0.38 mmol) was dissolved in DMF (3 mL), and glycine methyl ester (49 mg, 0.39 mmol), EDCI (88 mg, 0.46 mmol), HOBt (62 mg, 0.46 mmol) and DIPEA (294 mg, 2.28 mmol) were added sequentially. The reaction solution was stirred at room temperature for 3 hours. The reaction mixture was directly purified by preparative chromatography column (Xbridge C18, 19 × 250 mm, 10 μm, 130 Å; Mobile phase: A: Water (containing 10 mmol/L $NH_4HCO_3$); B: ACN; Gradient: 20% B to 80% B over 20 minutes) to obtain **6-5** (85 mg, yield: 66%) as a white solid. LCMS: (ESI) *m/z* = 338.4 $[M+H]^+$.

Synthesis of Intermediate **6-6**

**6-5** (85 mg, 0.252 mmol) was dissolved in THF (2 mL) and $H_2O$ (2 mL), and NaOH (50 mg, 1.26 mmol) was added. The reaction solution was stirred at room temperature for 16 hours. The reaction mixture was acidified with 3N dilute hydrochloric acid. The reaction mixture was directly purified by preparative chromatography column (Xbridge C18, 19 × 250 mm, 10 μm, 130 Å; Mobile phase: A: Water (containing 10 mmol/L $NH_4HCO_3$); B: ACN; Gradient: 20% B to 80% B over 20 minutes) to obtain **6-6** (53 mg, yield: 65%) as a white solid. LCMS: (ESI) m/z = 324.2 $[M+H]^+$.

Synthesis of Intermediate **6-7**

**6-6** (53 mg, 0.164 mmol) was dissolved in DMF (2 mL), and **5-17** (67 mg, 0.164 mmol), EDCI (37 mg, 0.197 mmol), HOBt (27 mg, 0.197 mmol) and DIPEA (126 mg, 0.984 mmol) were added sequentially. The reaction solution was stirred at room temperature for 3 hours. The reaction mixture was directly purified by preparative chromatography column (Xbridge C18, 19 × 250 mm, 10 μm, 130 Å; Mobile phase: A: Water (containing 10 mmol/L $NH_4HCO_3$); B: ACN; Gradient: 35% B to 95% B over 20 minutes) to obtain **6-7** (60 mg, yield: 52%) as a white solid. LCMS: (ESI) *m/z* =717.3 $[M+H]^+$.

Synthesis of Intermediate **6-8**

**6-7** (60 mg, 0.84 mmol) was dissolved in MeOH (5 mL), and Pd/C (10% on carbonate, 21 mg) was added. The reaction solution was stirred under hydrogen flow at room temperature for 2 hours. The reaction mixture was filtered and concentrated to obtain crude product **6-8** as a white solid. LCMS: (ESI) *m/z* = 627.1 $[M+H]^+$.

Synthesis of Intermediate **6-9**

**6-8** (54 mg, crude) was dissolved in DMF (5 mL), and **$N_3$-PEG5-Tos** (75 mg, 0.179 mmol) was added. The reaction solution was stirred at 75 °C for 16 hours. The reaction mixture was directly purified by preparative chromatography column (Xbridge C18, 19 × 250 mm, 10 μm, 130 Å; Mobile phase: A: Water (containing 10 mmol/L $NH_4HCO_3$); B: ACN; Gradient: 35% B to 95% B over 20 minutes) to obtain **6-9** (36 mg, yield: 42%) as a white solid. LCMS: (ESI) *m/z* = 873.3 $[M+H]^+$.

Synthesis of Compound **6**

**6-9** (36 mg, 0.045 mmol) was dissolved in THF (5 mL) and $H_2O$ (5 mL), and $LiOH{\cdot}H_2O$ (7.5 mg, 0.18 mmol) was added. The reaction solution was stirred at room temperature for 13 hours. LCMS monitored complete reaction. The reaction mixture was acidified with 3N dilute hydrochloric acid. The reaction mixture was concentrated, dried, dissolved in DCM (3 mL), and ice-cold TFA (3 mL) was added. The reaction solution was stirred at room temperature for 16 hours. LCMS monitored complete reaction. Then it was concentrated and dried in vacuo to obtain crude product. The crude

product was purified by preparative chromatography column (Xbridge C18, 19 × 250 mm, 10 μm, 130 Å; Mobile phase: A: Water (containing 10 mmol/L $NH_4HCO_3$); B: ACN; Gradient: 25% B to 95% B over 20 minutes) to obtain **6** (15 mg, yield: 45%) as a white solid. LCMS: (ESI) *m/z* = 758.2 $[M+H]^+$.

## Synthesis of Compound 7

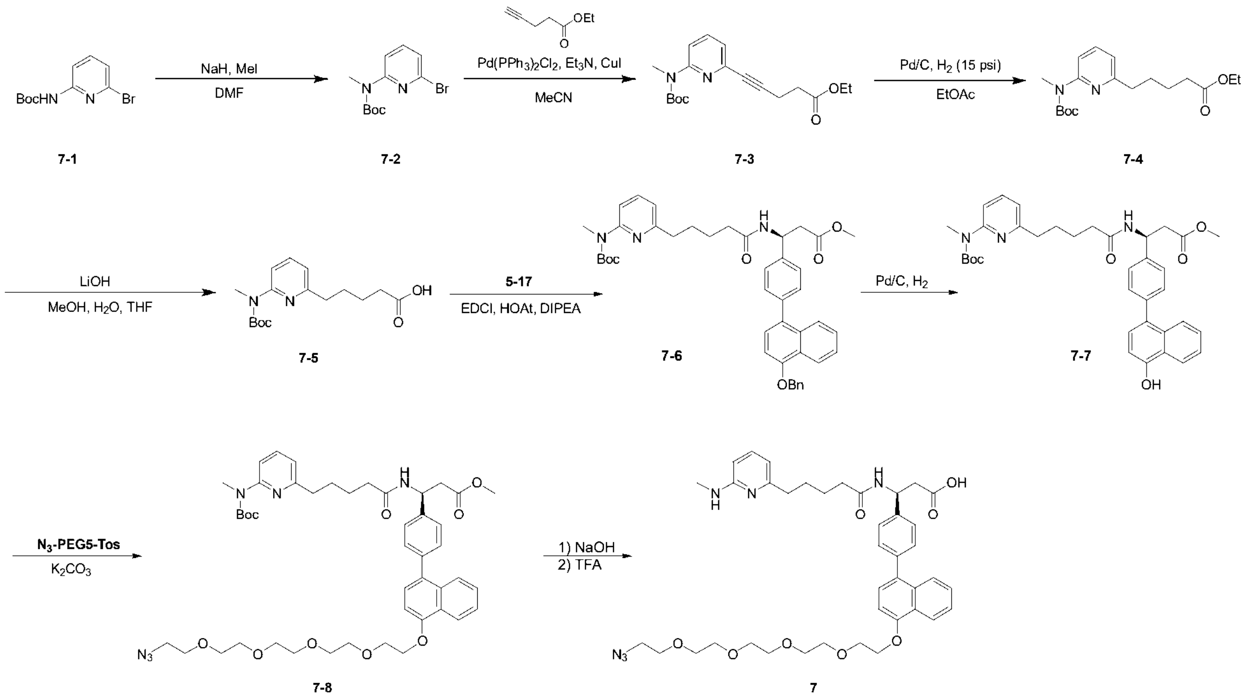


Synthesis of Intermediate **7-2**

At 0 °C, Compound **7-1** (9.00 g, 32.95 mmol) was dissolved in N,N-dimethylformamide (100 mL). The whole mixture was purged with $N_2$ three times, then sodium hydride (2.64 g, 65.90 mmol, 60% purity) was added, and stirred at 0 °C for 1 hour. Then iodomethane (14.03 g, 98.86 mmol) was added dropwise at 0 °C, and stirred at 0 °C for 1 hour. After completion of the reaction, the mixture was quenched by adding saturated ammonium chloride solution (10 mL), diluted with water (200 mL), and extracted with ethyl acetate (150 mL × 2). The combined organic phases were washed with saturated brine, dried over anhydrous sodium sulfate, filtered, and evaporated to dryness. The residue was purified by flash silica gel chromatography (eluent 0~8% ethyl acetate/petroleum ether) to obtain colorless oily Compound **7-2** (9.20 g, 97.2% yield). MS *m/z* (ESI): 231.0/233.0 $[M-56+H]^+$; $^1H$ NMR (400 MHz, $CDCl_3$) δ 7.73 (d, *J* = 8.3 Hz, 1H), 7.46 (t, *J* = 7.9 Hz, 1H), 7.15 (d, *J* = 7.6 Hz, 1H), 3.39 (s, 3H), 1.52 (s, 9H).

Synthesis of Intermediate **7-3**

At 25 °C, Compound **7-2** (5.00 g, 17.41 mmol), ethyl 4-pentynoate (3.29 g, 26.12 mmol), triethylamine (5.29 g, 52.24 mmol), bis(triphenylphosphine)palladium(II) dichloride (611.08 mg, 870.62 μmol) and copper(I) iodide (165.81 mg, 870.62 μmol) were dissolved in acetonitrile (60 mL). The whole mixture was purged with nitrogen three times, and stirred at 60 °C for 12 hours. After completion of the reaction, the solvent was evaporated. The residue was diluted with water (200 mL) and extracted with ethyl acetate (150 mL × 2). The combined organic phases were washed with saturated brine, dried over anhydrous sodium sulfate, filtered, and evaporated to dryness. The residue was purified by flash silica gel chromatography (eluent 0~10% ethyl acetate/petroleum ether) to obtain colorless oily Compound **7-3** (5.30 g, 91.6% yield). MS *m/z* (ESI): 355.1 $[M+Na]^+$; $^1H$ NMR (400 MHz, $CDCl_3$) δ 7.7-7.5 (m, 2H), 7.09 (dd, *J* = 1.0, 7.1 Hz, 1H), 4.17 (q, *J* = 7.1 Hz, 2H), 3.38 (s, 3H), 2.8-2.7 (m, 2H), 2.7-2.6 (m, 2H), 1.49 (s, 9H), 1.27 (t, *J* = 7.1 Hz, 3H).

Synthesis of Intermediate **7-4**

At 25 °C, Compound **7-3** (5.00 g, 17.41 mmol) was dissolved in ethyl acetate (60 mL), and wet palladium on carbon (1.00 g, 10% purity) was added under nitrogen atmosphere. The whole mixture was purged with hydrogen three times to reach a pressure of 15 psi, and stirred at 25 °C for 3 hours. After completion of the reaction, the mixture was directly filtered and evaporated to dryness to obtain colorless oily Compound **7-4** (4.00 g, 98.8% yield). MS *m/z* (ESI): 337.2 $[M+H]^+$; $^1H$ NMR (400 MHz, $CDCl_3$) δ 7.6-7.5 (m, 1H), 7.5-7.4 (m, 1H), 6.83 (d, *J* = 7.3 Hz, 1H), 4.11 (q, *J* = 7.1 Hz, 2H), 3.37 (s, 3H), 2.73 (t, *J* = 7.3 Hz, 2H), 2.33 (t, *J* = 7.3 Hz, 2H), 1.8-1.6 (m, 4H), 1.50 (s, 9H), 1.24 *(t, J*= 7.1 Hz, 3H).

Synthesis of Intermediate **7-5**

At 25 °C, Compound **7-4** (2.50 g, 7.43 mmol) was dissolved in a mixed solvent of water (3 mL), tetrahydrofuran (15 mL) and methanol (3 mL). Then lithium hydroxide monohydrate (623.67 mg, 14.86 mmol) was added, and stirred at 25 °C for 1 hour. After completion of the reaction, water (100 mL) and ethyl acetate (50 mL) were added. The aqueous phase was collected and washed with ethyl acetate (50 mL × 3), then acidified by adding citric acid monohydrate (4.68 g). After acidification, the mixture was extracted with ethyl acetate (50 mL × 3). The combined organic phases were washed with water (50 mL × 2), dried over anhydrous sodium sulfate, filtered, and evaporated to dryness to obtain pink solid Compound **7-5** (1 g, 48.0% yield). MS *m/z* (ESI): 331.1 $[M+Na]^+$; $^1H$ NMR (400 MHz, $CDCl_3$) δ 9.25 (s, 1H), 7.6-7.5 (m, 1H), 7.42 (d, *J* = 8.3 Hz, 1H), 6.85 (d, *J= 7.3* Hz, 1H), 3.38 (s, 3H), 2.75 (t, *J* = 7.4 Hz, 2H), 2.40 (t, *J*= 7.3 Hz, 2H), 1.9-1.7 (m, 4H), 1.51 (s, 9H).

Synthesis of Intermediate **7-6**

**7-5** (47 mg, 0.152 mmol, 1.0 equiv.), **5-17** (80 mg, 0.152 mmol), HOAt(31 mg, 0.228 mmol), EDCI (44 mg, 0.228 mmol) and DMF (2 mL) were sequentially added to a 50 mL flask, followed by addition of DIPEA (78 μL). The mixture was stirred at room temperature for 16 hours. The reaction solution was quenched with 20 mL of water under stirring. A white solid precipitated. After centrifugation, the supernatant was removed, and the residue was dried in vacuo to obtain **7-6** (100 mg, yield: 94%) as a pale yellow solid. LCMS: (ESI) *m/z* = 702.4 $[M+H]^+$.

Synthesis of Intermediate **7-7**

**7-6** (100 mg, 0.142 mmol, 1.0 equiv.), Pd/C (30 mg, 0.142 mmol) and methanol (5 mL) were sequentially added to a 50 mL flask. The system was purged with hydrogen twice and stirred under hydrogen for 2 hours. The reaction solution was filtered, the Pd/C was washed with methanol (1 mL × 2), and the filtrate was concentrated to obtain **7-7** (90 mg) as a pale yellow viscous liquid. LCMS: (ESI) *m/z* = 612.4 $[M+H]^+$.

Synthesis of Intermediate **7-8**

**7-7** (90 mg, 0.147 mmol, 1.0 equiv.), **$N_3$-PEG5-Tos** (123 mg, 0.294 mmol), potassium carbonate (42 mg, 0.305 mmol) and DMF (2 mL) were sequentially added to a 100 mL flask. The mixture was stirred at 80 °C for 16 hours. The reaction solution was filtered. The filtrate was directly purified by reverse phase chromatography to obtain **7-8** (80 mg, yield: 63%) as a colorless viscous liquid. LCMS: (ESI) *m/z* = 857.5 $[M+H]^+$ , 879.5 $[M+Na]^+$.

Synthesis of Compound **7**

**7-8** (80 mg, 0.0933 mmol, 1.0 equiv.) was dissolved in THF (1 mL), cooled to 0 °C, and sodium hydroxide solution (0.933 mmol/L, 1 mL, 0.933 mmol) was added. The mixture was stirred at 0 °C for 3 hours. The reaction solution was concentrated and dried. The residue was dissolved in dichloromethane (1 mL), and trifluoroacetic acid (3 mL) was carefully added. The mixture was stirred at room temperature for 2 hours. The reaction solution was concentrated to obtain crude product (140 mg), which was used directly in the next step. 100 mg of this crude product was purified by reverse phase preparative chromatography column (Xbridge C18 21.2 mm × 250 mm, 10 μm, 110 Å; Mobile phase: A: Water (containing 0.05% TFA), B: Acetonitrile (containing 0.05% TFA); Gradient: 20% B to 80% B over 20 minutes) to obtain Compound **7** (40 mg, yield: 70%) as a white solid. LCMS: (ESI) *m/z* = 743.4 $[M+H]^+$. $^1H$ NMR (DMSO-*d6*+$D_2O$, 400 MHz) δ 8.29 (d, *J* = 8.4 Hz, 1H), 7.80-7.72 (m, 2H), 7.59-7.48 (m, 2H), 7.46 (d, *J* = 8.4 Hz, 2H), 7.39 (d, *J* = 8.4 Hz, 2H), 7.32 (d, *J* = 7.6 Hz, 1H), 7.07 (d, *J*= 8.4 Hz, 1H), 6.78 (d, *J* = 9.2 Hz, 1H), 8.67 (d, *J* = 7.2 Hz, 1H), 5.30 (dd, *J* = 7.2, 7.6 Hz, 1H), 4.37-4.30 (m, 2H), 3.97-3.91 (m, 2H), 3.73-3.67 (m, 2H), 3.62-3.57 (m, 2H), 3.57-3.48 (m, 10 H), 3.36-3.30 (m, 2H), 2.90 (s, 3H), 2.82-2.77 (m, 2H), 2.74-2.67 (m, 2H), 2.25-2.16 (m, 2H), 1.65-1.52 (m, 4H).

**Synthesis of Compound 8**

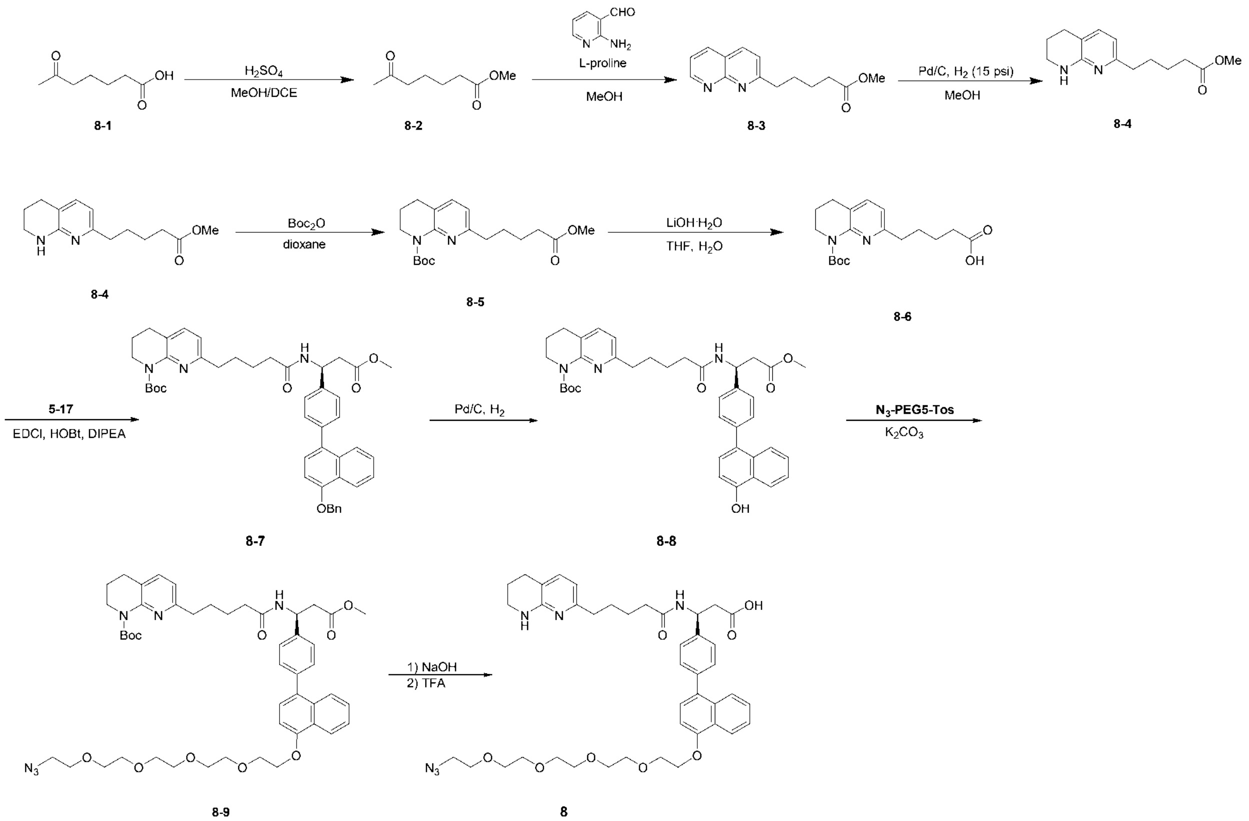


Synthesis of Intermediate **8-2**

At room temperature, Compound **8-1** (50.0 g, 347 mmol) was dissolved in dichloroethane (100 mL) and methanol (250 mL), concentrated sulfuric acid (1.70 g, 17.3 mmol, 924 μL) was added, and stirred at 90 °C for 12 hours. The reaction mixture was concentrated under reduced pressure. The residue was purified by flash silica gel chromatography (eluent 0~20% ethyl acetate/petroleum ether) to obtain yellow oily Compound **8-2** (50.0 g, 91.1% yield). $^1$H NMR (400 MHz, $CDCl_3$) δ 3.65 (s, 3H), 2.44 (t, *J* = 6.57 Hz, 2H), 2.31 (t, *J* = 6.75 Hz, 2H), 2.13 (s, 3H), 1.65 - 1.55 (m, 4H).

Synthesis of Intermediate **8-3**

At room temperature, Compound **8-2** (5.00 g, 31.6 mmol) was dissolved in methanol (50 mL), 2-amino-3-pyridinecarboxaldehyde (3.86 g, 31.6 mmol) and L-proline (1.82 g, 15.8 mmol) were added, and stirred at 80 °C for 12 hours. The reaction mixture was concentrated under reduced pressure. The residue was purified by flash silica gel chromatography (eluent 0~50% ethyl acetate/petroleum ether) to obtain yellow solid Compound **8-3** (2.70 g, 35.0% yield). MS *m/z* (ESI): 245.1 $[M+H]^+$; $^1$H NMR (400 MHz, $CDCl_3$) δ 9.09 (dd, *J* = 3.94, 1.69 Hz, 1H), 8.16 (dd, *J* = 8.00, 1.63 Hz, 1H), 8.11 (d, *J* = 8.38 Hz, 1H), 7.45 (dd, *J* = 8.00, 4.25 Hz, 1H), 7.40 (d, *J* = 8.25 Hz, 1H), 3.66 (s, 3H), 3.08 (t, *J* = 7.69 Hz, 2H), 2.39 (t, *J* = 7.50 Hz, 2H), 2.02 - 1.92 (m, 2H), 1.83 - 1.73 (m, 2H).

Synthesis of Intermediate **8-4**

At room temperature, Compound **8-3** (2.70 g, 11.1 mmol) was dissolved in methanol (30 mL), purged with nitrogen 3 times, wet palladium on carbon (1.18 g, 1.11 mmol, 10% purity) was added. The mixture was evacuated, purged with nitrogen 3 times then with hydrogen 3 times, and stirred under hydrogen (15 psi) atmosphere at 25 °C for 12 hours. The reaction mixture was concentrated under reduced pressure. The residue was purified by flash silica gel chromatography (eluent 0~30% ethyl acetate/petroleum ether) to obtain pale yellow solid Compound **8-4** (2.50 g, 91.1% yield). MS *m/z* (ESI): 249.1 $[M+H]^+$; $^1$H NMR (400 MHz, $CDCl_3$) δ 7.05 (d, *J* = 7.25 Hz, 1H), 6.37 - 6.31 (m, 1H), 4.74 (s, 1H), 3.66 (d, *J* =

2.13 Hz, 3H), 3.44 - 3.35 (m, 2H), 2.69 (t, *J* = 6.13 Hz, 2H), 2.55 (s, 2H), 2.37 - 2.30 (m, 2H), 1.94 - 1.87 (m, 2H), 1.69 (s, 4H).

Synthesis of Intermediate **8-5**

At room temperature, Compound **8-4** (2.00 g, 8.05 mmol) was dissolved in 1,4-dioxane (20 mL), di-tert-butyl dicarbonate (8.79 g, 40.3 mmol, 9.25 mL) was added, and stirred at 80 °C for 5 hours. The reaction mixture was concentrated under reduced pressure. The residue was purified by flash silica gel chromatography (eluent 0~15% ethyl acetate/petroleum ether) to obtain colorless oily Compound **8-5** (1.80 g, 64.1% yield). MS *m/z* (ESI): 349.0 $[M+H]^+$; $^1H$ NMR (400 MHz, $CDCl_3$) δ 7.29 (d, *J* = 7.63 Hz, 1H), 6.81 (d, *J* = 7.63 Hz, 1H), 3.80 - 3.72 (m, 2H), 3.66 (s, 3H), 2.76 - 2.69 (m, 4H), 2.35 (t, *J* = 7.32 Hz, 2H), 1.96 - 1.88 (m, 2H), 1.78 - 1.68 (m, 4H), 1.52 (s, 9H).

Synthesis of Intermediate **8-6**

At room temperature, Compound **8-5** (1.80 g, 5.17 mmol) was dissolved in tetrahydrofuran (4 mL) and water (1 mL), lithium hydroxide monohydrate (867 mg, 20.7 mmol) was added, and stirred at 25 °C for 15 hours. The reaction solution was diluted with ethyl acetate (10 mL) and extracted with water (5 mL × 3). The aqueous phases were combined, adjusted to pH 6 with citric acid, and extracted with ethyl acetate (10 mL × 3). The combined organic phases were dried over anhydrous sodium sulfate, filtered, and evaporated to dryness. The crude product was purified by slurrying with methyl tert-butyl ether to obtain white solid Compound **8-6** (1.30 g, 75.3% yield). MS *m/z* (ESI): 335.0 $[M+H]^+$; $^1H$ NMR (400 MHz, DMSO-$d_6$) δ 11.98 (s, 1H), 7.40 (d, *J* = 7.63 Hz, 1H), 6.88 (d, *J* = 7.63 Hz, 1H), 3.65 - 3.58 (m, 2H), 2.68 (t, *J* = 6.63 Hz, 2H), 2.61 (t, *J* = 7.57 Hz, 2H), 2.22 (t, *J* = 7.32 Hz, 2H), 1.85 - 1.77 (m, 2H), 1.67 (t, *J* = 7.57 Hz, 2H), 1.59 - 1.50 (m, 2H), 1.43 (s, 9H).

Synthesis of Intermediate **8-7**

**8-6** (50 mg, 0.152 mmol, 1.0 equiv.), **5-17** (80 mg, 0.152 mmol), HOAt (31 mg, 0.228 mmol), EDCI (44 mg, 0.228 mmol) and DMF (2 mL) were added to a 50 mL flask, followed by addition of DIPEA (78 μL). The mixture was stirred at room temperature for 16 hours. Water (20 mL) was added to the reaction solution with stirring. A solid precipitated. After centrifugation, the supernatant was discarded. The residue was dried in vacuo to obtain crude product **8-7** (110 mg, yield: 100%) as a pale yellow solid. LCMS: (ESI) *m/z* = 728.4 $[M+H]^+$.

Synthesis of Intermediate **8-8**

**8-7** (110 mg, 0.151 mmol, 1.0 equiv.), Pd/C (10%, 25 mg) and methanol (5 mL) were added to a 100 mL flask. The system was purged with hydrogen twice and stirred under hydrogen atmosphere for 2 hours. The reaction solution was filtered, concentrated, and dried in vacuo to obtain **8-8** (100 mg, yield: 100%) as a pale yellow viscous liquid. LCMS: (ESI) *m/z* = 638.4 $[M+H]^+$.

Synthesis of Intermediate **8-9**

**8-8** (100 mg, 0.15 mmol, 1.0 equiv.), **$N_3$-PEG5-Tos** (131 mg, 0.30 mmol), potassium carbonate (42 mg, 0.30 mmol) and DMF (2 mL) were added to a 50 mL flask. The mixture was stirred at 80 °C for 16 hours. The reaction solution was cooled to room temperature and directly purified by reverse phase column (Mobile phase: A: Water (containing 0.05% trifluoroacetic acid), B: Acetonitrile; Gradient: 25% B to 70% B over 20 minutes) to obtain **8-9** (110 mg, yield: 83%) as a colorless viscous liquid. LCMS: (ESI) *m/z* = 883.5 $[M+H]^+$ (50.59%), 783.5 $[M+H]^+$ (de-Boc, 40.41%).

Synthesis of Compound **8**

**8-9** (110 mg, 0.124 mmol, 1.0 equiv.) was dissolved in THF (1 mL), cooled to 0 °C, then 1.24 mmol/L NaOH solution (1 mL) was added. The mixture was stirred at 0 °C for 3 hours. The reaction solution was concentrated and dried. The residue was dissolved in dichloromethane (1 mL), and trifluoroacetic acid (3 mL) was carefully added. The mixture was stirred at room temperature for 2 hours. The reaction solution was concentrated. The residue was purified by preparative chromatography (Xbridge C18 21.2 mm × 250 mm, 10 μm, 110 Å; Mobile phase: A: Water (containing 0.05% TFA), B: Acetonitrile (containing 0.05% TFA); Gradient: 25% B to 80% B over 20 minutes) to obtain **8** (46 mg, yield: 67%) as a white solid. LCMS: (ESI) *m/z* = 769.5 $[M+H]^+$. $^1H$ NMR (DMSO-*d6*+$D_2O$, 400 MHz) δ 8.31 (d, *J* = 8.0 Hz, 1H), 7.73 (d, *J* = 8.0 Hz, 1H), 7.57 (dd, *J* = 7.2, 7.6 Hz, 1H), 7.51 (dd, *J* = 7.6, 8.4 Hz, 1H), 7.46 (d, *J* = 7.6 Hz, 2H), 7.38 (d, *J* = 8.0 Hz, 2H), 7.33 (d, *J* = 8.0 Hz, 1H), 7.08 (d, *J* = 8.0 Hz, 1H), 6.53 (d, *J* = 7.2 Hz, 1H), 5.30 (dd, *J* = 7.2, 7.6 Hz, 1H), 4.36-4.31 (m, 2H), 3.97-3.92 (m, 2H), 3.74-3.69 (m, 2H), 3.65-3.59 (m, 2H), 3.58-3.48 (m, 10H), 3.35-3.28 (m, 4H), 2.85-2.78 (m, 2H), 2.65-2.57 (m, 4H),

2.45-2.18 (m, 2H), 1.76-1.68 (m, 2H), 1.62-1.47 (m, 4H).

**Synthesis of Compound 9**

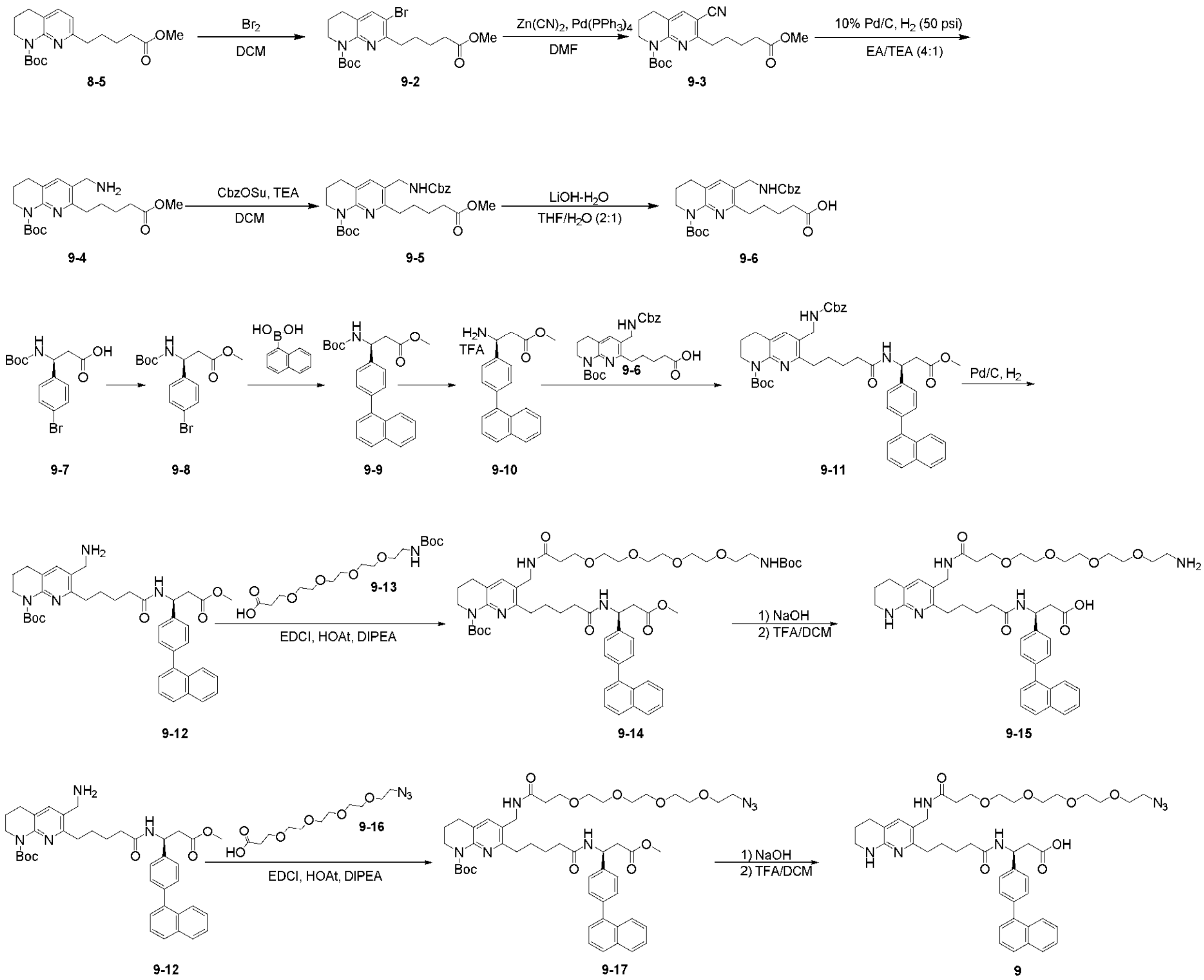


Synthesis of Intermediate **9-2**

At 25 °C, to a solution of Compound **8-5** (12.0 g, 34.4 mmol) in dichloromethane (100 mL) was added bromine (5.50 g, 34.4 mmol, 1.77 mL). The mixture was stirred at 25 °C for 3 hours. After completion of the reaction, the reaction was quenched by adding saturated sodium bicarbonate solution (50 mL), and extracted with ethyl acetate (100 mL × 2). The combined organic phases were washed with saturated brine, dried over anhydrous sodium sulfate, filtered, and evaporated to dryness. The residue was purified by flash silica gel chromatography (eluent 0~10% ethyl acetate/petroleum ether) to obtain colorless oily Compound **9-2** (8.20 g, 55.7% yield). MS *m/z* (ESI): 427.1 $[M+H]^+$; $^1H$ NMR (400 MHz, $CDCl_3$) δ 7.49 (s, 1H), 3.77 - 3.71 (m, 2H), 3.67 (s, 3H), 2.87 (t, *J* = 7.32 Hz, 2H), 2.72 (t, *J* = 6.63 Hz, 2H), 2.38 (t, *J* = 7.07 Hz, 2H), 1.91 (quin, *J* = 6.28 Hz, 2H), 1.79 - 1.72 (m, 4H), 1.51 (s, 9H).

Synthesis of Intermediate **9-3**

At 25 °C, to Compound **9-2** (9.00 g, 21.1 mmol) dissolved in N,N-dimethylformamide (110 mL) were added tetrakis(triphenylphosphine)palladium (2.43 g, 2.11 mmol) and zinc cyanide (4.95 g, 42.1 mmol). After addition, the whole mixture was purged with nitrogen three times, and stirred at 120 °C for 3 hours. After completion of the reaction, the reaction was quenched by adding saturated sodium bicarbonate solution (50 mL), and extracted with ethyl acetate (100 mL × 2). The combined organic phases were washed with saturated brine, dried over anhydrous sodium sulfate, filtered,

and evaporated to dryness. The residue was purified by flash silica gel chromatography (eluent 0~15% ethyl acetate/petroleum ether) to obtain colorless oily Compound **9-3** (5.80 g, 73.7% yield). MS m/z (ESI): 374.2 $[M+H]^+$; $^1H$ NMR (400 MHz, $CDCl_3$) δ 7.52 (s, 1H), 3.81 - 3.72 (m, 2H), 3.65 (s, 3H), 2.91 (t, *J* = 7.57 Hz, 2H), 2.73 (t, *J* = 6.50 Hz, 2H), 2.36 (t, *J* = 7.32 Hz, 2H), 1.93 (quin, *J* = 6.25 Hz, 2H), 1.84 - 1.69 (m, 4H), 1.52 (s, 9H).

Synthesis of Intermediate **9-4**

At 25 °C, under nitrogen protection, to a solution of Compound **9-3** (5.80 g, 15.5 mmol) in ethyl acetate (50 mL) and triethylamine (12.5 mL) was added wet palladium on carbon (1.65 g, 10% purity). After addition, the whole mixture was purged with nitrogen three times, then with hydrogen three times, and stirred under hydrogen (50 psi) atmosphere at 25 °C for 96 hours. After completion of the reaction, the mixture was filtered, and the filtrate was concentrated to obtain Compound **9-4** (5.00 g, crude). MS *m/z* (ESI): 378.2 $[M+H]^+$; $^1H$ NMR (400 MHz, $CDCl_3$) δ 7.35 (s, 1H), 3.84 (s, 2H), 3.80 - 3.71 (m, 2H), 3.67 (s, 3H), 2.74 (q, *J* = 6.25 Hz, 4H), 2.37 (t, *J* = 6.94 Hz, 2H), 1.92 (quin, *J* = 6.28 Hz, 2H), 1.84 - 1.70 (m, 4H), 1.52 (s, 9H).

Synthesis of Intermediate **9-5**

At 25 °C, to Compound **9-4** (4.95 g, 13.1 mmol) dissolved in dichloromethane (50 mL) were added triethylamine (4.64 g, 45.9 mmol, 6.39 mL) and benzyloxycarbonyl succinimide (3.59 g, 14.4 mmol). After addition, the mixture was stirred at 25 °C for 30 minutes. After completion of the reaction, the mixture was diluted with water (50 mL) and extracted with dichloromethane (50 mL × 2). The combined organic phases were washed with saturated brine, dried over anhydrous sodium sulfate, filtered, and evaporated to dryness. The residue was purified by flash silica gel chromatography (eluent 0~26% ethyl acetate/petroleum ether) to obtain white solid Compound **9-5** (4.40 g, 65.6% yield). MS *m/z* (ESI): 512.3 $[M+H]^+$; $^1H$ NMR (400 MHz, $CDCl_3$) δ 7.42 - 7.29 (m, 5H), 7.27 (d, *J* = 3.38 Hz, 1H), 5.14 (s, 2H), 4.34 (d, *J* = 5.50 Hz, 2H), 3.78 - 3.69 (m, 2H), 3.65 (s, 3H), 2.78 - 2.64 (m, 4H), 2.35 (t, *J* = 6.75 Hz, 2H), 1.90 (quin, *J* = 6.22 Hz, 2H), 1.74 (s, 4H), 1.51 (s, 9H).

Synthesis of Intermediate **9-6**

At 25 °C, to Compound **9-5** (4.20 g, 8.21 mmol) dissolved in tetrahydrofuran (40 mL) and water (20 mL) was added lithium hydroxide monohydrate (1.03 g, 24.6 mmol). After addition, the mixture was stirred at 25 °C for 12 hours. After completion of the reaction, the mixture was concentrated to remove tetrahydrofuran, adjusted to pH = 6 with saturated citric acid, and extracted with ethyl acetate (25 mL × 2). The combined organic phases were washed with saturated brine, dried over anhydrous sodium sulfate, filtered, and evaporated to dryness. The crude product was slurried with methyl tert-butyl ether to obtain white solid Compound **9-6** (1.00 g, 24.5% yield). MS m/z (ESI): 498.3 $[M+H]^+$; $^1H$ NMR (400 MHz, DMSO-$d_6$) δ 11.98 (s, 1H), 7.76 (t, *J* = 5.75 Hz, 1H), 7.43 - 7.20 (m, 6H), 5.04 (s, 2H), 4.16 (d, *J* = 5.88 Hz, 2H), 3.63 - 3.58 (m, 2H), 2.69 - 2.60 (m, 4H), 2.23 (t, *J*= 7.13 Hz, 2H), 1.80 (quin, *J* = 6.19 Hz, 2H), 1.70 - 1.51 (m, 4H), 1.42 (s, 9H).

Synthesis of Intermediate **9-8**

Methanol (50 mL) was added to a 250 mL flask, cooled to 0 °C in an ice bath. $SOCl_2$ (0.5 mL, 6.9 mmol) was added slowly dropwise with stirring, followed by addition of acetone acetal (20 mL) and Boc-(S)-3-amino-3-(4-bromophenyl)-propionic acid (1.0 g, 2.9 mmol). The mixture was stirred at room temperature for 16 hours. The reaction solution was concentrated to dryness. The residue was dissolved in dichloromethane (20 mL). Triethylamine (1.2 mL, 8.7 mmol) and $(Boc)_2O$ (759 mg, 3.48 mmol) were added sequentially. The mixture was stirred at room temperature for another 2 hours. The reaction solution was concentrated. The residue was purified by silica gel column to obtain **9-8** (0.9 g, yield: 86.6%) as a pale yellow solid. LCMS: (ESI) *m/z* = 380.1 $[M+Na]^+$.

Synthesis of Intermediate **9-9**

4-(1-Naphthyl)phenylboronic acid (258 mg, 1.5 mmol, 1.5 equiv.), **9-8** (358 mg, 1.0 mmol), XPhos Pd G2 (157 mg, 0.2 mmol), $K_3PO_4$ (424 mg, 2.0 mol) and THF (10 mL) were added to a 100 mL flask. The system was purged with nitrogen twice and stirred at room temperature under nitrogen protection for 16 hours. The reaction was filtered and concentrated. The crude product was purified by silica gel column to obtain **9-9** (425 mg, yield: 69.9%) as a pale yellow solid. LCMS: (ESI) *m/z* = 428.3 $[M+Na]^+$, 833.4 $[2M+Na]^+$.

Synthesis of Intermediate **9-10**

Methanol (10 mL) was added to a 100 mL flask. Trifluoroacetic acid (2 mL) was carefully added with stirring. The mixture was stirred at room temperature for 10 minutes. Then **9-9** (0.425 g) was added. The mixture was stirred at room temperature for 2 hours, concentrated. The residue was dissolved in acetonitrile (5 mL), water (50 mL) was added, and lyophilized to obtain crude product **9-10** (370 mg, yield: 100%) as a pale yellow solid. LCMS: (ESI) *m/z* = 306.3 $[M+H]^+$.

Synthesis of Intermediate **9-11**

**9-10** (100 mg, 0.2 mmol, 1.0 equiv.), **9-6** (69 mg, 0.2 mmol), HOAT (41 mg, 0.3 mmol), EDCI (58 mg, 0.3 mmol) and DMF (2 mL) were added to a 100 mL flask, followed by addition of DIPEA (102 μL). The mixture was stirred at room temperature for 16 hours. The reaction solution was directly purified by reverse phase chromatography column to obtain **9-11** (130 mg, yield: 82.8%) as a pale yellow solid. LCMS: (ESI) *m/z* = 785.5 $[M+H]^+$. $^1H$ NMR (400 MHz, DMSO-*d6*) δ 8.43 (d, *J* = 8.4 Hz, 1H), 8.00 (d, *J* = 8.0 Hz, 1H), 7.95 (d, *J* = 8.4 Hz, 1H), 7.81-7.73 (m, 2H), 7.60-7.51 (m, 2H), 7.50-7.39 (m, 6H), 7.37-7.17 (m, 6H), 5.37-5.29 (m, 1H), 5.03 (s, 2H), 5.17 (d, *J* = 5.6 Hz, 2H), 3.62-3.52 (m, 2H), 3.57 (s, 3H), 2.88-2.81 (m, 2H), 1.82-1.72 (m, 2H), 1.68-1.52 (m, 4H), 1.41 (s, 9H).

Synthesis of Intermediate **9-12**

**9-11** (130 mg, 0.166 mmol, 1.0 equiv.), 10% Pd/C (60 mg) and methanol (10 mL) were added to a 100 mL flask. The system was purged with hydrogen twice. The mixture was stirred under hydrogen at room temperature for 70 minutes. The reaction solution was filtered and concentrated to obtain **9-12** (102 mg, yield: 94%) as a pale yellow viscous liquid. LCMS: (ESI) *m/z* = 651.5 $[M+H]^+$.

Synthesis of Intermediate **9-14**

**9-12** (102 mg, 0.166 mmol, 1.0 equiv.), BocNH-$PEG_5$-COOH (61 mg, 0.166 mmol), HOAT (34 mg, 0.249 mmol), EDCI (48 mg, 0.249 mmol) and DMF (2 mL) were sequentially added to a 100 mL flask, followed by addition of DIPEA (83 μL). The mixture was stirred at room temperature for 16 hours. The reaction solution was directly purified by reverse phase chromatography column to obtain **9-14** (130 mg, yield: 83%) as a pale yellow solid. LCMS: (ESI) *m/z* = 998.3 $[M+H]^+$.

Synthesis of Compound **9-15**

**9-14** (130 mg, 0.13 mmol, 1.0 equiv.) and THF (5 mL) were added to a 100 mL flask, followed by addition of 2 mL of NaOH solution (0.975 mol/L, 1.95 mmol). The mixture was stirred at room temperature for 3 hours. The reaction solution was concentrated and dried. The residue was dissolved in dichloromethane (1 mL), and trifluoroacetic acid (3 mL) was carefully added. The mixture was stirred at room temperature for 4 hours. The reaction solution was concentrated and dried to obtain 180 mg of crude product, which was used directly in the next step. 90 mg of this crude product was purified by preparative reverse phase chromatography column (Xbridge C18 21.2 mm × 250 mm, 10 μm, 110 Å; Mobile phase: A: Water (containing 0.05% TFA), B: Acetonitrile (containing 0.05% TFA); Gradient: 20% B to 80% B over 20 minutes) to obtain **9** (42 mg, yield: 63%) as a white solid. LCMS: (ESI) *m/z* = 784.5 $[M+H]^+$. $^1H$ NMR (400 MHz, DMSO-*d6*+$D_2O$) δ 8.45 (t, *J* = 5.6 Hz, 1H, NH), 8.02 (d, *J* = 8.0 Hz, 1H), 7.97 (d, *J* = 7.6 Hz, 1H), 7.78 (d, *J* = 8.4 Hz, 1H), 7.63-7.54 (m, 2H), 7.52-7.39 (m, 7H), 5.31 (t, *J* = 7.6 Hz, 1H), 4.11 (s, 2H), 3.63-3.57 (m, 4H), 3.57-3.51(m, 4H), 3.50 (s, 4H), 3.47 (s, 4H), 3.36-3.29 (m, 2H), 3.00-2.94 (m, 2H), 2.82-2.76 (m, 2H), 2.72-2.62 (m, 4H), 2.39-2.32 (m, 2H), 2.24-2.15 (m, 2H), 1.80-1.71 (m, 2H), 1.64-1.48 (m, 4H).

Synthesis of Compound **9-17**

**9-12** (200 mg, 0.307 mmol, 1.0 equiv.), **$N_3$-PEG5-COOH** (89 mg, 0.307 mmol), HOAT (63 mg, 0.46 mmol), EDCI (88 mg, 0.46 mmol) and DMF (5 mL) were sequentially added to a 100 mL flask, followed by addition of DIPEA (83 μL). The mixture was stirred at room temperature for 16 hours. The reaction solution was directly purified by reverse phase chromatography column to obtain **9-17** (120 mg, yield: 42%) as a pale yellow solid. LCMS: (ESI) *m/z* = 924.5 $[M+H]^+$.

Synthesis of Compound **9**

**9-17** (120 mg, 0.13 mmol, 1.0 equiv.) and THF (5 mL) were added to a 100 mL flask, followed by addition of an aqueous solution (2 mL) of NaOH (78 mg, 1.95 mmol). The mixture was stirred at room temperature for 3 hours. The reaction solution was concentrated and dried. The residue was dissolved in dichloromethane (1 mL), and trifluoroacetic

acid (3 mL) was carefully added. The mixture was stirred at room temperature for 4 hours. The reaction solution was concentrated and dried. The crude product was purified by preparative reverse phase chromatography column (Mobile phase: A: Water (containing 10 mmol/L $NH_4HCO_3$), B: Acetonitrile; Gradient: 5% B to 50% B over 20 minutes) to obtain **9** (65 mg, yield: 62%) as a white solid. LCMS: (ESI) *m/z* = 810.3 $[M+H]^+$.

**Synthesis of Compound 13**

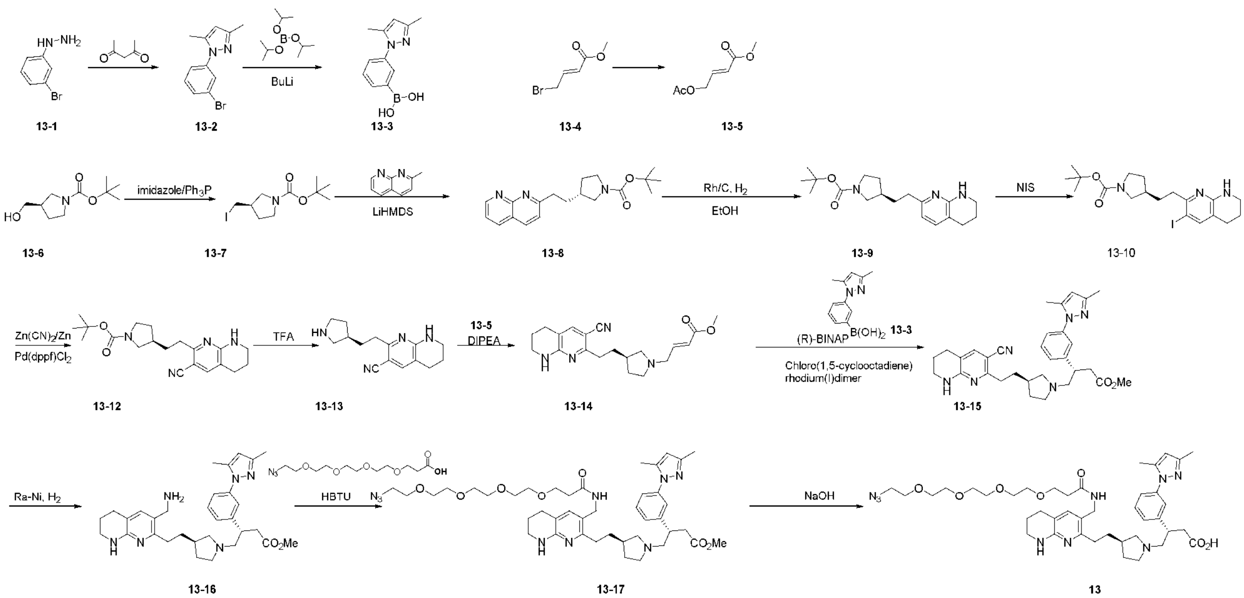


13-1 13-2 13-3 13-4 13-5 13-6 13-7 13-8 13-9 13-10 13-12 13-13 13-14 13-15 13-16 13-17 13

Synthesis of Intermediate **13-2**

3-bromophenylhydrazine hydrochloride (11.12 g, 50 mmol, 1.0 equiv.) was dissolved in ethanol (40 mL), and 2,4-pentanedione (5.5 g, 55 mmol) and potassium acetate (5.9 g, 60 mmol) were added sequentially. The mixture was stirred under reflux for 3 hours. The reaction solution was concentrated to remove most of the ethanol. The residue was dissolved in ethyl acetate (200 mL), washed with saturated brine (50 mL), dried over anhydrous sodium sulfate, filtered, and concentrated. The residue was purified by silica gel chromatography column to obtain **13-2** (7.5 g, yield: 60%) as a pale yellow oily liquid. LCMS: (ESI) *m/z* = 252.0 $[M+H]^+$. $^1H$ NMR (400 MHz, DMSO-d6) $\delta$ 7.72 (dd, *J* = 1.6, 2.0 Hz, 1H), 7.59-7.55 (m, 1H), 7.55-7.51 (m, 1H), 7.45 (dd, *J* = 8.0, 8.0 Hz, 1H), 6.09 (s, 1H), 2.32 (s, 3H), 2.18 (s, 3H).

Synthesis of Intermediate **13-3**

A solution of **13-2** (2.51 g, 10.0 mmol, 1.0 equiv.) in THF (30 mL) was cooled to -78 °C in a dry ice bath. n-butyllithium solution (4.8 mL, 12.0 mmol) was added dropwise. After addition, stirring was continued at this temperature for 10 minutes. Triisopropyl borate (2.44 g, 13 mmol) was carefully added dropwise over 20 minutes. After addition, the mixture was slowly warmed to room temperature and stirred for another 2 hours. The reaction solution was quenched with 2 N hydrochloric acid and the pH was adjusted to 5-6. The mixture was extracted with ethyl acetate (100 mL), washed with saturated brine (20 mL), dried over anhydrous sodium sulfate, filtered, and concentrated. The residue was dissolved in ethyl acetate (5 mL). Petroleum ether (50 mL) was slowly added with stirring. A white precipitate formed, which was filtered and dried in vacuo to obtain **13-3** (1.0 g, yield: 46%) as a white solid. LCMS: (ESI) *m/z* = 217.0 $[M+H]^+$.

Synthesis of Intermediate **13-5**

Methyl 4-bromo-2-butenoate (5.0 g, 27.9 mmol) was dissolved in acetonitrile (40 mL), and sodium acetate (3.4 g, 41.9 mmol) was added. The mixture was stirred at 50 °C for 3 days. LCMS indicated most of the starting material was converted. The reaction solution was filtered and concentrated. The residue was purified by reverse phase chromatography column to obtain **13-5** (1.9 g, yield: 43%) as an anhydrous oily liquid. LCMS: (ESI) *m/z* = 181.1 $[M+Na]^+$.

Synthesis of Intermediate **13-7**

Triphenylphosphine (17.3 g, 66 mmol) and imidazole (3.97 g, 66 mmol) were placed in a 500 mL three-necked flask equipped with a stir bar. The flask was evacuated for 20 minutes and purged with nitrogen. Anhydrous dichloromethane (200 mL) was added via syringe, and the mixture was cooled to 0 °C. Iodine (16.8 g, 66.0 mmol) was quickly added under nitrogen atmosphere. The reaction solution was stirred at room temperature for 10 minutes until all iodine dissolved, then cooled again to 0 °C. (R)-N-tert-butoxycarbonyl-3-hydroxymethyl-pyrrolidine (10.05 g, 50 mmol) dissolved in 20 mL of anhydrous dichloromethane was added dropwise to the reaction solution. After addition, stirring was continued at 0 °C for 16 hours. The reaction solution was filtered to remove solid precipitate and concentrated. The residue was purified by silica gel chromatography column to obtain **13-7** (7.5 g, yield: 48%) as a pale yellow oily liquid. LCMS: (ESI) *m*/*z* = 255.9 $[M+H-56]^+$; $^1H$ NMR (400 MHz, DMSO-d6) δ 3.47-3.35 (m, 2H), 3.32(s, 1H), 3.30 (s, 1H), 3.26-3.15 (m, 1H), 2.93-2.84 (m, 1H), 2.47-2.35 (m, 1H), 2.03-1.92 (m ,1H), 1.63-1.47 (m, 1H), 1.39 (s, 9H).

Synthesis of Intermediate **13-8**

2-methyl-[1,8]-naphthyridine (1.94 g, 13.5 mmol) and **13-7** (4.2 g, 13.5 mmol) were dissolved in tetrahydrofuran (20 mL) and cooled to 0 °C. LiHMDS (14.85 mL, 14.85 mmol) was added dropwise. After addition, stirring was continued at 0 °C for 16 hours. The reaction was quenched with water (50 mL), extracted with ethyl acetate (100 mL × 2), washed with saturated brine (20 mL), dried over anhydrous sodium sulfate, filtered, and concentrated. The residue was purified by reverse phase chromatography column to obtain **13-8** (2.2 g, yield: 50%) as a brown solid. LCMS: (ESI) *m*/*z* = 328.1 $[M+H]^+$; $^1H$ NMR (400 MHz, DMSO-*d6*) δ 9.04 (d, *J* = 4.0 Hz, 1H), 8.43 (d, *J* = 8.4 Hz, 1H), 8.38 (d, *J* = 8.4 Hz, 1H), 7.59 (d, *J* = 8.8 Hz, 1H), 7.58 (dd, *J* = 4.0, 4.0 Hz, 1H), 3.52-3.40 (m, 1H), 3.36-3.30 (m, 1H), 3.20-3.08 (m, 1H), 3.05-2.92 (m, 2H), 2.90-2.78 (m, 1H), 2.22-2.05 (m, 1H), 2.04-1.93 (m, 1H),1.93-1.82 (m, 2H), 1.60-1.44 (m, 1H), 1.39-1.38 (m, 9H).

Synthesis of Intermediate **13-9**

Intermediate **13-8** (3.9 g, 11.9 mmol) was dissolved in ethanol (50 mL), and Ru/C (500 mg) was added. The reaction system was purged with hydrogen twice and stirred under hydrogen at room temperature for 6 hours. The mixture was filtered, washed with ethanol (2 mL × 2), and concentrated. The residue was purified by reverse phase chromatography column to obtain **13-9** (3.3 g, yield: 84%) as a pale yellow liquid. LCMS: (ESI) *m*/*z* = 784.5 $[M+H]^+$; $^1H$ NMR (400 MHz, DMSO-d6) δ 7.01 (d, *J* = 7.2 Hz, 1H), 6.27 (s, 1H), 6.26 (d, *J* = 7.2 Hz, 1H), 3.45-3.35 (m, 1H), 3.33-3.26 (m,1H), 3.26-3.19 (m, 2H), 3.18-3.05 (m, 1H), 2.76 (dd, *J* = 8.4, 10.0 Hz, 1H), 2.59 (t, *J* = 6.4 Hz, 2H), 2.48-2.37 (m, 2H), 2.12-1.98 (m, 1H), 1.98-1.87 (m, 1H), 1.78-1.70 (m, 2H), 1.62 (q, *J* = 7.6 Hz, 2H), 1.38 (s, 9H).

Synthesis of Intermediate **13-10**

Intermediate **13-9** (662 mg, 2.0 mmol, 1.0 equiv.) was dissolved in acetonitrile (10 mL), and NIS (675 mg, 3.0 mmol) was added. The mixture was stirred at room temperature for 16 hours. The reaction solution was added to 100 mL of ethyl acetate, washed with saturated $Na_2SO_3$ solution (30 mL) and saturated brine (20 mL), dried over anhydrous sodium sulfate, filtered, and concentrated. The crude product was purified by silica gel column to obtain **13-10** (670 mg, yield: 90.7%) as a pale yellow oily liquid. LCMS: (ESI) *m*/*z* = 458.0 $[M+H]^+$; $^1H$ NMR (400 MHz, DMSO-*d6*) δ 7.42 (s, 1H), 6.59 (s, 1H), 3.45-3.39 (m, 1H), 3.34-3.26 (m, 1H), 3.25-3.18 (m, 2H), 3.17-3.11 (m, 1H), 2.83-2.75 (m, 1H), 2.66-2.55 (m, 4H), 2.16-2.05 (m, 1H), 2.05-1.90 (m, 1H), 1.75-1.64 (m, 2H), 1.60 (q, *J*= *7.6* Hz, 2H), 1.56-1.38 (m, 1H), 1.39 (s, 9H).

Synthesis of Intermediate **13-12**

**13-10** (660 mg, 1.31 mmol) was dissolved in DMF (20 mL), and $Zn(CN)_2$ (308 mg, 2.62 mmol), $Pd_2(dba)_3$ (119 mg, 0.13 mmol), $Pd(dppf)_2Cl_2$ (105 mg, 0.13 mmol), zinc powder (426 mg) and water (0.2 mL) were added sequentially. The mixture was stirred at 115 °C for 16 hours. The reaction solution was cooled and purified by preparative chromatography column to obtain **13-12** (310 mg, yield: 66%) as a brown solid. LCMS: (ESI) *m*/*z* = 356.7 $[M+H]^+$; $^1H$ NMR (400 MHz, DMSO-d6) δ 7.54 (s, 1H), 7.39 (s, 1H), 3.45-3.39 (m, 1H), 3.34-3.26 (m, 3H), 3.21-3.10 (m, 1H), 2.81-2.75 (m, 1H), 2.66-2.57 (m, 4H), 2.16-2.02 (m, 1H), 2.02-1.90 (m, 1H), 1.75-1.66 (m, 2H), 1.68 (q, *J* = 7.2 Hz, 2H), 1.54-1.37 (m, 1H), 1.38 (s, 9H).

Synthesis of Intermediate **13-13**

**13-12** (0.3 g, 0.84 mmol) was dissolved in DCM (5 mL), cooled to 0 °C, and trifluoroacetic acid (2 mL) was added. The mixture was stirred at 0 °C for 4 hours. LCMS monitored reaction completion. The mixture was concentrated to

dryness to obtain crude product **13-13** (410 mg) as a pale yellow solid. LCMS: (ESI) *m/z* = 257.0 $[M+H]^+$.

Synthesis of Intermediate **13-14**

**13-5** (133 mg, 0.84 mmol) and $Pd(dppf)_2Cl_2$ (34 mg) were dissolved in anhydrous dichloromethane (10 mL) and stirred under nitrogen protection for 10 minutes. Then a solution **of 13-13** (407 mg, 0.84 mmol) and DIPEA (0.86 mL, 5.04 mmol) in dichloromethane (10 mL) was added. The mixture was stirred at 0 °C for 4 hours. LCMS monitored reaction completion. The reaction solution was filtered and concentrated. The residue was purified by preparative chromatography column to obtain **13-14** (100 mg, total yield for two steps: 33.5%) as a pale yellow oily liquid. LCMS: (ESI) *m/z* = 354.9 $[M+H]^+$.

Synthesis of Intermediate **13-15**

**13-14** (100 mg, 0.282 mmol), **13-3** (183 mg, 0.846 mmol), potassium hydroxide (32 mg, 0.564 mmol), water (148 μL), (1,5-cyclooctadiene)chlororhodium(I) dimer (23 mg, 0.045 mmol), R-(+)-1,1'-binaphthalene-2,2'-bis(diphenylphosphine) (35 mg, 0.028 mmol) and 1,4-dioxane (3 mL) were sequentially added to a 50 mL round bottom flask. The mixture was stirred at 90 °C for 6 hours. After monitoring reaction completion, the reaction solution was filtered and concentrated. The crude product was purified by reverse phase chromatography column to obtain **13-15** (96 mg, yield: 64.9%) as a pale yellow oily liquid. LCMS: (ESI) *m/z* = 527.1 $[M+H]^+$.

Synthesis of Intermediate **13-16**

**13-16** (48 mg, 0.091 mmol), Ra-Ni (50 mg), ammonia methanol solution (7N, 0.5 mL, 3.5 mmol) and methanol (5 mL) were sequentially added to a 50 mL round bottom flask. The reaction system was purged with hydrogen twice and stirred under hydrogen at room temperature overnight. LCMS showed about 50% conversion of starting material. The reaction solution was filtered and concentrated to obtain crude product **13-16** (50 mg) as a pale yellow oily liquid. LCMS: (ESI) *m/z* = 531.2 $[M+H]^+$.

Synthesis of Intermediate **13-17**

The crude product **13-16** (~0.091 mmol) obtained from the previous step was dissolved in DMF (2 mL). **$N_3$-PEG5-Tos** (16 mg), PyBOP (33 mg, 0.0637 mmol) and DIPEA (31 μL) were added sequentially. The mixture was stirred at room temperature for 2 hours. After completion of the reaction, the reaction solution was directly purified by preparative chromatography column to obtain **13-17** (16 mg, total yield for two steps: 21.9%) as a pale yellow oily liquid. LCMS: (ESI) *m/z* = 804.6 $[M+H]^+$; 403.0 $[M+2H]/2^+$.

Synthesis of Compound **13**

**13-15** (16 mg, 0.02 mmol) was dissolved in tetrahydrofuran (1 mL), and sodium hydroxide solution (1 mL, 0.62 mmol/L) was added. The mixture was stirred at room temperature for 2 hours. LCMS monitored reaction completion. The reaction was quenched with a small amount of dry ice. The reaction solution was directly purified by preparative chromatography column to obtain **13** (8 mg, yield: 50%) as a colorless oily liquid. LCMS: (ESI) *m/z* = 790.7 $[M+H]^+$; 812.5 $[M+Na]^+$; 396.0 $[M+2H]/2^+$; $^1H$ NMR (400 MHz, DMSO-*d6*+$D_2O$) δ 8.09 (dd, *J* = 4.4, 4.8 Hz, 1H), 7.42 (dd, *J* = 8.0, 8.0 Hz, 1H), 7.33 (s, 1H), 7.30 (d, *J* = 8.0 Hz, 1H), 7.26 (d, *J* = 8.0 Hz, 1H), 6.98 (s, 1H), 6.07 (s, 1H), 4.07 (d, *J= 5.6* Hz, 2H), 3.60-3.45 (m, 16 H), 3.37 (t, *J* = 4.8 Hz, 2H), 3.36-3.22 (m, 1H), 3.22-3.15 (m, 2H), 3.05-2.97 (m, 1H), 2.97-2.89 (m, 1H), 2.85-2.76 (m, 2H), 2.75-2.65 (m, 3H), 2.61-2.55 (m, 2H), 2.52-2.40 (m, 2H), 2.35-2.30 (m, 3H), 2.27 (s, 3H), 2.17 (s, 3H), 2.17-2.05 (m, 1H), 2.02-1.90 (m,1H), 1.79-1.70 (m, 2H), 1.65-1.50 (m, 2H), 1.47-1.37 (m, 1H).

400 mg of Compound **13** was purified by SFC (Column: OX 25*250mm, 10μm), Mobile phase: [Carbon dioxide - Acetonitrile/Methanol (0.5% Ammonia, 7M in MeOH)], B%: 45%, isocratic elution mode) to obtain white solids **isomer1: 13-P1** (Major) and **isomer2: 13-P2** (Minor).

**Isomer1:** 155 mg

MS *m/z* (ESI): 395.8 $[M+2H]/2^+$;
Analytical Chiral SFC: RT= 2.202 min, >99.5%, Column: R-R-Whelk-O1 4.6*100mm, 5μm, Carbon dioxide - 50% Acetonitrile/Methanol (0.2% Ammonia, 7M in MeOH), B%: 45%, Flow rate 3 mL/min. $^1H$ NMR (400 MHz, DMSO-*d6*

$+D_2O$) δ 8.09 (dd, *J* = 4.4, 4.8 Hz, 1H), 7.45 (dd, *J*= 7.6, 7.6 Hz, 1H), 7.34 (s, 2H), 7.30 (d, *J* = 7.6 Hz, 1H), 7.01 (s, 1H), 6.09 (s, 1H), 4.09 (s, 2H), 3.62-3.45 (m, 16 H), 3.37 -3.28 (m, 4H), 3.26-3.05 (m, 2H), 3.20-3.10 (m, 1H), 23.10-2.95 (m, 2H), 2.91-2.95 (m, 1H), 2.85-2.70 (m, 2H), 2.65-2.56 (m, 2H), 2.53-2.41 (m, 3H), 2.34 (t, *J* = 6.4 Hz, 2H), 2.28 (s, 3H), 2.27-2.16 (m, 1H), 2.18 (s, 3H), 2.14-2.00 (m, 1H), 1.79-1.70 (m, 2H), 1.65-1.46 (m, 3H).

**Isomer2:** 15 mg

MS *m/z* (ESI): 395.7 $[M+2H]/2^+$;
Analytical Chiral SFC: RT = 2.600 min, >99.5%, Column: R-R-Whelk-O1 4.6*100 mm, 5μm, Carbon dioxide - 50% Acetonitrile/Methanol (0.2% Ammonia, 7M in MeOH), B%: 45%, Flow rate 3 mL/min. $^1H$ NMR (400 MHz, DMSO-*d6* $+D_2O$) δ 8.11 (dd, *J* = 5.2, 5.2 Hz, 1H), 7.40 (dd, *J* = 7.6, 7.6 Hz, 1H), 7.29 (d, *J* = 4.4 Hz, 2H), 7.25 (dd, *J* = 6.8, 7.6 Hz, 1H), 6.96 (s, 1H), 6.05 (s, 1H), 4.05 (s, 2H), 3.60-3.41 (m, 16 H), 3.38 -3.25 (m, 3H), 3.23-3.05 (m, 4H), 2.92-2.70 (m, 4H), 2.60-2.53 (m, 2H), 2.48-2.35 (m, 4H), 2.30 (t, *J* = 6.0 Hz, 2H), 2.25 (s, 3H), 2.20-2.06 (m, 1H), 2.19 (s, 3H), 2.02-1.90 (m, 1H), 1.75-1.65 (m, 2H), 1.62-1.38 (m, 3H).

**Synthesis of Compound 14**

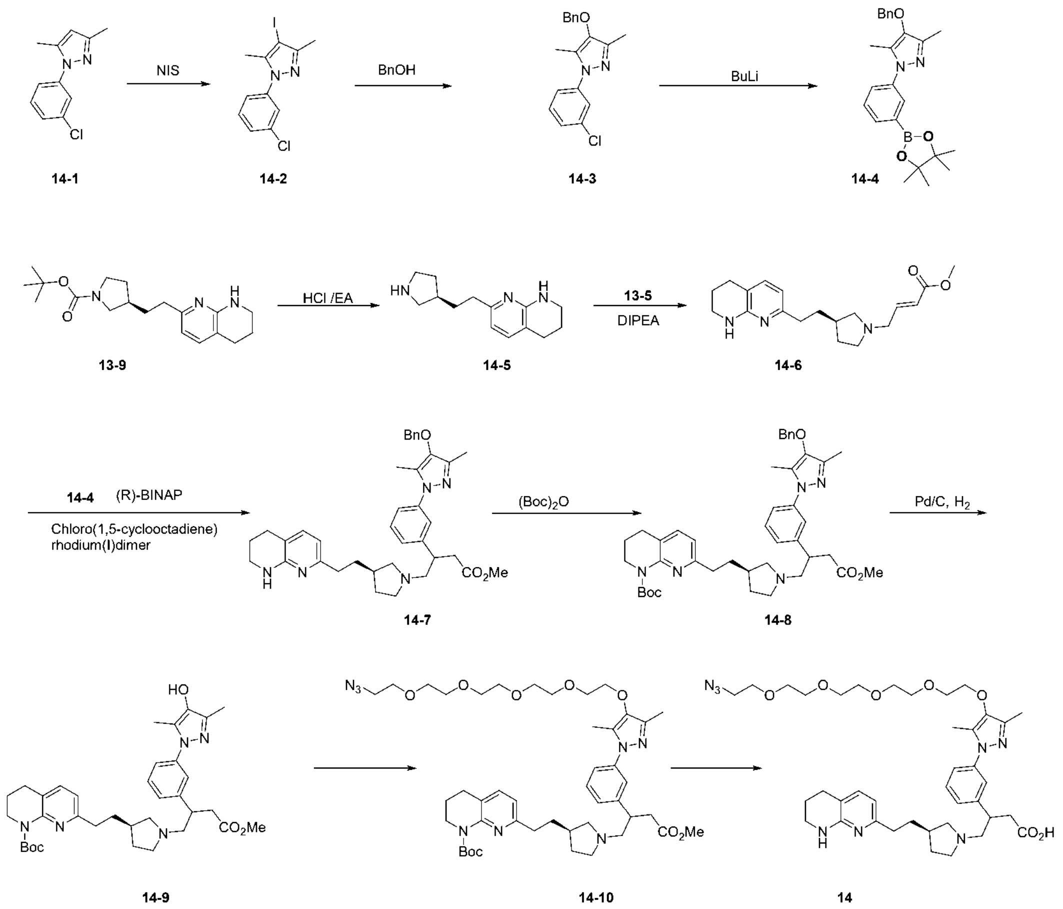


Synthesis of Intermediate **14-2**

**14-1** (5.0 g, 24.2 mmol, 1.0 eq.) was dissolved in ethyl acetate (100 mL), and NIS (5.99 g, 26.6 mmol) was added in one portion. The mixture was stirred at room temperature for 16 hours. The reaction solution was washed with saturated sodium sulfite solution (20 mL) and saturated brine (10 mL), dried over anhydrous sodium sulfate, filtered, and concentrated. The residue was purified by silica gel chromatography column to obtain **14-2** (7.3 g, yield: 90%) as a pale yellow solid. LCMS: (ESI) *m/z* = 333.0 $[M+H]^+$; $^1H$ NMR (400 MHz, DMSO-*d6*) δ 7.61 (dd, *J* = 2.0, 2.4 Hz, 1H), 7.57-7.46 (m,

3H), 2.34 (s, 3H), 2.19 (s, 3H).

Synthesis of Intermediate **14-3**

**14-2** (7.0 g, 21 mmol, 1.0 eq.), 3,4,7,8-tetramethyl-1,10-phenanthroline (995 mg, 4.2 mmol), benzyl alcohol (4.54 g, 42 mmol), copper(I) iodide (800 mg, 4.2 mmol), cesium carbonate (13.7 g, 42 mmol) and toluene (100 mL) were added to a 500 mL round bottom flask. The mixture was stirred under nitrogen protection and refluxed for 72 hours. LCMS showed product formation. The reaction solution was cooled, filtered, and concentrated. The residue was purified by silica gel chromatography column to obtain **14-3** (1.2 g, yield: 18%) as a colorless oily liquid. LCMS: (ESI) *m*/*z* = 313.0 $[M+H]^+$; $^1H$ NMR (400 MHz, DMSO-*d6*) δ 7.58-7.34 (m, 9H), 4.88 (s, 2H), 2.19 (s, 3H), 2.10 (s, 3H).

Synthesis of Intermediate **14-4**

**14-3** (1.2 g, 3.85 mmol) was dissolved in 1,4-dioxane (100 mL). Bis(pinacolato)diboron (1.95 g, 7.69 mmol), $Pd_2(dba)_3$ (528 mg, 0.578 mmol), X-Phos (366 mg, 0.769 mmol), and potassium acetate (1.13 g, 11.55 mmol) were added sequentially. The mixture was stirred under nitrogen protection at 115 °C for 16 hours. LCMS monitored reaction completion. The mixture was filtered and concentrated. The crude product was purified by reverse phase chromatography column to obtain **14-4** (1.3 g, yield: 83.8%) as a brown solid. LCMS: (ESI) *m*/*z* = 405.0 $[M+H]^+$; 322.7 (de-pinacol product) $[M+H]^+$.

Synthesis of Intermediate **14-5**

Intermediate **13-9** (1.6 g, 4.89 mmol) was dissolved in dichloromethane (10 mL), cooled to 0 °C, and hydrogen chloride-dioxane solution (4 N, 20 mL) was added. The mixture was stirred at 0 °C for 4 hours. LCMS monitored reaction completion. The mixture was concentrated and dried in vacuo to obtain crude product **14-5** (1.2 g) as a pale yellow solid. LCMS: (ESI) *m*/*z* = 232.1 $[M+H]^+$.

Synthesis of Intermediate **14-6**

**13-5** (514 mg, 3.25 mmol) and $Pd(dppf)_2Cl_2$ (146 mg) were dissolved in dichloromethane DCM (30 mL) and stirred at room temperature for 10 minutes. The mixture was cooled to 0 °C, and a solution of **14-5** (1.1 g, 3.61 mmol) and DIPEA (3.7 mL, 21.66 mmol) in dichloromethane (20 mL) was added. The mixture was stirred under nitrogen protection at 0 °C for 16 hours. LCMS monitored reaction completion. The mixture was filtered and concentrated. The crude product was purified by reverse phase chromatography column to obtain **14-6** (1.0 g, yield: 84%) as a pale brown oily liquid. LCMS: (ESI) *m*/*z* = 330.1 $[M+H]^+$; $^1H$ NMR (400 MHz, DMSO-d6) δ 7.00 (d, *J*= 7.6 Hz, 1H), 6.85 (dt, *J* = 5.6, 16.0 Hz, 1H), 6.25 (s, 1H), 6.23 (d, *J* = 7.6 Hz, 1H), 5.98 (d, *J* = 16.0 Hz, 1H), 3.66 (s, 3H), 3.26-3.10 (m, 4H), 2.68 (t, *J* = 7.2 Hz, 1H), 2.62-2.51 (m, 3H), 2.45-2.31 (m, 4H), 2.11-1.96 (m, 2H), 1.95-1.83 (m, 1H), 179-1.68 (m, 2H), 1.65-1.54 (m, 2H), 1.38-1.25 (m, 1H).

Synthesis of Intermediate **14-7**

**14-6** (310 mg, 0.9141 mmol), **14-4** (1.14 g, 2.82 mmol), potassium hydroxide (105 mg, 1.88 mmol), water (500 μL), (1,5-cyclooctadiene)chlororhodium(I) dimer (46 mg, 0.0491 mmol), R-(+)-1,1'-binaphthalene-2,2'-bis(diphenylphosphine) (117 mg, 0.188 mmol) and 1,4-dioxane (8 mL) were sequentially added to a 50 mL round bottom flask. The system was purged with nitrogen twice and stirred under nitrogen protection in a 90 °C oil bath for 6 hours. The reaction was complete, filtered, and concentrated. The crude product was purified by reverse phase chromatography column to obtain **14-7** (550 mg, yield: 96%) as a pale yellow solid. LCMS: (ESI) *m*/*z* = 607.7 $[M+H]^+$; 304.4 $[M+2H]/2^+$.

Synthesis of Intermediate **14-8**

**14-7** (100 mg, 0.164 mmol, 1.0 equiv.) and di-tert-butyl dicarbonate (72 mg, 0.329 mmol) were dissolved in tetrahydrofuran (3 mL) under nitrogen protection. The mixture was cooled to 0 °C, and LiHMDS (1 N in THF, 0.25 mL, 0.25 mmol) was added dropwise. After addition, stirring was continued at 0 °C for 6 hours. The reaction was quenched with 10% ammonium chloride solution, extracted with ethyl acetate (20 mL × 2), washed with saturated brine (5 mL), dried over anhydrous sodium sulfate, filtered, and concentrated. The crude product was purified by preparative chromatography column to obtain **14-8** (45 mg, yield: 38%) as a white solid. LCMS: (ESI) *m*/*z* = 708.3 $[M+H]^+$.

Synthesis of Intermediate **14-9**

**14-8** (45 mg, 0.064 mmol) was dissolved in methanol (4 mL), and Pd/C (10 mg) was added. The reaction system was purged with hydrogen twice and stirred under hydrogen at room temperature for 3 hours. The reaction was complete, filtered, and concentrated to obtain crude product **14-9** (40 mg) as a yellow solid. LCMS: (ESI) *m*/*z* = 618.2 $[M+H]^+$.

Synthesis of Intermediate **14-10**

**14-9** (40 mg, 0.064 mmol, 1.0 equiv.) was dissolved in DMF (2 mL), cooled to 0 °C, and NaH (60%, 30 mg) was added under nitrogen protection. The mixture was stirred for 2 minutes, then **$N_3$-PEG5-Tos** (53 mg, 0.128 mmol) was added. The mixture was stirred at 0 °C for 4 hours. LCMS monitored reaction completion. The reaction was quenched with water (5 mL) and a small amount of dry ice (about 2 g). A precipitate formed. The supernatant was removed by centrifugation, and the residual solid was dried in vacuo to obtain crude product **14-10** (60 mg), which was used directly in the next step. LCMS: (ESI) *m*/*z* = 863.7 $[M+H]^+$; 432.5 $[M+2H]/2^+$.

Synthesis of Compound **14**

The crude product **14-10** (60 mg, about 0.064 mmol) obtained from the previous step was dissolved in tetrahydrofuran (1 mL), and 0.62 mol/L sodium hydroxide solution (1 mL) was added. The mixture was stirred at room temperature for 2 hours. LCMS monitored reaction completion. The reaction solution was concentrated and dried. The residue was dissolved in dichloromethane (2 mL), and trifluoroacetic acid TFA (4 mL) was added. The mixture was stirred at room temperature overnight. LCMS monitored reaction completion. The mixture was concentrated. The residue was purified by preparative chromatography column to obtain **14** (13 mg, total yield for three steps: 27%) as a white solid. LCMS: (ESI) m/z = 749.57 $[M+H]^+$; 375.5 $[M+2H]/2^+$; $^1H$ NMR (400 MHz, DMSO-*d6*+$D_2O$) δ 7.39 (dd, *J* = 7.6, 8.0 Hz, 1H), 7.30 (s, 1H), 7.27 (d, *J* = 8.0 Hz, 1H), 7.22 (d, *J* = 8.0 Hz, 1H), 7.02 (d, *J = 7.6* Hz, 1H), 6.25 (d, *J = 7.6* Hz, 1H), 3.99-3.93 (m, 2H), 3.66-3.49 (m, 16 H), 3.37 (t, *J* = 4.8 Hz, 2H), 3.33-3.25 (m, 1H), 3.25-3.20 (m, 2H), 2.93-2.81 (m, 2H), 2.75-2.55 (m, 6H), 2.42-2.26 (m, 4H), 2.22 (s, 3H), 2.16 (s, 3H), 2.05-1.96 (m, 1H), 1.94-1.84 (m, 1H), 1.78-1.70 (m, 2H), 1.65-1.54, 2H), 1.40-1.29 (m, 1H).

190 mg of Compound **14** was purified by SFC (Column: OJ-H 25*250mm, 10μm), Mobile phase: [Carbon dioxide - Methanol (0.2% Ammonia, 7M in MeOH)], B%: 25%, isocratic elution mode) to obtain white solids **isomer1: 14-P1** (Minor) and **isomer2: 14-P2** (Major).

**Isomer1:** 10 mg

MS *m*/*z* (ESI): 375.3 $[M+2H]/2^+$;
Analytical Chiral SFC: RT = 2.580 min, >99.5% purity, Column: OJ-H 4.6*100mm, 5μm, Carbon dioxide - Methanol (0.2% Ammonia, 7M in MeOH), B%: 20%, Flow rate 3 mL/min. $^1H$ NMR (400 MHz, DMSO-*d6*+$D_2O$) δ (dd, *J* = 7.6, 8.0 Hz, 1H), 7.30 (s, 1H), 7.27 (d, *J* = 8.0 Hz, 1H), 7.21 (d, *J* = 7.6 Hz, 1H), 7.00 (d, *J* = 7.2 Hz, 1H), 6.23 (d, *J* = 7.6 Hz, 1H), 3.97-3.92 (m, 2H), 3.66-3.49 (m, 16 H), 3.36 (t, *J* = 4.8 Hz, 2H), 3.33-3.25 (m, 1H), 3.25-3.18 (m, 2H), 2.96-2.86 (m, 2H), 2.80-2.61 (m, 4H), 2.60-2.53 (m, 2H), 2.47-2.13 (m, 3H), 2.29-2.18 (m, 1H), 2.21 (s, 3H), 2.14 (s, 3H), 2.07-1.97 (m, 1H), 1.96-1.84 (m, 1H), 1.75-1.67 (m, 2H), 1.64-1.52 (m, 2H), 1.40-1.29 (m, 1H).

**Isomer2:** 65 mg

MS *m*/*z* (ESI): 375.2 $[M+H]/2^+$;
Analytical Chiral SFC: RT = 3.498 min, >99.5% purity, Column: OJ-H 4.6*100mm, 5μm, Carbon dioxide - Methanol (0.2% Ammonia, 7M in MeOH), B%: 20%, Flow rate 3 mL/min. $^1H$ NMR (400 MHz, DMSO-*d6*+$D_2O$) δ 7.42 (dd, *J* = 7.2, 8.0 Hz, 1H), 7.33 (s, 1H), 7.31 (d, *J* = 8.0 Hz, 1H), 7.24 (d, *J*= 7.6 Hz, 1H), 7.04 (d, *J* = 7.2 Hz, 1H), 6.27 (d, *J* = 7.6 Hz, 1H), 3.99-3.93 (m, 2H), 3.68-3.60 (m, 2 H), 3.60-3.51 (m, 14 H), 3.37 (t, *J* = 4.8 Hz, 2H), 3.35-3.25 (m, 1H), 3.25-3.21 (m, 2H), 3.13-3.08 (m, 1H), 3.00-2.94 (m, 1H), 2.81-2.69 (m, 4H), 2.63-2.57 (m, 2H), 2.50-2.35 (m, 3H), 2.23 (s, 3H), 2.17 (s, 3H), 2.13-2.04 (m, 1H), 2.03-1.90 (m, 1H), 1.78-1.70 (m, 2H), 1.68-1.54, 2H), 1.46-1.35 (m, 1H).

## Synthesis of Compound 15

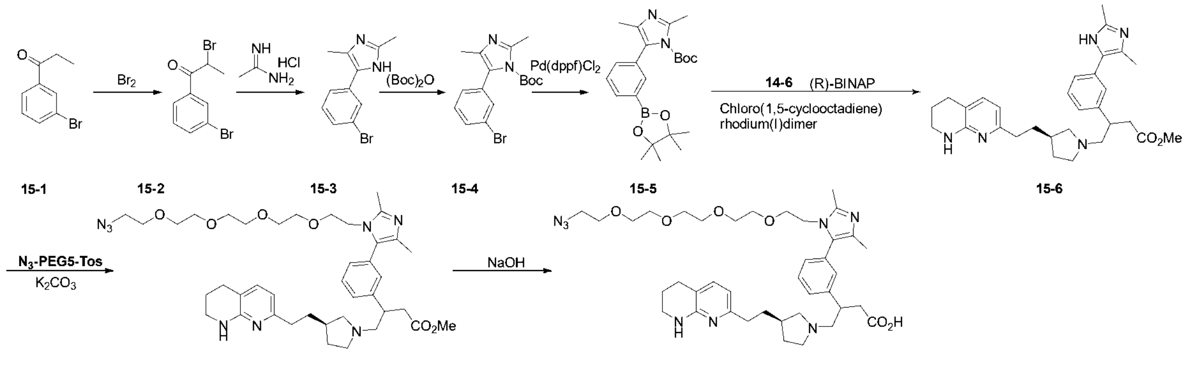


15-1 15-2 15-3 15-4 15-5 15-6

15-7 15

Synthesis of Intermediate **15-2**

(3-bromophenyl)-1-propanone (5.0 g, 23.47 mmol) was dissolved in dichloromethane (30 mL), and bromine (1.2 mL, 23.47 mmol) was carefully added dropwise. The mixture was stirred at room temperature for 4 hours. The reaction solution was poured into water (50 mL), extracted with dichloromethane (100 mL × 2), dried over anhydrous sodium sulfate, filtered, and concentrated to obtain crude product **15-2** (8.9 g) as a colorless liquid. LCMS: (ESI) *m/z* = 292.5 $[M+H]^+$.

Synthesis of Intermediate **15-3**

Acetamidine hydrochloride (2.88 g, 30.5 mmol) was dissolved in DMF (20 mL), and potassium carbonate (8.4 g, 61 mmol) was added. The mixture was stirred at 50 °C for 30 minutes. Then a solution of **15-2** (8.9 g, 30.5 mmol) in chloroform (10 mL) was added, and stirring was continued at 50 °C overnight. LCMS monitored reaction completion. The mixture was filtered and concentrated. The residue was purified by reverse phase chromatography column to obtain 1.6 g of crude product. The crude product was dissolved in dichloromethane (10 mL). Petroleum ether (20 mL) was added in portions with stirring. A precipitate formed. Stirring was continued for 30 minutes. The mixture was filtered, washed with petroleum ether (1 mL × 2), and dried in vacuo to obtain **15-3** (1.1 g, total yield for two steps: 14%) as a pale yellow solid. LCMS: (ESI) *m/z* = 333.0 $[M+H]^+$. $^1$H NMR (400 MHz, DMSO-*d6*) δ 11.73 (s, 1H), 7.78 (s, 1H), 7.57 (s, 1H), 7.32 (s, 2H), 2.34 (s, 3H), 2.25 (s, 3H).

Synthesis of Intermediate **15-4**

**15-3** (1.0 g, 3.98 mmol) was dissolved in dichloromethane (20 mL). Di-tert-butyl dicarbonate (1.3 g, 5.97 mmol), DMAP (49 mg), and TEA (1.66 mL) were added sequentially. The mixture was stirred at room temperature overnight. LCMS monitored reaction completion. The reaction solution was concentrated. The residue was purified by silica gel chromatography column to obtain **15-4** (1.1 g, yield: 78%) as a pale yellow solid. LCMS: (ESI) *m/z* = 295.0 $[M+H-56]^+$; $^1$H NMR (400 MHz, DMSO-d6) δ 7.72 (dd, *J* = 1.6, 2.0 Hz, 1H), 7.55 (ddd, *J* = 1.2, 1.6, 8.0 Hz, 1H), 7.49 (ddd, *J* = 1.2, 2.0, 8.0 Hz, 1H), 7.38 (dd, *J* = 8.0, 8.0 Hz, 1H), 2.51 (s, 3H), 2.45 (s, 3H), 1.60 (s, 9H).

Synthesis of Intermediate **15-5**

**15-4** (1.0 g, 2.85 mmol) was dissolved in 1,4-dioxane (50 mL). Bis(pinacolato)diboron (1.16 g, 4.56 mmol), $Pd(dppf)_2Cl_2$ (346 mg, 0.427 mmol), and potassium acetate (839 mg) were added sequentially. The system was purged with nitrogen twice. The mixture was stirred under nitrogen protection at 90 °C overnight. LCMS monitoring showed formation of the target product. The mixture was filtered and concentrated. The crude product was purified by silica gel chromatography column to obtain 15-5 (750 mg, yield: 69%) as a pale yellow oily liquid. LCMS: (ESI) *m/z* = 399.1 $[M+H]^+$; 343.1$[M+H-56]^+$.

Synthesis of Intermediate **15-6**

**14-6** (150 mg, 0.455 mmol), **15-5** (543 mg, 1.365 mmol), potassium hydroxide (51 mg, 0.911 mmol), water (240 μL), (1,5-cyclooctadiene)chlororhodium(I) dimer (23 mg, 0.045 mmol), R-(+)-1,1'-binaphthalene-2,2'-bis(diphenylphosphine) (62 mg, 0.091 mmol) and 1,4-dioxane (4 mL) were sequentially added to a 50 mL round bottom flask. The mixture was stirred under nitrogen protection at 90 °C for 6 hours. LCMS monitored reaction completion. The mixture was filtered and concentrated. The crude product was purified by preparative chromatography column to obtain **15-6** (66 mg, yield: 29%) as a white solid. LCMS: (ESI) *m/z* = 502.1 $[M+H]^+$; 251.6 $[M+2H]/2^+$.

Synthesis of Intermediate **15-7**

**15-6** (25 mg, 0.05 mmol), potassium carbonate (21 mg, 0.15 mmol), **$N_3$-PEG5-Tos** (104 mg, 0.25 mmol) and DMF (2 mL) were sequentially added to a 50 mL round bottom flask. The mixture was stirred at 80 °C overnight. LCMS monitoring showed about 50% conversion of starting material. The reaction solution was cooled to room temperature. Water (10 mL) was added with stirring. A precipitate formed. After centrifugation, the supernatant was removed. The residue was dried in vacuo to obtain crude product **15-7,** which was used directly in the next step. LCMS: (ESI) *m/z* = 747.3 $[M+H]^+$.

Synthesis of Compound **15**

The crude product **15-7** (about 0.05 mmol) obtained from the previous step was dissolved in tetrahydrofuran (1 mL), cooled to 0 °C, and then 0.5N sodium hydroxide solution (1 mL) was added. The mixture was stirred at 0 °C for 2 hours. LMCS monitored reaction completion. The reaction was quenched with a small amount of dry ice. The reaction solution was directly purified by preparative chromatography column to obtain **15** (6.6 mg, total yield for two steps: 18%) as a white solid. LCMS: (ESI) *m/z* = 733.3 $[M+H]^+$; 367.2 $[M+2H]/2^+$; $^1H$ NMR (400 MHz, DMSO-*d6*+$D_2O$) δ 7.44 (s, 1H), 7.40 (d, *J* = 8.0 Hz, 1H), 7.34 (dd, *J* = 7.6 Hz, 1H), 7.12 (d, *J* = 7.2 Hz, 2H), 6.33 (d, *J* = 7.6 Hz, 1H), 4.05 (t, *J* = 4.8 Hz, 2H), 3.65 (t, *J* = 4.2 Hz, 2H), 3.59-3.45 (m, 16H), 3.40-3.31 (m, 4H), 3.28-3.00 (m, 6H), 2.98-2.88 (m, 1H), 2.86-2.75 (m, 1H), 2.65-2.58 (m, 2H), 2.49-2.42 (m, 2H), 2.35 (s, 3H), 2.34 (s, 3H), 2.25-2.14 (m, 1H), 2.13-2.01 (m, 1H), 1.78-1.71 (m, 2H), 1.70-1.60 (m, 2H), 1.59-1.47 (m,1H).

**Synthesis of Compound 16**

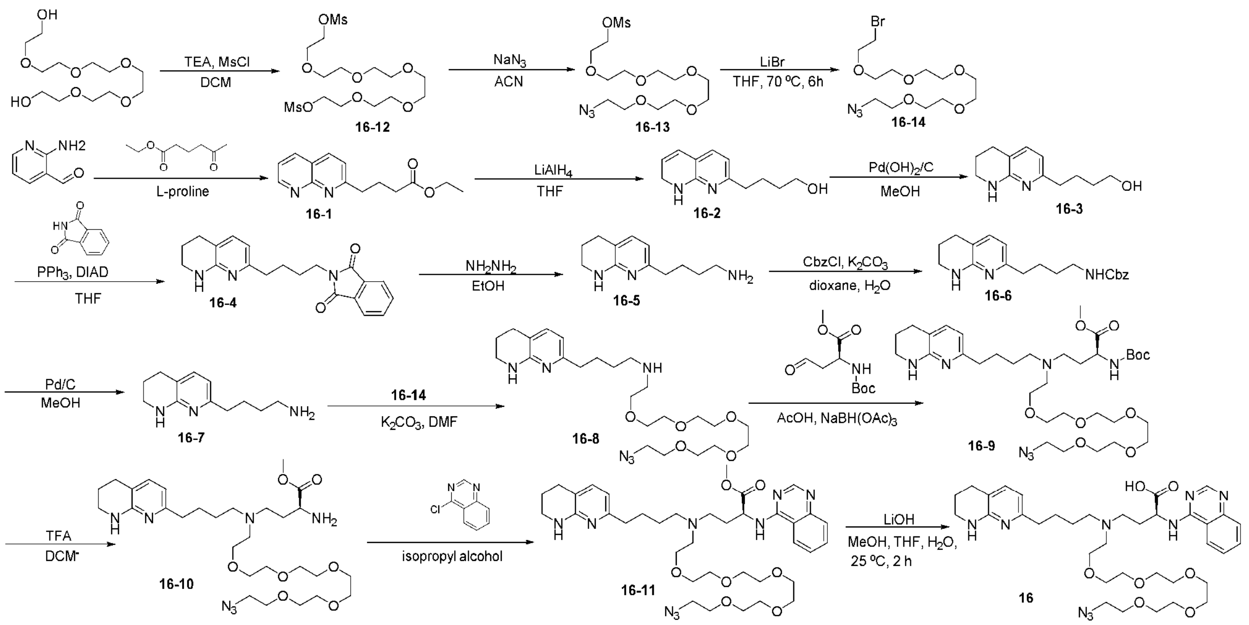


Synthesis of Intermediate **16-12**

To a solution of 2-[2-[2-[2-(2-hydroxyethoxy)ethoxy]ethoxy]-ethoxy]-ethanol (10 g, 35.42 mmol) and triethylamine (14.34 g, 141.68 mmol) in dichloromethane (100 mL) was added methanesulfonyl chloride (8.93 g, 77.92 mmol) at 0 °C under nitrogen protection, and the mixture was stirred at 25 °C for 16 hours. LCMS showed complete consumption of starting material and detection of product. The mixture was filtered, and the filtrate was washed with 0.1 N hydrochloric acid solution (100 mL). The combined organic layer was dried over anhydrous sodium sulfate, filtered, and concentrated in vacuo to obtain **16-12** (13.00 g, crude) as a brown oil, which was used directly in the next step. LCMS: (ESI) *m*/*z* = 358.10 $[M-28+H]^+$.

Synthesis of Intermediate **16-13**

To a solution of **16-12** (13 g, 29.65 mmol) in acetonitrile (130 mL) was added sodium azide (2.41 g, 37.06 mmol) at 25 °C, and the mixture was stirred at 85 °C for 16 hours. LCMS showed complete consumption of starting material and detection of product. The mixture was filtered, and the filtrate was concentrated to obtain crude product. The crude product was purified by silica gel column chromatography (EA/PE = 70%) to obtain **16-13** (6.00 g, 14.01 mmol, 23.63% yield, 90% purity) as a brown oil. LCMS: (ESI) *m*/*z* = 358.10 $[M-28+H]^+$.

Synthesis of Intermediate **16-14**

To a solution of **16-13** (6 g, 15.57 mmol) in tetrahydrofuran (60 mL) was added lithium bromide (6.76 g, 77.83 mmol) at 25 °C, and the mixture was stirred at 70 °C for 6 hours. LCMS showed complete consumption of starting material and detection of product. The mixture was filtered, and the filtrate was concentrated to obtain a residue. Water was added to the residue, extracted with ethyl acetate (100 mL × 2), and washed with brine (100 mL). The combined organic layer was dried over anhydrous sodium sulfate, filtered, and concentrated in vacuo to obtain crude product. The crude product was purified by silica gel column chromatography (EA/PE = 60%) to obtain **16-14** (4.10 g, 9.97 mmol, 64.02% yield, 90% purity) as a brown oil. LCMS: (ESI) *m*/*z* = 342.20 $[M-28+H]^+$.

Synthesis of Intermediate **16-1**

To a mixture of 2-aminopyridine-3-carbaldehyde (25 g, 204.71 mmol) and L-proline (11.78 g, 102.35 mmol) in ethanol (300 mL) was added ethyl acetoacetate (32.38 g, 204.75 mmol) at 25 °C, and the mixture was stirred at 80 °C for 16 hours. LCMS showed complete consumption of starting material and detection of product. The mixture was concentrated to obtain a residue. Water was added to the residue, extracted with ethyl acetate (500 mL × 2), and washed with brine (500 mL). The combined organic layer was dried over anhydrous sodium sulfate, filtered, and concentrated in vacuo to obtain crude product. The crude product was purified by silica gel column chromatography (EA/PE = 50%) to obtain **16-1** (31.00 g, 114.21 mmol, 27.90% yield, 90% purity) as a yellow solid. LCMS: (ESI) m/z = 245.1 $[M+H]^+$; $^1H$ NMR (400 MHz, $CDCl_3$) δ 9.09 (dd, *J* = 4.3, 2.0 Hz, 1H), 8.17 (dd, *J* = 8.1, 2.0 Hz, 1H), 8.12 (d, *J*= 8.3 Hz, 1H), 7.46 (dd, *J* = 8.1, 4.3 Hz, 1H), 7.41 (d, *J* = 8.3 Hz, 1H), 4.12 (q, *J* = 7.1 Hz, 2H), 3.14 - 3.08 (m, 2H), 2.45 (t, *J* = 7.4 Hz, 2H), 2.30 - 2.22 (m, 2H), 1.25 (t, *J* = 7.1 Hz, 3H).

Synthesis of Intermediate **16-2**

To a solution of ethyl 4-(1,8-naphthyridin-2-yl)butanoate (15 g, 61.40 mmol) in tetrahydrofuran (200 mL) was added lithium aluminum hydride (1 M, 92.10 mL) at -78 °C under nitrogen protection, and the mixture was stirred at -78 °C for 1 hour. Then the mixture was stirred at 25 °C for 2 hours. LCMS showed complete consumption of starting material and detection of product. Water (4 mL), aqueous sodium hydroxide solution (4 mL, 15%), and water (12 mL) were added to the mixture and stirred for 0.5 hour. The mixture was filtered. The filtrate was dried over anhydrous sodium sulfate, filtered, and concentrated in vacuo to obtain crude product. The crude product was purified by silica gel column chromatography (EA/PE = 90%) to obtain **16-2** (7.00 g, 25.70 mmol, 20.93% yield, 75% purity) as a yellow oil. LCMS: (ESI) *m*/*z* = 205.1 $[M+H]^+$.

Synthesis of Intermediate **16-3**

A mixture of **16-2** (7 g, 34.27 mmol) and palladium hydroxide on carbon (1 g) in methanol (50 mL) was stirred at 25 °C under hydrogen atmosphere for 16 h. LCMS showed complete consumption of starting material and detection of product. The mixture was filtered, and the filtrate was concentrated in vacuo to obtain crude product. The crude product was purified by silica gel column chromatography (MeOH/DCM = 4%) to obtain **16-3** (5.80 g, 25.30 mmol, 36.92% yield, 90% purity) as a brown oil. LCMS: (ESI) *m*/*z* = 207.1 $[M+H]^+$; $^1H$ NMR (400 MHz, DMSO-$d_6$) δ 7.02 (d, *J* = 7.3 Hz, 1H), 6.29 - 6.21 (m, 2H), 4.35 (s, 1H), 3.38 (t, *J* = 6.2 Hz, 2H), 3.23 - 3.21 (m, 2H), 2.59 (t, *J* = 6.2 Hz, 2H), 2.41 (t, *J* = 7.6 Hz, 2H), 1.77 - 1.71 (m, 2H), 1.61 - 1.54 (m, 2H), 1.44 - 1.38 (m, 2H).

Synthesis of Intermediate **16-4**

To a solution of **16-3** (5.35 g, 36.36 mmol) and triphenylphosphine (9.54 g, 36.36 mmol) in tetrahydrofuran (100 mL) was added diisopropyl azodicarboxylate (10.78 g, 53.32 mmol, 10.47 mL) at 0 °C, and the mixture was stirred at 25 °C for 16 hours. LCMS showed complete consumption of starting material and detection of product. Water was added to the mixture, and extracted with ethyl acetate (200 mL × 2). The combined organic layer was washed with brine (200 mL), dried over anhydrous sodium sulfate, filtered, and concentrated in vacuo to obtain crude product. The crude product was purified by silica gel column chromatography (EA/PE = 40%) to obtain **16-4** (8.00 g, 14.31 mmol, 59.04% yield, 60% purity) as a brown oil.

Synthesis of Intermediate **16-5**

To a solution of **16-4** (8 g, 23.85 mmol) in ethanol (60 mL) was added hydrazine hydrate (3.82 g, 95.41 mmol, 3.82 mL, 80% purity) at 25 °C, and the mixture was stirred at 70 °C for 5 hours. LCMS showed complete consumption of starting material and detection of product. The mixture was filtered, and the filtrate was concentrated in vacuo to obtain crude product. The crude product was purified by silica gel column chromatography (MeOH/DCM = 9%) to obtain **16-5** (1.60 g, crude) as a brown oil. LCMS: (ESI) m/z = 206.20 $[M+H]^+$.

Synthesis of Intermediate **16-6**

To a mixture of **16-5** (2 g, 9.74 mmol) and potassium carbonate (2.69 g, 19.48 mmol) in dioxane (20 mL) and water (5 mL) was added benzyl chloroformate (1.83 g, 10.72 mmol) at 25 °C, and the mixture was stirred at 25 °C for 5 hours. LCMS showed complete consumption of starting material and detection of product. Water was added to the mixture, extracted with ethyl acetate (100 mL × 2), and washed with brine (100 mL). The combined organic layer was dried over anhydrous sodium sulfate, filtered, and concentrated in vacuo to obtain crude product. The crude product was purified by silica gel column chromatography (EA/PE = 60%) to obtain **16-6** (1.40 g, 3.71 mmol, 38.10% yield, 90% purity) as a brown oil. LCMS: (ESI) m/z = 340.20 $[M+H]^+$.

Synthesis of Intermediate **16-7**

To a solution of **16-6** (1.4 g, 4.12 mmol) in methanol (15 mL) was added palladium on carbon (0.3 g, 10%) at 25 °C, and the reaction solution was stirred under hydrogen atmosphere for 16 hours. LCMS showed complete consumption of starting material and detection of product. The reaction solution was filtered, and the filtrate was concentrated in vacuo to obtain **16-7** (700.00 mg, crude) as a brown oil. LCMS: (ESI) *m/z* = 206.1 $[M+H]^+$.

Synthesis of Intermediate **16-8**

To a mixture of **16-7** (0.7 g, 3.41 mmol) and potassium carbonate (942.47 mg, 6.82 mmol) in DMF (10 mL) was added **16-14** (1.26 g, 3.41 mmol) at 25 °C, and the mixture was stirred at 70 °C for 16 hours. LCMS showed complete consumption of starting material and detection of product. Water was added to the mixture, and extracted with dichloromethane (100 mL × 2). The combined organic layer was washed with brine (100 mL), dried over anhydrous sodium sulfate, filtered, and concentrated in vacuo to obtain crude product. The crude product was purified by silica gel column chromatography (MeOH/DCM = 8%) to obtain **16-8** (800.00 mg, 1.46 mmol, 42.69% yield, 90% purity) as a brown oil.

Synthesis of Intermediate **16-9**

To a mixture of **16-8** (0.8 g, 1.62 mmol) and methyl (2S)-2-(tert-butoxycarbonylamino)-4-oxobutanoate (411.41 mg, 1.78 mmol) in 1,2-dichloroethane (10 mL) was added acetic acid (145.69 mg, 2.43 mmol, 138.88 μL) and sodium triacetoxyborohydride (514.18 mg, 2.43 mmol) at 0 °C. The mixture was stirred at 25 °C for 2 hours. LCMS showed complete consumption of starting material and detection of product. Water was added to the mixture, and extracted with dichloromethane (50 mL × 2). The combined organic layer was washed with brine (50 mL), dried over anhydrous sodium sulfate, filtered, and concentrated in vacuo to obtain crude product. The crude product was purified by silica gel column chromatography (MeOH/DCM = 5%) to obtain **16-9** (700.00 mg, 887.48 μmol, yield 54.87%, purity 90%). LCMS: (ESI) m/z = 710.6 $[M+H]^+$.

Synthesis of Intermediate **16-10**

To a solution of **16-9** (0.7 g, 986.09 μmol) in dichloromethane (10 mL) was added trifluoroacetic acid (4 mL) at 25 °C, and this mixture was stirred at 25 °C for 2 hours. LCMS showed complete consumption of starting material and detection of product. The mixture was concentrated to obtain a residue. Aqueous sodium carbonate solution was added to the residue to adjust pH = 8. The mixture was extracted with dichloromethane (100 mL × 2). The combined organic layer was washed with brine (100 mL), dried over anhydrous sodium sulfate, filtered, and concentrated in vacuo to obtain **16-10** (500.00 mg, crude) as a brown oil. LCMS: (ESI) m/z = 610.5 $[M+H]^+$.

Synthesis of Intermediate **16-11**

To a solution of **16-10** (0.5 g, 820.00 μmol) in isopropanol (5 mL) was added 4-chloroquinazoline (148.46 mg, 902.00 μmol) at 25 °C. The mixture was concentrated in vacuo to obtain crude product. The crude product was purified by silica gel column chromatography (MeOH/DCM = 5%) to obtain **16-11** (260.00 mg, 281.89 μmol, 34.38% yield, 80% purity) as a brown oil. LCMS: (ESI) *m/z* = 738.5 $[M+H]^+$.

Synthesis of Compound **16**

**16-11** (0.26 g, 352.36 μmol) was dissolved in methanol (1 mL), tetrahydrofuran (1 mL), and water (0.5 mL), and lithium hydroxide (67.51 mg, 2.82 mmol) was added at 25 °C. This mixture was stirred at 25 °C for 2 h. LCMS showed complete consumption of starting material and detection of product. The mixture was filtered, and the filtrate was collected to obtain crude product. The crude product was purified by preparative high performance liquid chromatography (Column: Welchmax C18 21.2 × 250 mm, 10 μm: 25-25% (A= $H_2O$, B = ACN)) to obtain Compound **16** (50.00 mg, 66.05 μmol, yield 18.74%, purity 95.62%) as a gray gum. LCMS: (ESI) *m/z* = 724.5 $[M+H]^+$ ; $^1H$ NMR (400 MHz, DMSO-d6) δ 8.39 (s, 1H), 8.09 - 7.96 (m, 2H), 7.77 - 7.70 (m, 1H), 7.64 (d, *J*= 9.1 Hz, 1H), 7.53 - 7.43 (m, 1H), 6.96 (d, *J*= 7.2 Hz, 1H), 6.30 (s, 1H), 6.16 (d, *J*= 7.3 Hz, 1H), 4.24 - 4.17 (m, 1H), 3.60 - 3.57 (m, 2H), 3.56 - 3.51 (m, 4H), 3.50 - 3.48 (m, 4H), 3.46 - 3.43 (m, 4H), 3.39 - 3.37 (m, 10H), 3.23 - 3.19 (m, 2H), 2.58 (t, *J* = 6.3 Hz, 2H), 2.48 - 2.34 (m, 4H), 2.34 - 2.30 (m, 2H), 2.13 - 2.01 (m, 1H), 1.94 - 1.83 (m, 1H), 1.75 - 1.70 (m, 2H), 1.51 - 1.41 (m, 2H), 1.34 - 1.24 (m, 2H).

**Synthesis of Compound 17**

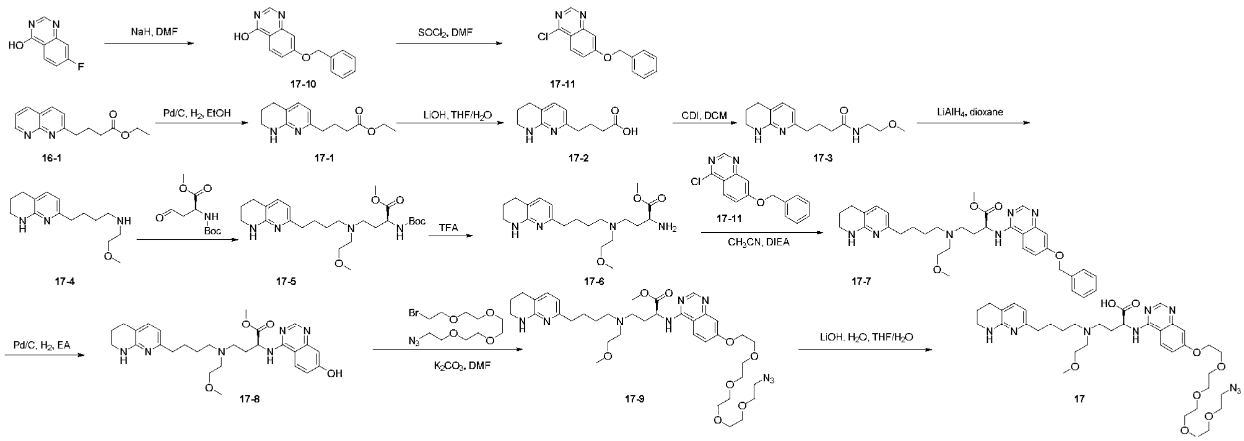


Synthesis of Intermediate **17-10**

Sodium hydride (974.80 mg, 24.37 mmol, 60% purity) was added to a solution of benzyl alcohol (2.64 g, 24.37 mmol, 2.52 mL) in N,N-dimethylformamide (20 mL). Then 7-fluoroquinazolin-4-ol (2 g, 12.18 mmol) was added, and the mixture was heated to 140 °C and reacted for 4 hours. After cooling to room temperature, the reaction was quenched with ice water, then 4M concentrated hydrochloric acid was added to adjust the pH to 3, resulting in a precipitate. The precipitate was filtered out, washed with water and diethyl ether. The solid was dried under vacuum to obtain **17-10** (2.80 g, crude) as a gray solid. LCMS: m/z $[M+H]^+$ = 253.1, RT = 0.891 min.

Synthesis of Intermediate **17-11**

A mixture of **17-10** (2.8 g, 11.10 mmol), thionyl chloride (10.00 mL) and DMF (0.3 mL) was heated at 90 °C for 3 hours. Excess thionyl chloride was removed by rotary evaporation. The residue was azeotroped with toluene and dried under vacuum to obtain **17-11** (2.80 g, crude) as a gray solid, which was used directly in the next step without further purification. LCMS: *m*/*z* = 271.0 $[M+H]^+$.

Synthesis of Intermediate **17-1**

To a solution of **16-1** (5 g, 20.47 mmol) in ethanol (50 mL) was added 10% palladium on carbon (800 mg, 6.59 mmol). The reaction was stirred under hydrogen atmosphere for 16 hours. Liquid chromatography detected that all starting material was converted to product. The catalyst was filtered off, and the filtrate was concentrated under reduced pressure to obtain **17-1** (4.80 g, crude) as a yellow oil. $^1$H NMR (400 MHz, $CDCl_3$) δ 7.02 (s, 1H), 6.31 (s, 1H), 4.82 (s, 1H), 4.12 - 4.04 (m, 2H), 3.41 - 3.32 (m, 2H), 2.66 (s, 2H), 2.56 (d, *J* = 8.6 Hz, 2H),2.31 (d, *J* = 15.1 Hz, 2H), 1.98 (q, *J* = 7.7 Hz, 2H), 1.88 (p, *J* = 6.0 Hz, 2H), 1.22 (d, *J* = 14.3 Hz, 3H). LCMS: m/z= 249.1 $[M+H]^+$.

Synthesis of Intermediate **17-2**

To a solution of **17-1** (4.7 g, 18.93 mmol) in tetrahydrofuran (30 mL) was added lithium hydroxide monohydrate (3.97 g, 94.64 mmol) at 25 °C, and the mixture was stirred at 60 °C for 4 hours. LCMS showed starting material converted to product. The mixture was added to 4M HCl (aq) to adjust the pH to 6, and extracted with (20% isopropanol in dichloromethane mixed solution) (100 mL × 2). The combined organic layer was washed with brine (50 mL × 2), dried over anhydrous sodium sulfate, filtered, and concentrated in vacuo to obtain **17-2** (4.10 g, crude) as a white solid, which was used in the next step without further purification. $^1$H NMR (400 MHz, $CD_3OD$) δ 7.56 (d, *J* = 9.4 Hz, 1H), 6.60 (d, *J = 7.3* Hz, 1H), 3.53 - 3.43 (m, 2H), 2.87 - 2.68 (m, 4H), 2.35 (t, *J* = 7.2 Hz, 2H), 2.02 - 1.87 (m, 4H). LCMS: *m*/*z* = 221.1 $[M+H]^+$.

Synthesis of Intermediate **17-3**

To a solution of **17-2** (4.1 g, 18.61 mmol) in dichloromethane (40 mL) was added 1,1'-carbonyldiimidazole (3.32 g, 20.48 mmol) and 2-methoxyethylamine (1.54 g, 20.44 mmol) at 0 °C, and the mixture was stirred at 25 °C for 2 hours. Liquid chromatography showed starting material converted to product. The mixture was diluted with water, extracted with dichloromethane (50 mL × 2), and washed with brine (50 mL × 2). The combined organic layer was dried over anhydrous sodium sulfate, filtered, and concentrated in vacuo. The crude product was washed with methyl tert-butyl ether and filtered to obtain **17-3** (2.90 g, 10.46 mmol, 56.17% yield) as a white solid. $^1$H NMR (400 MHz, $CD_3Cl$) δ 7.06 (d, *J* = 7.3 Hz, 1H), 6.32 (d, *J* = 7.3 Hz, 1H), 5.37 (s, 1H), 3.48 (q, *J*= 3.5, 3.1 Hz, 4H), 3.41 - 3.37 (m, 2H), 3.37 (s, 3H), 2.68 *(t, J*= 6.3 Hz, 2H), 2.58 *(t, J*= 7.0 Hz, 2H), 2.19 (t, *J* = 7.0 Hz, 2H), 1.97 - 1.84 (m, 4H). LCMS: *m*/*z* = 278.1 $[M+H]^+$.

Synthesis of Intermediate **17-4**

To a solution of **17-3** (2.4 g, 8.65 mmol) in dioxane (19 mL) was added lithium aluminum hydride (1 M, 19.04 mL) at 0 °C, and the mixture was stirred at 110 °C for 1 hour. Liquid chromatography showed starting material converted to product. The mixture was cooled to room temperature, then water (2 mL), aqueous sodium hydroxide solution (1M, 2 mL), and water (2 mL) were carefully added sequentially. The mixture was dried over anhydrous sodium sulfate and filtered. The filtrate was concentrated in vacuo to obtain **17-4** (2.20 g, crude) as a yellow oil. $^1$H NMR (400 MHz, $CD_3Cl$) δ 7.03 (d, *J* = 7.3 Hz, 1H), 6.32 (d, *J* = 7.3 Hz, 1H), 4.83 (s, 1H), 3.48 (t, *J* = 5.1 Hz, 2H), 3.38 (t, *J* = 5.5 Hz, 2H), 3.34 (s, 3H), 2.80 - 2.73 (m, 2H), 2.69 - 2.62 (m, 4H), 2.57 - 2.51 (m, 2H), 1.91 - 1.86 (m, 2H), 1.66 (d, *J* = 7.9 Hz, 2H), 1.56 (s, 2H). LCMS: m/z = 264.1 $[M+H]^+$.

Synthesis of Intermediate **17-5**

To a mixture of **17-4** (250 mg, 949.21 μmol) and methyl (2S)-2-(tert-butoxycarbonylamino)-4-oxobutanoate (241.45 mg, 1.04 mmol) in dichloroethane (4 mL) was added sodium triacetoxyborohydride (301.76 mg, 1.42 mmol) and acetic acid (85.50 mg, 1.42 mmol) at 0 °C. The mixture was stirred at 25 °C for 2 hours. The reaction solution was diluted with methanol and then concentrated in vacuo. The concentrate was dissolved in dichloromethane and saturated aqueous sodium bicarbonate solution. The organic phase was separated, and the aqueous layer was extracted with dichloromethane. The combined organic phases were dried over sodium sulfate, filtered, and concentrated in vacuo to obtain crude product. The crude product was purified by column chromatography (0-10% methanol: dichloromethane) to obtain **17-5** (265.00 mg, 553.67 μmol, 58.33% yield). LCMS: *m*/*z* = 479.4 $[M+H]^+$.

Synthesis of Intermediate **17-6**

To a solution of **17-5** (265 mg, 553.67 μmol) in dichloromethane (2 mL) was added trifluoroacetic acid (1 mL) at 25 °C. The mixture was stirred at 25 °C for 2 hours. It showed starting material converted to product. The solvent was removed by rotary evaporation, and the residue was diluted with water, then the pH of the solution was adjusted to 7-8 with aqueous sodium bicarbonate solution. It was then extracted with dichloromethane. The combined organic layer was dried over anhydrous sodium sulfate, filtered, and concentrated in vacuo to obtain crude product **17-6** (200.00 mg, crude) as a yellow oil. $^1$H NMR (400 MHz, DMSO-$d_6$) δ 7.01 (d, *J* = 7.3 Hz, 1H), 6.24 (d, *J* = 7.2 Hz, 2H), 3.59 (s, 3H), 3.27 - 3.16 (m, 6H), 2.59 (t, *J* = 6.2 Hz, 2H), 2.39 (dt, *J* = 13.7, 7.7 Hz, 6H), 1.93 (s, 2H), 1.71 (d, *J* = 12.6 Hz, 4H), 1.51 (dd, *J* = 20.7, 8.5 Hz, 4H), 1.41 - 1.32 (m, 2H). LCMS: (ESI) *m/z* = 379.3 [M+H]$^+$.

Synthesis of Intermediate **17-7**

A mixture of **17-6** (200 mg, 528.39 μmol) and 7-benzyloxy-4-chloroquinazoline (143.04 mg, 528.35 μmol) in acetonitrile (5 mL) was refluxed at 90 °C for 16 hours. After cooling to room temperature, the solvent was removed, and the residue was purified by column chromatography (methanol: dichloromethane = 0-3%) to obtain **17-7** (120.00 mg, 195.84 μmol, 37.06% yield) as a yellow oil. LCMS: (ESI) *m/z* = 613.4 [M+H]$^+$.

Synthesis of Intermediate **17-8**

To a solution of **17-7** (200 mg, 326.39 μmol) in ethyl acetate (10 mL) was added palladium hydroxide on carbon (40 mg). This reaction was heated to 70 °C under hydrogen atmosphere and stirred for 16 hours. LCMS showed starting material was consumed and product was detected. The reaction mixture was cooled to room temperature. The reaction mixture was filtered, the precipitated solid was washed with ethyl acetate, and the filtrate was concentrated in vacuo to obtain **17-8** (130.00 mg, crude) as a yellow oil. LCMS: (ESI) *m/z* = 523.4 [M+H]$^+$; $^1$H NMR (400 MHz, DMSO-*d6*) δ 10.33 (s, 1H), 8.34 (d, *J* = 7.0 Hz, 1H), 8.27 (s, 1H), 8.11 (d, *J* = 9.1 Hz, 1H), 6.99 (s, 1H), 6.91 (d, *J* = 6.6 Hz, 2H), 6.16 (s, 1H), 6.09 (d, *J*= 7.2 Hz, 1H), 4.80 (s, 1H), 3.59 (s, 3H), 3.20 - 3.16 (m, 2H), 3.07 (s, 3H), 2.63 - 2.51 (m, 5H), 2.38 - 2.25 (m, 3H), 1.96 (d, *J* = 20.3 Hz, 2H), 1.74 - 1.67 (m, 2H), 1.56 - 1.25 (m, 6H), 1.22 (d, *J* = 9.7 Hz, 2H).

Synthesis of Intermediate **17-9**

To a solution of **17-8** in N,N-dimethylformamide (3 mL) was added potassium carbonate (68.65 mg, 497.48 μmol) and 1-azido-2-[2-[2-[2-(2-bromoethoxy)ethoxy]ethoxy]-ethane (113.59 mg, 348.23 μmol), and the resulting reaction mixture was heated at 80 °C for 3 hours. LCMS showed starting material was consumed and product was detected. The reaction solution was cooled to ambient temperature, quenched with water, and extracted with ethyl acetate. The combined organic phases were washed with brine, dried over anhydrous sodium sulfate, filtered, and concentrated to obtain **17-9** (190.00 mg, crude) as a yellow oil. LCMS: (ESI) *m/z* = 768.6 [M+H]$^+$.

Synthesis of Compound **17**

To a solution of **17-9** (190 mg, 247.42 μmol) in tetrahydrofuran (3 mL) was added water (1 mL) and LiOH·OH (51.91 mg, 1.24 mmol), and the resulting reaction mixture was stirred at 25 °C for 2 hours. LC-MS showed starting material was consumed and product was detected. The reaction mixture was filtered and concentrated. The residue was purified by reverse phase preparative high performance liquid chromatography to obtain Compound **17** (25 mg, 33.16 μmol, 13.40% yield) as a white solid. LCMS: (ESI) *m/z* = 754.6 [M+H]$^+$; $^1$H NMR (400 MHz, DMSO-d6) δ 8.36 (s, 1H), 8.18 (d, *J* = 4.4 Hz, 1H), 8.12 (d, *J* = 9.1 Hz, 1H), 7.17-7.12 (m, 1H), 7.09 (d, *J* = 2.5 Hz, 1H), 6.97 (d, *J* = 7.1 Hz, 1H), 6.32 (s, 1H), 6.16 (d, *J* = 7.4 Hz, 1H), 4.60 (d, *J* = 5.8 Hz, 1H), 4.23 (t, *J* = 4.4 Hz, 2H), 3.80 (t, *J* = 4.3 Hz, 2H), 3.62-3.52 (m, 11H), 3.38 (s, 8H), 3.21 (t, *J* = 5.1 Hz, 2H), 3.14 (s, 3H), 2.80-2.70 (m, 2H), 2.67-2.54 (m, 4H), 2.47-2.43 (m, 1H), 2.34 (t, *J* = 7.3 Hz, 2H), 2.10-1.91 (m, 2H), 1.76-1.70 (m, 2H), 1.57-1.34 (m, 4H).

Synthesis of Compound **18**

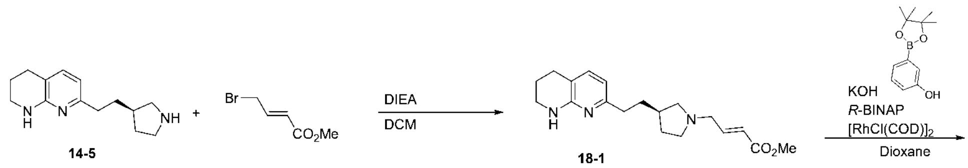


14-5 18-1

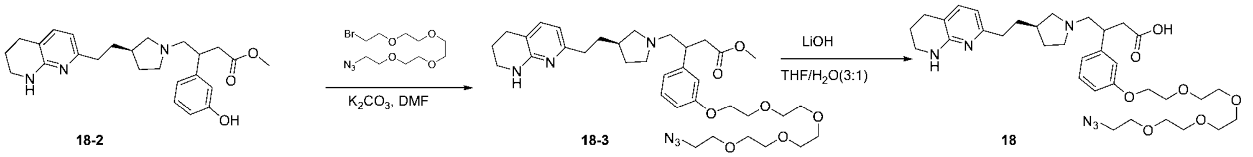


18-2 18-3 18

Synthesis of Intermediate **18-1**

To a solution of **14-5** (1.35 g, 3.87 mmol) in dichloromethane (20 mL) under nitrogen protection was added N,N-diisopropylethylamine (2.50 g, 19.36 mmol). The reaction solution was cooled to 0 °C, and methyl (E)-4-bromobut-2-enoate (693.07 mg, 3.87 mmol) was slowly added. The mixture was stirred at room temperature for 18 h. LCMS showed product formation. The reaction mixture was diluted with 30 mL of water. The organic phase was separated, and the aqueous layer was further extracted with dichloromethane (50 mL × 2). The organic phases were combined, dried over anhydrous sodium sulfate, filtered, and concentrated. The crude product was purified on a silica gel column, eluting with 0-20% methanol/dichloromethane, to obtain **18-1** (0.786 g, 2.34 mmol, yield 60.39%) as a yellow oil, stored at 0-4 °C. LCMS: *m/z* = 330.2 $[M+H]^+$; $^1H$ NMR (400 MHz, $CDCl_3$) δ 7.08 (d, *J* = 6.7 Hz, 1H), 6.97 (dt, *J* = 15.5, 5.9 Hz, 1H), 6.33 (d, *J* = 7.1 Hz, 1H), 5.97 (d, *J* = 15.7 Hz, 1H), 3.73 (s, 3H), 3.40 (s, 2H), 3.29 - 3.15 (m, 2H), 2.83 (d, *J* = 7.4 Hz, 1H), 2.76 - 2.61 (m, 4H), 2.59 - 2.41 (m, 3H), 2.15 (p, *J* = 7.9 Hz, 2H), 2.01 (dd, *J* = 12.4, 8.0 Hz, 1H), 1.95 - 1.86 (m, 2H), 1.73 (q, *J* = 7.6 Hz, 2H), 1.45 (dq, *J* = 14.8, 6.8 Hz, 1H).

Synthesis of Intermediate **18-2**

To a solution of **18-1** (530 mg, 1.61 mmol) in dioxane (3 mL) was added potassium hydroxide (3.8 M, 1.27 mL) and 3-(4,4,5,5-tetramethyl-1,3,2-dioxaborolan-2-yl)phenol (1.77 g, 8.04 mmol). The mixture was degassed with argon for 15 minutes. In another vial, R-BINAP (120.21 mg, 193.06 μmol) and $[RhCl(COD)]_2$ (39.66 mg, 80.44 μmol) were dissolved in 1 mL of 1,4-dioxane, and each was degassed with argon for 15 min. The two were mixed and degassed with argon for another 10 min. The reaction mixture was stirred at 50 °C for 18 h. LCMS showed product formation. The reaction mixture was diluted with 5 mL of water. The organic phase was separated, and the aqueous phase was further extracted with ethyl acetate (20 mL × 2). The combined organic phases were dried over sodium sulfate, filtered, and concentrated. The crude product was purified by preparative high performance liquid chromatography to obtain **18-2** (221.00 mg, 516.57 μmol, yield 32.11%, purity 99%) as an off-white solid. LCMS: *m/z* = 424.4 $[M+H]^+$; $^1H$ NMR (400 MHz, $CDCl_3$) δ 7.14 - 7.05 (m, 2H), 6.74 - 6.59 (m, 3H), 6.31 (d, *J* = 7.3 Hz, 1H), 5.31 (s, 1H), 3.58 (d, *J* = 6.4 Hz, 3H), 3.38 (t, *J*= 5.2 Hz, 2H), 3.25 (p, *J*= 7.3 Hz, 1H), 2.90 - 2.29 (m, 14H), 2.19 - 2.01 (m, 2H), 1.76 - 1.56 (m, 2H), 1.36 (ddt, *J* = 13.6, 9.6, 6.9 Hz, 1H).

Synthesis of Intermediate **18-3**

To a stirred solution of **18-2** (150 mg, 354.15 μmol) in N,N-dimethylformamide (3 mL) was added potassium carbonate (97.89 mg, 708.30 μmol) and 1-azido-2-[2-[2-[2-(2-bromoethoxy)ethoxy]ethoxy]butyrate ethoxyethane (161.73 mg, 495.81 μmol). The reaction solution was stirred at 100 °C overnight. LCMS showed product formation. The reaction was cooled to room temperature, quenched with water, and extracted with ethyl acetate. The organic phase was washed with brine, dried over anhydrous sodium sulfate, filtered, and concentrated. Purification on a silica gel column (MeOH: DCM = 0-10%) gave **18-3** (130.00 mg, 192.43 μmol, yield 54.33%, purity 99%) as a yellow oil. LCMS: *m/z* = 669.5 $[M-N2+H]^+$; $^1H$ NMR (400 MHz, $CDCl_3$) δ 7.23 - 7.07 (m, 2H), 6.83 - 6.69 (m, 3H), 6.32 (d, *J*= 7.4 Hz, 1H), 4.15 - 4.05 (m, 2H), 3.87 - 3.79 (m, 2H), 3.75 - 3.61 (m, 16H), 3.56 (s, 3H), 3.42 - 3.35 (m, 4H), 2.86 (dd, *J* = 15.5, 6.4 Hz, 1H), 2.69 (t, *J* = 6.4 Hz, 2H), 2.59 - 2.31 (m, 5H), 2.25 - 1.84 (m, 10H), 1.78 - 1.66 (m, 2H), 1.44 - 1.36 (m, 1H).

Synthesis of Compound **18**

To a solution of **18-3** (130 mg, 194.37 μmol) in tetrahydrofuran/water (3.0 mL) was added lithium hydroxide (18.62 mg, 777.49 μmol). The reaction mixture was stirred at 25 °C for 18 h. LCMS showed product formation. The solvent was removed by rotary evaporation. The crude product was dissolved in ACN/$H_2O$ (1:1) and purified by high performance liquid

chromatography (Nanochrom ChromCore C18 21.2 × 250 mm, 10 μm, $H_2O$-ACN 40-60%) to obtain **18** (55.73 mg, 81.22 μmol, yield 41.78%, purity 95.423%, SFC: ee% = 64.89%) as a yellow oil. LCMS: *m/z* = 655.6 [M+H]$^+$ ; $^1$H NMR (400 MHz, $CDCl_3$) δ 7.21 (t, *J* = 8.0 Hz, 1H), 7.15 - 7.04 (m, 1H), 6.84 - 6.71 (m, 3H), 6.29 (d, *J* = 7.1 Hz, 1H), 4.16 - 4.08 (m, 2H), 3.89 - 3.83 (m, 2H), 3.77 - 3.65 (m, 14H), 3.48 - 3.37 (m, 5H), 3.08 - 2.66 (m, 8H), 2.59 - 2.41 (m, 3H), 2.29 (s, 1H), 2.20 - 1.98 (m, 2H), 1.88 (d, 3H), 1.52 (d, 2H).

The compound was purified by SFC (Column: DAICEL CHIRALPAK AD (250mm*50mm, 10μm), Mobile phase: [Carbon dioxide - Acetonitrile/Methanol (0.1% Ammonia)], B%: 50%, isocratic elution mode) to obtain yellow oily compound **isomer1: 18-P1** and yellow oily compound **isomer2: 18-P2.**

**Isomer1:** 294.1 mg

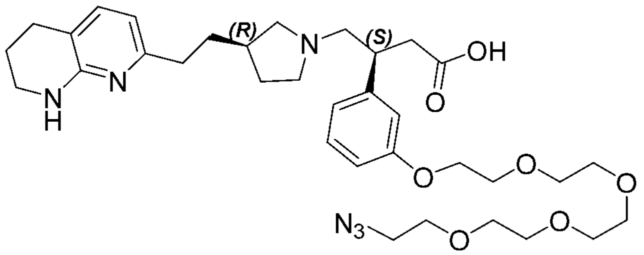

MS m/z (ESI): 655.4 [M+H]$^+$;

Analytical Chiral SFC: RT = 0.963 min, >99.5% purity, Chiralpak AD-3 50*4.6mm I.D., 3μm, Carbon dioxide - Methanol/Acetonitrile = 4/1 (0.05% DEA), B%: 40%, Flow rate 4 mL/min. $^1$H NMR (400 MHz, $CDCl_3$) δ 7.26-7.19 (m, 1H), 7.14 (d, *J*= 7.0 Hz, 1H), 6.84-6.74 (m, 3H), 6.30 (d, *J* = 7.3 Hz, 1H), 4.19-4.09 (m, 2H), 3.91-3.84 (m, 2H), 3.81-3.59 (m, 15H), 3.48-3.37 (m, 5H), 3.09-2.91 (m, 2H), 2.81-2.66 (m, 5H), 2.59-2.36 (m, 3H), 2.35-1.98 (m, 3H), 1.97-1.82 (m, 3H), 1.68-1.39 (m, 2H).

**Isomer2:** 88.21 mg

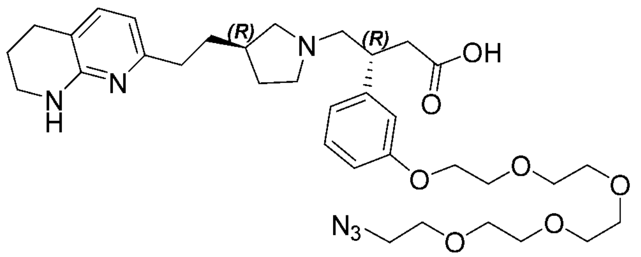

MS m/z (ESI): 655.4 [M+H]$^+$;

Analytical Chiral SFC: RT = 1.465 min, 98.4% purity, Chiralpak AD-3 50*4.6mm I.D., 3μm, Carbon dioxide - Methanol/Acetonitrile = 4/1 (0.05% DEA), B%: 40%, Flow rate 4 mL/min.

$^1$H NMR (400 MHz, $CDCl_3$) δ 7.23 (t, *J* = 7.9 Hz, 1H), 7.09 (d, *J* = 7.5 Hz, 1H), 6.85-6.69 (m, 3H), 6.32 (d, *J* = 7.3 Hz, 1H), 5.71-5.34 (m, 1H), 4.17-4.07 (m, 2H), 3.87 (t, *J* = 4.8 Hz, 2H), 3.77-3.55 (m, 15H), 3.45-3.37 (m, 4H), 3.36-3.00 (m, 4H), 2.88-2.78 (m, 3H), 2.71 (t, *J* = 6.3 Hz, 2H), 2.56 (t, *J* = 7.3 Hz, 3H), 2.40-2.25 (m, 1H), 2.22-2.06 (m, 1H), 1.96-1.71 (m, 4H), 1.69-1.52 (m, 1H).

**Synthesis of Compound 20**

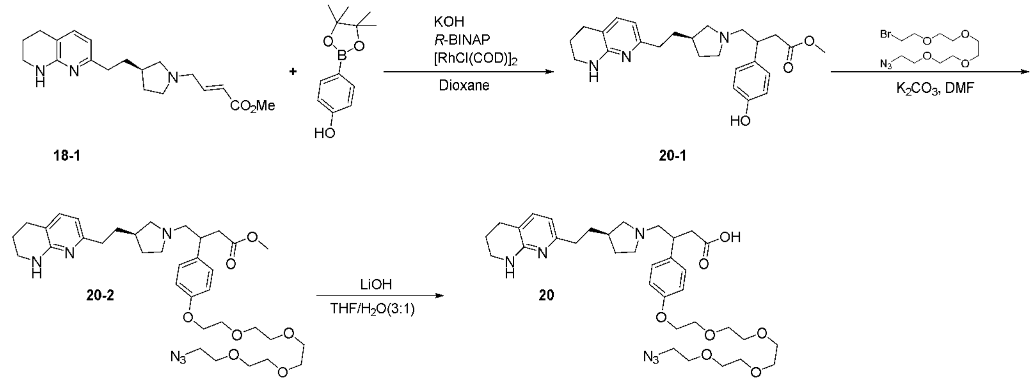


18-1 20-1 20-2 20

Synthesis of Intermediate **20-1**

To a solution of **18-1** (600 mg, 1.82 mmol) in dioxane (8 mL) was added potassium hydroxide (3.8 M, 1.44 mL) and 3-(4,4,5,5-tetramethyl-1,3,2-dioxaborolan-2-yl)phenol (2.00 g, 9.11 mmol). The mixture was degassed with argon for 15 minutes. In another vial, R-BINAP (136.09 mg, 218.56 μmol) and $[RhCl(COD)]_2$ (44.90 mg, 91.06 μmol) were dissolved in 3 mL of 1,4-dioxane, and each was degassed with argon for 15 min. The two were mixed and degassed with argon for another 10 min. The reaction mixture was stirred at 50 °C for 18 h. LCMS showed product formation. The reaction mixture was diluted with 5 mL of water. The organic phase was separated, and the aqueous phase was further extracted with ethyl acetate (20 mL × 2). The combined organic phases were dried over anhydrous sodium sulfate, filtered, and concentrated. Preparation gave **20-1** (206.00 mg, 481.51 μmol, 26.44% yield, 99% purity) as a yellow oil. LCMS: *m/z* = 424.3 $[M+H]^+$ ; $^1H$ NMR (400 MHz, $CDCl_3$) δ 7.07 (d, *J* = 7.3 Hz, 1H), 6.96 (dd, *J* = 8.4, 5.7 Hz, 2H), 6.62 - 6.57 (m, 2H), 6.34 - 6.30 (m, 1H), 5.03 (s, 1H), 3.55 (s, 3H), 3.41 - 3.34 (m, 2H), 3.19 (p, *J*= 7.2, 6.8 Hz, 1H), 2.90 - 2.20 (m, 14H), 2.05 (dq, *J* = 25.6, 8.3 Hz, 2H), 1.66 (p, *J* = 7.2 Hz, 2H), 1.34 (td, *J*= 13.2, 12.5, 6.2 Hz, 1H).

Synthesis of Intermediate **20-2**

To a stirred solution of **20-1** (206 mg, 486.37 μmol) in N,N-dimethylformamide (3 mL) was added potassium carbonate (134.44 mg, 972.74 μmol) and 1-azido-2-[2-[2-[2-[2-(2-bromoethoxy)ethoxy]ethoxy]butyrate ethoxyethane (222.11 mg, 680.92 μmol). The reaction solution was stirred at 100 °C overnight. LCMS showed product formation. The reaction was cooled to room temperature, quenched with water, and extracted with ethyl acetate. The organic phase was washed with brine, dried over anhydrous sodium sulfate, filtered, and concentrated. Purification on a silica gel column (MeOH: DCM = 0-10%) gave **20-2** (214.00 mg, 310.37 μmol, 63.81% yield, 97% purity) as an orange oil. LCMS: *m/z* = 669.5 $[M\text{-}N2+H]^+$; $^1H$ NMR (400 MHz, $CDCl_3$) δ 7.12 - 7.04 (m, 3H), 6.82 (d, *J* = 8.7 Hz, 2H), 6.32 (d, *J* = 7.3 Hz, 1H), 4.11 - 4.06 (m, 2H), 3.85 - 3.81 (m, 2H), 3.72 - 3.70 (m, 2H), 3.66 (d, *J* = 1.7 Hz, 12H), 3.54 (d, *J*= 1.5 Hz, 3H), 3.39 - 3.35 (m, 3H), 2.86 (dd, *J* = 15.3, 6.1 Hz, 1H), 2.68 (t, *J* = 6.3 Hz, 3H), 2.61 - 2.27 (m, 6H), 2.24 - 1.83 (m, 9H), 1.69 (ddt, *J* = 10.9, 8.3, 4.0 Hz, 2H), 1.44 - 1.33 (m, 1H).

Synthesis of Compound **20**

To a solution of **20-2** (214 mg, 319.97 μmol) in tetrahydrofuran/water (4.0 mL) was added lithium hydroxide (38.32 mg, 1.60 mmol). The reaction mixture was stirred at 25 °C for 18 h. LCMS showed product formation. The reaction solution was evaporated to dryness. The crude product was dissolved in ACN/$H_2O$ (1:1) and purified by high performance liquid chromatography (Nanochrom ChromCore C18 21.2 × 250 mm, 10 μm, (0.1% FA) $H_2O$-ACN 35-54%) to obtain Compound **20** (102.20 mg, 148.45 μmol, 46.40% yield, 95% purity, SFC: ee% = 55.6%) as a yellow oil. LCMS: *m/z* = 655.5 $[M+H]^+$; $^1H$ NMR (400 MHz, $CDCl_3$) δ 7.11 - 7.01 (m, 3H), 6.83 (d, *J* = 8.7 Hz, 2H), 6.27 (t, *J* = 7.1 Hz, 1H), 4.12 - 4.06 (m, 2H), 3.85 - 3.79 (m, 2H), 3.73 - 3.63 (m, 16H), 3.37 (q, *J* = 6.0, 5.0 Hz, 5H), 3.29 - 3.06 (m, 1H), 3.04 - 2.62 (m, 7H), 2.59 - 2.23 (m, 4H), 2.19 - 1.95 (m, 2H), 1.92 - 1.75 (m, 3H), 1.51 (dtd, *J* = 55.2, 10.1, 9.0, 5.0 Hz, 2H).

The compound was purified by SFC (Column: DAICEL CHIRALPAK AD (250mm*50mm, 10μm), Mobile phase: [Carbon dioxide - Acetonitrile/Methanol (0.1% Ammonia)], B%: 50%, isocratic elution mode) to obtain yellow oily compound **isomer1: 20-P1** and yellow oily compound **isomer2: 20-P2**.

**Isomer1:** 800.0 mg

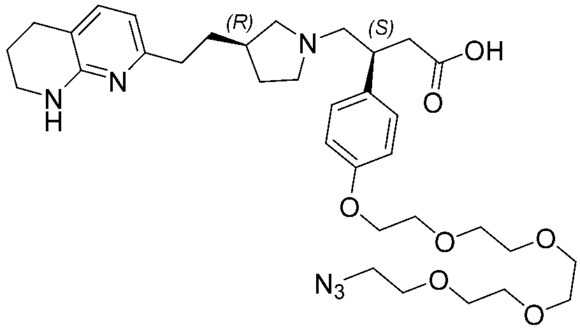

MS $m/z$ (ESI): 655.4 $[M+H]^+$;
Analytical Chiral SFC: RT = 1.325 min, >99.5% purity, Chiralpak AD-3 50*4.6mm I.D., 3μm, Carbon dioxide - Methanol/Acetonitrile = 4/1 (0.05% DEA), B%: 40%, Flow rate 4 mL/min. $^1H$ NMR (400 MHz, $CDCl_3$) δ 7.14-7.10 (m, 3H), 6.89-6.86 (m, 2H), 6.30 (d, $J$ = 7.2 Hz, 1H), 4.14-4.12 (m, 2H), 3.87-3.80 (m, 2H), 3.75-3.67 (m, 15H), 3.42-3.40 (m, 6H), 2.99 (m, 2H), 2.74-2.69 (m, 5H), 2.55-2.50 (m, 3H), 2.20-1.87 (m, 5H), 1.63-1.44 (m, 2H).

**Isomer2:** 88.2 mg

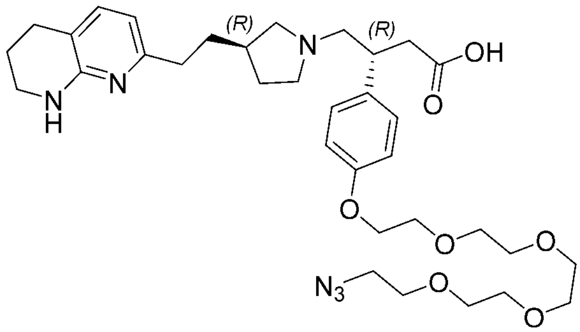

MS $m/z$ (ESI): 655.4 $[M+H]^+$;
Analytical Chiral SFC: RT = 1.835 min, 97.3% purity, Chiralpak AD-3 50*4.6mm I.D., 3μm, Carbon dioxide - Methanol/Acetonitrile = 4/1 (0.05% DEA), B%: 40%, Flow rate 4 mL/min.

$^1H$ NMR (400 MHz, $CDCl_3$) δ 7.14-7.07 (m, 3H), 6.87 (d, $J$ = 8.4 Hz, 2H), 6.32 (d, $J$ = 7.6 Hz, 1H), 4.13-4.11 (m, 2H), 3.86-3.74 (m, 2H), 3.73-3.67 (m, 15H), 3.42-3.40 (m, 5H), 3.30-3.05 (m, 4H), 2.95-2.75 (m, 3H), 2.73-2.69 (m, 2H), 2.59-2.56 (m, 2H), 2.40-2.05 (m, 2H), 1.93-1.61 (m, 5H).

Compounds **53, 57, 58, 59, 61, 72, 83, 86, 87, 49, 71, 84** were synthesized similarly to **18** and 20. The general synthetic method is as follows:

Synthesis of Compounds **53, 57, 58, 59, 61, 72, 83, 86, 87**

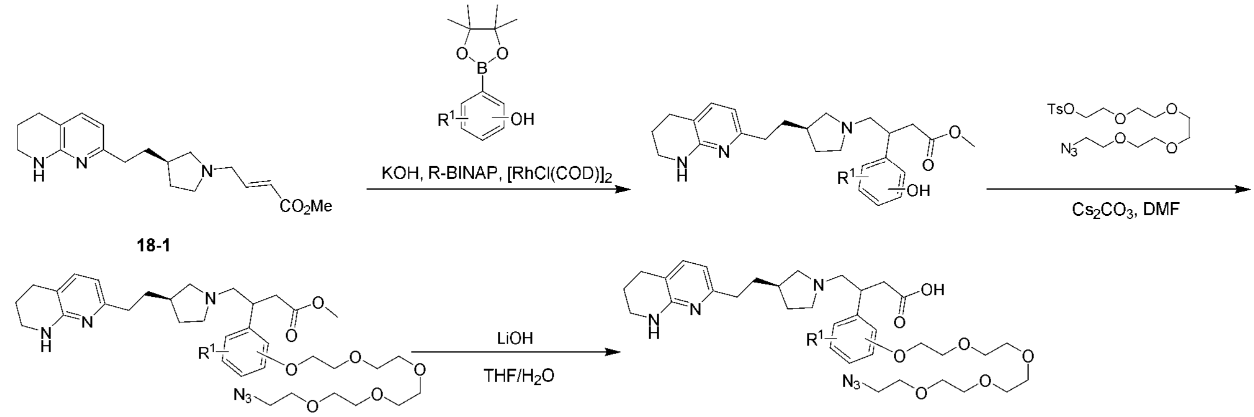


18-1

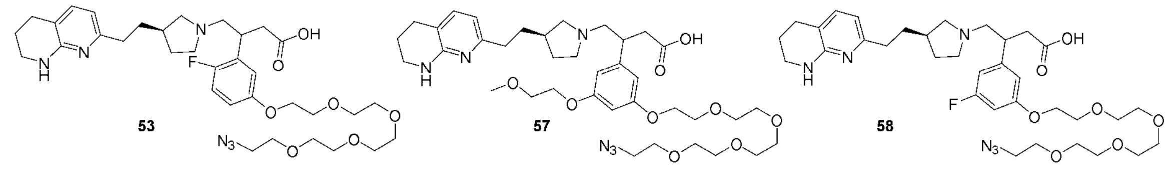

53 57 58

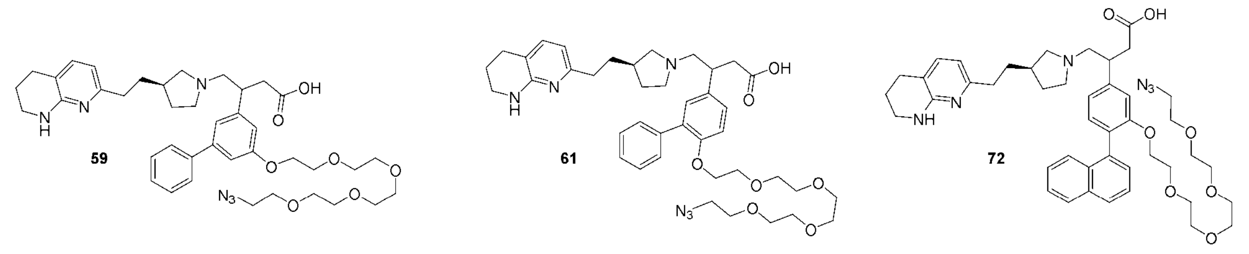

59 61 72

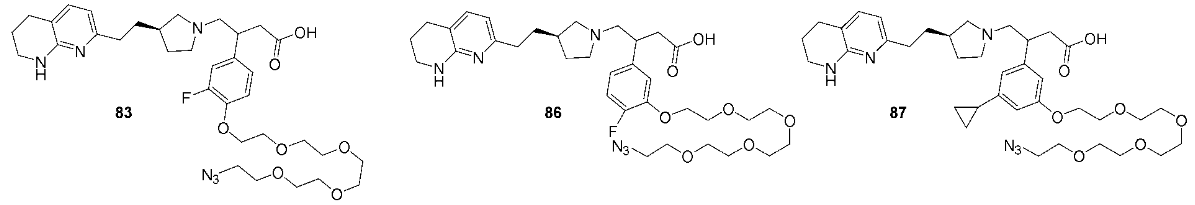

83 86 87

Compound **53:** Yellow oily compound, 98.08% purity, SFC: ee%: 55.52%. MS *m/z* (ESI): 673.5 $[M+H]^+$; $^1$H NMR (400 MHz, $CDCl_3$) δ 7.08-6.92 (m, 2H), 6.67-6.47 (m, 2H), 6.29-6.16 (m, 1H), 4.07-3.98 (m, 2H), 3.77 (t, *J* = 4.8 Hz, 2H), 3.68-3.54 (m, 14H), 3.37-3.24 (m, 4H), 3.23-3.00 (m, 1H), 2.97-2.79 (m, 2H), 2.75-2.56 (m, 5H), 2.50-2.31 (m, 3H), 2.30-2.15 (m, 1H), 2.13-1.89 (m, 2H), 1.87-1.71 (m, 3H), 1.57-1.30 (m, 2H), 1.29-1.05 (m, 1H).

Compound **57:** Yellow oily compound, >99.5% purity, SFC: ee%: 26.98%. MS m/z (ESI): 729.3 $[M+H]^+$; $^1$H NMR (400 MHz, $CDCl_3$) δ 7.16 (d, *J* = 7.1 Hz, 1H), 6.39 (s, 2H), 6.36 - 6.24 (m, 2H), 4.10 (d, *J* = 4.8 Hz, 4H), 3.86 (d, *J* = 5.0 Hz, 2H), 3.77 - 3.73 (m, 4H), 3.72 - 3.67 (m, 12H), 3.48 - 3.46 (m, 3H), 3.42 - 3.39 (m, 2H), 3.23 - 3.15 (m, 1H), 3.07 - 2.93 (m, 2H), 2.90 - 2.82 (m, 2H), 2.80 - 2.69 (m, 5H), 2.64 - 2.57 (m, 2H), 2.54 - 2.45 (m, 2H), 2.41 - 2.27 (m, 2H), 2.20 - 2.16 (m, 1H), 2.10 - 2.04 (m, 1H), 1.96 - 1.91 (m, 2H), 1.67 - 1.57 (m, 1H), 1.54 - 1.42 (m, 1H).

Compound **58:** Yellow oily compound, 99.0% purity, SFC: ee%: 88.76%. MS m/z (ESI): 781.3 $[M+H]^+$; $^1$H NMR (400 MHz, DMSO-$d_6$) δ 7.02 (d, *J* = 7.3 Hz, 1H), 6.71-6.63 (m, 3H), 6.31-6.21 (m, 2H), 4.14-4.03 (m, 2H), 3.74 - 3.71 (m, 2H), 3.62-3.57 (m, 5H), 3.56 - 3.52 (m, 10H), 3.38 (s, 2H), 3.25-3.22 (m, 2H), 2.84-2.57 (m, 8H), 2.44-2.33 (m, 4H), 2.27 (s, 1H), 2.07-1.85 (m, 2H), 1.78-1.69 (m, 2H), 1.65-1.55 (m, 2H), 1.39-1.29 (m, 1H).

Compound **59:** Yellow oily compound, 99.4% purity, SFC: ee%: 41.84%. MS m/z (ESI): 731.5 $[M+H]^+$; $^1$H NMR (400 MHz, $CDCl_3$) δ 7.57 (t, *J* = 8.9 Hz, 2H), 7.45 (t, *J* = 7.5 Hz, 2H), 7.41-7.32 (m, 1H), 7.18-7.08 (m, 1H), 7.06-6.92 (m, 2H), 6.80-6.67 (m, 1H), 6.37-6.22 (m, 1H), 4.21 (s, 2H), 3.90 (t, *J* = 4.6 Hz, 2H), 3.76 (d, *J* = 5.0 Hz, 2H), 3.73-3.64 (m, 11H), 3.51-3.19 (m, 6H), 3.07-2.30 (m, 12H), 2.23-1.50 (m, 7H).

Compound **61:** Yellow oily compound, 99.4% purity, SFC: ee%: 72.42%. MS *m/z* (ESI): 731.5 $[M+H]^+$; $^1$H NMR (400 MHz, $CDCl_3$) δ 7.62-7.52 (m, 2H), 7.44-7.38 (m, 2H), 7.36-7.30 (m, 1H), 7.19-7.05 (m, 3H), 6.94 (d, *J* = 8.3 Hz, 1H), 6.36-6.27 (m, 1H), 4.11 (t, *J* = 4.7 Hz, 2H), 3.78 (t, *J* = 4.5 Hz, 2H), 3.70-3.63 (m, 10H), 3.62-3.59 (m, 4H), 3.51-3.36 (m, 5H), 3.33-3.14 (m, 2H), 2.84 (m, 4H), 2.81-2.69 (m, 4H), 2.64-2.41 (m, 3H), 2.33-2.00 (m, 3H), 1.97-1.81 (m, 3H), 1.53-1.37 (m, 1H).

Compound **72:** Yellow solid compound, >99.5% purity, SFC: ee%: 21.66%. MS *m/z* (ESI): 781.5 $[M+H]^+$; $^1H$ NMR (400 MHz, $CDCl_3$) δ 7.88-7.79 (m, 2H), 7.58 (dd, *J* = 8.3, 19.1 Hz, 1H), 7.53-7.41 (m, 2H), 7.41-7.33 (m, 2H), 7.23 (dd, *J* = 3.7, 7.6 Hz, 1H), 7.11 (dd, *J* = 7.2, 19.6 Hz, 1H), 6.95-6.80 (m, 2H), 6.30 (dd, *J* = 7.3, 10.5 Hz, 1H), 4.00 (q, *J* = 4.7 Hz, 2H), 3.65-3.60 (m, 6H), 3.58-3.53 (m, 3H), 3.50-3.39 (m, 7H), 3.38-3.35 (m, 2H), 3.23 (q, *J* = 4.8 Hz, 3H), 3.18-3.07 (m, 3H), 3.06-2.89 (m, 3H), 2.88-2.73 (m, 2H), 2.73-2.67 (m, 2H), 2.66-2.54 (m, 2H), 2.48 (ddd, *J* = 5.9, 12.3, 18.3 Hz, 1H), 2.40-2.27 (m, 1H), 2.24-1.99 (m, 2H), 1.89-1.74 (m, 2H), 1.70-1.39 (m, 2H).

Compound **83:** Yellow oily compound, >99.5% purity, SFC: ee%: 72.38%. MS *m/z* (ESI): 673.6 $[M+H]^+$; $^1H$ NMR (400 MHz, $CDCl_3$) δ 7.16 (d, *J* = 7.3 Hz, 1H), 7.02-6.79 (m, 3H), 6.30 (d, *J* = 7.3 Hz, 1H), 4.19 (t, *J* = 4.9 Hz, 2H), 3.88 (t, *J* = 4.9 Hz, 2H), 3.80-3.73 (m, 2H), 3.72-3.67 (m, 11H), 3.48-3.35 (m, 5H), 3.05-2.90 (m, 2H), 2.83-2.77 (m, 1H), 2.76-2.65 (m, 4H), 2.63-2.57 (m, 1H), 2.51-2.39 (m, 3H), 2.19-1.99 (m, 4H), 1.97-1.82 (m, 4H), 1.48-1.38 (m, 1H).

Compound **86:** Yellow oily compound, >99.5% purity, SFC: ee%: 62.76%. MS *m/z* (ESI): 673.4 $[M+H]^+$; $^1H$ NMR (400 MHz, $CDCl_3$) δ 7.20-7.08 (m, 1H), 7.05-6.95 (m, 1H), 6.88-6.65 (m, 2H), 6.36-6.25 (m, 1H), 4.24-4.17 (m, 2H), 3.89 (t, *J* = 4.7 Hz, 2H), 3.79-3.73 (m, 2H), 3.73-3.65 (m, 11H), 3.47-3.38 (m, 4H), 3.01-2.93 (m, 1H), 2.80 (d, *J*= 6.4 Hz, 2H), 2.75-2.66 (m, 4H), 2.58 (d, *J*= 7.1 Hz, 1H), 2.51-2.37 (m, 4H), 2.17-2.01 (m, 3H), 1.97-1.76 (m, 4H), 1.64-1.53 (m, 1H), 1.45-1.35 (m, 1H).

Compound **87:** Yellow oily compound, 98.6% purity, SFC: ee%: 35.20%. MS m/z (ESI): 695.4 $[M+H]^+$; $^1H$ NMR (400 MHz, $CDCl_3$) δ 7.15-7.06 (m, 1H), 6.57-6.45 (m, 3H), 6.36-6.27 (m, 1H), 4.16-4.05 (m, 2H), 3.85 (t, *J* = 4.9 Hz, 2H), 3.80-3.63 (m, 15H), 3.49-3.34 (m, 5H), 3.17 (m, 1H), 3.03-2.94 (m, 1H), 2.88-2.80 (m, 1H), 2.78-2.66 (m, 4H), 2.59-2.41 (m, 3H), 2.34 (m, 1H), 2.19-2.01 (m, 2H), 1.97-1.70 (m, 5H), 1.68-1.52 (m, 1H), 1.50-1.38 (m, 1H), 0.96 (ddd, *J* = 1.5, 4.6, 9.0 Hz, 2H), 0.75-0.64 (m, 2H).

Synthesis of Compounds **49, 71, 84**

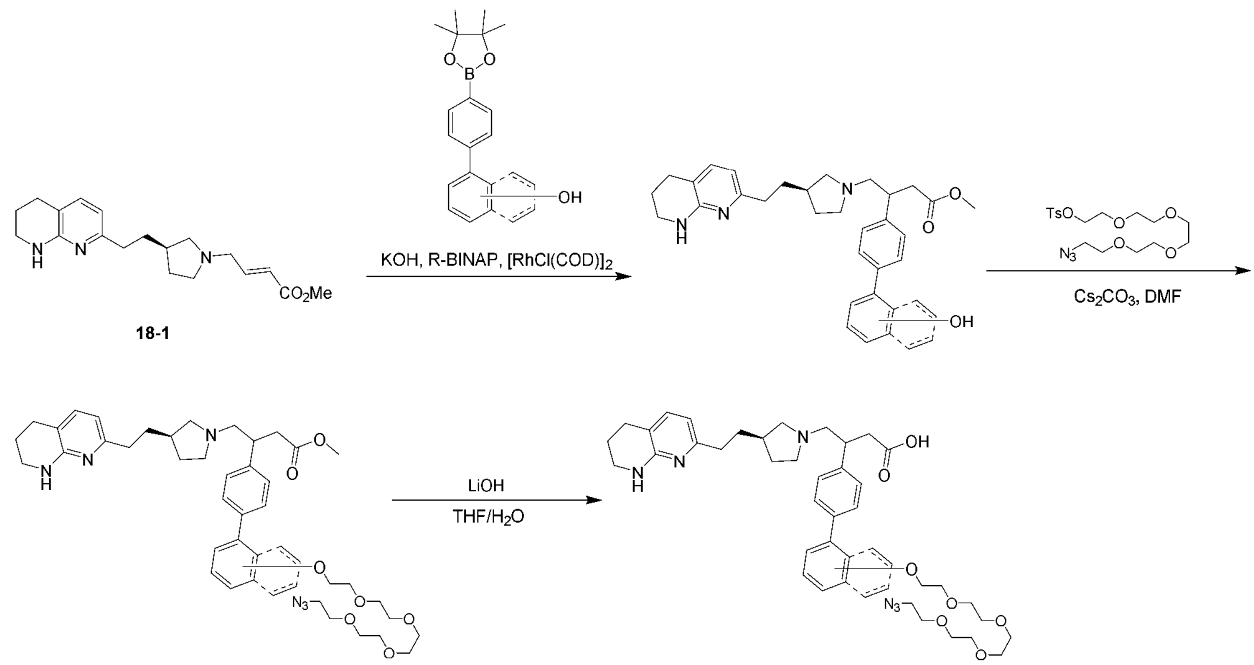


**18-1**

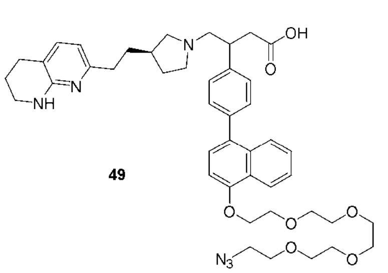

**49**

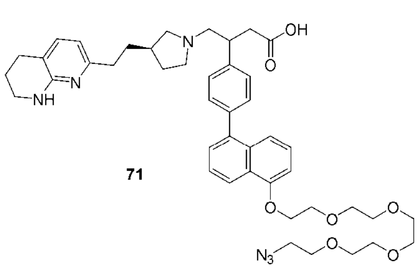

**71**

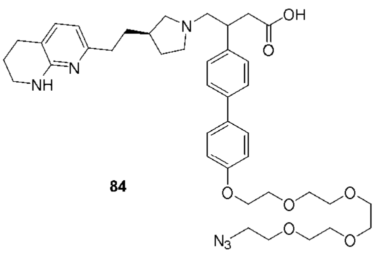

**84**

Compound **49:** Yellow oily compound, 98.4% purity, SFC: ee%: 78.58%. MS *m/z* (ESI): 781.3 $[M+H]^+$; $^1H$ NMR (400 MHz, DMSO-$d_6$) δ 8.31-8.24 (m, 1H), 8.17 (s, 1H), 7.78 (d, *J* = 7.8 Hz, 1H), 7.53 (t, *J* = 7.4 Hz, 2H), 7.38 (s, 3H), 7.34 (d,

*J* = 7.9 Hz, 1H), 7.05 (t, *J* = 7.4 Hz, 2H), 6.40 (s, 1H), 6.27 (d, *J* = 7.3 Hz, 1H), 4.40-4.28 (m, 2H), 3.96-3.89 (m, 2H), 3.72-3.68 (m, 2H), 3.63-3.49 (m, 13H), 3.40-3.31 (m, 4H), 3.27-3.21 (m, 2H), 2.98 (s, 1H), 2.92-2.84 (m, 2H), 2.83-2.76 (m, 1H), 2.71-2.57 (m, 4H), 2.43 (t, *J* = 7.9 Hz, 2H), 2.13-2.02 (m, 1H), 2.01-1.90 (m, 1H), 1.79-1.71 (m, 2H), 1.70-1.57 (m, 2H), 1.50-1.33 (m, 2H).

Compound **71:** Yellow oily compound, 99.2% purity, SFC: ee%: 54.01%. MS *m/z* (ESI): 781.4 $[M+H]^+$; $^1H$ NMR (400 MHz, $CDCl_3$) δ 8.34 (d, *J* = 8.4 Hz, 1H), 7.56-7.48 (m, 2H), 7.47-7.39 (m, 3H), 7.29 (s, 3H), 7.17-7.07 (m, 1H), 6.86 (d, *J* = 7.5 Hz, 1H), 6.36-6.27 (m, 1H), 4.35 (t, *J*= 4.9 Hz, 2H), 4.04 (t, *J* = 4.9 Hz, 2H), 3.89-3.81 (m, 2H), 3.79-3.52 (m, 13H), 3.50-3.35 (m, 5H), 3.33-2.44 (m, 11H), 2.41-1.39 (m, 8H).

Compound **84:** Yellow oily compound, 97.8% purity, SFC: ee%: 24.20%. MS *m/z* (ESI): 731.4 $[M+H]^+$; $^1H$ NMR (400 MHz, $CDCl_3$) δ 7.58-7.45 (m, 4H), 7.28-7.20 (m, 2H), 7.16-7.06 (m, 1H), 7.03-6.95 (m, 2H), 6.30 (t, *J* = 7.0 Hz, 1H), 4.23-4.13 (m, 2H), 3.95-3.86 (m, 2H), 3.79-3.74 (m, 2H), 3.64 (s, 12H), 3.58-2.64 (m, 14H), 2.61-2.25 (m, 1H), 2.61-1.36 (m, 10H).

Synthesis of Compound **85**

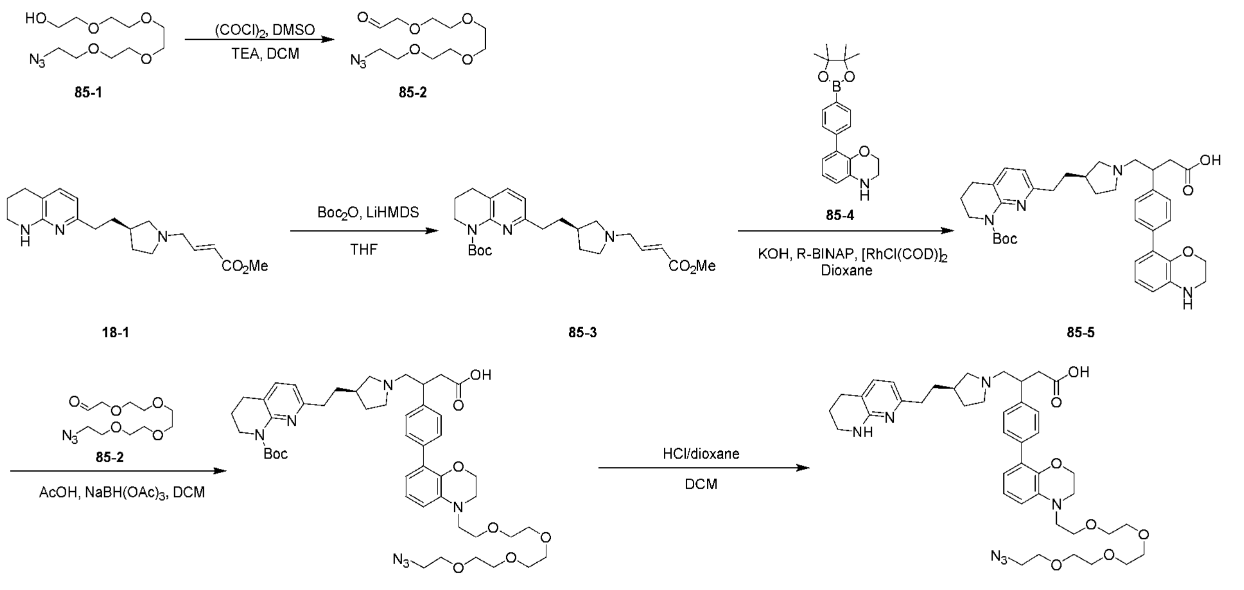


Synthesis of Intermediate **85-2**

At 25 °C, oxalyl chloride (289.3 mg, 2.28 mmol) was dissolved in dichloromethane (6 mL), cooled to -65 °C, then dimethyl sulfoxide (534.2 mg, 6.84 mmol) was added dropwise to the reaction solution. After addition, the mixture was stirred at -65 °C for 30 minutes. Then Compound **85-1** (500.0 mg, 1.90 mmol) was added to the reaction solution, and stirred at -65 °C for 30 minutes. Triethylamine (1.38 g, 13.7 mmol) was added, stirred at -65 °C for 20 minutes, then warmed to 15 °C and reacted for 2 hours. After completion of the reaction, the mixture was diluted with water (10 mL) and extracted with dichloromethane (6 mL × 3). The organic phases were combined, dried over anhydrous sodium sulfate, filtered, and evaporated to dryness to obtain crude product as a colorless oily Compound **85-2** (400.0 mg). The crude product was used directly in the next step. $^1H$ NMR (400 MHz, $CDCl_3$) δ 9.72 (s, 1H), 4.15 (d, *J* = 0.7 Hz, 2H), 3.74-3.64 (m, 14H), 3.38 (t, *J*= 5.1 Hz, 2H).

Synthesis of Intermediate **85-3**

At 25 °C, Compound **18-1** (4.00 g, 12.1 mmol) was dissolved in tetrahydrofuran (100 mL), then di-tert-butyl dicarbonate (3.44 g, 15.8 mmol) was added at 25 °C. Under nitrogen protection, the mixture was cooled to 0 °C, and lithium bis(trimethylsilyl)amide (14.6 mL, 1 M, 14.6 mmol) was slowly added dropwise to the reaction solution. Stirred at 0 °C for 0.5 hour. After completion of the reaction, the mixture was diluted with saturated ammonium chloride solution (150 mL) and extracted with ethyl acetate (100 mL × 3). The organic phases were combined, washed with saturated brine, dried over anhydrous sodium sulfate, filtered, and evaporated to dryness. The residue was purified by flash silica gel chromatography

(eluent 0 ~ 50% tetrahydrofuran/ethyl acetate) to obtain yellow oily Compound **85-3** (2.20 g, 42.18% yield). $^1$H NMR (400 MHz, $CDCl_3$) δ 7.30-7.26 (m, 1H), 6.98 (td, *J*= 6.1, 15.7 Hz, 1H), 6.78 (d, *J* = 7.6 Hz, 1H), 5.98 (td, *J* = 1.6, 15.6 Hz, 1H), 3.81-3.68 (m, 5H), 3.31-3.14 (m, 2H), 2.85 (t, *J* = 7.7 Hz, 1H), 2.76-2.63 (m, 5H), 2.51-2.41 (m, 1H), 2.28-2.12 (m, 2H), 2.09-1.98 (m, 1H), 1.90 (quin, *J* = 6.4 Hz, 2H), 1.80 (q, *J* = 7.6 Hz, 2H), 1.43-1.58 (m, 10H).

Synthesis of Intermediate **85-5**

At 25 °C, Compound **85-3** (400.0 mg, 1.21 mmol) was dissolved in dioxane (12 mL), and Compound **85-4** (420.0 mg, 1.25 mmol) and potassium hydroxide (156.8 mg, 2.79 mmol) were added. Under nitrogen protection, (R)-(+)-2,2-bis(diphenylphosphino)-1,1-binaphthyl (58.0 mg, 93.1 μmol) and (1,5-cyclooctadiene)chlororhodium(I) dimer (23.0 mg, 46.6 μmol) were added. The mixture was stirred at 80 °C under nitrogen protection for 4 hours. After completion of the reaction, the mixture was diluted with water (20 mL) and extracted with ethyl acetate (15 mL × 3). The organic phases were combined, dried over anhydrous sodium sulfate, filtered, and evaporated to dryness. Combined with other batches, the residue was purified by flash silica gel chromatography (eluent 0 ~ 50% tetrahydrofuran/petroleum ether, then changed to 0~5% methanol/dichloromethane) to obtain black-yellow oily Compound **85-5** (220.0 mg). The crude product was used directly in the next step. MS *m*/*z* (ESI): 627.6 [M+H]$^+$.

Synthesis of Intermediate **85-7**

At 25 °C, Compound **85-5** (200.0 mg) was dissolved in dichloromethane (4 mL), and Compound **85-2** (82.0 mg, 313.98 μmol) and acetic acid (9.20 mg, 153.16 μmol) were added. The mixture was cooled to 0 °C and stirred for 30 minutes. Sodium triacetoxyborohydride (48.7 mg, 229.7 μmol) was added. The mixture was stirred at 25 °C for 6 hours. After completion of the reaction, the mixture was diluted with water (10 mL) and extracted with dichloromethane (6 mL × 2). The organic phases were combined, dried over anhydrous sodium sulfate, filtered, and evaporated to dryness. Combined with other batches, the residue was purified by flash silica gel chromatography (eluent 0 ~ 20% tetrahydrofuran/petroleum ether, then changed to 0~5% methanol/dichloromethane) to obtain yellow oily Compound **85-7** (160.0 mg). MS *m*/*z* (ESI): 872.6 [M+H]$^+$.

Synthesis of Compound **85**

At 25 °C, Compound **85-7** (400.0 mg) was dissolved in dichloromethane (2 mL), then hydrochloric acid/dioxane (4M, 10 mL) was added at 25 °C. The mixture was stirred at 25 °C for 2 hours. After completion of the reaction, the mixture was filtered and evaporated to dryness. The residue was purified by high performance liquid chromatography (Column: C18 150*40 mm), Mobile phase: [Water (formic acid) - Acetonitrile], Gradient: 7% ~ 47% B over 9 min), and lyophilized to obtain white solid Compound **85** (36.0 mg, 42.48% yield, 96.9 purity, SFC: ee% = 55.06%). MS m/z (ESI): 772.3 [M+H]$^+$; $^1$H NMR (400 MHz, $CDCl_3$) δ 10.37 (s, 1H), 8.52 (s, 1H), 7.47 (d, *J*= 8.2 Hz, 2H), 7.25-7.21 (m, 1H), 7.20-7.13 (m, 2H), 6.88-6.82 (m, 1H), 6.67 (d, *J*= 7.6 Hz, 1H), 6.62 (dd, *J* = 1.3, 7.6 Hz, 1H), 6.28 (d, *J* = 7.2 Hz, 1H), 4.25-4.14 (m, 2H), 3.73-3.69 (m, 2H), 3.68-3.59 (m, 14H), 3.51 (t, *J* = 5.9 Hz, 2H), 3.46 (t, *J* = 4.5 Hz, 4H), 3.37 (t, *J* = 5.0 Hz, 3H), 3.34-3.10 (m, 3H), 3.04 (s, 2H), 2.97-2.85 (m, 3H), 2.76-2.61 (m, 4H), 2.41-2.29 (m, 1H), 2.25-2.13 (m, 1H), 1.95-1.74 (m, 4H), 1.71-1.59 (m, 1H).

**Synthesis of 5FAM-Labeled Compounds**

General Synthetic Method:

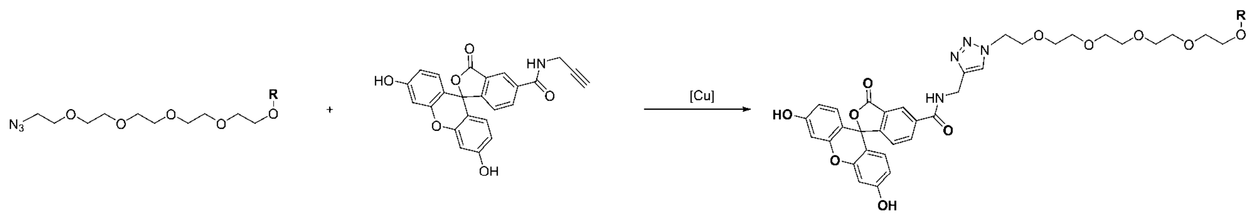


Compound Propargylamine-5FAM Compound-FAM

**Propargylamine-5FAM** (1.1 equiv.) was dissolved in a mixture of isopropanol and water (1:1). The **azido compound** (1.0 equiv.), $CuSO_4$ (3.0 equiv.) and ascorbic acid (4.0 equiv.) were added sequentially. The mixture was

stirred at room temperature for 2 hours. LCMS detected reaction completion. The mixture was filtered, and the filtrate was purified by preparative chromatography column to obtain the **SFAM-labeled compound** as a yellow solid.

**1-5FAM:** LCMS: (ESI) *m/z* = 600.6 $[M+2H]/2^+$; 400.7 $[M+3H]/3^+$
HPLC: 97.50% (214 nm), RT = 17.80 min. Mobile Phase: A: Water (0.05% TFA) B: ACN (0.05% TFA). Gradient: 20% B hold for 3 minutes, increase to 80% B over 20 minutes, then to 95% B over 2 minutes, hold for 5 minutes, decrease to 20% B over 0.1 minutes. Flow Rate: 1.0 mL/min. Column: XBridge peptide BEH column C18, 4.6 x 150 mm, 3.5 μm, 130 Å. Column Temperature: 40 °C.

**ING-5FAM:**

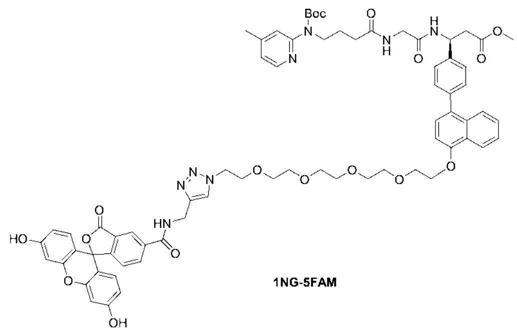

1NG-5FAM

LCMS: (ESI) *m/z* = 657.3 [M+2H]/2+
HPLC: 97.06% (214 nm), RT = 15.98 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 x 150 mm, 3.5 μm, 130 Å. Column Temperature: 40 °C.

**1-3-5FAM:**

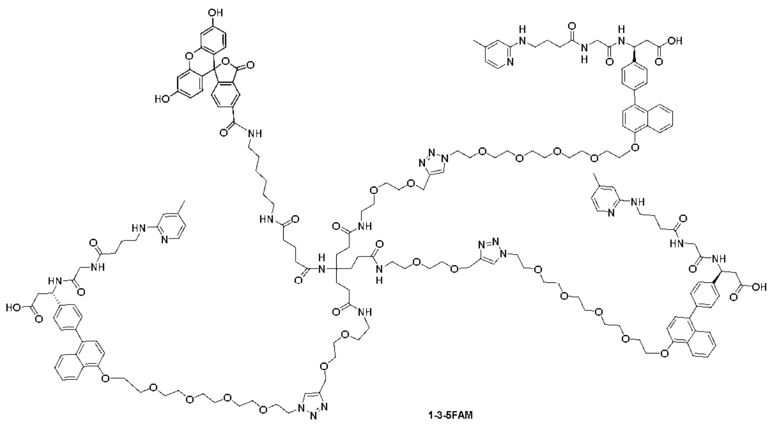

1-3-5FAM

LCMS: (ESI) m/z = 1184.3 $[M+3H]/3^+$; 888.7 $[M4+H]/4^+$; 711.3 $[M+5H]/5^+$
HPLC: 83.92 % (214 nm), RT = 13.95 min. Mobile Phase: A: Water (0.05% TFA) B: ACN (0.05% TFA). Gradient: 20% B hold for 3 minutes, increase to 80% B over 20 minutes, then to 95% B over 2 minutes, hold for 5 minutes, decrease to 20% B over 0.1 minutes. Flow Rate: 1.0 mL/min. Column: XBridge peptide BEH column C18, 4.6 × 150 mm, 3.5 μm, 130 Å. Column Temperature: 40 °C.
**4-5FAM:** LCMS: (ESI) *m/z* = 1212.7 $[M+H]^+$

Preparation Conditions:

Column: Gemini C18 21.2 mm × 250 mm, 10 μm, 110 Å; Mobile Phase: A: Water (containing 10 mmol/L

$NH_4HCO_3$) B: ACN; Gradient: 5% B to 65% B over 20 minutes. HPLC: 97.08% (214 nm), RT = 16.53 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 40 °C.
**5-5FAM:** LCMS: (ESI) *m*/*z* = 613.5 $[M+2H]/2^+$

Preparation Conditions:

Column: Gemini C18 21.2 mm × 250 mm, 10 μm, 110 Å; Mobile Phase: A: Water (containing 10 mmol/L $NH_4HCO_3$) B: ACN; Gradient: 5% B to 65% B over 20 minutes. HPLC: 95.26% (214 nm), RT = 18.53 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 40 °C.
**6-5FAM:** LCMS: (ESI) *m*/*z* = 586.4 [M+2H]/2+

Preparation Conditions:

Column: Gemini C18 21.2 mm × 250 mm, 10 μm, 110 Å; Mobile Phase: A: Water (containing 10 mmol/L $NH_4HCO_3$) B: ACN; Gradient: 5% B to 65% B over 20 minutes. HPLC: 96.93% (214 nm), RT = 17.53 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 40 °C.
**7-5FAM:** LCMS: (ESI) *m*/*z* = 1156.5 $[M+H]^+$; 579.0 $[M+2H]/2^+$

Preparation Conditions:

Reverse Phase Column: Gemini C18 21.2 mm × 250 mm, 10 μm, 110 Å; Mobile Phase: A: Water (containing 10 mmol/L $NH_4HCO_3$), B: Acetonitrile; Gradient: 5% B to 65% B over 20 minutes. HPLC: 95.99 % (214 nm), RT = 18.55 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 40 °C.
**8-5FAM:** LCMS: (ESI) m/z = 1182.5 $[M+H]^+$; 591.5 $[M+2H]/2^+$

Preparation Conditions:

Reverse Phase Column: Gemini C18 21.2 mm × 250 mm, 10 μm, 110 Å; Mobile Phase: A: Water (containing 10 mmol/L $NH_4HCO_3$), B: Acetonitrile; Gradient: 5% B to 65% B over 20 minutes. HPLC: 96.34 % (214 nm), RT = 18.16 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 40 °C.
**16-5FMA:** LCMS: (ESI) m/z = 569.2 $[M+2H]/2^+$

Preparation Conditions:

Column: Gemini C18, 19 × 250 mm, 10 μm, 130 Å; Mobile Phase: A: Water (containing 10 mmol/L $NH_4HCO_3$) B: ACN; Gradient: 5% B to 65% B over 20 minutes. HPLC: 93.18% (214 nm), RT = 13.89 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 40 °C.
**17-5FAM:** LCMS: (ESI) *m*/*z* = 584.2 $[M+2H]/2^+$

Preparation Conditions:

Column: Gemini C18, 19 × 250 mm, 10 μm, 130 Å; Mobile Phase: A: Water (containing 10 mmol/L $NH_4HCO_3$) B: ACN; Gradient: 5% B to 65% B over 20 minutes. HPLC: 94.10% (214 nm), RT = 14.49 min. Mobile Phase: A: Water (0.05% TFA) B: ACN (0.05% TFA). Gradient: 5% B hold for 3 minutes, increase to 45% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 40 °C.

**18-5FAM:** LCMS: (ESI) $m/z$ = 535.0 $[M+2H]/2^+$

Preparation Conditions:

Column: Gemini C18 21.2 mm × 250 mm, 10 μm, 110 Å; Mobile Phase: A: Water (containing 10 mmol/L $NH_4HCO_3$) B: ACN; Gradient: 5% B to 65% B over 20 minutes. HPLC: 95.83% (214 nm), RT = 15.58 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 25 °C.

**20-5FAM:** LCMS: (ESI) $m/z$ = 535.0 $[M+2H]/2^+$

Preparation Conditions:

Column: Gemini C18 21.2 mm × 250 mm, 10 μm, 110 Å; Mobile Phase: A: Water (containing 10 mmol/L $NH_4HCO_3$) B: ACN; Gradient: 5% B to 65% B over 20 minutes. HPLC: 94.50% (214 nm), RT = 15.46 min. Mobile Phase: A: Water (0.05% TFA) B: ACN (0.05% TFA). Gradient: 5% B hold for 3 minutes, increase to 65% B over 20 minutes, then to 95% B over 2 minutes, hold for 5 minutes, decrease to 5% B over 0.1 minutes. Flow Rate: 1.0 mL/min. Column: XBridge peptide BEH column C18, 4.6 × 150 mm, 3.5 μm, 130 Å. Column Temperature: 40 °C.

Synthesis of **9-5FAM** Labeled Compound

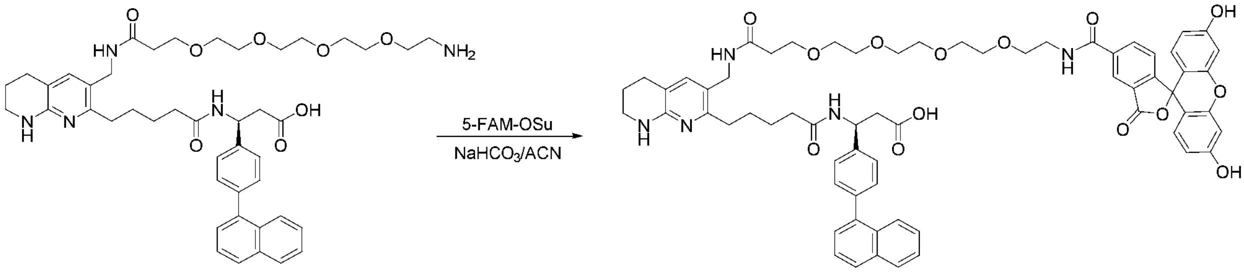


9-15 9-5FAM

Compound **9-15** (90 mg, 0.065 mmol, 1.0 equiv.) was dissolved in a mixture of water (2 mL) and acetonitrile (1 mL). **5-FAM-OSu** (31 mg, 0.065 mmol) and saturated $NaHCO_3$ solution (1 mL) were added sequentially. The reaction solution was stirred at room temperature in the dark for 2 hours. The reaction solution was directly purified by preparative reverse phase chromatography column (Gemini C18 21.2 mm × 250 mm, 10 μm, 110 Å; Mobile phase: A: Water (containing 10 mmol/L $NH_4HCO_3$), B: Acetonitrile; Gradient: 26% B to 36% B over 20 minutes) to obtain **9-5FAM** (22 mg, yield: 30%) as a yellow solid. LCMS: (ESI) $m/z$ = 1142.5 $[M+H]^+$; 572.2 $[M+2H]/2^+$. HPLC: 99.13 % (214 nm), RT = 15.00 min.

Preparation Conditions:

Mobile Phase: A: Water (0.05% TFA) B: ACN (0.05% TFA). Gradient: 20% B hold for 3 minutes, increase to 80% B over 20 minutes, then to 95% B over 2 minutes, hold for 5 minutes, decrease to 20% B over 0.1 minutes. Flow Rate: 1.0 mL/min. Column: XBridge peptide BEH column C18, 4.6 × 150 mm, 3.5 μm, 130 Å. Column Temperature: 40 °C.

**Example 2. Synthesis of Oligonucleotides**

The synthesis method of oligonucleotides was identical to standard phosphoramidite solid-phase synthesis (all synthesized by Suzhou Biosyntech Co., Ltd.), comprising four iterative steps: deprotection, coupling, capping, and oxidation/sulfurization. Synthesis initiated from the solid support, with nucleotides sequentially added in the 3'→5' direction using commercially available phosphoramidite monomers (2'-F RNA, 2'-OMe RNA; Shanghai Hongene Biotechnology Co., Ltd.). Upon completion of solid-phase synthesis, the solid support was transferred to a centrifuge tube and incubated with 28% $NH_4OH$ in ethanol/$H_2O$ (3:1 v/v) at 50°C for 16 hours to cleave the oligonucleotide from the solid support into solution. The supernatant was centrifuged and transferred to a new tube, concentrated to dryness, and redissolved in deionized water. Purification was performed using a C18 reversed-phase column with a mobile phase of 0.1

M TEAA and acetonitrile. The target oligonucleotide fraction was collected, lyophilized, identified as the desired product by LC-MS, and quantified by UV spectroscopy (260 nm). The obtained sense and antisense strands were annealed at an equimolar ratio to form the complementary double-stranded siRNA (dsRNA), which was subsequently adjusted to the desired concentration.

### Example 3. Conjugation Reaction of Small Molecule Ligand Compounds and Oligonucleotides

The synthesized oligonucleotide containing tri-alkynes (1.0 equiv.) was dissolved in a buffer salt solution/DMSO (3:1). The azide-modified small molecule ligand (15 equiv.) dissolved in DMSO was added. After vortexing, pre-prepared THPTA: $CuSO_4.5H_2O$ = 5:1 (equivalents of 15 equiv. and 3 equiv., respectively) was added. After vortexing, sodium ascorbate (25 equiv.) was quickly added. The reaction system was continued to react at 40 °C for 2 h. LCMS monitored complete reaction, and then HPLC reverse phase purification was performed to obtain the desired oligonucleotide. The target oligonucleotide was collected, lyophilized, identified as the target product by LC-MS, and then quantified by UV (260 nm). The obtained sense strand and antisense strand were annealed according to the molar ratio to obtain complementary paired double-stranded siRNA, and the concentration was adjusted as needed.

**Table 1. siRNA Compounds Targeting Lung Tissue RAGE-mRNA**

| Duplex | | (5'-3') | Calc MW | Found MW | SEQ ID NO. |
|---|---|---|---|---|---|
| **D0** | SS | (**TA**)mC*mGmGmAmAmUmGmGfAfAfAmCmUmGmAmAmCmAmCmAmA*IB (SEQ ID NO. 1) | 8157.9 | 8159.1 | 1 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| **D1** | SS | **Tri-L1-** (**TTA**)mC*mGmGmAmAmUmGmGfAfAfAmCmUmGmAmAmCmAmCmAmA*IB (SEQ ID NO. 1) | 10515.6 | 10516.9 | 3 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| **D2** | SS | **Tri-L6-** (**TTA**)mC*mGmGmAmAmUmGmGfAfAfAmCmUmGmAmAmCmAmCmAmA*IB (SEQ ID NO. 1) | 10431.4 | 10432.9 | 4 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| **D3** | SS | **Tri-L8-** (**TTA**)mC*mGmGmAmAmUmGmGfAfAfAmCmUmGmAmAmCmAmCmAmA*IB (SEQ ID NO. 1) | 10464.6 | 10465.8 | 5 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| **D4** | SS | **Tri-L9-** (**TTA**)mC*mGmGmAmAmUmGmGfAfAfAmCmUmGmAmAmCmAmCmAmA*IB (SEQ ID NO. 1) | 10587.8 | 10589.6 | 6 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| **D5** | SS | **Tri-L13p1-** (**TTA**)mC*mGmGmAmAmUmGmGfAfAfAmCmUmGmAmAmCmAmCmAmA*IB (SEQ ID NO. 1) | 10527.8 | 10528.6 | 7 |
| | AS | VPmU*fU*mG*fUmGfUmUfCmAfGmUfUmUfCmCfAmUfUmCfC*mG (SEQ ID NO. 2) | 6879.3 | 6879.9 | 2 |

(continued)

| Duplex | | (5'-3') | Calc MW | Found MW | SEQ ID NO. |
|---|---|---|---|---|---|
| D6 | SS | **Tri-L14p2-** (**TTA**)mC*mGmGmAmAmUmGmGfAfAfAmCmUmGmAmAmCmAmCmAmA*IB (SEQ ID NO. 1) | 10404.7 | 10405.6 | 8 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| D7 | SS | **Tri-L18p1-** (**TTA**)mC*mGmGmAmAmUmGmGfAfAfAmCmUmGmAmAmCmAmCmAmA*IB (SEQ ID NO. 1) | 10122.3 | 10122.9 | 9 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| D8 | SS | **Tri-L20p1-** (**TTA**)mC*mGmGmAmAmUmGmGfAfAfAmCmUmGmAmAmCmAmCmAmA*IB (SEQ ID NO. 1) | 10122.3 | 10122.4 | 10 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| D9 | SS | **Tri-L5p1-** (**TTA**)mC*mGmGmAmAmUmGmGfAfAfAmCmUmGmAmAmCmAmCmAmA*IB (SEQ ID NO. 1) | 10593.7 | 10595.1 | 11 |
| | AS | VPmU*fU*mG*fUmGfUmUfCmAfGmUfUmUfCmCfAmUfUmCfC*mG (SEQ ID NO. 2) | 6879.3 | 6879.9 | 2 |
| D10 | SS | **Tri-L5p2-** (**TTA**)mC*mGmGmAmAmUmGmGfAfAfAmCmUmGmAmAmCmAmCmAmA*IB (SEQ ID NO. 1) | 10593.7 | 10595.3 | 12 |
| | AS | VPmU*fU*mG*fUmGfUmUfCmAfGmUfUmUfCmCfAmUfUmCfC*mG (SEQ ID NO. 2) | 6879.3 | 6879.9 | 2 |

Where m represents 2'-O-methyl modification, f represents 2'-fluoro modification, * represents phosphorothioate modification.

**Tri-alkyne (TA):**

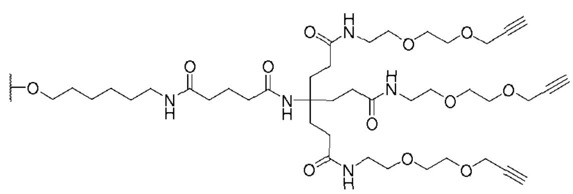

**Tri-L1-(TTA):**

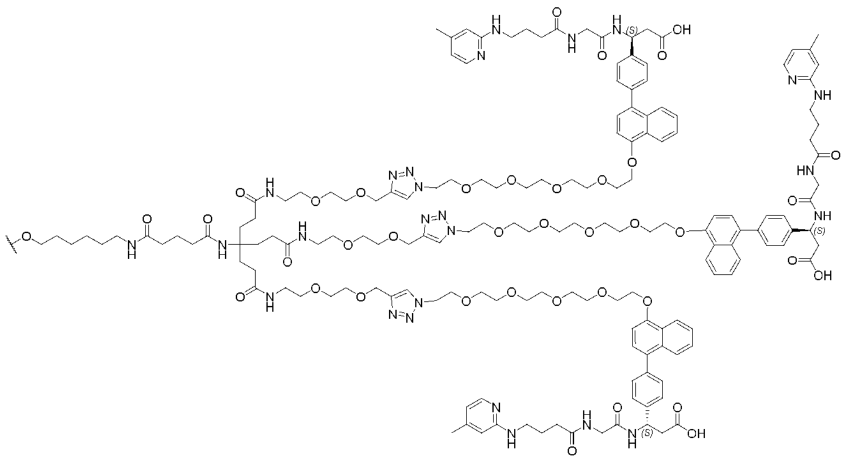

**Tri-L6-(TTA):**

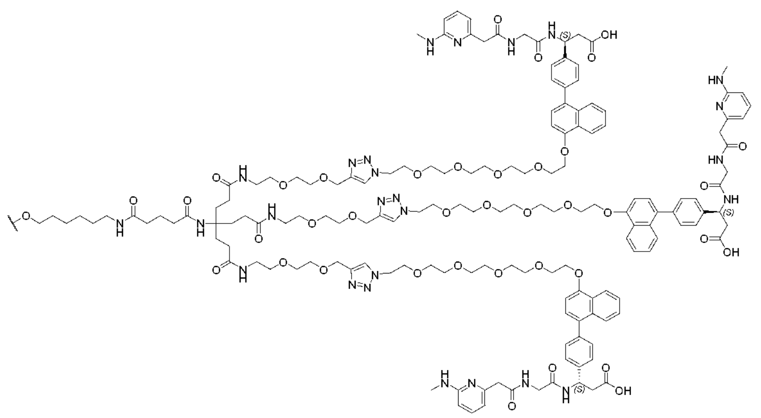

**Tri-L8-(TTA):**

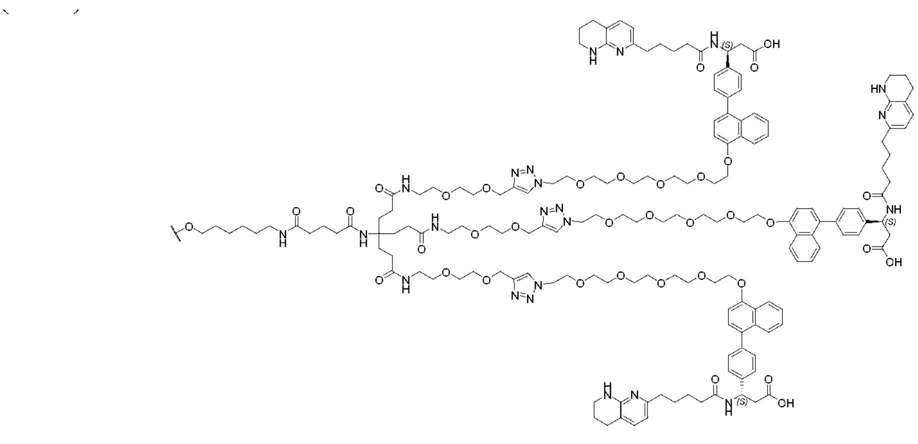

**Tri-L9-(TTA):**

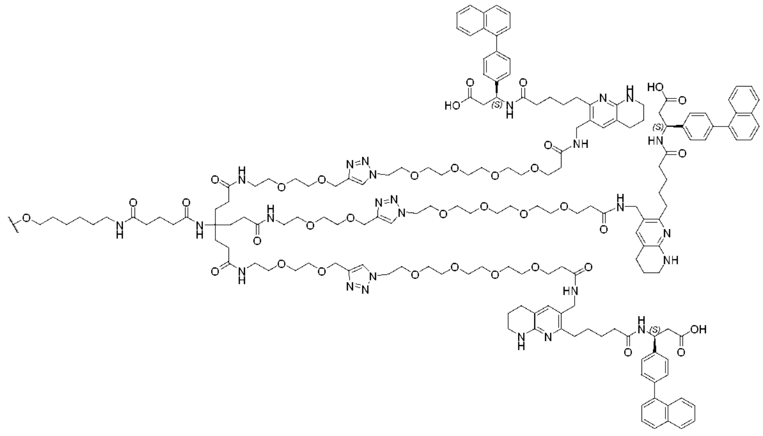

**Tri-L13p1-(TTA):**

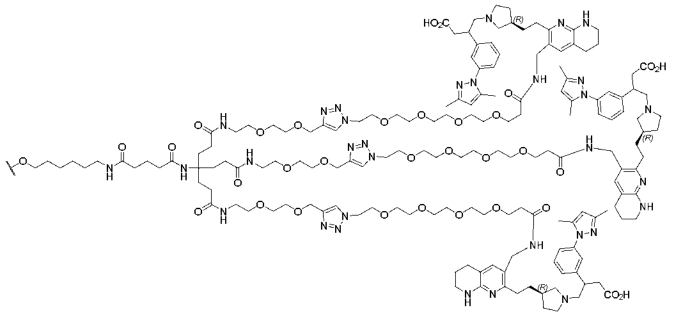

**Tri-L14p2-(TTA):**

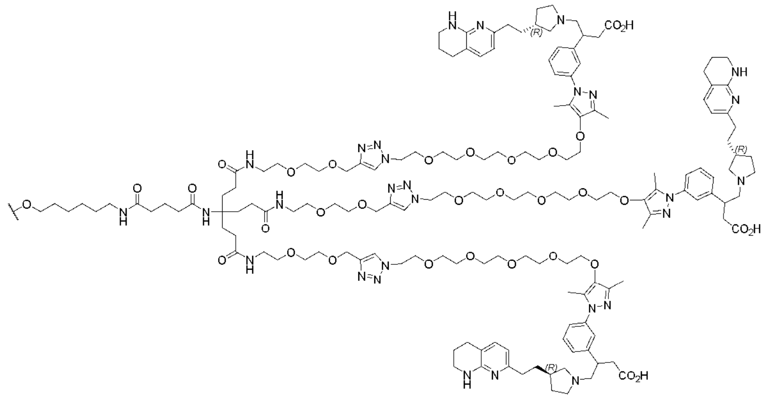

**Tri-L18p1-(TTA):**

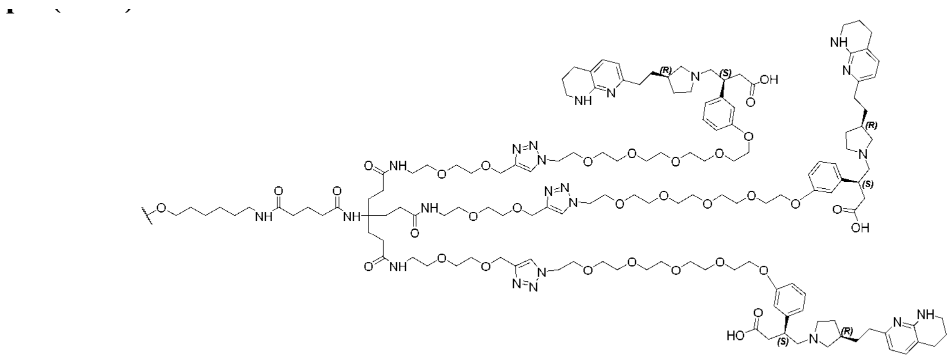

**Tri-L20p1-(TTA):**

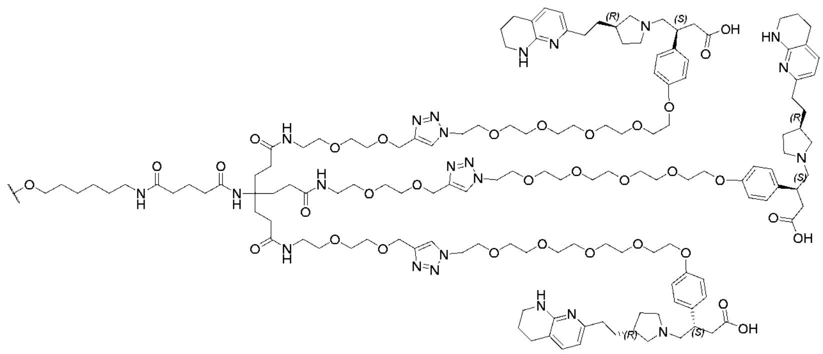

**Tri-L5p1-(TTA), Tri-L5p2-(TTA):**

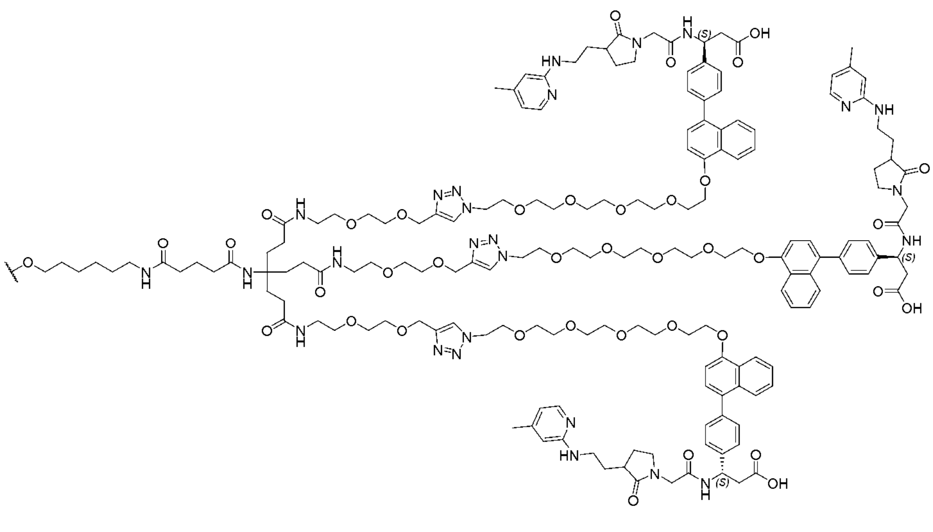

VPmU = 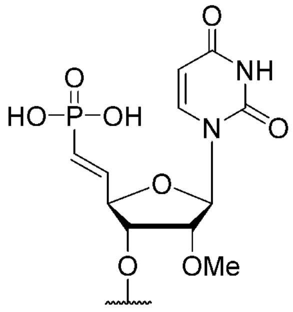 ; IB = 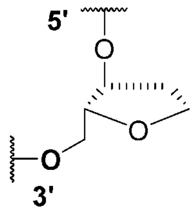

**Example 4. *In Vitro* Evaluation of Small Molecule Ligand Binding Affinity to Integrins**

A. Evaluation of Compound Binding Activity to Integrin αvβ6 Receptor using Homogeneous Time-Resolved Fluorescence (HTRF) Technology

Main Experimental Reagents and Consumables

| Name | Brand | Catalog No. |
|---|---|---|
| Integrin receptor protein αvβ6 | Acrobiosystems | IT6-H52E1 |
| LAP (TGF-beta 1) | Acrobiosystems | LAP-H82F8 |
| Anti-His Tb | Cisbio | 61HI2TLB |
| SA-XL665 | Cisbio | 610SAXLB |
| Cilengitide | MCE | HY-16141 |
| Opti-384 | PerkinElmer | 6007299 |
| ECHO Qualified 384-Well | Labcyte | PP-0200 |

Experimental Method

Compound Preparation: Reference compound (Cilengitide) and test compounds were prepared as 10 mM stock solutions in DMSO and stored. The positive reference compound was set at 10 concentration points, starting dilution concentration of 10 mM, serially diluted 3-fold with DMSO in an ECHO Qualified 384-Well plate (final DMSO concentration in assay 0.5%, final concentration of positive reference compound 2.54-50000 nM). Test compounds were also set at 10 concentration points, starting dilution concentration of 0.02 mM, serially diluted 3-fold with DMSO in an ECHO Qualified 384-Well plate (final DMSO concentration in assay 0.5%, final concentration of positive reference compound 0.005-100 nM).

Reagent Preparation: Assay buffer was prepared. The buffer was used to prepare 40 nM integrin receptor protein αvβ6, 20 nM ligand protein LAP (TGF β1), and detection reagents 1 nM Anti-His Tb and 20 nM SA-XL665 respectively.

Experimental Operation and Detection: First, Echo650® was used to transfer 100 nL each of the serially diluted reference compound (Cilengitide), test compounds, and DMSO solution (positive control and negative control) from the ECHO Qualified 384-Well plate to an Opti-384 plate. Then 5 μL of integrin receptor protein αvβ6 was added (5 μL of assay buffer was added for negative control), and incubated at room temperature for 20 minutes. Next, 5 μL of ligand protein LAP, and 10 μL of the Anti-His Tb and SA-XL665 mixture were added respectively, incubated at room temperature for 1 hour. The above plate was placed on EnVision (PerkinElmer, SN:1051284) for detection, excitation wavelength 340 nm, emission wavelengths 615 nm & 665 nm.

Data Processing

Inhibition rate and fit IC50 was calculated using Excel and XL-Fit respectively, formula as follows:

$$Inhibition \% = (Max-Signal)/(Max-Min)*100$$

$$Y = Bottom + (Top-Bottom)/(1+(IC50/X)*HillSlope),$$

Y and X represent %inhibition and compound concentration respectively

Experimental Results

Table 1. Binding Activity of Compounds to Integrin αvβ6 Receptor

| Compound ID | IC50 (nM) | Hillslope | Min | Max |
|---|---|---|---|---|
| Cilengitide | 141.79 | 1.2 | 11.0 | 98.2 |
| **1-5FAM** | 1.41 | 11.3 | 0.3 | 97.0 |
| **1NG-5FAM** | 41.90 | 2.5 | 2.0 | 100.0 |
| **5FAM** | >100 | -11.5 | -2.8 | 6.3 |
| **1-3-5FAM** | 2.52 | 1.6 | -1.4 | 107.0 |
| **4-5FAM** | 7.23 | 1.1 | 6.6 | 100.0 |
| **5-5FAM** | 2.66 | 1.3 | -3.9 | 106.8 |
| **6-5FAM** | 1.60 | 1.2 | -12.4 | 95.8 |
| **7-5FAM** | 3.17 | 1.2 | -4.0 | 101.8 |
| **8** | 1.37 | 2.5 | -2.0 | 107.0 |
| **9-5FAM** | 1.48 | 2.0 | -2.2 | 103.1 |
| **18-5FAM** | 1.80 | 2.3 | -5.6 | 95.0 |
| **20-5FAM** | 1.37 | 2.7 | -2.8 | 98.0 |
| **16-5FAM** | 1.77 | 1.4 | -5.2 | 100.9 |
| **17-5FAM** | 7.99 | 1.5 | -5.6 | 98.9 |

Table 3. Binding Activity of Compounds to Integrin αvβ6 Receptor

| **Compound** | IC50 (nM) | Compound | IC50 (nM) |
|---|---|---|---|
| **Cilengitide** | 104.6 | **Cilengitide** | 98.2 |
| **1** | 2.5 | **1** | 2.3 |
| **2-P2** | 2.6 | **13** | 1.7 |
| **18-P1** | 3.2 | **14** | 2.8 |
| **18-P2** | 13.9 | **15** | 2.5 |
| **20-P1** | 3.7 | **Cilengitide** | 104.6 |
| **20-P2** | 13.4 | **1** | 2.5 |
| **5-P1** | 3.6 | **2-P2** | 2.5 |
| **5-P2** | 3.9 | **2-P1** | 7.9 |
| **53** | 3.8 | **13** | 2.5 |
| **57** | 3.3 | **13-P1** | 4.5 |
| **58** | 3.2 | **14** | 4.9 |
| **59** | 2.9 | **14-P2** | 3.5 |

(continued)

| **Compound** | IC50 (nM) | Compound | IC50 (nM) |
|---|---|---|---|
| **83** | 3.7 | **14-P1** | 9.4 |
| **60** | 2.8 | | |
| **49** | 3.1 | | |
| **84** | 3.8 | | |
| **71** | 3.0 | | |
| **75** | 3.1 | | |
| **86** | 4.5 | | |

The results showed that all the ligand small molecules exhibited excellent *in vitro* binding ability to the αvβ6 receptor.

B. Evaluation of Compound Binding Activity to Integrin αvβ5 Receptor using Homogeneous Time-Resolved Fluorescence (HTRF) Technology

Main Experimental Reagents and Consumables

| Name | Brand | Catalog No. |
|---|---|---|
| Integrin receptor protein αvβ5 | Acrobiosystems | IT5-H52W5 |
| Vitronectin | Acrobiosystems | VIN-H8113 |
| SA-Tb | Cisbio | 610SATLB |
| Anti-His XL665 | Cisbio | 61HISXLA |
| Cilengitide | MCE | HY-16141 |
| Opti-384 | PerkinElmer | 6007299 |
| ECHO Qualified 384-Well | Labcyte | PP-0200 |

Experimental Method

Compound Preparation: Same as A.

Reagent Preparation: Assay buffer was prepared. The buffer was used to prepare 20 nM integrin receptor protein αvβ5, 40 nM ligand protein Vitronectin (VTN), and detection reagents 1 nM SA-Tb and 14 nM Anti-His XL665 respectively.

Experimental Operation and Detection: First, Echo650® was used to transfer 200 nL each of the serially diluted reference compound (Cilengitide), test compounds, and DMSO solution (positive control and negative control) from the ECHO Qualified 384-Well plate to an Opti-384 plate. Then 5 μL of integrin receptor protein αvβ5 was added (5 μL of assay buffer was added for negative control), incubated at room temperature for 20 minutes. Next, 5 μL of ligand protein VTN, and 10 μL of the SA-Tb and Anti-His XL665 mixture were added respectively, incubated at room temperature for 1 hour. The above plate was placed on EnVision (PerkinElmer, SN:1051284) for detection, excitation wavelength 340 nm, emission wavelengths 615 nm & 665 nm.

Data Processing: Same as A
Experimental Results are shown in Table 4

C. Evaluation of Compound Binding Activity to Integrin αvβ1 Receptor using Fluorescence Polarization (FP) Technology

Main Experimental Reagents and Consumables

| Name | Brand | Catalog No. |
|---|---|---|
| Integrin receptor protein αvβ1 | Acrobiosystems | IT1-H52E1 |
| Cyclo[-RGDy-K(5-FAM)] | Anaspec | AS-65160 |
| Cilengitide | MCE | HY-16141 |
| Opti-3 84F | PerkinElmer | 6007279 |
| ECHO Qualified 384-Well | Labcyte | PP-0200 |

Experimental Method

Compound Preparation: Same as A.

Reagent Preparation: Assay buffer was prepared. The buffer was used to prepare 20 nM integrin receptor protein αvβ1, 10 nM ligand peptide Cyclo[-RGDy-K(5-FAM)] respectively.

Experimental Operation and Detection: First, Echo650® was used to transfer 200 nL each of the serially diluted reference compound (Cilengitide), test compounds, and DMSO solution (positive control and negative control) from the ECHO Qualified 384-Well plate to an Opti-384 plate. Then 10 μL of integrin receptor protein αvβ1 was added (10 μL of assay buffer was added for negative control), incubated at room temperature for 20 minutes. 10 μL of ligand peptide Cyclo [-RGDy-K(5-FAM)] was added, incubated at room temperature for 1 hour. The above plate was placed on EnVision (PerkinElmer, SN:1051284) for detection, excitation wavelength 480 nm, emission wavelengths 535nm(S) & 535nm(P).

Data Processing: Same as A
Experimental Results are shown in Table 4

Table 4. Binding Activity of Compounds to Different Integrin Receptors

| Compound | IC50 (nM) | | |
|---|---|---|---|
| | αvβ6 | αvβ1 | αvβ5 |
| **Cilengitide** | 104.6 | 10.4 | 2.7 |
| **1** | 2.5 | 9.7 | 3.4 |
| **2-P2** | 2.6 | 5.4 | 0.7 |
| **5-P1** | 3.6 | 753.1 | 83.4 |
| **5-P2** | 3.9 | 333.1 | 142.1 |
| **13-P1** | 3.6 | 5.3 | 0.8 |
| **14-P2** | 3.9 | 10.3 | 0.7 |
| **18-P1** | 3.2 | 9.1 | 0.6 |
| **20-P1** | 3.7 | 8.1 | 0.7 |

The results indicated that the ligand small molecules **5-P1** and **5-P2** exhibited specific binding ability to the αvβ6 receptor.

**Example 5. *In Vivo* RAGE Target Screening of Small Molecule Ligand Delivery in Rats**

Male SD rats, 8 per group, were administered a single intratracheal dose on Day 0 (compounds **D1, D4, D5, D6, D8, D9,** and **D10).** Compound dose 2 mg/kg, preparation concentration set at 2 mg/mL, dosing volume 1 mL/kg. On Days -4, 14, and 28, rats were anesthetized with 1-4% isoflurane, blood was collected from the orbit (non-fasting), and approximately 150 μL of serum was collected (whole blood samples were allowed to stand at room temperature for 30

minutes, then centrifuged at 4 °C, 2000g, for 10 minutes to obtain serum), temporarily stored in a -80 °C freezer. It was used for ELISA detection of sRAGE, single well detection. Lungs were collected for RAGE mRNA detection.

The results showed that all tested compounds of the present invention could effectively inhibit RAGE-mRNA (Figure 1) and protein (Figure 2) expression in rats. Among them, **D9** (ligand small molecule **5-p1), D10** (ligand small molecule **5-p2)** and **D4** (ligand small molecule **9)** showed comparable inhibition of sRAGE protein to **D1** (ligand small molecule **1).**

## Claims

1. A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ia:

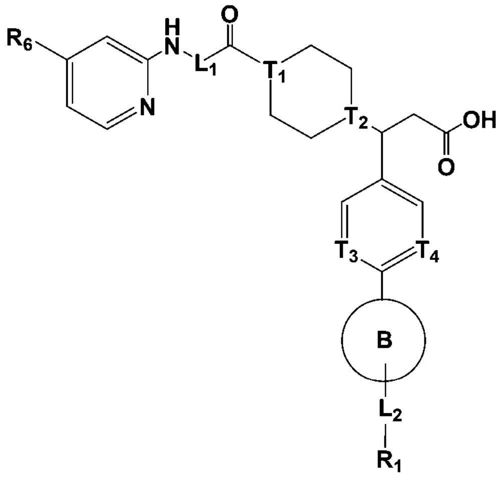

(Ia)

wherein,

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
ring B is

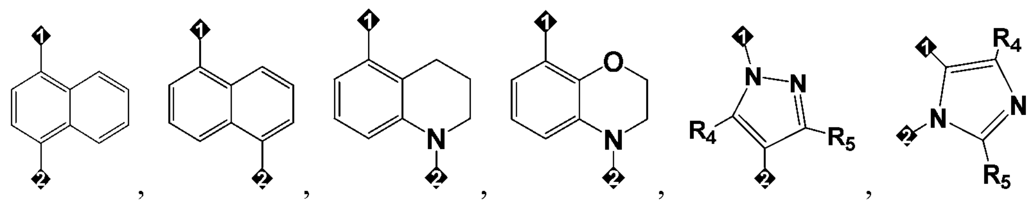

, , , , , ,

or absent, wherein attachment point 2 is connected to $L_2$;
$R_4$, $R_5$ and $R_6$ are each independently H, methyl, ethyl, n-propyl, isopropyl, cyclopropyl, or cyclobutyl; preferably $R_4$, $R_5$ and $R_6$ are each independently methyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;
$R_1$ is $-N_3$, $-NH_2$, $-COOH$, $-NHC(O)CH_2SC(O)CH_3$, or

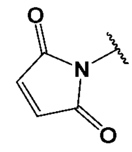 ;

preferably, the compound is selected from Compounds 4, 21, 22, 23, 24, 25, 26, 27, 28, 29, 30, 31, 32, 33, and 34.

**2.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib:

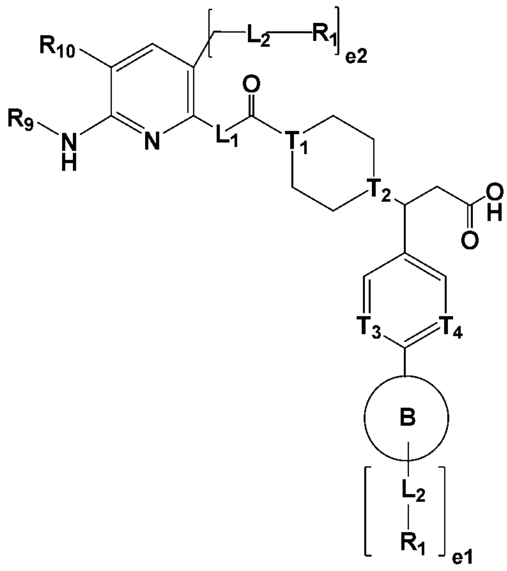 (Ib)

wherein,

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
one of e1 and e2 is 1, and the other is 0;
ring B is

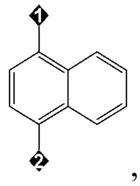 , 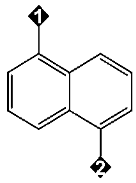 , 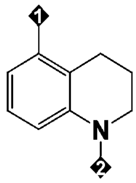 , 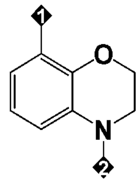 , 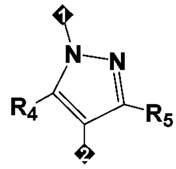 , 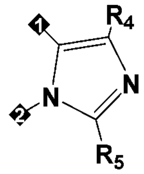 ,

or absent, wherein attachment point 2 is connected to $L_2$, and attachment point 2 is absent when e1 is 0;
$R_4$ and $R_5$ are each independently H, methyl, ethyl, n-propyl, isopropyl, cyclopropyl, or cyclobutyl; preferably $R_4$, $R_5$ and $R_6$ are each independently methyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-NHC(O)(CH_2)_q(OCH_2CH_2)_p-$, wherein the right side is connected to $R_1$, p and q are each independently an integer from 1 to 10; preferably q is 2 or 3; preferably p is 3, 4, 5, 6, or 7;
$R_1$ is $-N_3$, $-NH_2$, $-COOH$, $-NHC(O)CH_2SC(O)CH_3$, or

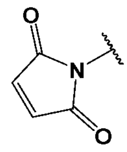

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group;
preferably, the compound is Compound 35.

**3.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa or IIb:

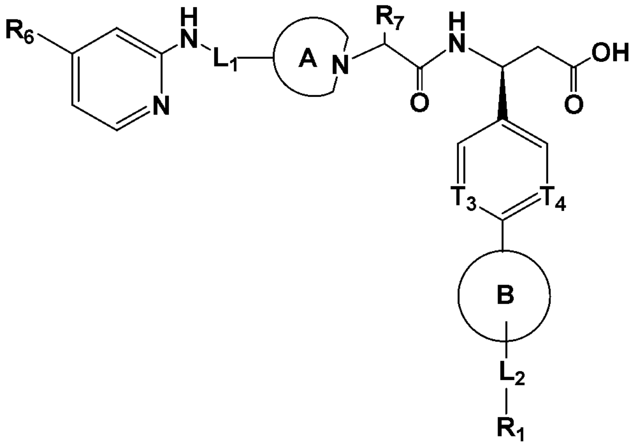

(IIa)

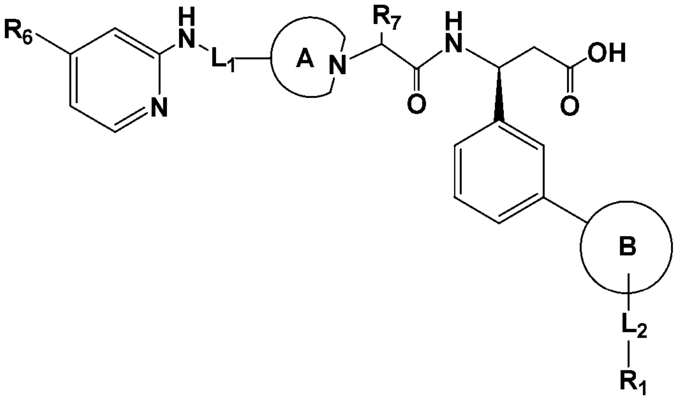

(IIb)

wherein,

$T_3$ and $T_4$ are each independently C or N; preferably one of $T_3$ and $T_4$ is N; more preferably $T_3$ and $T_4$ are both C;
ring A is

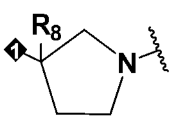, 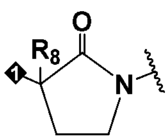, 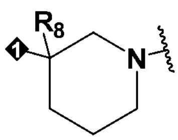, or 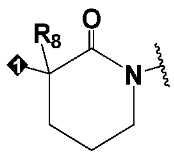,

wherein attachment point 1 is connected to $L_1$; $R_8$ is H, methyl, ethyl, n-propyl, isopropyl, cyclopropyl, cyclobutyl,

or halogen, preferably H or halogen, more preferably H or F;
ring B is

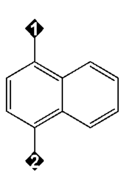 , 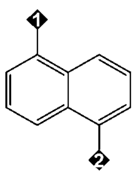 , 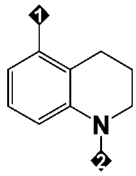 , 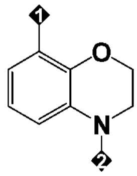 , 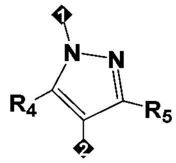 , 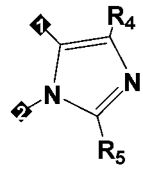 ,

or absent, wherein attachment point 2 is connected to $L_2$;
$R_4$, $R_5$, $R_6$, and $R_7$ are each independently H, methyl, ethyl, n-propyl, isopropyl, cyclopropyl, or cyclobutyl; preferably $R_4$, $R_5$ and $R_6$ are each independently methyl; preferably $R_7$ is H;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;
$R_1$ is $-N_3$, $-NH_2$, -COOH, $-NHC(O)CH_2SC(O)CH_3$, or

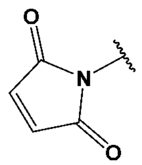 ;

preferably, the compound is any one of Compounds 5, 36, 37, 38, 39, 40, 41, 42, 43, or 44.

4. A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa or IIIb:

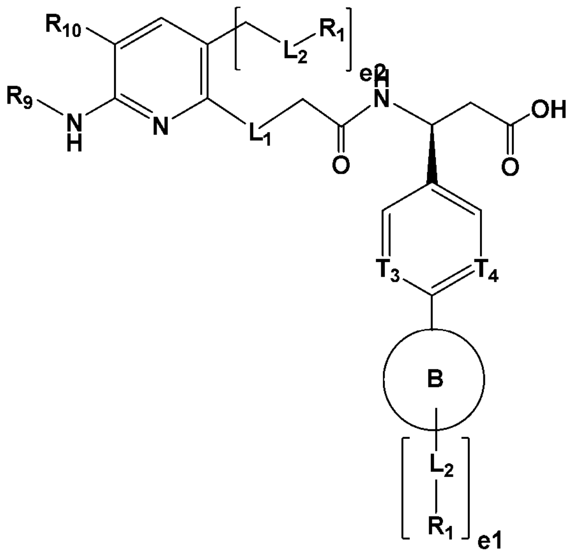

(IIIa)

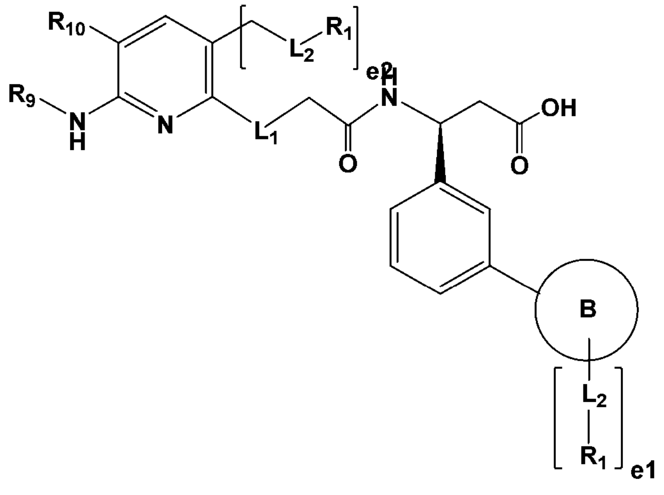
(IIIb)

wherein,

$T_3$ and $T_4$ are each independently C or N; preferably one of $T_3$ and $T_4$ is N; more preferably $T_3$ and $T_4$ are both C; one of e1 and e2 is 1, and the other is 0;
ring B is

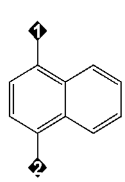 , 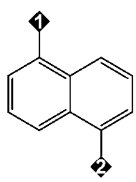 , 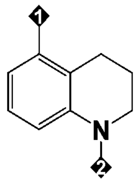 , 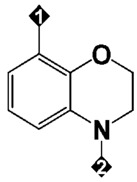 , 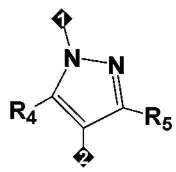 , 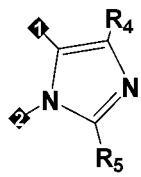 ,

or absent, wherein attachment point 2 is connected to $L_2$, and attachment point 2 is absent when e1 is 0;
$R_4$ and $R_5$ are each independently H, methyl, ethyl, n-propyl, isopropyl, cyclopropyl, or cyclobutyl, preferably methyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-NHC(O)(CH_2)_q(OCH_2CH_2)_p-$, wherein the right side is connected to $R_1$, p and q are each independently an integer from 1 to 10; preferably q is 2 or 3; preferably p is 3, 4, 5, 6, or 7;
$R_1$ is $-N_3$, $-NH_2$, -COOH, $-NHC(O)CH_2SC(O)CH_3$, or

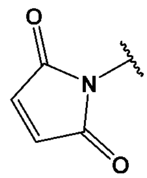 ;

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group;
preferably, the compound is Compound 6, 7, 8, 9, 45, 46, or 47.

**5.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof,

wherein the compound has a structure represented by Formula IVa:

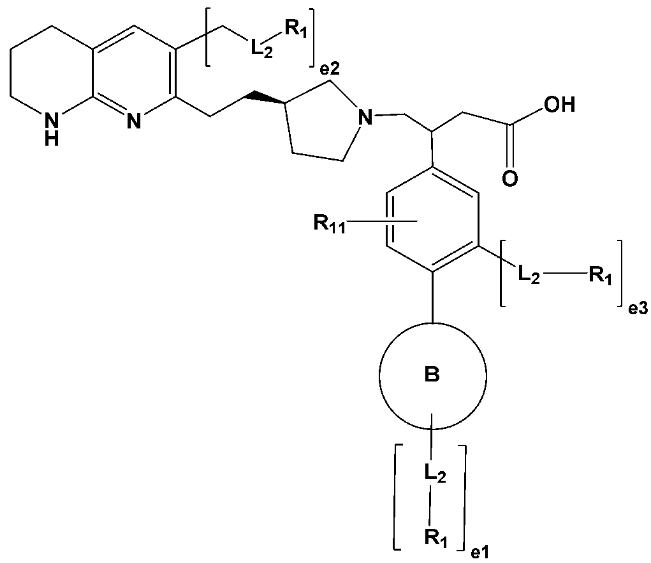

(IVa)

wherein, one of e1, e2, and e3 is 1, and the other two are 0;

ring B is

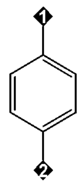 , 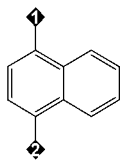 , 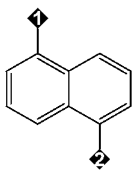 , 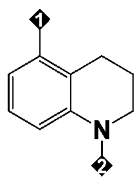 , 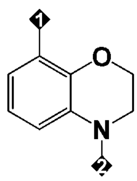 , 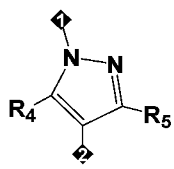 , 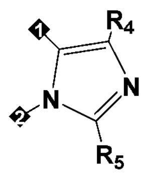 ,

or absent, wherein attachment point 2 is connected to $L_2$, and attachment point 2 is absent when e1 is 0;
$R_4$ and $R_5$ are each independently H, methyl, ethyl, n-propyl, isopropyl, cyclopropyl, or cyclobutyl; preferably $R_4$ and $R_5$ are both methyl;
when e1 or e3 is 1, $L_2$ is $-(OCH_2CH_2)_p-$, $-(CH_2)_q(OCH_2CH_2)_p-$, $-NHC(O)(CH_2CH_2O)_p-$, - $NHC(O)(CH_2)_q(OCH_2CH_2)_p-$, $-C(O)NH(CH_2CH_2O)_p-$, $-C(O)NH(CH_2)_q(OCH_2CH_2)_p-$, - $C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-NHC(O)(CH_2)q(OCH_2CH_2)_p-$, wherein the right side is connected to $R_1$, p and q are each independently an integer from 1 to 10; preferably q is 2 or 3; preferably p is 3, 4, 5, 6, or 7;
$R_1$ is $-N_3$, $-NH_2$, -COOH, $-NHC(O)CH_2SC(O)CH_3$, or

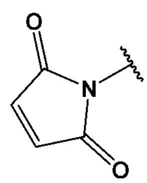 ;

$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy;
preferably, the compound is Compound 13, 14, 15, 18, 20, 48, 49, 53, 54, 55, 56, 57, 58, 59, 60, 61, 62, 63, 71, 72, 73, 83, 84, 85, 86, or 87.

**6.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof,

wherein the compound has a structure represented by Formula IVb:

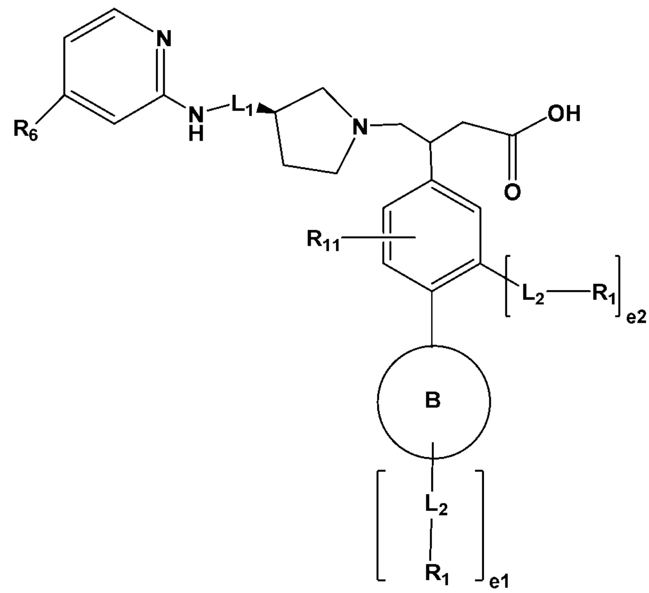

(IVb)

one of e1 and e2 is 1, and the other is 0;
ring B is

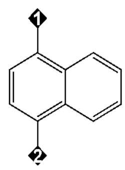 , 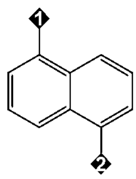 , 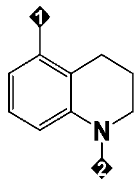 , 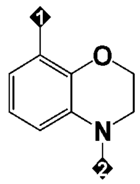 , 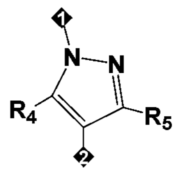 , 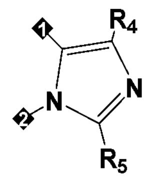 ,

or absent, wherein attachment point 2 is connected to $L_2$;
$R_4$, $R_5$ and $R_6$ are each independently H, methyl, ethyl, n-propyl, isopropyl, cyclopropyl, or cyclobutyl; preferably $R_4$, $R_5$ and $R_6$ are all methyl;
$L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q-(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or - $C(O)(CH_2)_q(OCH_2CH_2)_p-$, wherein the left side is connected to ring B, the right side is connected to $R_1$, and p and q are each independently an integer from 1 to 10; preferably q is 2 or 3; preferably p is 3, 4, 5, 6, or 7;
$R_1$ is $-N_3$, $-NH_2$, -COOH, $-NHC(O)CH_2SC(O)CH_3$, or

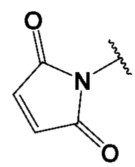 ;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
$R_{11}$ is H, halogen, halomethyl, methyl, ethyl, n-propyl, isopropyl, cyclopropyl, cyclobutyl, phenyl, morpholinyl, methoxyethoxy, or ethoxymethoxy; preferably $R_{11}$ is H;
preferably, the compound is Compound 50, 51, 52, 74, 75, 76, 77, 78, 79, or 80.

**7.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va:

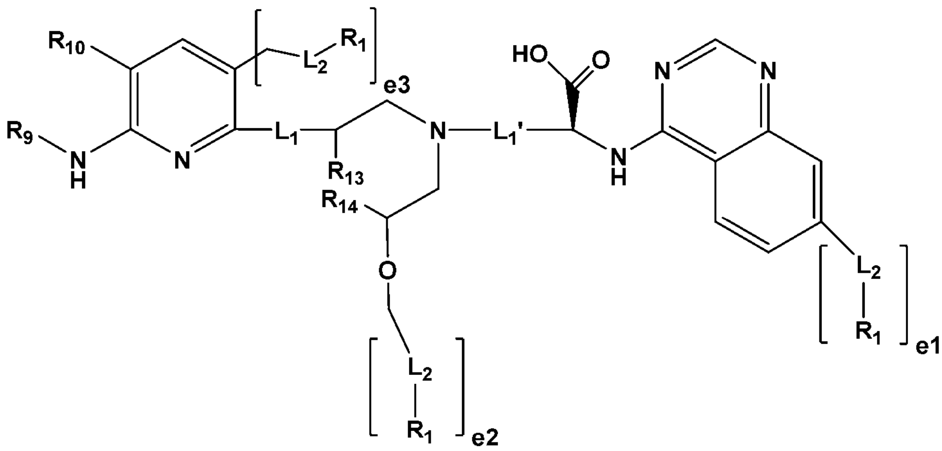

(Va)

one of e1, e2, and e3 is 1, and the other two are 0;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;

$R_{13}$ and $R_{14}$ are each H, or $R_{13}$, $R_{14}$, together with the C and N atoms to which they are attached, form a piperidinyl group;

$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;

when e2 is 1, $L_2$ is $-(CH_2)q(OCH_2CH_2)_p-$, wherein the right side is connected to $R_1$, p and q are each independently an integer from 1 to 10; preferably q is 1 or 2; preferably p is 3, 4, 5, 6, or 7;

when e3 is 1, $L_2$ is $-NHC(O)(CH_2)q(OCH_2CH_2)_p-$, wherein the right side is connected to $R_1$, p and q are each independently an integer from 1 to 10; preferably q is 2 or 3; preferably p is 3, 4, 5, 6, or 7;

$R_1$ is $-N_3$, $-NH_2$, $-COOH$, $-NHC(O)CH_2SC(O)CH_3$, or

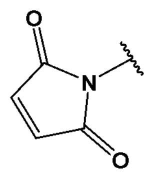
;

preferably, the compound is Compound 16, 17, 64, 65, 66, or 67.

**8.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb:

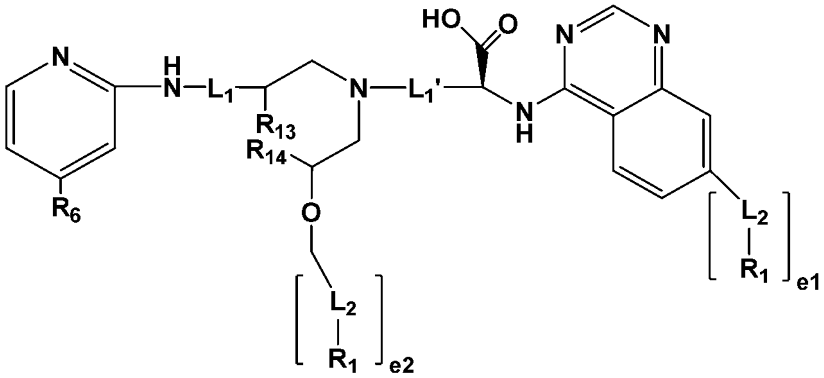

(Vb)

wherein

one of e1 and e2 is 1, and the other is 0;
$R_6$ is H, methyl, ethyl, n-propyl, isopropyl, cyclopropyl, or cyclobutyl, preferably methyl;
$R_{13}$ and $R_{14}$ are each H, or $R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;
when e2 is 1, $L_2$ is $-(CH_2)q(OCH_2CH_2)_p-$, wherein the right side is connected to $R_1$, p and q are each independently an integer from 1 to 10; preferably q is 1 or 2; preferably p is 3, 4, 5, 6, or 7;
$R_1$ is $-N_3$, $-NH_2$, -COOH, $-NHC(O)CH_2SC(O)CH_3$, or

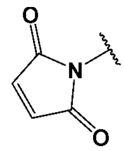
;

preferably, the compound is Compound 69 or 70.

9. A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc:

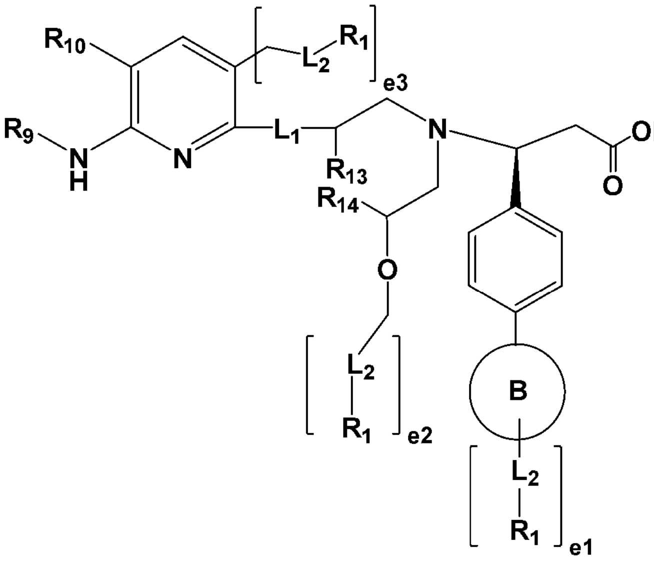

(Vc)

one of e1, e2, and e3 is 1, and the other two are 0;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;

$R_{13}$ and $R_{14}$ are each H, or $R_{13}$, $R_{14}$, together with the C and N atoms to which they are attached, form a piperidinyl group;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

when e1 is 1, $L_2$ is $-(OCH_2CH_2)_p-$, $-(CH_2)_q(OCH_2CH_2)_p-$, $-NHC(O)(CH_2CH_2O)_p-$, $-NHC(O)(CH_2)_q(OCH_2CH_2)_p-$, $-C(O)NH(CH_2CH_2O)_p-$, $-C(O)NH(CH_2)_q(OCH_2CH_2)_p-$, $-C(O)(CH_2CH_2O)_p-$, or $-C(O)(CH_2)_q(OCH_2CH_2)_p-$, wherein the right side is connected to $R_1$, and p and q are each independently an integer from 1 to 10; preferably q is 2 or 3; preferably p is 3, 4, 5, 6, or 7;

when e2 is 1, $L_2$ is $-(CH_2)q(OCH_2CH_2)_p-$, wherein the right side is connected to $R_1$, p and q are each independently an integer from 1 to 10; preferably q is 1 or 2; preferably p is 3, 4, 5, 6, or 7;

when e3 is 1, $L_2$ is $-NHC(O)(CH_2)q(OCH_2CH_2)_p-$, wherein the right side is connected to $R_1$, p and q are each independently an integer from 1 to 10; preferably q is 2 or 3; preferably p is 3, 4, 5, 6, or 7;

$R_1$ is $-N_3$, $-NH_2$, $-COOH$, $-NHC(O)CH_2SC(O)CH_3$, or

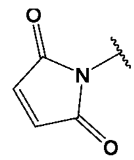 ;

ring B is

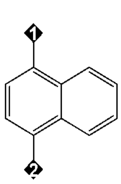 , 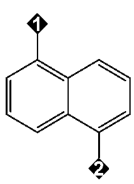 , 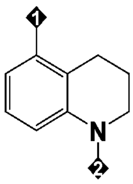 , 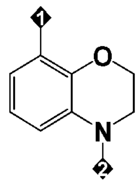 , 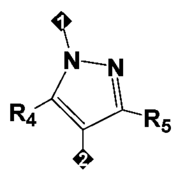 , 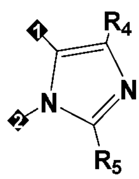 ,

or absent, wherein attachment point 2 is connected to $L_2$, and attachment point 2 is absent when e1 is 0;

$R_4$ and $R_5$ are each independently H, methyl, ethyl, n-propyl, isopropyl, cyclopropyl, or cyclobutyl; preferably $R_4$ and $R_5$ are both methyl;

preferably, the compound is Compound 68.

**10.** A compound, or a tautomer, a mesomer, a racemate, an enantiomer, or a diastereomer thereof, or a deuterated

compound form thereof, or a mixture form thereof, or a pharmaceutically acceptable salt, prodrug, or solvate thereof, wherein the compound has a structure represented by Formula Ia':

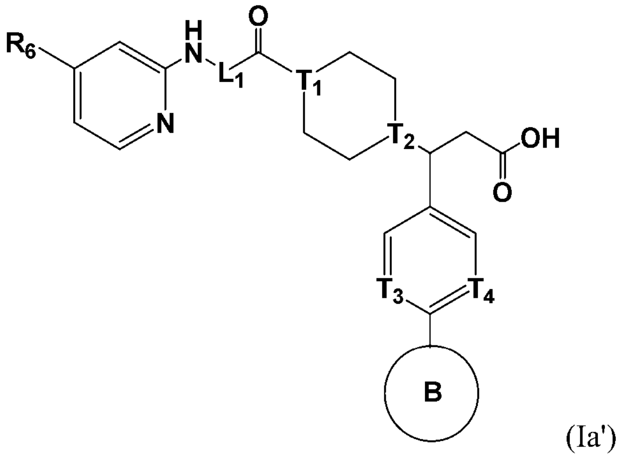

(Ia')

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
ring B is

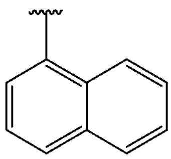 , 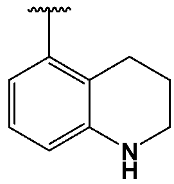 , 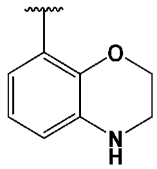 , 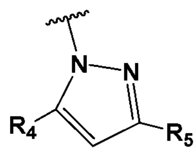 , 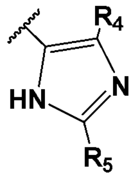 ;

or absent;
$R_4$, $R_5$ and $R_6$ are each independently H, methyl, ethyl, n-propyl, isopropyl, cyclopropyl, or cyclobutyl; preferably $R_4$, $R_5$ and $R_6$ are each independently methyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
preferably, the compound is selected from Compounds 4', 21', 22', 23', 24', 31', 32', 33', and 34'.

**11.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib':

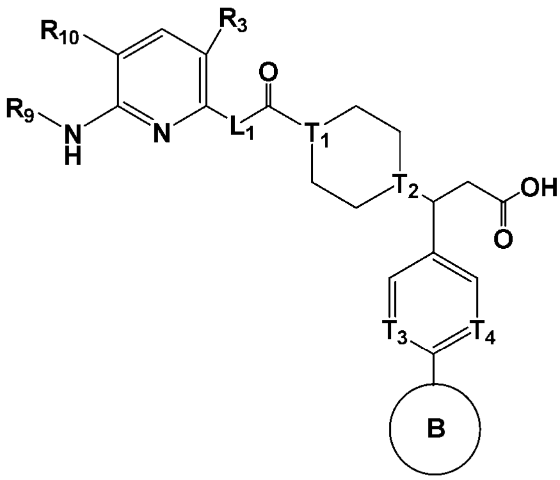

(Ib')

wherein,

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
ring B is

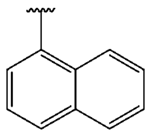, 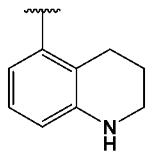, 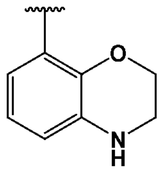, 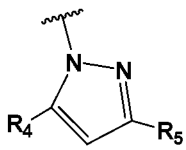, 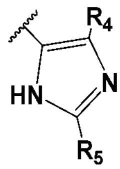,

or absent;
$R_4$ and $R_5$ are each independently H, methyl, ethyl, n-propyl, isopropyl, cyclopropyl, or cyclobutyl; preferably $R_4$, $R_5$ and $R_6$ are each independently methyl;
$R_3$ is H, methyl, ethyl, n-propyl, isopropyl, cyclopropyl, cyclobutyl, amino, or $C_{1-3}$ alkylamino; preferably $R_3$ is H or methylamino;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group;
preferably, the compound is Compound 35'.

**12.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa' or IIb':

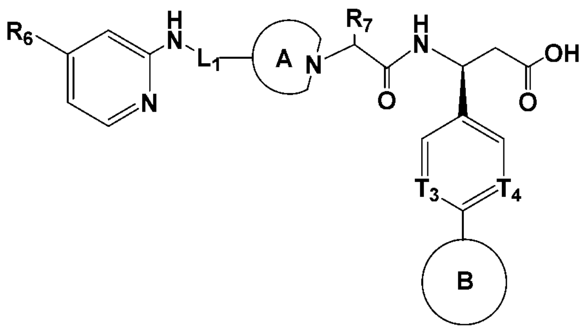

(IIa')

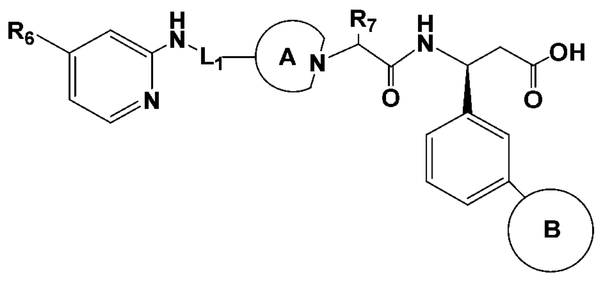

(IIb')

wherein,

$T_3$ and $T_4$ are each independently C or N; preferably one of $T_3$ and $T_4$ is N; more preferably $T_3$ and $T_4$ are both C;
ring A is

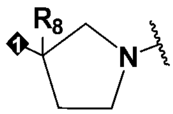, 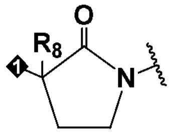, 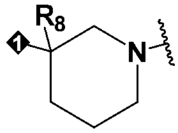, or 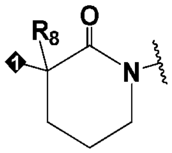,

wherein attachment point 1 is connected to $L_1$; $R_8$ is H, methyl, ethyl, n-propyl, isopropyl, cyclopropyl, cyclobutyl, or halogen, preferably H or halogen, more preferably H or F;
ring B is

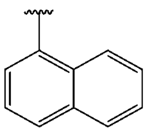, 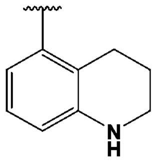, 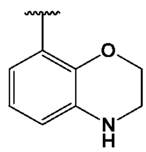, 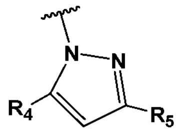, 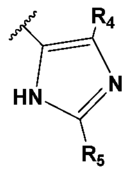,

or absent;
$R_4$, $R_5$, $R_6$, and $R_7$ are each independently H, methyl, ethyl, n-propyl, isopropyl, cyclopropyl, or cyclobutyl; preferably $R_4$, $R_5$ and $R_6$ are each independently methyl; preferably $R_7$ is H;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
preferably, the compound is any one of Compounds 5', 36', 37', 38', 39', 40', 41', 42', 43', or 44'.

**13.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa' or IIIb':

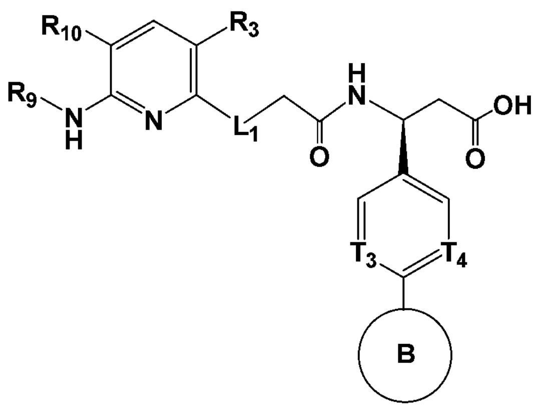

(IIIa')

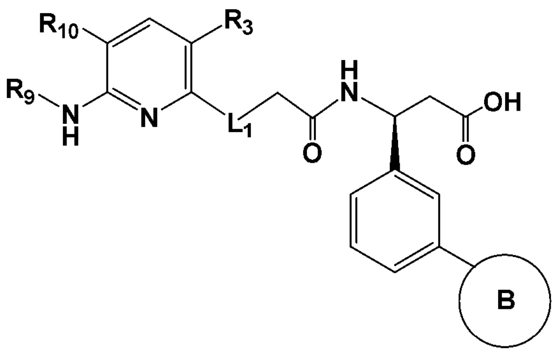

(IIIb')

wherein,

$T_3$ and $T_4$ are each independently C or N; preferably one of $T_3$ and $T_4$ is N; more preferably $T_3$ and $T_4$ are both C;

ring B is

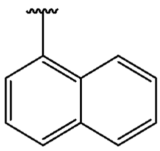, 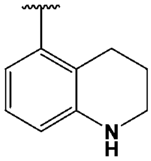, 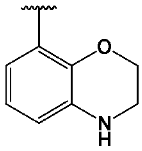, 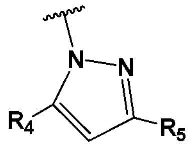, or 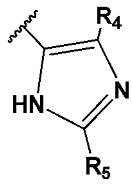;

$R_4$ and $R_5$ are each independently H, methyl, ethyl, n-propyl, isopropyl, cyclopropyl, or cyclobutyl, preferably methyl;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

$R_3$ is H, methyl, ethyl, n-propyl, isopropyl, cyclopropyl, cyclobutyl, amino, or $C_{1-3}$ alkylamino; preferably $R_3$ is H or methylamino;

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group;

preferably, the compound is Compound 6', 7', 8', 9', or 47'.

**14.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa':

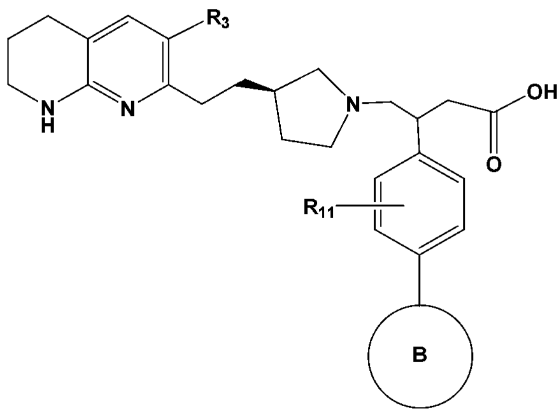

(IVa')

wherein,

ring B is

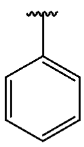, 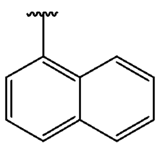, 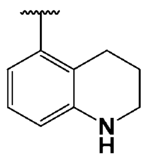, or 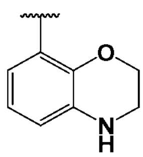;

$R_4$ and $R_5$ are each independently H, methyl, ethyl, n-propyl, isopropyl, cyclopropyl, or cyclobutyl; preferably $R_4$ and $R_5$ are both methyl;

$R_3$ is H, methyl, ethyl, n-propyl, isopropyl, cyclopropyl, cyclobutyl, amino, or $C_{1-3}$ alkylamino; preferably $R_3$ is H or methylamino;

$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy;

preferably, the compound is Compound 48', 49', 59', 60', 73', or 85'.

**15.** A compound, or a tautomer, a mesomer, a racemate, an enantiomer, or a diastereomer thereof, or a deuterated

compound form thereof, or a mixture form thereof, or a pharmaceutically acceptable salt, prodrug, or solvate thereof, wherein the compound has a structure represented by Formula IVb':

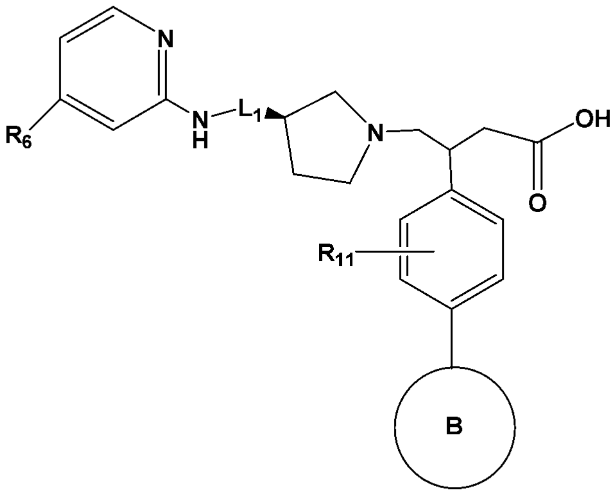

(IVb')

wherein

ring B is

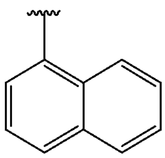
, 
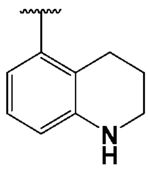
, 
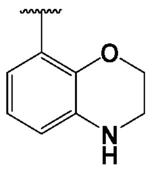
, 
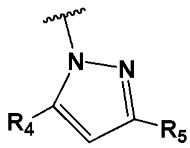
, 
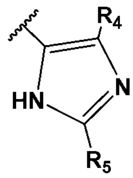
,

or absent;

$R_4$, $R_5$ and $R_6$ are each independently H, methyl, ethyl, n-propyl, isopropyl, cyclopropyl, or cyclobutyl; preferably $R_4$, $R_5$ and $R_6$ are all methyl;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

$R_{11}$ is H, halogen, halomethyl, methyl, ethyl, n-propyl, isopropyl, cyclopropyl, cyclobutyl, phenyl, morpholinyl, methoxyethoxy, or ethoxymethoxy; preferably $R_{11}$ is H;

preferably, the compound is Compound 50', 51', 52', 76', or 79'.

**16.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va':

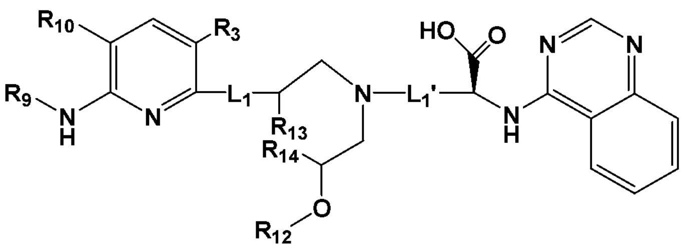

(Va')

wherein

$R_3$ is H, methyl, ethyl, n-propyl, isopropyl, cyclopropyl, cyclobutyl, amino, or $C_{1-3}$ alkylamino; preferably $R_3$ is H or methylamino;

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;
$R_{12}$ is methyl, ethyl, n-propyl, isopropyl, cyclopropyl, or cyclobutyl; preferably $R_{12}$ is methyl;
$R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3; preferably, the compound is Compound 64'.

**17.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb':

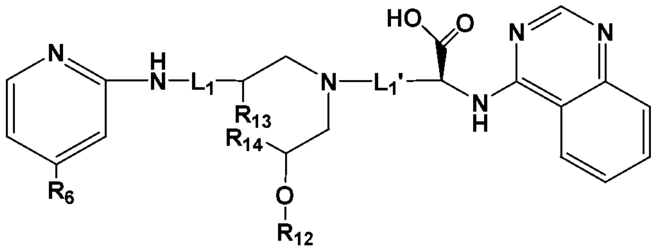

(Vb')

wherein,

$R_6$ is H, methyl, ethyl, n-propyl, isopropyl, cyclopropyl, or cyclobutyl, preferably methyl;
$R_{12}$ is methyl, ethyl, n-propyl, isopropyl, cyclopropyl, or cyclobutyl; preferably $R_{12}$ is methyl;
$R_{13}$ and $R_{14}$ are each H, or $R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3; preferably, the compound is Compound 69' or 70'.

**18.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vc':

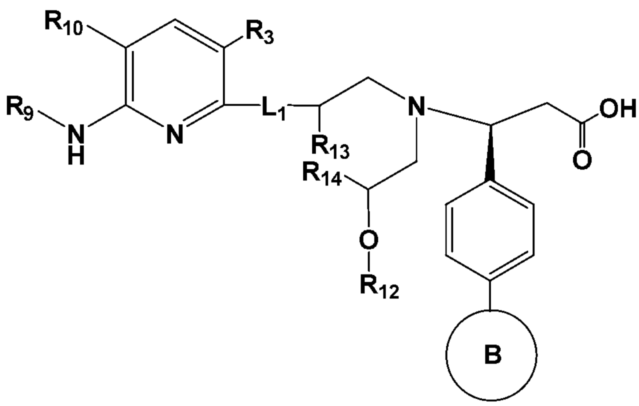

(Vc')

$R_3$ is H, methyl, ethyl, n-propyl, isopropyl, cyclopropyl, cyclobutyl, amino, or $C_{1-3}$ alkylamino; preferably $R_3$ is H or methylamino;
$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;
$R_{12}$ is methyl, ethyl, n-propyl, isopropyl, cyclopropyl, or cyclobutyl; preferably $R_{12}$ is methyl;
$R_{13}$ and $R_{14}$ are each H, or $R_{13}$, $R_{14}$, together with the C and N atoms to which they are attached, form a piperidinyl group;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1,

2, 3, 4, 5, or 6; n is an integer from 0 to 10; preferably n is 0, 1, 2, or 3;
ring B is

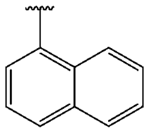, 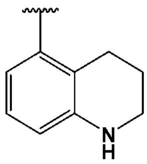, 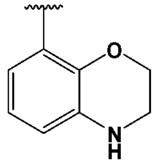, 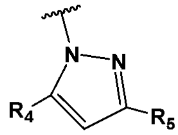, 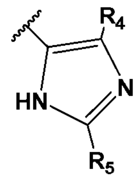,

or absent;
$R_4$ and $R_5$ are each independently H, methyl, ethyl, n-propyl, isopropyl, cyclopropyl, or cyclobutyl; preferably $R_4$ and $R_5$ are both methyl;
preferably, the compound is Compound 68'.

**19.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ia":

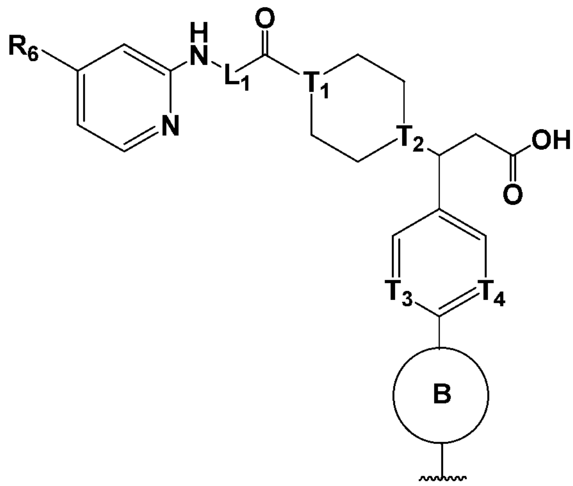

(Ia")

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
ring B is

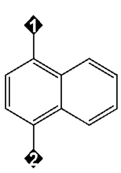, 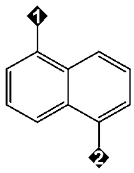, 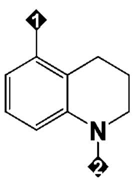, 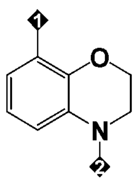, 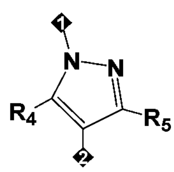, 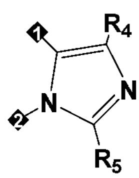,

or absent, wherein attachment point 2 is the wave line in Formula Ia", and the wave line ~~~~~ indicates a bond;
$R_4$, $R_5$ and $R_6$ are each independently H, methyl, ethyl, n-propyl, isopropyl, cyclopropyl, or cyclobutyl; preferably $R_4$, $R_5$ and $R_6$ are each independently methyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
preferably, the compound is selected from: 4", 21", 22", 23", 24", 31", 32", 33", and 34".

**20.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Ib":

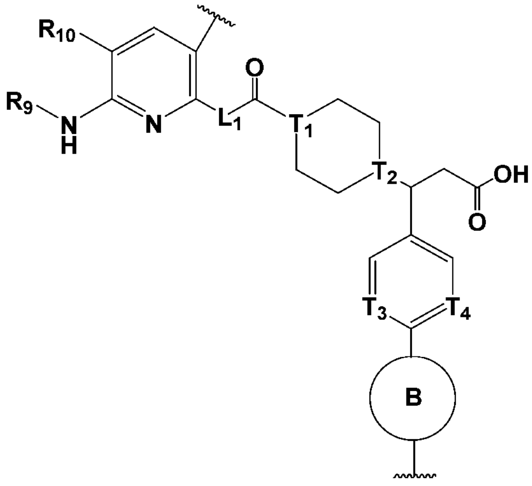

(Ib")

wherein,

$T_1$ and $T_2$ are each independently C or N, and at least one is N; preferably $T_1$ and $T_2$ are both N;
$T_3$ and $T_4$ are each independently C or N; preferably one of $T_3$ and $T_4$ is N; more preferably $T_3$ and $T_4$ are both C;
ring B is

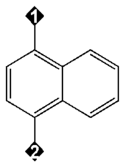 , 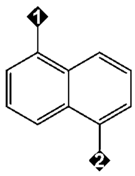 , 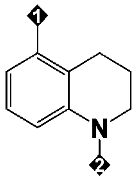 , 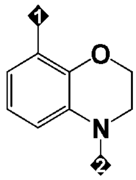 , 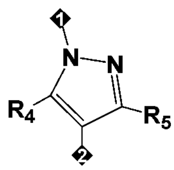 , 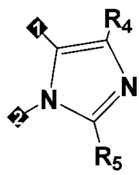 ,

or absent, wherein attachment point 2 is the wave line connected to ring B in Formula Ib";
$R_4$ and $R_5$ are each independently H, methyl, ethyl, n-propyl, isopropyl, cyclopropyl, or cyclobutyl; preferably $R_4$ and $R_5$ are each independently methyl;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group; and
the wave line in Formula Ib" indicates a bond, and one of the two wave lines is absent;
preferably, wherein the compound has a structure represented by Formula Ib"-1:

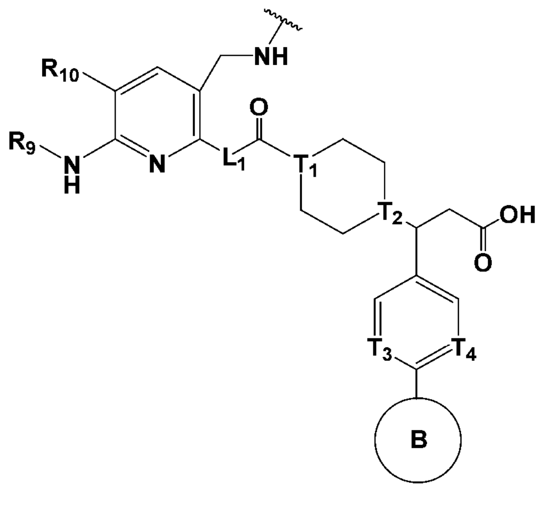

(Ib"-1)

wherein $T_1$, $T_2$, $T_3$, $T_4$, $L_1$, $R_9$, $R_{10}$, and the wave line have the same definitions as the corresponding substituents in Formula Ib",
ring B is

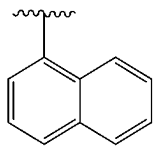, 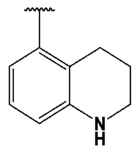, 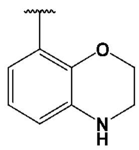, 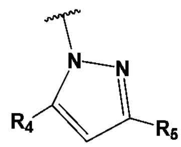, 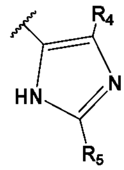,

or absent, wherein $R_4$ and $R_5$ are each independently H, methyl, ethyl, n-propyl, isopropyl, cyclopropyl, or cyclobutyl; preferably $R_4$ and $R_5$ are each independently methyl;
more preferably, the compound is selected from: 35".

**21.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIa" or IIb":

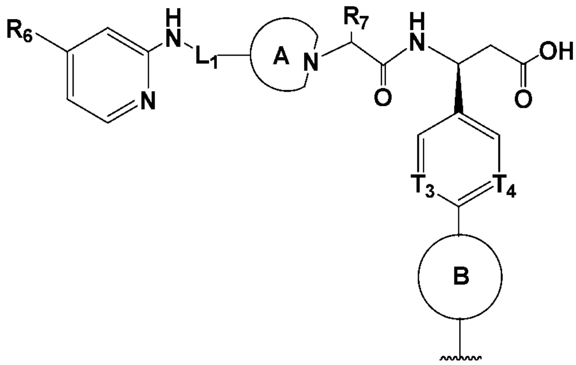
(IIa")

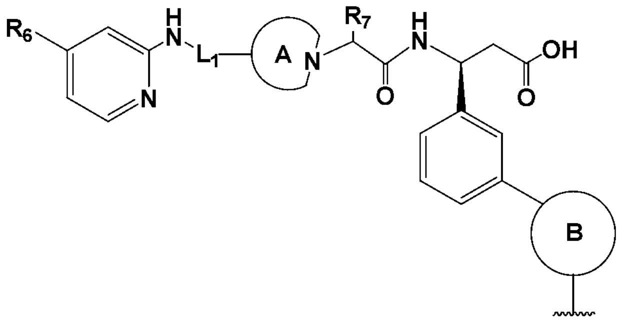
(IIb")

wherein,

$T_3$ and $T_4$ are each independently C or N; preferably one of $T_3$ and $T_4$ is N; more preferably $T_3$ and $T_4$ are both C;
ring A is

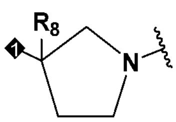, 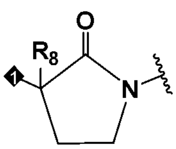, 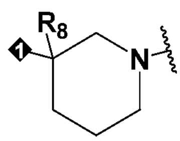, or 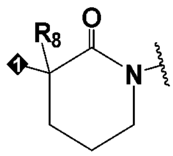,

wherein attachment point 1 is connected to $L_1$; $R_8$ is H, methyl, ethyl, n-propyl, isopropyl, cyclopropyl, cyclobutyl, or halogen, preferably H or halogen, more preferably H or F;

ring B is

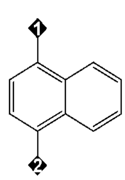, 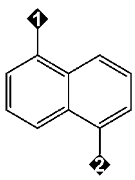, 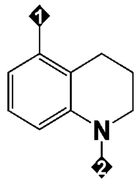, 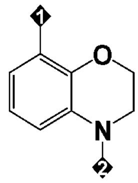, 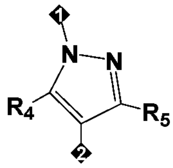, 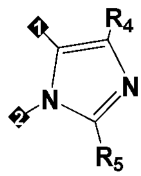,

or absent, wherein attachment point 2 is the wave line in Formula IIa" or IIb", and the wave line indicates a bond; $R_4$, $R_5$, $R_6$, and $R_7$ are each independently H, methyl, ethyl, n-propyl, isopropyl, cyclopropyl, or cyclobutyl; preferably $R_4$, $R_5$ and $R_6$ are each independently methyl; preferably $R_7$ is H;
$L_1$ is $-(CH_2)_n-$, $-(CH_2)_mC(O)NH(CH_2)_n-$, or $-(CH_2)_mNHC(O)(CH_2)_n-$; m and n are each independently an integer from 1 to 10; preferably m and n are each independently 1, 2, 3, 4, 5, or 6;
preferably, the compound is selected from: 5", 36", 37", 38", 39", 40", 41", 42", 43", and 44".

**22.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IIIa" or IIIb":

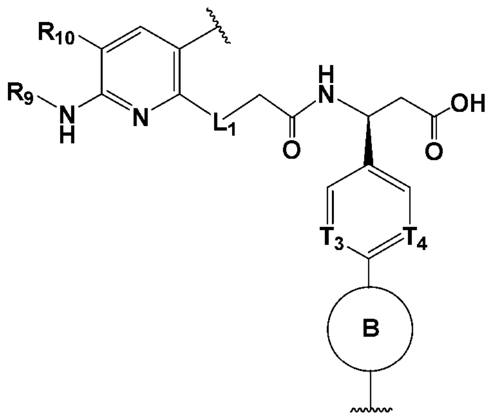

(IIIa")

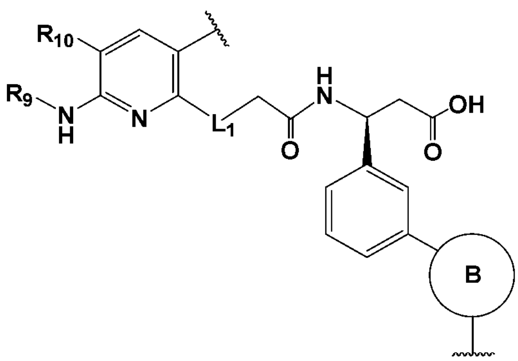

(IIIb")

wherein,

$T_3$ and $T_4$ are each independently C or N; preferably one of $T_3$ and $T_4$ is N; more preferably $T_3$ and $T_4$ are both C;
ring B is

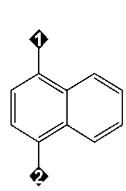, 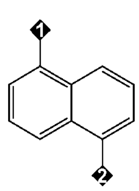, 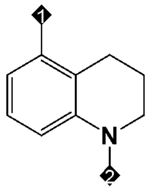, 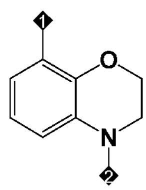, 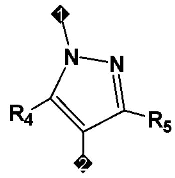, 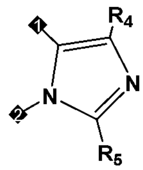,

or absent, wherein attachment point 2 is the wave line connected to ring B in Formula IIIa" or IIIb";

$R_4$ and $R_5$ are each independently H, methyl, ethyl, n-propyl, isopropyl, cyclopropyl, or cyclobutyl, preferably methyl;

$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;

$R_9$ is H, methyl, ethyl, n-propyl, isopropyl, cyclopropyl, or cyclobutyl; $R_{10}$ is H, or $R_9$, $R_{10}$, together with the C and N to which they are attached, form a piperidinyl group; preferably $R_9$ is methyl and $R_{10}$ is H, or $R_9$, $R_{10}$, together with the C and N to which they are attached, form a piperidinyl group; and

the wave line in Formula IIIa" and Formula IIIb" indicates a bond, and one of the two wave lines is absent;

or, wherein the compound has a structure represented by Formula IIIa"-1 or IIIb"-1:

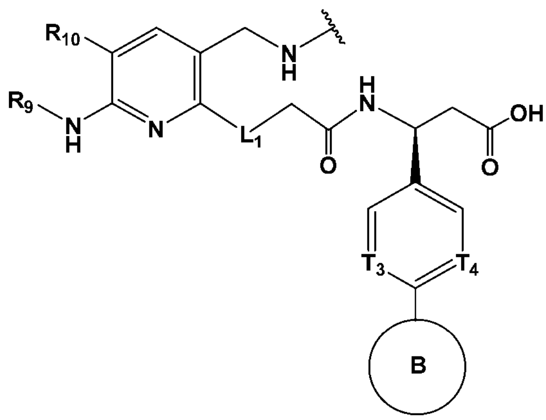

(IIIa"-1)

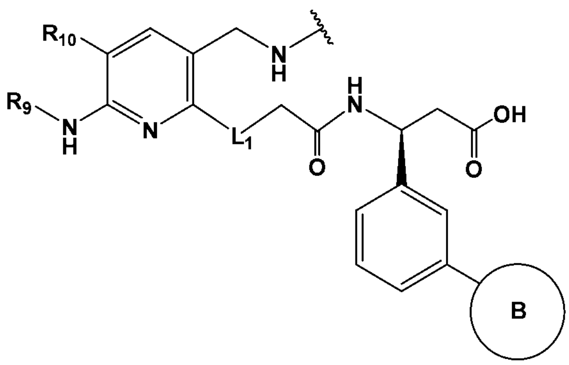

(IIIb"-1)

wherein $T_3$, $T_4$, $L_1$, $R_9$, $R_{10}$, and the wave line have the same definitions as the corresponding substituents in Formula IIIa" or IIIb",

ring B is

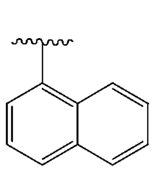, 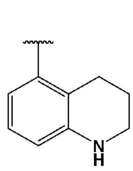, 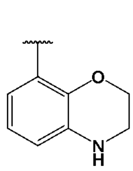, 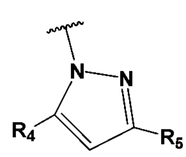, 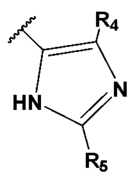,

or absent, wherein $R_4$ and $R_5$ are each independently H, methyl, ethyl, n-propyl, isopropyl, cyclopropyl, or

cyclobutyl; preferably $R_4$ and $R_5$ are each independently methyl;
preferably, the compound is selected from: 6", 7", 8", 9", 45", 46", and 47".

**23.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVa":

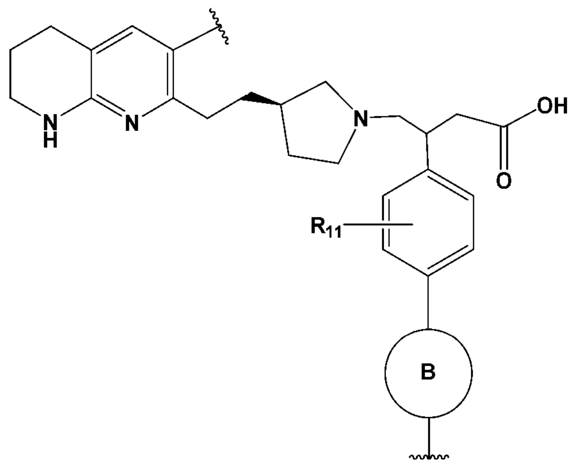

(IVa")

wherein, ring B is

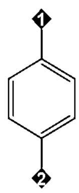
,
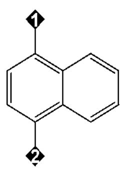
,
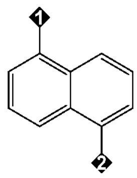
,
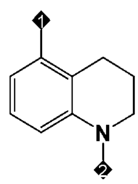
,
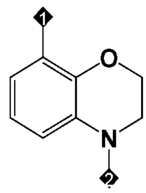
,
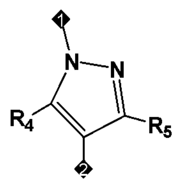
,
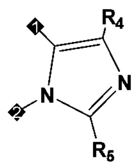
,

or absent, wherein attachment point 2 is the wave line connected to ring B in Formula IVa";
$R_4$ and $R_5$ are each independently H, methyl, ethyl, n-propyl, isopropyl, cyclopropyl, or cyclobutyl; preferably $R_4$ and $R_5$ are both methyl;
$R_{11}$ is H, halogen, halo-$C_{1-4}$ alkyl, methyl, ethyl, n-propyl, isopropyl, cyclopropyl, cyclobutyl, phenyl, morpholinyl, methoxyethoxy, or ethoxymethoxy; and
the wave line in Formula IVa" indicates a bond, and one of the two wave lines is absent;
or, wherein the compound has a structure represented by Formula IVa"-1:

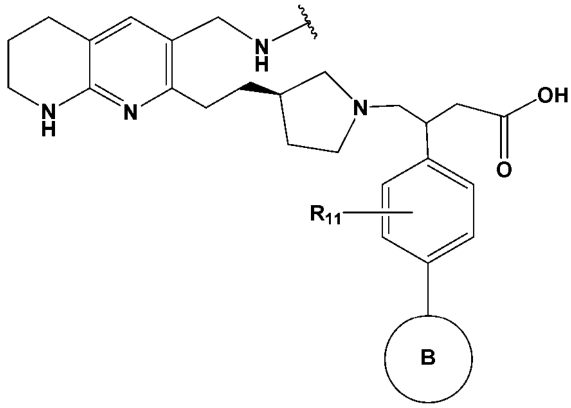

(IVa"-1)

wherein $R_{11}$ and the wave line have the same definitions as $R_{11}$ and the wave line in Formula IVa", ring B is

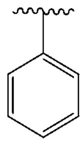 , 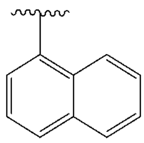 , 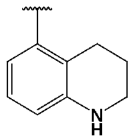 , 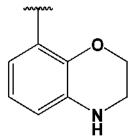 , 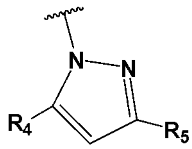 , 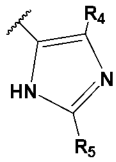 ,

or absent, wherein $R_4$ and $R_5$ are each independently H, methyl, ethyl, n-propyl, isopropyl, cyclopropyl, or cyclobutyl; preferably $R_4$ and $R_5$ are each independently methyl;
preferably, the compound is selected from: 13", 14", 15", 18", 20", 48", 49", 53", 54", 55", 56", 57", 58", 59", 60", 61", 62", 63", 71", 72", 73", 83", 84", 85", 86", and 87".

**24.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula IVb":

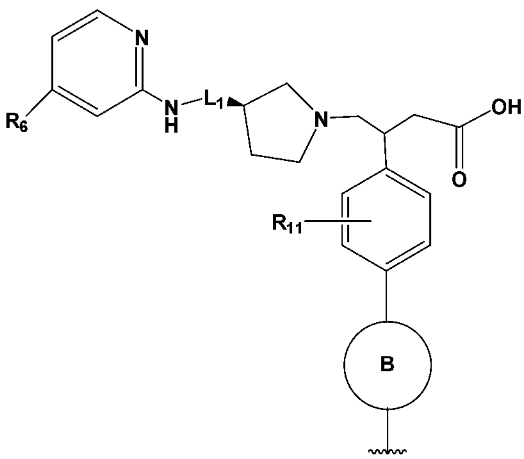

(IVb")

ring B is

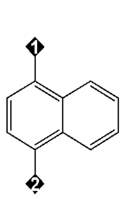 , 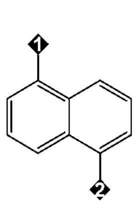 , 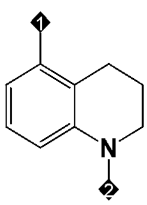 , 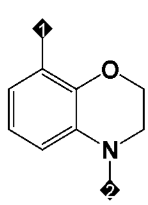 , 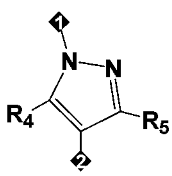 , 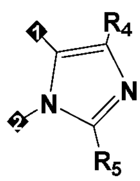 ,

or absent, wherein attachment point 2 is the wave line in Formula IVb", and the wave line indicates a bond; $R_4$, $R_5$ and $R_6$ are each independently H, methyl, ethyl, n-propyl, isopropyl, cyclopropyl, or cyclobutyl; preferably $R_4$, $R_5$ and $R_6$ are all methyl;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
$R_{11}$ is H, halogen, halomethyl, methyl, ethyl, n-propyl, isopropyl, cyclopropyl, cyclobutyl, phenyl, morpholinyl, methoxyethoxy, or ethoxymethoxy; preferably $R_{11}$ is H;
preferably, the compound is selected from: 50", 51", 52", 74", 75", 76", 77", 78", 79", and 80".

**25.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Va":

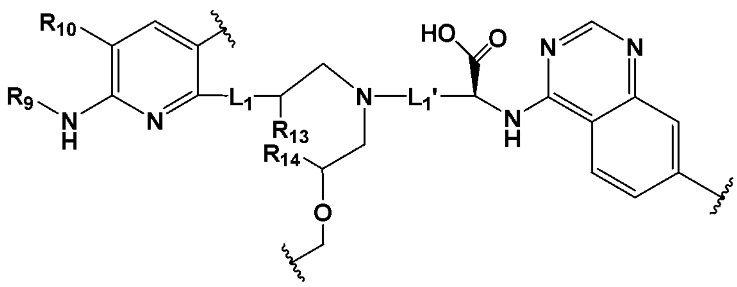

(Va")

wherein

$R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;
$R_{13}$ and $R_{14}$ are each H, or $R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3; and the wave line - in Formula Va" indicates a bond, and two of the three wave lines are absent;
or, wherein the compound has a structure represented by Formula Va"-1, Va"-2, or Va"-3:

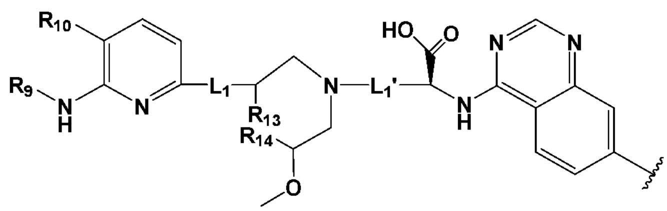

(Va"-1)

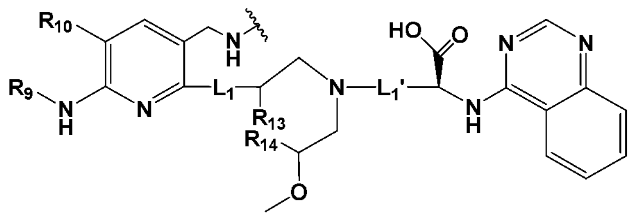

(Va"-2)

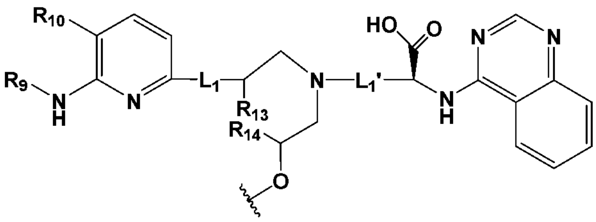

(Va"-3)

wherein, $R_9$, $R_{10}$, $R_{13}$, $R_{14}$, $L_1$, $L_1$', and the wave line have the same definitions as the corresponding substituents in Formula Va";
preferably, the compound is selected from: 16", 17", 64", 65", 66", and 67".

**26.** A compound, or a tautomer, a mesomer, a racemate, an enantiomer, or a diastereomer thereof, or a deuterated compound form thereof, or a mixture form thereof, or a pharmaceutically acceptable salt, prodrug, or solvate thereof, wherein the compound has a structure represented by Formula Vb":

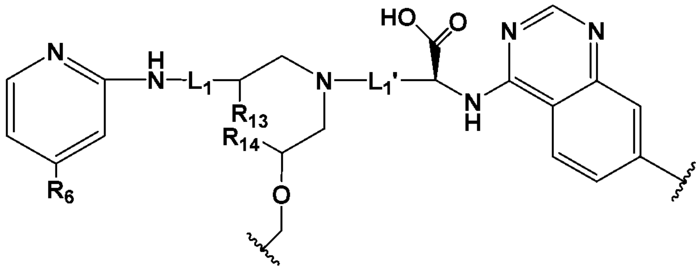

(Vb")

wherein, $R_6$ is H, methyl, ethyl, n-propyl, isopropyl, cyclopropyl, or cyclobutyl, preferably methyl;
$R_{13}$ and $R_{14}$ are each H, or $R_{13}$, $R_{14}$, together with the C and N atoms to which they are attached, form a piperidinyl group;
$L_1$ and $L_1$' are each independently $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3; and the wave line in Formula Vb" indicates a bond, and one of the two wave lines is absent;
or, wherein the compound has a structure represented by Formula Vb"-1 or Vb"-2:

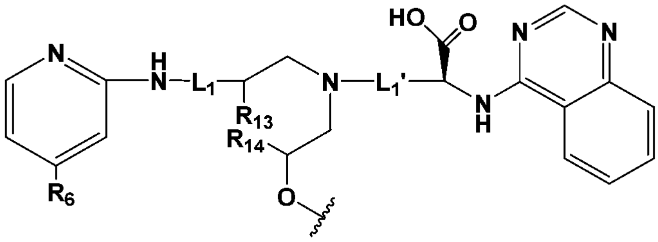

(Vb"-1)

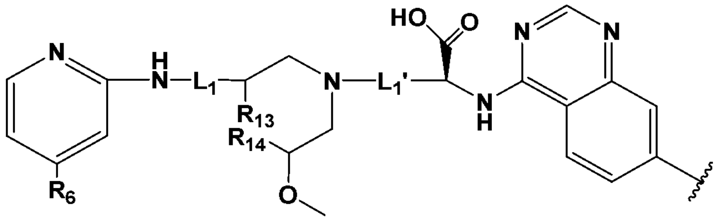

(Vb"-2)

wherein $R_6$, $R_{13}$, $R_{14}$, $L_1$, $L_1$', and the wave line have the same definitions as the corresponding substituents in Formula Vb";
preferably, the compound is selected from: 69" and 70".

**27.** A compound, or a tautomer, a mesomer, a racemate, an enantiomer, or a diastereomer thereof, or a deuterated

compound form thereof, or a mixture form thereof, or a pharmaceutically acceptable salt, prodrug, or solvate thereof, wherein the compound has a structure represented by Formula Vc":

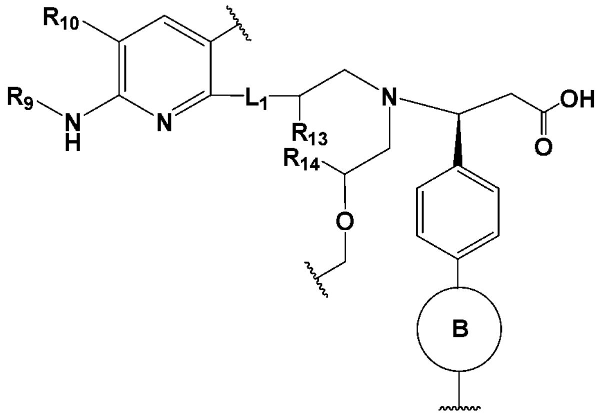

(Vc")

wherein $R_9$ is H, methyl, ethyl, n-propyl, isopropyl, cyclopropyl, or cyclobutyl; $R_{10}$ is H, or $R_9$, $R_{10}$, together with the C and N atoms to which they are attached, form a piperidinyl group; preferably $R_9$ is methyl and $R_{10}$ is H;
$R_{13}$ and $R_{14}$ are each H, or $R_{13}$, $R_{14}$, together with the C and N atoms to which they are attached, form a piperidinyl group;
$L_1$ is $-(CH_2)_m-$, $-(CH_2)_mC(O)NH(CH_2)_n-$, or $-(CH_2)_mNHC(O)(CH_2)_n-$; m is an integer from 1 to 10; preferably m is 1, 2, 3, 4, 5, or 6; n is an integer from 0 to 10; preferably n is 0, 1, 2, or 3;
ring B is

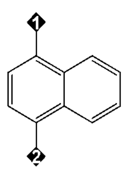 , 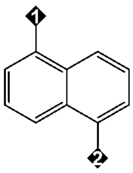 , 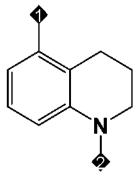 , 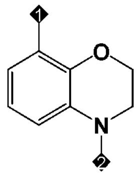 , 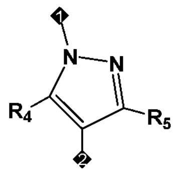 , 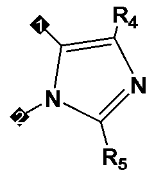 ,

or absent, wherein attachment point 2 is the wave line connected to ring B in Formula Vc";
$R_4$ and $R_5$ are each independently H, methyl, ethyl, n-propyl, isopropyl, cyclopropyl, or cyclobutyl; preferably $R_4$ and $R_5$ are both methyl; and
the wave line in Formula Vc" indicates a bond, and two of the three wave lines are absent;
or, wherein the compound has a structure represented by Formula Vc"-1:

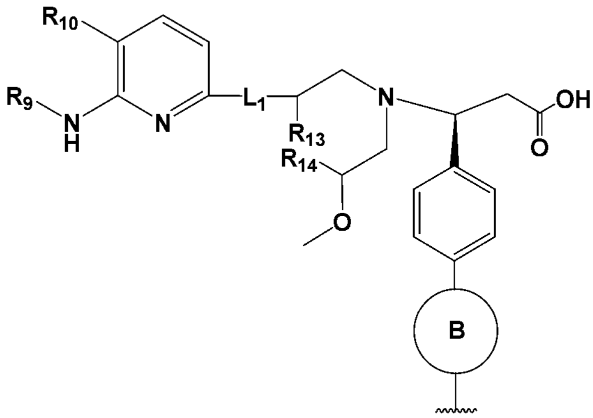

(Vc"-1)

wherein $R_9$, $R_{10}$, $R_{13}$, $R_{14}$, $L_1$, ring B, and the wave line have the same definitions as the corresponding substituents in Formula Vc";
or, wherein the compound has a structure represented by Formula Vc"-2 or Vc"-3:

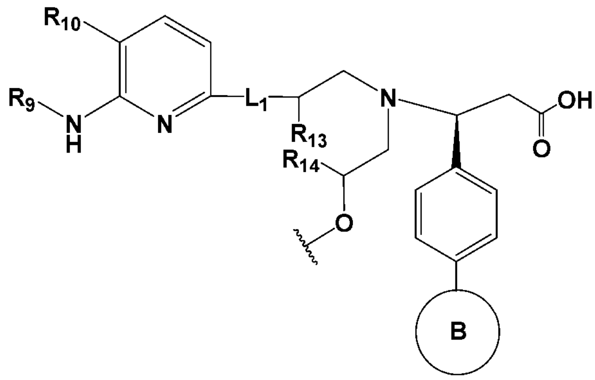

(Vc"-2)

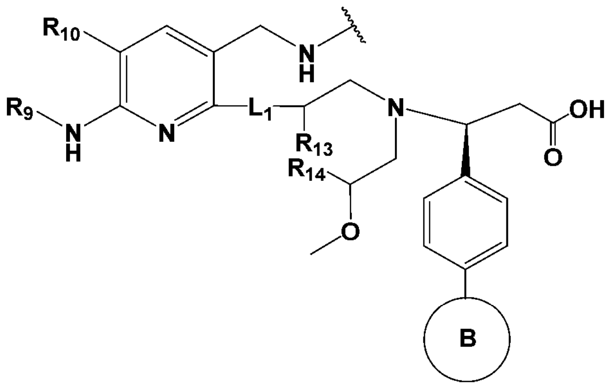

(Vc"-3)

wherein $R_9$, $R_{10}$, $R_{13}$, $R_{14}$, $L_1$, and the wave line have the same definitions as the corresponding substituents in Formula Vc", and
ring B is

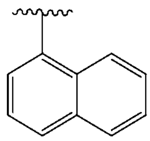 , 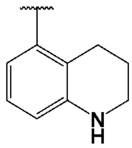 , 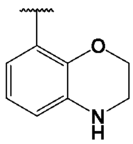 , 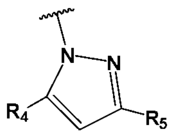 , 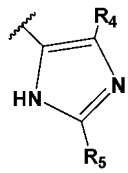 ,

or absent, wherein $R_4$ and $R_5$ are each independently H, methyl, ethyl, n-propyl, isopropyl, cyclopropyl, or cyclobutyl; preferably $R_4$ and $R_5$ are each independently methyl;
preferably, the compound is selected from: 68".

**28.** An RNAi agent, or a pharmaceutically acceptable salt, prodrug, or solvate thereof, wherein the RNAi agent has a structure represented by Formula VI, VIa, VIb, VIc, VId, VIe, VIf, VIg, VIh, VIi, VIj, or VIk:

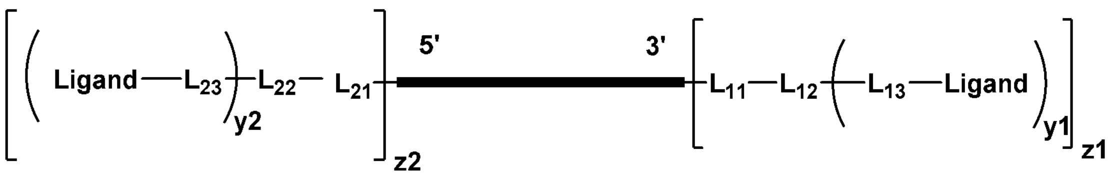

**(VI)**

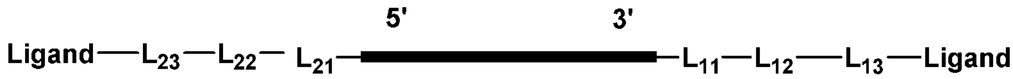

(VIa)

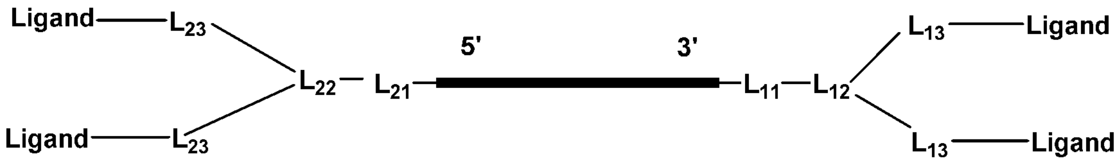

(VIb)

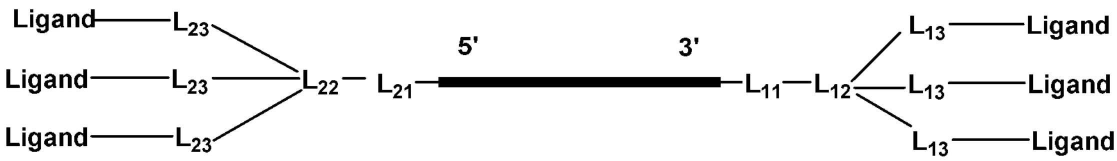

(VIc)

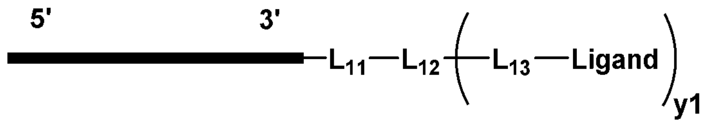

(VId)

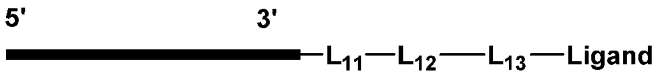

(VIe)

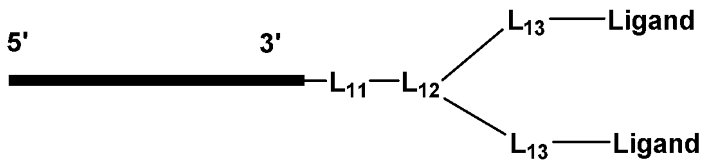

(VIf)

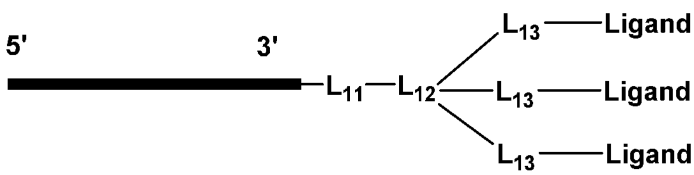

(VIg)

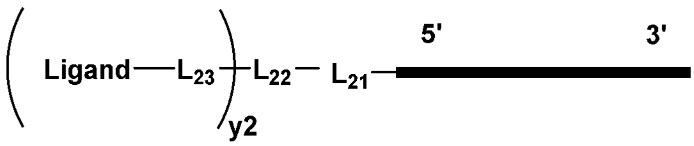

(VIh)

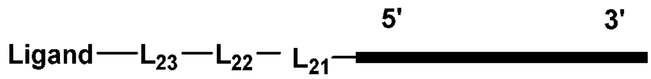

(VIi)

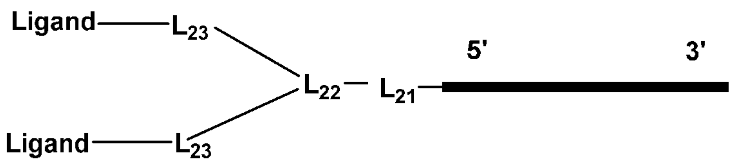

(VIj)

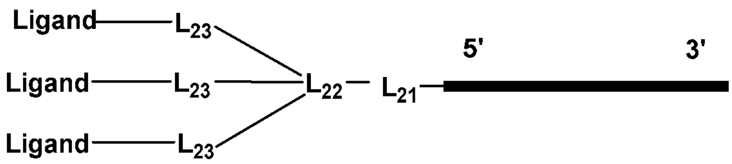

(VIk)

wherein,

is an oligonucleotide, preferably siRNA or ASO, the siRNA or ASO being connected to $L_{21}$ and/or $L_{11}$ via a phosphate group or phosphorothioate group;
z1 and z2 are each independently 0 or 1, and z1 and z2 are not both 0;
y1 and y2 are each independently 1, 2, or 3;
each Ligand is independently selected from Formula Ia" of claim 19, Formula Ib" or Ib"-1 of claim 20, Formula IIa" or IIb" of claim 21, Formula IIIa", IIIb", IIIa"-1, or IIIb"-1 of claim 22, Formula IVa" or IVa"-1 of claim 23, Formula IVb" of claim 24, Formula Va", Va"-1, Va"-2, or Va"-3 of claim 25, Formula Vb", Vb"-1, or Vb"-2 of claim 26, and Formula Vc", Vc"-1, Vc"-2, or Vc"-3 of claim 27;
$L_{11}$ and $L_{21}$ are each independently selected from:

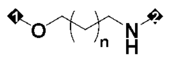
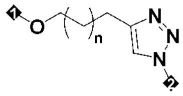
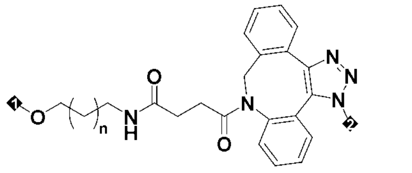
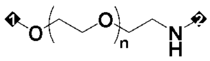
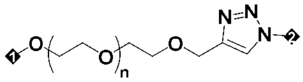
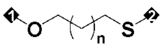
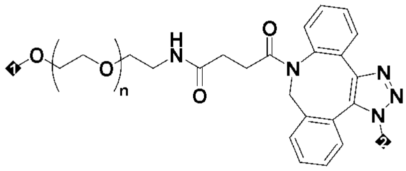
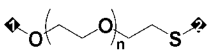
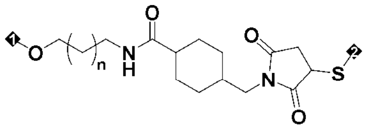
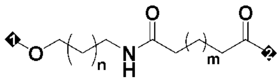
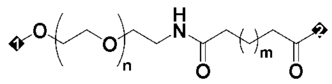
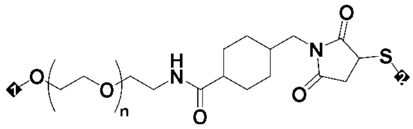
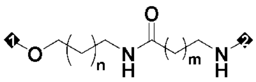
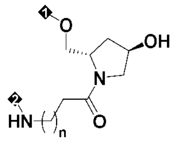
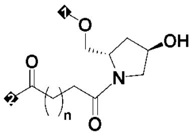
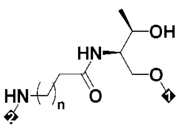
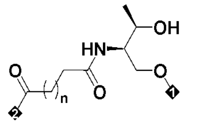
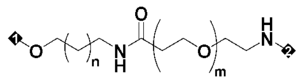
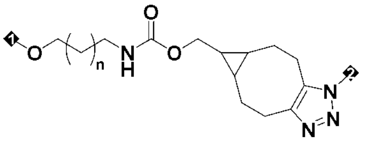
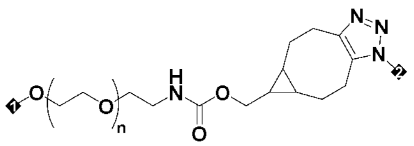

wherein n, m are integers from 0 to 10, preferably integers from 2 to 6, and attachment point 1 is connected to the oligonucleotide, attachment point 2 is connected to $L_{12}$ or $L_{22}$;
$L_{12}$ and $L_{22}$ are each independently a bond or a branching group, the branching group being independently selected from:

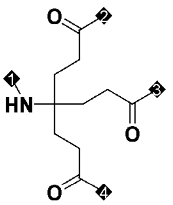
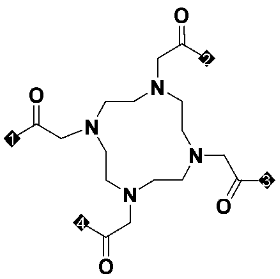
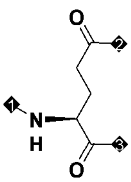
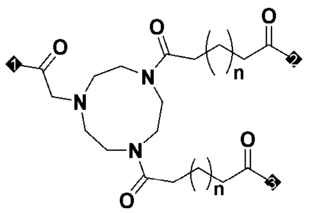

wherein n is an integer from 0 to 10, preferably an integer from 2 to 6, and attachment point 1 is connected to $L_{11}$ or $L_{21}$, attachment points 2, 3, and 4 are connected to $L_{13}$ or $L_{23}$;
$L_{13}$ and $L_{23}$ are each independently selected from:

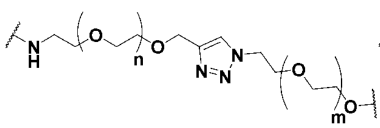
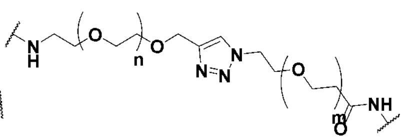
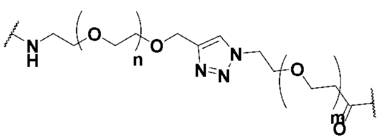

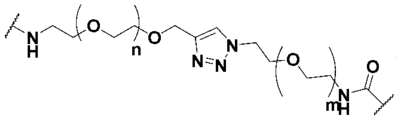
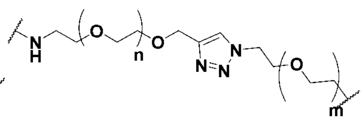
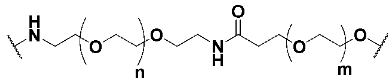

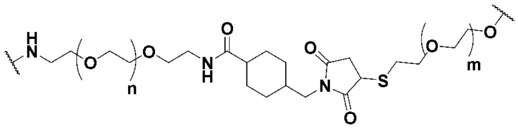
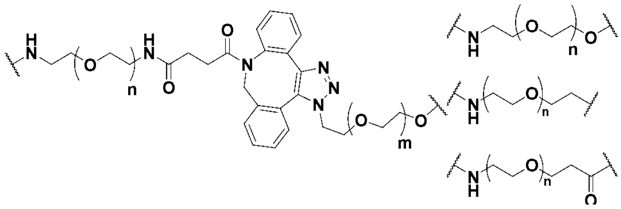

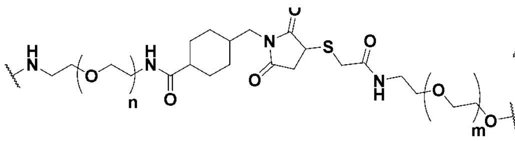
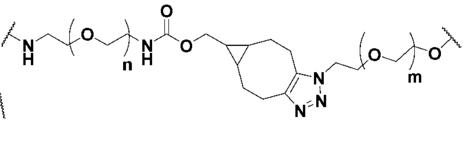
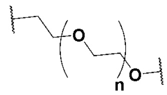

wherein n, m are integers from 0 to 10, preferably integers from 2 to 6, and the wave line on the left side of the formula is connected to $L_{12}$ or $L_{22}$, the wave line on the right side of the formula is connected to the Ligand; preferably, $L_{21}$, $L_{22}$ and $L_{23}$ together form a structure selected from the following, wherein the left side of the formula is connected to the oligonucleotide via a phosphate or phosphorothioate group:

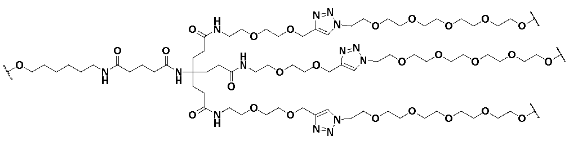
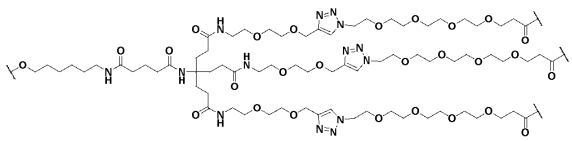

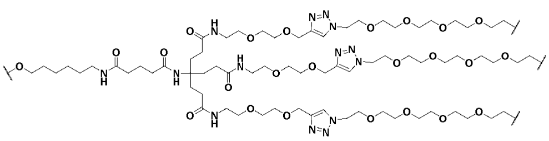
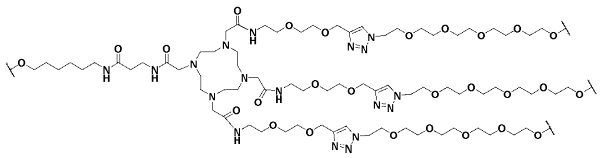

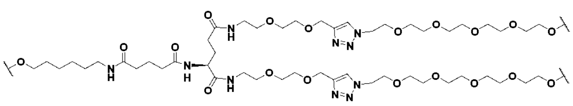
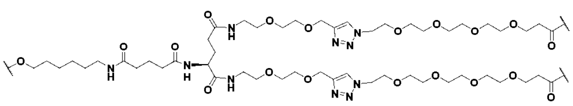

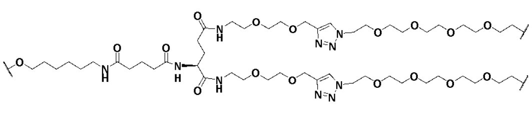
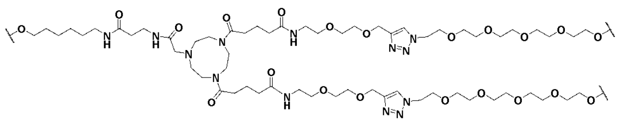

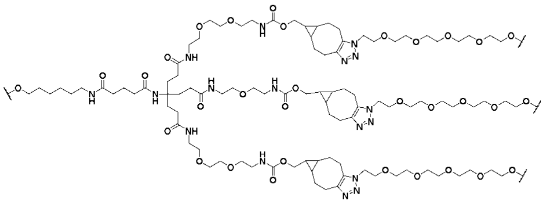
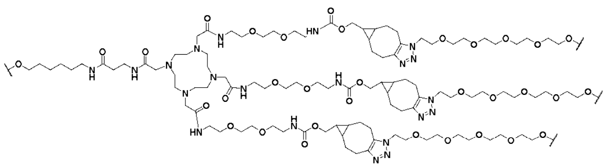

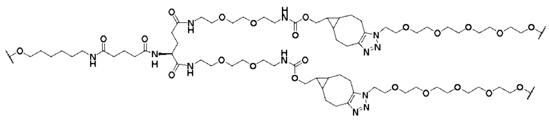
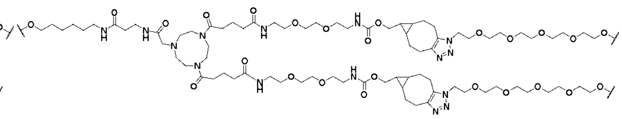

;

preferably, $L_{11}$, $L_{12}$ and $L_{13}$ together form a structure selected from the following, wherein the left side of the formula is connected to the oligonucleotide via a phosphate or phosphorothioate group:

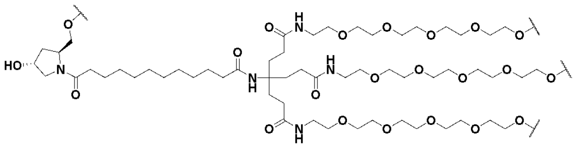
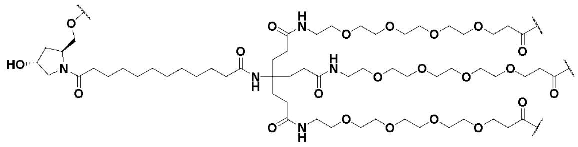

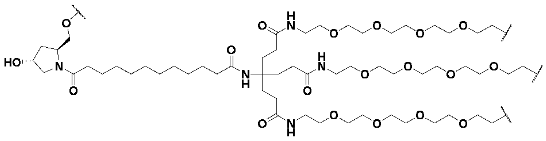
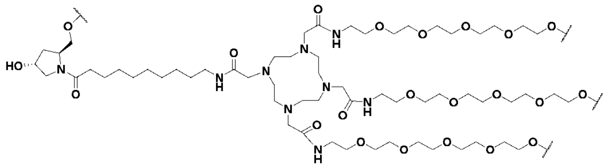

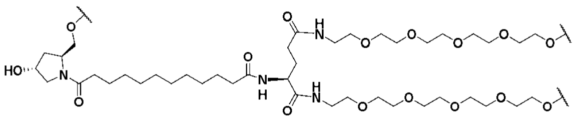
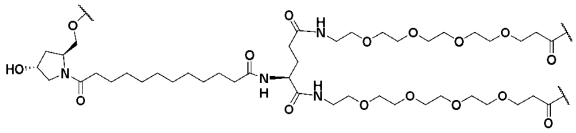

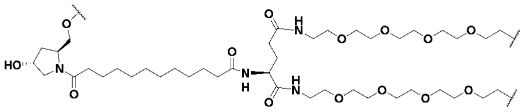
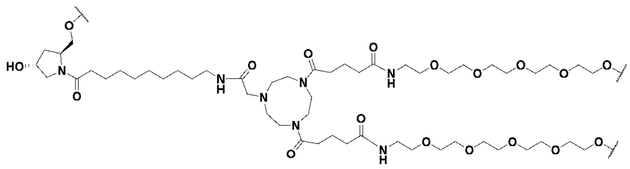

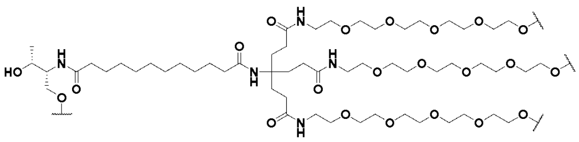
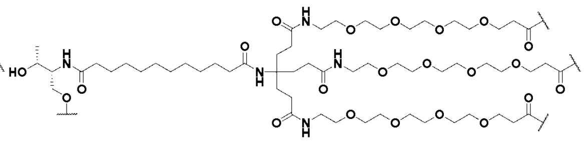

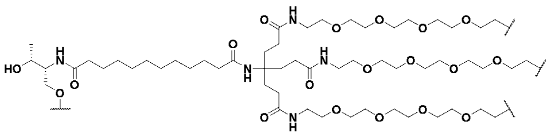
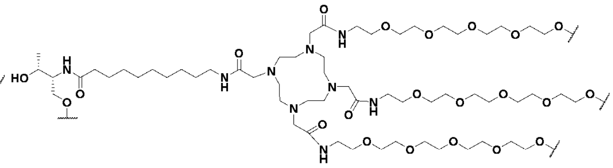

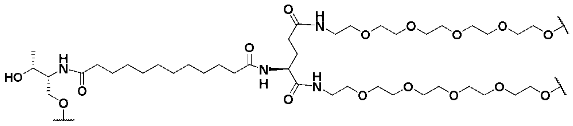
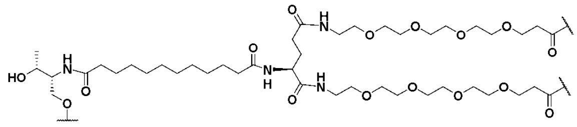

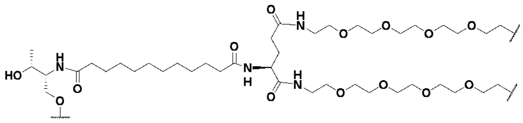
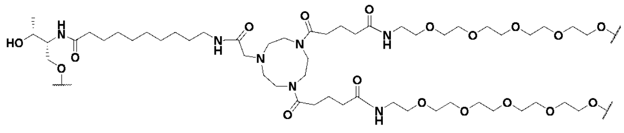

;

preferably, the structure of the RNAi agent is selected from Formulas 201-21, 202-21, 203-21, 204-21, 205-21, 206-21, 207-21, 208-21, 209-21, 210-21, 211-21, 212-21, 213-5, 214-5, 215-5, 216-5, 217-5, 218-5, 219-5, 220-5, 221-5, 222-5, 223-5, 224-5, 225-6, 226-6, 227-6, 228-6, 229-6, 230-6, 231-6, 232-6, 233-6, 234-6, 235-6, 236-6, 237-7, 238-7, 239-7, 240-7, 241-7, 242-7, 243-7, 244-7, 245-7, 246-7, 247-7, 248-7, 249-8, 250-8, 251-8, 252-8, 253-8, 254-8, 255-8, 256-8, 257-8, 258-8, 259-8, 260-8, 261-9, 262-9, 263-9, 264-9, 265-9, 266-9, 267-9, 268-9, 269-9, 270-9, 271-9, 272-9, 273-13, 274-13, 275-13, 276-13, 277-13, 278-13, 279-13, 280-13, 281-13, 282-13, 283-13, 284-13, 285-14, 286-14, 287-14, 288-14, 289-14, 290-14, 291-14, 292-14, 293-14, 294-14, 295-14, 296-14, 297-15, 298-15, 299-15, 300-15, 301-15, 302-15, 303-15, 304-15, 305-15, 306-15, 307-15, 308-15, 309-18, 310-18, 311-18, 312-18, 313-18, 314-18, 315-18, 316-18, 317-18, 318-18, 319-18, 320-18, 321-20, 322-20, 323-20, 324-20, 325-20, 326-20, 327-20, 328-20, 329-20, 330-20, 331-20, 332-20, 333-16, 334-16, 335-16, 336-16, 337-16, 338-16, 339-16, 340-16, 341-16, 342-16, 343-16, 344-16, 345-17, 346-17, 347-17, 348-17, 349-17, 350-17, 351-17, 352-17, 353-17, 354-17, 355-17, and 356-17.

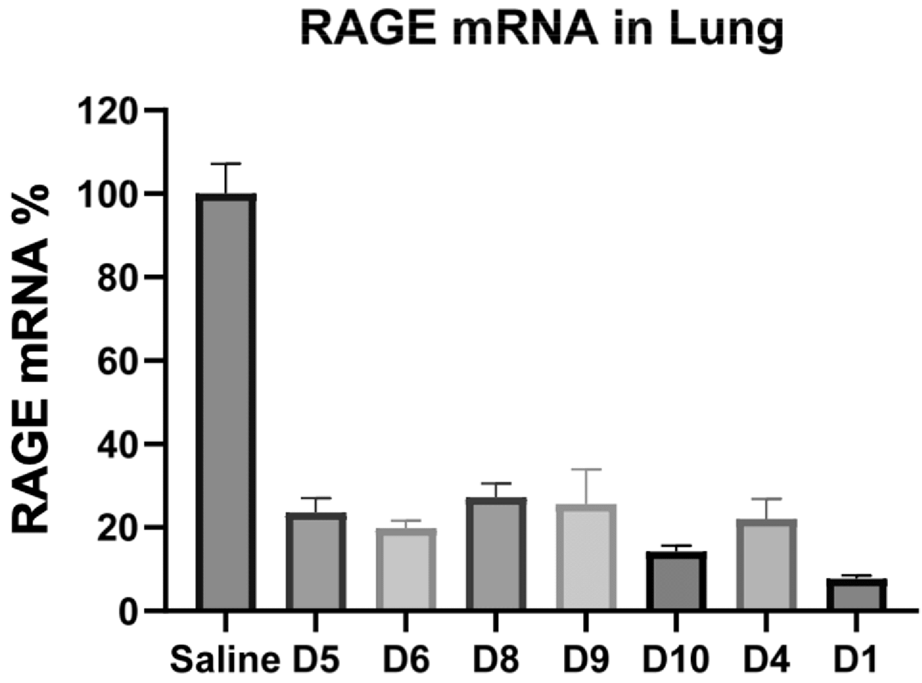
RAGE mRNA in Lung
RAGE mRNA %
120
100
80
60
40
20
0
Saline
D5
D6
D8
D9
D10
D4
D1

FIG. 1

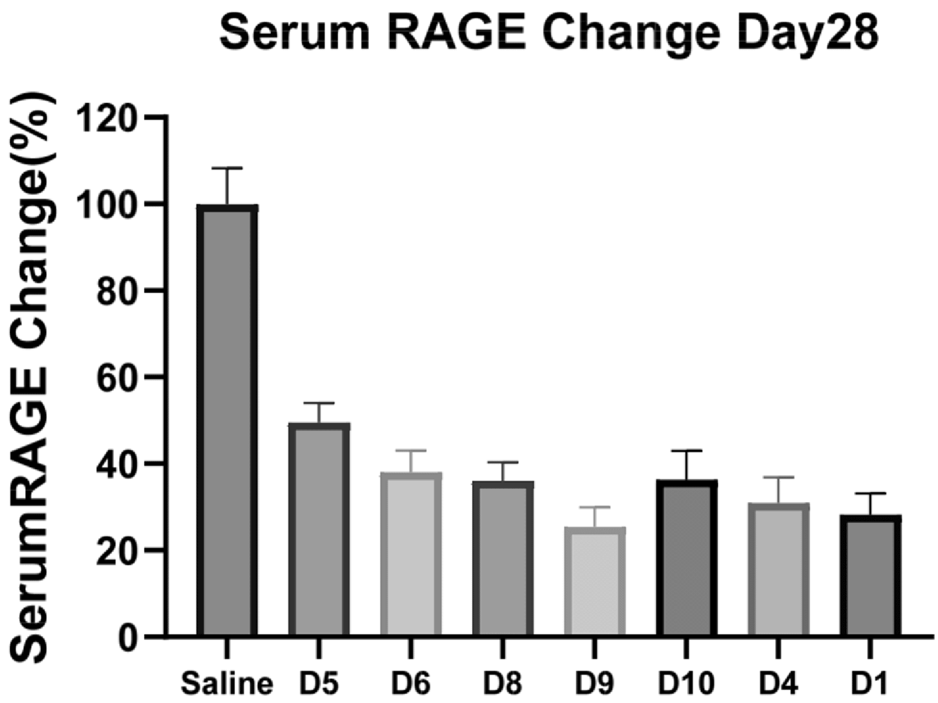
Serum RAGE Change Day28
SerumRAGE Change(%)
120
100
80
60
40
20
0
Saline
D5
D6
D8
D9
D10
D4
D1

FIG. 2

**INTERNATIONAL SEARCH REPORT**

International application No. **PCT/CN2024/121785**

**A. CLASSIFICATION OF SUBJECT MATTER**

C07D 213/74(2006.01)i; A61K31/44(2006.01)i

According to International Patent Classification (IPC) or to both national classification and IPC

**B. FIELDS SEARCHED**

Minimum documentation searched (classification system followed by classification symbols)

IPC: C07D, A61K

Documentation searched other than minimum documentation to the extent that such documents are included in the fields searched

Electronic data base consulted during the international search (name of data base and, where practicable, search terms used)

CNTXT, ENTXTC, CNKI, ENTXT, DWPI, ISI Web of Science, STN(REG, CAPLUS): 云合智药(苏州)生物科技有限公司; 靶向整合素; RNAi剂; 缀合小核酸; 递送; YUNHE ZHIYAO (SUZHOU) BIOTECHNOLOGY CO., LTD.: targeting integrin; RNAi agents; conjugation; small nucleic acid; delivery.

**C. DOCUMENTS CONSIDERED TO BE RELEVANT**

| Category* | Citation of document, with indication, where appropriate, of the relevant passages | Relevant to claim No. |
|---|---|---|
| A | CN 101023072 A (JANSSEN PHARMACEUTICA NV) 22 August 2007 (2007-08-22) abstract, claims 1-90, and description, embodiment 2 | 1-28 |
| A | CN 1688572 A (JANSSEN PHARMACEUTICA NV) 26 October 2005 (2005-10-26) abstract, and claims 1-69 | 1-28 |
| A | CN 102652023 A (GE HEALTHCARE LIMITED) 29 August 2012 (2012-08-29) abstract, and claims 1-32 | 1-28 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **31 December 2024** | **07 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/121785**

| Patent document cited in search report | Publication date (day/month/year) | Patent family member(s) | Publication date (day/month/year) |
|---|---|---|---|
| CN 101023072 A | 22 August 2007 | KR 20070010125 A | 22 January 2007 |
| | | MXPA 06009503 A | 19 April 2007 |
| | | WO 2005082889 A1 | 09 September 2005 |
| | | BRPI 0418552 A | 22 May 2007 |
| | | CA 2556768 A1 | 09 September 2005 |
| | | US 2008058359 A1 | 06 March 2008 |
| | | US 7879881 B2 | 01 February 2011 |
| | | NO 20064212 L | 15 November 2006 |
| | | IS 8532 A | 15 September 2006 |
| | | ECSP 066778 A | 16 November 2006 |
| | | EP 1718635 A1 | 08 November 2006 |
| | | ZA 200607756 B | 25 June 2008 |
| | | IL 177502 A0 | 10 December 2006 |
| | | JP 2007523907 A | 23 August 2007 |
| | | RS 20060470 A | 28 November 2008 |
| | | EA 200601517 A1 | 29 June 2007 |
| | | US 2011092528 A1 | 21 April 2011 |
| | | US 8110683 B2 | 07 February 2012 |
| | | US 2004224986 A1 | 11 November 2004 |
| | | HRP 20060298 A2 | 31 March 2007 |
| | | AU 2004316476 A1 | 09 September 2005 |
| | | MEP 48908 A | 10 February 2011 |
| CN 1688572 A | 26 October 2005 | US 2004077684 A1 | 22 April 2004 |
| | | UA 83467 C2 | 25 July 2008 |
| | | JP 2005539049 A | 22 December 2005 |
| | | JP 4554364 B2 | 29 September 2010 |
| | | DK 1539739 T3 | 07 March 2011 |
| | | CY 1111194 T1 | 11 June 2015 |
| | | BR 0313534 A | 12 July 2005 |
| | | SI 1539739 T1 | 29 April 2011 |
| | | WO 2004020435 A1 | 11 March 2004 |
| | | IL 166534 A0 | 15 January 2006 |
| | | IL 166534 A | 31 May 2012 |
| | | ATE 489378 T1 | 15 December 2010 |
| | | PL 375562 A1 | 28 November 2005 |
| | | PL 219749 B1 | 31 July 2015 |
| | | PT 1539739 E | 05 January 2011 |
| | | CA 2496127 A1 | 16 February 2005 |
| | | CA 2496127 C | 29 May 2012 |
| | | DE 60335118 D1 | 05 January 2011 |
| | | RU 2005104115 A | 27 August 2005 |
| | | RU 2333210 C2 | 10 September 2008 |
| | | AR 041010 A1 | 27 April 2005 |
| | | ES 2355139 T3 | 23 March 2011 |
| | | KR 20050031464 A | 06 April 2005 |
| | | AU 2003259891 A1 | 19 March 2004 |
| | | NO 20051273 L | 10 May 2005 |
| | | NO 332036 B1 | 04 June 2012 |
| | | MXPA 05001859 A | 03 June 2005 |
| | | EP 1539739 A1 | 15 June 2005 |

Form PCT/ISA/210 (patent family annex) (July 2022)

**INTERNATIONAL SEARCH REPORT**
**Information on patent family members**

International application No.
**PCT/CN2024/121785**

| Patent document cited in search report | Publication date (day/month/year) | Patent family member(s) | Publication date (day/month/year) |
|---|---|---|---|
| | | EP 1539739 B1 | 24 November 2010 |
| | | AU 2010236002 A1 | 11 November 2010 |
| | | AU 2010236002 B2 | 03 May 2012 |
| | | CR 7692 A | 14 January 2009 |
| CN 102652023 A | 29 August 2012 | JP 2013514326 A | 25 April 2013 |
| | | WO 2011073340 A1 | 23 June 2011 |
| | | US 2012244074 A1 | 27 September 2012 |
| | | EP 2512523 A1 | 24 October 2012 |
| | | GB 0922014 D0 | 03 February 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0003683 A
- US 5976567 A
- US 5981501 A
- US 6534484 B
- US 6586410 B
- US 6815432 B
- US 20100324120 A
- WO 9640964 A
- US 056230
- WO 2009127060 A, SNALP
- US 2010022614 W, XTC
- US 0963933 W
- WO 2010129709 A
- WO 2019089765 A1


### Non-patent literature cited in the description


- *Org Biomol Chem.*, 26 May 2016, vol. 14 (25), 5992-6009